# EUROPEAN PATENT APPLICATION

(11) **EP 1 792 912 A2**
(43) Date of publication of application: **06.06.2007**
(21) Application number: 06077108.6
(22) Date of filing: 01.04.2003
(51) Int. Cl.: C07K 14/00, A61K 38/17, G01N 33/53

(54) **Novel proteins and nucleic acids encoding same**

(30) Priority: 02.04.2002 US 115479; 05.04.2002 US 370349 P; 08.04.2002 US 370969 P; 12.04.2002 US 372019 P; 22.04.2002 US 374379 P; 30.05.2002 US 384543 P; 03.06.2002 US 160619; 15.08.2002 US 403748 P; 04.11.2002 US 287226; 31.03.2003 US 403161
(62) Divisional of application: 03726178.1
(71) Applicant: Curagen Corporation, Brandford, CT 06405 (US)
(72) Inventor: Anderson, David W., Branford, CT 06405 (US); Bento, Patricia, Wolcott, CT 06716 (US); Boldog, Ferenc L., North Haven, CT 06473 (US); Burgess, Catherine E., Wethersfield, CT 06109 (US); Casman, Stacie J., North Haven, CT 06473 (US); Furtak, Katarzyna, Ansonia, CT 06401 (US); Gorman, Linda, Branford, CT 06405 (US); Gould-Rothberg, Bonnie E., Guilford, CT 06437 (US); Gunther, Erik, Branford, CT 06405 (US); Heyes, Melvyn P., New Haven, CT 06512 (US); Li, Li, Branford, CT 06405 (US); Spytek, Kimberly A., Ellington, CT 06029 (US); Stone, David J., Guilford, CT 06437 (US); Zhong, Mei, Branford, CT 06405 (US); Malyanker, Uriel M., Branford, CT 06405 (US); Edinger, Shlomit R., New Haven, CT 06515 (US); Patturajan, Meera, Branford, CT 06405 (US); Rothenberg, Mark E., Clinton, CT 06413 (US); Smithson, Glenda, Guilford, CT 06435 (US)
(74) Representative: Peter, Beate

(57) **Abstract**

The present invention provides novel isolated polynucleotides and small molecule target polypeptides encoded by the polynucleotides. Antibodies that immunospecifically bind to a novel small molecule target polypeptide or any derivative, variant, mutant or fragment of that polypeptide, polynucleotide or antibody are disclosed, as are methods in which the small molecule target polypeptide, polynucleotide and antibody are utilized in the detection and treatment of a broad range of pathological states. More specifically, the present invention discloses methods of using recombinantly expressed and/or endogenously expressed proteins in various screening procedures for the purpose of identifying therapeutic antibodies and therapeutic small molecules associated with diseases. The invention further discloses therapeutic, diagnostic and research methods for diagnosis, treatment, and prevention of disorders involving any one of these novel human nucleic acids and proteins.

## Description

### FIELD OF THE INVENTION

The present invention relates to novel polypeptides that are targets of small molecule drugs and that have properties related to stimulation of biochemical or physiological responses in a cell, a tissue, an organ or an organism. More particularly, the novel polypeptides are gene products of novel genes, or are specified biologically active fragments or derivatives thereof. Methods of use encompass diagnostic and prognostic assay procedures as well as methods of treating diverse pathological conditions.

### BACKGROUND

Eukaryotic cells are characterized by biochemical and physiological processes which under normal conditions are exquisitely balanced to achieve the preservation and propagation of the cells. When such cells are components of multicellular organisms such as vertebrates, or more particularly organisms such as mammals, the regulation of the biochemical and physiological processes involves intricate signaling pathways. Frequently, such signaling pathways involve extracellular signaling proteins, cellular receptors that bind the signaling proteins and signal transducing components located within the cells.

Signaling proteins may be classified as endocrine effectors, paracrine effectors or autocrine effectors. Endocrine effectors are signaling molecules secreted by a given organ into the circulatory system, which are then transported to a distant target organ or tissue. The target cells include the receptors for the endocrine effector, and when the endocrine effector binds, a signaling cascade is induced. Paracrine effectors involve secreting cells and receptor cells in close proximity to each other, for example two different classes of cells in the same tissue or organ. One class of cells secretes the paracrine effector, which then reaches the second class of cells, for example by diffusion through the extracellular fluid. The second class of cells contains the receptors for the paracrine effector; binding of the effector results in induction of the signaling cascade that elicits the corresponding biochemical or physiological effect. Autocrine effectors are highly analogous to paracrine effectors, except that the same cell type that secretes the autocrine effector also contains the receptor. Thus the autocrine effector binds to receptors on the same cell, or on identical neighboring cells. The binding process then elicits the characteristic biochemical or physiological effect.

Signaling processes may elicit a variety of effects on cells and tissues including by way of nonlimiting example induction of cell or tissue proliferation, suppression of growth or proliferation, induction of differentiation or maturation of a cell or tissue, and suppression of differentiation or maturation of a cell or tissue.

Many pathological conditions involve dysregulation of expression of important effector proteins. In certain classes of pathologies the dysregulation is manifested as diminished or suppressed level of synthesis and secretion of protein effectors. In other classes of pathologies the dysregulation is manifested as increased or up-regulated level of synthesis and secretion of protein effectors. In a clinical setting a subject may be suspected of suffering from a condition brought on by altered or mis-regulated levels of a protein effector of interest. Therefore there is a need to assay for the level of the protein effector of interest in a biological sample from such a subject, and to compare the level with that characteristic of a nonpathological condition. There also is a need to provide the protein effector as a product of manufacture. Administration of the effector to a subject in need thereof is useful in treatment of the pathological condition. Accordingly, there is a need for a method of treatment of a pathological condition brought on by a diminished or suppressed levels of the protein effector of interest. In addition, there is a need for a method of treatment of a pathological condition brought on by a increased or up-regulated levels of the protein effector of interest.

Small molecule targets have been implicated in various disease states or pathologies. These targets may be proteins, and particularly enzymatic proteins, which are acted upon by small molecule drugs for the purpose of altering target function and achieving a desired result. Cellular, animal and clinical studies can be performed to elucidate the genetic contribution to the etiology and pathogenesis of conditions in which small molecule targets are implicated in a variety of physiologic, pharmacologic or native states. These studies utilize the core technologies at CuraGen Corporation to look at differential gene expression, protein-protein interactions, large-scale sequencing of expressed genes and the association of genetic variations such as, but not limited to, single nucleotide polymorphisms (SNPs) or splice variants in and between biological samples from experimental and control groups. The goal of such studies is to identify potential avenues for therapeutic intervention in order to prevent, treat the consequences or cure the conditions.

In order to treat diseases, pathologies and other abnormal states or conditions in which a mammalian organism has been diagnosed as being, or as being at risk for becoming, other than in a normal state or condition, it is important to identify new therapeutic agents. Such a procedure includes at least the steps of identifying a target component within an affected tissue or organ, and identifying a candidate therapeutic agent that modulates the functional attributes of the target. The target component may be any biological macromolecule implicated in the disease or pathology. Commonly the target is a polypeptide or protein with specific functional attributes. Other classes of macromolecule may be a nucleic acid, a polysaccharide, a lipid such as a complex lipid or a glycolipid; in addition a target may be a sub-cellular structure or extra-cellular structure that is comprised of more than one of these classes of macromolecule. Once such a target has been identified, it may be employed in a screening assay in order to identify favorable candidate therapeutic agents from among a large population of substances or compounds.

In many cases the objective of such screening assays is to identify small molecule candidates; this is commonly approached by the use of combinatorial methodologies to develop the population of substances to be tested. The implementation of high throughput screening methodologies is advantageous when working with large, combinatorial libraries of compounds.

### SUMMARY OF THE INVENTION

The invention includes nucleic acid sequences and the novel polypeptides they encode. The novel nucleic acids and polypeptides are referred to herein as NOVX, or NOV1, NOV2, NOV3, *etc.,* nucleic acids and polypeptides. These nucleic acids and polypeptides, as well as derivatives, homologs, analogs and fragments thereof, will hereinafter be collectively designated as "NOVX" nucleic acid, which represents the nucleotide sequence selected from the group consisting of SEQ ID NO: 2n-1, wherein n is an integer between 1 and 48, or polypeptide sequences, which represents the group consisting of SEQ ID NO: 2n, wherein n is an integer between 1 and 48.

In one aspect, the invention provides an isolated polypeptide comprising a mature form of a NOVX amino acid. One example is a variant of a mature form of a NOVX amino acid sequence, wherein any amino acid in the mature form is changed to a different amino acid, provided that no more than 15% of the amino acid residues in the sequence of the mature form are so changed. The amino acid can be, for example, a NOVX amino acid sequence or a variant of a NOVX amino acid sequence, wherein any amino acid specified in the chosen sequence is changed to a different amino acid, provided that no more than 15% of the amino acid residues in the sequence are so changed. The invention also includes fragments of any of these. In another aspect, the invention also includes an isolated nucleic acid that encodes a NOVX polypeptide, or a fragment, homolog, analog or derivative thereof.

Also included in the invention is a NOVX polypeptide that is a naturally occurring allelic variant of a NOVX sequence. In one embodiment, the allelic variant includes an amino acid sequence that is the translation of a nucleic acid sequence differing by a single nucleotide from a NOVX nucleic acid sequence. In another embodiment, the NOVX polypeptide is a variant polypeptide described therein, wherein any amino acid specified in the chosen sequence is changed to provide a conservative substitution. In one embodiment, the invention discloses a method for determining the presence or amount of the NOVX polypeptide in a sample. The method involves the steps of: providing a sample; introducing the sample to an antibody that binds immunospecifically to the polypeptide; and determining the presence or amount of antibody bound to the NOVX polypeptide, thereby determining the presence or amount of the NOVX polypeptide in the sample. In another embodiment, the invention provides a method for determining the presence of or predisposition to a disease associated with altered levels of a NOVX polypeptide in a mammalian subject. This method involves the steps of: measuring the level of expression of the polypeptide in a sample from the first mammalian subject; and comparing the amount of the polypeptide in the sample of the first step to the amount of the polypeptide present in a control sample from a second mammalian subject known not to have, or not to be predisposed to, the disease, wherein an alteration in the expression level of the polypeptide in the first subject as compared to the control sample indicates the presence of or predisposition to the disease.

In a further embodiment, the invention includes a method of identifying an agent that binds to a NOVX polypeptide. This method involves the steps of: introducing the polypeptide to the agent; and determining whether the agent binds to the polypeptide. In various embodiments, the agent is a cellular receptor or a downstream effector.

In another aspect, the invention provides a method for identifying a potential therapeutic agent for use in treatment of a pathology, wherein the pathology is related to aberrant expression or aberrant physiological interactions of a NOVX polypeptide. The method involves the steps of: providing a cell expressing the NOVX polypeptide and having a property or function ascribable to the polypeptide; contacting the cell with a composition comprising a candidate substance; and determining whether the substance alters the property or function ascribable to the polypeptide; whereby, if an alteration observed in the presence of the substance is not observed when the cell is contacted with a composition devoid of the substance, the substance is identified as a potential therapeutic agent. In another aspect, the invention describes a method for screening for a modulator of activity or of latency or predisposition to a pathology associated with the NOVX polypeptide. This method involves the following steps: administering a test compound to a test animal at increased risk for a pathology associated with the NOVX polypeptide, wherein the test animal recombinantly expresses the NOVX polypeptide. This method involves the steps of measuring the activity of the NOVX polypeptide in the test animal after administering the compound of step; and comparing the activity of the protein in the test animal with the activity of the NOVX polypeptide in a control animal not administered the polypeptide; wherein a change in the activity of the NOVX polypeptide in the test animal relative to the control animal indicates the test compound is a modulator of latency of, or predisposition to, a pathology associated with the NOVX polypeptide. In one embodiment, the test animal is a recombinant test animal that expresses a test protein transgene or expresses the transgene under the control of a promoter at an increased level relative to a wild-type test animal, and wherein the promoter is not the native gene promoter of the transgene. In another aspect, the invention includes a method for modulating the activity of the NOVX polypeptide, the method comprising introducing a cell sample expressing the NOVX polypeptide with a compound that binds to the polypeptide in an amount sufficient to modulate the activity of the polypeptide.

The invention also includes an isolated nucleic acid that encodes a NOVX polypeptide, or a fragment, homolog, analog or derivative thereof. In a preferred embodiment, the nucleic acid molecule comprises the nucleotide sequence of a naturally occurring allelic nucleic acid variant. In another embodiment, the nucleic acid encodes a variant polypeptide, wherein the variant polypeptide has the polypeptide sequence of a naturally occurring polypeptide variant. In another embodiment, the nucleic acid molecule differs by a single nucleotide from a NOVX nucleic acid sequence. In one embodiment, the NOVX nucleic acid molecule hybridizes under stringent conditions to the nucleotide sequence selected from the group consisting of SEQ ID NO: 2n-1, wherein n is an integer between I and 48, or a complement of the nucleotide sequence. In another aspect, the invention provides a vector or a cell expressing a NOVX nucleotide sequence.

In one embodiment, the invention discloses a method for modulating the activity of a NOVX polypeptide. The method includes the steps of: introducing a cell sample expressing the NOVX polypeptide with a compound that binds to the polypeptide in an amount sufficient to modulate the activity of the polypeptide. In another embodiment, the invention includes an isolated NOVX nucleic acid molecule comprising a nucleic acid sequence encoding a polypeptide comprising a NOVX amino acid sequence or a variant of a mature form of the NOVX amino acid sequence, wherein any amino acid in the mature form of the chosen sequence is changed to a different amino acid, provided that no more than 15% of the amino acid residues in the sequence of the mature form are so changed. In another embodiment, the invention includes an amino acid sequence that is a variant of the NOVX amino acid sequence, in which any amino acid specified in the chosen sequence is changed to a different amino acid, provided that no more than 15% of the amino acid residues in the sequence are so changed.

In one embodiment, the invention discloses a NOVX nucleic acid fragment encoding at least a portion of a NOVX polypeptide or any variant of the polypeptide, wherein any amino acid of the chosen sequence is changed to a different amino acid, provided that no more than 10% of the amino acid residues in the sequence are so changed. In another embodiment, the invention includes the complement of any of the NOVX nucleic acid molecules or a naturally occurring allelic nucleic acid variant. In another embodiment, the invention discloses a NOVX nucleic acid molecule that encodes a variant polypeptide, wherein the variant polypeptide has the polypeptide sequence of a naturally occurring polypeptide variant. In another embodiment, the invention discloses a NOVX nucleic acid, wherein the nucleic acid molecule differs by a single nucleotide from a NOVX nucleic acid sequence.

In another aspect, the invention includes a NOVX nucleic acid, wherein one or more nucleotides in the NOVX nucleotide sequence is changed to a different nucleotide provided that no more than 15% of the nucleotides are so changed. In one embodiment, the invention discloses a nucleic acid fragment of the NOVX nucleotide sequence and a nucleic acid fragment wherein one or more nucleotides in the NOVX nucleotide sequence is changed from that selected from the group consisting of the chosen sequence to a different nucleotide provided that no more than 15% of the nucleotides are so changed. In another embodiment, the invention includes a nucleic acid molecule wherein the nucleic acid molecule hybridizes under stringent conditions to a NOVX nucleotide sequence or a complement of the NOVX nucleotide sequence. In one embodiment, the invention includes a nucleic acid molecule, wherein the sequence is changed such that no more than 15% of the nucleotides in the coding sequence differ from the NOVX nucleotide sequence or a fragment thereof.

In a further aspect, the invention includes a method for determining the presence or amount of the NOVX nucleic acid in a sample. The method involves the steps of: providing the sample; introducing the sample to a probe that binds to the nucleic acid molecule; and determining the presence or amount of the probe bound to the NOVX nucleic acid molecule, thereby determining the presence or amount of the NOVX nucleic acid molecule in the sample. In one embodiment, the presence or amount of the nucleic acid molecule is used as a marker for cell or tissue type.

In another aspect, the invention discloses a method for determining the presence of or predisposition to a disease associated with altered levels of the NOVX nucleic acid molecule of in a first mammalian subject. The method involves the steps of: measuring the amount of NOVX nucleic acid in a sample from the first mammalian subject; and comparing the amount of the nucleic acid in the sample of step (a) to the amount of NOVX nucleic acid present in a control sample from a second mammalian subject known not to have or not be predisposed to, the disease; wherein an alteration in the level of the nucleic acid in the first subject as compared to the control sample indicates the presence of or predisposition to the disease.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences, their encoded polypeptides, antibodies, and other related compounds. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table A provides a summary of the NOVX nucleic acids and their encoded polypeptides

**TABLE A. Requences and Corresponding SEQ ID Numbers**

| **NOVX Assignment** | **Internal Identification** | **SEQ ID NO (nucleic acid)** | **SEQ ID NO (amino acid)** | **Homology** |
|---|---|---|---|---|
| NOV1a | CG164221-01 | 1 | 2 | Sequence 1 from Patent WO02074987 - Homo sapiens |
| NOV1b | CG164221-02 | 3 | 4 | Sequence 1 from Patent WO02074987 - Homo sapiens |
| NOV2a | CG180777-01 | 5 | 6 | Cell cycle related kinase - Homo sapiens |
| NOV2b | CG180777-02 | 7 | 8 | Cell cycle related kinase - Homo sapiens |
| NOV3a | CG181825-01 | 9 | 10 | Aquaporin 6 (Aquaporin-2 like) (hKID) - Homo sapiens |
| NOV4a | CG50183-01 | 11 | 12 | C-C chemokine receptor type 11 (C-C CKR-11) (CC-CKR-11) (CCR-11) (Chemokine receptor-like 1)(CCRL1) (CCX CKR) - Homo sapiens |
| NOV5a | CG50249-01 | 13 | 14 | Voltage gated potassium channel Kv3.2b (Potassium voltage-gated potassium channel subfamily C member 2) - Homo sapiens |
| NOV5b | CG50249-02 | 15 | 16 | Voltage gated potassium channel Kv3.2b (Potassium voltage-gated potassium channel subfamily C member 2) - Homo sapiens |
| NOV5c | CG50249-03 | 17 | 18 | Voltage gated potassium channel Kv3.2b (Potassium voltage-gated potassium channel subfamily C member 2) - Homo sapiens |
| NOV5d | CG50249-04 | 19 | 20 | Voltage gated potassium channel Kv3.2b (Potassium voltage-gated potassium channel subfamily C member 2) - Homo sapiens |
| NOV6a | CG54236-02 | 21 | 22 | Cysteinyl leukotriene receptor 2 (CysLTR2) (PSEC0146) (HG57) (HPN321) (hGPCR21) - Homo sapiens |
| NOV6b | CG54236-01 | 23 | 24 | Cysteinyl leukotriene receptor 2 (CysLTR2) (PSEC0146) (HG57) (HPN321) (hGPCR21) - Homo sapiens |
| NOV7a | CG54566-01 | 25 | 26 | Sequence 11 from Patent WO0194416 - Homo sapiens |
| NOV8a | CG55912-01 | 27 | 28 | Voltage-dependent calcium channel gamma-8 subunit (Neuronal voltage-gated calcium channel gamma-8 subunit) - Rattus norvegicus |
| NOV9a | CG56001-01 | 29 | 30 | D-beta-hydroxybutyrate dehydrogenase, mitochondrial precursor (EC 1.1.1.30) (BDH) (3-hydroxybutyrate dehydrogenase) - Homo sapiens |
| NOV9b | CG56001-02 | 31 | 32 | D-beta-hydroxybutyrate dehydrogenase, mitochondrial precursor (EC 1.1.1.30) (BDH) (3-hydroxybutyrate dehydrogenase) - Homo sapiens |
| NOV10a | CG56151-01 | 33 | 34 | Solute carrier family 2, facilitated glucose transporter, member 2 (Glucose transporter type 2, liver) - Homo sapiens |
| NOV10b | 246837923 | 35 | 36 | Solute carrier family 2, facilitated glucose transporter, member 2 (Glucose transporter type 2, liver) - Homo sapiens |
| NOV10c | 246837941 | 37 | 38 | Solute carrier family 2, facilitated glucose transporter, member 2 (Glucose transporter type 2, liver) - Homo sapiens |
| NOV10d | CG56151-02 | 39 | 40 | Solute carrier family 2, facilitated glucose transporter, member 2 (Glucose transporter type 2, liver) - Homo sapiens |
| NOV10e | CG56151-03 | 41 | 42 | Solute carrier family 2, facilitated glucose transporter, member 2 (Glucose transporter type 2, liver) - Homo sapiens |
| NOV10f | CG56151-04 | 43 | 44 | Solute carrier family 2, facilitated glucose transporter, member 2 (Glucose transporter type 2, liver) - Homo sapiens |
| NOV11a | CG56155-02 | 45 | 46 | Plasma kallikrein precursor (EC 3.4.21.34) (Plasma prekallikrein) (Kininogenin) (Fletcher factor) - Homo sapiens |
| NOV11b | 227803167 | 47 | 48 | Plasma kallikrein precursor (EC 3.4.21.34) (Plasma prekallikrein) (Kininogenin) (Fletcher factor) - Homo sapiens |
| NOV11c | CG56155-01 | 49 | 50 | Plasma kallikrein precursor (EC 3.4.21.34) (Plasma prekallikrein) (Kininogenin) (Fletcher factor) - Homo sapiens |
| NOV11d | CG56155-03 | 51 | 52 | Plasma kallikrein precursor (EC 3.4.21.34) (Plasma prekallikrein) (Kininogenin) (Fletcher factor) - Homo sapiens |
| NOV12a | CG56262-01 | 53 | 54 | Putative calcium binding transporter-Homo sapiens |
| NOV13a | CG56829-01 | 55 | 56 | Testis-specific serine/threonine kinase 3 (Similar to serine/threonine kinase) (TSSK3) - Homo sapiens |
| NOV14a | CG57183-01 | 57 | 58 | Fibroblast growth factor receptor 3 precursor (EC 2.7.1.112) (FGFR-3) - Homo sapiens |
| NOV14b | CG57183-02 | 59 | 60 | Fibroblast growth factor receptor 3 precursor (EC 2.7.1.112) (FGFR-3) - Homo sapiens |
| NOV15a | CG57341-01 | 61 | 62 | Sequence 2 from Patent WO0144446 - Homo sapiens |
| NOV16a | CG57460-01 | 63 | 64 | Hypothetical protein FLJ37478-Homo sapiens |
| NOV17a | CG57570-01 | 65 | 66 | Solute carrier family 41 member 1 - Homo sapiens |
| NOV18a | CG57758-02 | 67 | 68 | Na+coupled citrate transporter protein - Homo sapiens |
| NOV18b | CG57758-01 | 69 | 70 | Na+coupled citrate transporter protein - Homo sapiens |
| NOV18c | CG57758-03 | 71 | 72 | Na+coupled citrate transporter protein - Homo sapiens |
| NOV18d | CG57758-04 | 73 | 74 | Na+coupled citrate transporter protein - Homo sapiens |
| NOV18e | CG57758-05 | 75 | 76 | Na+coupled citrate transporter protein - Homo sapiens |
| NOV19a | CG59693-01 | 77 | 78 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19b | CG59693-02 | 79 | 80 | Aldo-keto reductase family 1 member Cl (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19c | CG59693-03 | 81 | 82 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19d | CG59693-04 | 83 | 84 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19e | CG59693-05 | 85 | 86 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19f | CG59693-06 | 87 | 88 | Aldo-keto reductase family 1 member C1 (EC 1.1.1-) (Trans-1,2-dihydrobenzene- 1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19g | CG59693-07 | 89 | 90 | Aldo-keto reductase family I member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19h | CG59693-08 | 91 | 92 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV19i | CG59693-09 | 93 | 94 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens |
| NOV20a | CG93188-01 | 95 | 96 | Adult male lung cDNA, RIKEN full-length enriched library, clone:1200003C15 product:hypothetical protein, full insert sequence - Mus musculus |

Table A indicates the homology of NOVX polypeptides to known protein families. Thus, the nucleic acids and polypeptides, antibodies and related compounds according to the invention corresponding to a NOVX as identified in column 1 of Table A will be useful in therapeutic and diagnostic applications implicated in, for example, pathologies and disorders associated with the known protein families identified in column 5 of Table A.

Pathologies, diseases, disorders and condition and the like that are associated with NOVX sequences include, but are not limited to, *e.g.,* cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, metabolic disturbances associated with obesity, transplantation, adrenoleukodystrophy, congenital adrenal hyperplasia, prostate cancer, diabetes, metabolic disorders, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, infectious disease, anorexia, cancer-associated cachexia, cancer, neurodegenerative disorders, Alzheimer's Disease, Parkinson's Disorder, immune disorders, hematopoietic disorders, and the various dyslipidemias, the metabolic syndrome X and wasting disorders associated with chronic diseases and various cancers, as well as conditions such as transplantation and fertility.

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOV-X nucleic acids and polypeptides can also be used to identify proteins that are members of the family to which the NOVX polypeptides belong.

Consistent with other known members of the family of proteins, identified in column 5 of Table A, the NOVX polypeptides of the present invention show homology to, and contain domains that are characteristic of, other members of such protein families. Details of the sequence relatedness and domain analysis for each NOVX are presented in Example A.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit diseases associated with the protein families listed in Table A.

The NOVX nucleic acids and polypeptides are also useful for detecting specific cell types. Details of the expression analysis for each NOVX are presented in Example C. Accordingly, the NOVX nucleic acids, polypeptides, antibodies and related compounds according to the invention will have diagnostic and therapeutic applications in the detection of a variety of diseases with differential expression in normal vs. diseased tissues, *e.g.,* detection of a variety of cancers. SNP analysis for each NOVX, if applicable, is presented in Example D.

Additional utilities for NOVX nucleic acids and polypeptides according to the invention are disclosed herein.

### NOVX clones

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

The NOVX genes and their corresponding encoded proteins are useful for preventing, treating or ameliorating medical conditions, *e.g.*, by protein or gene therapy. Pathological conditions can be diagnosed by determining the amount of the new protein in a sample or by determining the presence of mutations in the new genes. Specific uses are described for each of the NOVX genes, based on the tissues in which they are most highly expressed. Uses include developing products for the diagnosis or treatment of a variety of diseases and disorders.

The NOVX nucleic acids and proteins of the invention are useful in potential diagnostic and therapeutic applications and as a research tool. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration *in vitro* and *in vivo* (vi) a biological defense weapon.

In one specific embodiment, the invention includes an isolated polypeptide comprising an amino acid sequence selected from the group consisting of: (a) a mature form of the amino acid sequence selected from the group consisting of SEQ ID NO: 2n, wherein n is an integer between 1 and 48; (b) a variant of a mature form of the amino acid sequence selected from the group consisting of SEQ ID NO: 2n, wherein n is an integer between 1 and 48, wherein any amino acid in the mature form is changed to a different amino acid, provided that no more than 15% of the amino acid residues in the sequence of the mature form are so changed; (c) an amino acid sequence selected from the group consisting of SEQ ID NO: 2n, wherein n is an integer between 1 and 48; (d) a variant of the amino acid sequence selected from the group consisting of SEQ ID NO:2n, wherein n is an integer between 1 and 48 wherein any amino acid specified in the chosen sequence is changed to a different amino acid, provided that no more than 15% of the amino acid residues in the sequence are so changed; and (e) a fragment of any of (a) through (d).

In another specific embodiment, the invention includes an isolated nucleic acid molecule comprising a nucleic acid sequence encoding a polypeptide comprising an amino acid sequence selected from the group consisting of: (a) a mature form of the amino acid sequence given SEQ ID NO: 2n, wherein n is an integer between 1 and 48; (b) a variant of a mature form of the amino acid sequence selected from the group consisting of SEQ ID NO: 2n, wherein n is an integer between 1 and 48 wherein any amino acid in the mature form of the chosen sequence is changed to a different amino acid, provided that no more than 15% of the amino acid residues in the sequence of the mature form are so changed; (c) the amino acid sequence selected from the group consisting of SEQ ID NO: 2n, wherein n is an integer between 1 and 48; (d) a variant of the amino acid sequence selected from the group consisting of SEQ ID NO: 2n, wherein n is an integer between 1 and 48, in which any amino acid specified in the chosen sequence is changed to a different amino acid, provided that no more than 15% of the amino acid residues in the sequence are so changed; (e) a nucleic acid fragment encoding at least a portion of a polypeptide comprising the amino acid sequence selected from the group consisting of SEQ ID NO: 2n, wherein n is an integer between 1 and 48 or any variant of said polypeptide wherein any amino acid of the chosen sequence is changed to a different amino acid, provided that no more than 10% of the amino acid residues in the sequence are so changed; and (f) the complement of any of said nucleic acid molecules.

In yet another specific embodiment, the invention includes an isolated nucleic acid molecule, wherein said nucleic acid molecule comprises a nucleotide sequence selected from the group consisting of: (a) the nucleotide sequence selected from the group consisting of SEQ ID NO: 2n-1, wherein n is an integer between I and 48; (b) a nucleotide sequence wherein one or more nucleotides in the nucleotide sequence selected from the group consisting of SEQ ID NO: 2n-1, wherein n is an integer between 1 and 48 is changed from that selected from the group consisting of the chosen sequence to a different nucleotide provided that no more than 15% of the nucleotides are so changed; (c) a nucleic acid fragment of the sequence selected from the group consisting of SEQ ID NO: 2n-1, wherein n is an integer between 1 and 48; and (d) a nucleic acid fragment wherein one or more nucleotides in the nucleotide sequence selected from the group consisting of SEQ ID NO: 2n-1, wherein n is an integer between 1 and 48 is changed from that selected from the group consisting of the chosen sequence to a different nucleotide provided that no more than 15% of the nucleotides are so changed.

### NOVX Nucleic Acids and Polypeptides

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (*e.g.,* NOVX mRNAs) and fragments for use as PCR primers for the amplification and/or mutation ofNOVX nucleic acid molecules. As used herein, the term "nucleic acid molecule" is intended to include DNA molecules (*e.g.,* cDNA or genomic DNA), RNA molecules (*e.g.,* mRNA), analogs of the DNA or RNA generated using nucleotide analogs, and derivatives, fragments and homologs thereof. The nucleic acid molecule may be single-stranded or double-stranded, but preferably is comprised double-stranded DNA.

A NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product encoded by the corresponding gene.
Alternatively, it may be defined as the polypeptide, precursor or proprotein encoded by an ORF described herein. The product "mature" form arises, by way of nonlimiting example, as a result of one or more naturally occurring processing steps that may take place within the cell (*e.g.*, host cell) in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristylation or phosphorylation. In general, a mature polypeptide or protein may result from the operation of only one of these processes, or a combination of any of them.

The term "probe", as utilized herein, refers to nucleic acid sequences of variable length, preferably between at least about 10 nucleotides (nt), about 100 nt, or as many as approximately, *e.g.,* 6;000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- stranded or double-stranded and designed to have specificity in PCR, membrane-based hybridization technologies, or ELISA-like technologies.

The term "isolated" nucleic acid molecule, as used herein, is a nucleic acid that is separated from other nucleic acid molecules which are present in the natural source of the nucleic acid. Preferably, an "isolated" nucleic acid is free of sequences which naturally flank the nucleic acid (*i.e.,* sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5kb or 0.1 kb of nucleotide sequences which naturally flank the nucleic acid molecule in genomic DNA of the cell/tissue from which the nucleic acid is derived (*e.g.*, brain, heart, liver, spleen, *etc.*)*.* Moreover, an "isolated" nucleic acid molecule, such as a cDNA molecule, can be substantially free of other cellular material, or culture medium, or of chemical precursors or other chemicals.

A nucleic acid molecule of the invention, *e.g.*, a nucleic acid molecule having the nucleotide sequence of SEQ ID N0:2*n*-1, wherein n is an integer between 1 and 48; or a complement of this nucleotide sequence, can be isolated using standard molecular biology techniques and the sequence information provided herein. Using all or a portion of the nucleic acid sequence of SEQ ID NO:2*n*-1, wherein n is an integer between I and 48, as a hybridization probe, NOVX molecules can be isolated using standard hybridization and cloning techniques (*e.g.,* as described in Sambrook, et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template with appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e.g.,* using an automated DNA synthesizer.

As used herein, the term "oligonucleotide" refers to a series of linked nucleotide residues. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NO:2*n*-1, wherein n is an integer between 1 and 48, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NO:2*n*-1, wherein n is an integer between 1 and 48, or a portion of this nucleotide sequence (*e.g.,* a fragment that can be used as a probe or primer or a fragment encoding a biologically-active portion of a NOVX polypeptide). A nucleic acid molecule that is complementary to the nucleotide sequence of SEQ ID NO:2*n*-1, wherein n is an integer between 1 and 48, is one that is sufficiently complementary to the nucleotide sequence of SEQ ID NO:2*n*-1, wherein n is an integer between 1 and 48, that it can hydrogen bond with few or no mismatches to the nucleotide sequence shown in SEQ ID NO:2*n*-1, wherein n is an integer between 1 and 48, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

A "fragment" provided herein is defined as a sequence of at least 6 (contiguous) nucleic acids or at least 4 (contiguous) amino acids, a length sufficient to allow for specific hybridization in the case of nucleic acids or for specific recognition of an epitope in the case of amino acids, and is at most some portion less than a full length sequence. Fragments may be derived from any contiguous portion of a nucleic acid or amino acid sequence of choice.

A full-length NOVX clone is identified as containing an ATG translation start codon and an in-frame stop codon. Any disclosed NOVX nucleotide sequence lacking an ATG start codon therefore encodes a truncated C-terminal fragment of the respective NOVX polypeptide, and requires that the corresponding full-length cDNA extend in the 5' direction of the disclosed sequence. Any disclosed NOVX nucleotide sequence lacking an in-frame stop codon similarly encodes a truncated N-terminal fragment of the respective NOVX polypeptide, and requires that the corresponding full-length cDNA extend in the 3' direction of the disclosed sequence.

A "derivative" is a nucleic acid sequence or amino acid sequence formed from the native compounds either directly, by modification or partial substitution. An "analog" is a nucleic acid sequence or amino acid sequence that has a structure similar to, but not identical to, the native compound, *e.g.*, they differs from it in respect to certain components or side chains. Analogs may be synthetic or derived from a different evolutionary origin and may have a similar or opposite metabolic activity compared to wild type. A "homolog" is a nucleic acid sequence or amino acid sequence of a particular gene that is derived from different species.

Derivatives and analogs may be full length or other than full length. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the proteins under stringent, moderately stringent, or low stringent conditions. *See e.g.,* Ausubel, et al., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences include those sequences coding for isoforms of NOVX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for a NOVX polypeptide of species other than humans, including, but not limited to: vertebrates, and thus can include, *e.g*., frog, mouse, rat, rabbit, dog, cat cow, horse, and other organisms. Homologous nucleotide sequences also include, but are not limited to, naturally occurring allelic variations and mutations of the nucleotide sequences set forth herein.
A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NO:2*n*-1, wherein n is an integer between 1 and 48, as well as a polypeptide possessing NOVX biological activity. Various biological activities of the NOVX proteins are described below.

A NOVX polypeptide is encoded by the open reading frame ("ORF") of a NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, *e.g.,* a stretch of DNA that would encode a protein of 50 amino acids or more.

The nucleotide sequences determined from the cloning of the human NOVX genes allows for the generation of probes and primers designed for use in identifying and/or cloning NOVX homologues in other cell types, *e.g.,* from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence of SEQ ID NO.2*n*-1, wherein n is an integer between I and 48; or an anti-sense strand nucleotide sequence of SEQ ID NO:2*n*-1, wherein n is an integer between 1 and 48; or of a naturally occurring mutant of SEQ ID NO:2*n*-1, wherein n is an integer between 1 and 48.

Probes based on the human NOVX nucleotide sequences can be used to detect transcripts or genomic sequences encoding the same or homologous proteins. In various embodiments, the probe has a detectable label attached, *e.g.*, the label can be a radioisotope, a fluorescent compound, an enzyme, or an enzyme co-factor. Such probes can be used as a part of a diagnostic test kit for identifying cells or tissues which mis-express a NOVX protein, such as by measuring a level of a NOVX-encoding nucleic acid in a sample of cells from a subject *e.g.,* detecting NOVX mRNA levels or determining whether a genomic NOVX gene has been mutated or deleted.

"A polypeptide having a biologically-active portion of a NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion of SEQ ID NO:2*n*-1, wherein n is an integer between 1 and 48, that encodes a polypeptide having a NOVX biological activity (the biological activities of the NOVX proteins are described below), expressing the encoded portion ofNOVX protein (*e.g*., by recombinant expression in *vitro)* and assessing the activity of the encoded portion of NOVX.

### NOVX Nucleic Acid and Polypeptide Variants

The invention further encompasses nucleic acid molecules that differ from the nucleotide sequences of SEQ ID NO:2*n*-1, wherein n is an integer between 1 and 48, due to degeneracy of the genetic code and thus encode the same NOVX proteins as that encoded by the nucleotide sequences of SEQ ID NO:2*n*-1, wherein n is an integer between I and 48. In another embodiment, an isolated nucleic acid molecule of the invention has a nucleotide sequence encoding a protein having an amino acid sequence of SEQ ID NO:2*n*, wherein n is an integer between 1 and 48.

In addition to the human NOVX nucleotide sequences of SEQ ID NO:2*n*-1, wherein n is an integer between 1 and 48, it will be appreciated by those skilled in the art that DNA sequence polymorphisms that lead to changes in the amino acid sequences of the NOVX polypeptides may exist within a population (*e.g.,* the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding a NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from a human SEQ ID NO:2*n*-1, wherein n is an integer between 1 and 48, are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NO:2*n*-1, wherein n is an integer between 1 and 48. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least about 65% homologous to each other typically remain hybridized to each other.

Homologs (*i.e.,* nucleic acids encoding NOVX proteins derived from species other than human) or other related sequences (*e.g.,* paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% ofthe probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30 °C for short probes, primers or oligonucleotides (*e.g.,* 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to a sequence of SEQ ID NO:2*n*-1, wherein n is an integer between 1 and 48, corresponds to a naturally-occurring nucleic acid molecule. As used herein, a "naturally-occurring" nucleic acid molecule refers to an RNA or DNA molecule having a nucleotide sequence that occurs in nature (*e.g.,* encodes a natural protein).

In a second embodiment, a nucleic acid sequence that is hybridizable to the nucleic acid molecule comprising the nucleotide sequence of SEQ ID NO:2*n*-1, wherein n is an integer between 1 and 48, or fragments, analogs or derivatives thereof, under conditions of moderate stringency is provided. A non-limiting example of moderate stringency hybridization conditions are hybridization in 6X SSC, 5X Reinhardt's solution, 0.5% SDS and 100 mg/ml denatured salmon sperm DNA at 55 °C, followed by one or more washes in 1X SSC, 0.1% SDS at 37°C. Other conditions of moderate stringency that may be used are well-known within the art. *See, e.g.,* Ausubel, et al. (eds.), 1993, CURRENT PROTOCOLS IN MOLECULAR BIOLOGY, John Wiley & Sons, NY, and Krieger, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences of SEQ ID NO:2*n*-1, wherein n is an integer between 1 and 48, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g*., as employed for cross-species hybridizations). *See, e.g.,* Ausubel, et al. (eds.), 1993, CURRENT-PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA78: 6789-6792.

### Conservative Mutations

In addition to naturally-occurring allelic variants of NOVX sequences that may exist in the population, the skilled artisan will further appreciate that changes can be introduced by mutation into the nucleotide sequences of SEQ ID NO:2*n*-1, wherein n is an integer between 1 and 48, thereby leading to changes in the amino acid sequences of the encoded NOVX protein, without altering the functional ability of that NOVX protein. For example, nucleotide substitutions leading to amino acid substitutions at "non-essential" amino acid residues can be made in the sequence of SEQ ID NO:2*n*, wherein n is an integer between 1 and 48. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are not particularly amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from SEQ ID NO:2*n*-1, wherein n is an integer between 1 and 48, yet retain biological activity. In one embodiment, the isolated nucleic acid molecule comprises a nucleotide sequence encoding a protein, wherein the protein comprises an amino acid sequence at least about 40% homologous to the amino acid sequences of SEQ ID NO:2*n*, wherein n is an integer between 1 and 48. Preferably, the protein encoded by the nucleic acid molecule is at least about 60% homologous to SEQ ID NO:2*n*, wherein n is an integer between 1 and 48; more preferably at least about 70% homologous to SEQ ID NO:2*n*, wherein n is an integer between 1 and 48; still more preferably at least about 80% homologous to SEQ ID NO:2*n*, wherein n is an integer between 1 and 48; even more preferably at least about 90% homologous to SEQ ID NO:2*n*, wherein n is an integer between 1 and 48; and most preferably at least about 95% homologous to SEQ ID NO:2*n*, wherein n is an integer between 1 and 48.

An isolated nucleic acid molecule encoding a NOVX-protein homologous to the protein of SEQ ID NO:2*n*, wherein n is an integer between 1 and 48, can be created by introducing one or more nucleotide substitutions, additions or deletions into the nucleotide sequence of SEQ ID NO:2*n*-1, wherein n is an integer between 1 and 48, such that one or more amino acid substitutions, additions or deletions are introduced into the encoded protein.

Mutations can be introduced any one of SEQ ID NO:2*n*-1, wherein n is an integer between 1 and 48, by standard techniques, such as site-directed mutagenesis and PCR-mediated mutagenesis. Preferably, conservative amino acid substitutions are made at one or more non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains *(e.g.,* lysine, arginine, histidine), acidic side chains *(e.g.,* aspartic acid, glutamic acid), uncharged polar side chains *(e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, a non-essential amino acid residue in the NOVX protein is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of a nucleic acid of SEQ ID NO:2*n*-1, wherein n is an integer between 1 and 48, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, HFY, wherein the letters within each group represent the single letter amino acid code.

In one embodiment, a mutant NOVX protein can be assayed for (*i*) the ability to form protein:protein interactions with other NOVX proteins, other cell-surface proteins, or biologically-active portions thereof, (*ii*) complex formation between a mutant NOVX protein and a NOVX ligand; or (*iii*) the ability of a mutant NOVX protein to bind to an intracellular target protein or biologically-active portion thereof; (*e.g.,* avidin proteins).

In yet another embodiment, a mutant NOVX protein can be assayed for the ability to regulate a specific biological function (*e.g.,* regulation of insulin release).

### Interfering RNA

In one aspect of the invention, NOVX gene expression can be attenuated by RNA interference. One approach well-known in the art is short interfering RNA (siRNA) mediated gene silencing where expression products of a NOVX gene are targeted by specific double stranded NOVX derived siRNA nucleotide sequences that are complementary to at least a 19-25 nt long segment of the NOVX gene transcript, including the 5' untranslated (UT) region, the ORF, or the 3' UT region. *See, e.g.,* PCT applications WO00/44895, WO99/32619, WO01/75164, WO01/92513, WO 01/29058, WO01/89304, WO02/16620, and WO02/29858, each incorporated by reference herein in their entirety. Targeted genes can be a NOVX gene, or an upstream or downstream modulator of the NOVX gene. Nonlimiting examples of upstream or downstream modulators of a NOVX gene include, *e.g.,* a transcription factor that binds the NOVX gene promoter, a kinase or phosphatase that interacts with a NOVX polypeptide, and polypeptides involved in a NOVX regulatory pathway.

According to the methods of the present invention, NOVX gene expression is silenced using short interfering RNA. A NOVX polynucleotide according to the invention includes a siRNA polynucleotide. Such a NOVX siRNA can be obtained using a NOVX polynucleotide sequence, for example by processing the NOVX ribopolynucleotide sequence in a cell-free system, such as but not limited to a Drosophila extract, or by transcription of recombinant double stranded NOVX RNA or by chemical synthesis of nucleotide sequences homologous to a NOVX sequence. *See*, *e.g*., Tuschl, Zamore, Lehmann, Bartel and Sharp (1999), Genes &Dev. 13: 3191-3197, incorporated herein by reference in its entirety. When synthesized, a typical 0.2 micromolar-scale RNA synthesis provides about 1 milligram of siRNA, which is sufficient for 1000 transfection experiments using a 24-well tissue culture plate format.

The most efficient silencing is generally observed with siRNA duplexes composed of a 21-nt sense strand and a 21-nt antisense strand, paired in a manner to have a 2-nt 3' overhang. The sequence of the 2-nt 3' overhang makes an additional small contribution to the specificity of siRNA target recognition. The contribution to specificity is localized to the unpaired nucleotide adjacent to the first paired bases. In one embodiment, the nucleotides in the 3' overhang are ribonucleotides. In an alternative embodiment, the nucleotides in the 3' overhang are deoxyribonucleotides. Using 2'-deoxyribonucleotides in the 3' overhangs is as efficient as using ribonucleotides, but deoxyribonucleotides are often cheaper to synthesize and are most likely more nuclease resistant.

A contemplated recombinant expression vector of the invention comprises a NOVX DNA molecule cloned into an expression vector comprising operatively-linked regulatory sequences flanking the NOVX sequence in a manner that allows for expression (by transcription of the DNA molecule) of both strands. An RNA molecule that is antisense to NOVX mRNA is transcribed by a first promoter (*e.g*., a promoter sequence 3' of the cloned DNA) and an RNA molecule that is the sense strand for the NOVX mRNA is transcribed by a second promoter (*e.g*., a promoter sequence 5' of the cloned DNA). The sense and antisense strands may hybridize in vivo to generate siRNA constructs for silencing of the NOVX gene. Alternatively, two constructs can be utilized to create the sense and anti-sense strands of a siRNA construct. Finally, cloned DNA can encode a construct having secondary structure, wherein a single transcript has both the sense and complementary antisense sequences from the target gene or genes. In an example of this embodiment, a hairpin RNAi product is homologous to all or a portion of the target gene. In another example, a hairpin RNAi product is a siRNA. The regulatory sequences flanking the NOVX sequence may be identical or may be different, such that their expression may be modulated independently, or in a temporal or spatial manner.

In a specific embodiment, siRNAs are transcribed intracellularly by cloning the NOVX gene templates into a vector containing, *e.g.,* a RNA pol III transcription unit from the smaller nuclear RNA (snRNA) U6 or the human RNase P RNA H1. One example of a vector system is the GeneSuppressor™ RNA Interference kit (commercially available from Imgenex). The U6 and H1 promoters are members of the type III class of Pol III promoters. The +1 nucleotide of the U6-like promoters is always guanosine, whereas the +1 for H1 promoters is adenosine. The termination signal for these promoters is defined by five consecutive thymidines. The transcript is typically cleaved after the second uridine. Cleavage at this position generates a 3' UU overhang in the expressed siRNA, which is similar to the 3' overhangs of synthetic siRNAs. Any sequence less than 400 nucleotides in length can be transcribed by these promoter, therefore they are ideally suited for the expression of around 21-nucleotide siRNAs in, *e.g.,* an approximately 50-nucleotide RNA stem-loop transcript.

A siRNA vector appears to have an advantage over synthetic siRNAs where long term knock-down of expression is desired. Cells transfected with a siRNA expression vector would experience steady, long-term mRNA inhibition. In contrast, cells transfected with exogenous synthetic siRNAs typically recover from mRNA suppression within seven days or ten rounds of cell division. The long-term gene silencing ability of siRNA expression vectors may provide for applications in gene therapy.

In general, siRNAs are chopped from longer dsRNA by an ATP-dependent ribonuclease called DICER. DICER is a member of the RNase III family of double-stranded RNA-specific endonucleases. The siRNAs assemble with cellular proteins into an endonuclease complex. *In vitro* studies in Drosophila suggest that the siRNAs/protein complex (siRNP) is then transferred to a second enzyme complex, called an RNA-induced silencing complex (RISC), which contains an endoribonuclease that is distinct from DICER. RISC uses the sequence encoded by the antisense siRNA strand to find and destroy mRNAs of complementary sequence. The siRNA thus acts as a guide, restricting the ribonuclease to cleave only mRNAs complementary to one of the two siRNA strands.

A NOVX mRNA region to be targeted by siRNA is generally selected from a desired NOVX sequence beginning 50 to 100 nt downstream of the start codon. Alternatively, 5' or 3' UTRs and regions nearby the start codon can be used but are generally avoided, as these may be richer in regulatory protein binding sites. UTR-binding proteins and/or translation initiation complexes may interfere with binding of the siRNP or RISC endonuclease complex. An initial BLAST homology search for the selected siRNA sequence is done against an available nucleotide sequence library to ensure that only one gene is targeted. Specificity of target recognition by siRNA duplexes indicate that a single point mutation located in the paired region of an siRNA duplex is sufficient to abolish target mRNA degradation. See, Elbashir et al. 2001 EMBO J. 20(23):6877-88. Hence, consideration should be taken to accommodate SNPs, polymorphisms, allelic variants or species-specific variations when targeting a desired gene.

In one embodiment, a complete NOVX siRNA experiment includes the proper negative control. A negative control siRNA generally has the same nucleotide composition as the NOVX siRNA but lack significant sequence homology to the genome. Typically, one would scramble the nucleotide sequence of the NOVX siRNA and do a homology search to make sure it lacks homology to any other gene.

Two independent NOVX siRNA duplexes can be used to knock-down a target NOVX gene. This helps to control for specificity of the silencing effect. In addition, expression of two independent genes can be simultaneously knocked down by using equal concentrations of different NOVX siRNA duplexes, *e.g*., a NOVX siRNA and an siRNA for a regulator of a NOVX gene or polypeptide. Availability of siRNA-associating proteins is believed to be more limiting than target mRNA accessibility.

A targeted NOVX region is typically a sequence of two adenines (AA) and two thymidines (TT) divided by a spacer region of nineteen (N19) residues (*e.g*., AA(N19)TT). A desirable spacer region has a G/C-content of approximately 30% to 70%, and more preferably of about 50%. If the sequence AA(N19)TT is not present in the target sequence, an alternative target region would be AA(N21). The sequence of the NOVX sense siRNA corresponds to (N19)TT or N21, respectively. In the latter case, conversion of the 3' end of the sense siRNA to TT can be performed if such a sequence does not naturally occur in the NOVX polynucleotide. The rationale for this sequence conversion is to generate a symmetric duplex with respect to the sequence composition of the sense and antisense 3' overhangs. Symmetric 3' overhangs may help to ensure that the siRNPs are formed with approximately equal ratios of sense and antisense target RNA-cleaving siRNPs. *See, e.g.,* Elbashir, Lendeckel and Tuschl (2001). Genes & Dev. 15: 188-200, incorporated by reference herein in its entirely. The modification of the overhang of the sense sequence of the siRNA duplex is not expected to affect targeted mRNA recognition, as the antisense siRNA strand guides target recognition.

Alternatively, if the NOVX target mRNA does not contain a suitable AA(N21) sequence, one may search for the sequence NA(N21). Further, the sequence of the sense strand and antisense strand may still be synthesized as 5' (N19)TT, as it is believed that the sequence of the 3'-most nucleotide of the antisense siRNA does not contribute to specificity. Unlike antisense or ribozyme technology; the secondary structure of the target mRNA does not appear to have a strong effect on silencing. *See*, Harborth, et al. (2001) J. Cell Science 114: 4557-4565, incorporated by reference in its entirety.

Transfection of NOVX siRNA duplexes can be achieved using standard nucleic acid transfection methods, for example, OLIGOFECTAMINE Reagent (commercially available from Invitrogen). An assay for NOVX gene silencing is generally performed approximately 2 days after transfection. No NOVX gene silencing has been observed in the absence of transfection reagent, allowing for a comparative analysis of the wild-type and silenced NOVX phenotypes. In a specific embodiment, for one well of a 24-well plate, approximately 0.84 µg of the siRNA duplex is generally sufficient. Cells are typically seeded the previous day, and are transfected at about 50% confluence. The choice of cell culture media and conditions are routine to those of skill in the art, and will vary with the choice of cell type. The efficiency oftransfection may depend on the cell type, but also on the passage number and the confluency of the cells. The time and the manner of formation of siRNA-liposame complexes (*e.g*., inversion versus vortexing) are also critical. Low transfection efficiencies are the most frequent cause of unsuccessful NOVX silencing. The efficiency of transfection needs to be carefully examined for each new cell line to be used. Preferred cell are derived from a mammal, more preferably from a rodent such as a rat or mouse, and most preferably from a human. Where used for therapeutic treatment, the cells are preferentially autologous, although non-autologous cell sources are also contemplated as within the scope of the present invention.

For a control experiment, transfection of 0.84 µg single-stranded sense NOVX siRNA will have no effect on NOVX silencing, and 0.84 µg antisense siRNA has a weak silencing effect when compared to 0.84 µg of duplex siRNAs. Control experiments again allow for a comparative analysis of the wild-type and silenced NOVX phenotypes. To control for transfection efficiency, targeting of common proteins is typically performed, for example targeting of lamin A/C or transfection of a CMV-driven EGFP-expression plasmid (*e.g*., commercially available from Clontech). In the above example, a determination of the fraction of lamin A/C knockdown in cells is determined the next day by such techniques as immunofluorescence, Western blot, Northern blot or other similar assays for protein expression or gene expression. Lamin A/C monoclonal antibodies may be obtained from Santa Cruz Biotechnology.

Depending on the abundance and the half life (or turnover) of the targeted NOVX polynucleotide in a cell, a knock-down phenotype may become apparent after I to 3 days, or even later. In cases where no NOVX knock-down phenotype is observed, depletion of the NOVX polynucleotide may be observed by immunofluorescence or Western blotting. If the NOVX polynucleotide is still abundant after 3 days, cells need to be split and transferred to a fresh 24-well plate far re-transfection. If no knock-down of the targeted protein is observed, it may be desirable to analyze whether the target mRNA (NOVX or a NOVX upstream or downstream gene) was effectively destroyed by the transfected siRNA duplex. Two days after transfection, total RNA is prepared, reverse transcribed using a target-specific primer, and PCR-amplified with a primer pair covering at least one exon-exon junction in order to control for amplification of pre-mRNAs. RT/PCR of a non-targeted mRNA is also needed as control. Effective depletion of the mRNA yet undetectable reduction of target protein may indicate that a large reservoir of stable NOVX protein may exist in the cell. Multiple transfection in sufficiently long intervals may be necessary until the target protein is finally depleted to a point where a phenotype may become apparent. If multiple transfection steps are required, cells are split 2 to 3 days after transfection. The cells may be transfected immediately after splitting.

An inventive therapeutic method of the invention contemplates administering a NOVX siRNA construct as therapy to compensate for increased or aberrant NOVX expression or activity. The NOVX ribopolynucleotide is obtained and processed into siRNA fragments, or a NOVX siRNA is synthesized, as described above. The NOVX siRNA is administered to cells or tissues using known nucleic acid transfection techniques, as described above. A NOVX siRNA specific for a NOVX gene will decrease or knockdown NOVX transcription products, which will lead to reduced NOVX polypeptide production, resulting in reduced NOVX polypeptide activity in the cells or tissues.

The present invention also encompasses a method of treating a disease or condition associated with the presence of a NOVX protein in an individual comprising administering to the individual an RNAi construct that targets the mRNA of the protein (the mRNA that encodes the protein) for degradation. A specific RNAi construct includes a siRNA or a double stranded gene transcript that is processed into siRNAs. Upon treatment, the target protein is not produced or is not produced to the extent it would be in the absence of the treatment.

Where the NOVX gene function is not correlated with a known phenotype, a control sample of cells or tissues from healthy individuals provides a reference standard for determining NOVX expression levels. Expression levels are detected using the assays described, *e.g.,* RT-PCR, Northern blotting, Western blotting, ELISA, and the like. A subject sample of cells or tissues is taken from a mammal, preferably a human subject, suffering from a disease state. The NOVX ribopolynucleotide is used to produce siRNA constructs, that are specific for the NOVX gene product. These cells or tissues are treated by administering NOVX siRNA's to the cells or tissues by methods described for the transfection of nucleic acids into a cell or tissue, and a change in NOVX polypeptide or polynucleotide expression is observed in the subject sample relative to the control sample, using the assays described. This NOVX gene knockdown approach provides a rapid method for determination of a NOVX minus (NOVX⁻) phenotype in the treated subject sample. The NOVX⁻ phenotype observed in the treated subject sample thus serves as a marker for monitoring the course of a disease state during treatment.

In specific embodiments, a NOVX siRNA is used in therapy. Methods for the generation and use of a NOVX siRNA are known to those skilled in the art. Example techniques are provided below.

### Production ofRNAs

Sense RNA (ssRNA) and antisense RNA (asRNA) of NOVX are produced using known methods such as transcription in RNA expression vectors. In the initial experiments, the sense and antisense RNA are about 500 bases in length each. The produced ssRNA and asRNA (0.5 µM) in 10 mM Tris-HCl (pH 7.5) with 20 mM NaCl were heated to 95° C for 1 min then cooled and annealed at room temperature for 12 to 16 h. The RNAs are precipitated and resuspended in lysis buffer (below). To monitor annealing, RNAs are electrophoresed in a 2% agarose gel in TBE buffer and stained with ethidium bromide. See, *e.g*., Sambrook et al., Molecular Cloning. Cold Spring Harbor Laboratory Press, Plainview, N.Y. (1989).

### Lysate Preparation

Untreated rabbit reticulocyte lysate (Ambion) are assembled according to the manufacturer's directions. dsRNA is incubated in the lysate at 30° C for 10 min prior to the addition of mRNAs. Then NOVX mRNAs are added and the incubation continued for an additional 60 min. The molar ratio of double stranded RNA and mRNA is about 200:1. The NOVX mRNA is radiolabeled (using known techniques) and its stability is monitored by gel electrophoresis.

In a parallel experiment made with the same conditions, the double stranded RNA is internally radiolabeled with a ³²P-ATP. Reactions are stopped by the addition of 2X-proteinase-K buffer and deproteinized as described previously (Tuschl et al., Genes Dev., 13:3191-3197 (1999)). Products are analyzed by electrophoresis in 15% or 18% polyacrylamide sequencing gels using appropriate RNA standards. By monitoring the gels for radioactivity, the natural production of 10 to 25 nt RNAs from the double stranded RNA can be determined.

The band of double stranded RNA, about 21-23 bps, is eluded. The efficacy of these 21-23 mers for suppressing NOVX transcription is assayed in vitro using the same rabbit reticulocyte assay described above using 50 nanomolar of double stranded 21-23 mer for each assay. The sequence of these 21-23 mers is then determined using standard nucleic acid sequencing techniques.

### RNA Preparation

21 nt RNAs, based on the sequence determined above, are chemically synthesized using Expedite RNA phosphoramidites and thymidine phosphoramidite (Proligo, Germany). Synthetic oligonucleotides are deprotected and gel-purified (Elbashir, Lendeckel, & Tuschl, Genes & Dev. 15, 188-200 (2001)), followed by Sep-Pak C18 cartridge (Waters, Milford, Mass., USA) purification (Tuschl, et al., Biochemistry, 32:11658-11668 (1993)).

These RNAs (20 µM) single strands are incubated in annealing buffer (100 mM potassium acetate, 30 mM HEPES-KOH at pH 7.4, 2 mM magnesium acetate) for I min at 90° C followed by 1 h at 37° C.

### Cell Culture

A cell culture known in the art to regularly express NOVX is propagated using standard conditions. 24 hours before transfection, at approx. 80% confluency, the cells are trypsinized and diluted 1:5 with fresh medium without antibiotics (1-3 X 105 cells/ml) and transferred to 24-well plates (500 ml/well). Transfection is performed using a commercially available lipofection kit and NOVX expression is monitored using standard techniques with positive and negative control. A positive control is cells that naturally express NOVX while a negative control is cells that do not express NOVX. Base-paired 21 and 22 nt siRNAs with overhanging 3' ends mediate efficient sequence-specific mRNA degradation in lysates and in cell culture. Different concentrations of siRNAs are used. An efficient concentration for suppression in vitro in mammalian culture is between 25 nM to 100 nM final concentration. This indicates that siRNAs are effective at concentrations that are several orders of magnitude below the concentrations applied in conventional antisense or ribozyme gene targeting experiments.

The above method provides a way both for the deduction of NOVX siRNA sequence and the use of such siRNA for in vitro suppression. In vivo suppression may be performed using the same siRNA using well known in-vivo transfection or gene therapy transfection techniques.

### Antisense Nucleic Acids

Another aspect of the invention pertains to isolated antisense nucleic acid molecules that are hybridizable to or complementary to the nucleic acid molecule comprising the nucleotide sequence of SEQ ID N0:2*n*-1, wherein n is an integer between 1 and 48, or fragments, analogs or derivatives thereof. An "antisense" nucleic acid comprises a nucleotide sequence that is complementary to a "sense" nucleic acid encoding a protein (*e.g*., complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of SEQ ID NO:2*n,* wherein n is an integer between 1 and 48, or antisense nucleic acids complementary to a NOVX nucleic acid sequence of SEQ ID NO:2*n*-1, wherein n is an integer between 1 and 48, are additionally provided.

In one embodiment, an antisense nucleic acid molecule is antisense to a "coding region" of the coding strand of a nucleotide sequence encoding a NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences that flank the coding region that are not translated into amino acids (*i.e.,* also referred to as 5' and 3' untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g*., an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g*., phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-carboxymethylaminomethyl-2-thiouridine, 5-(carboxyhydroxylmethyl) uracil, 5-carboxymethylaminomethyluracil, dihydrouracil, beta-D-galactosylqueosine, inosine, N6-isopentenyladenine, I-methylguanine, 1-methylinosine, 2,2-dimethylguanine, 2-methyladenine, 2-methylguanine, 5-methoxyuracil, 3-methylcytosine, 5-methylcytosine, N6-adenine, 7-methylguanine, 5-methylaminomethyluracil, 5-methoxyaminomethyl-2-thiouracil, 2-thiouracil, 4-thiouracil, beta-D-mannosylqueosine, 5'-methoxycarboxymethyluracil, 2-methylthio-N6-isopentenyladenine, uracil-5-oxyacetic acid (v), wybutoxosine, pseudouracil, queosine, 2-thiocytosine, 5-methyl-2-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diamiriopurine. Alternatively, the antisense nucleic acid can be produced biologically using an expression vector into which a nucleic acid has been subcloned in an antisense orientation (*i.e.,* RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein (*e.g.,* by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g*., by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. *See, e.g.,* Gaultier, et al., 1987. Nucl. Acids Res. 15: 6625-6641. The antisense nucleic acid molecule can also comprise a 2'-o-methylribonucleotide (*See, e.g.,* Inoue, et al. 1987. Nucl. Acids.Res. 15: 6131-6148) or a chimeric RNA-DNA analogue *(See, e.g.,* Inoue, et al., 1987. FEBS Lett. 215: 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.,* hammerhead ribozymes as described in Haselhoff and Gerlach 1988. Nature 334: 585-591) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX cDNA disclosed herein (*i.e.,* SEQ ID NO:2*n*-1, wherein n is an integer between 1 and 48). For example, a derivative of a *Tetrahymena* L-19 IVS RNA can be constructed in which the nucleotide sequence of the active site is complementary to the nucleotide sequence to be cleaved in a NOVX-encoding mRNA. *See, e.g.,* U.S. Patent 4,987,071 to Cech, et al. and U.S. Patent 5,116,742 to Cech, et al. NOVX mRNA can also be used to select a catalytic RNA having a specific ribonuclease activity from a pool of RNA molecules. *See, e.g.,* Bartel et al., (1993) Science 261:1411-1418.

Alternatively, NOVX gene expression can be inhibited by targeting nucleotide sequences complementary to the regulatory region of the NOVX nucleic acid (*e.g.,* the NOVX promoter and/or enhancers) to form triple helical structures that prevent transcription of the NOVX gene in target cells. *See, e.g.,* Helene, 1991. Anticancer Drug Des. 6: 569-84; Helene, et al. 1992. Ann. N.Y. Acad. Sci. 660: 27-36; Maher, 1992. Bioassays 14: 807-15.

In various embodiments, the NOVX nucleic acids can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids. *See, e.g.,* Hyrup, et al., 1996. Bioorg Med Chem 4: 5-23. As used herein, the terms "peptide nucleic acids" or "PNAs" refer to nucleic acid mimics (*e.g.,* DNA mimics) in which the deoxyribose phosphate backbone is replaced by a pseudopeptide backbone and only the four natural nucleotide bases are retained. The neutral backbone of PNAs has been shown to allow for specific hybridization to DNA and RNA under conditions of low ionic strength. The synthesis of PNA oligomer can be performed using standard solid phase peptide synthesis protocols as described in Hyrup, *et al.,* 1996. *supra;* Perry-O'Keefe, et al., 1996. Proc. Natl. Acad. Sci. USA 93: 14670-14675.

PNAs of NOVX can be used in therapeutic and diagnostic applications. For example, PNAs can be used as antisense or antigene agents for sequence-specific modulation of gene expression by, *e.g.,* inducing transcription or translation arrest or inhibiting replication. PNAs of NOVX can also be used, for example, in the analysis of single base pair mutations in a gene (*e.g.,* PNA directed PCR clamping; as artificial restriction enzymes when used in combination with other enzymes, *e.g.,* S₁ nucleases *(See,* Hyrup, *et al.,* 1996. *supra*), or as probes or primers for DNA sequence and hybridization *(See,* Hyrup, *et al.,* 1996, *supra;* Perry-O'Keefe, *et al.,* 1996. *supra*).

In another embodiment, PNAs of NOVX can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (*e.g*., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleotide bases, and orientation *(see,* Hyrup, et al., 1996. *supra*). The synthesis of PNA-DNA chimeras can be performed as described in Hyrup, *et al.,* 1996. *supra* and Finn, et al., 1996. Nucl Acids Res 24: 3357-3363. For example, a DNA chain can be synthesized on a solid support using standard phosphoramidite coupling chemistry, and modified nucleoside analogs, *e.g.,* 5'-(4-methoxytrityl)-amino-5'-deoxy-thymidine phosphoramidite, can be used between the PNA and the 5' end of DNA. *See, e.g.,* Mag, et al., 1989. Nucl Acid Res 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5' DNA segment and a 3' PNA segment. *See, e.g.,* Petersen, et al., 1975. Bioorg. Med. Chem. Lett. 5: 1119-11124.

In other embodiments, the oligonucleotide may include other appended groups such as peptides (*e.g.,* for targeting host cell receptors *in vivo),* or agents facilitating transport across the cell membrane *(see, e.g.,* Letsinger, et al., 1989. Proc. Natl. Acad. Sci. U.S.A. 86: 6553-6556; Lemaitre, et al., 1987. Proc. Natl. Acad. Sci. 84: 648-652; PCT Publication No. WO88/09810) or the blood-brain barrier *(see, e.g.,* PCT Publication No. WO 89/10134). In addition, oligonucleotides can be modified with hybridization-triggered cleavage agents *(see, e.g.,* Krol, et al., 1988. BioTechniques 6:958-976) or intercalating agents *(see, e.g.,* Zon, 1988. Pharm. Res. 5: 539-549). To this end, the oligonucleotide may be conjugated to another molecule, *e.g.,* a peptide, a hybridization triggered cross-linking agent, a transport agent, a hybridization-triggered cleavage agent, and the like.

### NOVX Polypeptides

A polypeptide according to the invention includes a polypeptide including the amino acid sequence of NOVX polypeptides whose sequences are provided in any one of SEQ ID NO:2*n*, wherein n is an integer between 1 and 48. The invention also includes a mutant or variant protein any of whose residues may be changed from the corresponding residues shown in any one of SEQ ID NO:2*n*, wherein n is an integer between 1 and 48, while still encoding a protein that maintains its NOVX activities and physiological functions, or a functional fragment thereof.

In general, a NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) of non-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, *i.e*., culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e.g.,* the amino acid sequence of SEQ ID NO:2*n*, wherein n is an integer between 1 and 48) that include fewer amino acids than the full-length NOVX proteins, and exhibit at least one activity of a NOVX protein. Typically, biologically-active portions comprise a domain or motif with at least one activity of the NOVX protein. A biologically-active portion of a NOVX protein can be a a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence of SEQ ID NO:2*n,* wherein n is an integer between 1 and 48. In other embodiments, the NOVX protein is substantially homologous to SEQ ID NO:2*n,* wherein n is an integer between I and 48, and retains the functional activity of the protein of SEQ ID NO:2*n*, wherein n is an integer between 1 and 48, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence of SEQ ID NO:2*n*, wherein n is an integer between 1 and 48, and retains the functional activity of the NOVX proteins of SEQ ID NO:2*n*, wherein n is an integer between 1 and 48.

### Determining Homology Between Two or More Sequences

To determine the percent homology of two amino acid sequences or of two nucleic acids, the sequences are aligned for optimal comparison purposes (*e.g*., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence of SEQ ID NO:2*n*-1, wherein n is an integer between 1 and 48.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g.,* A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides NOVX chimeric or fusion proteins. As used herein, a NOVX "chimeric protein" or "fusion protein" comprises a NOVX polypeptide operatively-linked to a non-NOVX polypeptide. An "NOVX polypeptide" refers to a polypeptide having an amino acid sequence corresponding to a NOVX protein of SEQ ID NO:2*n*, wherein n is an integer between 1 and 48, whereas a "non-NOVX polypeptide" refers to a polypeptide having an amino acid sequence corresponding to a protein that is not substantially homologous to the NOVX protein, *e.g.,* a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically-active portion of a NOVX protein. In another embodiment, a NOVX fusion protein comprises at least two biologically-active portions of a NOVX protein. In yet another embodiment, a NOVX fusion protein comprises at least three biologically-active portions of a NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is a NOVX protein containing a heterologous signal sequence at its N-terminus. In certain host cells (*e.g*., mammalian host cells), expression and/or secretion of NOVX can be increased through use of a heterologous signal sequence.

In yet another embodiment, the fusion protein is a NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo.* The NOVX-immunoglobulin fusion proteins can be used to affect the bioavailability of a NOVX cognate ligand. Inhibition of the NOVX ligand/NOVX interaction may be useful therapeutically for both the treatment of proliferative and differentiative disorders, as well as modulating (*e.g*., promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence *(see, e.g.,* Ausubel, et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide). A NOVX-encoding nucleic acid can be cloned into such an expression vector such that the fusion moiety is linked in-frame to the NOVX protein.

### NOVX Agonists and Antagonists

The invention also pertains to variants of the NOVX proteins that function as either NOVX agonists (*i.e.,* mimetics) or as NOVX antagonists. Variants of the NOVX protein can be generated by mutagenesis (*e.g*., discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists (*i.e.,* mimetics) or as NOVX antagonists can be identified by screening combinatorial libraries of mutants (*e.g.,* truncation mutants) of the NOVX proteins for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e.g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art. *See, e.g.,* Narang, 1983. Tetrahedron 39: 3; Itakura, et al., 1984. Annu. Rev. Biochem. 53: 323; Itakura, et al., 1984. Science 198: 1056; Ike, et al., 1983. Nucl. Acids Res. 11: 477.

### Polypeptide Libraries

In addition, libraries of fragments of the NOVX protein coding sequences can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e.g.,* Arkin and Yourvan, 1992. Proc. Natl. Acad. Sci. USA 89: 7811-7815; Delgrave, et al., 1993. Protein Engineering 6:327-331.

### Anti-NOVX Antibodies

Included in the invention are antibodies to NOVX proteins, or fragments of NOVX proteins. The term "antibody" as used herein refers to immunoglobulin molecules and immunologically active portions of immunoglobulin (Ig) molecules, *i.e.,* molecules that contain an antigen-binding site that specifically binds (immunoreacts with) an antigen. Such antibodies include, but are not limited to, polyclonal, monoclonal, chimeric, single chain, F_{ab}, F_{ab'} and F(_{ab'})₂ fragments, and an F_{ab} expression library. In general, antibody molecules obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated protein of the invention intended to serve as an antigen, or a portion or fragment thereof, can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence of SEQ ID NO:2*n*, wherein n is an integer between 1 and 48, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX that is located on the surface of the protein, *e.g*., a hydrophilic region. A hydrophobicity analysis of the human NOVX protein sequence will indicate which regions of a NOVX polypeptide are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g.,* Hopp and Woods, 1981, Proc. Nat. Acad. Sci. USA 78: 3824-3828; Kyte and Doolittle 1982, J. Mol. Biol. 157:105-142, each incorporated herein by reference in their entirety. Antibodies that are specific for one or more domains within an antigenic protein, or derivatives, fragments, analogs or homologs thereof, are also provided herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three-dimensional structural characteristics, as well as specific charge characteristics. A NOVX polypeptide or a fragment thereof comprises at least one antigenic epitope. An anti-NOVX antibody of the present invention is said to specifically bind to antigen NOVX when the equilibrium binding constant (K_{D}) is ≤1 µM, preferably ≤ 100 nM, more preferably ≤ 10 nM, and most preferably ≤ 100 pM to about 1 pM, as measured by assays such as radioligand binding assays or similar assays known to those skilled in the art.

A protein ofthe invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (*e.g*., rabbit, goat, mouse or other mammal) maybe immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (*e.g.,* aluminum hydroxide), surface active substances (*e.g.,* lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (*e.g*., from the blood) and further purified by well known techniques, such as affinity chromatography using protein A or protein G, which provide primarily the IgG fraction of immune serum. Subsequently, or alternatively, the specific antigen that is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, MONOCLONAL ANTIBODIES: PRINCIPLES AND PRACTICE, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). It is an objective, especially important in therapeutic applications of monoclonal antibodies, to identify antibodies having a high degree of specificity and a high binding affinity for the target antigen.

After the desired hybridoma cells are identified, the clones can be subcloned by limiting dilution procedures and grown by standard methods (Goding,1986). Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (*e.g.,* by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies essentially relate to antibody molecules in which the entire sequence of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals. For example, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison ( Nature 368, 812-13 (1994)); Fishwild et al,( Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse^{™} as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see *e.g*., U.S. Patent No. 4,946,778). In addition, methods can be adapted for the construction of F_{ab} expression libraries (see *e.g.,* Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CHI) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers that are recovered from recombinant cell culture. The preferred interface comprises at least a part of the CH3 region of an antibody constant domain. In this method, one or more small amino acid side chains from the interface of the first antibody molecule are replaced with larger side chains (*e.g*., tyrosine or tryptophan). Compensatory "cavities" of identical or similar size to the large side chain(s) are created on the interface of the second antibody molecule by replacing large amino acid side chains with smaller ones (*e.g*., alanine or threonine). This provides a mechanism for increasing the yield of the heterodimer over other unwanted end-products such as homodimers.

Bispecific antibodies can be prepared as full length antibodies or antibody fragments (*e.g.,* F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker that is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (*e.g*., CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, *e.g.,* the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176. 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g*., avidin) that is in turn conjugated to a cytotoxic agent.

### Immunoliposomes

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### Diagnostic Applications of Antibodies Directed Against the Proteins of the Invention

In one embodiment, methods for the screening of antibodies that possess the desired specificity include, but are not limited to, enzyme linked immunosorbent assay (ELISA) and other immunologically mediated techniques known within the art. In a specific embodiment, selection of antibodies that are specific to a particular domain of an NOVX protein is facilitated by generation of hybridomas that bind to the fragment of an NOVX protein possessing such a domain. Thus, antibodies that are specific for a desired domain within an NOVX protein, or derivatives, fragments, analogs or homologs thereof, are also provided herein.

Antibodies directed against a NOVX protein of the invention may be used in methods known within the art relating to the localization and/or quantitation of a NOVX protein (*e.g.,* for use in measuring levels of the NOVX protein within appropriate physiological samples, for use in diagnostic methods, for use in imaging the protein, and the like). In a given embodiment, antibodies specific to a NOVX protein, or derivative, fragment, analog or homolog thereof, that contain the antibody derived antigen binding domain, are utilized as pharmacologically active compounds (referred to hereinafter as "Therapeutics").

An antibody specific for a NOVX protein of the invention (*e.g*., a monoclonal antibody or a polyclonal antibody) can be used to isolate a NOVX polypeptide by standard techniques, such as immunoaffinity, chromatography or immunoprecipitation. An antibody to a NOVX polypeptide can facilitate the purification of a natural NOVX antigen from cells, or of a recombinantly produced NOVX antigen expressed in host cells. Moreover, such an anti-NOVX antibody can be used to detect the antigenic NOVX protein (*e.g.,* in a cellular lysate or cell supernatant) in order to evaluate the abundance and pattern of expression of the antigenic NOVX protein. Antibodies directed against a NOVX protein can be used diagnostically to monitor protein levels in tissue as part of a clinical testing procedure, *e.g.,* to, for example, determine the efficacy of a given treatment regimen. Detection can be facilitated by coupling (*i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Antibody Therapeutics

Antibodies of the invention, including polyclonal, monoclonal, humanized and fully human antibodies, may used as therapeutic agents. Such agents will generally be employed to treat or prevent a disease or pathology in a subject. An antibody preparation, preferably one having high specificity and high affinity for its target antigen, is administered to the subject and will generally have an effect due to its binding with the target. Such an effect may be one of two kinds, depending on the specific nature of the interaction between the given antibody molecule and the target antigen in question. In the first instance, administration ofthe antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand that may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight. Common dosing frequencies may range, for example, from twice daily to once a week.

### Pharmaceutical Compositions of Antibodies

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington : The Science And Practice Of Pharmacy 19th ed. (Alfonso R. Gennaro, et al., editors) Mack Pub. Co., Easton, Pa.: 1995; Drug Absorption Enhancement : Concepts, Possibilities, Limitations, And Trends, Harwood Academic Publishers, Langhome, Pa., 1994; and Peptide And Protein Drug Delivery (Advances In Parenteral Sciences, Vol. 4), 1991, M. Dekker, New York.

If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco et al., Proc. Natl. Acad. Sci. USA, 90: 7889-7893 (1993). The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for in vivo administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, *e.g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### ELISA Assay

An agent for detecting an analyte protein is an antibody capable of binding to an analyte protein, preferably an antibody with a detectable label. Antibodies can be polyclonal, or more preferably, monoclonal. An intact antibody, or a fragment thereof (*e.g.,* F_{ab} or F_{(ab)2}) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of the invention can be used to detect an analyte mRNA, protein, or genomic DNA in a biological sample *in vitro* as well as *in vivo.* For example, *in vitro* techniques for detection of an analyte mRNA include Northern hybridizations and *in situ* hybridizations. *In vitro* techniques for detection of an analyte protein include enzyme linked immunosorbent assays (ELISAs), Western blots, immunoprecipitations, and immunofluorescence. *In vitro* techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Theory of Enzyme Immunoassays", P. Tijssen, Elsevier Science Publishers, Amsterdam, 1985. Furthermore, *in vivo* techniques for detection of an analyte protein include introducing into a subject a labeled anti-an analyte protein antibody. For example, the antibody can be labeled with a radioactive marker whose presence and location in a subject can be detected by standard imaging techniques.

### NOVX Recombinant Expression Vectors and Host Cells

Another aspect of the invention pertains to vectors, preferably expression vectors, containing a nucleic acid encoding a NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, useful expression vectors in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to includes promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g.,* tissue-specific regulatory sequences). It will be appreciated by those skilled in the art that the design of the expression vector can depend on such factors as the choice of the host cell to be transformed, the level of expression ofprotein desired, *etc.* The expression vectors of the invention can be introduced into host cells to thereby produce proteins or peptides, including fusion proteins or peptides, encoded by nucleic acids as described herein (*e.g.,* NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d *(*Studier et al., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g.,* Wada, et al., 1992. Nucl. Acids Res. 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, et al., 1987. EMBO J. 6: 229-234), pMFa (Kurjan and Herskowitz, 1982. Cell 30: 933-943), pJRY88 *(*Schultz et al., 1987. Gene54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells *(e.g.,* SF9 cells) include the pAc series (Smith, et al., 1983. Mol. Cell. Biol. 3:2156-2165) and the pVL series (Lucklow and Summers, 1989. Virology 170: 31-39).

In yet another embodiment, a nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g.,* Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters (*e.g.,* the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.,* the murine box promoters (Kessel and Gruss, 1990. Science 249: 374-379) and the α-fetoprotein promoter (Campes and Tilghman, 1989. Genes Dev. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i.e*., express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, *etc.* A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g.,* an embryonic cell of the animal, prior to development of the animal.

A transgenic animal of the invention can be created by introducing a NOVX-encoding nucleic acid into the male pronuclei of a fertilized oocyte (*e.g.,* by microinjection, retroviral infection) and allowing the oocyte to develop in a pseudopregnant female foster animal. The human NOVX cDNA sequences, *i.e.,* any one of SEQ ID NO:2*n*-1, wherein n is an integer between 1 and 48, can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of a NOVX gene into which a deletion, addition or substitution has been introduced to thereby alter, *e.g.,* functionally disrupt, the NOVX gene. The NOVX gene can be a human gene (*e.g.,* the cDNA of any one of SEQ ID NO:2*n*-1, wherein n is an integer between 1 and 48), but more preferably, is a non-human homologue of a human NOVX gene. For example, a mouse homologue of human NOVX gene of SEQ ID NO:2*n*-1, wherein n is an integer between 1 and 48, can be used to construct a homologous recombination vector suitable for altering an endogenous NOVX gene in the mouse genome. In one embodiment, the vector is designed such that, upon homologous recombination, the endogenous NOVX gene is functionally disrupted (*i.e.,* no longer encodes a functional protein; also referred to as a "knock out" vector).

Alternatively, the vector can be designed such that, upon homologous recombination, the endogenous NOVX gene is mutated or otherwise altered but still encodes functional protein (*e.g*., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g.,* Thomas, et al., 1987. Cell 51: 503 for a description of homologous recombination vectors. The vector is ten introduced into an embryonic stem cell line (*e.g.,* by electroporation) and cells in which the introduced NOVX gene has homologously-recombined with the endogenous NOVX gene are selected. *See, e.g.,* Li, et al., 1992. Cell 69: 915.

The selected cells are then injected into a blastocyst of an animal (*e.g*., a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. Curr. Opin. Biotechnol. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *see, e.g.,* Lakso, et al., 1992. Proc. Natl. Acad Sci. USA 89: 6232-6236. Another example of a recombinase system is the FLP recombinase system of *Saccharomyces cerevisiae. See,* O'Gorman, et al., 1991. Science 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g*., by mating two transgenic animals, one containing a transgene encoding a selected protein and the other containing a transgene encoding a recombinase.

Clones of the non-human transgenic animals described herein can also be produced according to the methods described in Wilmut, et al., 1997. Nature385: 810-813. In brief, a cell (*e.g.,* a somatic cell) from the transgenic animal can be isolated and induced to exit the growth cycle and enter Go phase. The quiescent cell can then be fused, *e.g.,* through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e.g.,* the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral (*e.g*., inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound (*e.g.,* a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser that contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U:S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration *(see, e.g.,* U.S. Patent No. 5,328,470) or by stereotactic injection *(see, e.g.,* Chen, et al., 1994. Proc. Natl. Acad. Sci. USA91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express NOVX protein (*e.g.,* via a recombinant expression vector in a host cell in gene therapy applications), to detect NOVX mRNA (*e.g.,* in a biological sample) or a genetic lesion in a NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein (*e.g.;* diabetes (regulates insulin release); obesity (binds and transport lipids); metabolic disturbances associated with obesity, the metabolic syndrome X as well as anorexia and wasting disorders associated with chronic diseases and various cancers, and infectious disease (possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays

The invention provides a method (also referred to herein as a "screening assay") for identifying modulators, *i.e*., candidate or test compounds or agents (*e.g*., peptides, peptidomimetics, small molecules or other drugs) that bind to NOVX proteins or have a stimulatory or inhibitory effect on, *e.g*., NOVX protein expression or NOVX protein activity. The invention also includes compounds identified in the screening assays described herein.

In one embodiment, the invention provides assays for screening candidate or test compounds that bind to or modulate the activity of the membrane-bound form of a NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. Anticancer Drug Design 12: 145.

A "small molecule" as used herein, is meant to refer to a composition that has a molecular weight of less than about 5 kD and most preferably less than about 4 kD. Small molecules can be, *e.g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et al., 1993. Proc. Natl. Acad. Sci. U.S.A. 90:6909; Erb, et al., 1994. Proc. Natl. Acad. Sci. U.S.A. 91: 11422; Zuckermann, et al., 1994. J. Med. Chem. 37: 2678; Cho, et al., 1993. Science 261: 1303; Carrell, et al., 1994. Angew. Chem. Int. Ed Engl. 33: 2059; Carell, et al., 1994. Angew. Chem. Int. Ed. Engl. 33: 2061; and Gallop, et al., 1994. J. Med. Chem. 37: 1233.

Libraries of compounds may be presented in solution (*e.g*., Houghten, 1992. Biotechniques 13: 412-421), or on beads (Lam, 1991. Nature 354: 82-84), on chips (Fodor, 1993. Nature 364: 555-556), bacteria (Ladner, U.S. Patent No. 5,223,409), spores (Ladner, U.S. Patent 5,233,409), plasmids (Cull, et al., 1992. Proc. Natl. Acad. Sci. USA 89: 1865-1869) or on phage (Scott and Smith, 1990. Science 249: 386-390; Devlin, 1990. Science 249: 404-406; Cwirla, et al., 1990. Proc. Natl. Acad. Sci. U.S.A. 87: 6378-6382; Felici, 1991. J. Mol. Biol. 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to a NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (*e.g*., stimulate or inhibit) the activity of the NOVX protein or biologically-active portion thereof Determining the ability of the test compound to modulate the activity ofNOVX or a biologically-active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the invention. In one embodiment, a NOVX target molecule is a component of a signal transduction pathway that facilitates transduction of an extracellular signal (*e.g*., a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (*i.e.,* intracellular Ca²⁺, diacylglycerol, IP₃, etc.), detecting catalytic/enzymatic activity of the target an appropriate substrate, detecting the induction of a reporter gene (comprising a NOVX-responsive regulatory element operatively linked to a nucleic acid encoding a detectable marker, *e.g.,* luciferase), or detecting a cellular response, for example, cell survival, cellular differentiation, or cell proliferation.

In yet another embodiment, an assay of the invention is a cell-free assay comprising contacting a NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e.g.,* stimulate or inhibit) the activity of the NOVX protein or biologically-active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX protein can be accomplished by determining the ability of the NOVX protein further modulate a NOVX target molecule. For example, the catalytic/enzymatic activity of the target molecule on an appropriate substrate can be determined as described, *supra.*

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, N-dodecyl-N,N-dimethyl-3-ammonio-1-propane sulfonate, 3-(3-cbolamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e.g.,* at physiological conditions for salt and pH). Following incubation, the beads or microtiter plate wells are washed to remove any unbound components, the matrix immobilized in the case of beads, complex determined either directly or indirectly, for example; as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e.g.,* biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation.
Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression ofNOVX mRNA or protein in the cell is determined. The level of expression ofNOVX mRNA or protein in the presence of the candidate compound is compared to the level of expression of NOVX mRNA or protein in the absence of the candidate compound. The candidate compound can then be identified as a modulator ofNOVX mRNA or protein expression based upon this comparison. For example, when expression of NOVX mRNA or protein is greater (*i.e.,* statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay *(see, e.g.,* U.S. Patent No. 5,283,317; Zervos, et al., 1993. Cell 72: 223-232; Madura, et al., 1993. J. Biol. Chem. 268: 12046-12054; Bartel, et al., 1993. Biotechniques 14: 920-924; lwabuchi, et al., 1993. Oncogene8: 1693-1696; and Brent WO 94/10300), to identify other proteins that bind to or interact with NOVX ("NOVX-binding proteins" or "NOVX-bp") and modulate NOVX activity. Such NOVX-binding proteins are also involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e.g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a NOVX-dependent complex, the DNA-binding and activation domains of the transcription factor are brought into close proximity. This proximity allows transcription of a reporter gene (*e.g.,* LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences of SEQ ID NO:2*n*-1, wherein n is an integer between 1 and 48, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (*e.g*., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.,* D'Eustachio, et al., 1983. Science 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, et al., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g*., in McKusick, MENDELIAN INHERJTANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g.,* Egeland, et al., 1987. Nature, 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The NOVX sequences of the invention can also be used to identity individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If coding sequences, such as those of SEQ ID NO:2*n*-1, wherein n is an integer between I and 48, are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e.g*., blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers. The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (*e.g*., drugs) for therapeutic or prophylactic treatment of an individual based on the genotype of the individual (*e.g.,* the genotype of the individual examined to determine the ability of the individual to respond to a particular agent.)

Yet another aspect of the invention pertains to monitoring the influence of agents (*e.g.,* drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (*e.g*., mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO:2*n*-1, wherein n is an integer between 1 and 48, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (*e.g.,* Fab or F(ab')₂) can be used. The term "labeled", with regard to the probe or antibody, is intended to encompass direct labeling of the probe or antibody by coupling (*i.e*., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e.g.,* mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g*., serum), cell sample, or tissue.

Furthermore, the prognostic assays described herein can be used to determine whether a subject can be administered an agent (*e.g.,* an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e.g.,* wherein the presence of NOVX protein or nucleic acid is diagnostic for a subject that can be administered the agent to treat a disorder associated with aberrant NOVX expression or activity).

The methods of the invention can also be used to detect genetic lesions in a NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a NOVX-protein, or the misexpression of the NOVX gene. For example, such genetic lesions can be detected by ascertaining the existence of at least one of: (i) a deletion of one or more nucleotides from a NOVX gene; (*ii*) an addition of one or more nucleotides to a NOVX gene; (iii) a substitution of one or more nucleotides of a NOVX gene, (*iv*) a chromosomal rearrangement of a NOVX gene; (v) an alteration in the level of a messenger RNA transcript of a NOVX gene, (*vi*) aberrant modification of a NOVX gene, such as of the methylation pattern of the genomic DNA, *(vii)* the presence of a non-wild-type splicing pattern of a messenger RNA transcript of a NOVX gene, (*viii*) a non-wild-type level of a NOVX protein, (*ix*) allelic loss of a NOVX gene, and (*x*) inappropriate post-translational modification of a NOVX protein. As described herein, there are a large number of assay techniques known in the art which can be used for detecting lesions in a NOVX gene..A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) *(see, e.g.,* Landegran, et al., 1988. Science 241: 1077-1080; and Nakazawa, et al., 1994. Proc. Natl. Acad. Sci. USA 91: 360-364), the latter of which can be particularly useful for detecting point mutations in the NOVX-gene (*see,* Abravaya, et al., 1995. Nucl. Acids Res.23: 675-682). This method can include the steps of collecting a sample of cells from a patient, isolating nucleic acid (*e.g.,* genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication *(see,* Guatelli, et al., 1990. Proc. Natl. Acad. Sci. USA87: 1874-1878), transcriptional amplification system (*see,* Kwoh, et al., 1989. Proc. Natl. Acad Sci. USA 86: 1173-1177); Qβ Replicase *(see,* Lizardi, et al, 1988. BioTechnology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e.g*., DNA or RNA, to high-density arrays containing hundreds or thousands of oligonucleotides probes. *See, e.g.,* Cronin, et al., 1996. Human Mutation 7: 244-255; Kozal, et al., 1996. Nat. Med. 2: 753-759. For example, genetic mutations in NOVX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. Proc. Natl. Acad. Sci. USA 74: 560 or Sanger, 1977. Proc. Natl. Acad. Sci. USA 74: 5463. It is also contemplated that any of a variety of automated sequencing procedures can be utilized when performing the diagnostic assays *(see, e.g.,* Naeve, et al., 1995. Biotechniques 19: 448), including sequencing by mass spectrometry (see, *e.g.,* PCT International Publication No. WO 94/16101; Cohen, et al., 1996. Adv. Chromatography 36: 127-162; and Griffin, et al., 1993. Appl. Biochem. Biotechnol. 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, et al., 1985. Science 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, et al., 1988. Proc. Natl. Acad. Sci. USA 85: 4397; Saleeba, et al., 1992. Methods Enzymol. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E. coli* cleaves A at G/A mismatches and the thymidine DNA glycosylase from HeLa cells cleaves T at G/T mismatches. *See, e.g.,* Hsu, et al., 1994. Carcinogenesis 15: 1657-1662. According to an exemplary embodiment, a probe based on a NOVX sequence, *e.g.,* a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e.g.,* Orita, et al., 1989. Proc. Natl. Acad. Sci. USA: 86: 2766; Cotton, 1993. Mutat. Res. 285: 125-144; Hayashi, 1992. Genet. Anal. Tech. Appl. 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, et al., 1991. Trends Genet.7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, et al., 1985. Nature313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. Biophys. Chem. 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, et al., 1986. Nature324: 163; Saiki, et al., 1989. Proc. Natl. Acad. Sci. USA86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center ofthe molecule (so that amplification depends on differential hybridization; *see, e.g.,* Gibbs, et al., 1989. Nucl. Acids Res. 17: 2437-2448) or at the extreme 3'-terminus of one primer where, under appropriate conditions, mismatch can prevent, or reduce polymerase extension *(see, e.g.,* Prossner, 1993. Tibtech. 11: 238). In addition it may be desirable to introduce a novel restriction site in the region of the mutation to create cleavage-based detection. *See, e.g.,* Gasparini, et al., 1992. Mol. Cell Probes 6: 1. It is anticipated that in certain embodiments amplification may also be performed using *Taq* ligase for amplification. *See, e.g.,* Barany, 1991. Proc. Natl. Acad. Sci. USA 88: 189. In such cases, ligation will occur only if there is a perfect match at the 3'-terminus ofthe 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which maybe conveniently used, *e.g.,* in clinical settings to diagnose patients exhibiting symptoms or family history of a disease or illness involving a NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e.g.,* NOVX gene expression), as identified by a screening assay described herein can be administered to individuals to treat (prophylactically or therapeutically) disorders. The disorders include but are not limited to, *e.g*., those diseases, disorders and conditions listed above, and more particularly include those diseases, disorders, or conditions associated with homologs of a NOVX protein, such as those summarized in Table A.

In conjunction with such treatment, the pharmacogenomics (*i.e.,* the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g.,* drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g.,* Eichelbaum, 1996. Clin. Exp. Pharmacol. Physiol., 23: 983-985; Linder, 1997. Clin. Chem., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.,* N-acetyltransferase 2 (NAT 2) and cytochrome pregnancy zone protein precursor enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

### Monitoring of Effects During Clinical Trials

Monitoring the influence of agents (*e.g.,* drugs, compounds) on the expression or activity of NOVX (*e.g.,* the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e.g*., compound, drug or small molecule) that modulates NOVX activity (*e.g.,* identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i.e.,* a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g.,* an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of a NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, *i.e.,* to increase the effectiveness of the agent. Alternatively, decreased administration of the agent may be desirable to decrease expression or activity of NOVX to lower levels than detected, *i.e.,* to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. The disorders include but are not limited to, *e.g.,* those diseases, disorders and conditions listed above, and more particularly include those diseases, disorders, or conditions associated with homologs of a NOVX protein, such as those summarized in Table A.

These methods of treatment will be discussed more fully, below.

### Diseases and Disorders

Diseases and disorders that are characterized by increased (relative to a subject not suffering from the disease or disorder) levels or biological activity may be treated with Therapeutics that antagonize (*i.e.,* reduce or inhibit) activity. Therapeutics that antagonize activity may be administered in a therapeutic or prophylactic manner. Therapeutics that may be utilized include, but are not limited to: (i) an aforementioned peptide, or analogs, derivatives, fragments or homologs thereof; (*ii*) antibodies to an aforementioned peptide; (iii) nucleic acids encoding an aforementioned peptide; (iv) administration of antisense nucleic acid and nucleic acids that are "dysfunctional" (*i.e.,* due to a heterologous insertion within the coding sequences of coding sequences to an aforementioned peptide) that are utilized to "knockout" endogenous function of an aforementioned peptide by homologous recombination *(see, e.g.,* Capecchi, 1989. Science 244: 1288-1292); or *(*v*)* modulators (*i.e.,* inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i.e.,* are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g*., by Western blot analysis, immunoprecipitation followed by sodium dodecyl sulfate (SDS) polyacrylamide gel electrophoresis, immunocytochemistry, *etc.*) and/or hybridization assays to detect expression of mRNAs (*e.g.,* Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, a NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates-one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro* (*e.g.,* by culturing the cell with the agent) or, alternatively, *in vivo* (*e.g.,* by administering the agent to a subject). As such, the invention provides methods of treating an individual afflicted with a disease or disorder characterized by aberrant expression or activity of a NOVX protein or nucleic acid molecule. In one embodiment, the method involves administering an agent (*e.g*., an agent identified by a screening assay described herein), or combination of agents that modulates (*e.g*., up-regulates or down-regulates) NOVX expression or activity. In another embodiment, the method involves administering a NOVX protein or nucleic acid molecule as therapy to compensate for reduced or aberrant NOVX expression or activity.

Stimulation of NOVX activity is desirable *in situations* in which NOVX is abnormally downregulated and/or in which increased NOVX activity is likely to have a beneficial effect. One example of such a situation is where a subject has a disorder characterized by aberrant cell proliferation and/or differentiation (*e.g.,* cancer or immune associated disorders). Another example of such a situation is where the subject has a gestational disease (*e.g.,* preclampsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The NOVX nucleic acids and proteins of the invention are useful in potential prophylactic and therapeutic applications implicated in a variety of disorders. The disorders include but are not limited to, *e.g*., those diseases, disorders and conditions listed above, and more particularly include those diseases, disorders, or conditions associated with homologs of a NOVX protein, such as those summarized in Table A.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from diseases, disorders, conditions and the like, including but not limited to those listed herein.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule (*i.e.,* some peptides have been found to possess anti-bacterial properties). These materials are further useful in the generation of antibodies, which immunospecifically-bind to the novel substances of the invention for use in therapeutic or diagnostic methods.

The invention will be further described in the following examples, which do not limit the scope of the invention described in the claims.

### EXAMPLES

### Example A: Polynucleotide and Polypeptide Sequences, and Homology Data Example 1.

The NOV1 clone was analyzed, and the nucleotide and encoded polypeptide sequences are shown in Table 1A.

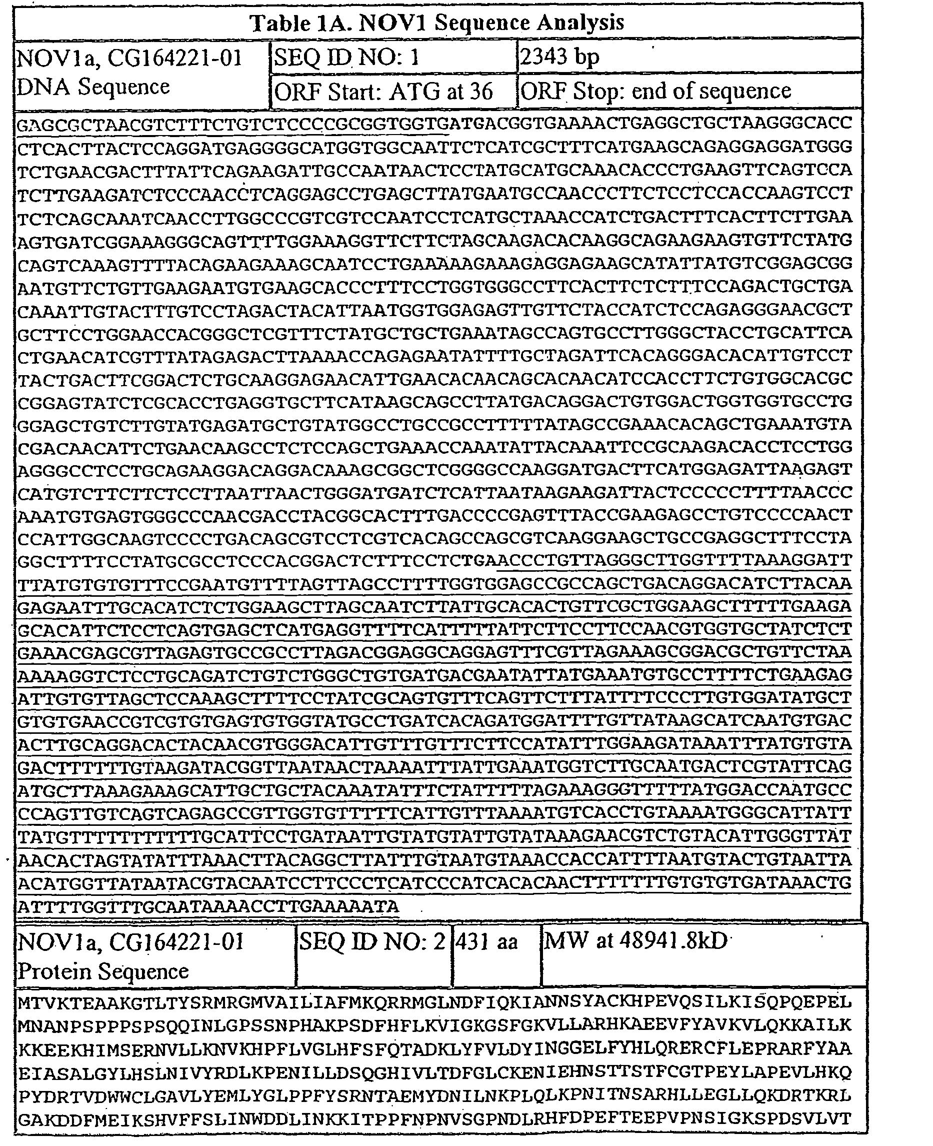

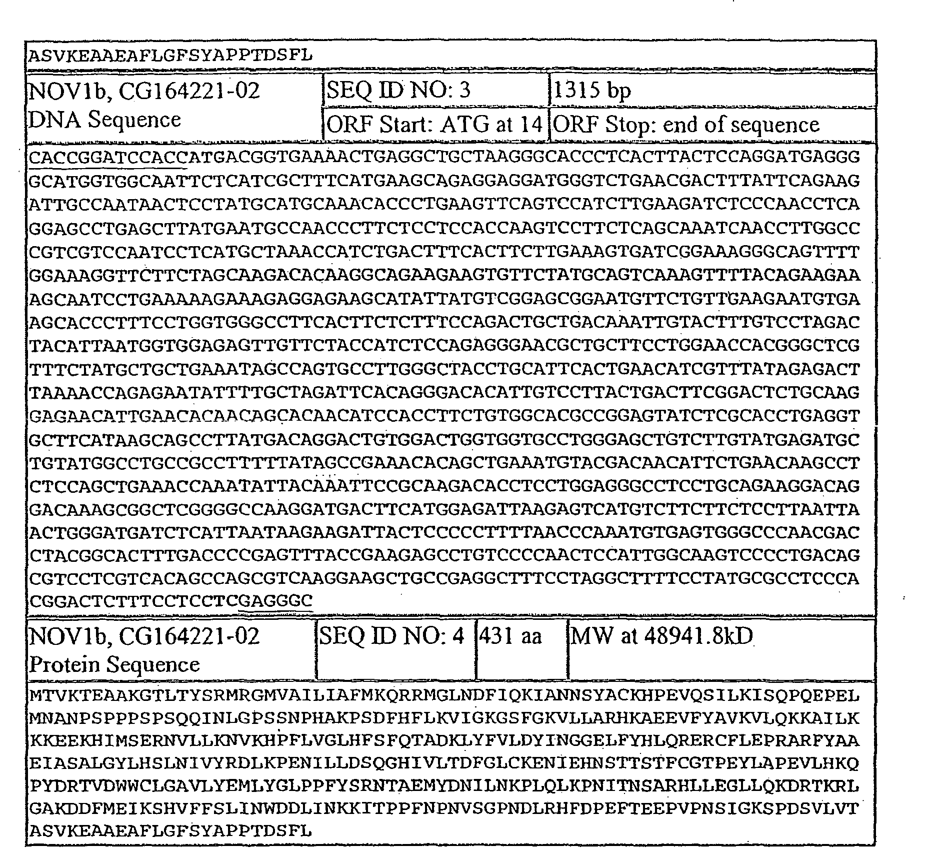

A ClustalW comparison of the above protein sequences yields the following sequence alignment shown in Table 1B.

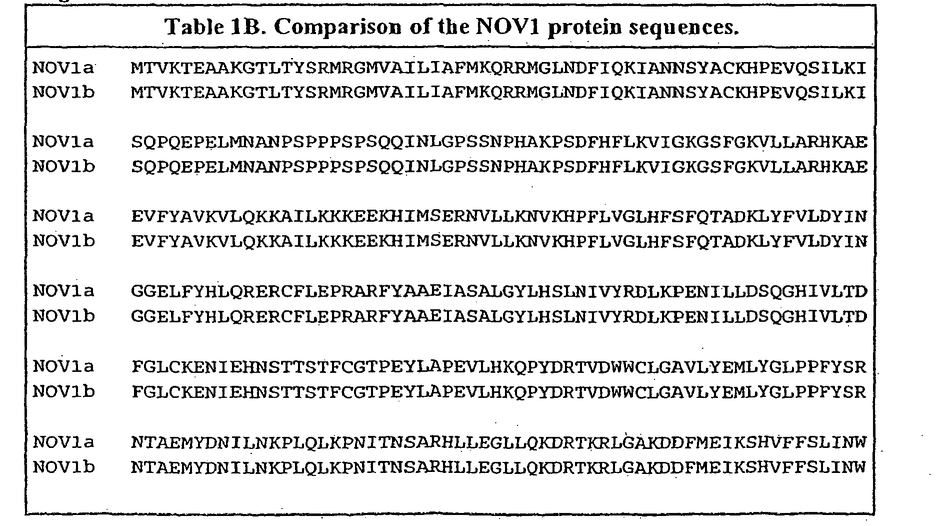

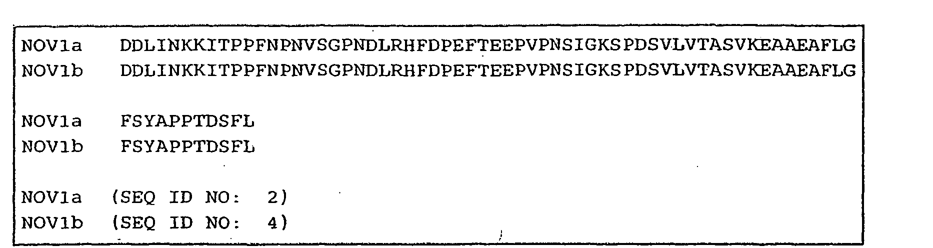

Further analysis of the NOV1a protein yielded the following properties shown in Table 1C.

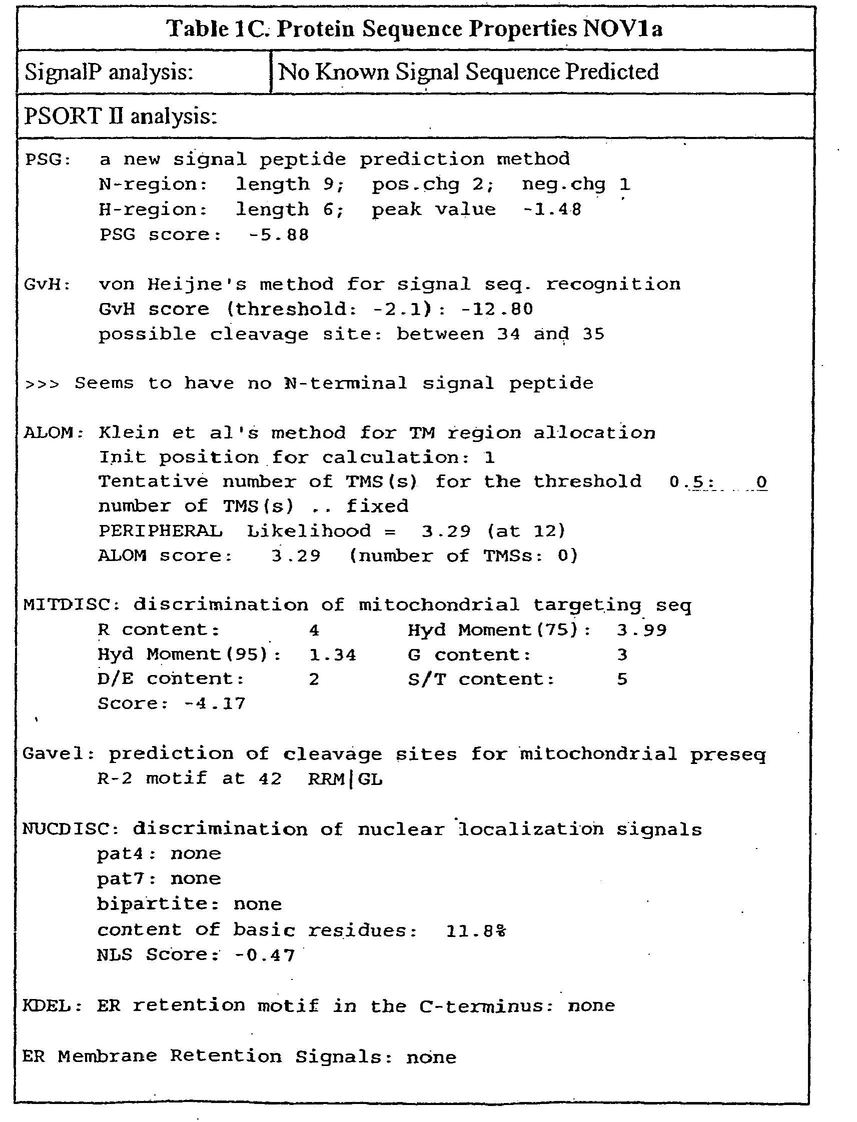

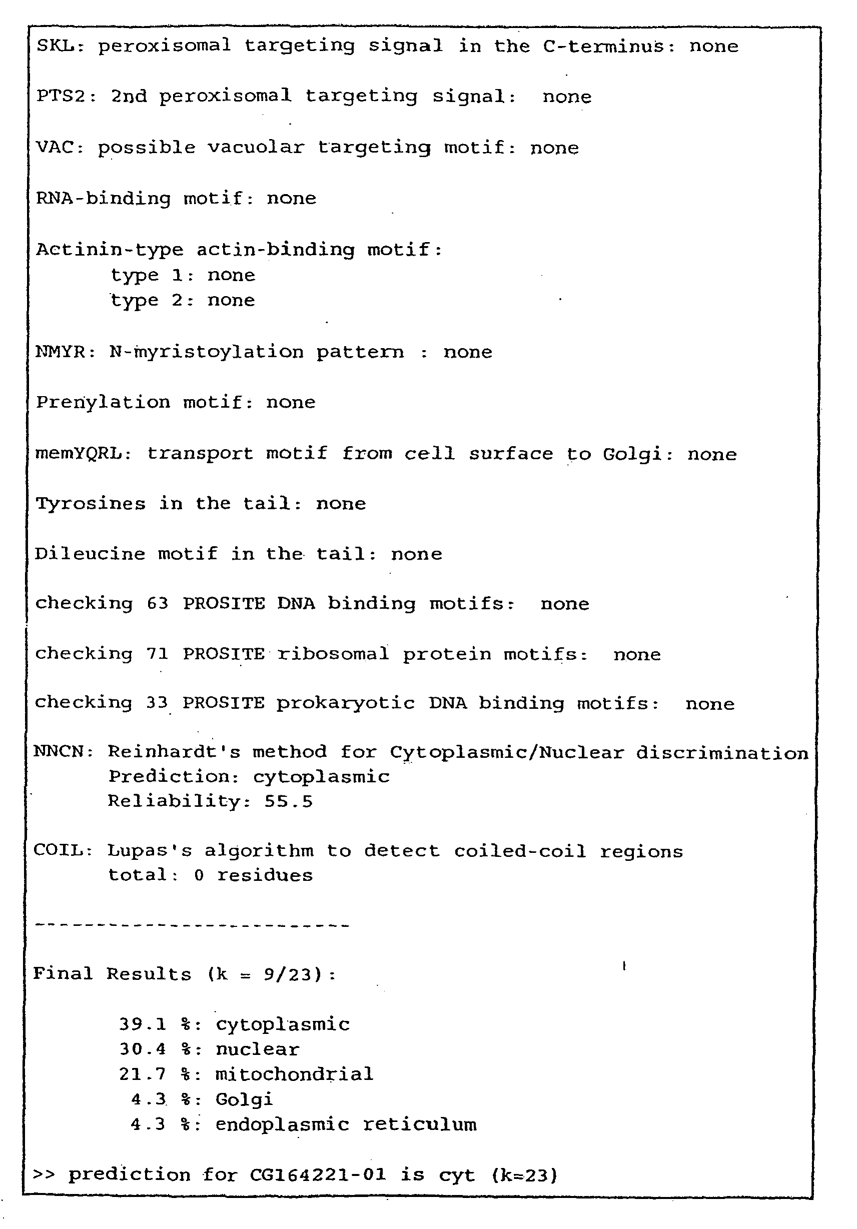

A search of the NOV1a protein against the Geneseq database, a proprietary database that contains sequences published in patents and patent publication, yielded several homologous proteins shown in Table 1D.

| **Table 1D. Geneseq Results for NOV1a** | | | | |
|---|---|---|---|---|
| **Geneseq Identifier** | **Protein/Organism/Length [Patent #, Date]** | **NOV1a Residues/ Match Residues** | **Identities/ Similarities for the Matched Region** | **Expect Value** |
| AAW90139 | Human sgk protein - Homo sapiens, 431 aa. [EP887081-A2, 30-DEC-1998] | 1..431 | 431/431 (100%) | 0.0 |
| | | 1..431 | 431/431 (100%) | |
| AAB65613 | Novel protein kinase, SEQ ID NO: 139 - Homo sapiens, 431 aa. [WO200073469-A2, 07-DEC-2000] | 1..431 | 430/431 (99%) | 0.0 |
| | | 1..431 | 431/431 (99%) | |
| AAB24115 | Human serum and glucocorticoid regulated kinase protein (HSGK) - Homo sapiens, 431 aa. [CN1259573-A, 12-JUL-2000] | 1..431 | 430/431 (99%) | 0.0 |
| | | 1..431 | 431/431 (99%) | |
| AAW77217 | Human cell-volume regulating kinase h-sgk - Homo sapiens, 431 aa. [EP861896-A2, 02-SEP-1998] | 1..431 | 430/431 (99%) | 0.0 |
| | | 1..431 | 431/431 (99%) | |
| AAY95279 | Human serum and glucocorticoid-induced protein kinase - Homo sapiens, 431 aa. [WO200035946-A1, 22-JUN-2000] | 1.431 | 429/431 (99%) | 0.0 |
| | | 1.431 | 430/431 (99%) | |

In a BLAST search of public sequence databases, the NOV a protein was found to have homology to the proteins shown in the BLASTP data in Table 1E.

| **Table 1E. Public BLASTP Results for NOV1a** | | | | |
|---|---|---|---|---|
| **Protein Accession Number** | **Protein/Organism/Length** | **NOV1a Residues/ Match Residues** | **Identities/ Similarities for the Matched Portion** | **Expect Value** |
| CAD58123 | Sequence 1 from Patent WO02074987 - Homo sapiens (Human), 431 aa. | 1..431 | 431/431 (100%) | 0.0 |
| | | 1..431 | 431/431 (100%) | |
| O00141 | Serine/threorune-protein kinase Sgk1 (EC 2.7.1.37) (Serum/glucocorticoid-regulated kinase 1) - Homo sapiens (Human), 431 aa. | 1..431 | 430/431 (99%) | 0.0 |
| | | 1..431 | 431/431 (99%) | |
| A48094 | serum and glucocorticoid-regulated kinase - rat, 431 aa. | 1..431 | 419/431(97%) | 0.0 |
| | | 1..431 | 426/431 (98%) | |
| Q9XT18 | Serine/threonine-protein kinase Sgk1 (EC 2.7.1.37) (Serum/glucocorticoid-regulated kinase 1) - Oryctolagus cuniculus (Rabbit), 431 aa. | 1..431 | 418/431 (96%) | 0.0 |
| | | 1.431 | 426/431 (97%) | |
| Q9WVC6 | Serine/threonine-protein kinase Sgk1 (EC 2.7.1.37). (Serum/glucocorticoid-regulated kinase 1) - Mus musculus (Mouse), 431 aa. | 1.431 | 416/431(96%) | 0.0 |
| | | 1..431 | 425/431 (98%) | |

PFam analysis predicts that the NOV1a protein contains the domains shown in the Table 1F.

| **Table 1F. Domain Analysis of NOV1a** | | | |
|---|---|---|---|
| **Pfam Domain** | **NOV1a Match Region** | **Identities/ Similarities for the Matched Region** | **Expect Value** |
| pkinase | 98..355 | 107/301 (36%) | 3.2e-88 |
| | | 212/301 (70%) | |
| pkinase_C | 356..430 | 28/77 (36%) | 2.2e-13 |
| | | 54/77 (70%) | |

### Example 2.

The NOV2 clone was analyzed, and the nucleotide and encoded polypeptide sequences are shown in Table 2A.

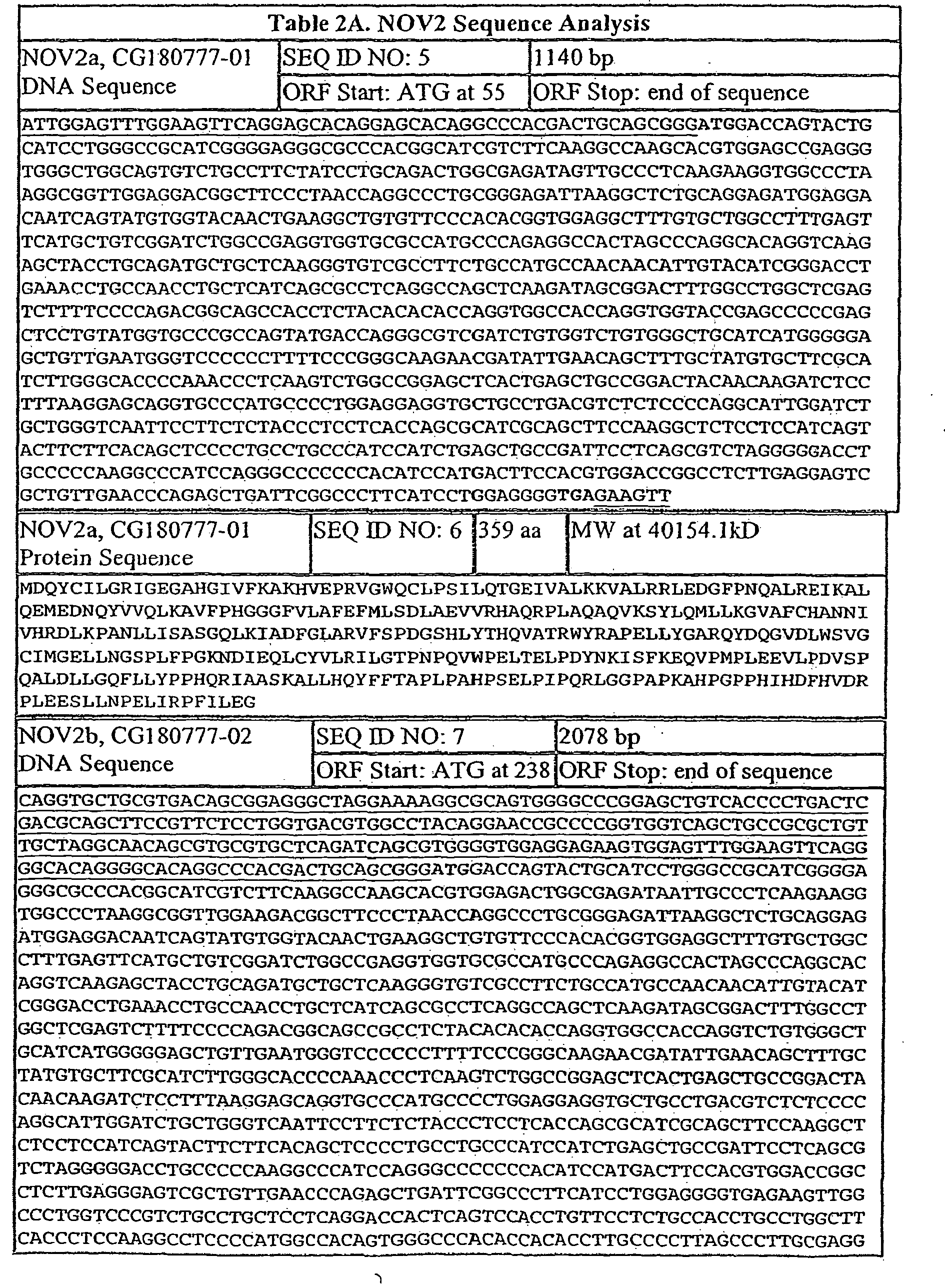

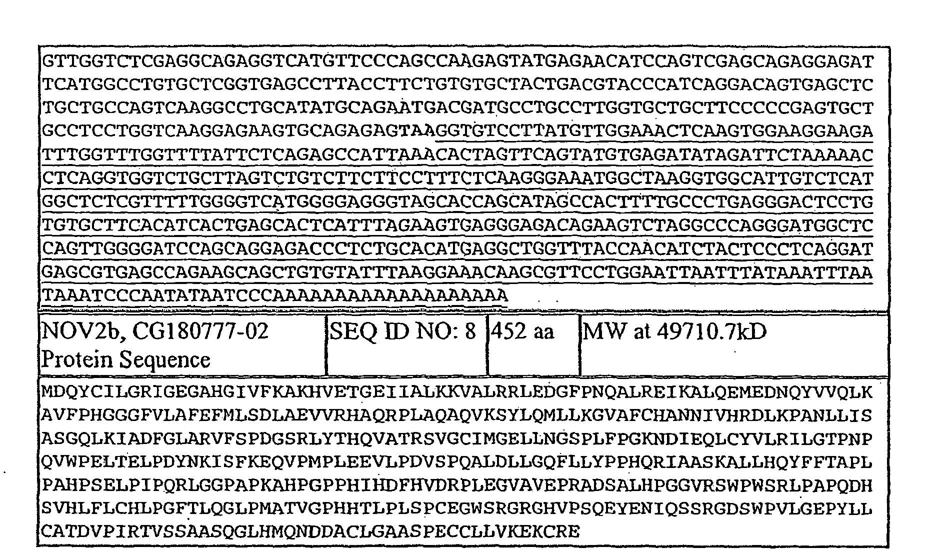

A ClustalW comparison of the above protein sequences yields the following sequence alignment shown in Table 2B.

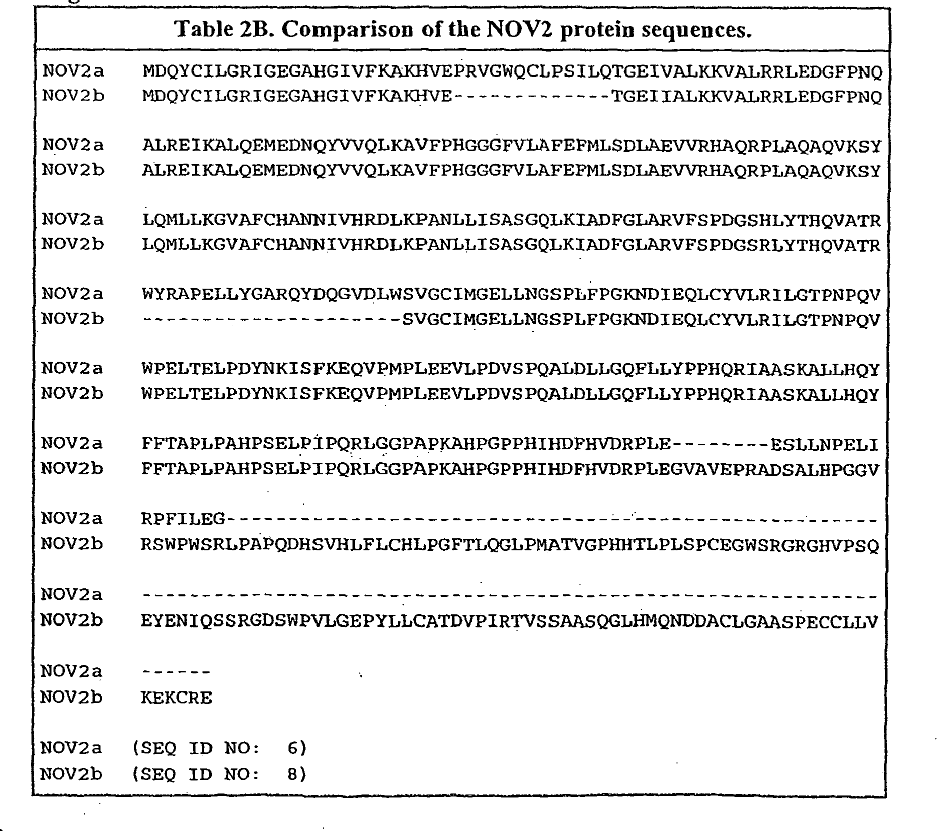

Further analysis of the NOV2a protein yielded the following properties shown in Table 2C.

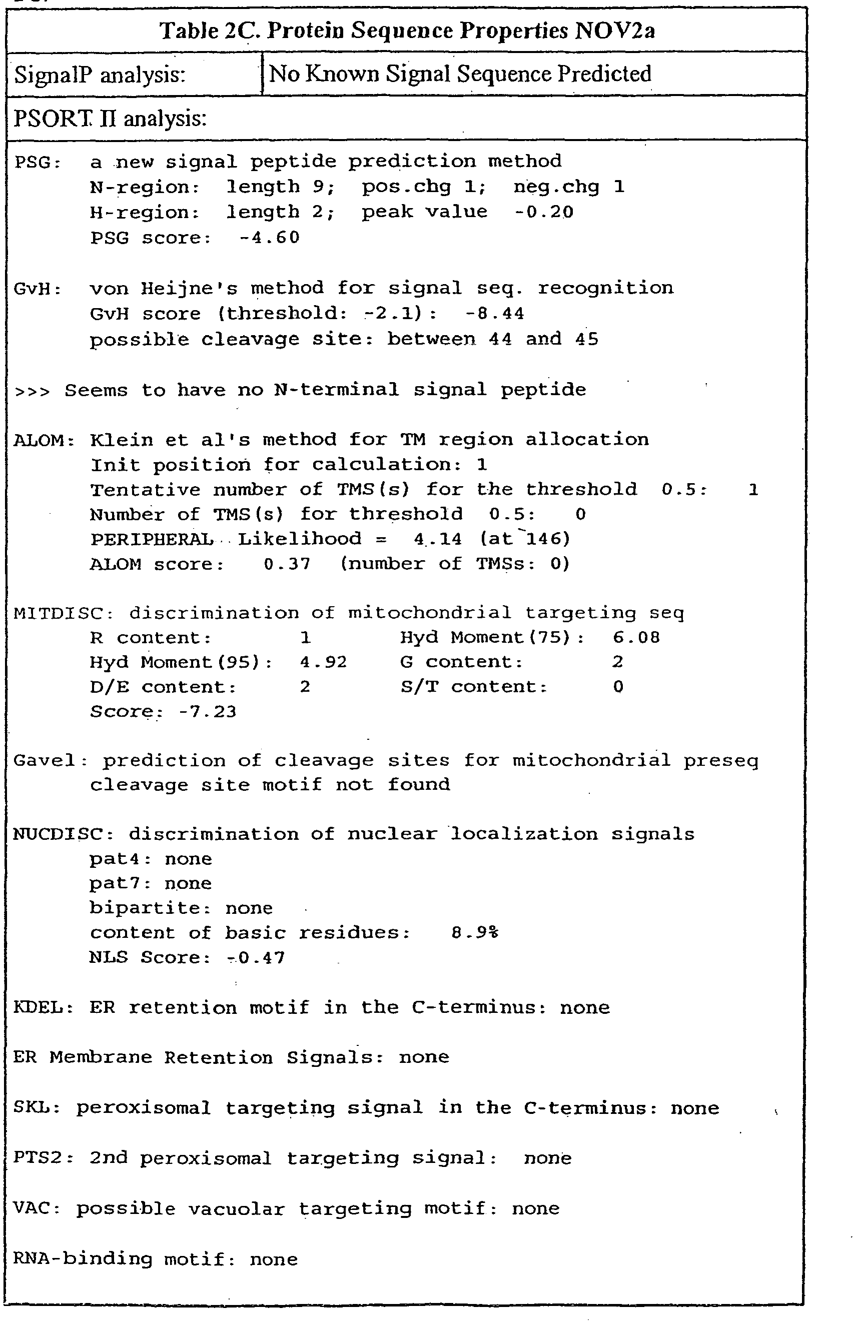

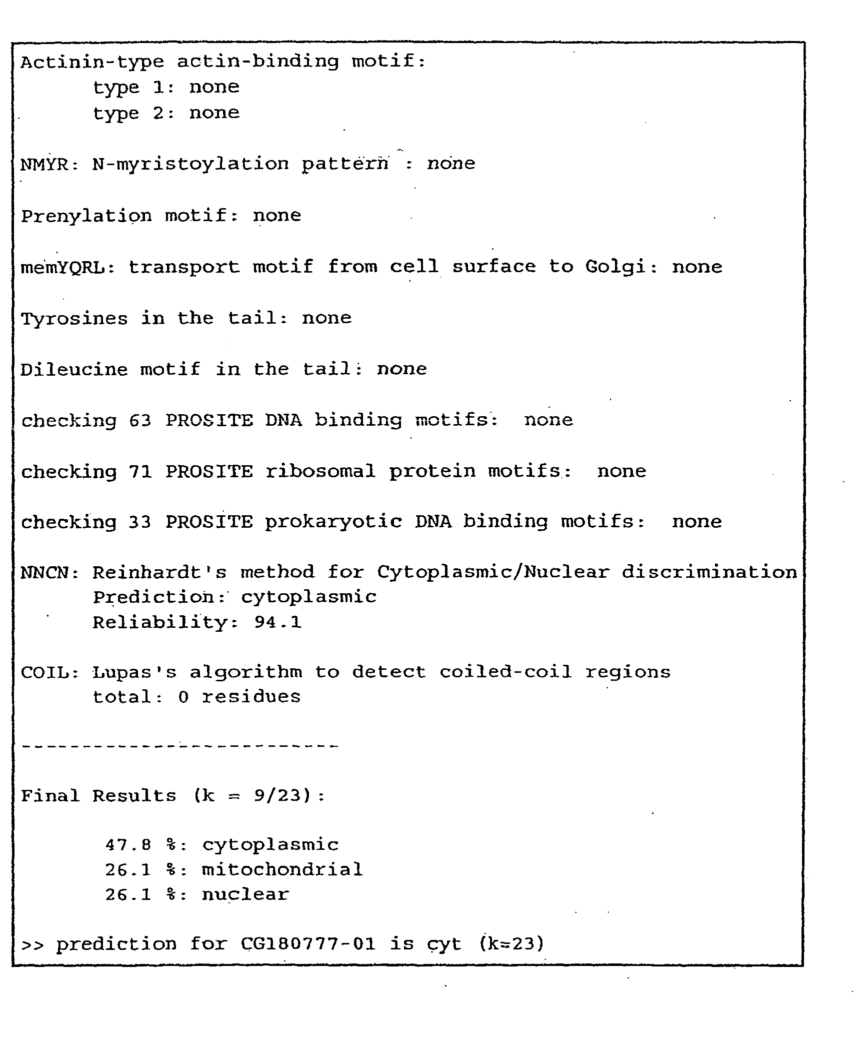

A search of the NOV2a protein against the Geneseq database, a proprietary database that contains sequences published in patents and patent publication, yielded several homologous proteins shown in Table 2D.

| **Table 2D. Geneseq Results for NOV2a** | | | | |
|---|---|---|---|---|
| **Geneseq Identifier** | **Protein/Organism/Length [Patent #, Date]** | **NOV2a Residues/ Match Residues** | **Identities/ Similarities for the Matched Region** | **Expect Value** |
| AAY90244 | Human cyclin dependent kinase, hPNQALRE, protein sequence #3 - Homo sapiens, 359 aa. [WO200036118-A2, 22-JUN-2000] | 1..359 | 357/359 (99%) | 0.0 |
| | | 1..359 | 358/359 (99%) | |
| AAY90245 | Human cyclin dependent kinase, hPNQALRE, protein sequence #4 - Homo sapiens, 345 aa. [WO200036118-A2,22-JUN-2000] | 1..359 | 344/359 (95%) | 0.0 |
| | | 1..345 | 344/359 (95%) | |
| AAE11784 | Human kinase (PKIN)-18 protein - Homo sapiens, 346 aa. [WO200181555-A2, 01-NOV-2001] | 1..359 | 345/359 (96%) | 0.0 |
| | | 1..346 | 345/359 (96%) | |
| AAY90243 | Human cyclin dependent kinase, hPNQALRE, protein sequence #2 - Homo sapiens, 346 aa. [WO200036118-A2, 22-JUN-2000] | 1..358 | 344/358 (96%) | 0.0 |
| | | 1..345 | 344/358 (96%) | |
| AAM93918 | Human polypeptide, SEQ ID NO: 4077 - Homo sapiens, 338 aa. [EP1130094-A2, 05-SEP-2001] | 1..359 | 337/359 (93%) | 0.0 |
| | | 1..338 | 337/359 (93%) | |

In a BLAST search of public sequence databases, the NOV2a protein was found to have homology to the proteins shown in the BLASTP data in Table 2E.

| **Table 2E. Public BLASTP Results for NOV2a** | | | | |
|---|---|---|---|---|
| **Protein Accession Number Match** | **Protein/Orgaiaisni/Length** | **NOV2a Residues/ Residues** | **Identities/ Similarities for the Matched Portion** | **Expect Value** |
| Q8IZL9 | Cell cycle related kinase - Homo sapiens (Human), 346 aa. | 1..359 | 345/359(96%) | 0.0 |
| | | 1..346 | 345/359(96%) | |
| Q9JHU3 | CDK-related protein kinase PNQLARE - Mus musculus (Mouse), 346 aa. | 1..359 | 325/359(90%) | 0.0 |
| | | 1..346 | 335/359 (92%) | |
| 095137 | Cell cycle related kinase (Cyclin-dependent protein kinase H) - Homo sapiens (Human), 452 aa. | 1..349 | 308/349 (88%) | e-174 |
| | | 1..315 | 310/349 (88%) | |
| Q9BUF4 | Similar to cell cycle related kinase - Homo sapiens (Human), 275 aa. | 1..294 | 259/294 (88%) | e-141 |
| | | 1..260 | 259/294 (88%) | |
| P29620 | CDC2+/CDC28-related protein kinase R2 (EC 2.7.1.-) - Oryza sativa (Rice), 424 aa. | 2..338 | 150/338 (44%) | 2e-73 |
| | | 17..330 | 200/338 (58%) | |

PFam analysis predicts that the NOV2a protein contains the domains shown in the Table 2F.

| **Table 2F. Domain Analysis of NOV2a** | | | |
|---|---|---|---|
| **Pfam Domain** | **NOV2a Match Region** | **Identities/ Similarities for the Matched Region** | **Expect Value** |
| pkinase | 4..301 | 104/317 (33%) | 3.7e-73 |
| | | 220/317 (69%) | |

### Example 3.

The NOV3 clone was analyzed, and the nucleotide and encoded polypeptide sequences are shown in Table 3A.

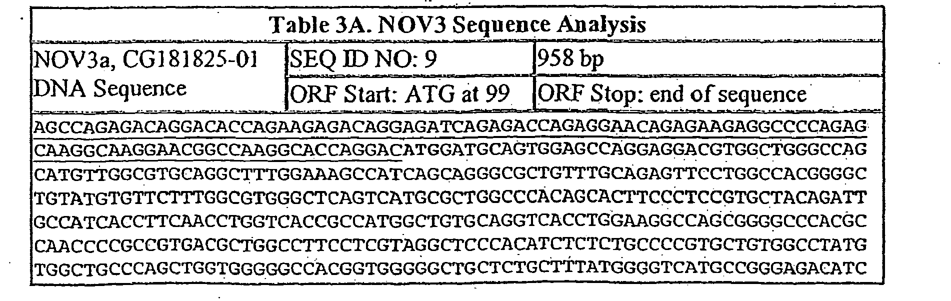

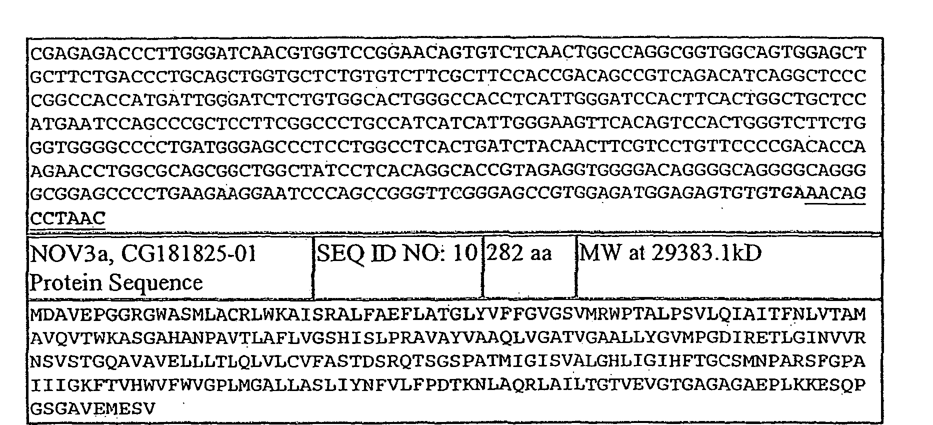

Further analysis of the NOV3a protein yielded the following properties shown in Table 3B.

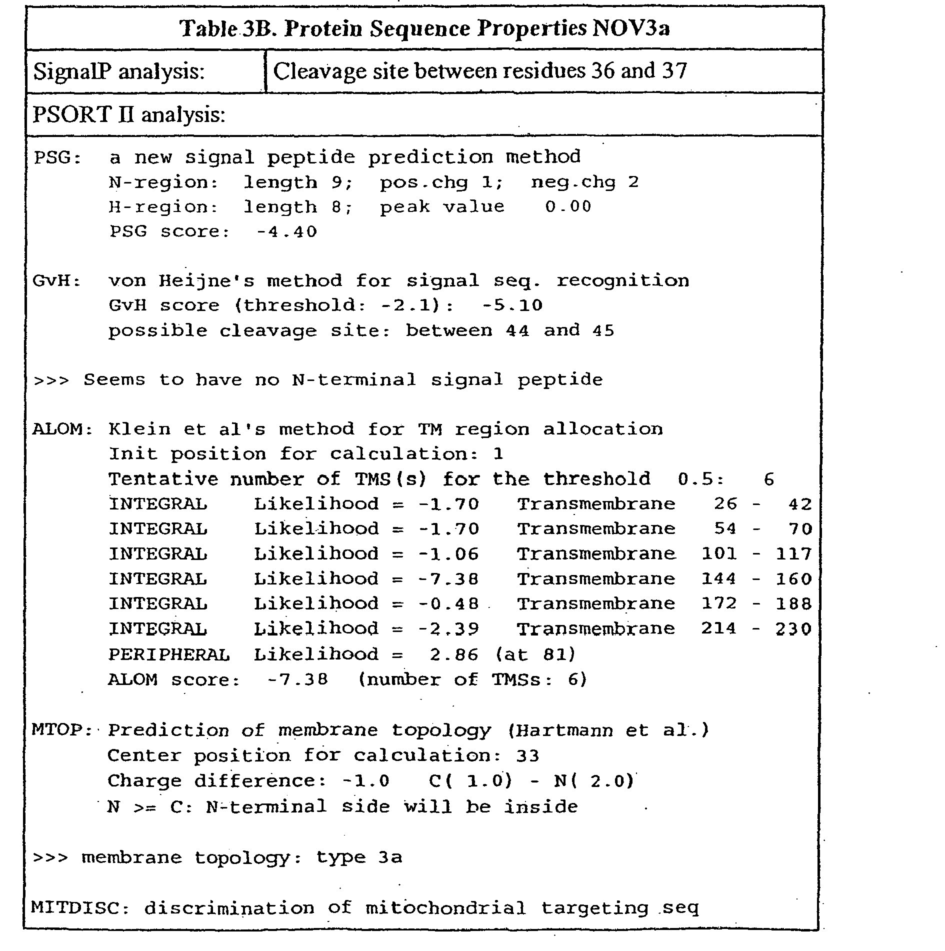

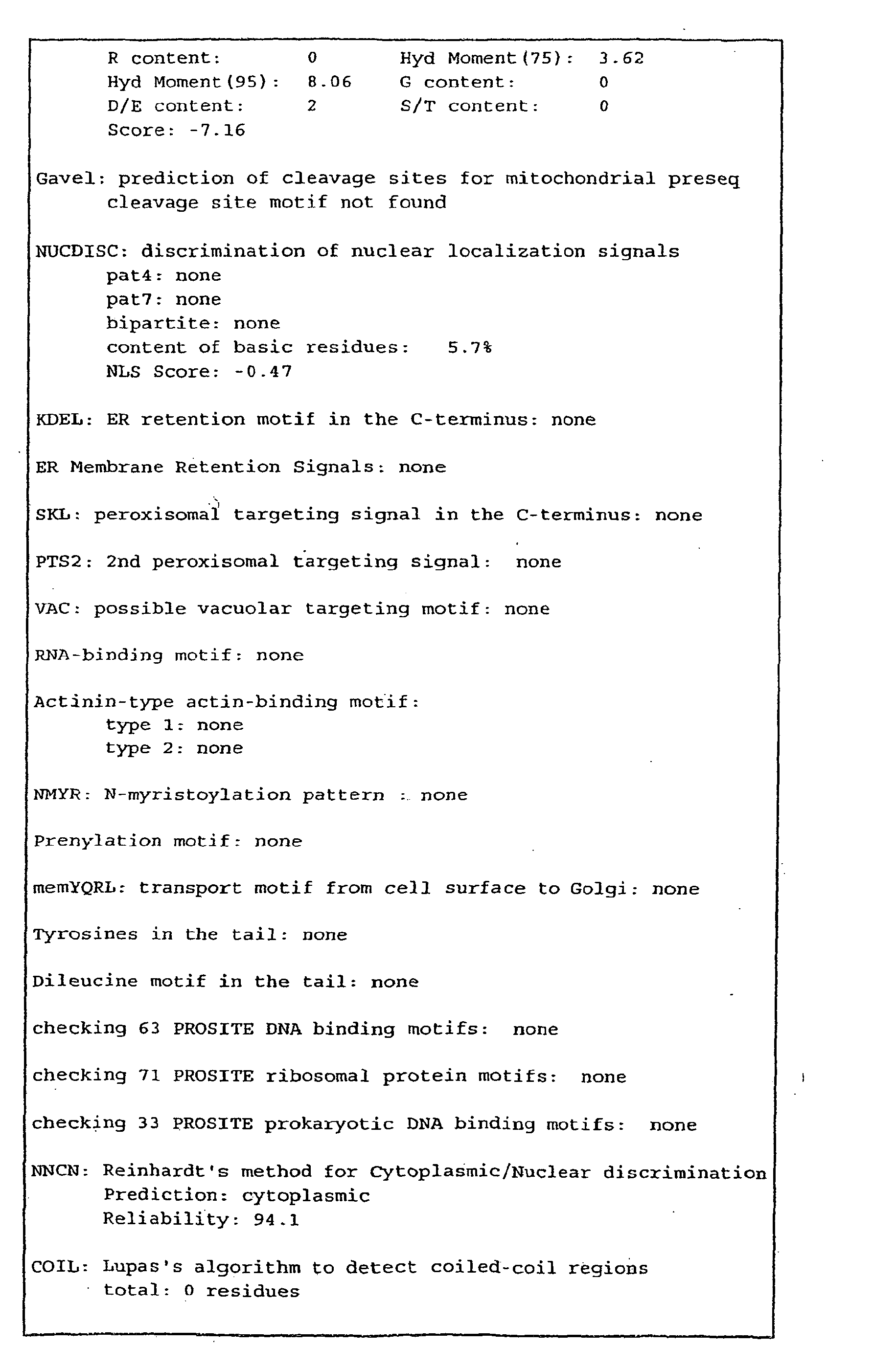

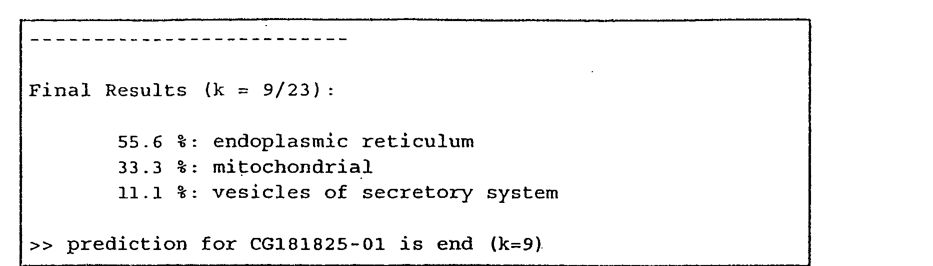

A search of the NOV3a protein against the Geneseq database, a proprietary database that contains sequences published in patents and patent publication, yielded several homologous proteins shown in Table 3C.

| **Table 3C. Geneseq Results for NOV3a** | | | | |
|---|---|---|---|---|
| **Geneseq Identifier** | **Protein/Organism/Length [Patent #, Date]** | **NOV3a Residues/ Match Residues** | **Identities/ Similarities for the Matched Region** | **Expect Value** |
| ABB97500 | Novel human protein SEQ ID NO: 768 - Homo sapiens, 265 aa. [WO200222660-A2, 21-MAR-2002] | 22..254 | 138/236(58%) | 1e-77 |
| | | 9..244 | 184/236 (77%) | |
| ABG96280 | Human ovarian cancer marker OV3 - Homo sapiens, 265 aa. [WO200271928-A2,19-SEP-2002] | 22..254 | 138/236 (58%) | 1e-77 |
| | | 9..244 | 184/236 (77%) | |
| AAR51070 | A water channel protein localized in the rat kidney collecting tubule - Rattus sp. (Sprague-Dawley), 271 aa. [EP591789-A, 13-APR-1994] | 22..251 | 138/232(59%) | 2e-76 |
| | | 8..239 | 175/232 (74%) | |
| AAW94319 | Rat aquaporin-5 - Rattus sp, 265 aa. [US5858702-A, 12-JAN-1999] | 22..254 | 133/236(56%) | 7e-75 |
| | | 9..244 | 181/236 (76%) | |
| AAW55787 | Rat aquaporin-5 - Rattus sp, 265 aa. [US5741671-A, 21-APR-1998] | 22..254 | 133/236 (56%) | 7e-75 |
| | | 9..244 | 181/236 (76%) | |

In a BLAST search of public sequence databases, the NOV3a protein was found to have homology to the proteins shown in the BLASTP data in Table 3D.

| **Table 3D. Public BLASTP Results for NOV3a** | | | | |
|---|---|---|---|---|
| **Protein Accession Number** | **Protein/Organism/Length** | **NOV3a Residues/ Match Residues** | **Identities/ Similarities for the Matched Portion** | **Expect Value** |
| Q13520 | Aquaporin 6 (Aquaporin-2 like) (hKID) - Homo sapiens (Human), 282 aa. | 1..282 | 275/282 (97%) | e-152 |
| | | 1..282 | 275/282 (97%) | |
| Q9WTY0 | Aquaporin-6 - Rattus norvegicus (Rat), 276 aa. | 4..279 | 213/276 (77%) | e-117 |
| | | 1..274 | 234/276 (84%) | |
| Q8C4A0 | Aquaporin 6 - Mus musculus (Mouse), 293 aa. | 4..278 | 211/275 (76%) | e-115 |
| | | 1..273 | 230/275 (82%) | |
| P41181 | Aquaporin-CD (AQP-CD) (Water channel protein for renal collecting duct) (ADH water channel) (Aquaporin 2) (Collecting duct water channel protein) (WCH-CD) - Homo sapiens (Human), 271 aa. | 22..251 | 148/232 (63%) | 6e-81 |
| | | 8..239 | 178/232 (75%) | |
| I4818 | water-channel aquaporin 2-human, 271 aa. | 22..251 | 147/232 (63%) | 5e-80 |
| | | 8..239 | 177/232 (75%) | |

PFam analysis predicts that the NOV3a protein contains the domains shown in the Table 3E.

| **Table 3E. Domain Analysis of NOV3a** | | | |
|---|---|---|---|
| **Pfam Domain** | **NOV3a Match Region** | **Identities/Similarities for the Matched Region** | **Expect Value** |
| MIP | 16..231 | 109/268 (41%) 185/268 (69%) | 4.3e-87 |

### Example 4.

The NOV4 clone was analyzed, and the nucleotide and encoded polypeptide sequences are shown in Table 4A.

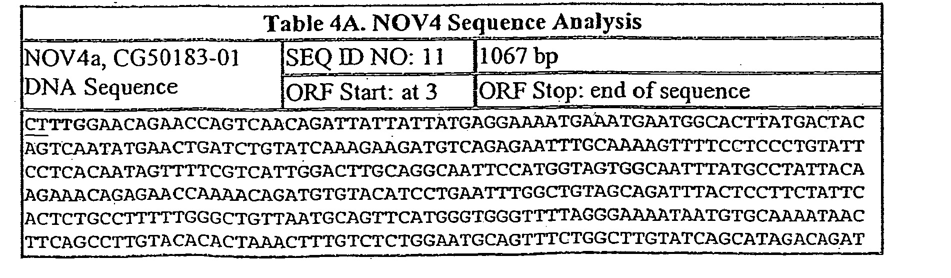

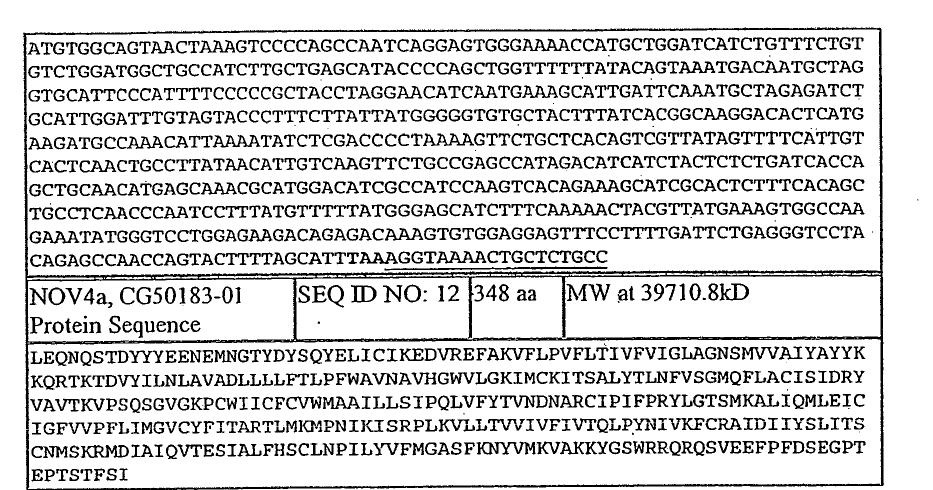

Further analysis of the NOV4a protein yielded the following properties shown in Table 4B.

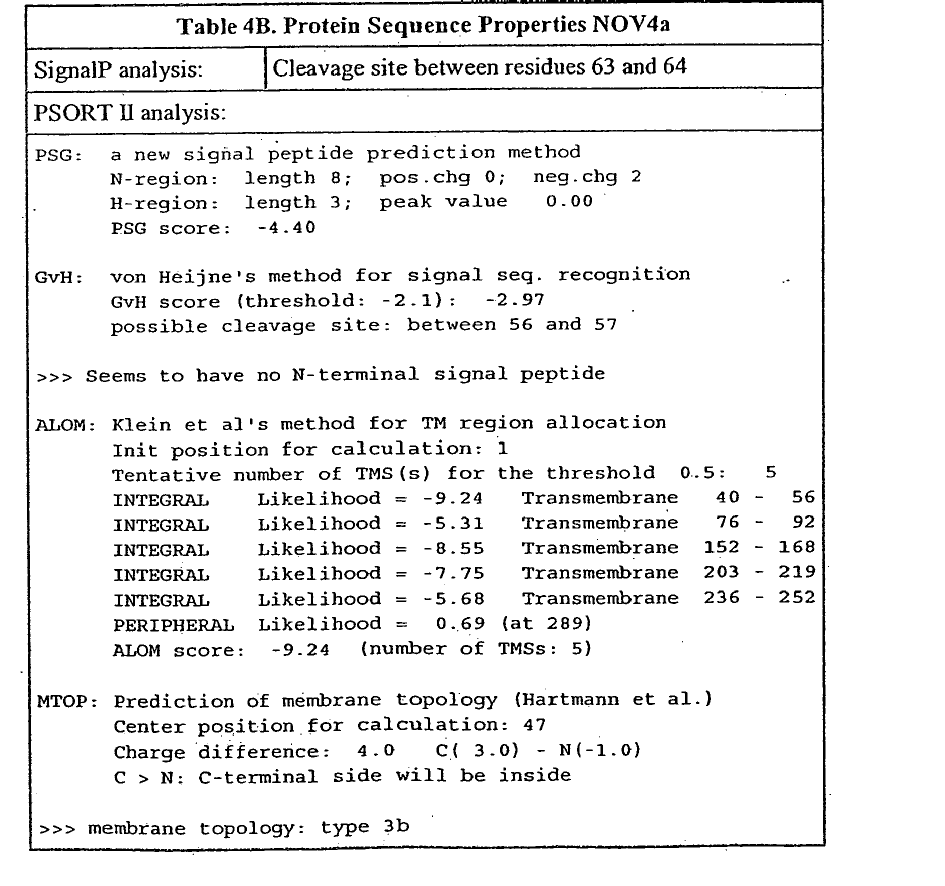

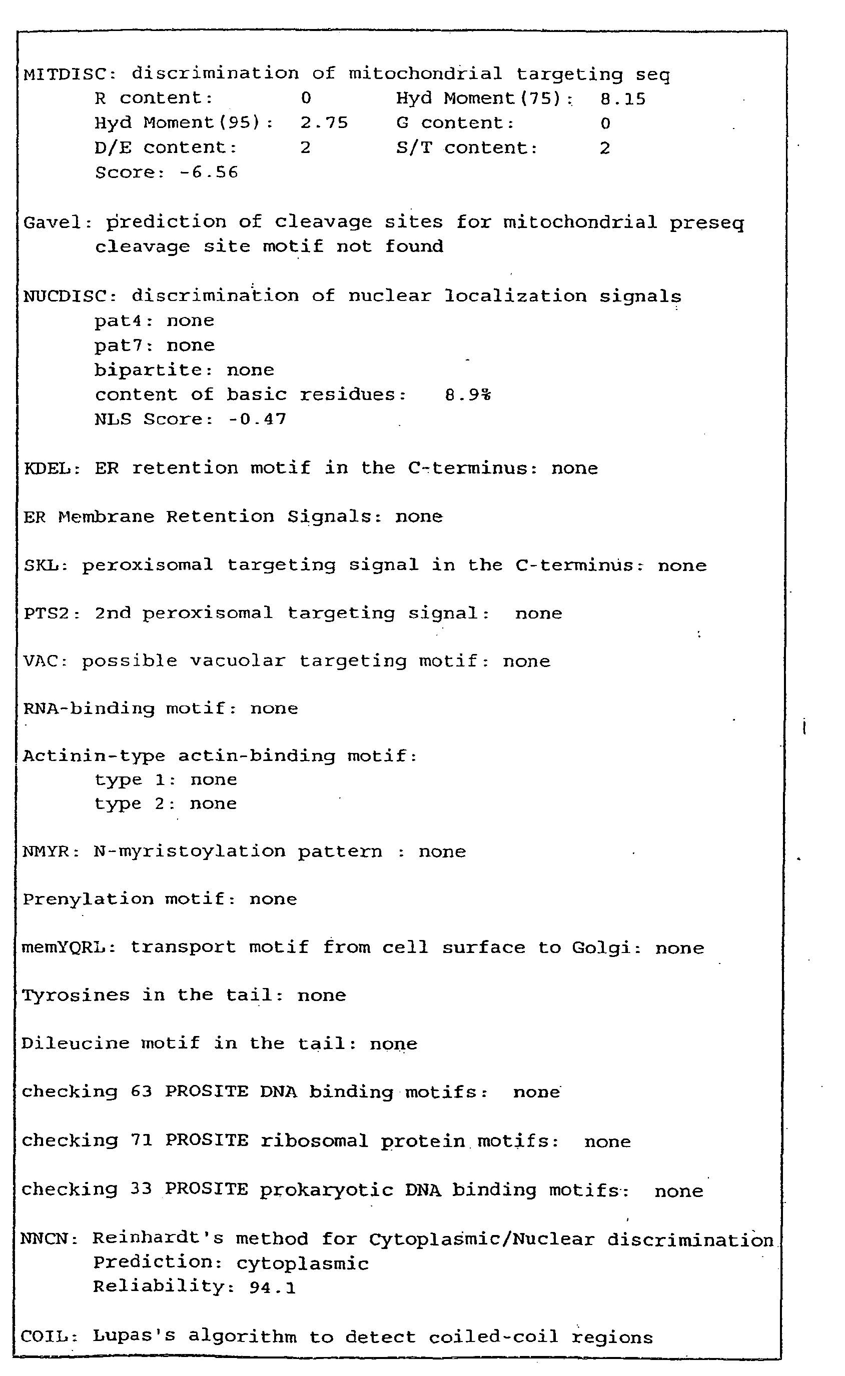

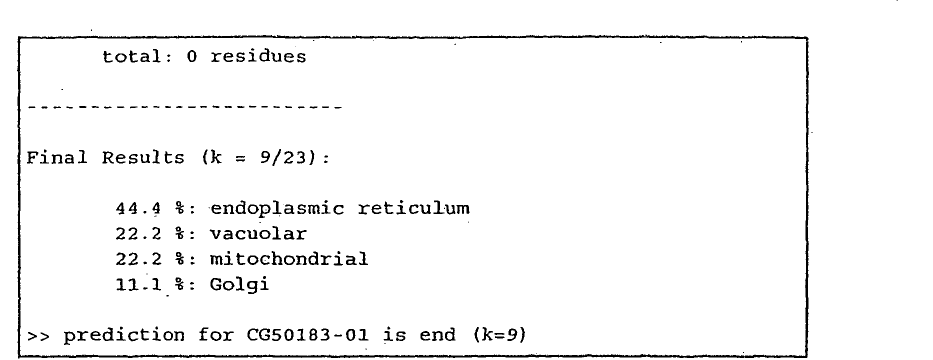

A search of the NOV4a protein against the Geneseq database, a proprietary database that contains sequences published in patents and patent publication, yielded several homologous proteins shown in Table 4C.

| **Table 4C. Geneseq Results for NOV4a** | | | | |
|---|---|---|---|---|
| **Geneseq Identifier** | **Protein/Organism/Length [Patent #, Date]** | **NOV4a Residues/Match Residues** | **Identities/ Similarities for the Matched Region** | **Expect Value** |
| ABP81716 | Human C-C chemokine receptor 11 protein SEQ ID NO:607 - Homo sapiens, 350 aa. [WO200261087-A2, 08-AUG-2002] | 1..348 3..350 | 348/348 (100%) 348/348 (100%) | 0.0 |
| AAB62389 | Human chemokine receptor CCX CKR polypeptide - Homo sapiens, 382 aa. [WO200127146-A2, 19-APR-2001] | 1..348 3..350 | 348/348 (100%) 348/348 (100%) | 0.0 |
| AAG67237 | Amino acid sequence of human chemokine receptor CCR11-Homo sapiens, 350 aa. [WO200166598-A2, 13-SEP-2001] | 1..348 3..350 | 348/348 (100%) 348/348 (100%) | 0.0 |
| AAU08994 | Human G protein-coupled receptor, GPCR, 2398 - Homo sapiens, 350 aa. [WO200164882-A2,07-SEP-2001] | 1..348 3..350 | 348/348(100%) 348/348 (100%) | 0.0 |
| AAG80119 | Human CCR11 protein - Homo sapiens, 350 aa. [WO200172830-A2, 04-OCT-2001] | 1..348 3..350 | 348/348(100%) 348/348(100%) | 0.0 |

In a BLAST search of public sequence databases, the NOV4a protein was found to have homology to the proteins shown in the BLASTP data in Table 4D.

| **Table 4D. Public BLASTP Results for NOV4a** | | | | |
|---|---|---|---|---|
| **Protein Accession Number** | **Protem/Organism/Length** | **NOV4a Residues/Match Residues** | **Identities/Similarities for the Matched Portion** | **Expect Value** |
| Q9NPB9 | C-C chemokine receptor type 11 (C-C CKR-11) (CC-CKR-11) (CCR-11) (Chemokine receptor-like 1) (CCRL1) (CCX CKR) - Homo sapiens (Human), 350 aa. | 1..348 3..350 | 348/348 (100%) 348/348 (100%) | 0.0 |
| CAC17062 | Sequence 1 from Patent WO0064941 - Homo sapiens (Human), 350 aa. | 1..348 3..350 | 346/348 (99%) 347/348 (99%) | 0.0 |
| P35350 | C-C chemokine receptor type 11 (C-C CKR-11) (CC-CKR-11) (CCR-11) (Possible gustatory receptor type B) (PPR1 protein) - Bos taurus (Bovine), 350 aa. | 1..348 3..350 | 299/348(85%) 326/348 (92%) | e-180 |
| Q924I3 | Chemokine receptor CCR11 - Mus musculus (Mouse), 350 aa. | 1..348 3..350 | 297/348 (85%) 323/348 (92%) | e-177 |
| Q8COM1 | Chemokine receptor CCR11 homolog - Mus musculus (Mouse), 350 aa. | 1..348 3..350 | 297/348 (85%) 323/348 (92%) | e-177 |

PFam analysis predicts that the NOV4a protein contains the domains shown in the Table 4E.

| **Table 4E. Domain Analysis of NOV4a** | | | |
|---|---|---|---|
| **Pfam Domain** | **NOV4a Match Region** | **Identities/ Similarities for the Matched Region** | **Expect Value** |
| 7tm_1 | 56..301 | 90/276 (33%) for the Matched Region 197/276 (71%) | 1.7e-76 |

### Example 5.

The NOV5 clone was analyzed, and the nucleotide and encoded polypeptide sequences are shown in Table 5A

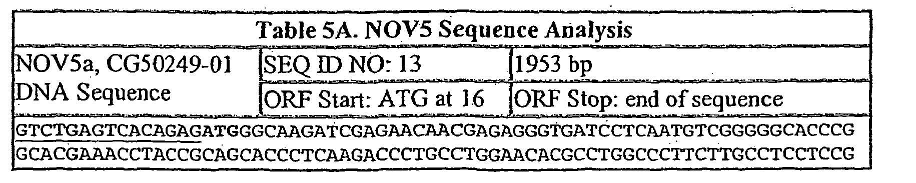

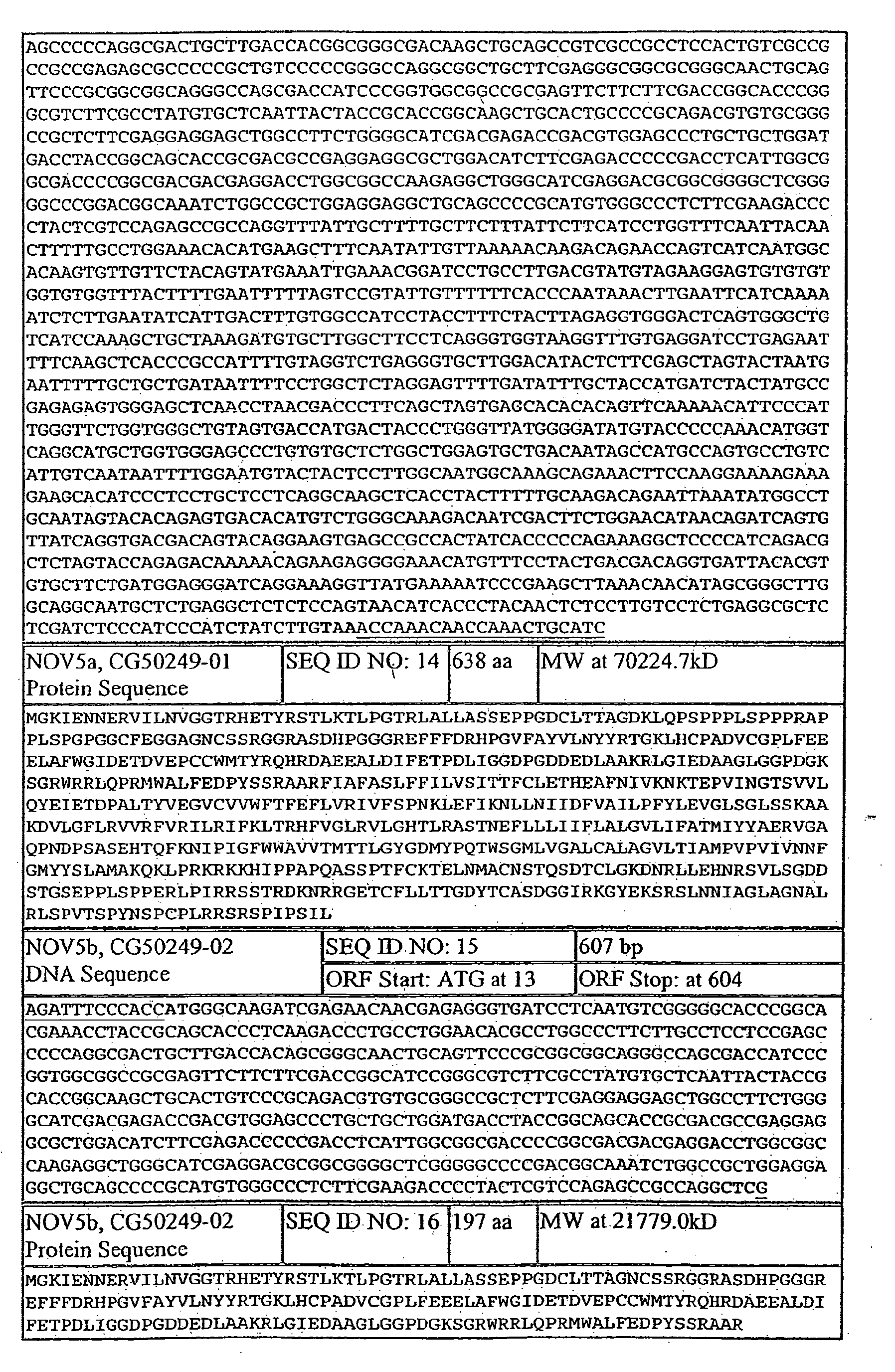

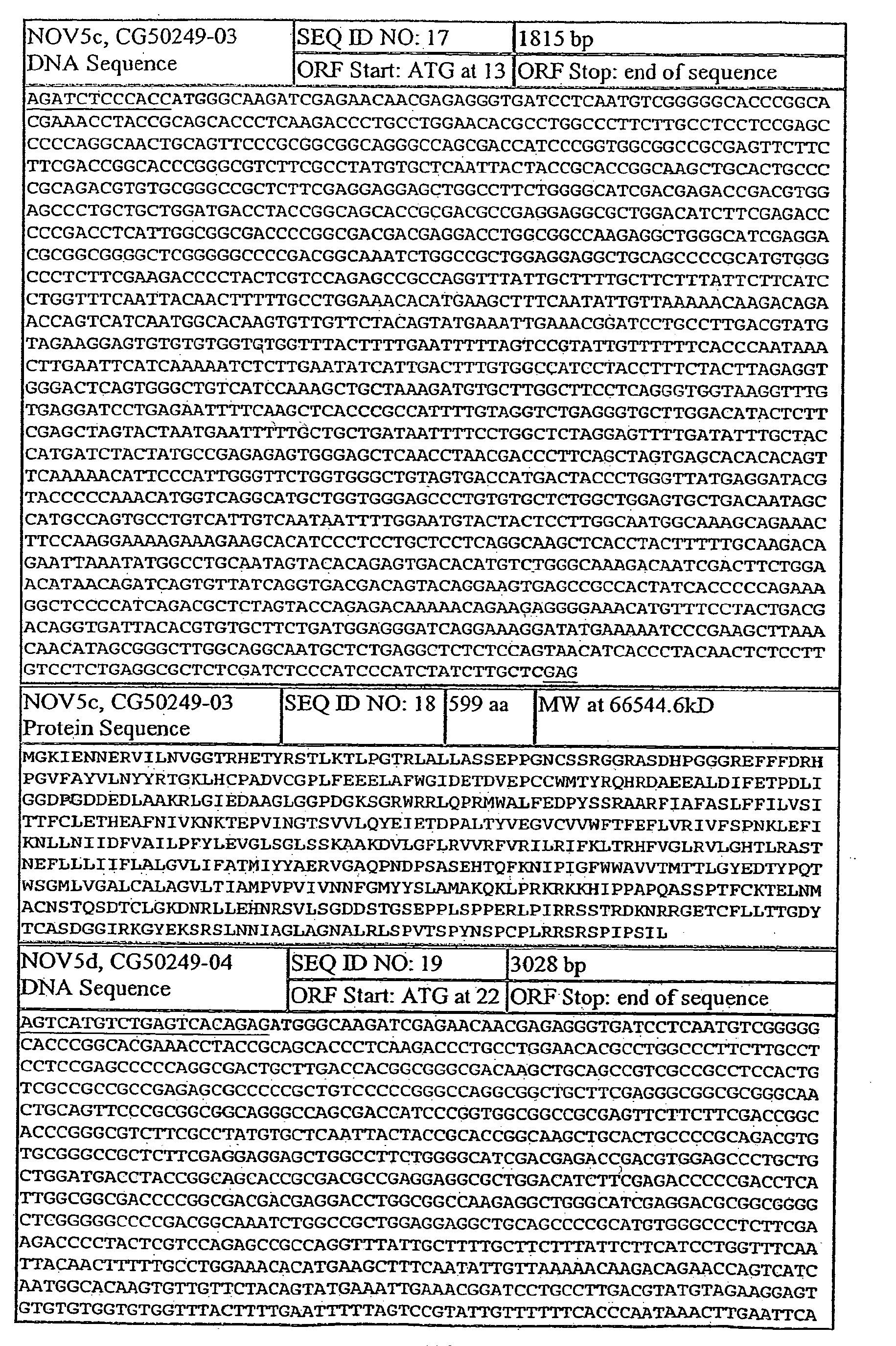

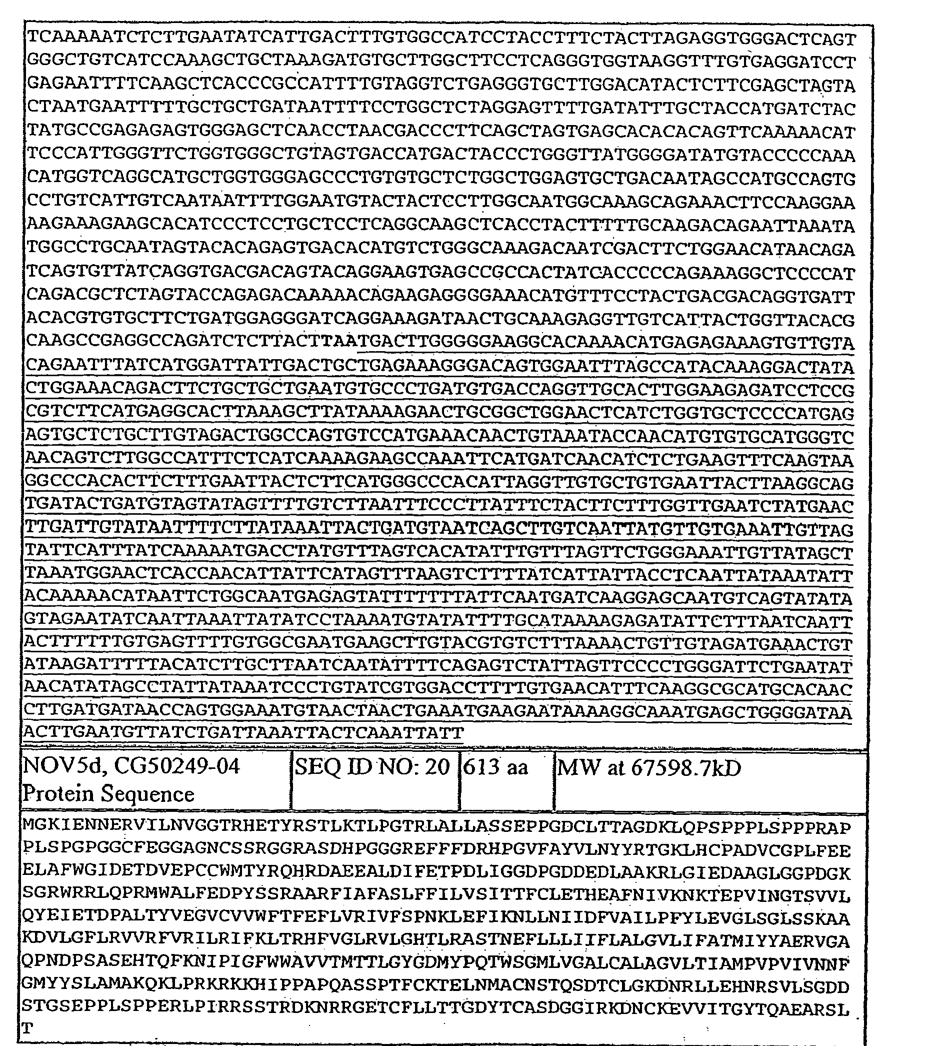

A ClustalW comparison of the above protein sequences yields the following sequence alignment shown in Table 5B.

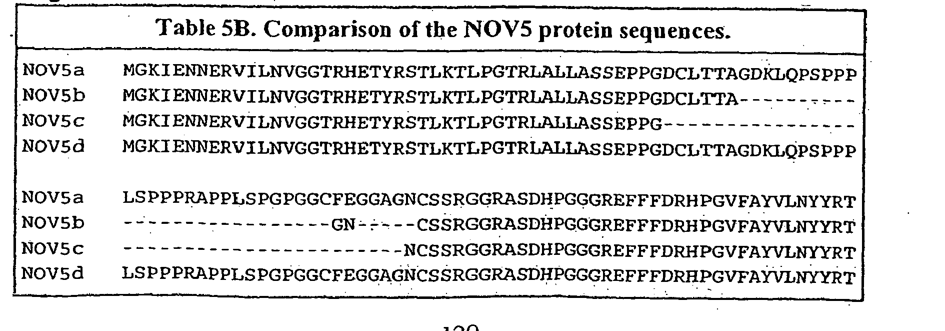

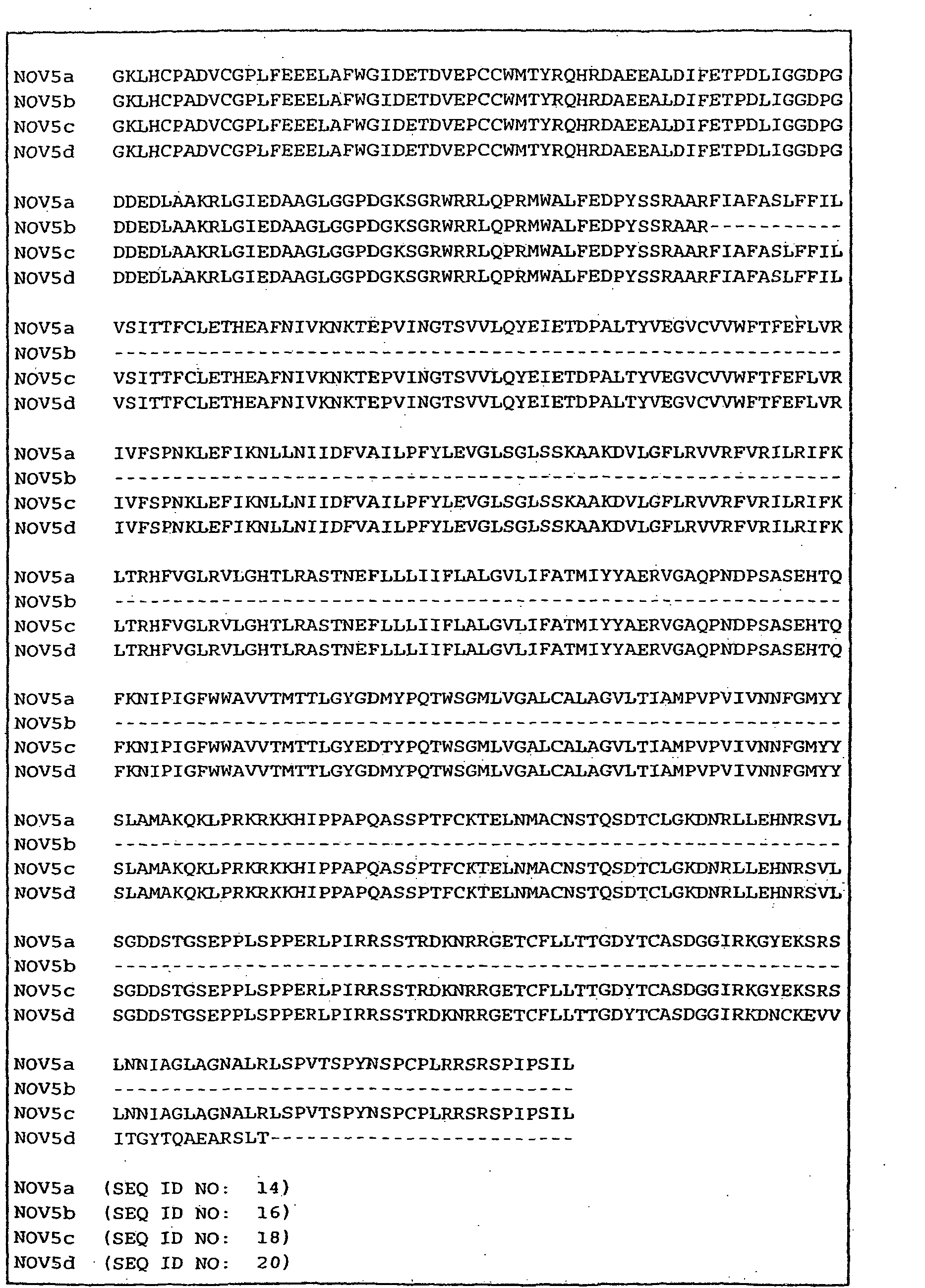

Further analysis of the NOV5a protein yielded the following properties shown in Table 5C.

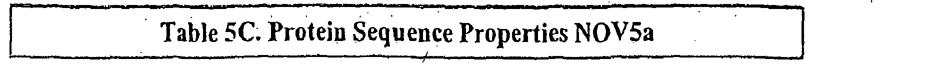

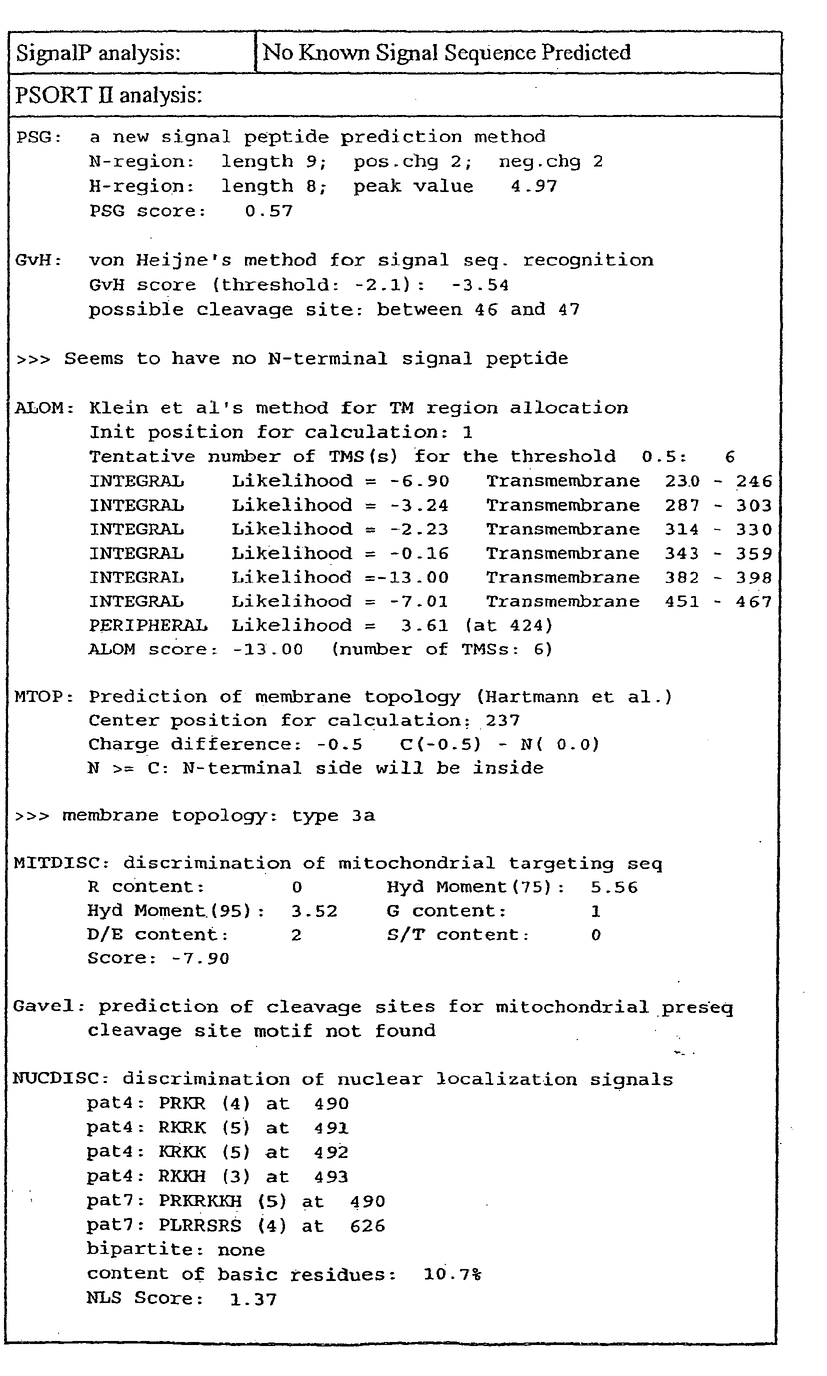

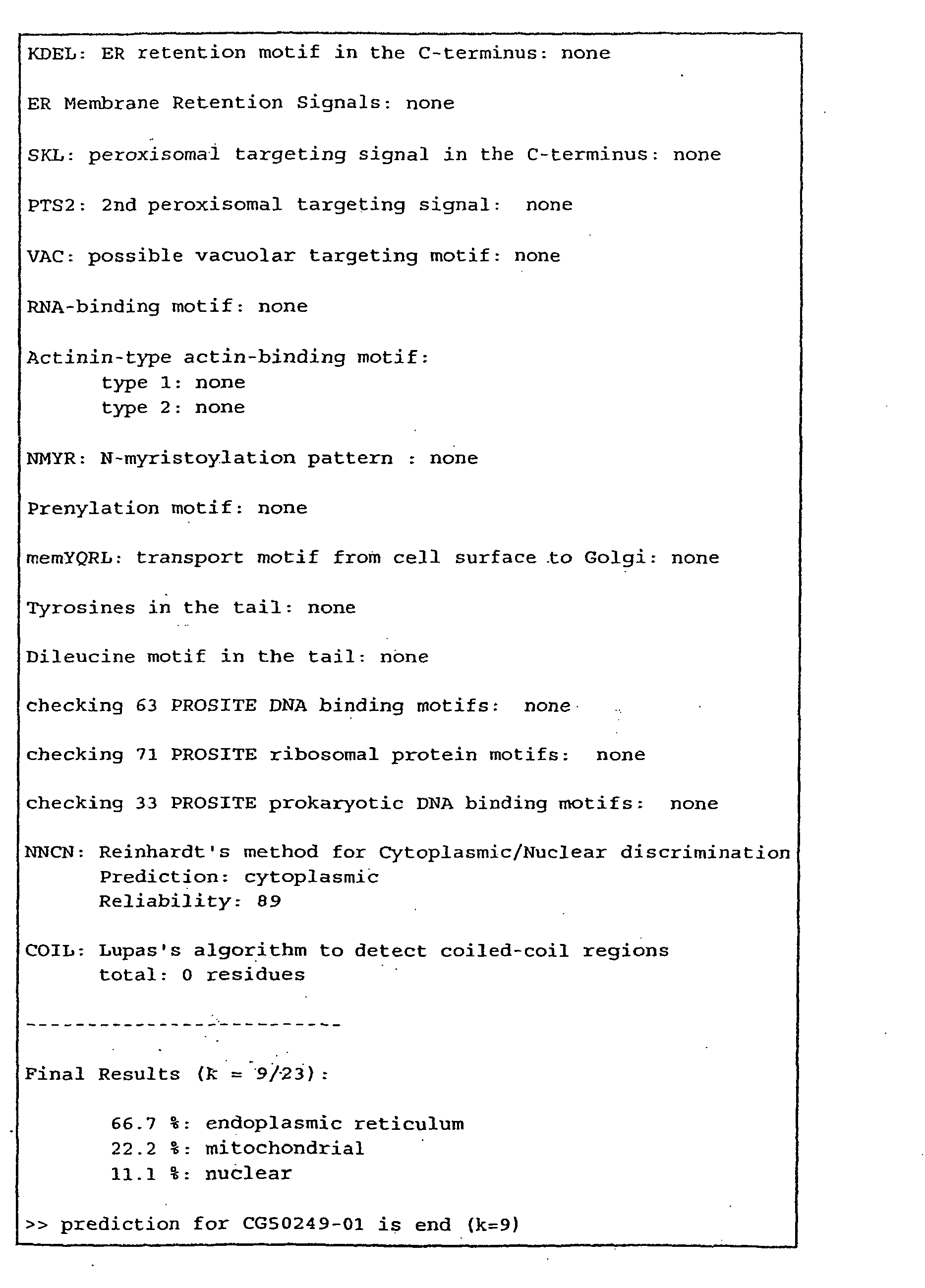

A search of the NOV5a protein against the Geneseq database, a proprietary database that contains sequences published in patents and patent publication, yielded several homologous proteins shown in Table 5D.

| **Table 5D. Geneseq Results for NOV5a** | | | | |
|---|---|---|---|---|
| **Geneseq Identifier** | **Protein/Organism/Length [Patent #, Date]** | **NOV5a Residues/ Match Residues** | **Identities/ Similarities for the Matched Region** | **Expect Value** |
| ABB78396 | Longer splice variant of a human voltage-gated potassium channel - Homo sapiens, 638 aa. [GB2372503-A, 28-AUG-2002] | 1..638 1..638 | 638/638 (100%) 638/638 (100%) | 0.0 |
| AAO14201 | Human transporter and ion channel TRICH-18 - Homo sapiens, 638 aa. [WO200204520-A2, 17-JAN-2002] | 1..638 1..638 | 638/638 (100%) 638/638 (100%) | 0.0 |
| ABP52157 | Human 53763 transporter protein SEQ ID NO:11 - Homo sapiens, 638 aa. [WO200255701-A2, 18-JUL-2002] | 1..638 1..638 | 638/638 (100%) 638/638 (100%) | 0.0 |
| ABG70285 | Human novel polypeptide #1 - Homo sapiens, 638 aa. [WO200257452-A2, 25-JUL-2002] | 1..638 1..638 | 638/638 (100%) 638/638 (100%) | 0.0 |
| ABB78396 | Longer splice variant of a human voltage-gated potassium channel - Homo sapiens, 638 aa. [GB2372503-A, 28-AUG-2002] | 1..638 1..638 | 638/638 (100%) 638/638 (100%) | 0.0 |

In a BLAST search of public sequence databases, the NOV5a protein was found to have homology to the proteins shown in the BLASTP data in Table 5E.

| **Table 5E. Public BLASTP Results for NOV5a** | | | | |
|---|---|---|---|---|
| **Protein Accession Number** | **Protein/Organism/Length** | **NOV5a Residues/ Match Residues** | **Identities/ Similarities for the Matched Portion** | **Expect Value** |
| Q96PR1 | Voltage gated potassium channel Kv3.2b (Potassium voltage-gated potassium channel subfamily C member 2) - Homo sapiens (Human), 638 aa. | 1..638 1..638 | 638/638 (100%) 638/638 (100%) | 0.0 |
| P22462 | Potassium voltage-gated channel subfamily C member 2 (Potassium channel Kv3.2) (KSHIIIA) - Rattus norvegicus (Rat), 638 aa. | 1..638 1..638 | 623/638(97%) 625/638 (97%) | 0.0 |
| Q96PR0 | Voltage gated potassium channel Kv3.2a - Homo sapiens (Human), 613 aa. | 1..593 1..593 | 593/593 (100%) 593/593 (100%) | 0.0 |
| A39402 | potassium channel protein IIIA form I, shaker-type - rat, 613 aa. | 1..593 1..593 | 578/593 (97%) 580/593 (97%) | 0.0 |
| S22703 | voltage-gated potassium channel protein Raw1 - rat, 624 aa. | 1..593 1..593 | 577/593 (97%) 579/593 (97%) | 0.0 |

PFam analysis predicts that the NOV5a protein contains the domains shown in the Table 5F.

| **Table 5F. Domain Analysis of NOV5a** | | | |
|---|---|---|---|
| **Pfam Domain** | **NOV5a Match Region** | **Identities/ Similarities for the Matched Region** | **Expect Value** |
| K_tetra | 9..156 | 50/161 (31%) 121/161 (75%) | 3.2e-42 |
| ion_trans | 281..472 | 53/233 (23%) 155/233 (67%) | 6.3e-43 |

### Example 6.

The NOV6 clone was analyzed, and the nucleotide and encoded polypeptide sequences are shown in Table 6A.

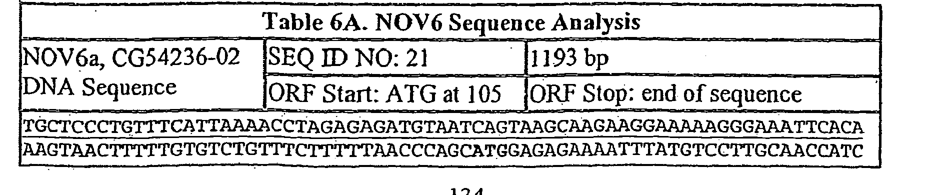

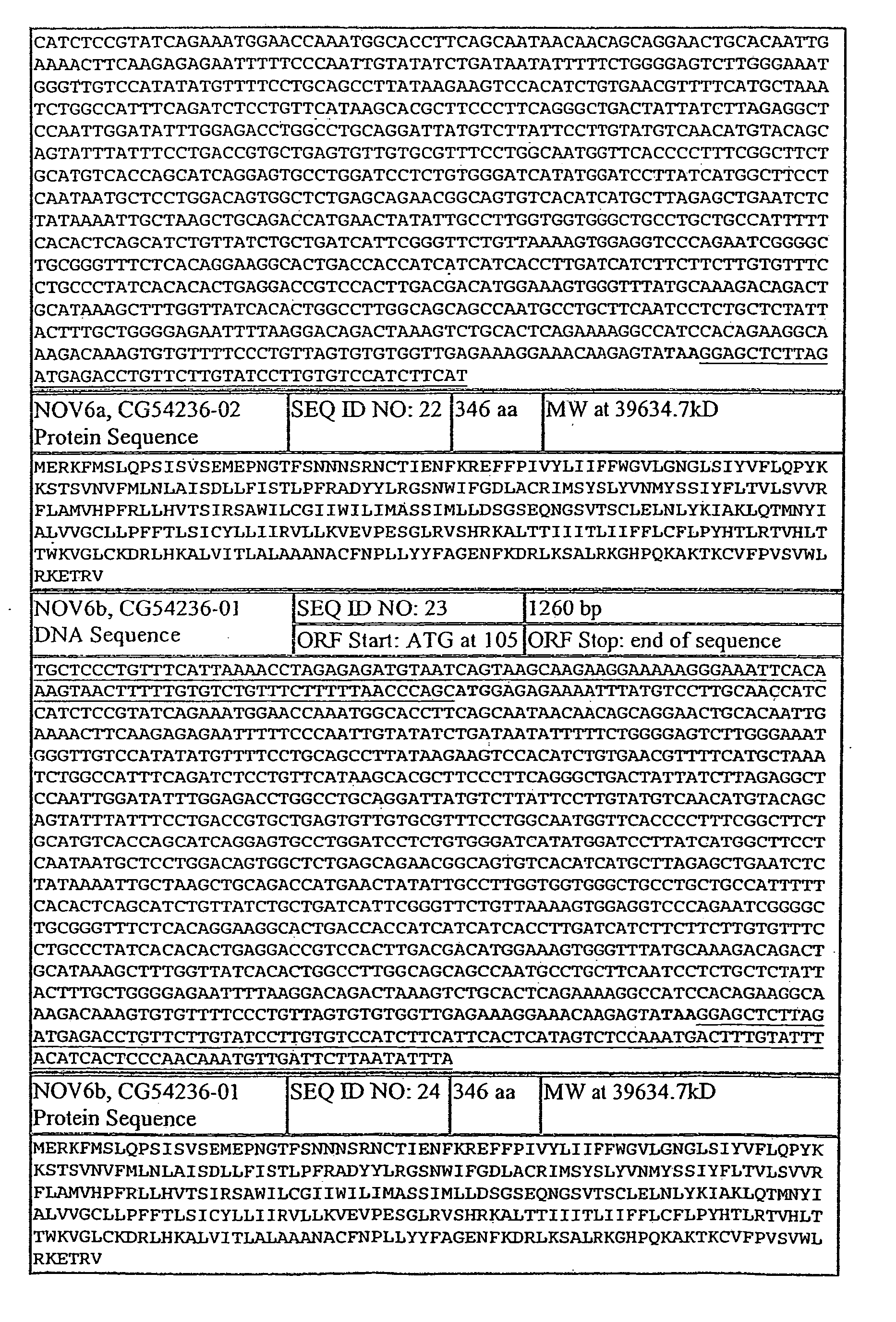

A ClustalW comparison of the above protein sequences yields the following sequence alignment shown in Table 6B.

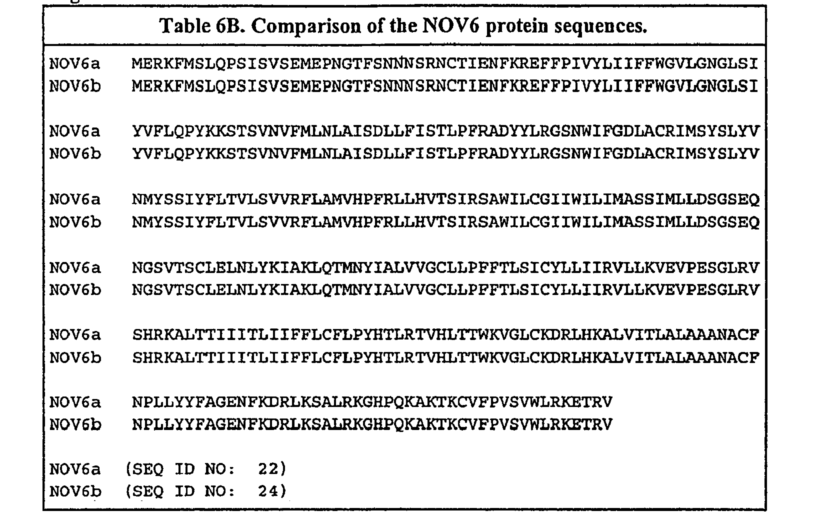

Further analysis of the NOV6a protein yielded the following properties shown in Table 6C.

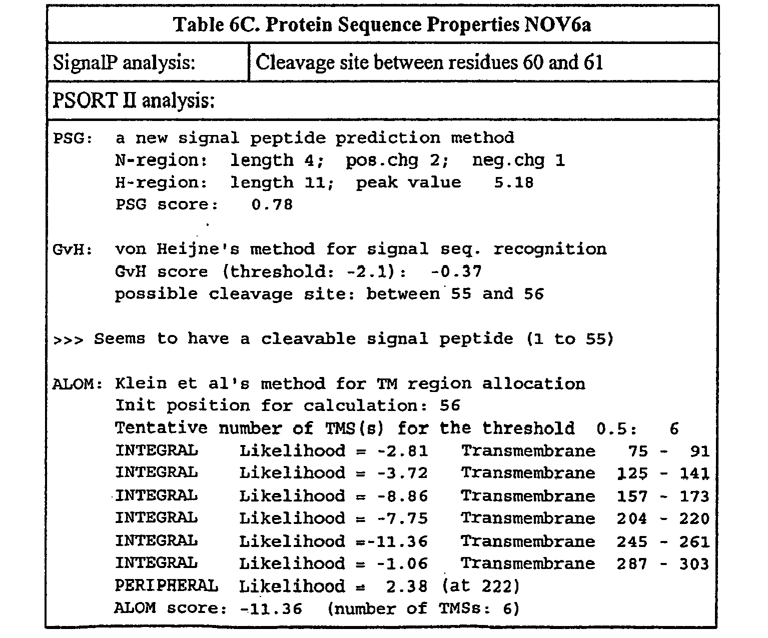

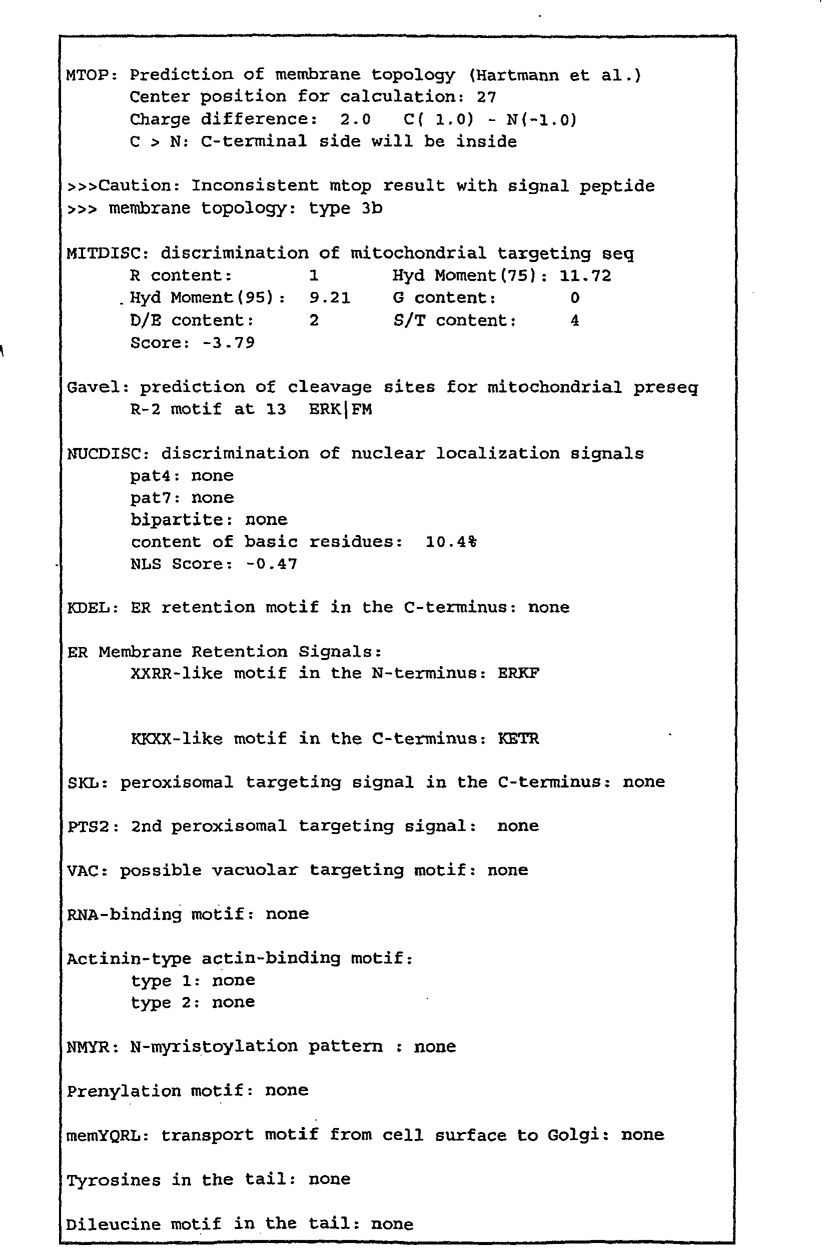

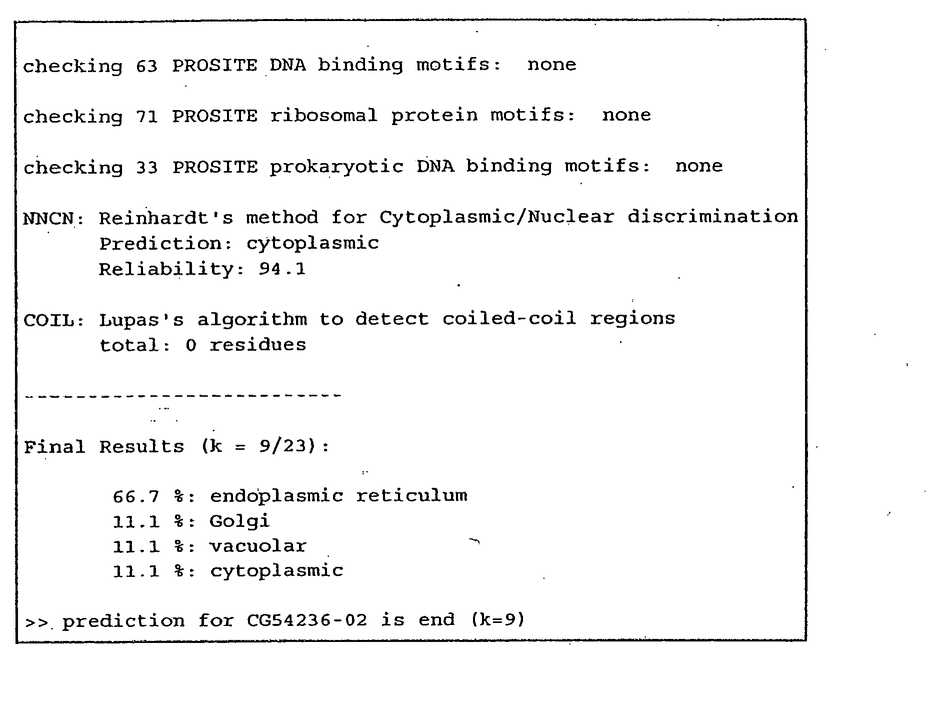

A search of the NOV6a protein against the Geneseq database, a proprietary database that contains sequences published in patents and patent publication, yielded several homologous proteins shown in Table 6D.

| **Table 6D. Geneseq Results for NOV6a** | | | | |
|---|---|---|---|---|
| **Geneseq Identifier** | **Protein/Organism/Length [Patent #, Date]** | **NOV6a Residues/Match Residues** | **Identities/ Similarities for the Matched Region** | **Expect Value** |
| ABP81707 | Human cysteinyl leukotriene CYSLT2 receptor protein SEQ ID NO:589 - Homo sapiens, 346 aa. [WO200261087-A2, 08-AUG-2002] | 1..346 1..346 | 346/346 (100%) 346/346 (100%) | 0.0 |
| ABP95624 | Human GPCR polypeptide SEQ ID NO 58 - Homo sapiens, 346 aa. [WO200216548-A2, 28-FEB-2002] | 1..346 1..346 | 346/346 (100%) 346/346 (100%) | 0.0 |
| ABU11923 | Human G-protein coupled receptor HGPRBMY11v1 - Homo sapiens, 346 aa. [WO200286123-A2, 31-OCT-2002] | 1..346 1..346 | 346/346 (100%) 346/346 (100%) | 0.0 |
| ABB05229 | Human LTD4-like G protein-coupled receptor protein SEQ ID NO:2 - Homo sapiens, 346 aa. [WO200194580-A1, 13-DEC-2001] | 1..346 1..346 | 346/346 (100%) 346/346 (100%) | 0.0 |
| AAG77965 | Human G-protein coupled receptor PFI-017* - Homo sapiens, 346 aa. [US2001039037-A1, 08-NOV-2001] | 1..346 1..346 | 346/346 (100%) 346/346 (100%) | 0.0 |

In a BLAST search of public sequence databases, the NOV 6a protein was found to have homology to the proteins shown in the BLASTP data in Table 6E.

| **Table 6E. Public BLASTP Results for NOV6a** | | | | |
|---|---|---|---|---|
| **Protein Accession Number** | **Protein/Organism/Length** | **NOV6a Residues/ Match Residues** | **Identities/ Similarities for the Matched Portion** | **Expect Value** |
| Q9NS75 | Cysteinyl leukotriene receptor 2 (CysLTR2) (PSEC0146) (HG57) (HPN321) (hGPCR21) - Homo sapiens (Human), 346 aa. | 1..346 1..346 | 346/346 (100%) 346/346 (100%) | 0.0 |
| CAC69290 | Sequence I from Patent WO0159118 - Homo sapiens (Human), 346 aa. | 1..346 1..346 | 344/346 (99%) 345/346 (99%) | 0.0 |
| Q95N03 | Cysteinyl leukotriene receptor 2 (CysLTR2) - Sus scrofa (Pig), 345 aa. | 1..346 1..345 | 275/347 (79%) 300/347 (86%) | e-158 |
| Q8R528 | Cysteinyl leukotriene 2 receptor - Mus musculus (Mouse), 309 aa. | 17..324 1..308 | 226/308 (73%) 256/308 (82%) | e-132 |
| Q920A1 | Cysteinyl leukotriene receptor 2 (CysLTR2) - Mus musculus (Mouse), 309 aa. | 17..324 1..308 | 224/308 (72%) 255/308 (82%) | e-131 |

PFam analysis predicts that the NOV6a protein contains the domains shown in the Table 6F.

| **Table 6F. Domain Analysis of NOV6a** | | | |
|---|---|---|---|
| **Pfam Domain** | **NOV6a Match Region** | **Identities/ Similarities for the Matched Region** | **Expect Value** |
| 7tm_1 | 55..305 | 89/277 (32%) 185/277 (67%) | 6.1e-54 |
| TAS2R | 33..324 | 64/314 (20%) 173/314 (55%) | 0.019 |

### Example 7.

The NOV7 clone was analyzed, and the nucleotide and encoded polypeptide sequences are shown in Table 7A.

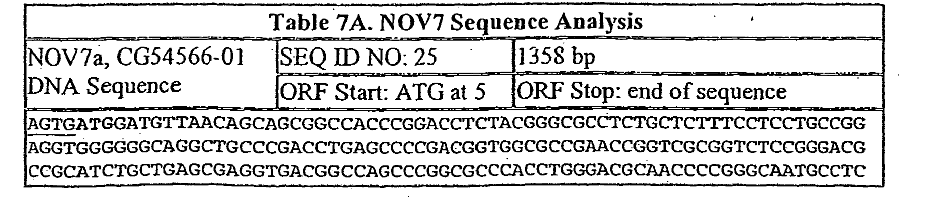

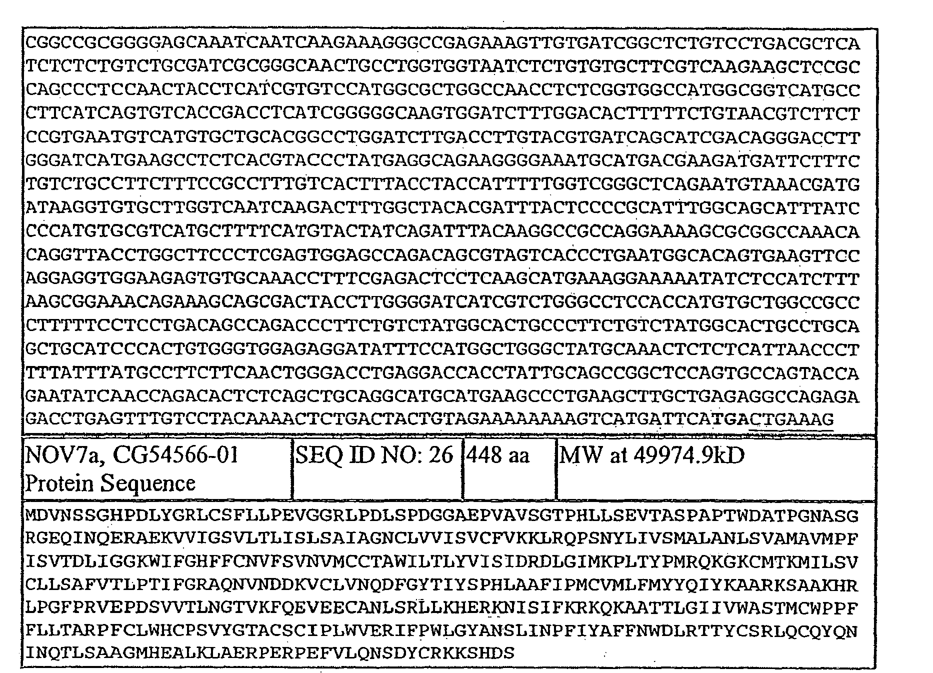

Further analysis of the NOV7a protein yielded the following properties shown in Table 7B.

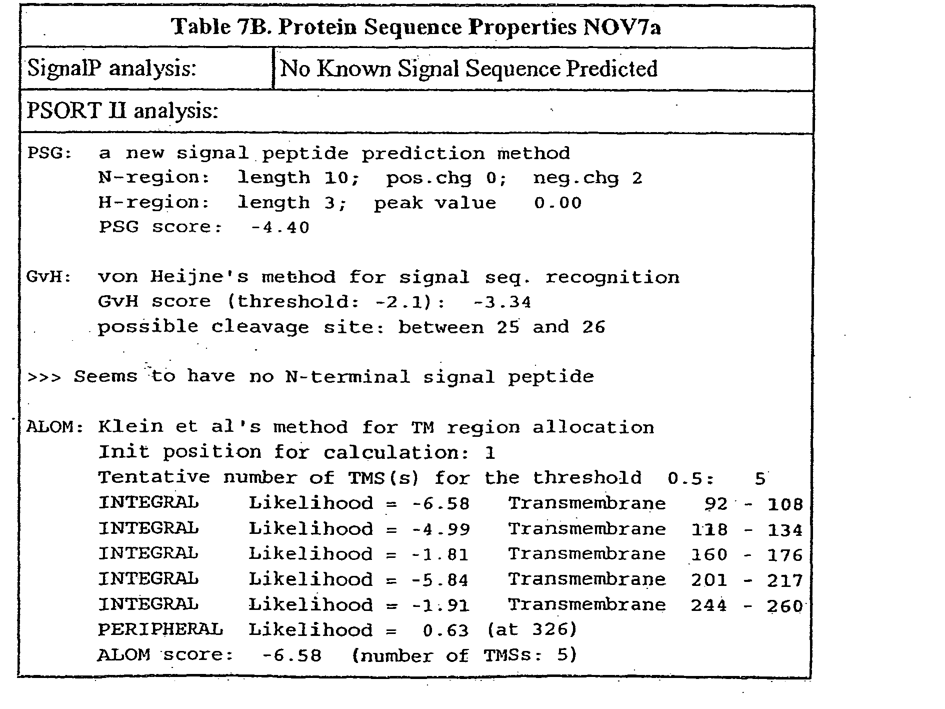

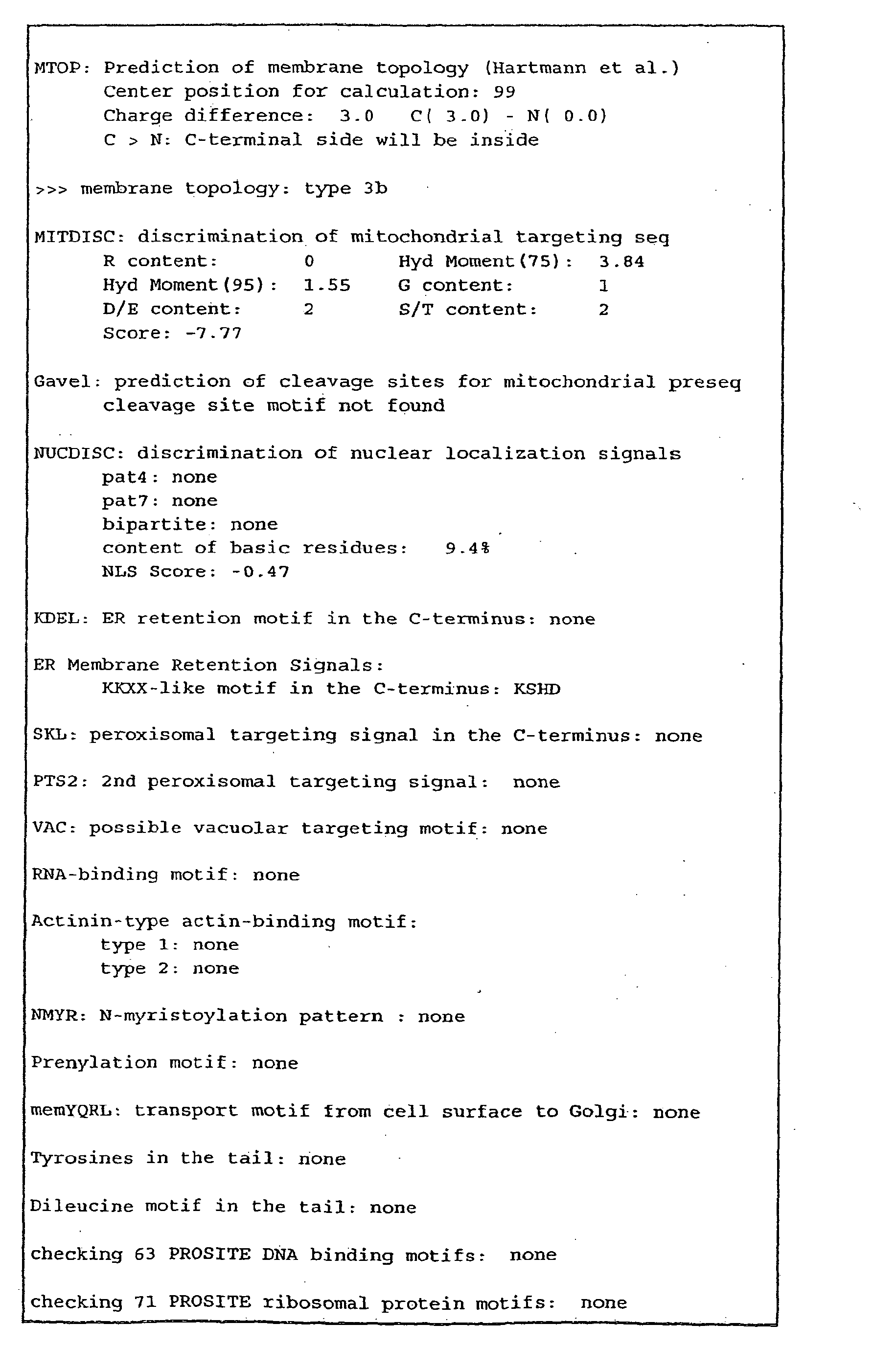

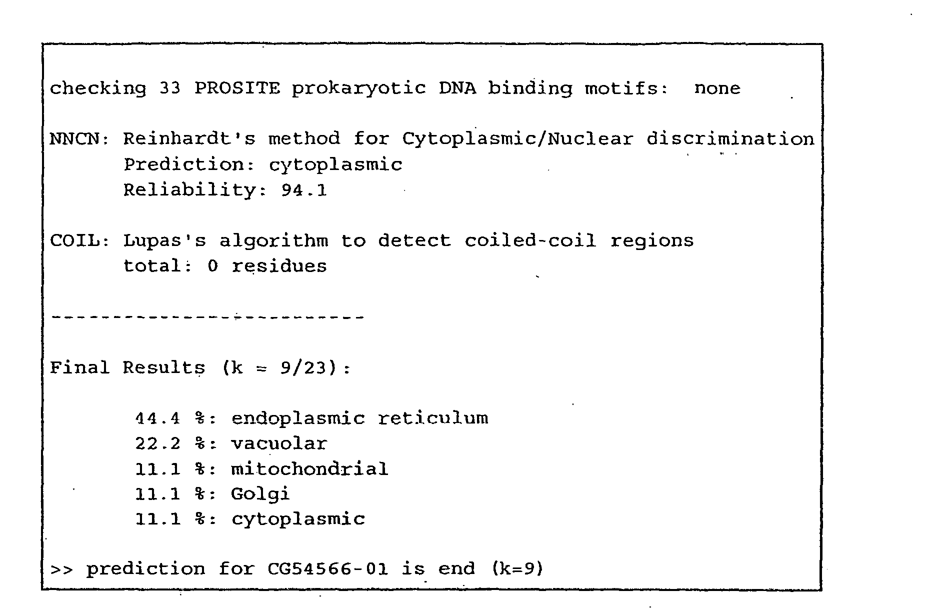

A search of the NOV7a protein against the Geneseq database, a proprietary database that contains sequences published in patents and patent publication, yielded several homologous proteins shown in Table 7C.

| **Table 7C. Geneseq Results for NOV7a** | | | | |
|---|---|---|---|---|
| **Geneseq Identifier** | **Protein/Organism/Length [Patent #, Date]** | **NOV7a Residues/Match Residues** | **Identities/Similarities for the Matched Region** | **Expect Value** |
| AAE15653 | Human 5-hydroxytryptamine-7-receptor-like protein, NOV4 - Homo sapiens, 448 aa. [WO200194416-A2,13-DEC-2001] | 1..448 1..448 | 448/448 (100%) 448/448 (1 00%) | 0.0 |
| ABP81770 | Human 5-HT7 receptor protein SEQ ID NO:22 - Homo sapiens, 445 aa. [WO200261087-A2, 08-AUG-2002] | 1..448 2..445 | 368/451 (81%) 396/451 (87%) | 0.0 |
| AAR54782 | Human brain serotonin receptor 5-HT4B protein - Homo sapiens, 445 aa. [WO9409828-A, 11-MAY-1994] | 1..448 2..445 | 368/451 (81%) 396/451 (87%) | 0.0 |
| ABB56329 | Non-endogenous human GPCR protein, SEQ ID NO: 451 - Homo sapiens, 445 aa. [WO200177172-A2, 18-OCT-2001] | 1..448 2..445 | 367/451 (81%) 395/451 (87%) | 0.0 |
| AAR57200 | Rat 5HT6 receptor - Rattus rattus, 448 aa. [FR2699922-A, 01-JUL-1994] | 1..448 2..448 | 357/454 (78%) 389/454 (85%) | 0.0 |

In a BLAST search of public sequence databases, the NOV7a protein was found to have homology to the proteins shown in the BLASTP data in Table 7D.

| **Table 7D. Public BLASTP Results for NOV7a** | | | | |
|---|---|---|---|---|
| **Protein Accession Number** | **Protein/Organism/Length** | **NOV7a Residues/Match Residues** | **Identities/ Similarities for the Matched Portion** | **Expect Value** |
| CAD32672 | Sequence 11 from Patent WO0194416 - Homo sapiens (Human), 448 aa. | 1.448 1..448 | 448/448 (100%) 448/448 (100%) | 0.0 |
| AAH47526 | 5-hydroxytryptamine (serotonin) receptor 7 (adenylate cyclase-coupled) - Homo sapiens (Human), 445 aa. | 1..448 2..445 | 368/451 (81%) 396/451 (87%) | 0.0 |
| P34969 | 5=hydroxytryptamine 7 receptor (5-HT-7) (5-HT-X) (Serotonin receptor) (5HT7) - Homo sapiens (Human), 479 aa. | 1..436 2..433 | 357/439(81%) 385/439 (87%) | 0.0 |
| P32305 | 5-hydroxytryptamine 7 receptor (5-HT-7) (5-HT-X) (Serotonin receptor) (5HT7) (GPRFO) - Rattus norvegicus (Rat), 448 aa. | 1..448 2..448 | 357/454 (78%) 389/454 (85%) | 0.0 |
| Q8SPH2 | Serotonin 5-hydroxytryptamine 7-a receptor - Sus scrofa (Pig), 447 aa. | 1..448 2..447 | 361/454 (79%) 387/454 (84%) | 0.0 |

PFam analysis predicts that the NOV7a protein contains the domains shown in the Table 7E

| **Table 7E. Domain Analysis of NOV7a** | | | |
|---|---|---|---|
| **Pfam Domain** | **NOV7a Match Region** | **Identities/ Similarities for the Matched Region** | **Expect Value** |
| 7tm_1 | 97..387 | 76/315 (24%) 219/315(70%) | 3.8e-56 |

### Example 8.

The NOV8 clone was analyzed, and the nucleotide and encoded polypeptide sequences are shown in Table 8A.

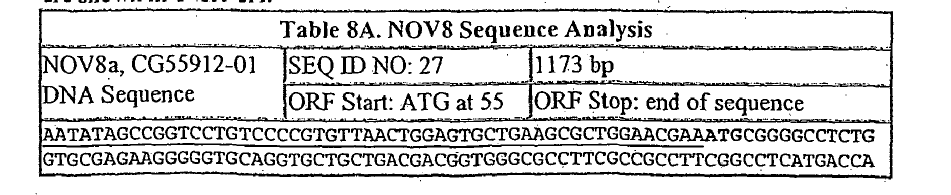

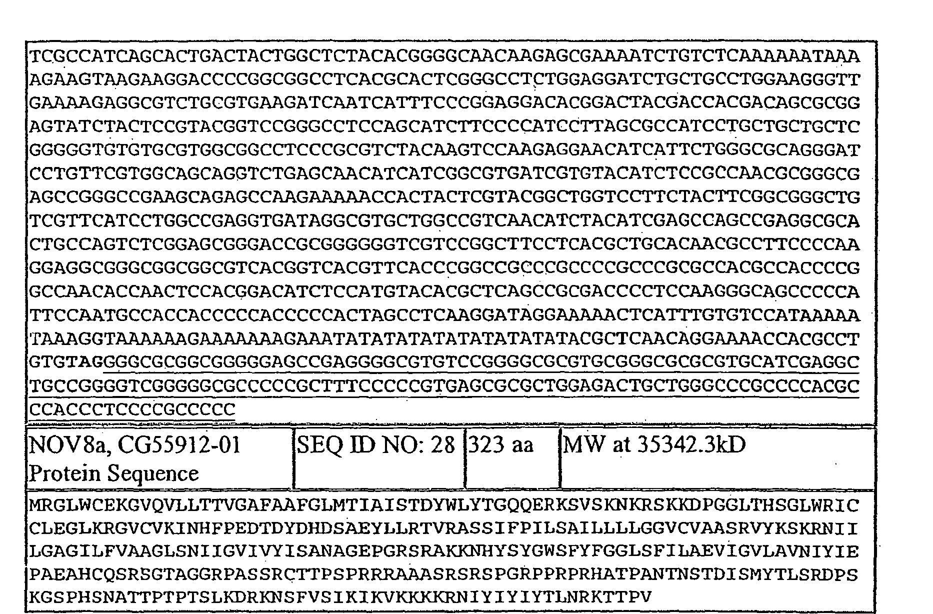

Further analysis of the NOV8a protein yielded the following properties shown in Table 8B

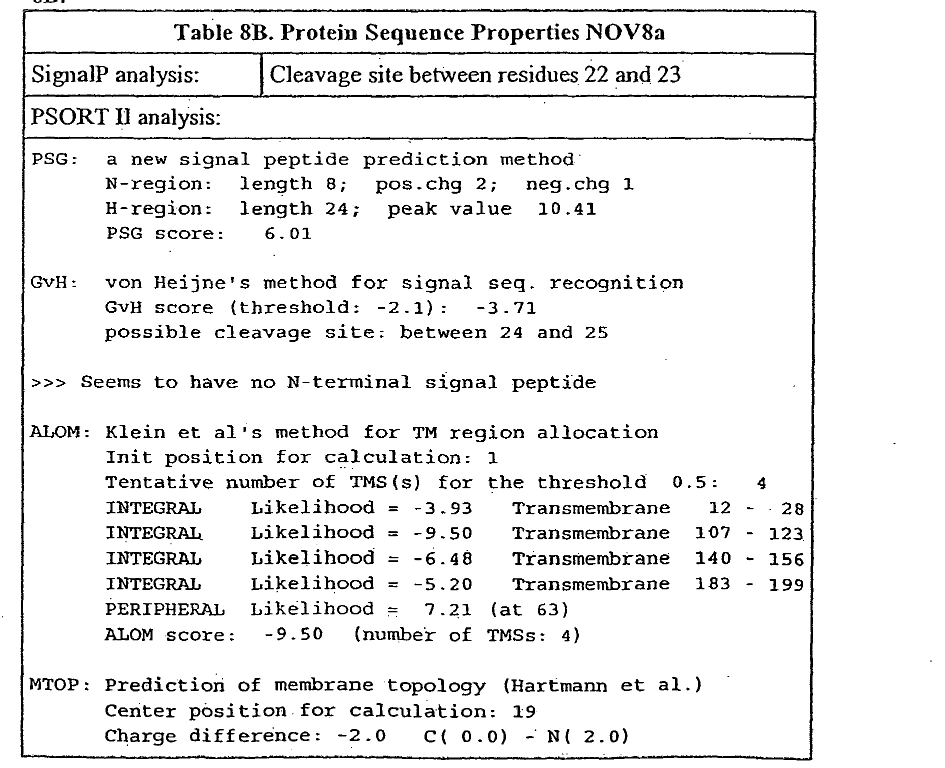

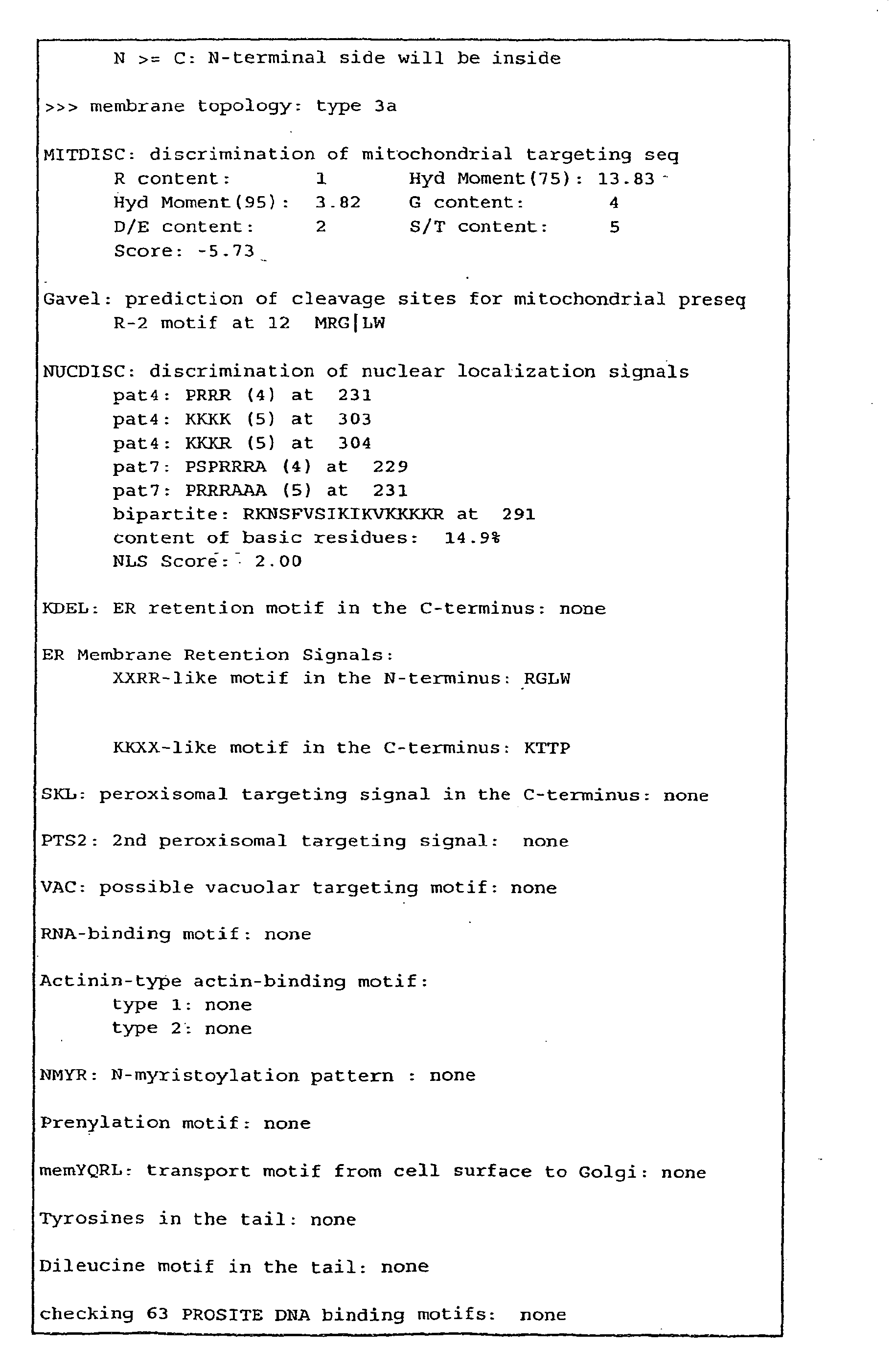

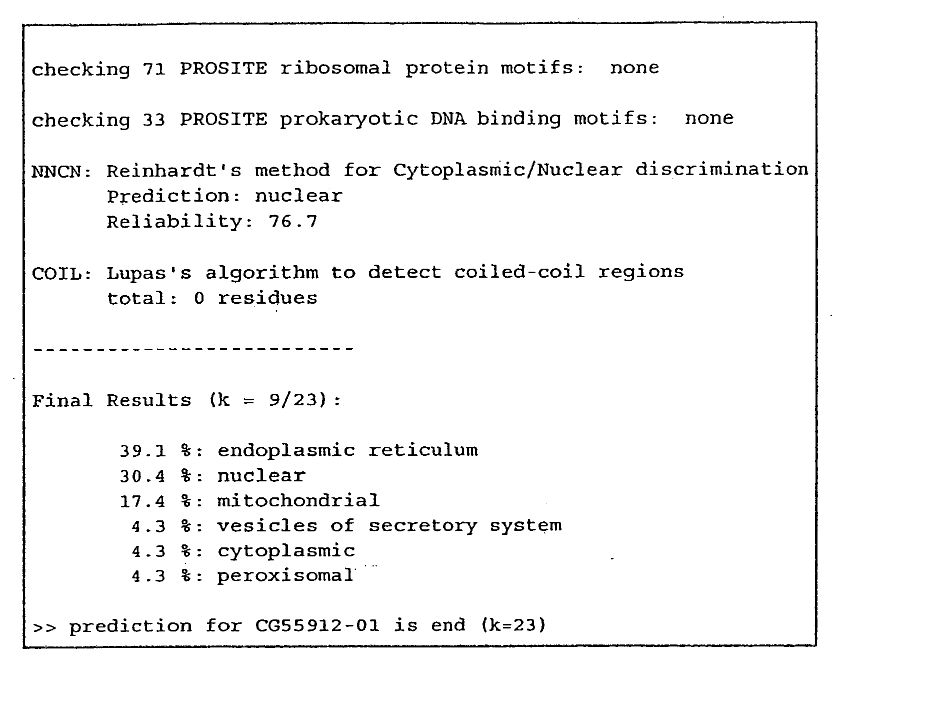

A search of the NOV8a protein against the Geneseq database, a proprietary database that contains sequences published in patents and patent publication, yielded several homologous proteins shown in Table 8C.

| **Table 8C. Geneseq Results for NOV8a** | | | | |
|---|---|---|---|---|
| **Geneseq Identifier** | **Proteia/Organism/Leagth [Patent #, Date]** | **NOV8a Residues/ Match Residues** | **Identities/ Similarities for the Matched Region** | **Expect Value** |
| AAY70462 | Human membrane channel protein-12 (MECHP-12) - Homo sapiens, 323 aa. [WO200012711-A2, 09-MAR-2000] | 7..323 5..323 | 183/327(55%) 233/327 (70%) | 6e-93 |
| ABB11805 | Human voltage gated Ca channel subunit homologue, SEQ ID NO:2175 - Homo sapiens, 325 aa. [WO200157188-A2,09-AUG-2001] | 7..323 7..325 | 183/327(55%) 233/327 (70%) | 1e-92 |
| AAY84376 | A human voltage-gated calcium channel designated CACNGLIKE1 - Homo sapiens, 323 aa. [WO200014223-A1, 16-MAR-2000] | 7..323 5..323 | 183/327 (55%) 233/327 (70%) | 1e-92 |
| AAU97153 | Mouse neuronal voltage-gated calcium channel gamma subunit Cacng2 - Murinae gen. sp, 323 aa. [US6365337-B1, 02-APR-2002] | 7..323 5..323 | 183/327 (55%) 232/327 (69%) | 2e-92 |
| AAY84374 | A human a neuronal voltage-gated calcium chanel polypeptide - Homo sapiens, 315 aa. [WO200014225-A1, 16-MAR-2000] | 6..323 4..315 | 173/324 (53%) 217/324 (66%) | 1e-85 |

In a BLAST search of public sequence databases, the NOV8a protein was found to have homology to the proteins shown in the BLASTP data in Table 8D.

| **Table 8D. Public BLASTP Results for NOV8a** | | | | |
|---|---|---|---|---|
| **Protein Accession Number** | **Protection/Organism/Length** | **NOV8a Residues/Match Residues** | **Identities/ Similarities for the Matched Portion** | **Expect Value** |
| Q8VHW5 | Voltage-dependent calcium channel gamma-8 subunit (Neuronal voltage- gated calcium channel gamma-8 subunit) - Rattus norvegicus (Rat), 421 aa. | 2..275 11..317 | 226/309(73%) 234/309 (75%) | e-113 |
| Q8WXS5 | Voltage-dependent calcium channel gamma-8 subunit (Neuronal voltage- gated calcium channel gamma-8 subunit) - Homo sapiens (Human), 425 aa. | 2..275 11..317 | 229/310 (73%) 236/310 (75%) | e-113 |
| Q8VHW2 | Voltage-dependent calcium channel gamma-8 subunit (Neuronal voltage- gated calcium channel gamma-8 subunit) - Mus musculus (Mouse), 423 aa. | 2..275 11..317 | 225/309 (72%) 234/309 (74%) | e-113 |
| Q9Y698 | Voltage-dependent calcium channel gamma-2 subunit (Neuronal voltage- gated calcium channel gamma-2 subunit) - Homo sapiens (Human), 323 aa. | 7..323 5..323 | 183/327 (55%) 233/327 (70%) | 4e-92 |
| O88602 | Voltage-dependent calcium channel gamma-2 subunit (Neuronal voltage- gated calcium channel gamma-2 subunit) (Stargazin) - Mus musculus (Mouse), 323 aa. | 7..323 5..323 | 183/327 (55%) 232/327 (69%) | 5e-92 |

PFam analysis predicts that the NOV8a protein contains the domains shown in the Table 8E.

| **Table 8E. Domain Analysis of NOV8a** | | | |
|---|---|---|---|
| **Pfam Domain** | **NOV8a Match Region** | **Identities/ Similarities for the Matched Region** | **Expect Value** |
| PMP22_Claudin | 8..198 | 51/198 (26%) 161/198 (81%) | 7.6e-53 |

### Example 9.

The NOV9 clone was analyzed, and the nucleotide and encoded polypeptide sequences are shown in Table 9A.

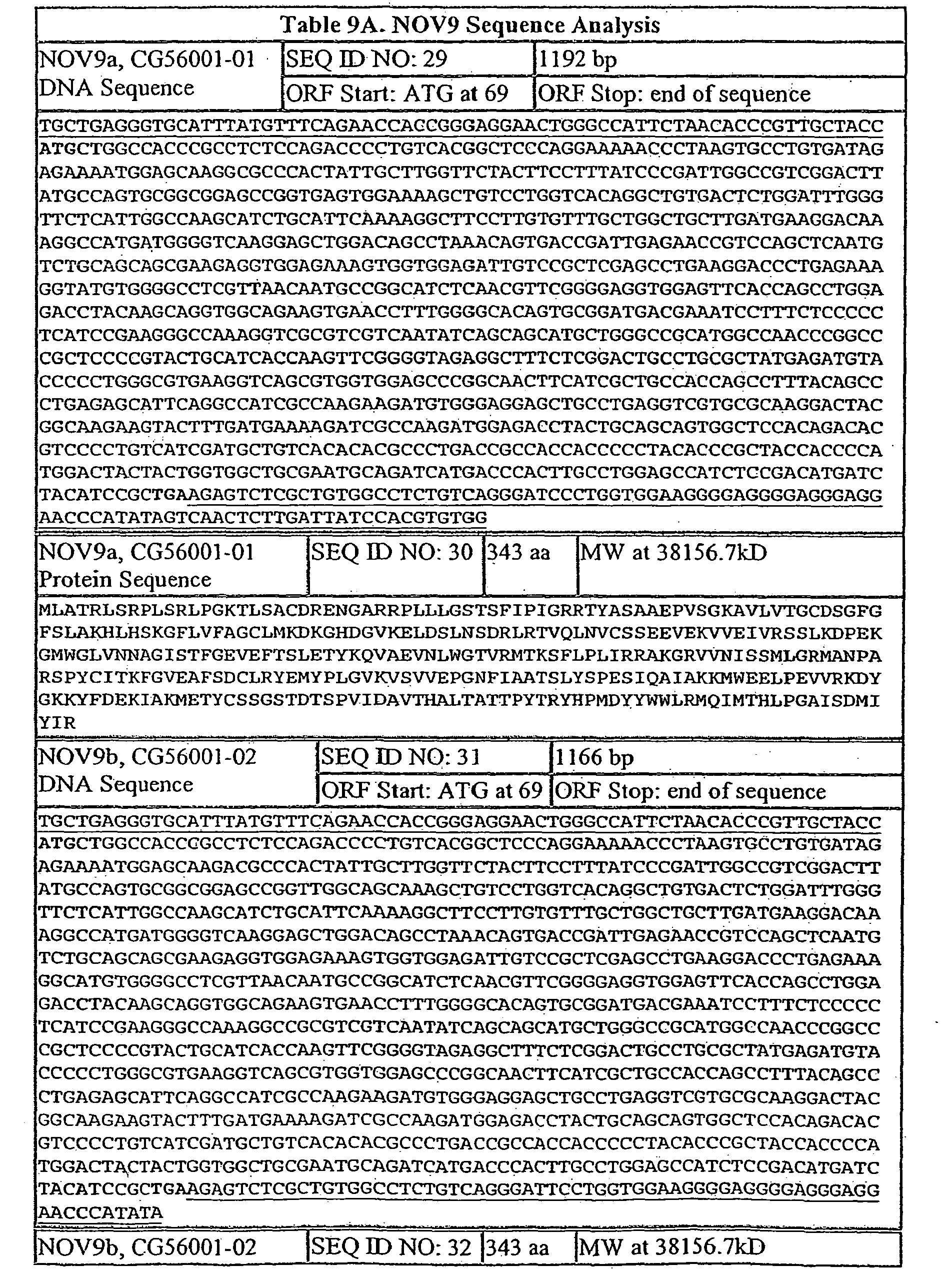

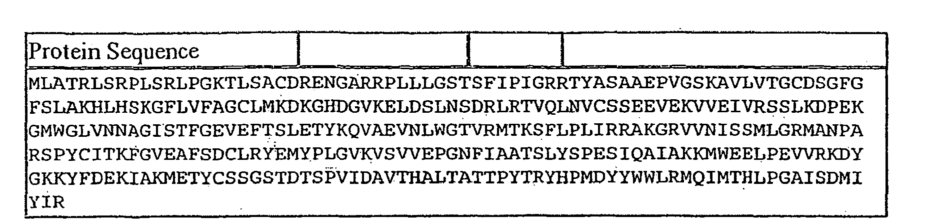

A ClustalW comparison of the above protein sequences yields the following sequence alignment shown in Table 9B.

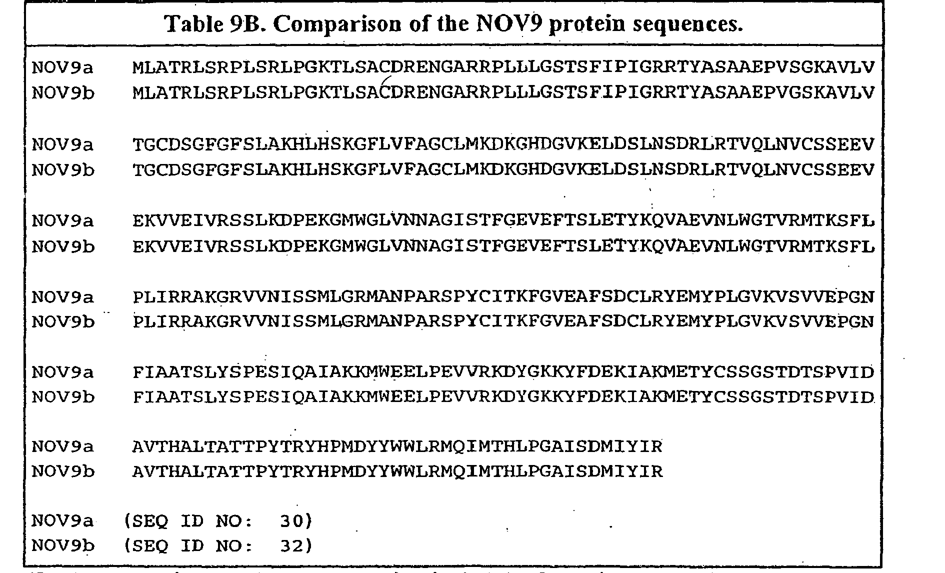

Further analysis of the NOV9a protein yielded the following properties shown in Table 9C.

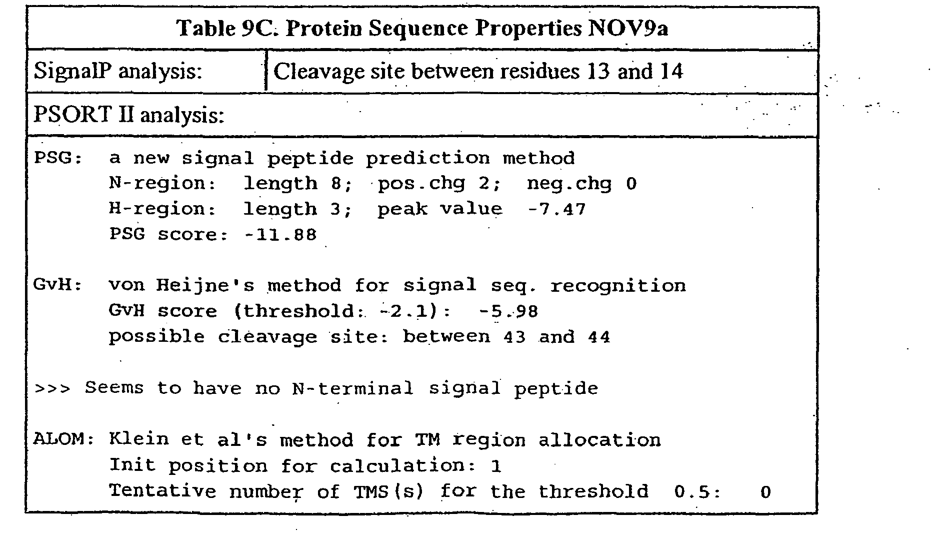

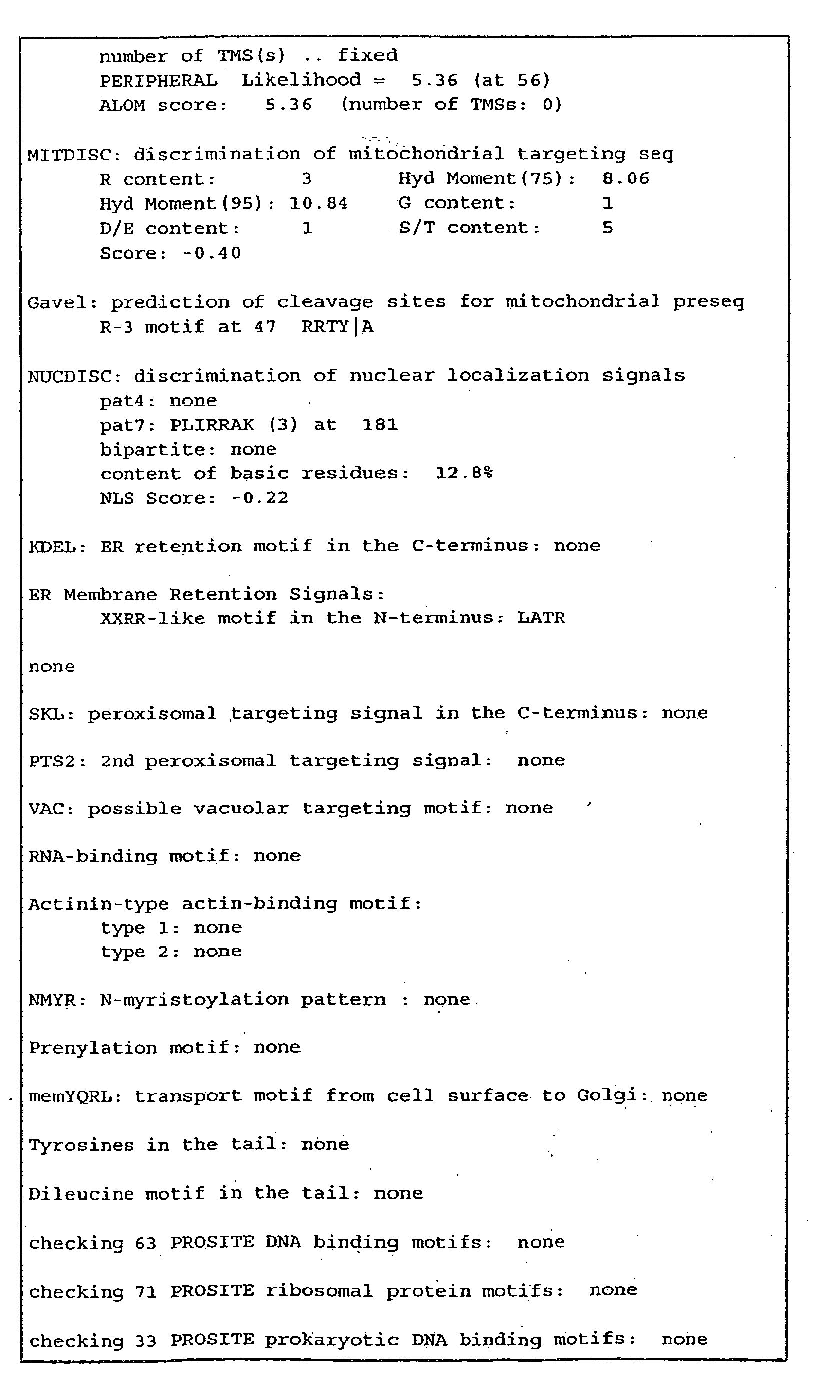

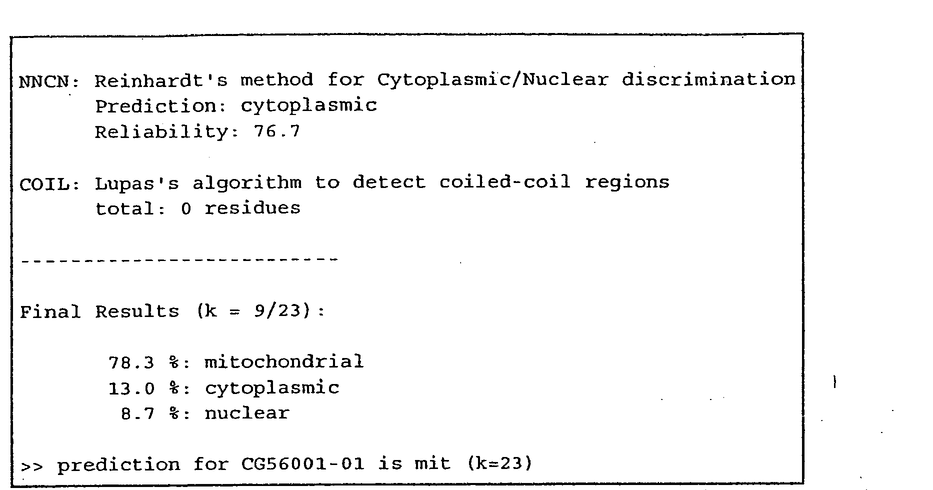

A search of the NOV9a protein against the Geneseq database, a proprietary database that contains sequences published in patents and patent publication, yielded several homologous proteins shown in Table 9D.

| **Table 9D. Geneseq Results for NOV9a** | | | | |
|---|---|---|---|---|
| **Geneseq Identifier** | **Protein/Organism/Length [Patent #, Date]** | **NOV9a Residues/Match Residues** | **Identities/Similarities for the Matched Region** | **Expect Value** |
| ABP53584 | Human NOV14a protein SEQ ID NO:32 - Homo sapiens, 343 aa. [WO200262999-A2, 15-AUG-2002] | 1..343 1..343 | 343/343 (100%) 343/343 (100%) | 0.0 |
| ABP53584 | Human NOV14a protein SEQ ID NO:32 - Homo sapiens, 343 aa. [WO200262999-A2, 15-AUG-2002] | 1..343 1..343 | 343/343 (100%) 343/343 (100%) | 0.0 |
| ABP53585 | Human NOV14b protein SEQ ID NO:34 - Homo sapiens, 343 aa. [WO200262999-A2, 15-AUG-2002] | 1..343 1..343 | 341/343 (99%) 341/343 (99%) | 0.0 |
| ABP53585 | Human NOV14b protein SEQ ID NO:34 - Homo sapiens, 343 aa. [WO200262999-A2, 15-AUG-2002] | 1..343 1..343 | 341/343 (99%) 341/343 (99%) | 0.0 |
| AAM78804 | Human protein SEQ ID NO 1466 - Homo sapiens, 343 aa. [WO200157190-A2, 09-AUG-2001] | 1..343 1..343 | 341/343 (99%) 341/343 (99%) | 0.0 |

In a BLAST search of public sequence databases, the NOV9a protein was found to have homology to the proteins shown in the BLASTP data in Table 9E.

| **Table 9E. Public BLASTP Results for NOV9a** | | | | |
|---|---|---|---|---|
| **Protein Accession Number** | **Protein/Organism/Length** | **NOV9a Residues/ Match Residues** | **Identities/ Similarities for the Matched Portion** | **Expect Value** |
| Q02338 | D-beta-hydroxybutyrate dehydrogenase, mitochondrial precursor (EC 1.1.1.30) (BDH) (3-hydroxybutyrate dehydrogenase) - Homo sapiens (Human), 343 aa. | 1..343 1..343 | 341/343 (99%) 341/343 (99%) | 0.0 |
| A42845 | 3-hydroxybutyrate dehydrogenase (EC 1.1.1.30) - human, 343 aa (fragment). | 11..343 11..343 | 319/333 (95%) 321/333 (95%) | 0.0 |
| P29147 | D-beta-hydroxybutyrate dehydrogenase, mitochondrial precursor (EC 1.1.1.30) (BDH) (3-hydroxybutyrate dehydrogenase) - Rattus norvegicus (Rat), 344 aa. | 1..342 2..343 | 297/342 (86%) 313/342 (90%) | e-174 |
| AAH43683 | Hypothetical protein - Mus musculus (Mouse), 343 aa. | 1..342 1..342 | 297/342 (86%) 313/342 (90%) | e-174 |
| Q8BK53 | D-beta-hydroxybutyrate dehydrogenase - Mus musculus (Mouse), 343 aa. | 1..342 1..342 | 295/342 (86%) 312/342 (90%) | e-173 |

PFam analysis predicts that the NOV9a protein contains the domains shown in the Table 9F.

| **Table 9F. Domain Analysis of NOV9a** | | | |
|---|---|---|---|
| **Pfam Domain** | **NOV9a Match Region** | **Identities/ Similarities for the Matched Region** | **Expect Value** |
| adh_short | 54..336 | 80/291 (27%) 235/291(81%) | 7.9e-79 |

### Example 10.

The NOV10 clone was analyzed, and the nucleotide and encoded polypeptide sequences are shown in Table 10A.

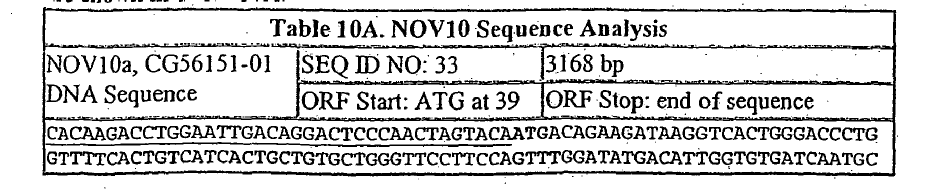

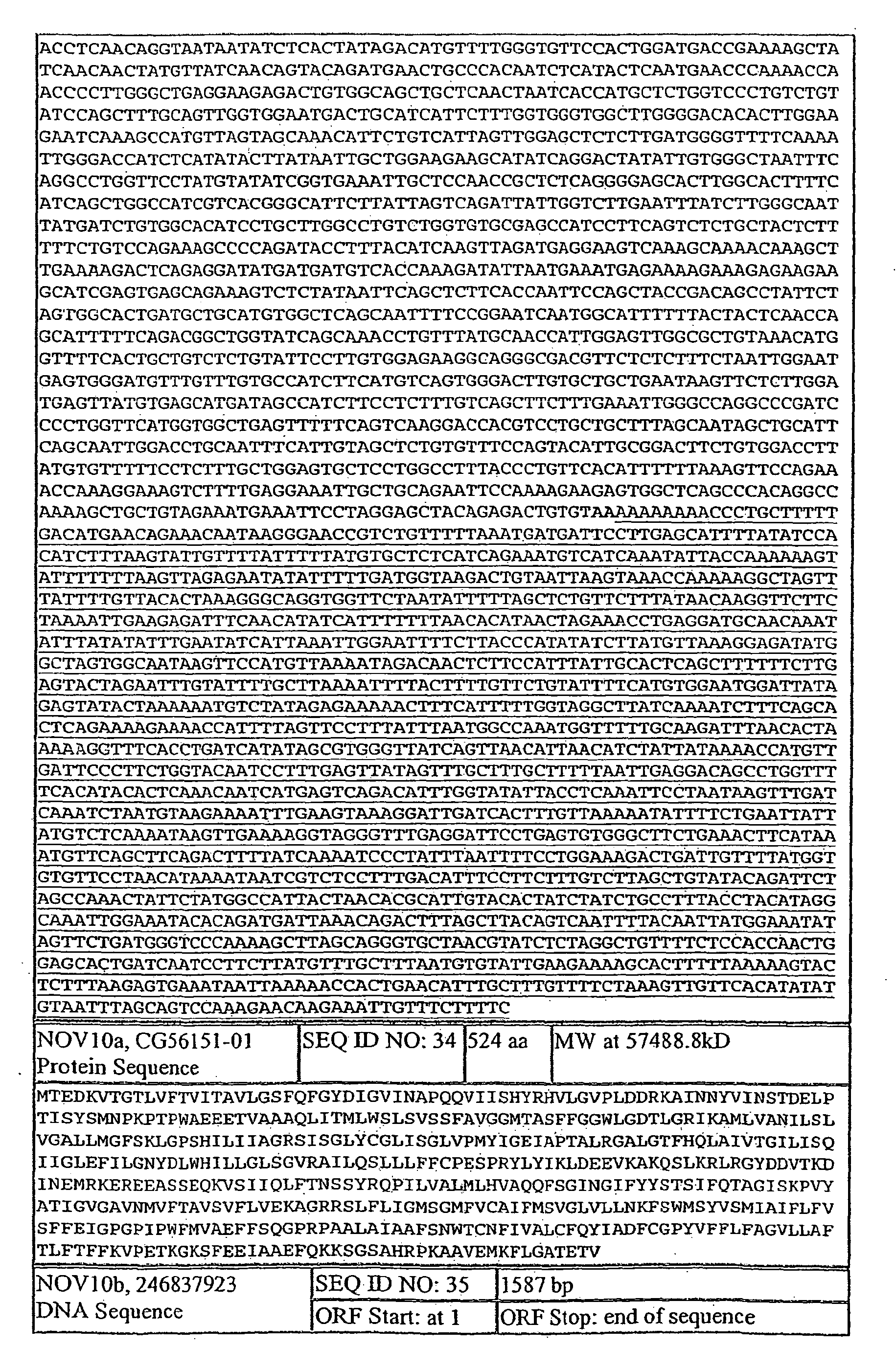

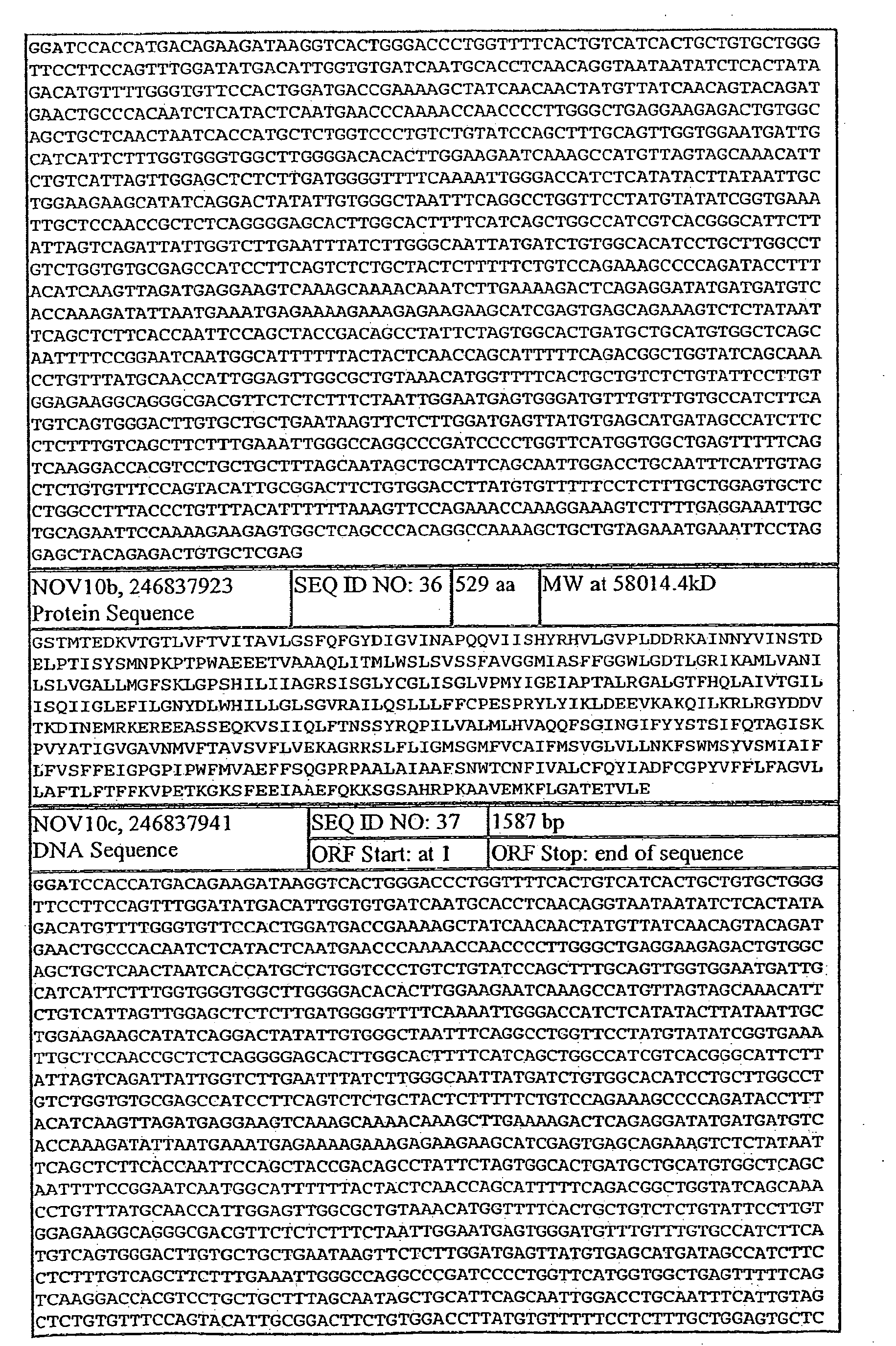

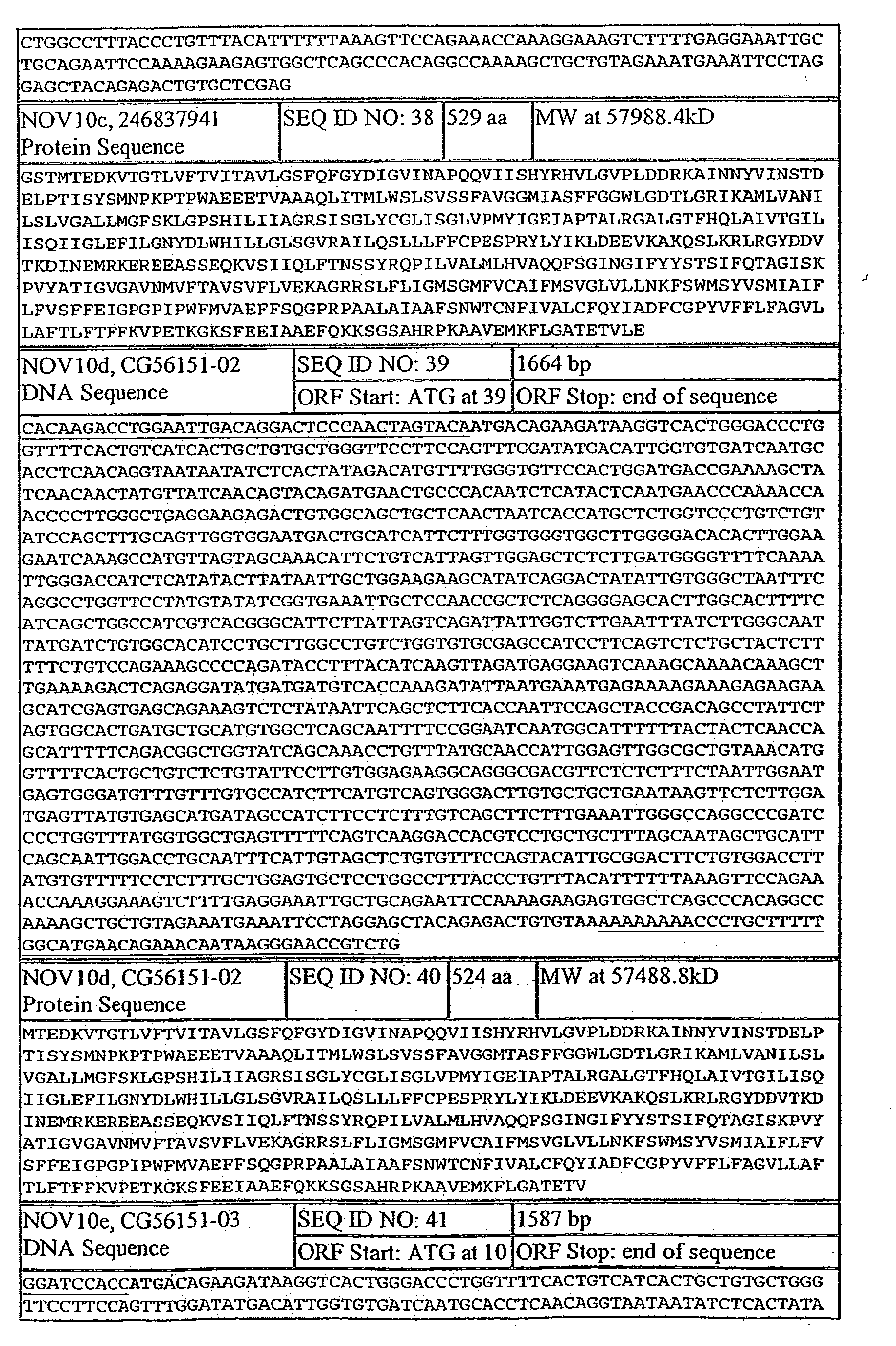

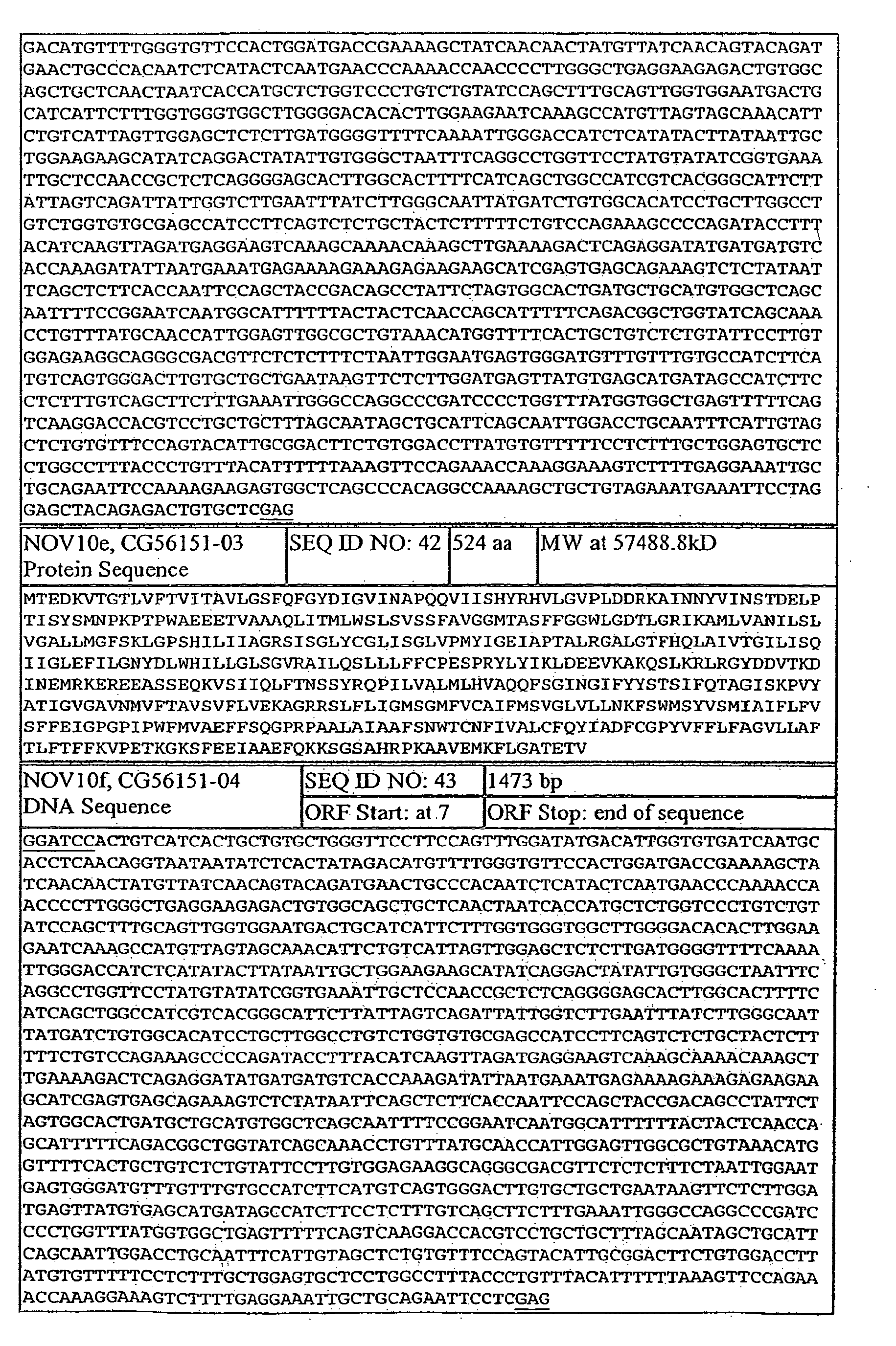

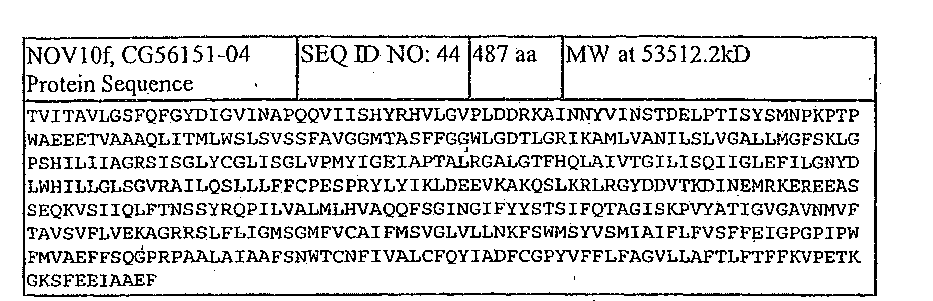

A ClustalW comparison of the above protein sequences yields the following sequence alignment shown in Table 10B.

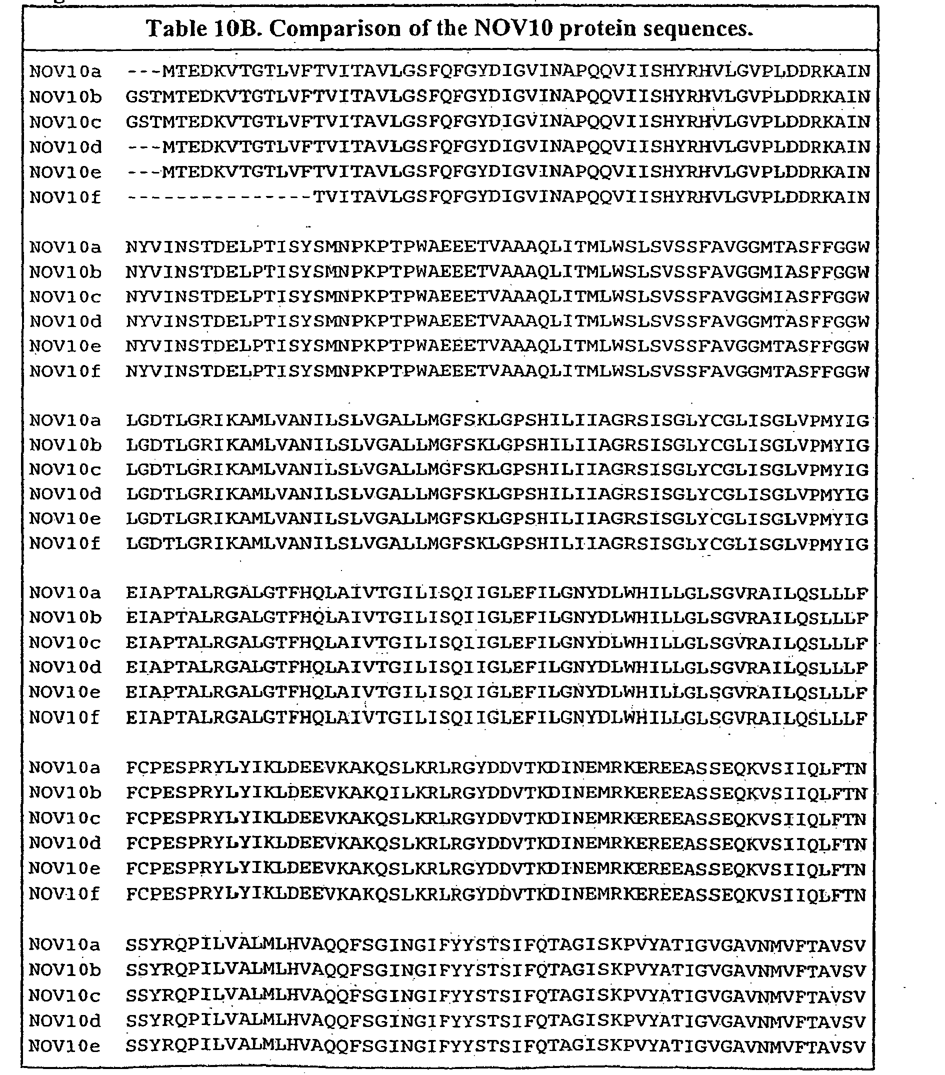

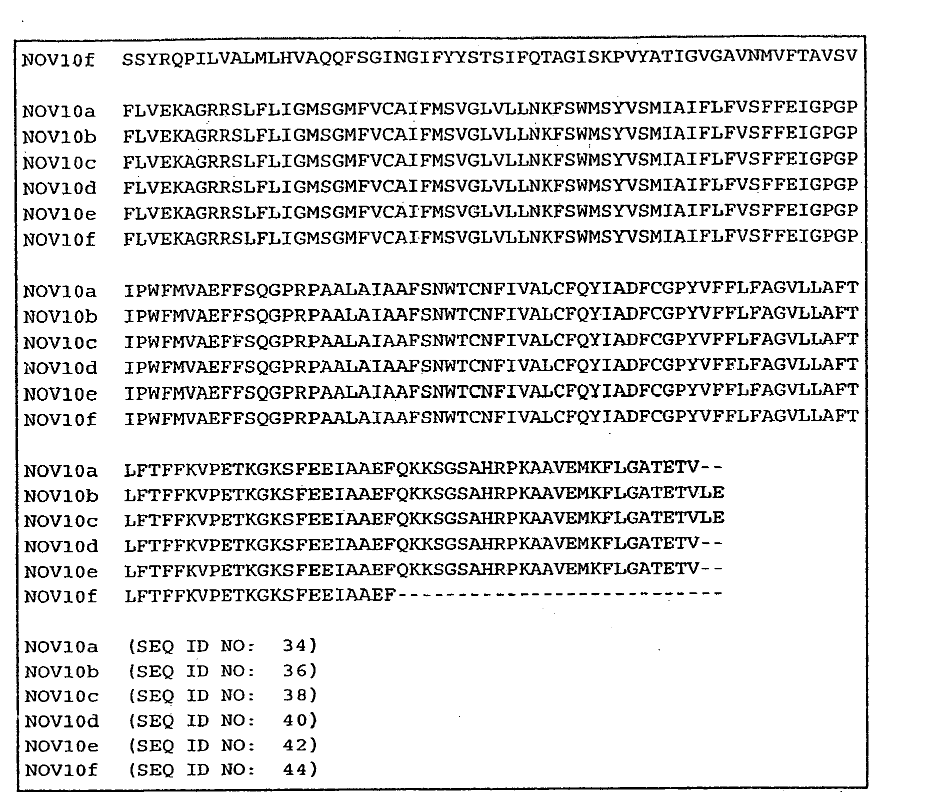

Further analysis of the NOV10a protein yielded the following properties shown in Table 10C.

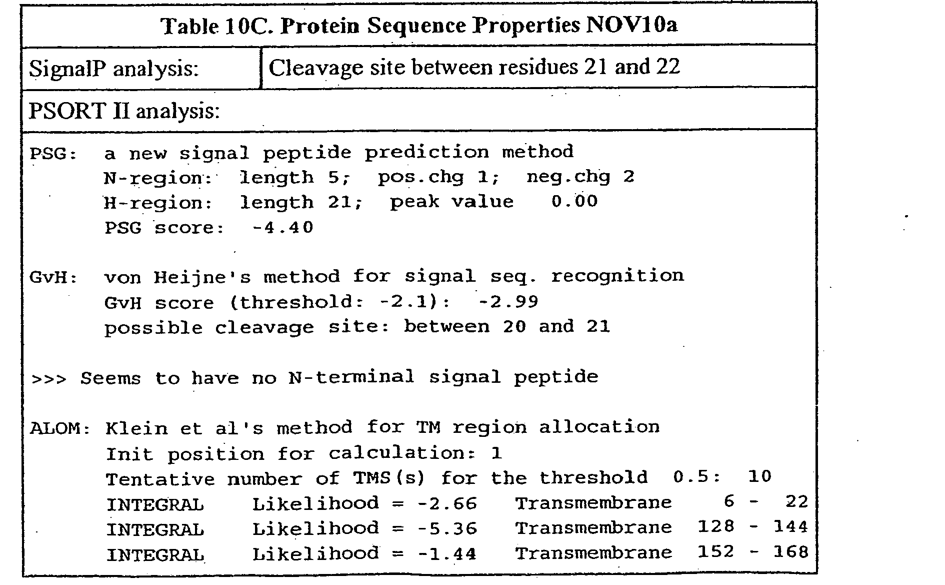

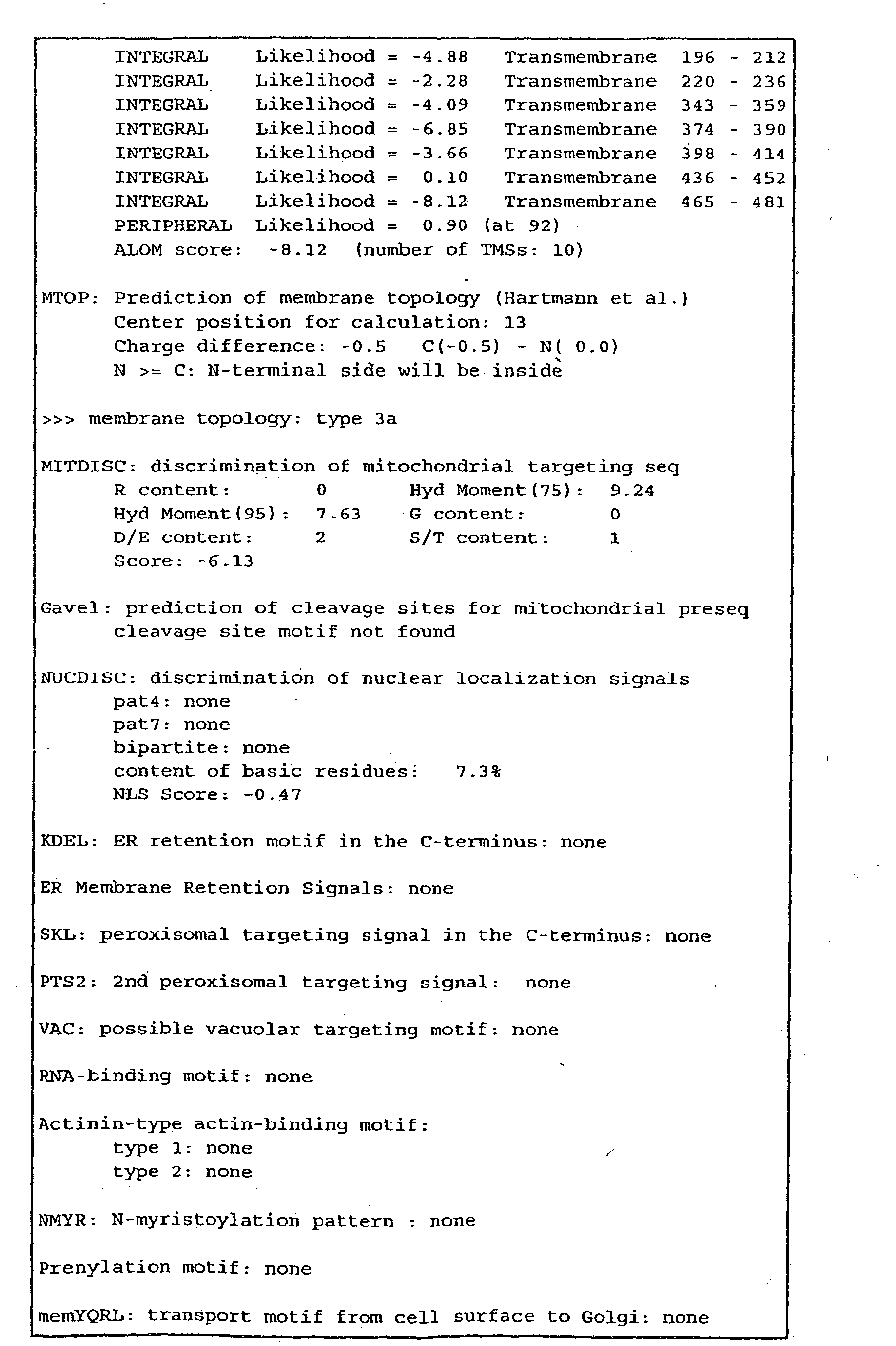

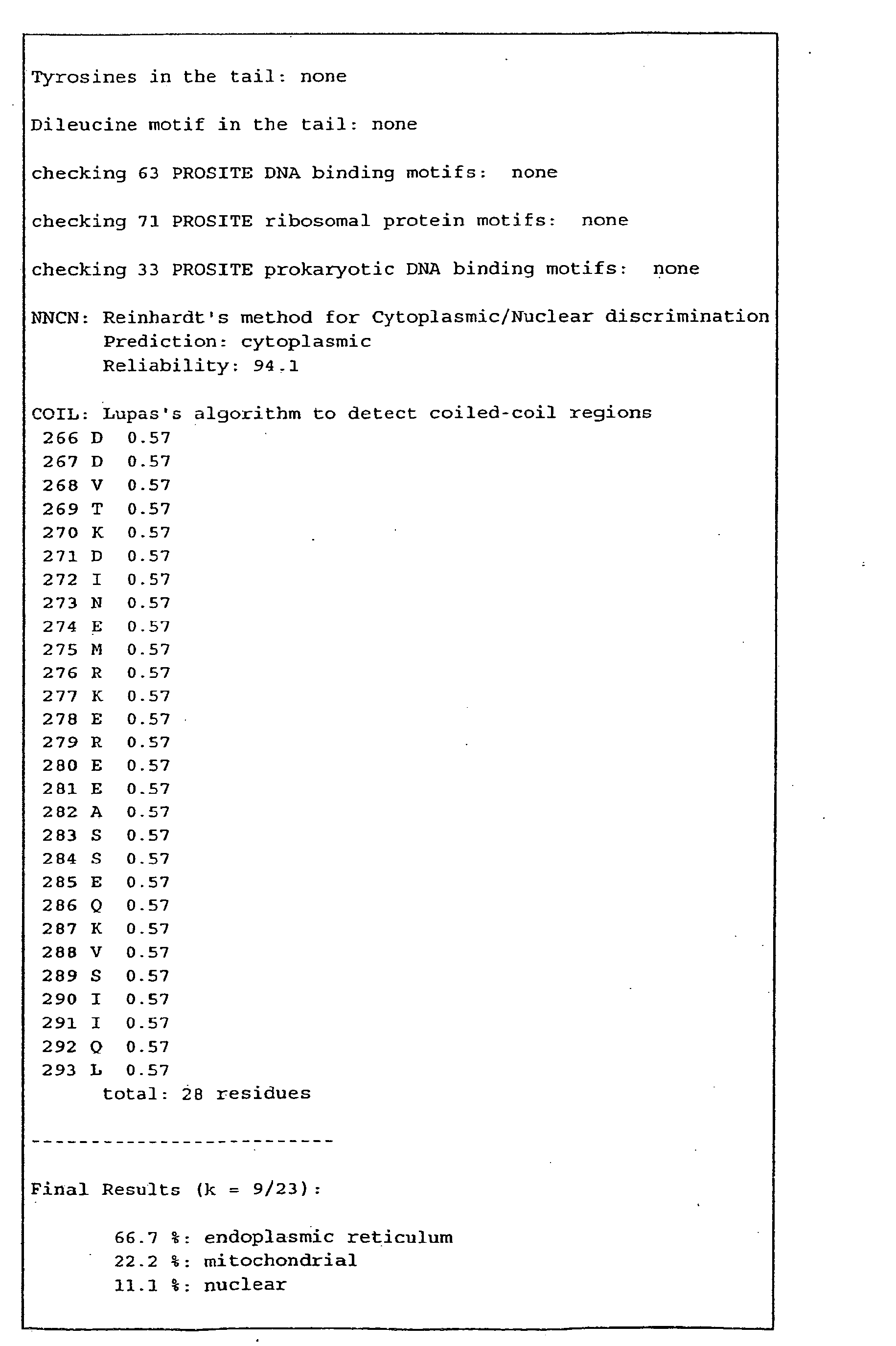

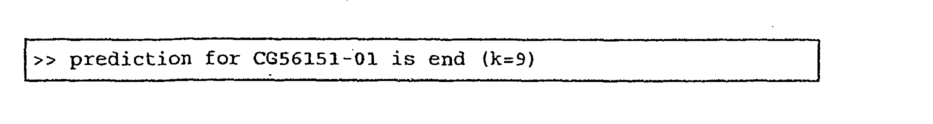

A search of the NOV10a protein against the Geneseq database, a proprietary database that contains sequences published in patents and patent publication, yielded several homologous proteins shown in Table 10D.

| **Table 10D. Geneseq Results for NOV10a** | | | | |
|---|---|---|---|---|
| **Geneseq Identifier** | **Protein/Organism/Length [Patent #, Date]** | **NOV10a Residues/Match Residues** | **Identities/Similarities for the Matched Region** | **Expect Value** |
| ABB05602 | Human glucose transporter protein SEQ ID NO:12-Homo sapiens, 524 aa. [US6323002-B1, 27-NOV-2001] | 1..524 1..524 | 524/524(100%) 524/524 (100%) | 0.0 |
| ABG76953 | Human protein, homologous to glucose transporter type 2, designated NOV5 - Homo sapiens, 524 aa. [WO200255705- A2, 18-JUL-2002] | 1..524 1..524 | 524/524 (100%) 524/524 (100%) | 0.0 |
| AAW17836 | Rat glucose transporter GLUT-2 - Rattus sp, 522 aa. [WO9715668-A2, 01-MAY-1997] | 1..524 1..522 | 427/524 (81%) 469/524 (89%) | 0.0 |
| AAY27288 | Glucose transporter protein GLUT2 - Homo sapiens, 534 aa. [US5942398-A, 24-AUG-1999] | 3..502 10..515 | 333/507 (65%) 407/507 (79%) | 0.0 |
| AAB30522 | Amino acid sequence of a consensus GLUT polypeptide - Synthetic, 493 aa. [US6136547-A, 24-OCT-2000] | 6..514 10..483 | 288/509(56%) 372/509(72%) | e-162 |

In a BLAST search of public sequence databases, the NOV10a protein was found to have homology to the proteins shown in the BLASTP data in Table 10E.

| **Table 10E. Public BLASTP Results for NOV10a** | | | | |
|---|---|---|---|---|
| **Protein Accession Number** | **Protein/Organisni/Length** | **NOV10a Residues/ Match Residues** | **Identities/ Similarities for the Matched Portion** | **Expect Value** |
| P11168 | Solute carrier family 2, facilitated glucose transporter, member 2 (Glucose transporter type 2, liver) - Homo sapiens (Human), 524 aa. | 1..524 1..524 | 524/524 (100%) 524/524 (100%) | 0.0 |
| P14246 | Solute carrier family 2, facilitated glucose transporter, member 2 (Glucose transporter type 2, liver) - Mus musculus (Mouse), 523 aa. | 1..524 1..523 | 429/524(81%) 471/524 (89%) | 0.0 |
| P12336 | Solute carrier family 2, facilitated glucose transporter, member 2 (Glucose transporter type 2, liver) - Rattus norvegicus (Rat), 522 aa. | 1..524 1..522 | 427/524 (81%) 469/524 (89%) | 0.0 |
| S06920 | glucose transport protein, hepatic - mouse, 523 aa. | 1..524 1..523 | 426/524 (81%) 468/524 (89%) | 0.0 |
| Q90592 | Solute carrier family 2, facilitated glucose transporter, member 2 (Glucose transporter type 2, liver) - Gallus gallus (Chicken), 533 aa. | 3..502 10..514 | 333/506(65%) 407/506 (79%) | 0.0 |

PFam analysis predicts that the NOV10a protein contains the domains shown in the Table 10F.

| **Table 10F. Domain Analysis of NOV10a** | | | |
|---|---|---|---|
| **Pfam Domain** | **NOV10a Match Region** | **Identities/ Similarities** for the Matched Region | **Expect Value** |
| OATP_C | 10..394 | 70/474 (15%) 251/474 (53%) | 0.2 |
| sugar_tr | 13..499 | 208/521 (40%) 436/521 (84%) | 1.4e-201 |

### Example 11.

The NOV11 clone was analyzed, and the nucleotide and encoded polypeptide sequences are shown in Table 11A.

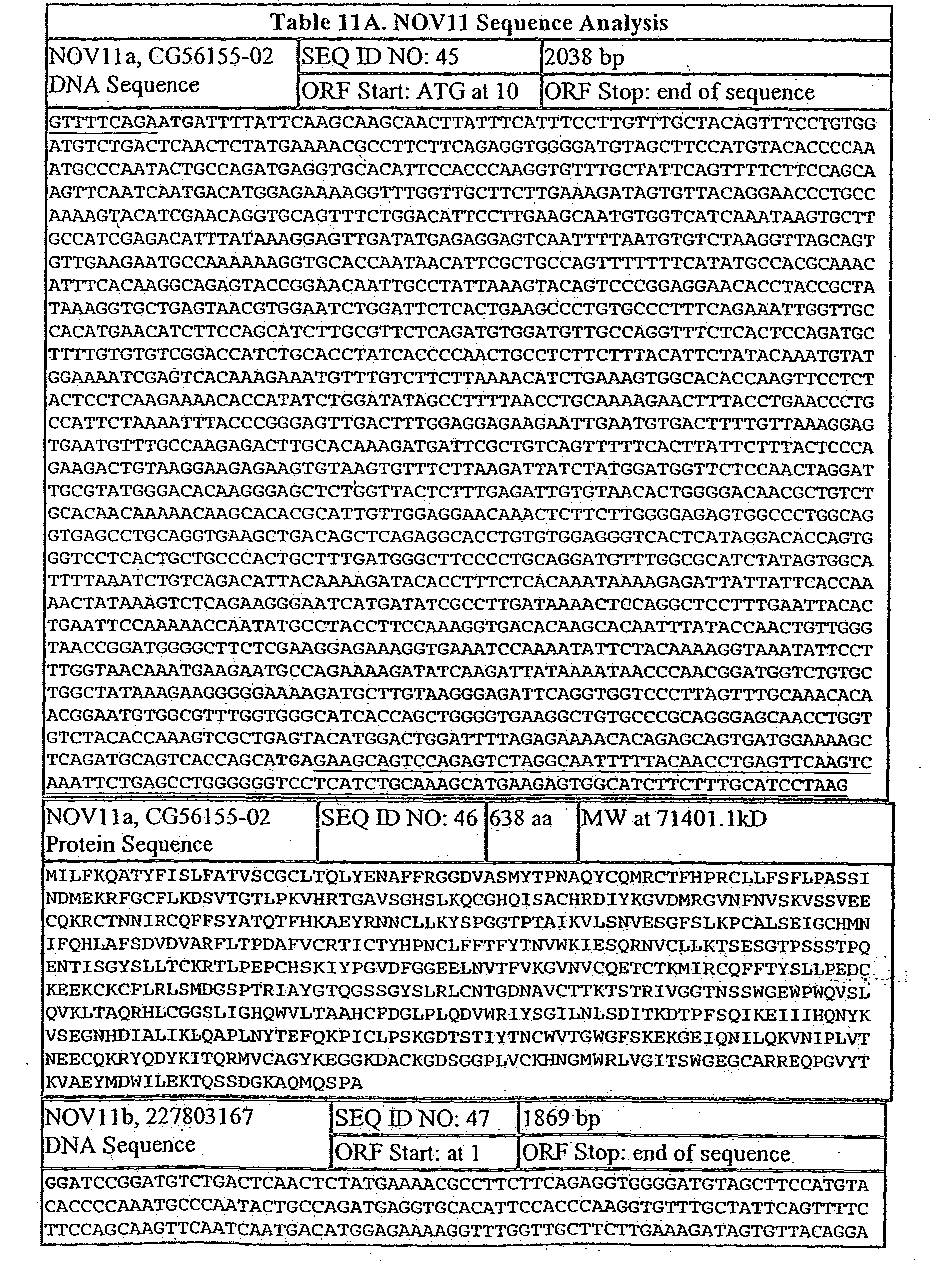

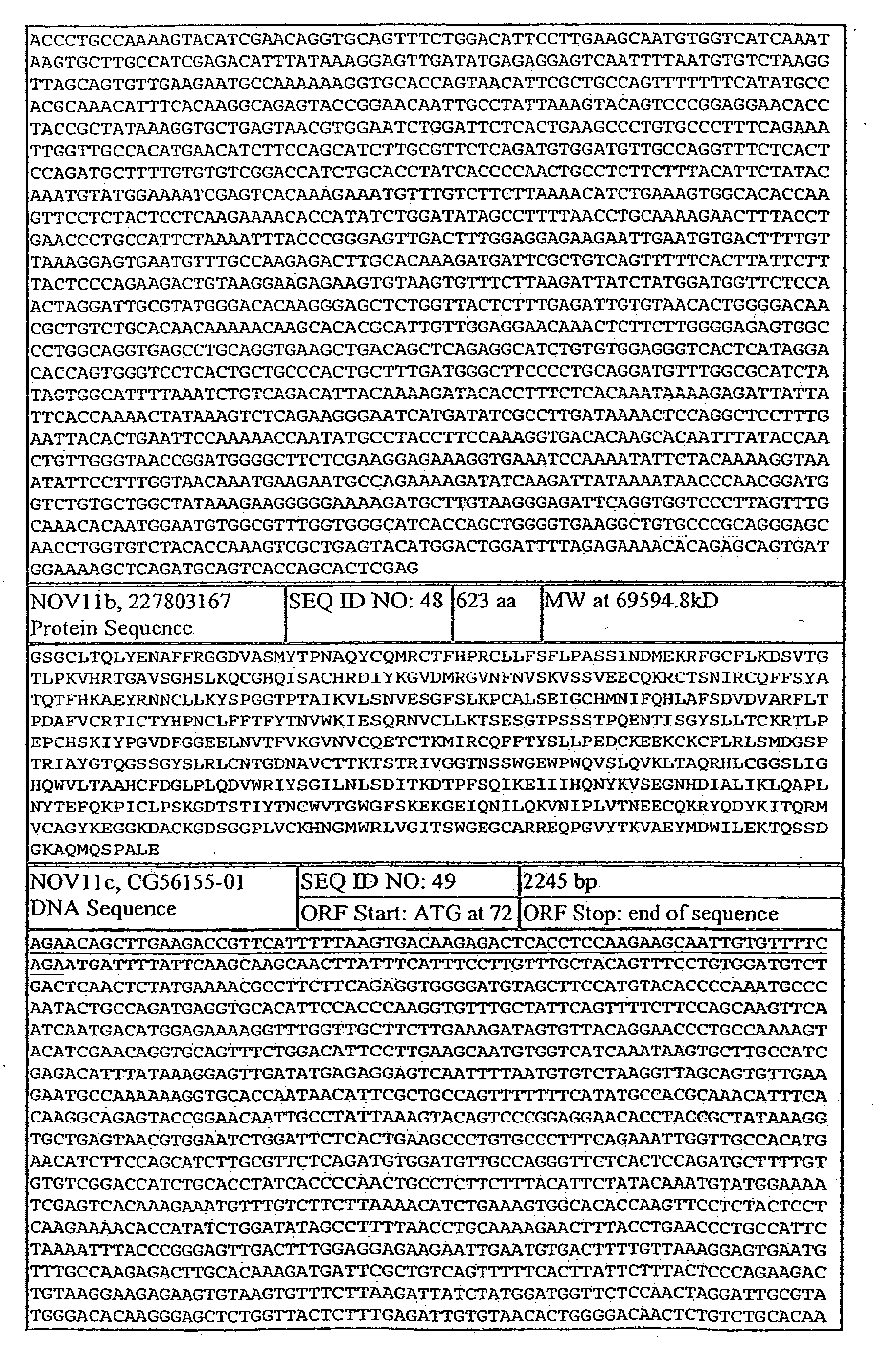

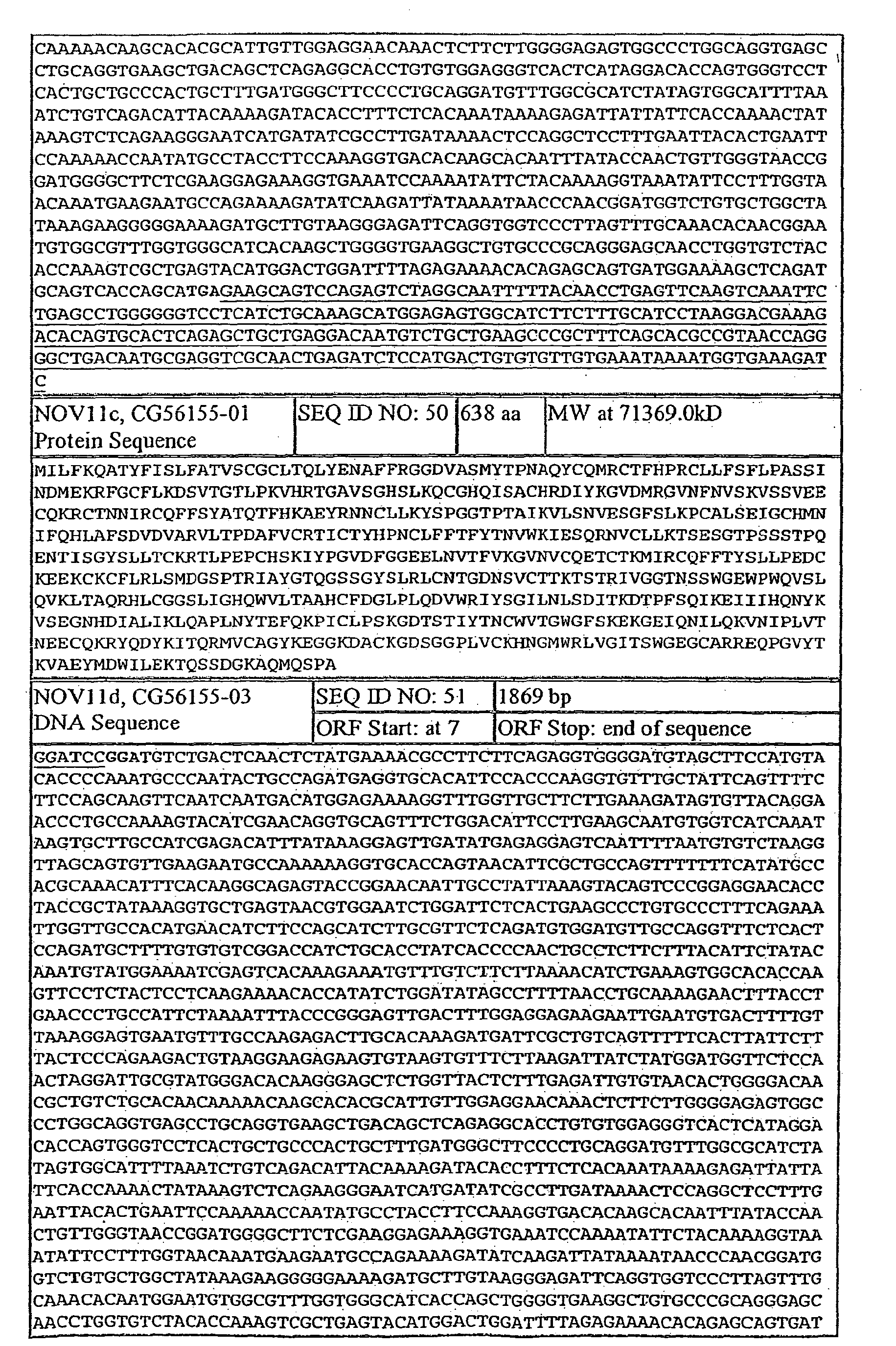

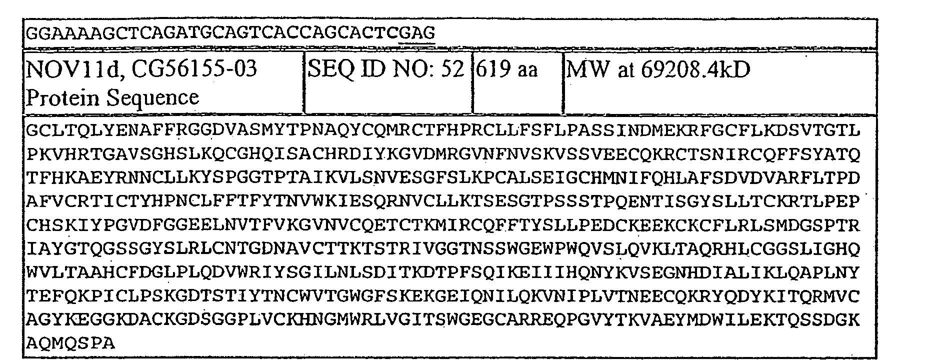

A ClustalW comparison of the above protein sequences yields the following sequence alignment shown in Table 11B.

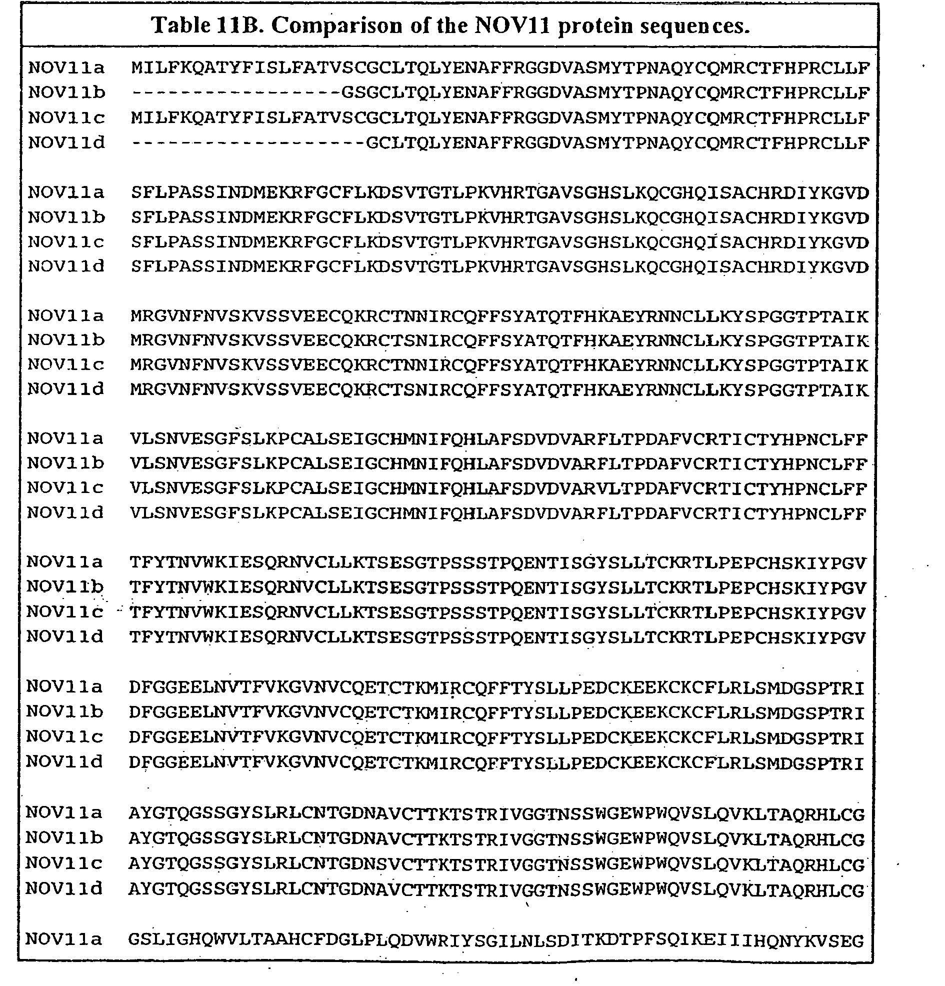

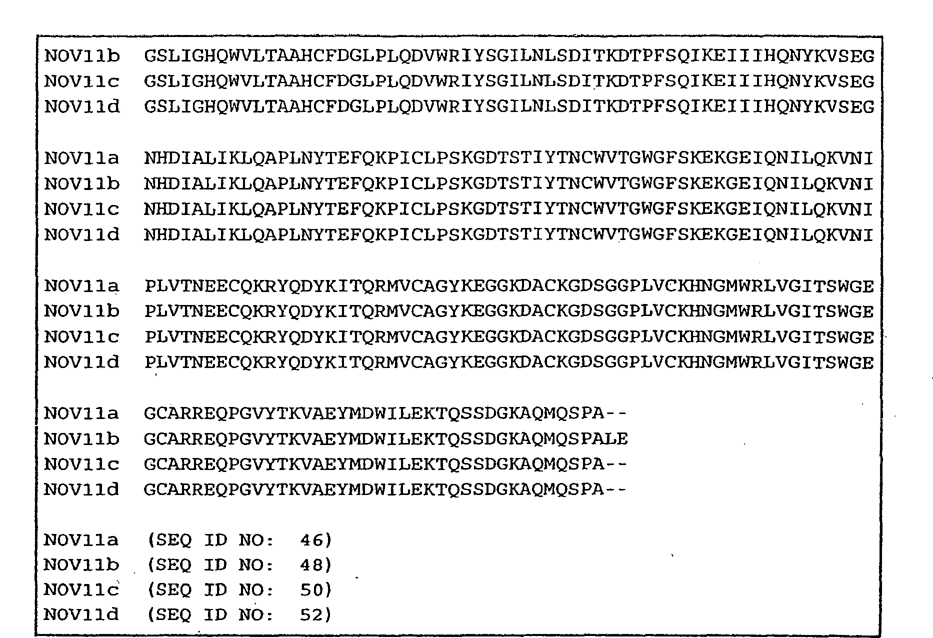

Further analysis of the NOV11a protein yielded the following properties shown in Table 11C.

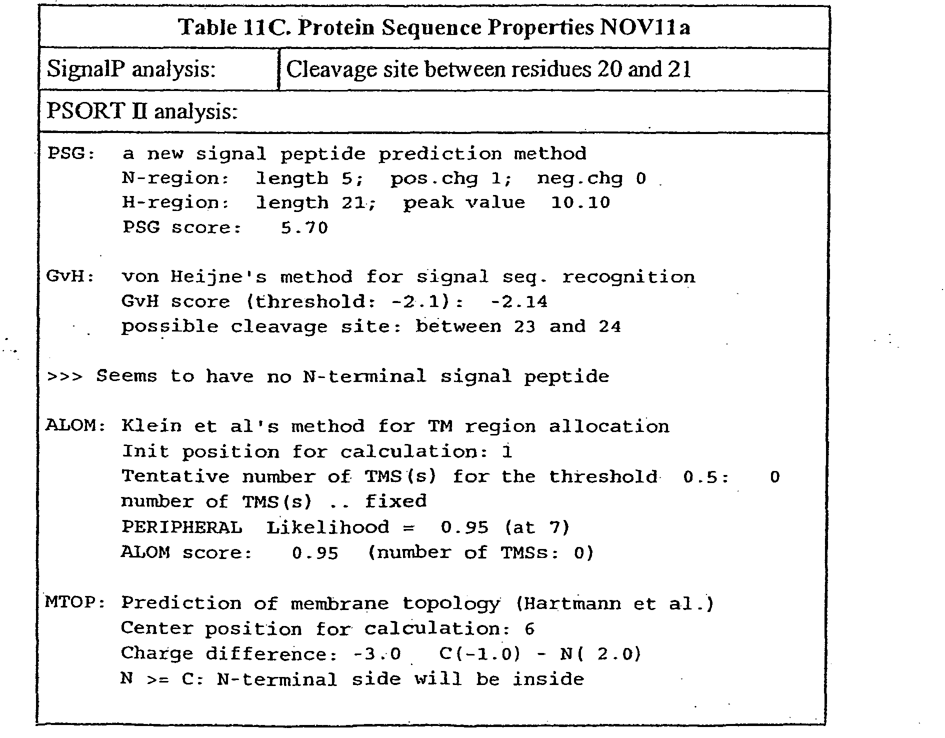

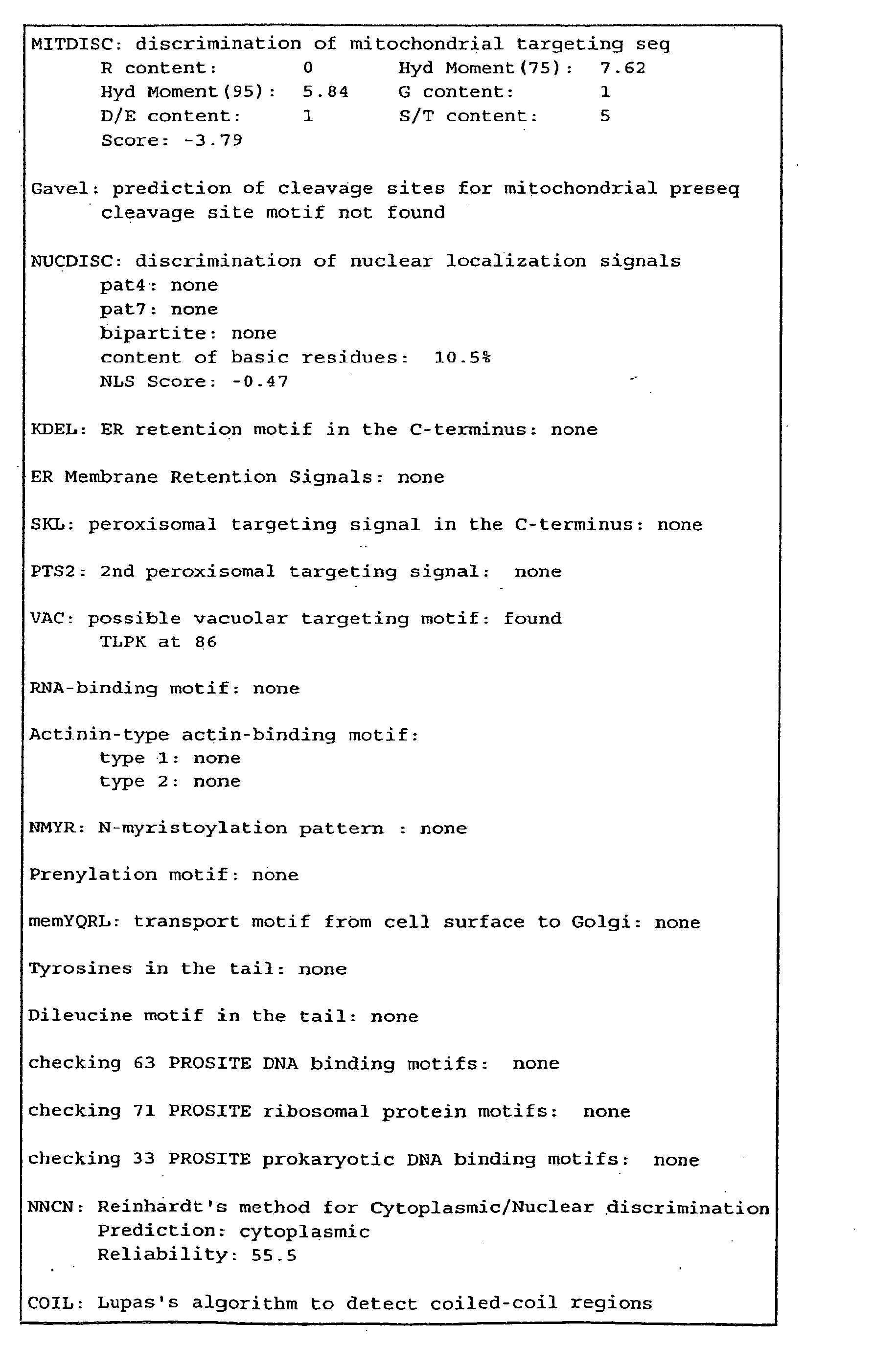

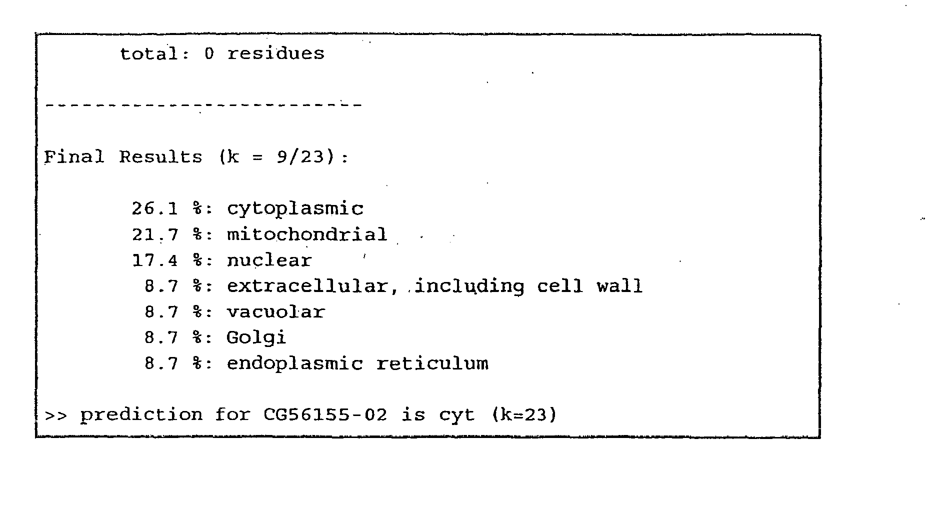

A search of the NOV11a protein against the Geneseq database, a proprietary database that contains sequences published in patents and patent publication, yielded several homologous proteins shown in Table 11D.

| **Table 11D. Geneseq Results for NOV11a** | | | | |
|---|---|---|---|---|
| **Geneseq Identifier** | **Protein/Organism/Length [Patent #, Date]** | **NOV11a Residues/Match Residues** | **Identities/ Similarities for the Matched Region** | **Expect Value** |
| ABG76951 | Human protein, homologous to Kallikrein, designated NOV3 - Homo sapiens, 638 aa. [WO200255705-A2, 18-JUL-2002] | 1..638 1..638 | 638/638 (100%) 638/638 (100%) | 0.0 |
| AAU68928 | Human protease domian of kallikrein I-Homo sapiens, 158 aa. [US6294663-B1, 25-SEP-2001] | 427..584 1..158 | 158/158 (100%) 158/158(100%) | 1e-92 |
| AAU82755 | Amino acid sequence of novel human protease #54 - Homo sapiens, 802 aa. [WO200200860-A2, 03-JAN-2002] | 319..621 513..797 | 115/306 (37%) 172/306 (55%) | 1e-56 |
| AAB240S2 | Human PRO618 protein sequence SEQ ID NO:24 - Homo sapiens, 802 aa. [WO200053754-A1, 14-SEP-2000] | 319..621 513..797 | 115/306(37%) 172/306 (55%) | 1e-56 |
| AAB44266 | Human PRO618 (UNQ354) protein sequence SEQ ID NO:169 - Homo sapiens, 802 aa. [WO200053756-A2, 14-SEP-2000] | 319..621 513..797 | 115/306(37%) 172/306(55%) | 1e-56 |

In a BLAST search of public sequence databases, the NOV11a protein was found to have homology to the proteins shown in the BLASTP data in Table 11E.

| **Table 11E. Public BLASTP Results for NOV11a** | | | | |
|---|---|---|---|---|
| **Protein Accession Number** | **Protein/Organism/Length** | **NOV11a Residues/ Match Residues** | **Identities/ Similarities for the Matched Portion** | **Expect Value** |
| P03952 | Plasma kallikrein precursor (EC 3.4.21.34) (Plasma prekallikrein) (Kininogenin) (Fletcher factor) - Homo sapiens (Human), 638 aa. | 1..638 1..638 | 636/638 (99%) 637/638 (99%). | 0.0 |
| O97506 | Kallikrein - Sus scrofa (Pig), 643 aa. | 1.635 9..643 | 503/635 (79%) 568/635 (89%) | 0.0 |
| Q8R0P5 | Kallikrein B, plasma 1 - Mus musculus (Mouse), 638 aa. | 1..638 1..638 | 486/638 (76%) 554/638 (86%) | 0.0 |
| P26262 | Plasma kallikrein precursor (EC 3.4.21.34) (Plasma prekallikrein) (Kininogenin) (Fletcher factor) - Mus musculus (Mouse), 638 aa. | 1..638 1..638 | 485/638 (76%) 553/638 (86%) | 0.0 |
| P14272 | Plasma kallikrein precursor (EC 3.4.21.34) (Plasma prekallikrein) (Kininogenin) (Fletcher factor) - Rattus norvegicus (Rat), 638 aa. | 1..638 1..638 | 476/638 (74%) 549/638 (85%) | 0.0 |

PFam analysis predicts that the NOV11a protein contains the domains shown in the Table 11F.

| **Table 11F. Domain Analysis of NOV11a** | | | |
|---|---|---|---|
| **Pfam Domain** | **NOV11a Match Region** | **Identities/Similarities for the Matched Region** | **Expect Value** |
| PAN | 21..104 | 19/112 (17%) 64/112 (57%) | 7.9e-14 |
| PAN | 111..194 | 23/111 (21%) 67/111 (60%) | 4.8e-15 |
| PAN | 201..284 | 19/111 (17%) 62/111 (56%) | 4e-10 |
| PAN | 292..375 | 22/H1(20%) 65/111 (59%) | 2.3e-09 |
| trypsin | 391..621 | 113/263(43%) 196/263(75%) | 5.2e-92 |

### Example 12.

The NOV12 clone was analyzed, and the nucleotide and encoded polypeptide sequences are shown in Table 12A.

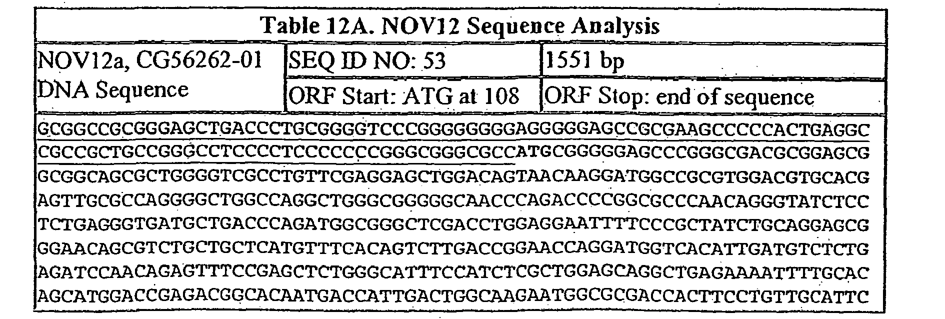

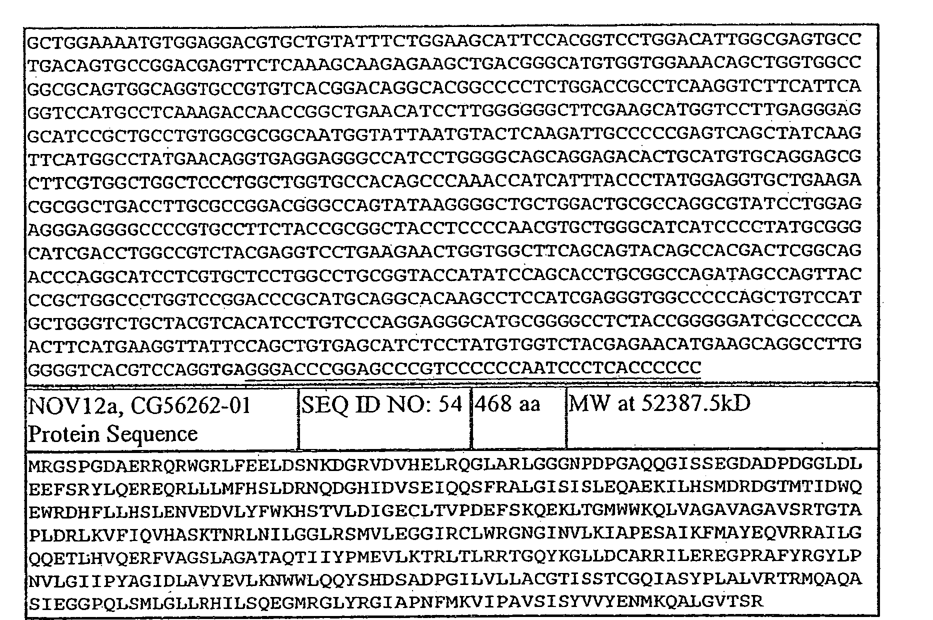

Further analysis of the NOV12a protein yielded the following properties shown in Table 12B.

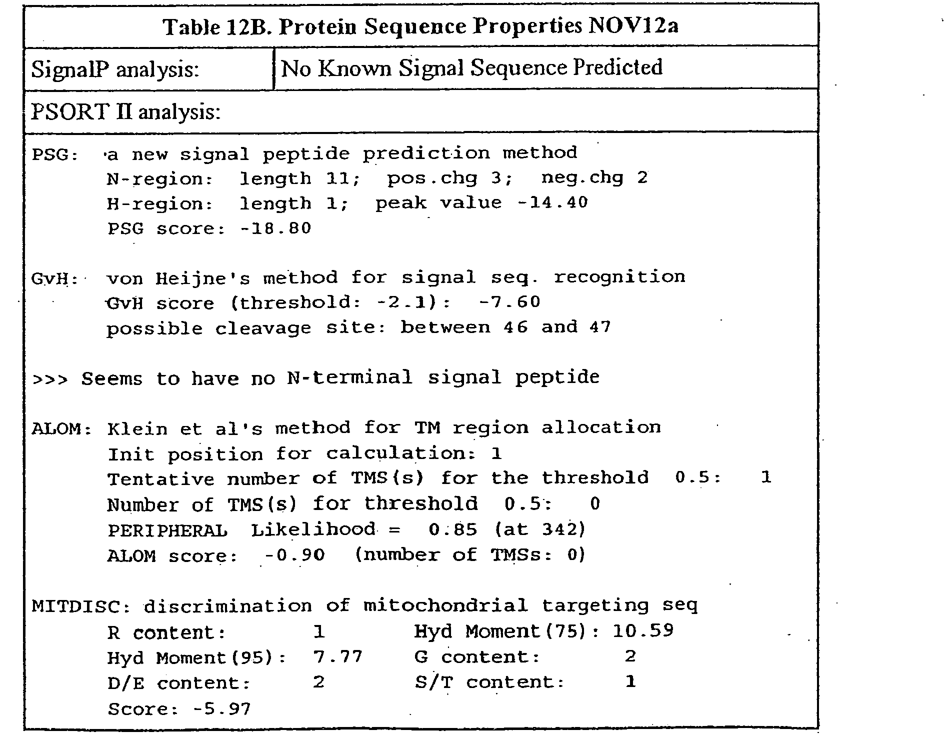

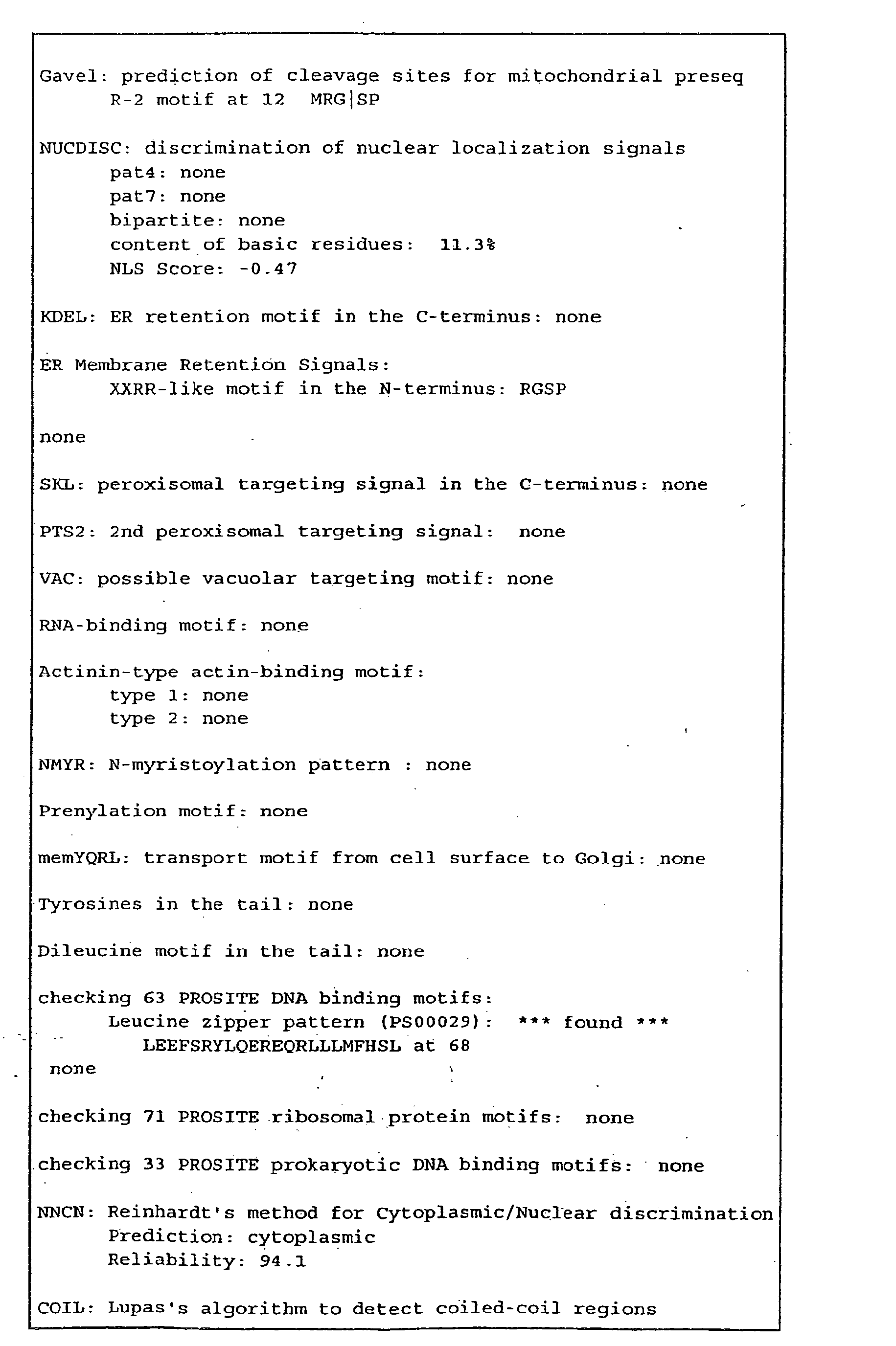

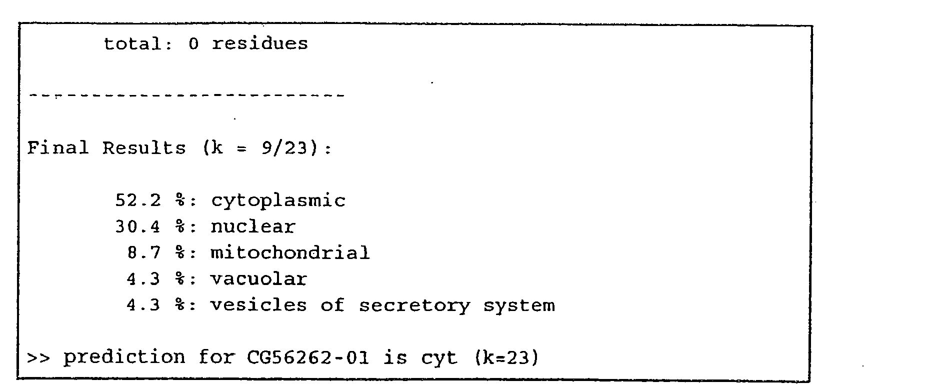

A search of the NOV12a protein against the Geneseq database, a proprietary database that contains sequences published in patents and patent publication, yielded several homologous proteins shown in Table 12C.

| **Table 12C. Geneseq Results for NOV12a** | | | | |
|---|---|---|---|---|
| **Geneseq Identifier** | **Protein/Organism/Length [Patent #, Date]** | **NOV12a Residues/Match Residues** | **Identities/Similarities for the Matched Region** | **Expect Value** |
| AAE22927 | Human transporter and ion channel (TRICH) 26 - Homo sapiens, 468 aa. [WO200222684-A2, 21-MAR-2002] | 1..468 1..468 | 463/468 (98%) 466/468 (98%) | 0.0 |
| AAU27869 | Human contig polypeptide sequence #22 - Homo sapiens, 509 aa. [WO200164834-A2, 07-SEP-2001] | 1..468 39..506 | 463/468 (98%) 466/468 (98%) | 0.0 |
| AAU27697 | Human full-length polypeptide sequence #22 - Homo sapiens, 471 aa. [WO200164834-A2, 07-SEP-2001] | 1..468 1..468 | 463/468 (98%) 466/468 (98%) | 0.0 |
| ABG22637 | Novel human diagnostic protein #22628 - Homo sapiens, 508 aa. [WO200175067-A2, 11-OCT-2001] | 1..468 39..508 | 442/470 (94%) 450/470 (95%) | 0.0 |
| ABG30434 | Human protein sequence #2 used for determining sequence of unknown gene - Homo sapiens, 456 aa. [JP2002176980-A, 25-JUN-2002] | 1..409 1..409 | 403/409 (98%) 406/409 (98%) | 0.0 |

In a BLAST search of public sequence databases, the NOV12a protein was found to have homology to the proteins shown in the BLASTP data in Table 12D.

| **Table 12D. Public BLASTP Results for NOV12a** | | | | |
|---|---|---|---|---|
| **Protein Accession Number** | **Protein/Organism/Length** | **NOV12a Residues/ Match Residues** | **Identities/ Similarities for the Matched Portion** | **Expect Value** |
| CAD55563 | Putative calcium binding transporter - Homo sapiens (Human), 438 aa. | 1..407 1..407 | 402/407 (98%) 405/407 (98%) | 0.0 |
| Q96NQ4 | Hypothetical protein FLJ30339 - Homo sapiens (Human), 384 aa. | 85..468 1..384 | 379/384 (98%) 382/384 (98%) | 0.0 |
| CAD20531 | Sequence 1 from Patent WO0174854 - Homo sapiens (Human), 460 aa (fragment). | 1..407 1..406 | 374/407 (91%) 388/407 (94%) | 0.0 |
| AAH43834 | Similar to hypothetical protein MGC36388 - Xenopus laevis (African clawed frog), 514 aa. | 6..468 53..514 | 302/463 (65%) 368/463 (79%) | e-177 |
| Q8BHG0 | Weakly similar to peroxisomal CA-dependent solute carrier - Mus musculus (Mouse), 502 aa. | 6..468 41..502 | 305/463 (65%) 366/463 (78%) | e-176 |

PFam analysis predicts that the NOV12a protein contains the domains shown in the Table 12E.

| **Table 12E. Domain Analysis of NOV12a** | | | |
|---|---|---|---|
| **Pfam Domain** | **NOV12a Match Region** | **Identities/Similarities for the Matched Region** | **Expect Value** |
| efhand | 13..41 | 8/29 (28%) | 0.00022 |
| | | 26/29 (90%) | |
| efhand | 81..109 | 8/29 (28%) | 0.012 |
| | | 21/29 (72%) | |
| mito_carr | 184..276 | 38/124 (31%) | 2.1e-24 |
| | | 78/124 (63%) | |
| mito_carr | 278..369 | 38/124 (31%) | 3.4e-32 |
| | | 81/124 (65%) | |
| mito_carr | 375..468 | 31/124 (25%) | 2.4e-23 |
| | | 76/124 (61%) | |

### Example 13.

The NOV13 clone was analyzed, and the nucleotide and encoded polypeptide sequences are shown in Table 13A.

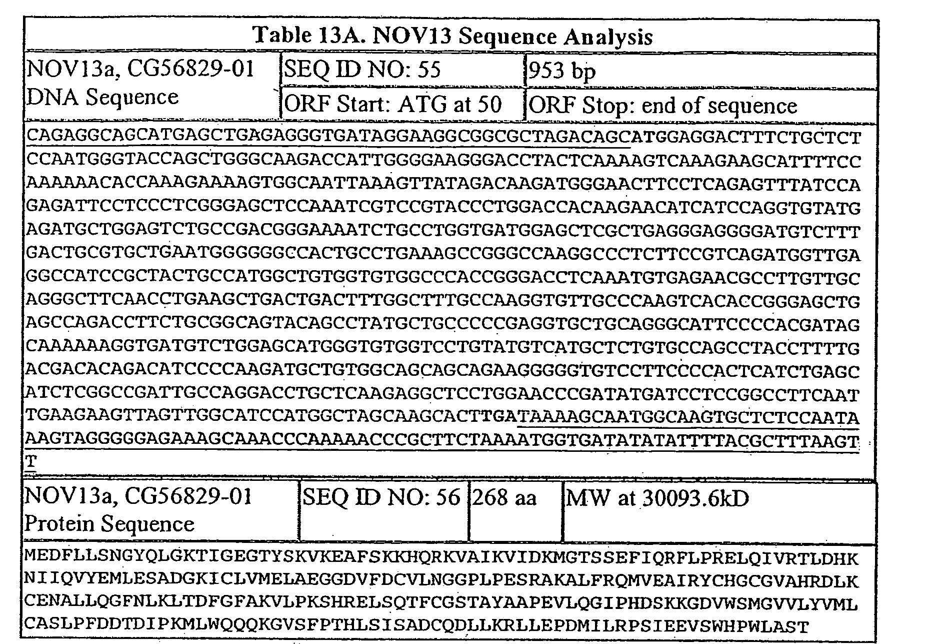

Further analysis of the NOV13a protein yielded the following properties shown in Table 13B.

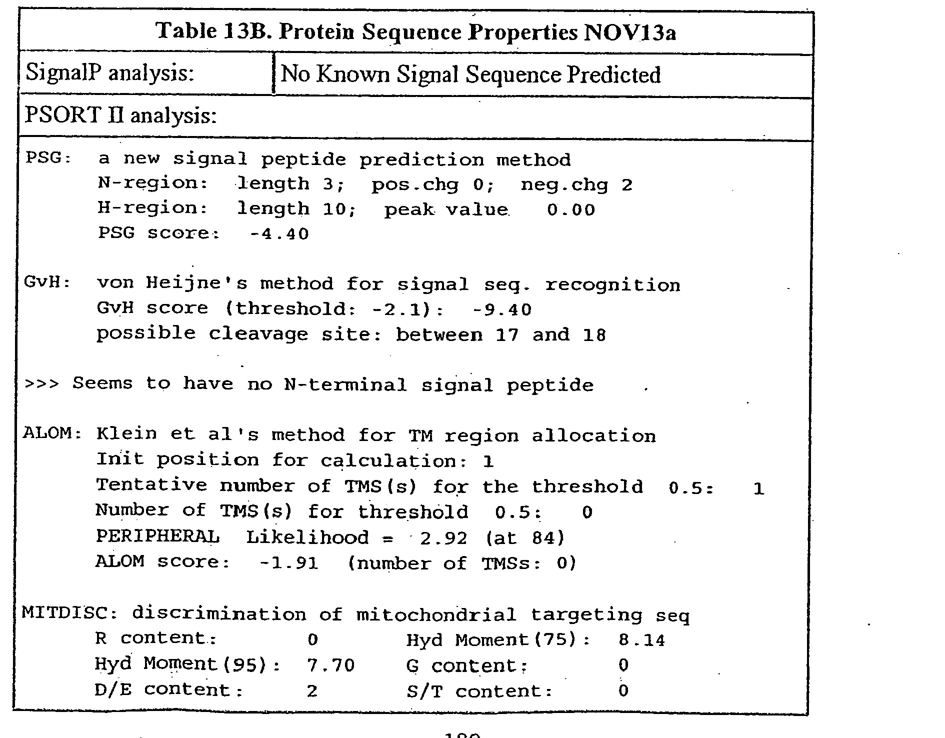

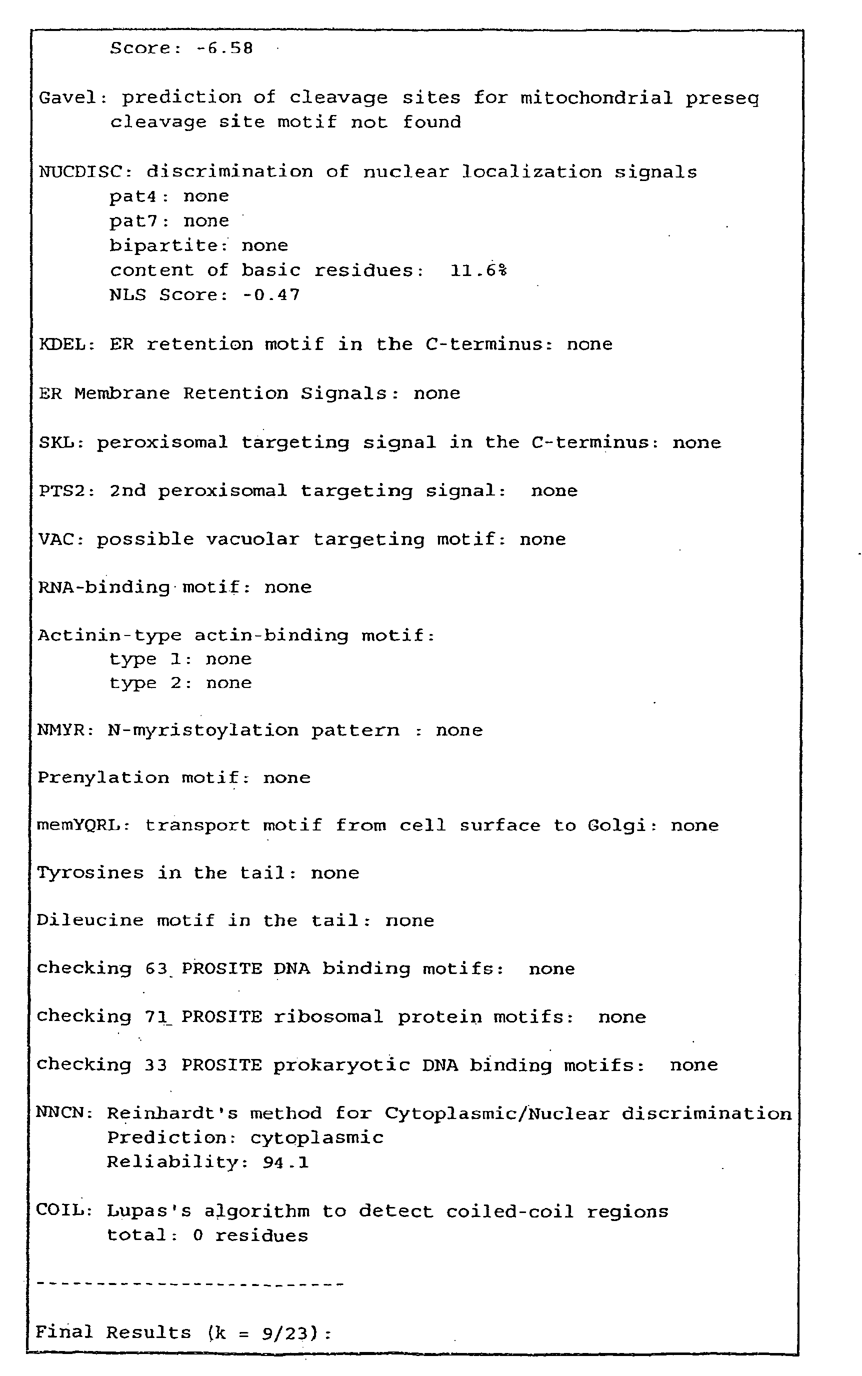

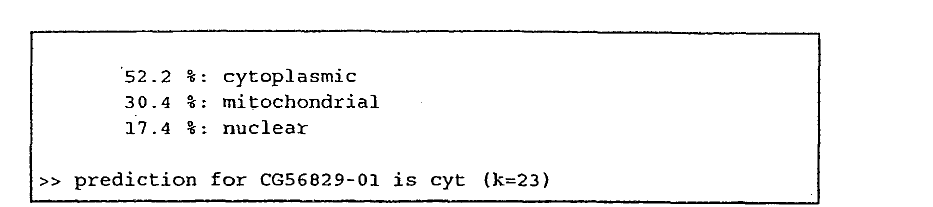

A search of the NOV13a protein against the Geneseq database, a proprietary database that contains sequences published in patents and patent publication, yielded several homologous proteins shown in Table 13C.

| **Table 13C. Geneseq Results for NOV13a** | | | | |
|---|---|---|---|---|
| **Geneseq Identifier** | **Protein/Organism/Length [Patent #, Date]** | **NOV13a Residues/ Match Residues** | **Identities/ Similarities for the Matched Region** | **Expect Value** |
| AAB65682 | Novel protein kinase, SEQ ID NO: 210 - Homo sapiens, 268 aa. [WO200073469-A2, 07-DEC-2000] | 1..268 1..268 | 266/268 (99%) 266/268 (99%) | e-155 |
| AAM47999 | Human hTSSK3 SEQ 10 NO 2 - Homo sapiens, 268 aa. [WO200185954-A2,15-NOV-2001] | 1..268 1..268 | 265/268 (98%) 265/268 (98%) | e-155 |
| AAE19154 | Human kinase polypeptide (PKIN-12) - Homo sapiens, 268 aa. [WO200208399-A2, 31-JAN-2002] | 1..268 1.268 | 265/268 (98%) 265/268 (98%) | e-155 |
| ABG30417 | Human testis specific kinase (TSSK) 3 protein - Homo sapiens, 268 aa. [WO200238732-A2, 16-MAY-2002] | 1..268 1..268 | 265/268 (98%) 265/268 (98%) | e-155 |
| ABG30419 | Mouse testis specific kinase (TSSK) 3b protein - Mus sp, 268 aa. [WO200238732-A2, 16-MAY-2002] | 1..268 1..268 | 259/268 (96%) 261/268 (96%) | e-151 |

In a BLAST search of public sequence databases, the NOV13a protein was found to have homology to the proteins shown in the BLASTP data in Table 13D.

| **Table 13D. Public BLASTP Results for NOV13a** | | | | |
|---|---|---|---|---|
| **Protein Accession Number** | **Protein/Organism/Length** | **NOV13a Residues/Match Residues** | **Identities/ Similarities for the Matched Portion** | **Expect Value** |
| Q96PN8 | Testis-specific serine/threonine kinase 3 (Similar to serine/threonine kinase) (TSSK3) - Homo sapiens (Human), 268 aa. | 1..268 1..268 | 265/268 (98%) 265/268 (98%) | e-154 |
| Q9D2E1 | 4930594121Rik protein (Testis-specific serine/threonine kinase 3b) - Mus musculus (Mouse), 268 aa. | 1..268 1..268 | 259/268 (96%) 261/268 (96%) | e-151 |
| Q9JL98 | Testis specific serine kinase-3 - Mus musculus (Mouse), 266 aa. | 1..268 1..266 | 240/273 (87%) 245/273 (88%) | e-134 |
| Q8IY55 | Hypothetical protein - Homo sapiens (Human), 358 aa. | 5..265 7..272 | 125/266 (46%) 190/266 (70%) | 1e-69 |
| Q96PF2 | Testis specific serine/threonine kinase 2 - Homo sapiens (Human), 358 aa. | 5..265 7.272 | 125/266 (46%) 190/266 (70%) | 1e-69 |

PFam analysis predicts that the NOV13a protein contains the domains shown in the Table 13E.

| **Table 13E. Domain Analysis of NOV13a** | | | |
|---|---|---|---|
| **Pfam Domain** | **NOV13a Match Region** | Identities/ **Similarities** for the Matched Region | **Expect Value** |
| pkinase | 10.265 | 99/299 (33%) 203/299 (68%) | 1.5e-80 |

### Example 14.

The NOV14 clone was analyzed, and the nucleotide and encoded polypeptide sequences are shown in Table 14A.

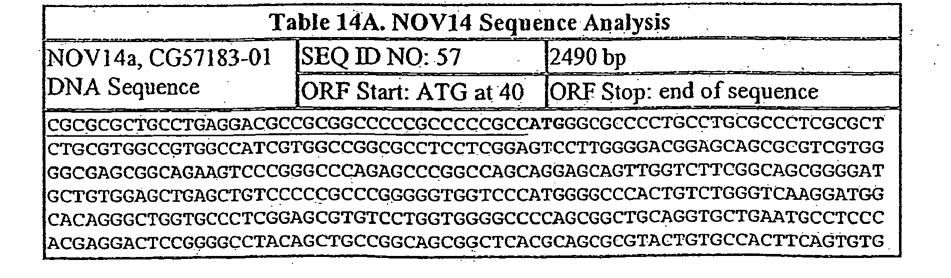

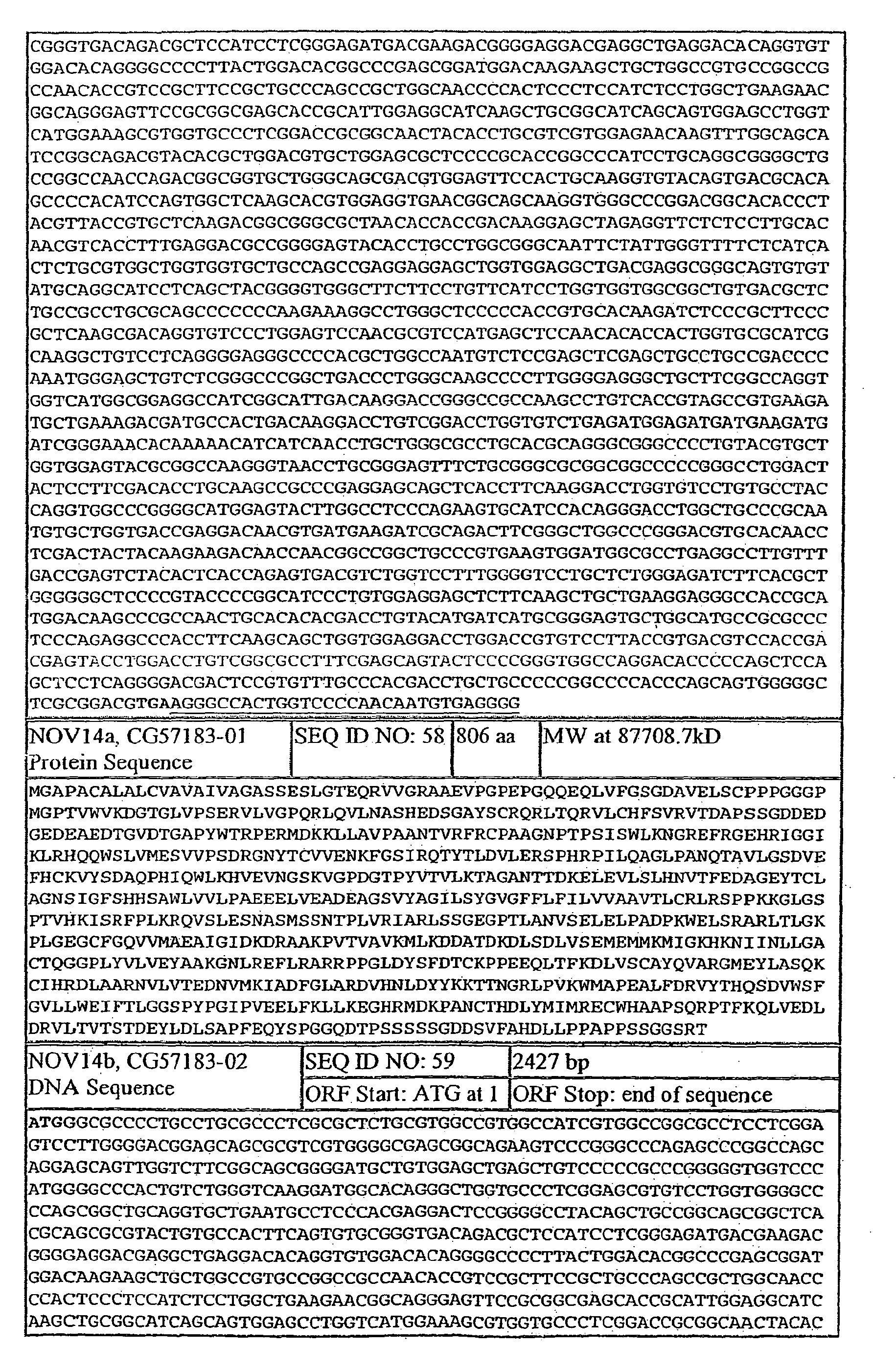

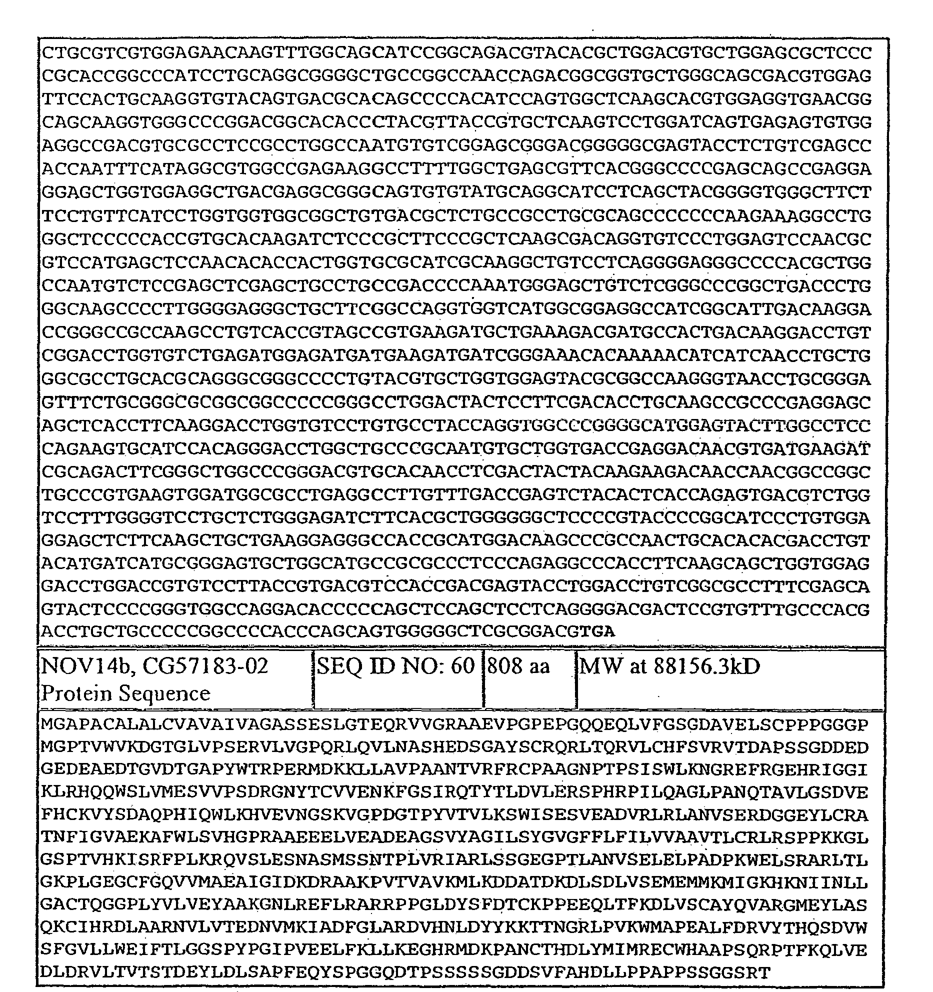

A ClustalW comparison of the above protein sequences yields the following sequence alignment shown in Table 14B.

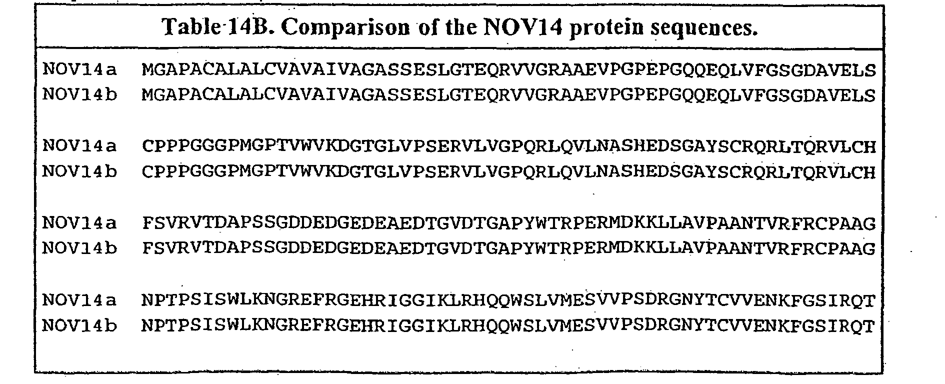

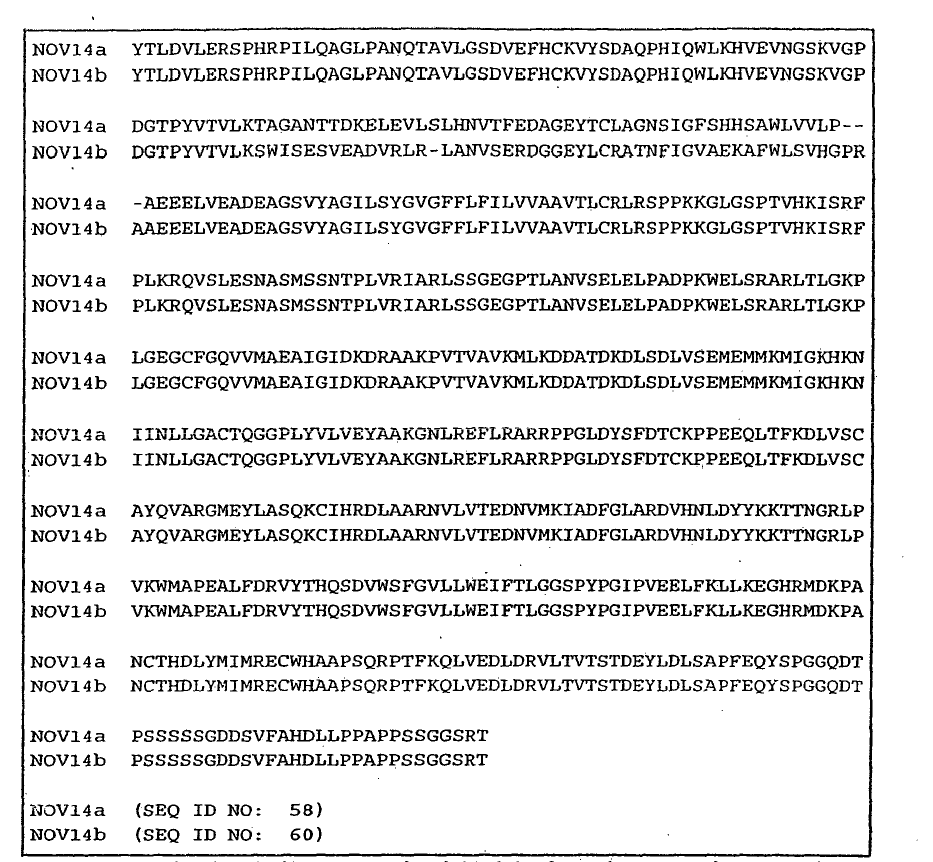

Further analysis of the NOV14a protein yielded the following properties shown in Table 14C.

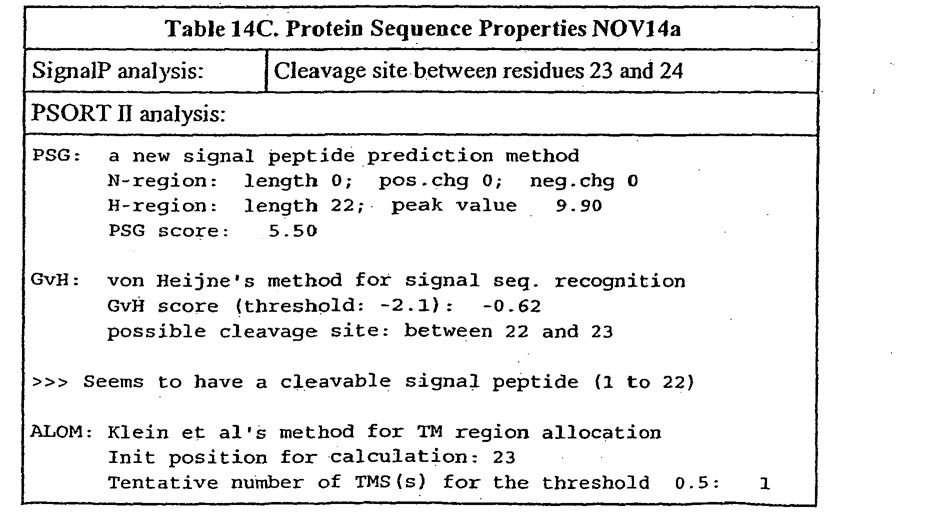

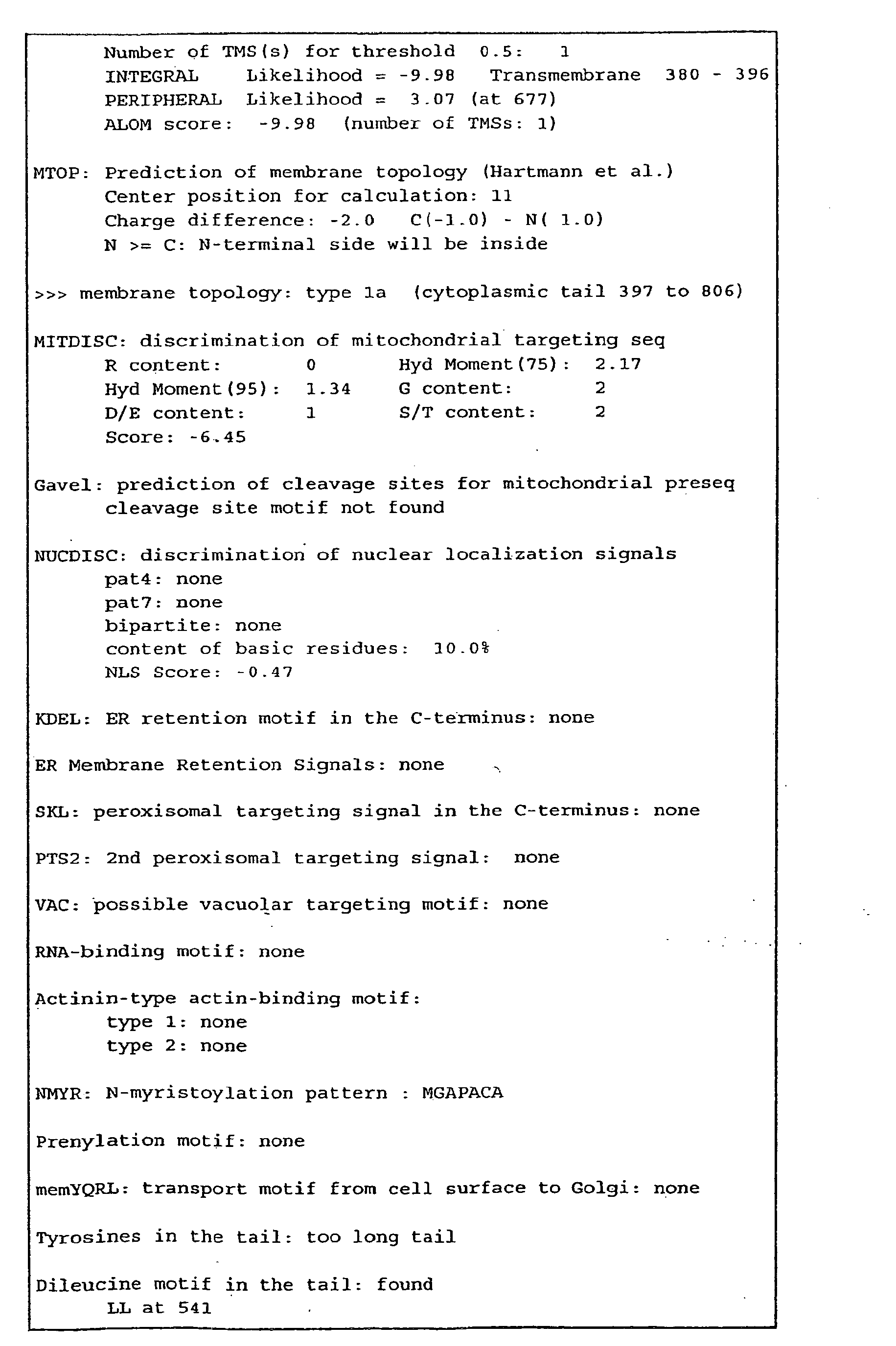

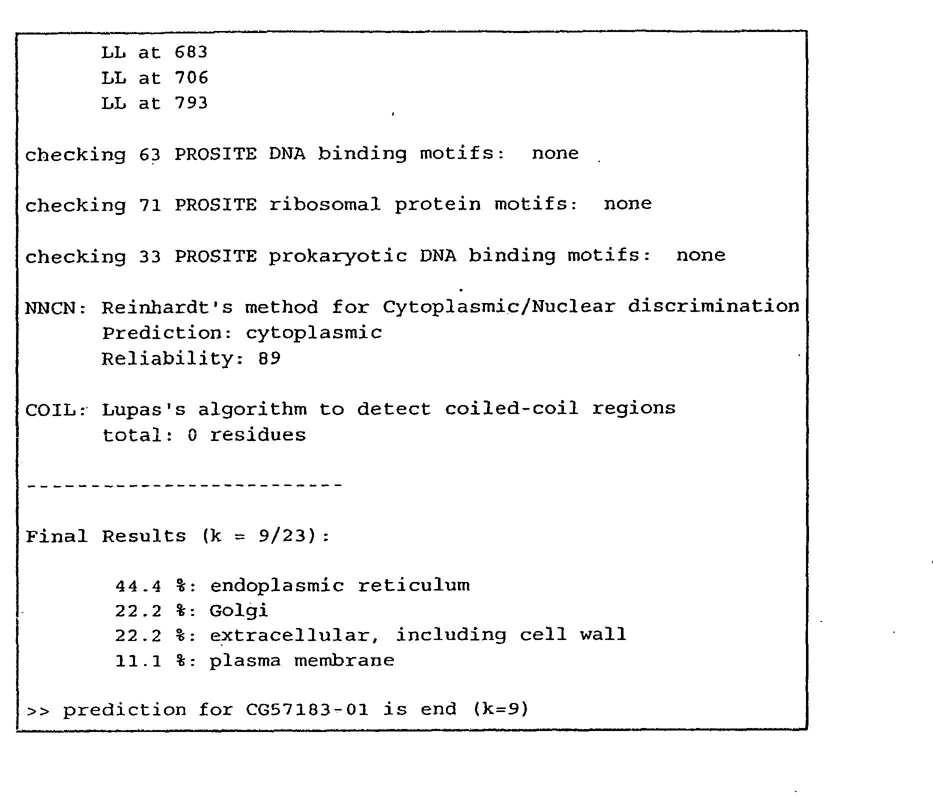

A search of the NOV14a protein against the Geneseq database, a proprietary database that contains sequences published in patents and patent publication, yielded several homologous proteins shown in Table 14D.

| **Table 14D. Geneseq Results for NOV14a** | | | | |
|---|---|---|---|---|
| **Geneseq Identifier** | **Protein/Organism/Length [Patent #, Date]** | **NOV14a Residues/Match Residues** | **Identities/ Similarities for the Matched Region** | **Expect Value** |
| AAE21976 | Human fibroblast growth factor receptor 3c (FGFR3c) mutant protein - Homo sapiens, 806 aa. [US6265632-B1, 24-JUL-2001] | 1..806 1..806 | 805/806 (99%) 805/806 (99%) | 0.0 |
| AAE21977 | Mouse fibroblast growth factor receptor 3 (FGFR3) mutant protein - Mus sp, 801 aa. [US6265632-B1,24-JUL-2001] | 1..806 1..801 | 745/807 (92%) 763/807 (94%) | 0.0 |
| AAU02978 | Angiotensin converting enzyme (ACEV) splice variant protein #78 - Homo sapiens, 561 aa. [WO200136632-A2, 25-MAY-2001] | 1..542 1..542 | 541/542 (99%) 541/542 (99%) | 0.0 |
| AAR21080 | flg receptor protein-Homo sapiens, 821 aa. [WO9200999-A, 23-JAN-1992] | 45..798 46..807 | 535/769 (69%) 615/769 (79%) | 0.0 |
| AAB84383 | Amino acid sequence of a fibroblast growth factor receptor - Homo sapiens, 820 aa. [US6255454-B1, 03-JUL-2001] | 39..798 33..803 | 508/774 (65%) 608/774 (77%) | 0.0 |

In a BLAST search of public sequence databases, the NOV14a protein was found to have homology to the proteins shown in the BLASTP data in Table 14E.

| **Table 14E. Public BLASTP Results for NOV14a** | | | | |
|---|---|---|---|---|
| **Protein Accession Number** | **Protein/Organism/Length** | **NOVl4a Residues/Match Residues** | **Identities/Similarities for the Matched Portion** | **Expect Value** |
| P22607 | Fibroblast growth factor receptor 3 precursor (EC 2.7.1.112) (FGFR-3) - Homo sapiens (Human), 806 aa. | 1..806 1..806 | 806/806 (100%) 806/806 (100%) | 0.0 |
| Q8NI15 | Fibroblast growth factor receptor 3 - Homo sapiens (Human), 769 aa (fragment). | 38..806 1..769 | 769/769(100%) 769/769 (100%) | 0.0 |
| A48991 | heparin-binding growth factor receptor - mouse, 800 aa. | 1..806 1..800 | 747/806 (92%) 765/806 (94%) | 0.0 |
| Q9JHX9 | Fibroblast growth factor receptor 3 - Rattus norvegicus (Rat), 800 aa. | 1..806 1..800 | 745/806 (92%) 764/806 (94%) | 0.0 |
| Q99052 | Fibroblast growth factor receptor - Mus musculus (Mouse), 800 aa. | 1..806 1..800 | 745/806 (92%) 764/806 (94%) | 0.0 |

PFam analysis predicts that the NOV14a protein contains the domains shown in the Table 14F.

| **Table 14F. Domain Analysis of NOV14a** | | | |
|---|---|---|---|
| **Pfam Domain** | **NOV14a Match Region** | **Identities/ Similarities for the Matched Region** | **Expect Value** |
| ig | 54..111 | 18/59 (31%) | 8.3e-05 |
| | | 40/59 (68%) | |
| ig | 169..230 | 21/65 (32%) | 5.6e-07 |
| | | 50/65 (77%) | |
| ig | 268..341 | 18/77(23%) | 1.6e-06 |
| | | 54/77 (70%) | |
| pkinase | 472..750 | 98/319 (31%) | 2.2e-91 |
| | | 237/319 (74%) | |

### Example 15.

The NOV15 clone was analyzed, and the nucleotide and encoded polypeptide sequences are shown in Table 15A.

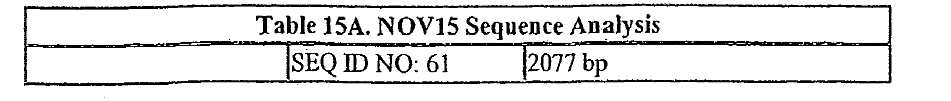

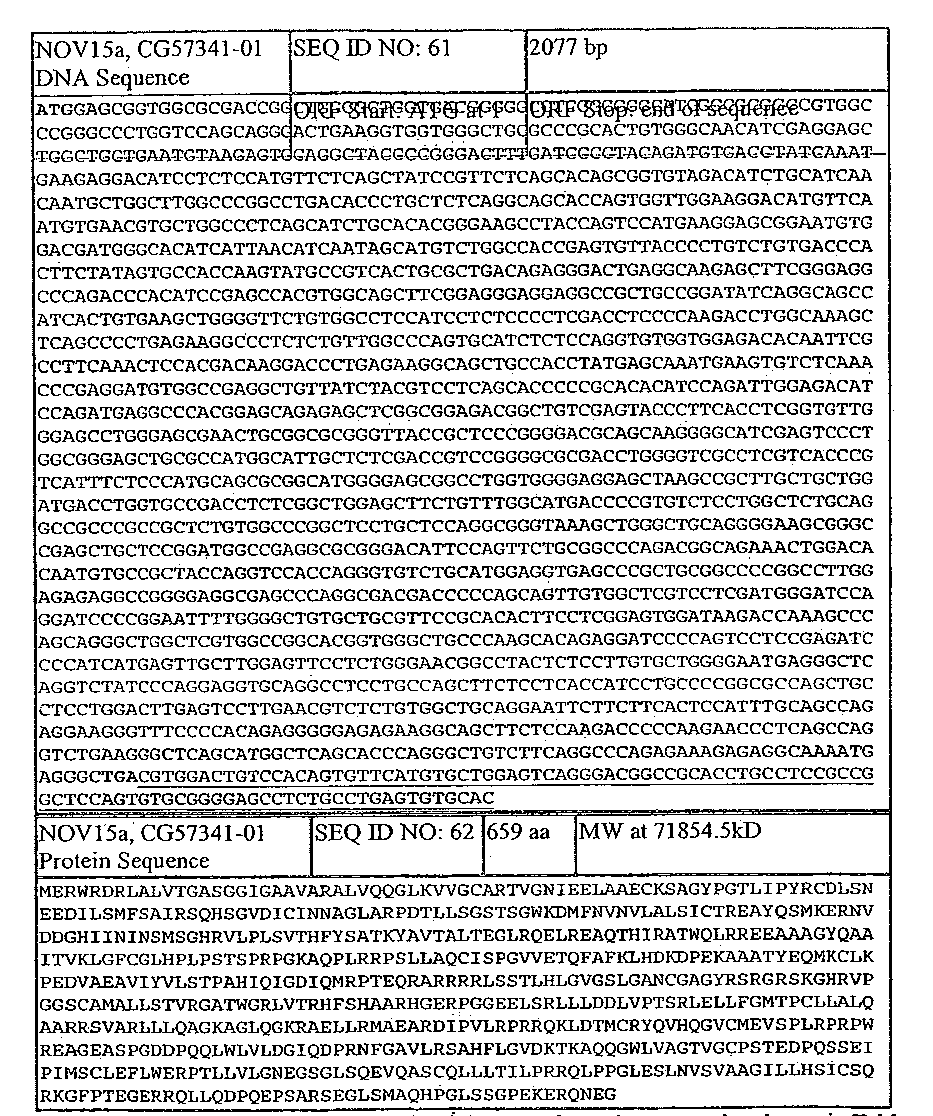

Further analysis of the NOV15a protein yielded the following properties shown in Table 15B.

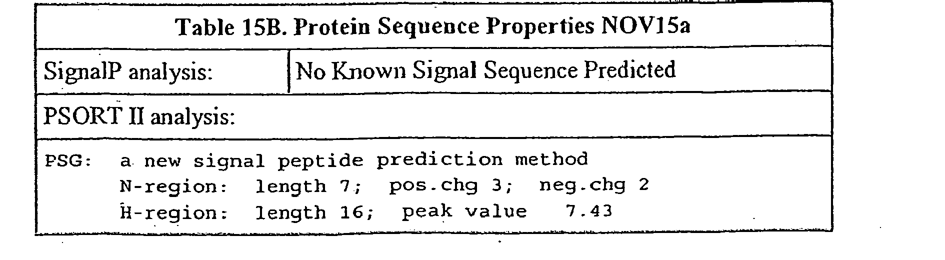

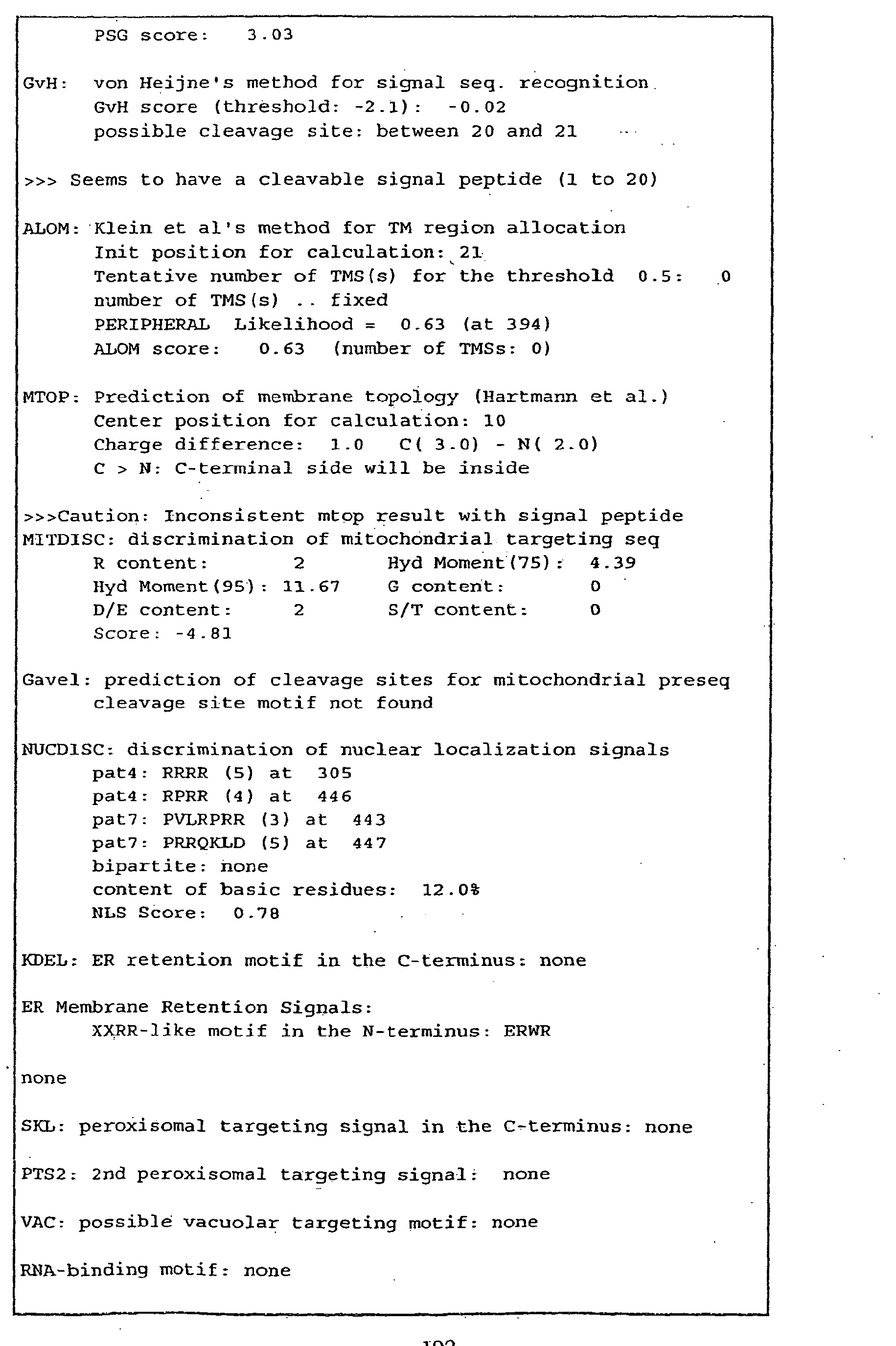

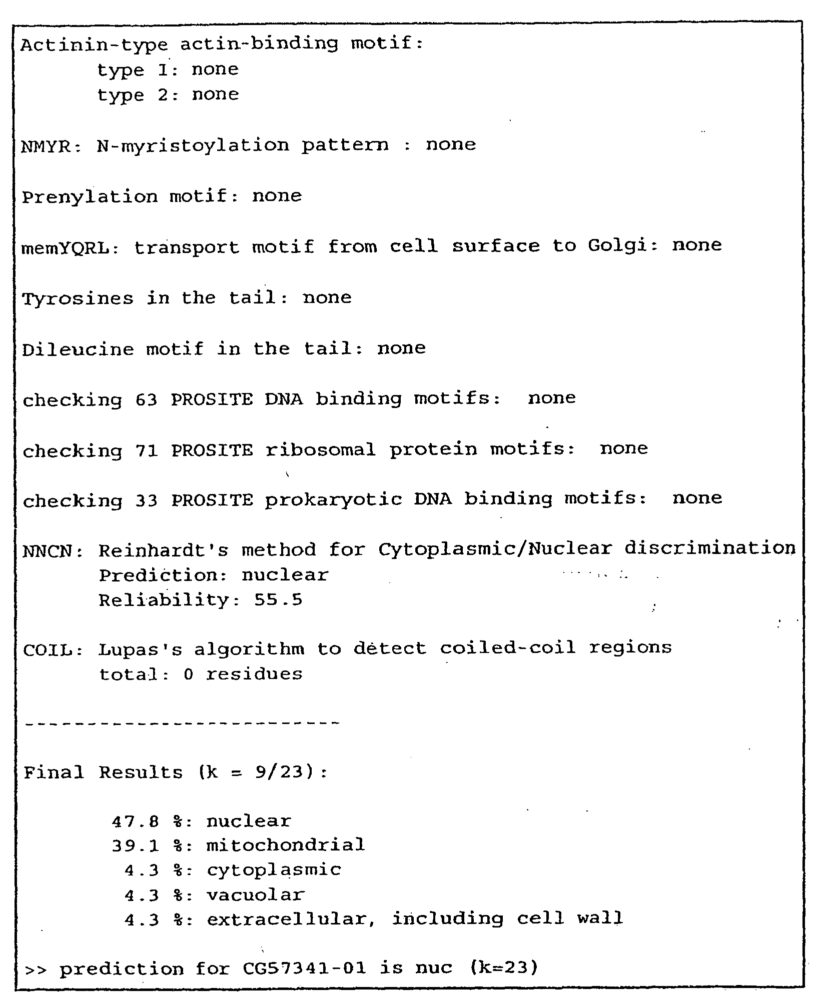

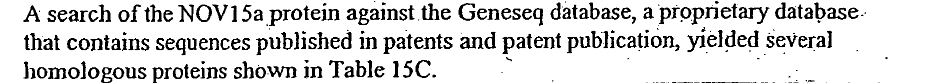

| **Table 15C. Geneseq Results for NOV15a** | | | | |
|---|---|---|---|---|
| **Geneseq Identifier** | **Protein/Organism/Length [Patent #, Date]** | **NOV15a Residues/ Match** Residues | **Identities/ Similarities for the Matched Region** | **Expect Value** |
| ABU10698 | Human secreted/transmembrane protein #189 - Homo sapiens, 260 aa. [US2002127584-A1, 12-SEP-2002] | 1..302 6..258 | 253/302 (83%) 253/302 (83%) | e-137 |
| AAU76220 | Human 21620 alcohol dehydrogenase (ADH) protein - Homo sapiens, 260 aa. [US2002010946-A1, 24-JAN-2002] | 1..302 6..258 | 253/302(83%) 253/302 (83%) | e-137 |
| ABG95914 | Human secreted/transmembrane protein PRO1774 - Homo sapiens, 260 aa. [US2002119130-A1, 29-AUG-2002] | 1..302 6..258 | 23/302(83%) 253/302 (83%) | e-137 |
| AAT387S89 | Human PRO1774 - Homo sapiens, 260 aa. [WO200116318-A2, 08-MAR-2001] | 1..302 6..258 | 253/302(83%) 253/302 (83%) | e-137 |
| AAB84364 | Amino acid sequence of human alcohol dehydrogenase 21620 - Homo sapiens, 260 aa. [WO200144446-A2, 21-JUN-2001] | 1..302 6..258 | 253/302(83%) 253/302(83%) | e-137 |

In a BLAST search of public sequence databases, the NOV15a protein was found to have homology to the proteins shown in the BLASTP data in Table 15D.

| **Table 15D. Public BLASTP Results for NOV15a** | | | | |
|---|---|---|---|---|
| **Protein Accession Number** | **Protein/Organism/Length** | **NOV15a Residues/ Match Residues** | **Identities/ Similarities for the Matched Portion** | **Expect Value** |
| CAC43880 | Sequence 2 from Patent WO0144446 - Homo sapiens (Human), 260 aa. | 1..302 6..258 | 253/302 (83%) 253/302 (83%) | e-136 |
| Q9BUC7 | Hypothetical protein - Homo sapiens (Human), 181 aa. | 75..302 1..179 | 179/228 (78%) 179/228 (78%) | 5e-92 |
| Q96GK2 | Hypothetical protein - Homo sapiens (Human), 158 aa. | 515..659 14..158 | 142/145(97%) 143/145 (97%) | 1e-76 |
| Q9H664 | Hypothetical protein FLJ22578 - Homo sapiens (Human), 155 aa. | 515..659 11..155 | 142/145 (97%) 143/145 (97%) | 1e-76 |
| Q8BRZ1 | Hypothetical protein - Mus musculus (Mouse), 197 aa. | 357..523 10..176 | 131/167 (78%) 144/167 (85%) | 6e-71 |

PFam analysis predicts that the NOV15a protein contains the domains shown in the Table 15E.

| **Table 15E. Domain Analysis of NOV15a** | | | |
|---|---|---|---|
| **Pfam Domain** | **NOV15a Match Region** | Identities/ **Similarities** for the Matched Region | **Expect Value** |
| adh_short | 5..319 | 73/337 (22%) 217/337 (64%) | 1.9e-33 |
| SpoU_methylase | 490..607 | 37/156 (24%) 91/156(58%) | le-05 |

### Example 16.

The NOV16 clone was analyzed, and the nucleotide and encoded polypeptide sequences are shown in Table 16A.

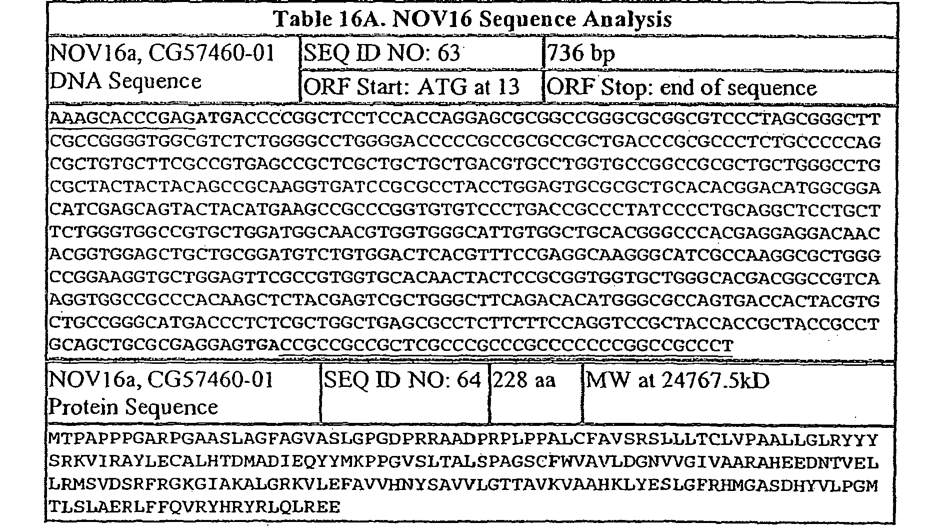

Further analysis of the NOV16a protein yielded the following properties shown in Table 16B.

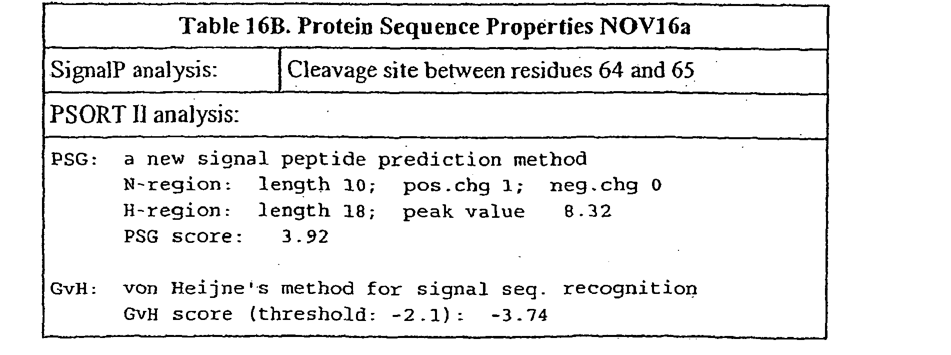

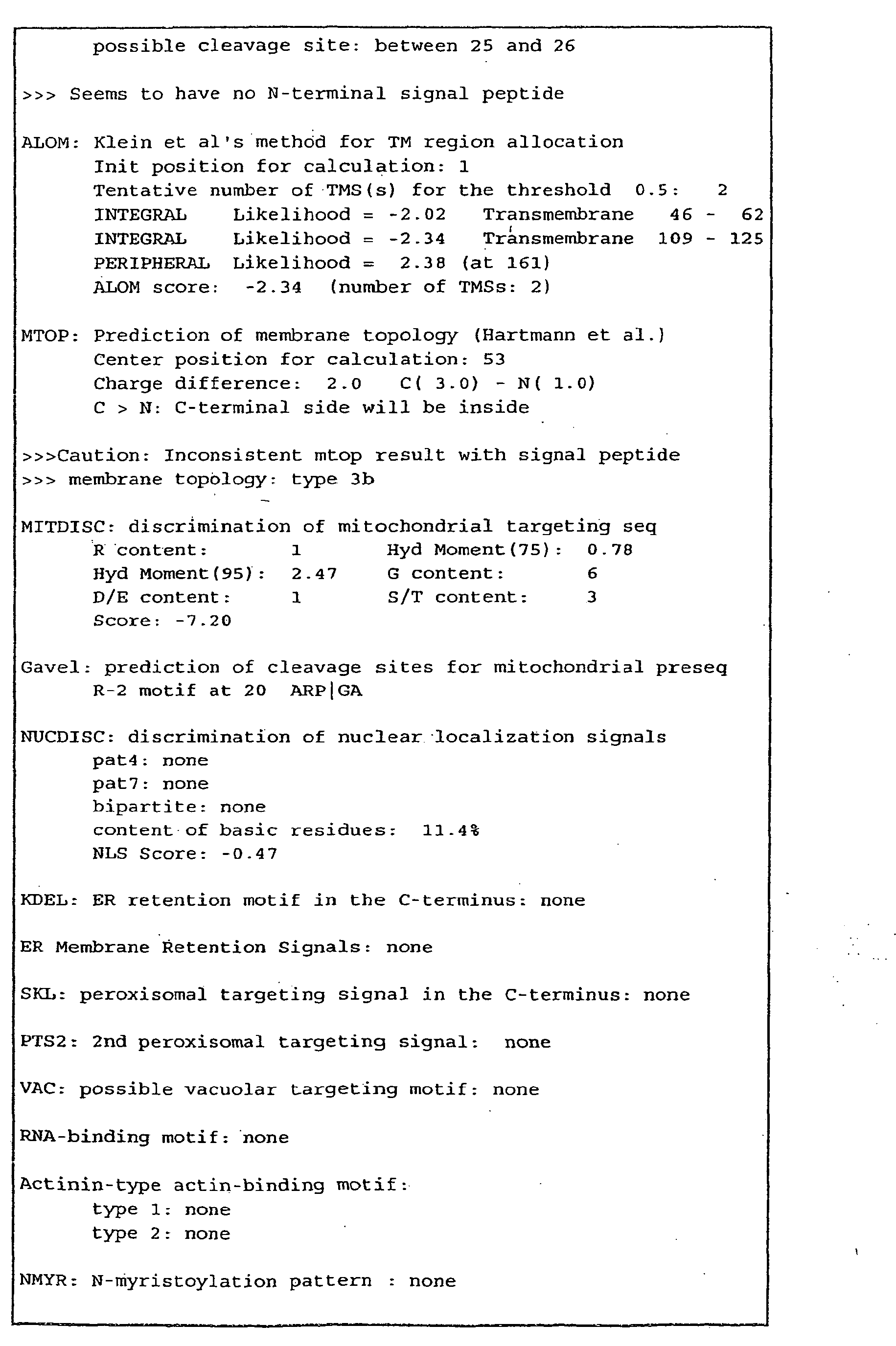

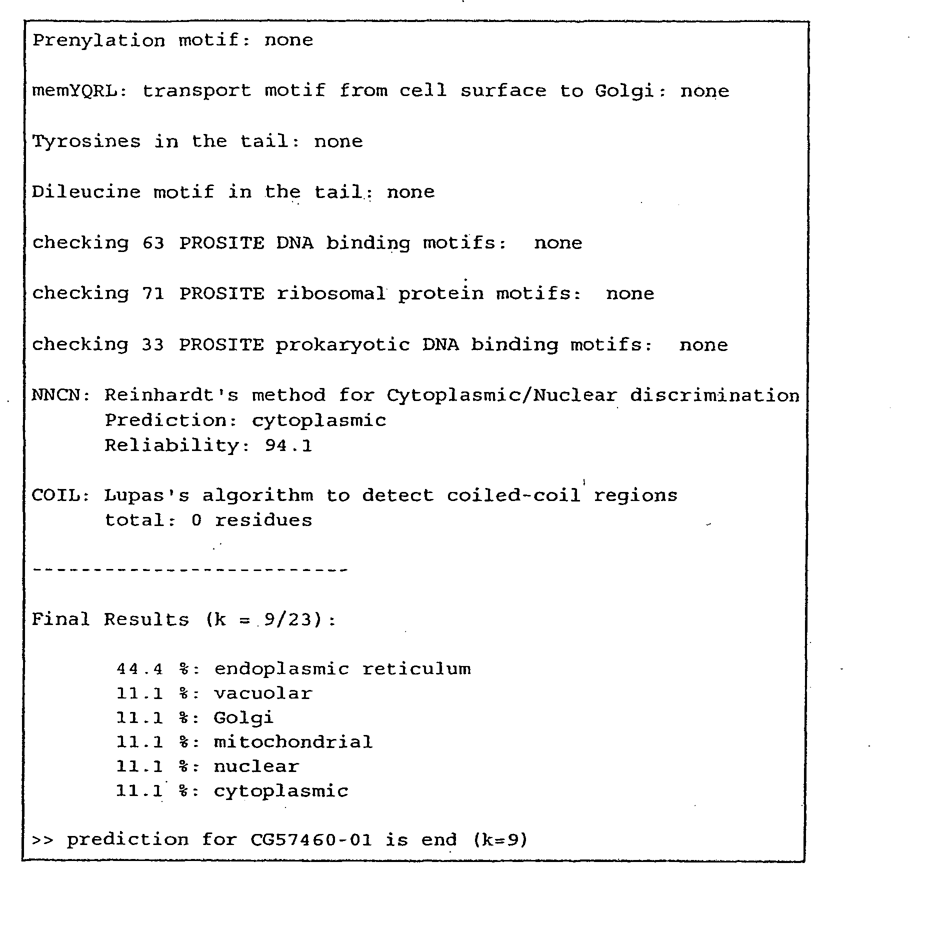

A search of the NOV16a protein against the Geneseq database, a proprietary database that contains sequences published in patents and patent publication, yielded several homologous proteins shown in Table 16C.

| **Table 16C. Geneseq Results for NOV16a** | | | | |
|---|---|---|---|---|
| **Geneseq Identifier** | **Protein/Organism/Length [Patent #, Date]** | **NOV16a Residues/ Match Residues** | **Identities/Similarities for the Matched Region** | **Expect Value** |
| AAE15442 | Human drug metabolising enzyme (DME)-9 - Homo sapiens, 218 aa. [WO200179468-A2, 25-OCT-2001] | 1..228 1..218 | 218/228 (95%) 218/228 (95%) | e-121 |
| ABU11545 | Human MDDT polypeptide SEQ ID 492 - Homo sapiens, 196 aa. [WO200279449-A2, 10-OCT-2002] | 42..228 20..196 | 176/187 (94%) 177/187 (94%) | 2e-95 |
| AAB19986 | Human camello 3 (Hcml3) protein (partial) - Homo sapiens, 144 aa. [WO200077024-A1, 21-DEC-2000] | 42..195 1..144 | 144/154 (93%) 144/154 (93%) | 1e-75 |
| AAB19985 | Human camello 2 (Hcm12) protein - Homo sapiens, 227 aa. [WO200077024-A1, 21-DEC-2000] | 47..200 56..203 | 63/158 (39%) 92/158 (57%) | 2e-21 |
| AAB19984 | Human camello 1 (Hcm11) protein - Homo sapiens, 227 aa. [WO200077024-A1, 21-DEC-2000] | 41..196 50..199 | 60/160 (37%) 88/160 (54%) | 1e-19 |

In a BLAST search of public sequence databases, the NOV16a protein was found to have homology to the proteins shown in the BLASTP data in Table 16D.

| **Table 16D. Public BLASTP Results for NOV16a** | | | | |
|---|---|---|---|---|
| **Protein Accession Number** | **Protein/Organism/Length** | **NOV16a Residues/ Match Residues** | **Identities/ Similarities for the Matched Portion** | **Expect Value** |
| Q8N9F0 | Hypothetical protein FLJ37478 - Homo sapiens (Human), 134 aa. | 85..228 1..134 | 134/144 (93%) 134/144 (93%) | 1e-69 |
| Q8K065 | Hypothetical protein-Mus musculus (Mouse), 53 aa (fragment). | 176..228 1..53 | 53/53(100%) 53/53 (100%) | 2e-23 |
| Q9UHF3 | Putative N-acetyltransferase Camello 2 - Homo sapiens (Human), 227 aa. | 47..200 56..203 | 63/158 (39%) 92/158 (57%) | 7e-21 |
| Q9UQ17 | GLA protein - Homo sapiens (Human), 227 aa. | 41..196 50..199 | 60/160 (37%) 88/160 (54%) | 4e-19 |
| Q9UHE5 | Putative N-acetyltransferase CML1 - Homo sapiens (Human), 227 aa. | 41..196 50..199 | 60/160 (37%) 88/160 (54%) | 4e-19 |

PFam analysis predicts that the NOV16a protein contains the domains shown in the Table 16E.

| **Table 16E. Domain Analysis of NOV16a** | | | |
|---|---|---|---|
| **Pfam Domain** | **NOV16a Match Region** | **Identities/Similarities for the Matched Region** | **Expect Value** |
| Acetyltransf | 111..191 | 28/82(34%) 65/82 (79%) | 1.8e-17 |

### Example 17.

The NOV17 clone was analyzed, and the nucleotide and encoded polypeptide sequences are shown in Table 17A.

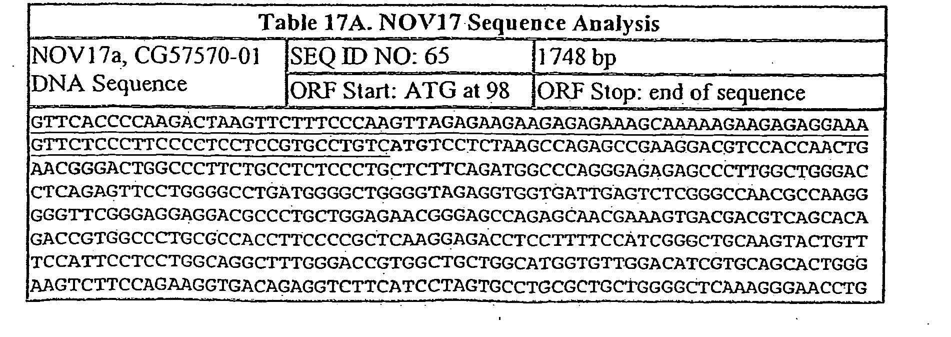

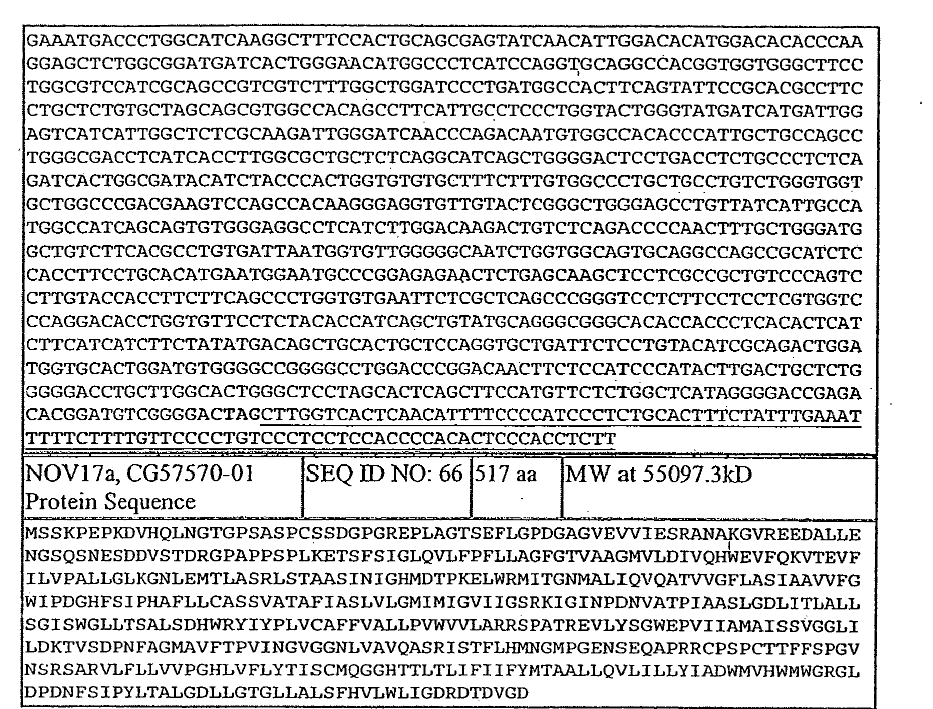

Further analysis of the NOV17a protein yielded the following properties shown in Table 17B.

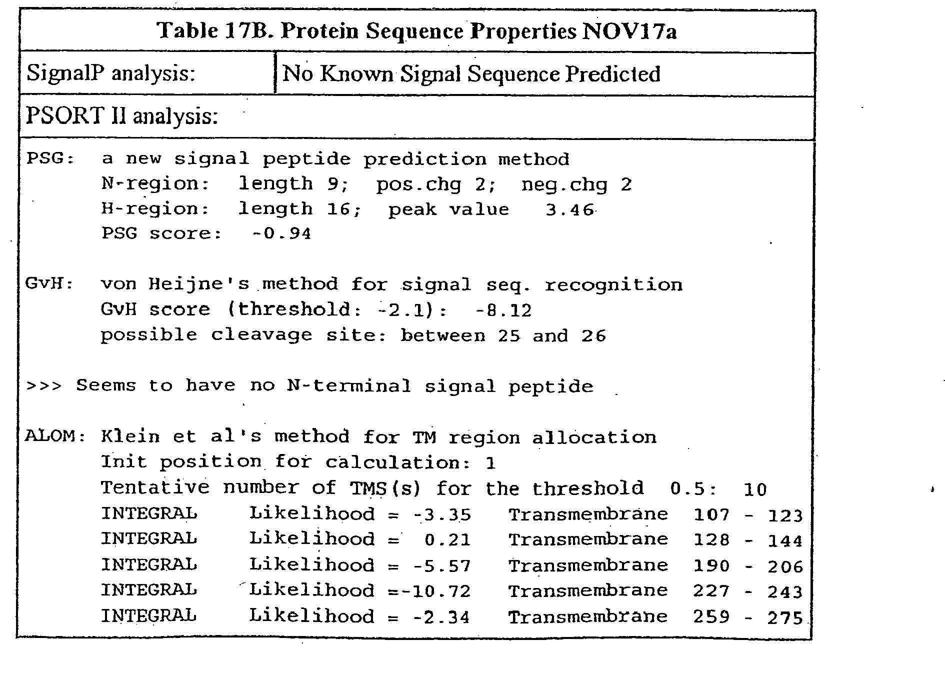

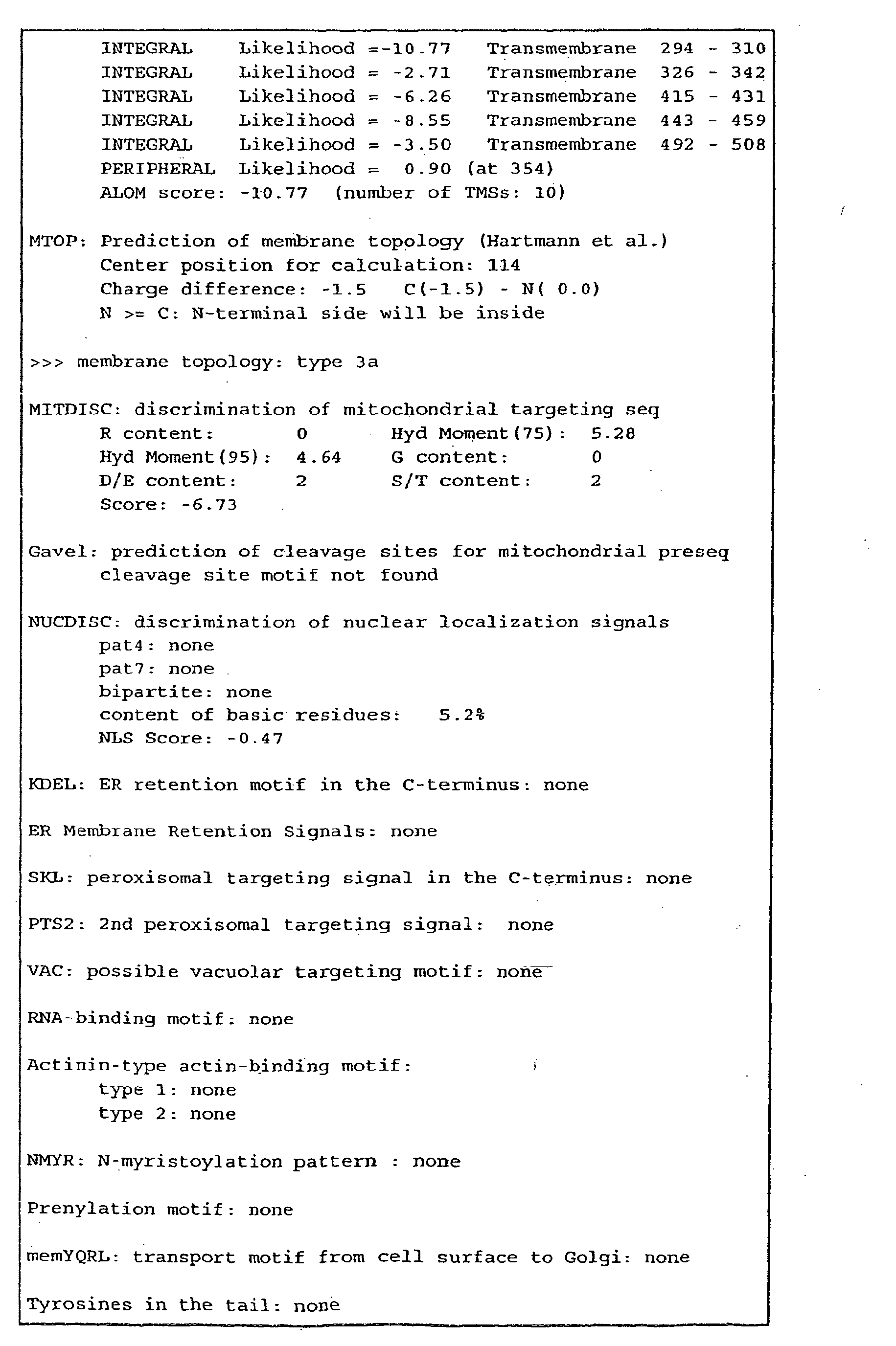

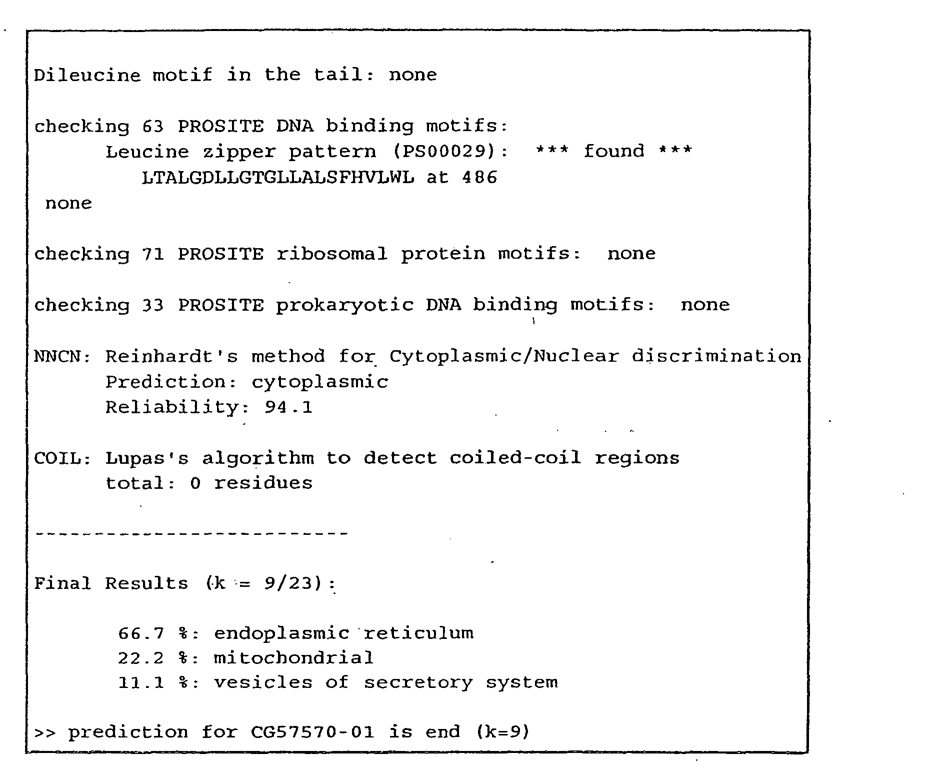

A search of the NOV17a protein against the Geneseq database, a proprietary database that contains sequences published in patents and patent publication, yielded several homologous proteins shown in Table 17C.

| **Table 17C. Geneseq Results for NOV17a** | | | | |
|---|---|---|---|---|
| **Geneseq Identifier** | **Protein/Organism/Length [Patent #, Date]** | **NOV17a Residues/Match Residues** | **Identities/Similarities for the Matched Region** | **Expect Value** |
| ABB08638 | Human transporter protein SEQ ID NO 2 - Homo sapiens, 513 aa. [WO200190360-A2,29-NOV-2001] | 1..517 1..513 | 506/517 (97%) 507/517 (97%) | 0.0 |
| ABP74113 | Human TRICH SEQ ID NO 18 - Homo sapiens, 573 aa. [WO200246415-A2, 13-JUN-2002] | 62..517 125..573 | 307/457 (67%) 373/457 (81%) | e-173 |
| AAB95482 | Human protein sequence SEQ ID NO:18007 - Homo sapiens, 490 aa. [EP1074617-A2, 07-FEB-2001] | 62..517 42..490 | 307/457 (67%) 373/457 (81%) | e-173 |
| AAY94882 | Human protein clone HP10031 - Homo sapiens, 487 aa. [WO200005367-A2, 03-FEB-2000] | 60..508 33..470 | 246/451(54%) 315/451 (69%) | e-129 |
| AAM47910 | Human initiation factor 46 - Homo sapiens, 414 aa. [CN1307045-A, 08-AUG-2001] | 107..508 1..397 | 227/404 (56%) 290/404 (71%) | e-121 |

In a BLAST search of public sequence databases, the NOV17a protein was found to have homology to the proteins shown in the BLASTP data in Table 17D.

| **Table 17D. Public BLASTP Results for NOV17a** | | | | |
|---|---|---|---|---|
| **Protein Accession Number** | **Protem/Orgamsm/Length** | **NOV17a Residues/ Match Residues** | **Identities/ Similarities for the Matched Portion** | **Expect Value** |
| Q8INJ1 | Solute carrier family 41 member 1 - Homo sapiens (Human), 513 aa. | 1..517 1..513 | 502/517 (97%) 504/517 (97%) | 0.0 |
| Q8BJA2 | Hypothetical divalent cation transporter containing protein - Mus musculus (Mouse), 512 aa. | 1..517 1..512 | 496/517 (95%) 499/517 (95%) | 0.0 |
| Q8BYR8 | Hypothetical divalent cation transporter containing protein - Mus musculus (Mouse), 573 aa. | 62..517 126..573 | 306/456 (67%) 373/456 (81%) | e-175 |
| Q9H0E5 | Hypothetical protein DKFZp434K0427 - Homo sapiens (Human), 490 aa. | 62..517 42..490 | 307/457 (67%) 373/457 (81%) | e-173 |
| Q96JW4 | Hypothetical protein FLJ14932 - Homo sapiens (Human), 490 aa. | 62..517 42..490 | 307/457 (67%) 373/457 (81%) | e-173 |

PFam analysis predicts that the NOV17a protein contains the domains shown in the Table 17E.

| **Table 17E. Domain Analysis of NOV17a** | | | |
|---|---|---|---|
| **Pfam Domain** | **NOV17a Match Region** | **Identities/ Similarities for the Matched Region** | **Expect Value** |
| MgtE | 138..276 | 39/140 (28%) 117/140 (84%) | 2.8e-26 |
| MgtE | 356..503 | 33/153 (22%) 117/140 (84%) | 1.1e-06 |

### Example 18.

The NOV18 clone was analyzed, and the nucleotide and encoded polypeptide sequences are shown in Table 18A.

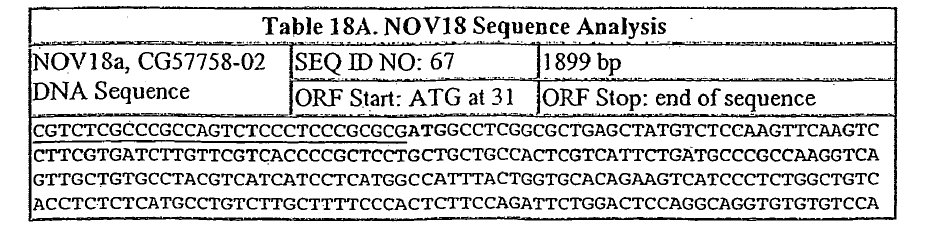

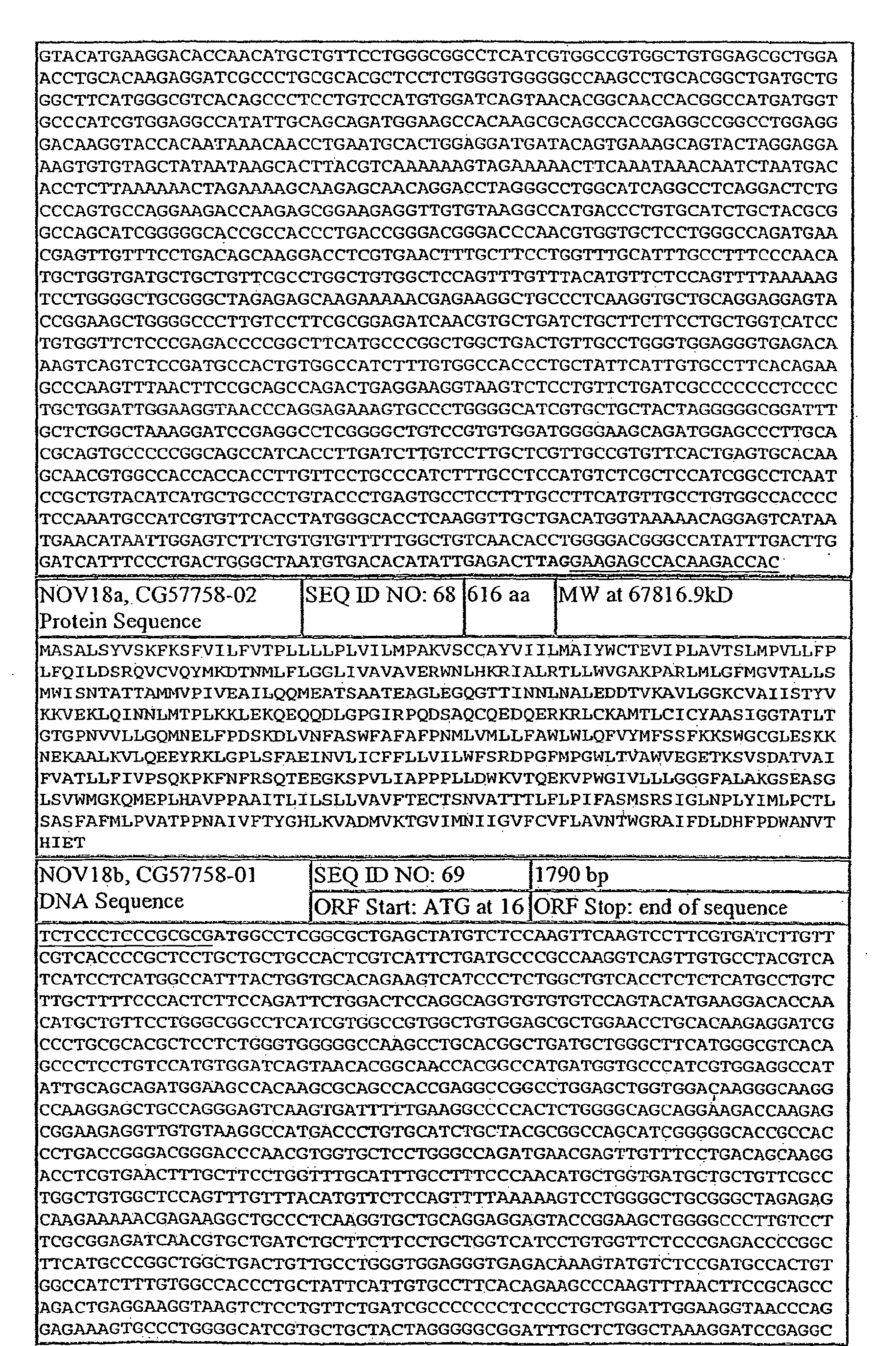

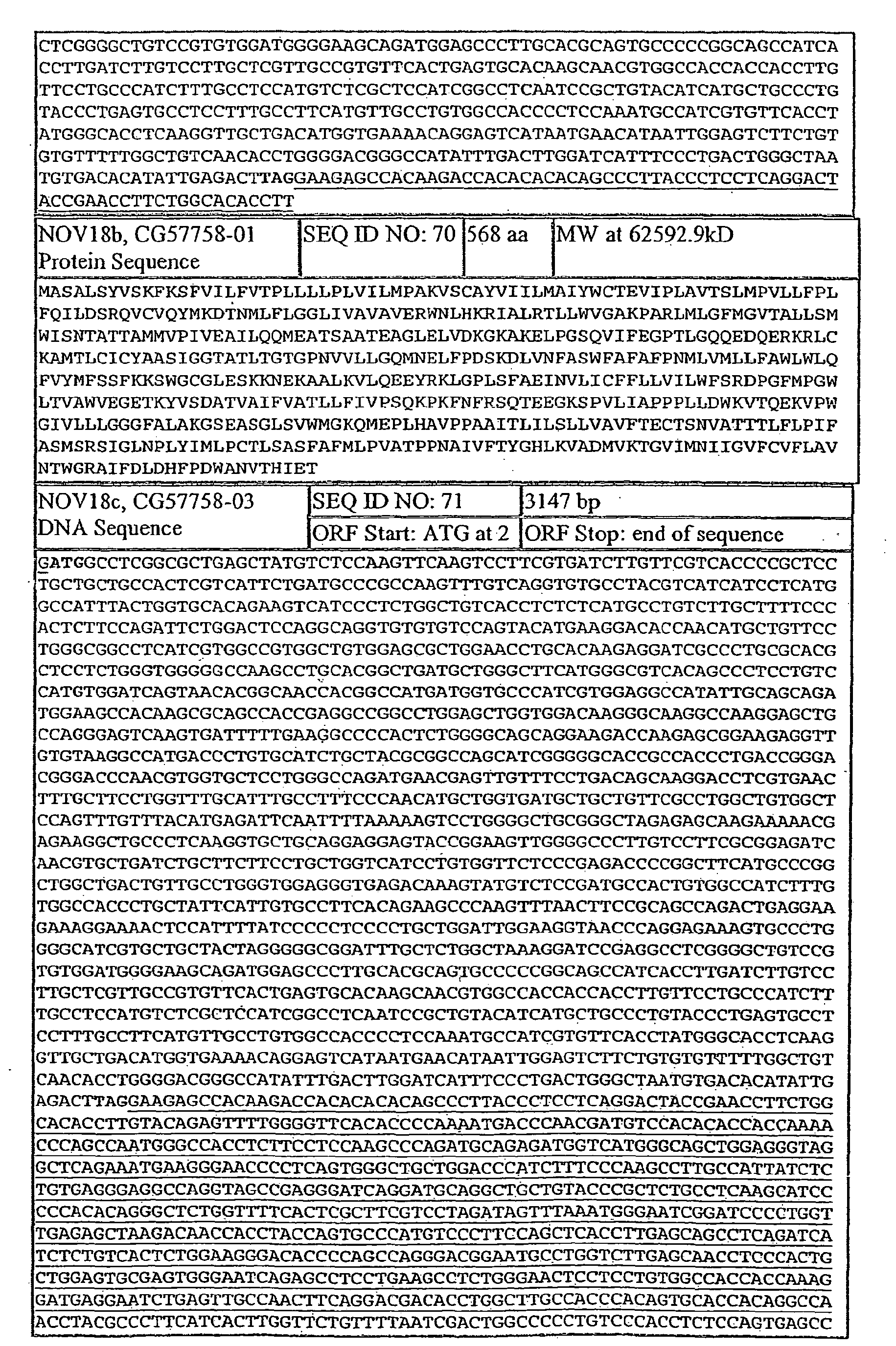

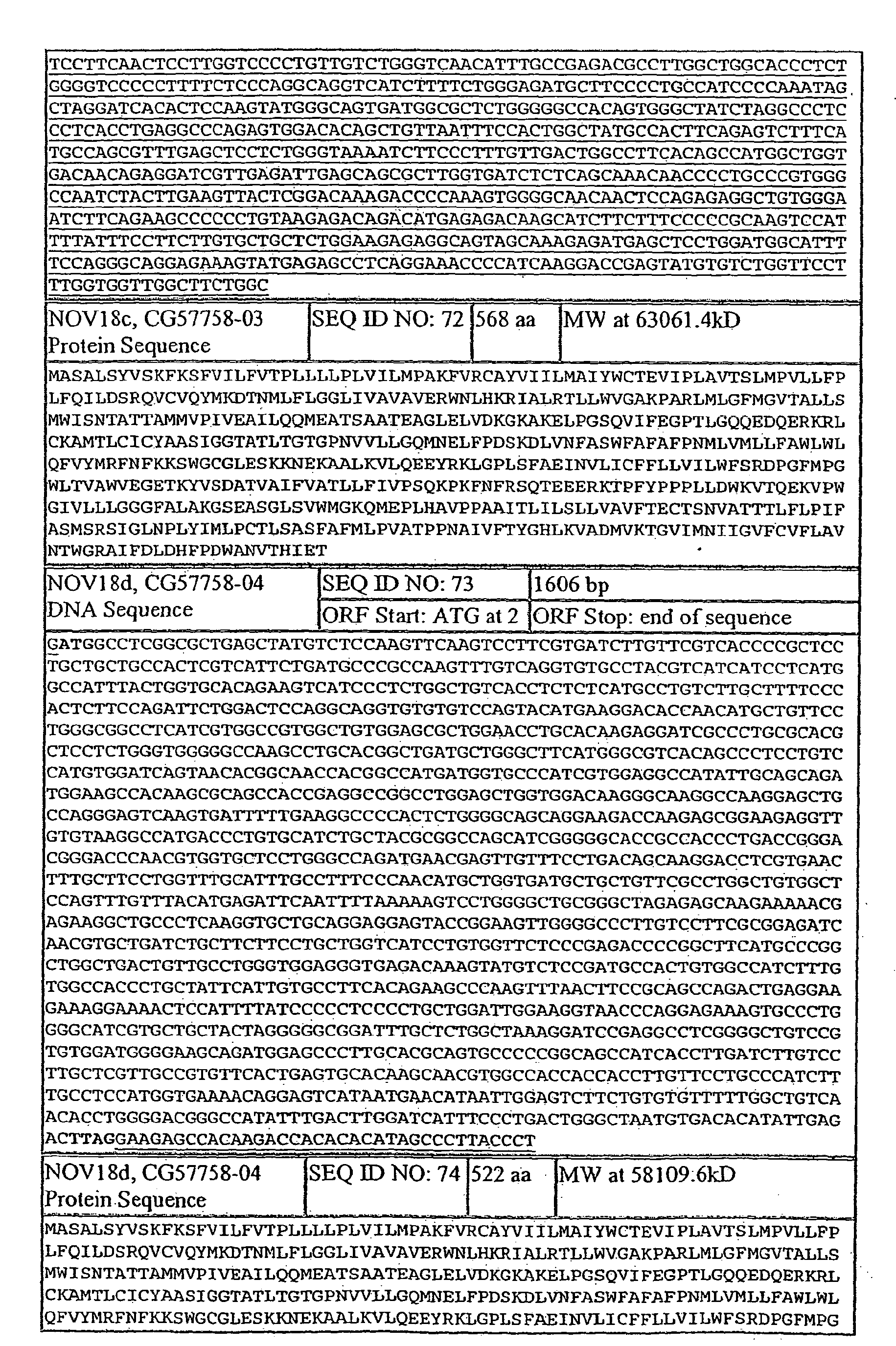

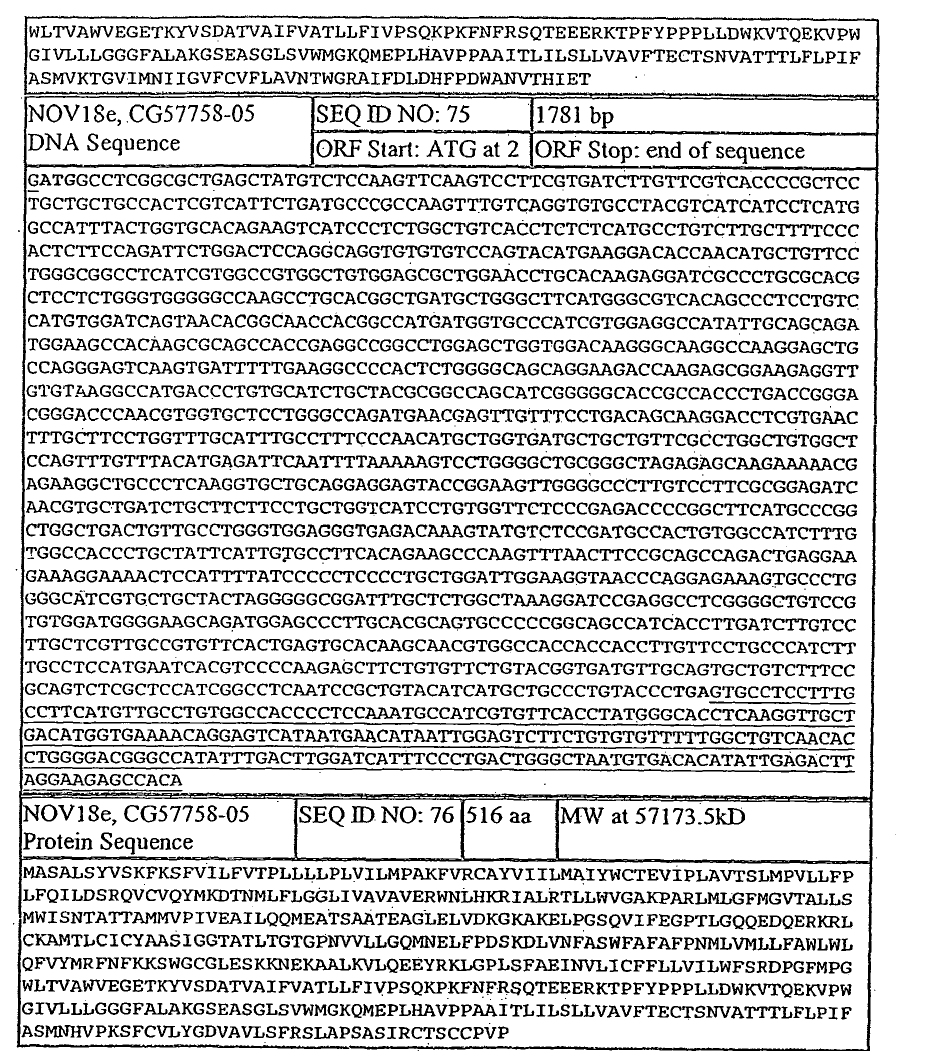

A ClustalW comparison of the above protein sequences yields the following sequence alignment shown in Table 18B.

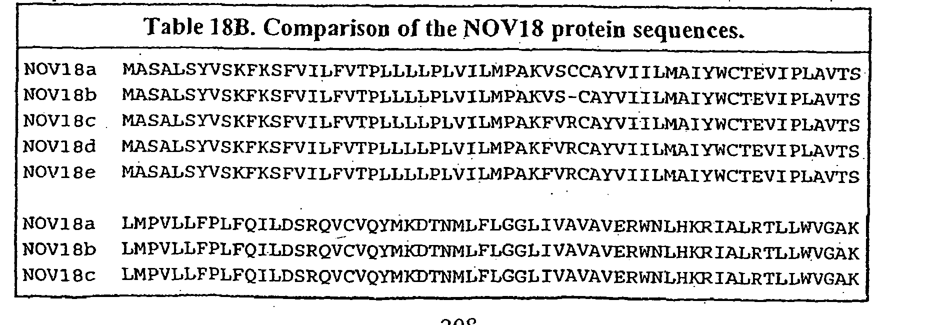

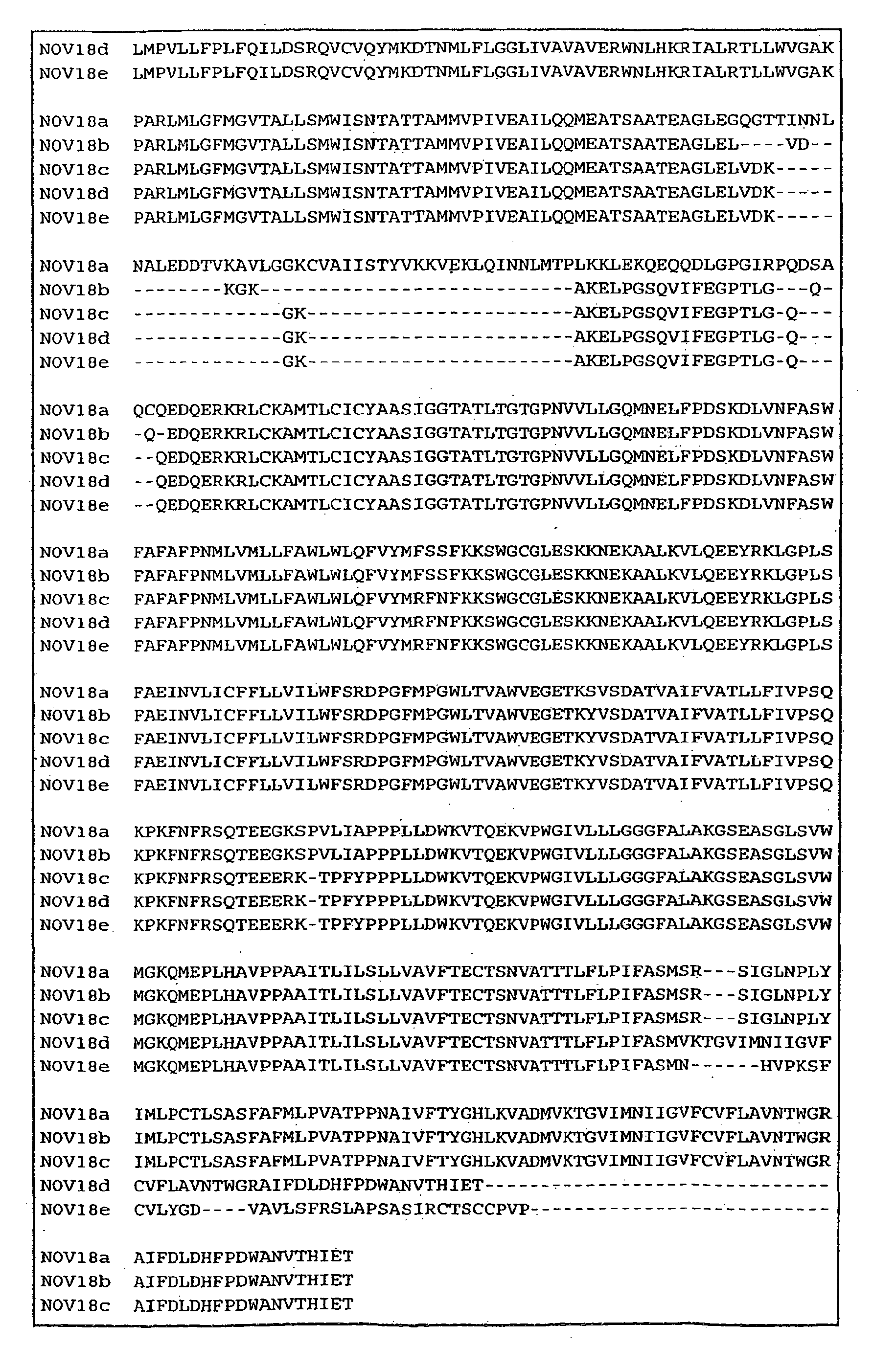

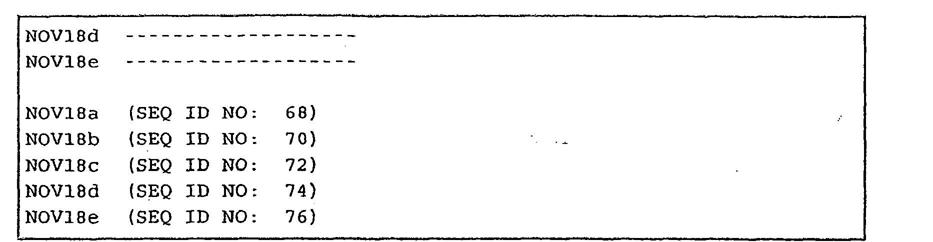

Further analysis of the NOV18a protein yielded the following properties shown in Table 18C.

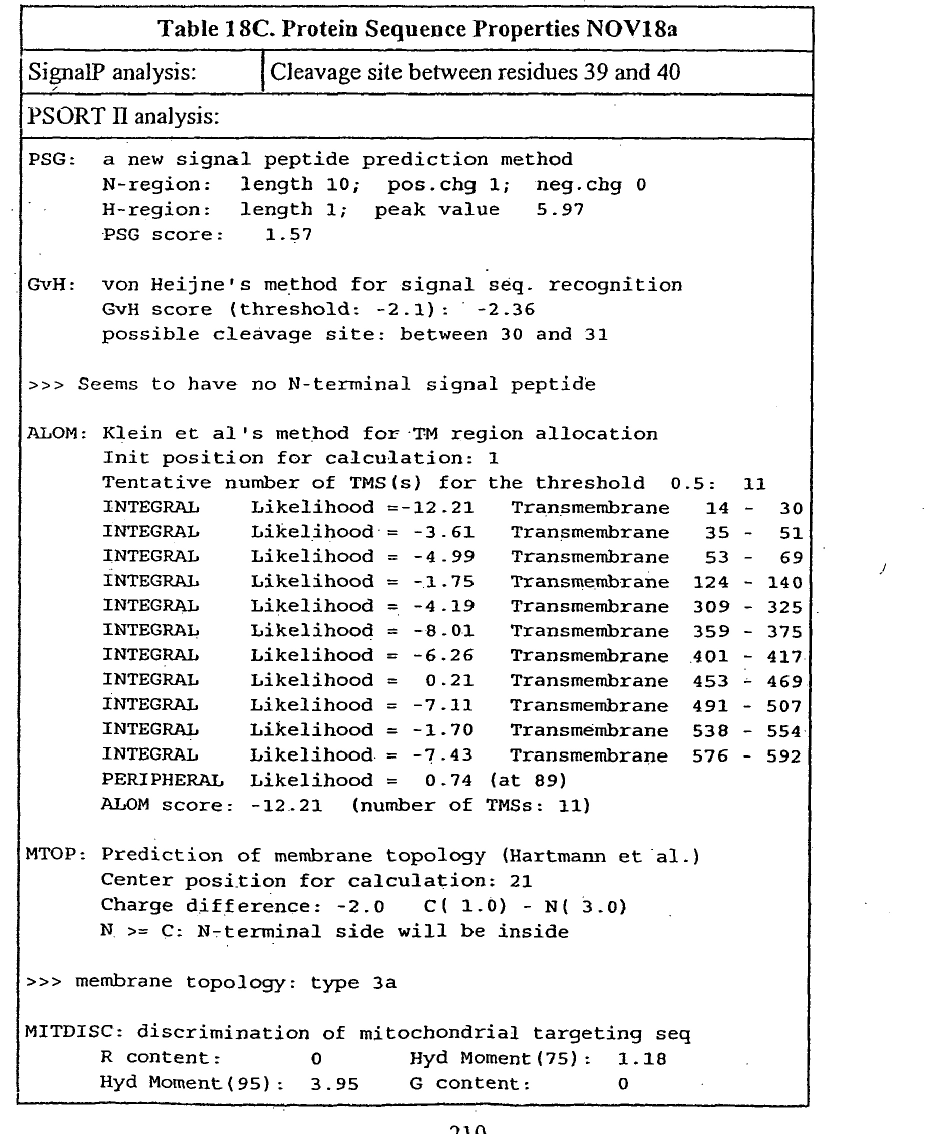

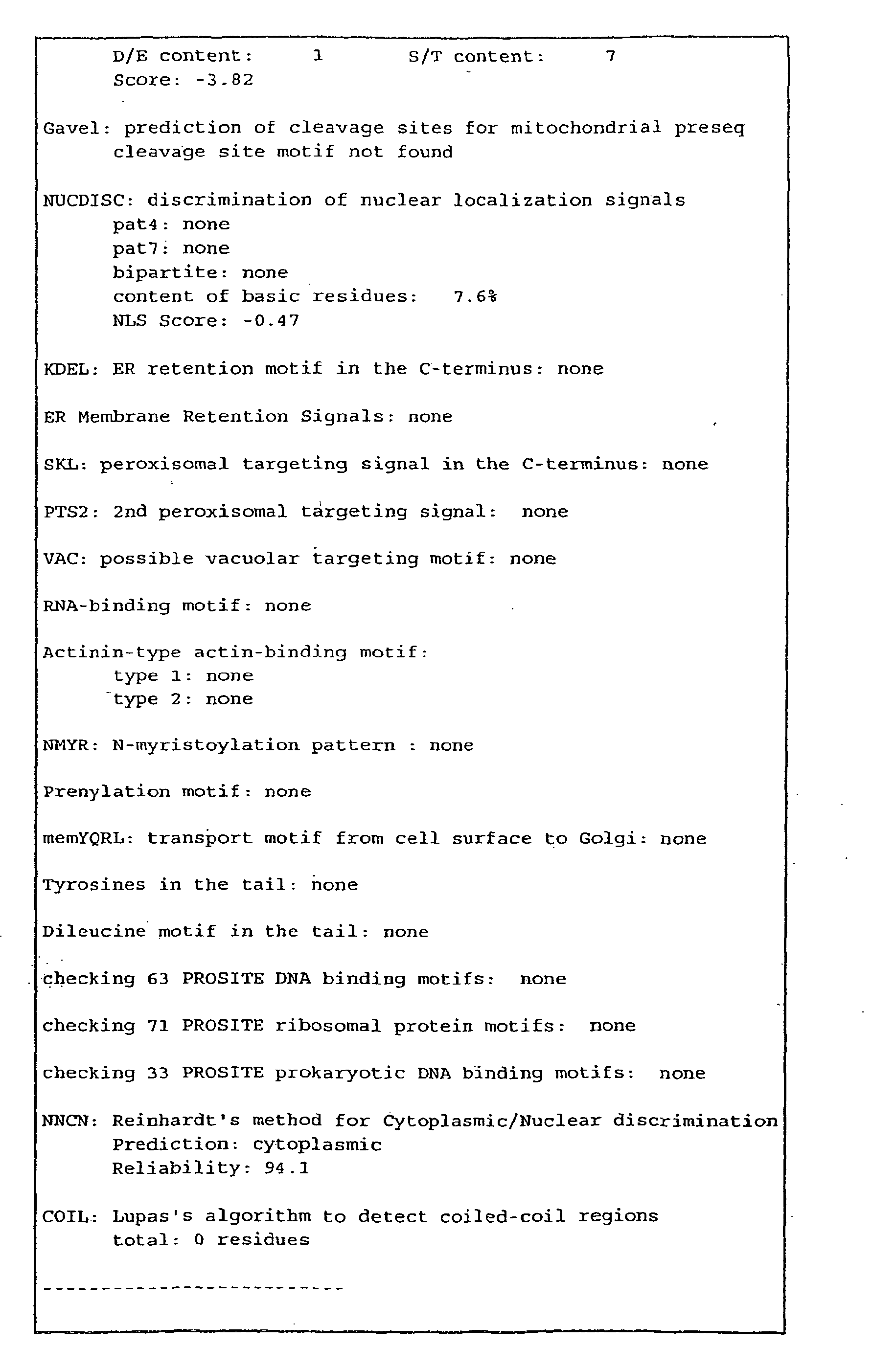

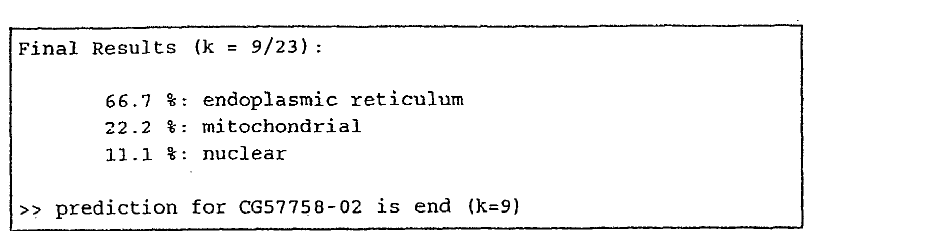

A search of the NOV18a protein against the Geneseq database, a proprietary database that contains sequences published in patents and patent publication, yielded several homologous proteins shown in Table 18D.

| **Table 18D. Geneseq Results for NOV18a** | | | | |
|---|---|---|---|---|
| **Geneseq Identifier** | **Protein/OrganismlLength [Patent #, Date]** | **NOV18a Residues/Match Residues** | **Identities/ Similarities for the Matched Region** | **Expect Value** |
| AAU79946 | Human transporter protein sequence - Homo sapiens, 568 aa. [US2002019028-A1, 14-FEB-2002] | 1..616 1..568 | 535/617(86%) 542/617 (87%) | 0.0 |
| AAB23625 | Human secreted protein SEQ ID NO: 50 - Homo sapiens, 627 aa. [WO200049134-A1 24-AUG-2000] | 10..614 9..623 | 253/623 (40%) 395/623 (62%) | e-141 |
| AAB36158 | Novel human transporter protein SEQ ID NO: 2 - Homo sapiens, 627 aa. [WO200065055-A2, 02-NOV-2000] | 10..614 9..623 | 253/623(40%) 395/623 (62%) | e-141 |
| ABB97450 | Novel human protein SEQ ID NO: 718 - Homo sapiens, 627 aa. [WO200222660-A2, 21-MAR-2002] | 10..614 9..623 | 253/623 (40%) 395/623 (62%) | e-141 |
| AAB42213 | Human ORFX ORF1977 polypeptide sequence SEQ ID NO:3954 - Homo sapiens, 627 aa. [WO200058473-A2, 05-OCT-2000] | 10..614 9..623 | 253/623(40%) 395/623 (62%) | e-141 |

In a BLAST search of public sequence databases, the NOV18a protein was found to have homology to the proteins shown in the BLASTP data in Table 18E.

| **Table 18E. Public BLASTP Results for NOV18a** | | | | |
|---|---|---|---|---|
| **Protein Accession Number** | **Protein/Organism/Length** | **NOV18a Residues/Match Residues** | **Identities/ Similarities for the Matched Portion** | **Expect Value** |
| AAN86530 | Na+-coupled citrate transporter protein - Homo sapiens (Human), 568 aa. | 1..616 1..568 | 537/617 (87%) 544/617 (88%) | 0.0 |
| Q8CJ44 | Sodium-coupled citrate transporter - Rattus norvegicus (Rat), 572 aa. | 1..616 1..572 | 422/620 (68%) 484/620 (78%) | 0.0 |
| 057661 | Intestinal sodium/LITHIUM-dependent dicarboxylate transporter (NA(+)/dicarboxylate cotransporter) - Xenopus laevis (African clawed frog), 622 aa. | 1..612 1..619 | 355/619 (57%) 472/619 (75%) | 0.0 |
| AAH44437 | Similar to solute carrier family 13, member 2 - Brachydanio rerio (Zebrafish) (Danio rerio), 613 aa. | 5..612 10..605 | 330/635 (51%) 443/635 (68%) | 0.0 |
| AAO27449 | Sodium dicarboxylate co-transporter - Didelphis marsupialis virginiana (North American opossum), 605 aa. | 11..611 11..587 | 324/602 (53%) 439/602 (72%) | 0.0 |

PFam analysis predicts that the NOV18a protein contains the domains shown in the Table 18F.

| **Table 18F. Domain Analysis of NOV18a** | | | |
|---|---|---|---|
| **Pfam Domain** | **NOV18a Match Region** | **Identities/ Similarities** for the Matched Region | **Expect Value** |
| Na_sulph_symp | 6..602 | 163/647 (25%) 447/647 (69%) | 3.5e-134 |

### Example 19.

The NOV19 clone was analyzed, and the nucleotide and encoded polypeptide sequences are shown in Table 19A.

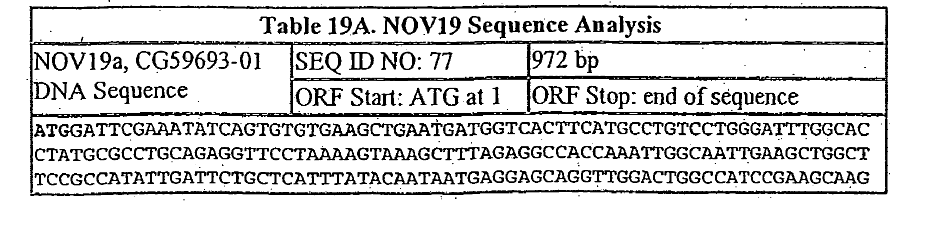

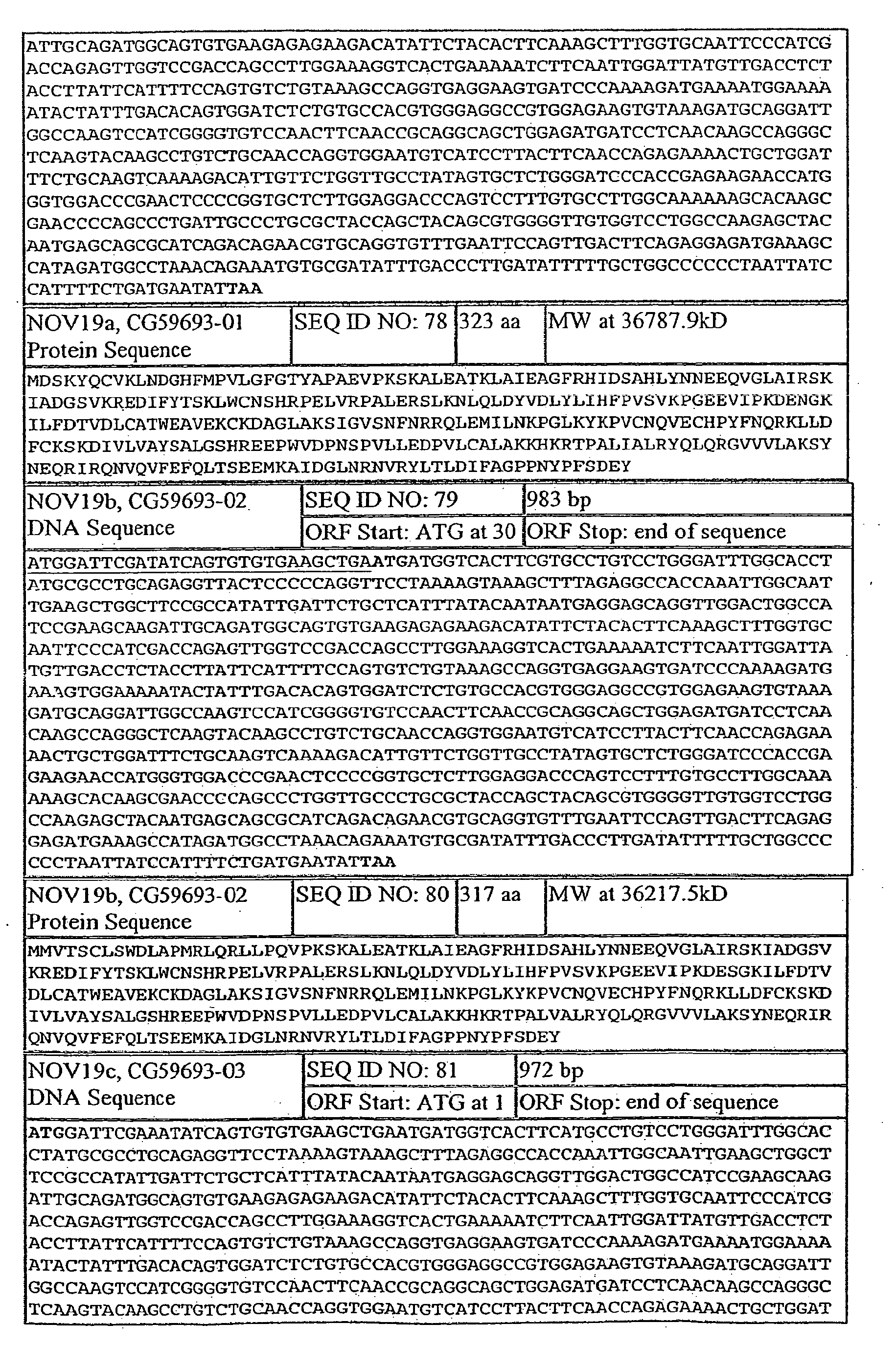

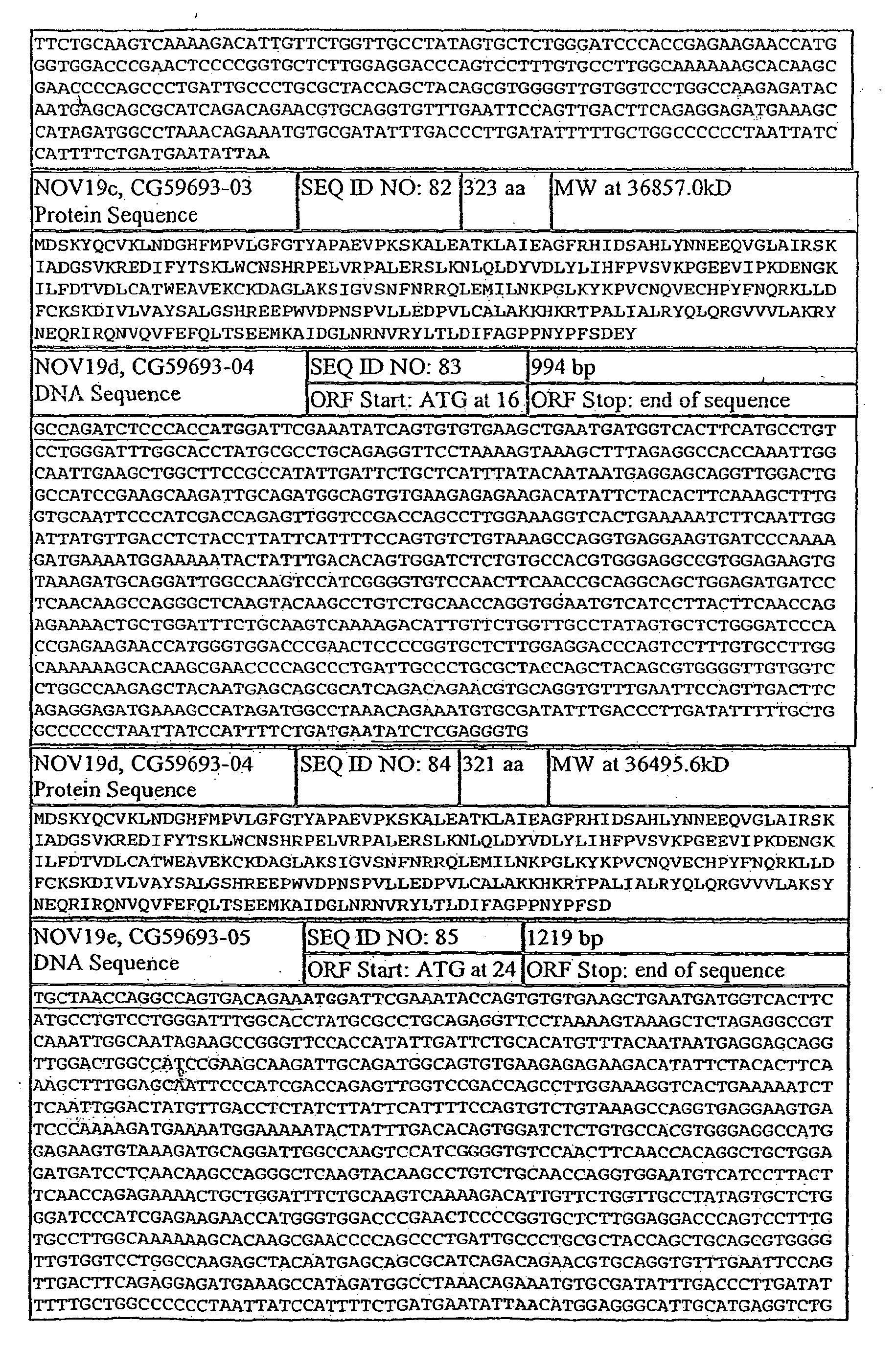

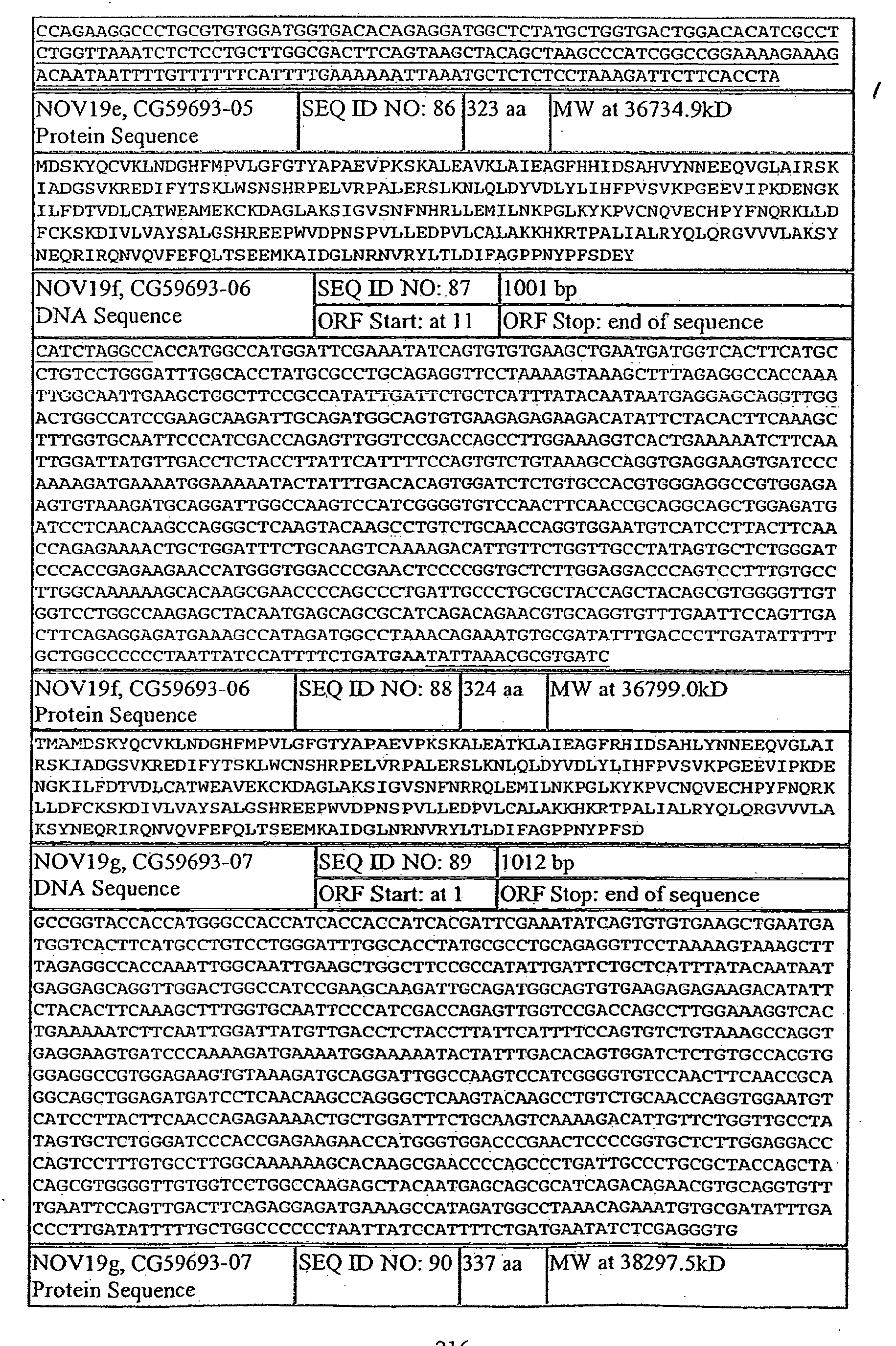

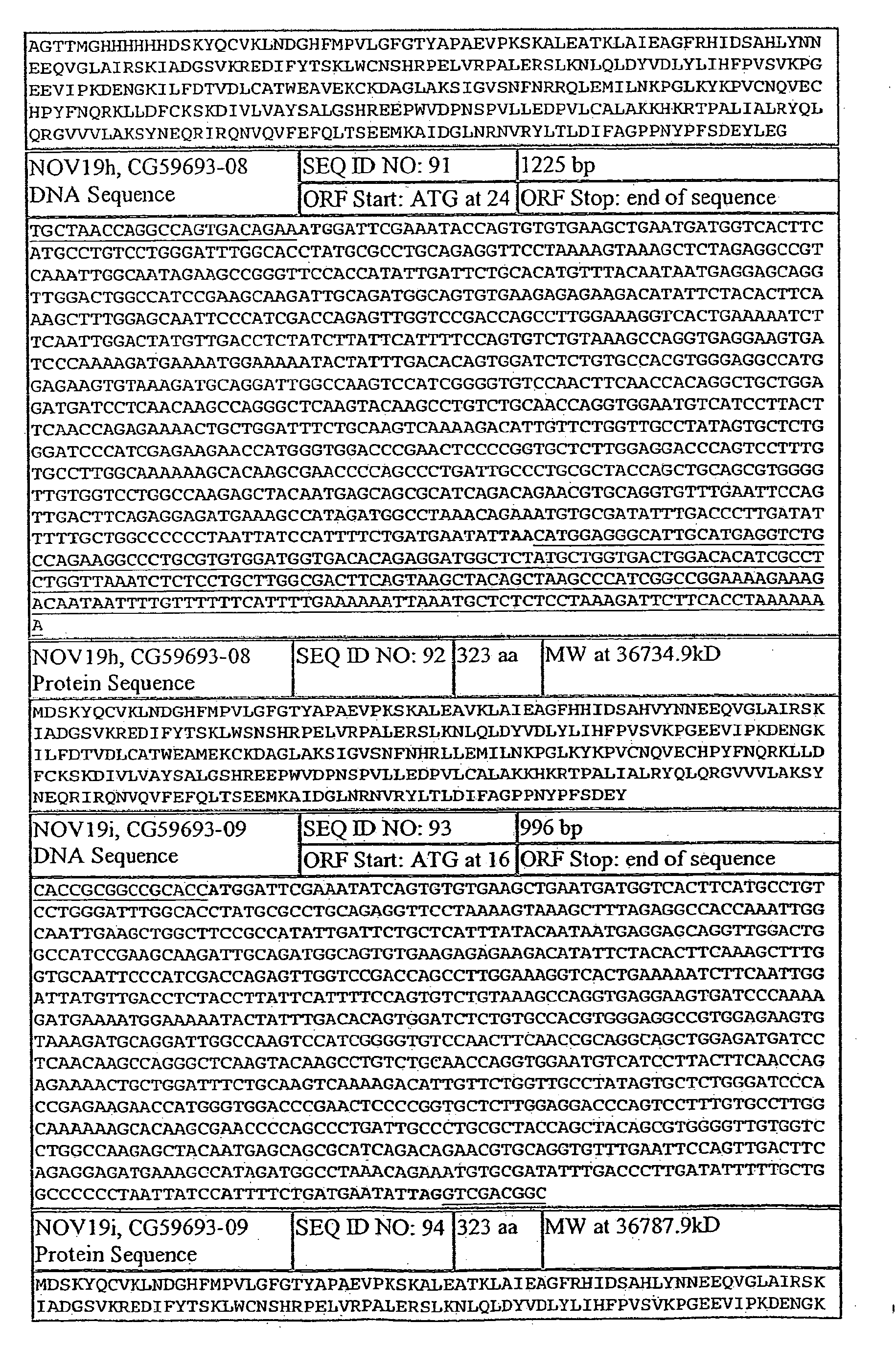

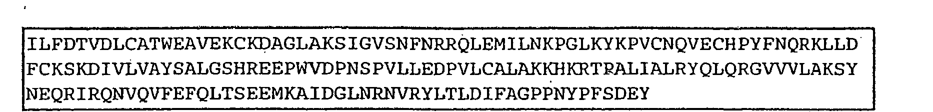

A ClustalW comparison of the above protein sequences yields the following sequence alignment shown in Table 19B.

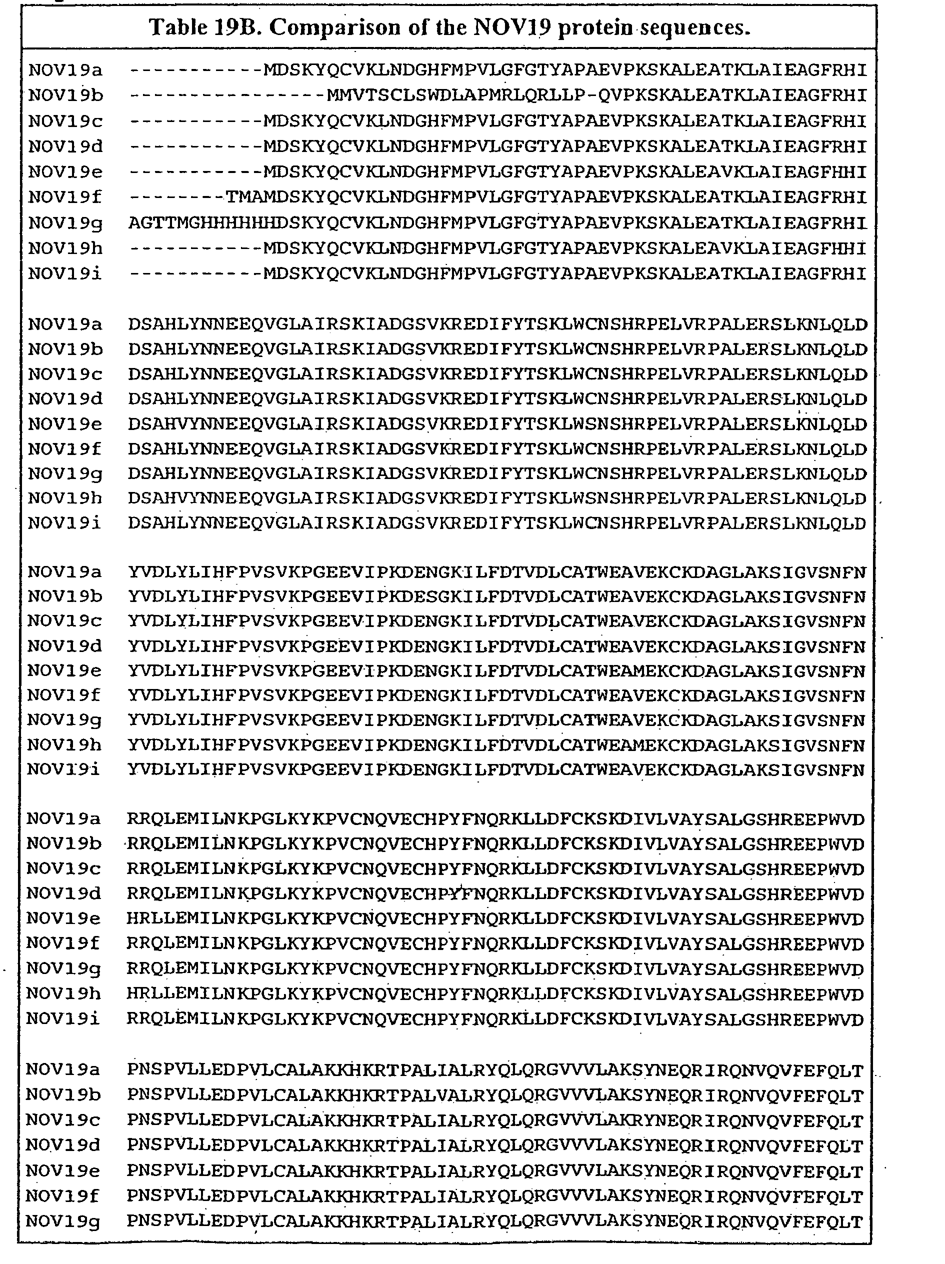

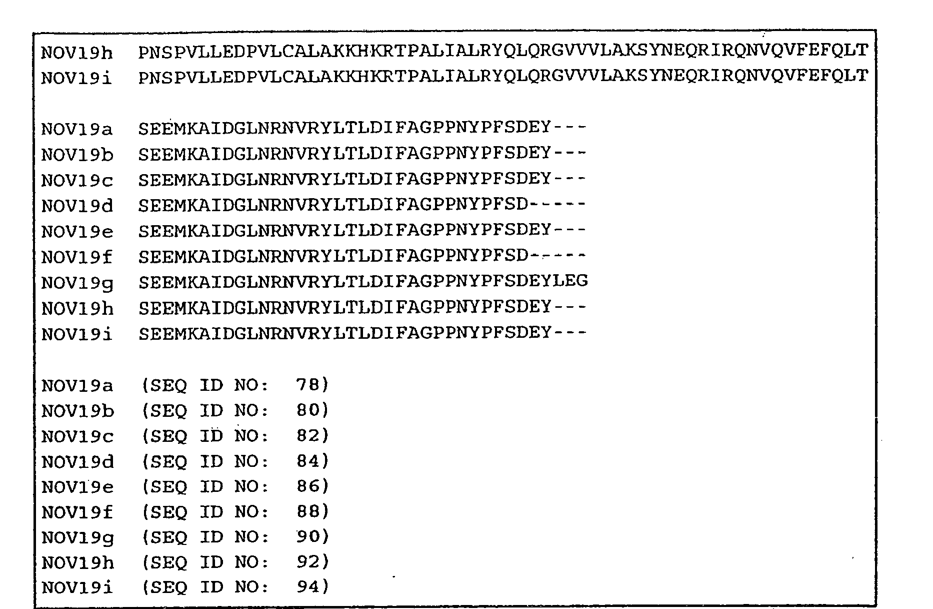

Further analysis of the NOV19a protein yielded the following properties shown in Table 19C.

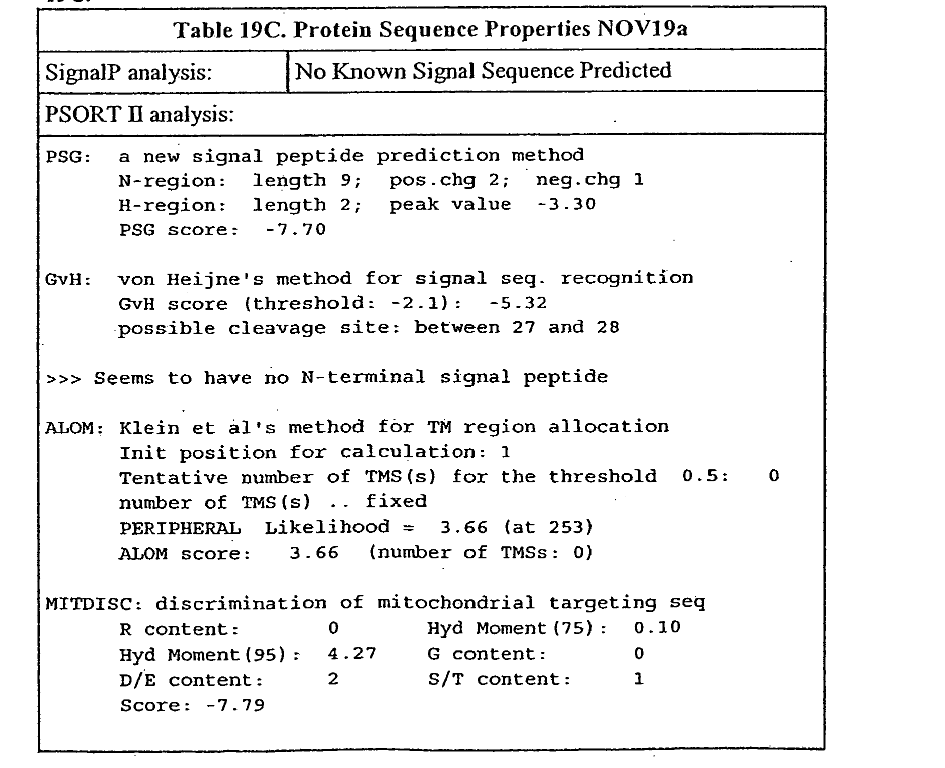

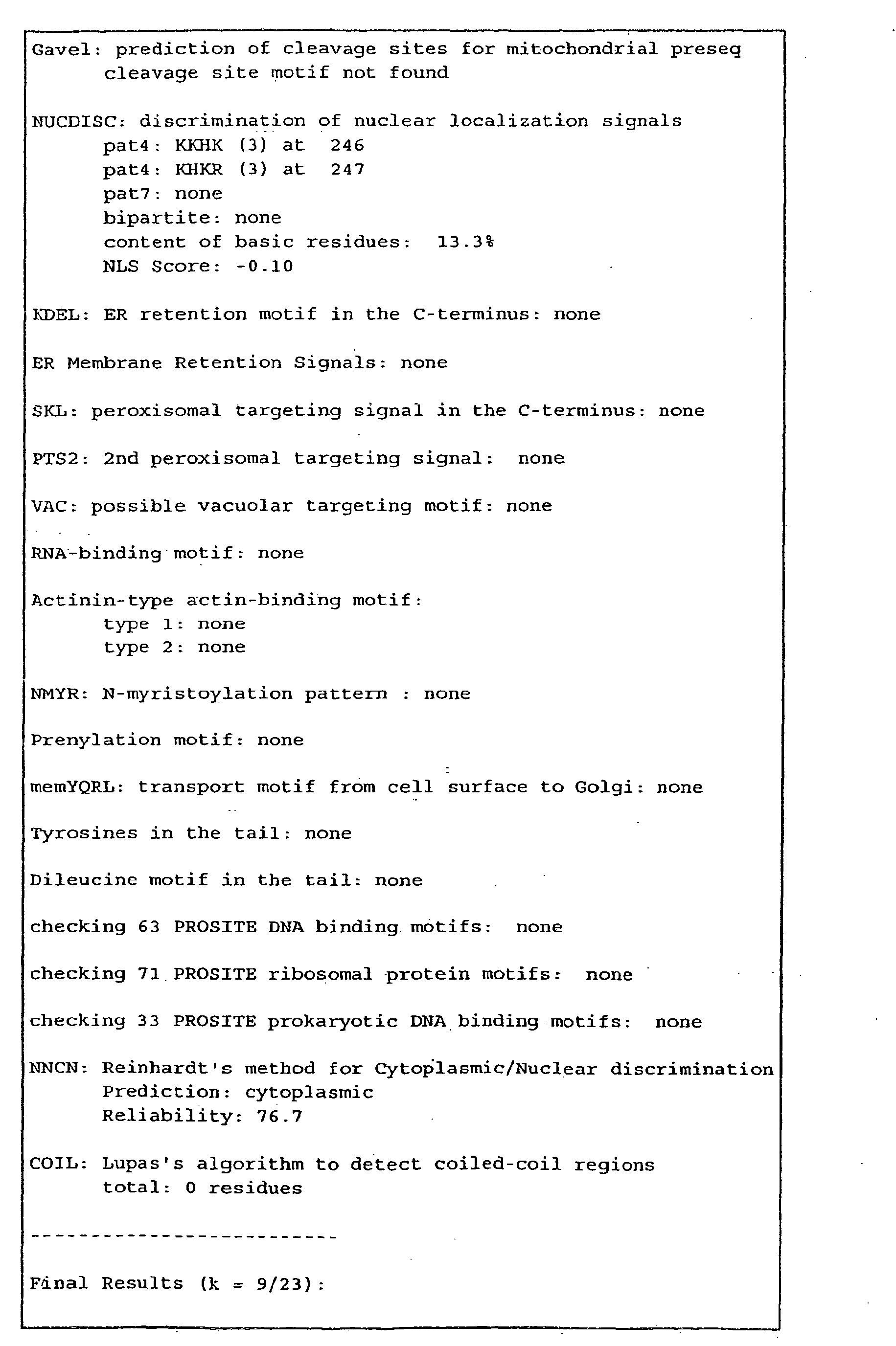

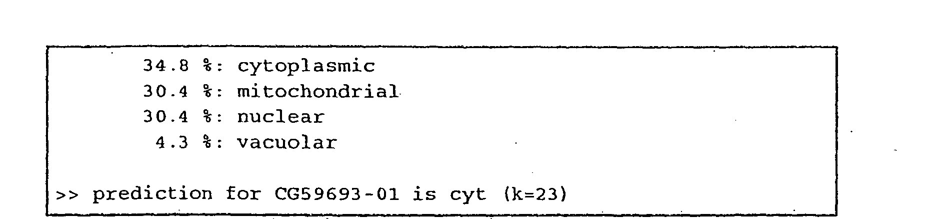

A search of the NOV19a protein against the Geneseq database, a proprietary database that contains sequences published in patents and patent publication, yielded several homologous proteins shown in Table 19D.

| **Table 19D. Geneseq Results for NOV19a** | | | | |
|---|---|---|---|---|
| **Geneseq Identifier** | **Protein/Organism/Length [Patent #, Date]** | **NOV19a Residues/ Match Residues** | **Identities/Similarities for the Matched Region** | **Expect Value** |
| AAB43444 | Human cancer associated protein sequence SEQ ID NO:889 - Homo sapiens, 336 aa. [WO200055350-A1, 21-SEP-2000] | 1..323 14..336 | 318/323 (98%) 318/323 (98%) | 0.0 |
| AAU85559 | Clone #59314 (L1426P) of lung tumour protein - Homo sapiens, 323 aa. [WO200204514-A2, 17-JAN-2002] | 1..323 1..323 | 311/323 (96%) 316/323 (97%) | 0.0 |
| ABB75050 | Human lung tumour L773P recombinant protein sequence SEQ ID NO:433 - Homo sapiens, 371 aa. [WO200200174-A2, 03-JAN-2002] | 29..323 77..371 | 288/295 (97%) 290/295 (97%) | e-168 |
| ABB74958 | Human lung tumour L773P protein sequence SEQ ID NO:172 - Homo sapiens, 364 aa. [WO200200174-A2, 03-JAN-2002] | 29..323 70..364 | 288/295 (97%) 290/295(97%) | e-168 |
| AAU85520 | L773P lung tumour protein - Homo sapiens, 364 aa. [WO200204514-A2, 17-JAN-2002] | 29..323 70..364 | 288/295 (97%) 290/295 (97%) | e-168 |

In a BLAST search of public sequence databases, the NOV19a protein was found to have homology to the proteins shown in the BLASTP data in Table 19E.

| **Table 19E. Public BLASTP Results for NOV19a** | | | | |
|---|---|---|---|---|
| **Protein Accession Number** | **Protein/Organism/Length** | **NOV19a Residues/ Match Residues** | **Identities/ Similarities for the Matched Portion** | **Expect Value** |
| Q04828 | Aldo-keto reductase family 1 member C1 (EC 1.1.1.-) (Trans-1,2-dihydrobenzene-1,2-diol dehydrogenase) (EC 1.3.1.20) (High-affinity hepatic bile acid-binding protein) (HBAB) (Chlordecone reductase homolog HAKRC) (Dihydrodiol dehydrogenase 2) (DD2) (20 alpha- hydroxysteroid dehydrogenase) - Homo sapiens (Human), 323 aa. | 1..323 1..323 | 323/323 (100%) 323/323 (100%) | 0.0 |
| P52895 | Aldo-keto reductase family 1 member C2 (EC 1.1.1.-) (Trans-1,2-dihydrobenzene-1,2-diol dehydrogenase) (EC 1.3.1.20) (Chlordecone reductase homolog HAKRD) (Dihydrodiol dehydrogenase/bile acid-binding protein) (DD/BABP) (Dihydrodiol dehydrogenase 2) (DD2) - Homo sapiens (Human), 323 aa. | 1..323 1..323 | 316/323(97%) 318/323 (97%) | 0.0 |
| I73676 | chlordecone reductase homolog (clone HAKRd) - human, 323 aa. | 1..323 1..323 | 313/323 (96%) 317/323 (97%) | 0.0 |
| I73675 | chlordecone reductase homolog (clone HAKRc) - human, 320 aa (fragment). | 4..323 1..320 | 312/320 (97%) 313/320 (97%) | 0.0 |
| Q95JH6 | 3(20)alpha-hydroxysteroid/dihydrodiol/indanol dehydrogenase (EC 1.1.1.112) - Macaca fuscata (Japanese macaque), 323 aa. | 1..323 1..323 | 304/323 (94%) 319/323 (98%) | 0.0 |

PFam analysis predicts that the NOV19a protein contains the domains shown in the Table 19F.

| **Table 19F. Domain Analysis of NOV19a** | | | |
|---|---|---|---|
| **Pfam Domain** | **NOV19a Match Region** | **Identities/ Similarities for the Matched Region** | **Expect Value** |
| aldo_ket_red | 10..303 | 164/369 (44%) 274/369 (74%) | 3.4e-156 |

### Example 20.

The NOV20 clone was analyzed, and the nucleotide and encoded polypeptide sequences are shown in Table 20A.

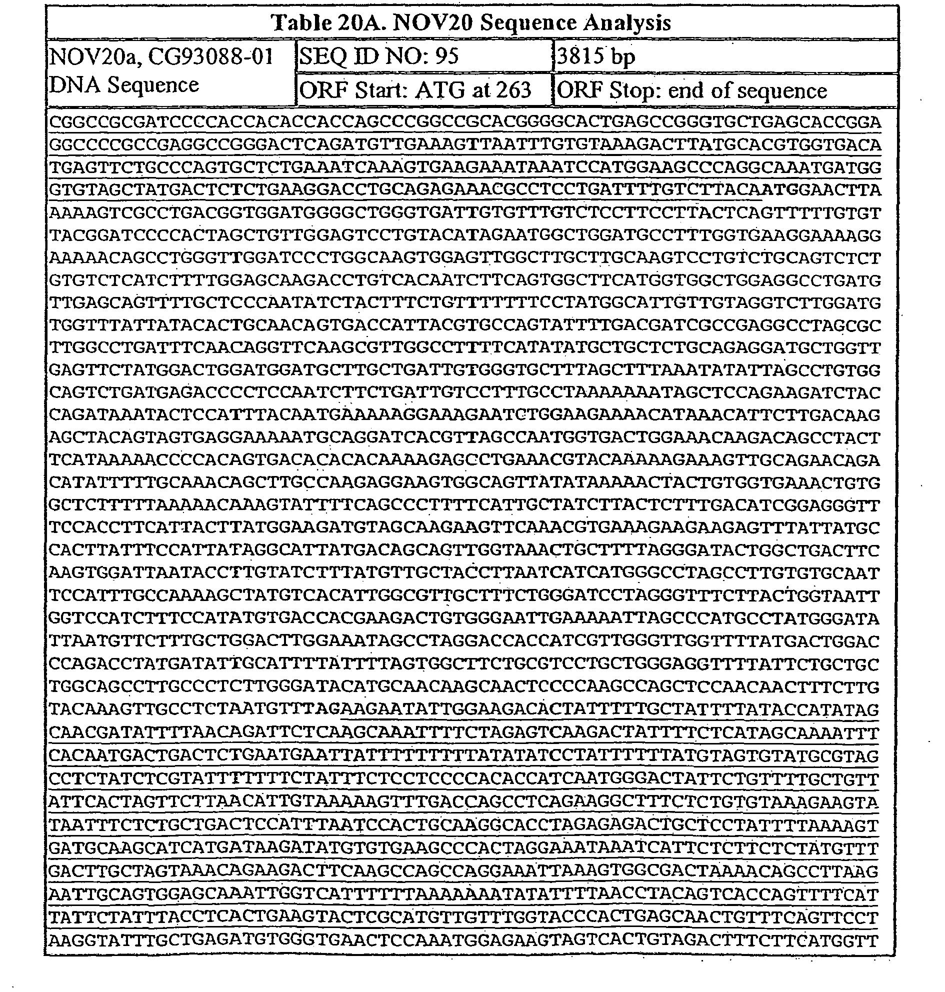

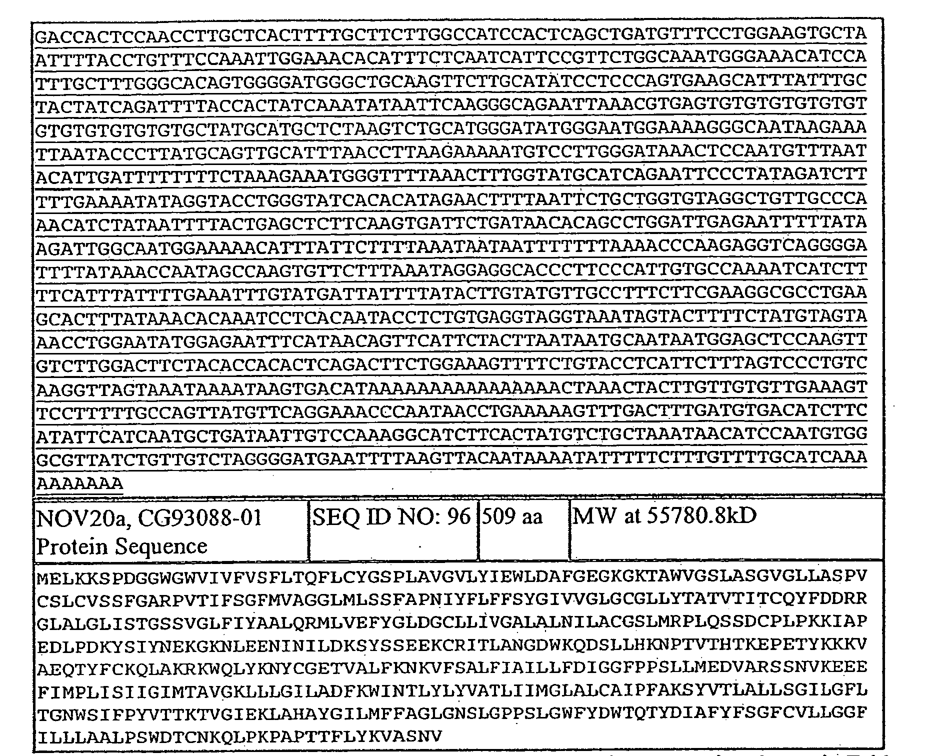

Further analysis of the NOV20a protein yielded the following properties shown in Table 20B.

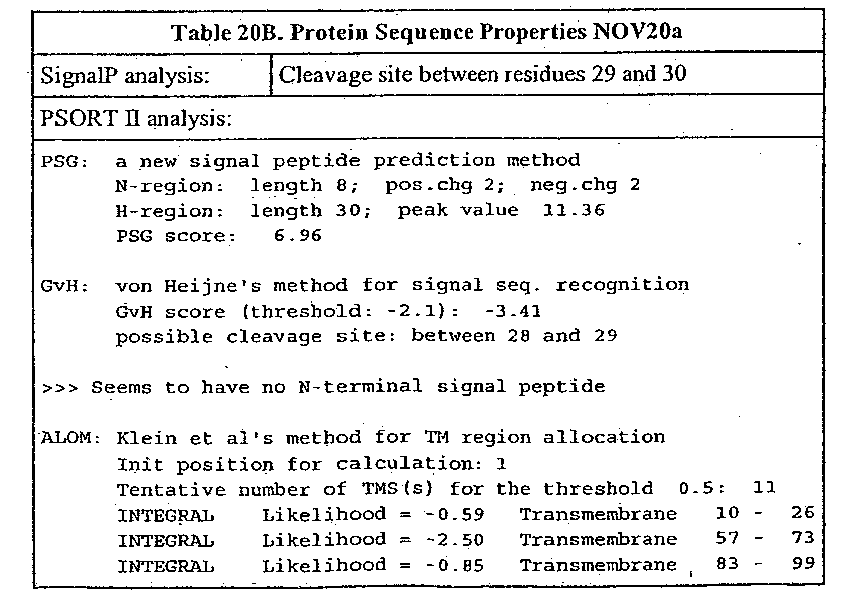

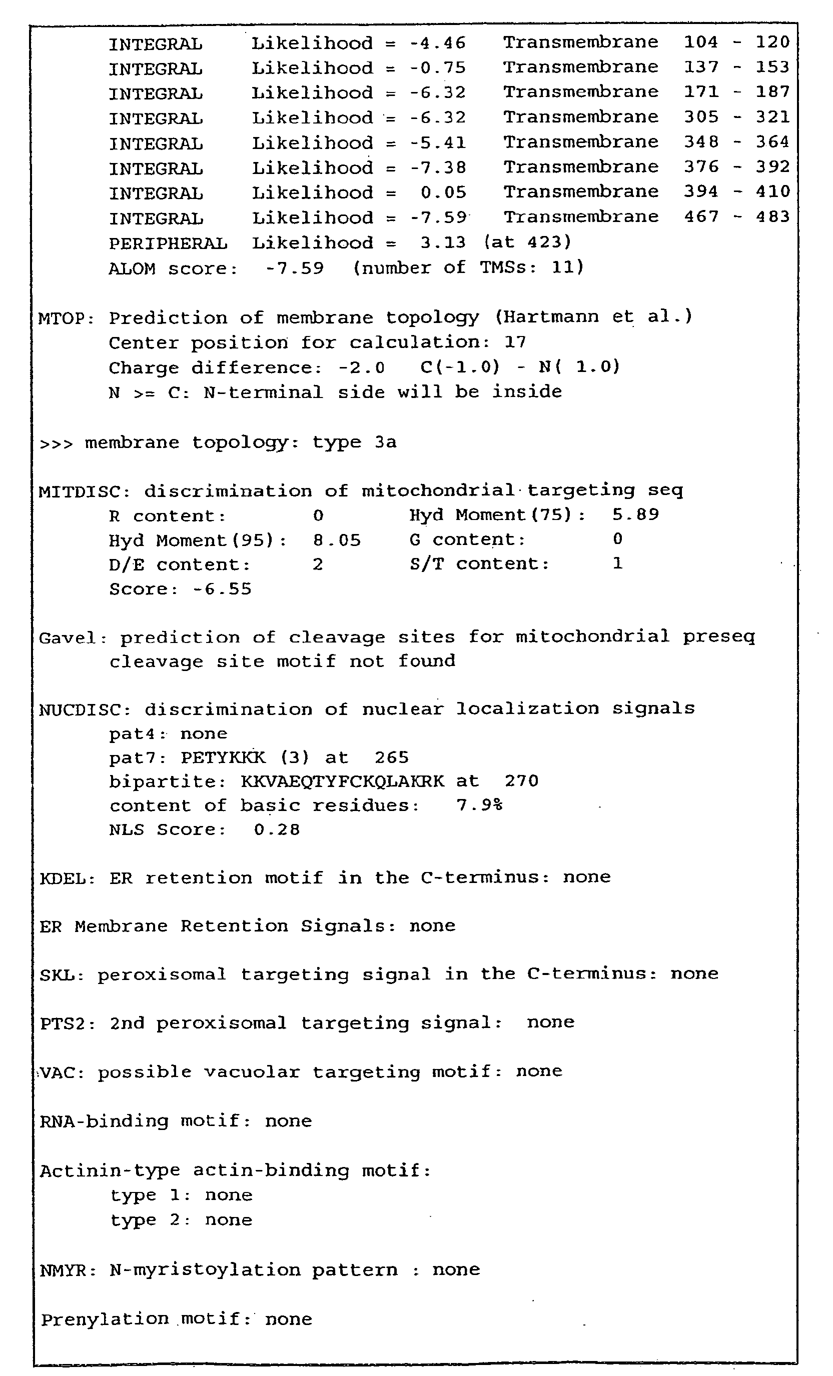

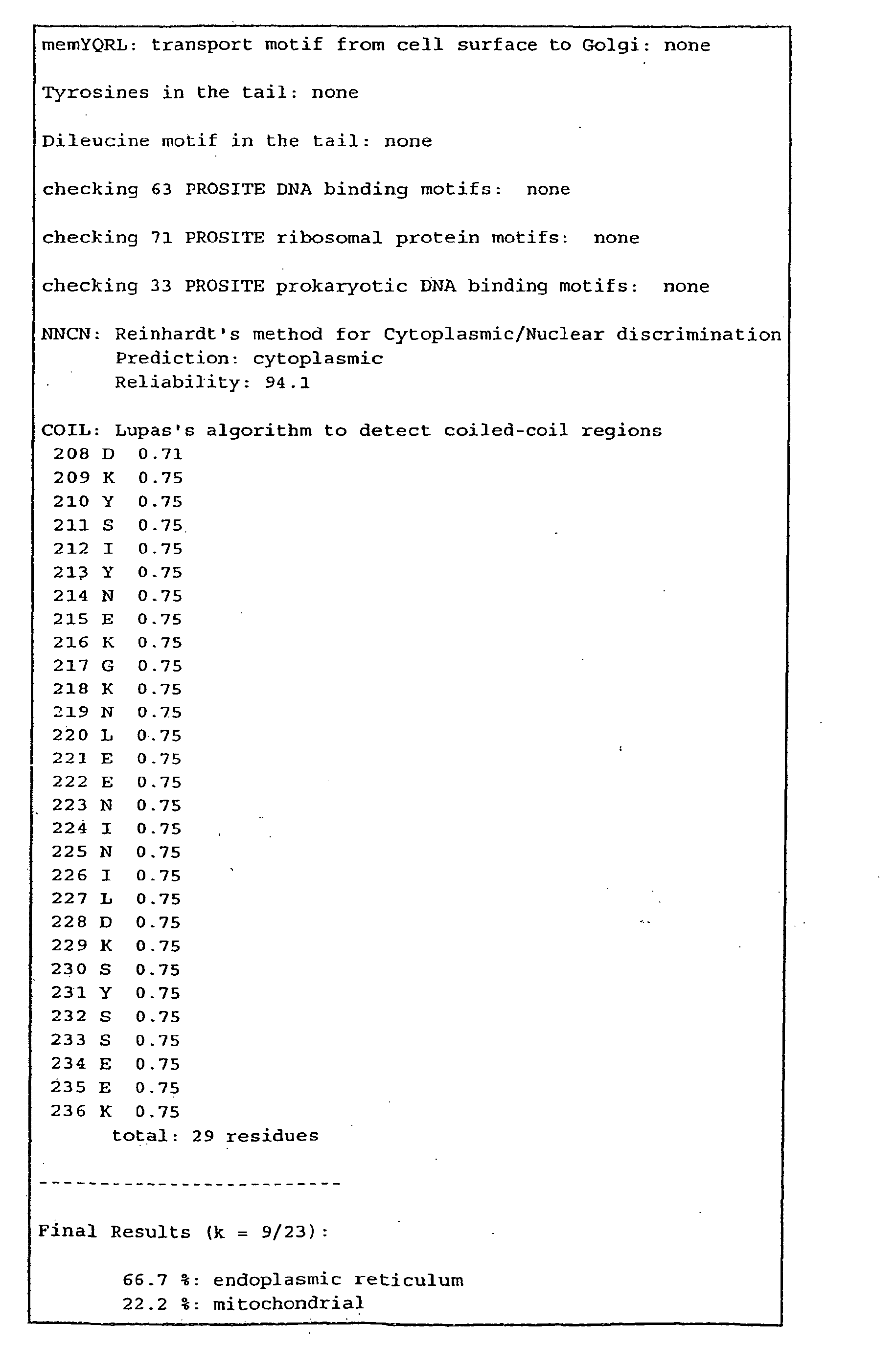

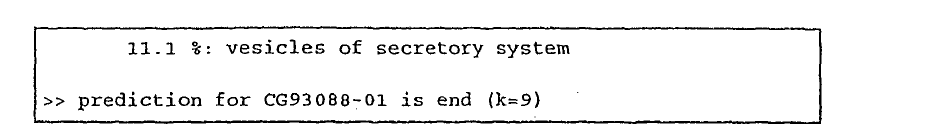

A search of the NOV20a protein against the Geneseq database, a proprietary database that contains sequences published in patents and patent publication, yielded several homologous proteins shown in Table 20C.

| **Table 20C. Geneseq Results for NOV20a** | | | | |
|---|---|---|---|---|
| **Geneseq Identifier** | **Protein/Organism/Length [Patent #, Date]** | **NOV20a Residues/ Match Residues** | **Identities/Similarities for the Matched Region** | **Expect Value** |
| ABU12075 | Human NOV19a CG93088-01 protein SEQ ID 70 - Homo sapiens, 509 aa. [WO200281625-A2, 17-OCT-2002] | 1..509 1..509 | 509/509 (100%) 509/509 (100%) | 0.0 |
| ABG61543 | Human transporter and ion channel, TRICH13, Incyte ID 4027693CD1 - Homo sapiens, 509 aa. [WO200240541-A2, 23-MAY-2002] | 1..509 1..509 | 507/509(99%) 508/509 (99%) | 0.0 |
| ABB83901 | Human transporter protein SEQ ID NO 2 - Homo sapiens, 509 aa. [WO200257310-A2, 25-JUL-2002] | 1..509 1..509 | 507/509 (99%) 508/509 (99%) | 0.0 |
| ABP51421 | Human MDDT SEQ ID NO 443 - Homo sapiens, 529 aa. [WO200240715-A2,23-MAY-2002] | 3..507 38..527 | 168/509 (33%) 265/509 (52%) | 3e-74 |
| ABG33043 | Human 25466 transporter protein - Homo sapiens, 510 aa. [EP1233024-A2,21-AUG-2002] | 3..480 22..490 | 164/482 (34%) 256/482 (53%) | 3e-74 |

In a BLAST search of public sequence databases, the NOV20a protein was found to have homology to the proteins shown in the BLASTP data in Table 20D.

| **Table 20D. Public BLASTP Results for NOV20a** | | | | |
|---|---|---|---|---|
| **Protein Accession Number** | **Protein/Organism/Length** | **NOV20a Residues/ Match Residues** | **Identities/ Similarities for the Matched Portion** | **Expect Value** |
| BAB23369 | Adult male lung cDNA, RIKEN full-length enriched library, clone:1200003C15 product:hypothetical protein, full insert sequence - Mus musculus (Mouse), 508 aa. | 1..509 1..508 | 445/511 (87%) 471/511 (92%) | 0.0 |
| Q9UFH8 | Hypothetical protein - Homo sapiens (Human), 157 aa (fragment). | 353..509 1..157 | 155/157 (98%) 156/157 (98%) | 3e-87 |
| Q9CPZ7 | 4930425B13Rik protein (1200003C15Rik protein) - Mus musculus (Mouse), 159 aa. | 352..509 1..159 | 148/159 (93%) 152/159(95%) | 2e-82 |
| CAD52855 | Sequence 1 from Patent EP1233024 - Homo sapiens (Human), 510 aa. | 3..480 22..490 | 164/482 (34%) 256/482 (53%) | 1e-73 |
| AAH47967 | Similar to solute carrier family 16 (monocarboxylic acid transporters), member 6 - Xenopus laevis (African clawed frog), 533 aa. | 5..509 22..499 | 140/509 (27%) 234/509 (45%) | 4e-50 |

PFam analysis predicts that the NOV20a protein contains the domains shown in the Table 20E.

| **Table 20E. Domain Analysis of NOV20a** | | | |
|---|---|---|---|
| **Pfam Domain** | **NOV20a Match Region** | **Identities/ Similarities for the Matched Region** | **Expect Value** |
| sugar_tr | 11..456 | 72/548 (13%) 278/548 (51%) | 0.17 |

### Example B: Sequencing Methodology and Identification of NOVX Clones

**1. GeneCalling™ Technology:** This is a proprietary method of performing differential gene expression profiling between two or more samples developed at CuraGen and described by Shimkets, et al., "Gene expression analysis by transcript profiling coupled to a gene database query" Nature Biotechnology 17:198-803 (1999). cDNA was derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then digested with up to as many as 120 pairs of restriction enzymes and pairs of linker-adaptors specific for each pair of restriction enzymes were ligated to the appropriate end. The restriction digestion generates a mixture of unique cDNA gene fragments. Limited PCR amplification is performed with primers homologous to the linker adapter sequence where one primer is biotinylated and the other is fluorescently labeled. The doubly labeled material is isolated and the fluorescently labeled single strand is resolved by capillary gel electrophoresis. A computer algorithm compares the electropherograms from an experimental and control group for each of the restriction digestions. This and additional sequence-derived information is used to predict the identity of each differentially expressed gene fragment using a variety of genetic databases. The identity of the gene fragment is confirmed by additional, gene-specific competitive PCR or by isolation and sequencing of the gene fragment.
**2. SeqCalling™ Technology:** cDNA was derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then sequenced using CuraGen's proprietary SeqCalling technology. Sequence traces were evaluated manually and edited for corrections if appropriate. cDNA sequences from all samples were assembled together, sometimes including public human sequences, using bioinformatic programs to produce a consensus sequence for each assembly. Each assembly is included in CuraGen Corporation's database. Sequences were included as components for assembly when the extent of identity with another component was at least 95% over 50 bp. Each assembly represents a gene or portion thereof and includes information on variants, such as splice forms single nucleotide polymorphisms (SNPs), insertions, deletions and other sequence variations.
3. **PathCalling™ Technology:** The NOVX nucleic acid sequences are derived by laboratory screening of cDNA library by the two-hybrid approach. cDNA fragments covering either the full length of the DNA sequence, or part of the sequence, or both, are sequenced. In silico prediction was based on sequences available in CuraGen Corporation's proprietary sequence databases or in the public human sequence databases, and provided either the full length DNA sequence, or some portion thereof.
   The laboratory screening was performed using the methods summarized below:
   cDNA libraries were derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then directionally cloned into the appropriate two-hybrid vector (Ga14-activation domain (Ga14-AD) fusion). Such cDNA libraries as well as commercially available cDNA libraries from Clontech (Palo Alto, CA) were then transferred from E.coli into a CuraGen Corporation proprietary yeast strain (disclosed in U. S. Patents 6,057,101 and 6,083,693, incorporated herein by reference in their entireties).
   Gal4-binding domain (GaI4-BD) fusions of a CuraGen Corportion proprietary library of human sequences was used to screen multiple Ga14-AD fusion cDNA libraries resulting in the selection of yeast hybrid diploids in each of which the Ga14-AD fusion contains an individual cDNA. Each sample was amplified using the polymerase chain reaction (PCR) using non-specific primers at the cDNA insert boundaries. Such PCR product was sequenced; sequence traces were evaluated manually and edited for corrections if appropriate. cDNA sequences from all samples were assembled together, sometimes including public human sequences, using bioinformatic programs to produce a consensus sequence for each assembly. Each assembly is included in CuraGen Corporation's database. Sequences were included as components for assembly when the extent of identity with another component was at least 95% over 50 bp. Each assembly represents a gene or portion thereof and includes information on variants, such as splice forms single nucleotide polymorphisms (SNPs), insertions, deletions and other sequence variations.
   Physical clone: the cDNA fragment derived by the screening procedure, covering the entire open reading frame is, as a recombinant DNA, cloned into pACT2 plasmid (Clontech) used to make the cDNA library. The recombinant plasmid is inserted into the host and selected by the yeast hybrid diploid generated during the screening procedure by the mating of both CuraGen Corporation proprietary yeast strains N106' and YULH (U. S. Patents 6,057,101 and 6,083,693).
4. RACE: Techniques based on the polymerase chain reaction such as rapid amplification of cDNA ends (RACE), were used to isolate or complete the sequence of the cDNA of the invention. Usually multiple clones were sequenced from one or more human samples to derive the sequences for fragments. Various human tissue samples from different donors were used for the RACE reaction. The sequences derived from these procedures were included in the SeqCalling Assembly process described in preceding paragraphs.
5. Exon Linking: The NOVX target sequences identified in the present invention were subjected to the exon linking process to confirm the sequence. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the exons to closely related human sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The PCR product derived from exon linking was cloned into the pCR2.1 vector from Invitrogen. The resulting bacterial clone has an insert covering the entire open reading frame cloned into the pCR2.1 vector. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. These procedures provide the sequence reported herein.
6. Physical Clone: Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

The PCR product derived by exon linking, covering the entire open reading frame, was cloned into the pCR2.1 vector from Invitrogen to provide clones used for expression and screening purposes.

### Example C. Quantitative expression analysis of clones in various cells and tissues

The quantitative expression of various clones was assessed using microtiter plates containing RNA samples from a variety of normal and pathology-derived cells, cell lines and tissues using real time quantitative PCR (RTQ PCR). RTQ PCR was performed on an Applied Biosystems ABI PRISM® 7700 or an ABI PRISM® 7900 HT Sequence Detection System. Various collections of samples are assembled on the plates, and referred to as Panel 1 (containing normal tissues and cancer cell lines), Panel 2 (containing samples derived from tissues from normal and cancer sources), Panel 3 (containing cancer cell lines), Panel 4 (containing cells and cell lines from normal tissues and cells related to inflammatory conditions), Panel 5D/5I (containing human tissues and cell lines with an emphasis on metabolic diseases), Al_comprebensive_panel (containing normal tissue and samples from autoinflammatory diseases), Panel CNSD.01 (containing samples from normal and diseased brains) and CNS_neurodegeneration_panel (containing samples from normal and Alzheimer's diseased brains).

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (Applied Biosystems; Catalog No. 4309169) and gene-specific primers according to the manufacturer's instructions.

In other cases, non-normalized RNA samples were converted to single strand cDNA (sscDNA) using Superscript II (Invitrogen Corporation; Catalog No. 18064-147) and random hexamers according to the manufacturer's instructions. Reactions containing up to 10 µg of total RNA were performed in a volume of 20 µl and incubated for 60 minutes at 42°C. This reaction can be scaled up to 50 µg of total RNA in a final volume of 100 µl. sscDNA samples are then normalized to reference nucleic acids as described previously, using 1X TaqMann® Universal Master mix (Applied Biosystems; catalog No. 4324020), following the manufacturer's instructions.

Probes and primers were designed for each assay according to Applied Biosystems Primer Express Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tm) range = 58°-60°C, primer optimal Tm = 59°C, maximum primer difference = 2°C, probe does not have 5'G, probe Tm must be 10°C greater than primer Tm, amplicon size 75bp to 100bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900nM each, and probe, 200nM.

PCR conditions: When working with RNA samples, normalized RNA from each tissue and each cell line was spotted in each well of either a 96 well or a 384-well PCR plate (Applied Biosystems). PCR cocktails included either a single gene specific probe and primers set, or two multiplexed probe and primers sets (a set specific for the target clone and another gene-specific set multiplexed with the target probe). PCR reactions were set up using TaqMan® One-Step RT-PCR Master Mix (Applied Biosystems, Catalog No. 4313803) following manufacturer's instructions. Reverse transcription was performed at 48°C for 30 minutes followed by amplification/PCR cycles as follows: 95°C 10 min, then 40 cycles of 95°C for 15 seconds, 60°C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100. Expression with CT values below 28 is considered as high expression, CT values between 28 and 32 is considered moderate and CT value between 32 to 35 is considered as low expression. All the relative expression with CT values above 35 is not considered as significant expression.

When working with sscDNA samples, normalized sscDNA was used as described previously for RNA samples. PCR reactions containing one or two sets of probe and primers were set up as described previously, using 1X TaqMan® Universal Master mix (Applied Biosystems; catalog No. 4324020), following the manufacturer's instructions. PCR amplification was performed as follows: 95°C 10 min, then 40 cycles of 95°C for 15 seconds, 60°C for 1 minute. Results were analyzed and processed as described previously.

### Panels 1,1.1,1.2, and 1.3D

The plates for Panels 1, 1.1, 1.2 and 1.3D include 2 control wells (genomic DNA control and chemistry control) and 94 wells containing cDNA from various samples. The samples in these panels are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in these panels are widely available through the American Type Culture Collection (ATCC), a repository for cultured cell lines, and were cultured using the conditions recommended by the ATCC. The normal tissues found on these panels are comprised of samples derived from all major organ systems from single adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose.

In the results for Panels 1, 1.1, 1.2 and 1.3D, the following abbreviations are used:
ca. = carcinoma,
* = established from metastasis,
met = metastasis,
s cell var = small cell variant,
non-s = non-sm = non-small,
squam = squamous,
pl. eff = pl effusion = pleural effusion,
glio = glioma,
astro = astrocytoma, and
neuro = neuroblastoma.

### General_screening_panel_v1.4, v1.5, v1.6 and 1.7

The plates for Panels 1.4, 1.5, 1.6 and 1.7 include 2 control wells (genomic DNA control and chemistry control) and 88 to 94 wells containing cDNA from various samples. The samples in Panels 1.4, 1.5, 1.6 and 1.7 are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in Panels 1.4, 1.5, 1.6 and 1.7 are widely available through the American Type Culture Collection (ATCC), a repository for cultured cell lines, and were cultured using the conditions recommended by the ATCC. The normal tissues found on Panels 1.4, 1.5, 1.6 and 1.7 are comprised of pools of samples derived from all major organ systems from 2 to 5 different adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose. Abbreviations are as described for Panels 1, 1.1, 1.2, and 1.3D.

### Panels 2D, 2.2, 2.3 and 2.4

The plates for Panels 2D, 2.2, 2.3 and 2.4 generally include 2 control wells and 94 test samples composed of RNA or cDNA isolated from human tissue procured by surgeons working in close cooperation with the National Cancer Institute's Cooperative Human Tissue Network (CHTN) or the National Disease Research Initiative (NDRI) or from Ardais or Clinomics). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologist at NDRI/ CHTN/Ardais/Clinomics). Unmatched RNA samples from tissues without malignancy (normal tissues) were also obtained from Ardais or Clinomics. This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen.

### HASS Panel v 1.0

The HASS panel v 1.0 plates are comprised of 93 cDNA samples and two controls. Specifically, 81 of these samples are derived from cultured human cancer cell lines that had been subjected to serum starvation, acidosis and anoxia for different time periods as well as controls for these treatments, 3 samples of human primary cells, 9 samples of malignant brain cancer (4 medulloblastomas and 5 glioblastomas) and 2 controls. The human cancer cell lines are obtained from ATCC (American Type Culture Collection) and fall into the following tissue groups: breast cancer, prostate cancer, bladder carcinomas, pancreatic cancers and CNS cancer cell lines. These cancer cells are all cultured under standard recommended conditions. The treatments used (serum starvation, acidosis and anoxia) have been previously published in the scientific literature. The primary human cells were obtained from Clonetics (Walkersville, MD) and were grown in the media and conditions recommended by Clonetics. The malignant brain cancer samples are obtained as part of a collaboration (Henry Ford Cancer Center) and are evaluated by a pathologist prior to CuraGen receiving the samples. RNA was prepared from these samples using the standard procedures. The genomic and chemistry control wells have been described previously.

### ARDAIS Panel v 1.0

The plates for ARDAIS panel v 1.0 generally include 2 control wells and 22 test samples composed of RNA isolated from human tissue procured by surgeons working in close cooperation with Ardais Corporation. The tissues are derived from human lung malignancies (lung adenocarcinoma or lung squamous cell carcinoma) and in cases where indicated many malignant samples have "matched margins" obtained from noncancerous lung tissue just adjacent to the tumor. These matched margins are taken from the tissue surrounding (i.e. immediately proximal) to the zone of surgery (designated "NAT", for normal adjacent tissue) in the results below. The tumor tissue and the "matched margins" are evaluated by independent pathologists (the surgical pathologists and again by a pathologist at Ardais). Unmatched malignant and non-malignant RNA samples from lungs were also obtained from Ardais. Additional information from Ardais provides a gross histopathological assessment of tumor differentiation grade and stage. Moreover, most samples include the original surgical pathology report that provides information regarding the clinical state of the patient.

### ARDAIS Prostate v 1.0

The plates for ARDAIS prostate 1.0 generally include 2 control wells and 68 test samples composed of RNA isolated from human tissue procured by surgeons working in close cooperation with Ardais Corporation. The tissues are derived from human prostate malignancies and in cases where indicated malignant samples have "matched margins" obtained from noncancerous prostate tissue just adjacent to the tumor. These matched margins are taken from the tissue surrounding (i.e. immediately proximal) to the zone of surgery (designated "NAT", for normal adjacent tissue) in the results below. The tumor tissue and the "matched margins" are evaluated by independent pathologists (the surgical pathologists and again by a pathologist at Ardais). RNA from unmatched malignant and non-malignant prostate samples were also obtained from Ardais. Additional information from Ardais provides a gross histopathological assessment of tumor differentiation grade and stage. Moreover, most samples include the original surgical pathology report that provides information regarding the clinical state of the patient.

### Panel 3D, 3.1 and 3.2

The plates of Panel 3D, 3.1, and 3.2 are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D, 3.1, 3.2, 1, 1.1., 1.2, 1.3D, 1.4, 1.5, and 1.6 are of the most common cell lines used in the scientific literature.

### Panels 4D, 4R, and 4.1D

Panel 4 includes samples on a 96 well plate (2 control wells, 94 test samples) composed of RNA (Panel 4R) or cDNA (Panels 4D/4.1D) isolated from various human cell lines or tissues related to inflammatory conditions. Total RNA from control normal tissues such as colon and lung (Stratagene, La Jolla, CA) and thymus and kidney (Clontech) was employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; IL-1 beta at approximately 1-5ng/ml, TNF alpha at approximately 5-10ng/ml, IFN gamma at approximately 20-50ng/ml, IL-4 at approximately 5-10ng/ml, IL-9 at approximately 5-10ng/ml, IL-13 at approximately 5-10ng/ml. Endothelial cells were sometimes starved for various times by culture in the basal media from Clonetics with 0.1% serum.

Mononuclear cells were prepared from blood of employees at CuraGen Corporation, using Ficoll. LAK cells were prepared from these cells by culture in DMEM 5% FCS (Hyclone), 100µM non essential amino acids (Gibco/Life Technologies, Rockville, MD), 1mM sodium pyruvate (Gibco), mercaptoethanol 5.5x10⁻⁵M (Gibco), and 10mM Hepes (Gibco) and Interleukin 2 for 4-6 days. Cells were then either activated with 10-20ng/ml PMA and 1-2µg/ml ionomycin, IL-12 at 5-10ng/ml, IFN gamma at 20-50ng/ml and IL-18 at 5-10ng/ml for 6 hours. In some cases, mononuclear cells were cultured for 4-5 days in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) with PHA (phytohemagglutinin) orPWM (pokeweed mitogen) at approximately 5µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶cells/ml in DMEM 5% FCS (Hyclone), 100µM non essential amino acids (Gibco), 1mM sodium pyruvate (Gibco), mercaptoethanol (5.5x10⁻⁵M) (Gibco), and 10mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100µM non essential amino acids (Gibco), 1mM sodium pyruvate (Gibco), mercaptoethanol 5.5x10⁻⁵M (Gibco), and 10mM Hepes (Gibco), 50ng/ml GMCSF and 5ng/ml IL-4 for 5-7 days. Macrophages were prepared by culture of monocytes for 5-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and 10% AB Human Serum or MCSF at approximately 50ng/ml. Monocytes, macrophages and dendritic cells were stimulated for 6 and 12-14 hours with lipopolysaccharide (LPS) at 100ng/ml. Dendritic cells were also stimulated with anti-CD40 monoclonal antibody (Pharmingen) at 10µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CDS, CD56, CD14 and CD19 Miltenyi beads and positive selection. CD45RO beads were then used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100µM non essential amino acids (Gibco), 1mM sodium pyruvate (Gibco), mercaptoethanol 5.5x10⁻⁵ M (Gibco), and 10mM Hepes (Gibco) and plated at 10⁶cells/ml onto Falcon 6 well tissue culture plates that had been coated overnight with 0.5 µg/ml anti-CD28 (Pharmingen) and 3ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶ cells/ml in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). To activate the cells, we used PWM at 5µg/ml or anti-CD40 (Pharmingen) at approximately 10µg/ml and IL-4 at 5-10ng/ml. Cells were harvested for RNA preparation at 24,48 and 72 hours.

To prepare the primary and secondary Th1/Th2 and Tr1 cells, six-well Falcon plates were coated overnight with 10µg/ml anti-CD28 (Pharmingen) and 2µg/ml OKT3 (ATCC), and then washed twice with PBS. Umbilical cord blood CD4 lymphocytes (Poietic Systems, German Town, MD) were cultured at 10⁵-10⁶cells/ml in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (4ng/ml). IL-12 (5ng/ml) and anti-IL4 (1µg/ml) were used to direct to Th1, while IL-4 (5ng/ml) and anti-IFN gamma (1µg/ml) were used to direct to Th2 and IL-10 at 5ng/ml was used to direct to Tr1. After 4-5 days, the activated Th1, Th2 and Tr1 lymphocytes were washed once in DMEM and expanded for 4-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (1ng/ml). Following this, the activated Th1, Th2 and Tr1 lymphocytes were re-stimulated for 5 days with anti-CD28/OKT3 and cytokines as described above, but with the addition of anti-CD95L (1µg/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-I, KU-812. EOL cells were further differentiated by culture in 0.1mM dbcAMP at 5x10⁵cells/ml for 8 days, changing the media every 3 days and adjusting the cell concentration to 5x 10⁵ cells/ml. For the culture of these cells, we used DMEM or RPMI (as recommended by the ATCC), with the addition of 5% FCS (Hyclone), 100µM non essential amino acids (Gibco), 1mM sodium pyruvate (Gibco), mercaptoethanol 5.5x10⁻⁵M (Gibco), 10mM Hepes (Gibco). RNA was either prepared from resting cells or cells activated with PMA at 10ng/ml and ionomycin at 1µg/ml for 6 and 14 hours. Keratinocyte line CCD106 and an airway epithelial tumor line NCI-H292 were also obtained from the ATCC. Both were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). CCD1106 cells were activated for 6 and 14 hours with approximately 5 ng/ml TNF alpha and 1ng/ml IL-1 beta, while NCI-H292 cells were activated for 6 and 14 hours with the following cytokines: 5ng/ml IL-4, 5ng/ml IL-9, 5ng/ml IL-13 and 25ng/ml IFN gamma.

For these cell lines and blood cells, RNA was prepared by lysing approximately 10⁷cells/ml using Trizol (Gibco BRL). Briefly, 1/10 volume of bromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15ml Falcon Tube. An equal volume of isopropanol was added and left at -20°C overnight. The precipitated RNA was spun down at 9,000 rpm for 15 min in a Sorvall SS34 rotor and washed in 70% ethanol. The pellet was redissolved in 300µl of RNAse-free water and 35µl buffer (Promega) 5µl DTT, 7µl RNAsin and 8µl DNAse were added. The tube was incubated at 37°C for 30 minutes to remove contaminating genomic DNA, extracted once with phenol chloroform and re-precipitated with 1/10 volume of 3M sodium acetate and 2 volumes of 100% ethanol. The RNA was spun down and placed in RNAse free water. RNA was stored at - 80°C.

### AI_comprehensive panel_v1.0

The plates for AI_comprehensive panel_v1.0 include two control wells and 89 test samples comprised of cDNA isolated from surgical and postmortem human tissues obtained from the Backus Hospital and Clinomics (Frederick, MD). Total RNA was extracted from tissue samples from the Backus Hospital in the Facility at CuraGen. Total RNA from other tissues was obtained from Clinomics.

Joint tissues including synovial fluid, synovium, bone and cartilage were obtained from patients undergoing total knee or hip replacement surgery at the Backus Hospital. Tissue samples were immediately snap frozen in liquid nitrogen to ensure that isolated RNA was of optimal quality and not degraded. Additional samples of osteoarthritis and rheumatoid arthritis joint tissues were obtained from Clinomics. Normal control tissues were supplied by Clinomics and were obtained during autopsy of trauma victims.

Surgical specimens ofpsoriatic tissues and adjacent matched tissues were provided as total RNA by Clinomics. Two male and two female patients were selected between the ages of 25 and 47. None of the patients were taking prescription drugs at the time samples were isolated.

Surgical specimens of diseased colon from patients with ulcerative colitis and Crohns disease and adjacent matched tissues were obtained from Clinomics. Bowel tissue from three female and three male Crohn's patients between the ages of 41-69 were used. Two patients were not on prescription medication while the others were taking dexamethasone, phenobarbital, or tylenol. Ulcerative colitis tissue was from three male and four female patients. Four of the patients were taking lebvid and two were on phenobarbital.

Total RNA from post mortem lung tissue from trauma victims with no disease or with emphysema, asthma or COPD was purchased from Clinomics. Emphysema patients ranged in age from 40-70 and all were smokers, this age range was chosen to focus on patients with cigarette-linked emphysema and to avoid those patients with alpha-1 anti-trypsin deficiencies. Asthma patients ranged in age from 36-75, and excluded smokers to prevent those patients that could also have COPD. COPD patients ranged in age from 35-80 and included both smokers and non-smokers. Most patients were taking corticosteroids, and bronchodilators.

In the labels employed to identify tissues in the A1_comprehensive panel_v1.0 panel, the following abbreviations are used:
AI = Autoimmunity
Syn = Synovial
Normal = No apparent disease
Rep22 /Rep20 = individual patients
RA = Rheumatoid arthritis
Backus = From Backus Hospital
OA = Osteoarthritis
(SS) (BA) (MF) = Individual patients
Adj = Adjacent tissue
Match control = adjacent tissues
-M = Male
-F = Female
COPD = Chronic obstructive pulmonary disease

### AI.05 chondrosarcoma

The AI.05 chondrosarcoma plates are comprised of SW1353 cells that had been subjected to serum starvation and treatment with cytokines that are known to induce MMP (1, 3 and 13) synthesis (eg. IL1beta). These treatments include: IL-1beta (10 ng/ml), IL-1beta + TNF-alpha (50 ng/ml), IL-1beta + Oncostatin (50 ng/ml) and PMA (100 ng/ml). The SW1353 cells were obtained from the ATCC (American Type Culture Collection) and were all cultured under standard recommended conditions. The SW1353 cells were plated at 3x10⁵ cells/ml (in DMEM medium-10 % FBS) in 6-well plates. The treatment was done in triplicate, for 6 and 18 h. The supernatants were collected for analysis of MMP 1, 3 and 13 production and for RNA extraction. RNA was prepared from these samples using the standard procedures.

### Panels 5D and 5I

The plates for Panel 5D and 5I include two control wells and a variety of cDNAs isolated from human tissues and cell lines with an emphasis on metabolic diseases. Metabolic tissues were obtained from patients enrolled in the Gestational Diabetes study. Cells were obtained during different stages in the differentiation of adipocytes from human mesenchymal stem cells. Human pancreatic islets were also obtained.

In the Gestational Diabetes study subjects are young (18 - 40 years), otherwise healthy women with and without gestational diabetes undergoing routine (elective) Caesarean section. After delivery of the infant, when the surgical incisions were being repaired/closed, the obstetrician removed a small sample (less than 1 cc) of the exposed metabolic tissues during the closure of each surgical level. The biopsy material was rinsed in sterile saline, blotted and fast frozen within 5 minutes from the time of removal. The tissue was then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus) and subcutaneous adipose. Patient descriptions are as follows:
Patient 2: Diabetic Hispanic, overweight, not on insulin
Patient 7-9: Nondiabetic Caucasian and obese (BMI>30)
Patient 10: Diabetic Hispanic, overweight, on insulin
Patient 11: Nondiabetic African American and overweight
Patient 12: Diabetic Hispanic on insulin

Adiocyte differentiation was induced in donor progenitor cells obtained from Osirus (a division of Clonetics/BioWhittaker) in triplicate, except for Donor 3U which had only two replicates. Scientists at Clonetics isolated, grew and differentiated human mesenchymal stem cells (HuMSCs) for CuraGen based on the published protocol found in Mark F. Pittenger, et al., Multilineage Potential of Adult Human Mesenchymal Stem Cells Science Apr 2 1999: 143-147. Clonetics provided Trizol lysates or frozen pellets suitable for mRNA isolation and ds cDNA production. A general description of each donor is as follows:
Donor 2 and 3 U: Mesenchymal Stem cells, Undifferentiated Adipose
Donor 2 and 3 AM: Adipose, AdiposeMidway Differentiated
Donor 2 and 3 AD: Adipose, Adipose Differentiated

Human cell lines were generally obtained from ATCC (American Type Culture Collection), NCI or the German tumor cell bank and fall into the following tissue groups: kidney proximal convoluted tubule, uterine smooth muscle cells, small intestine, liver HepG2 cancer cells, heart primary stromal cells, and adrenal cortical adenoma cells. These cells are all cultured under standard recommended conditions and RNA extracted using the standard procedures. All samples were processed at CuraGen to produce single stranded cDNA.

Panel 51 contains all samples previously described with the addition of pancreatic islets from a 58 year old female patient obtained from the Diabetes Research Institute at the University of Miami School of Medicine. Islet tissue was processed to total RNA at an outside source and delivered to CuraGen for addition to panel 51.

In the labels employed to identify tissues in the 5D and 5I panels, the following abbreviations are used:
GO Adipose = Greater Omentum Adipose
SK = Skeletal Muscle
UT = Uterus
PL = Placenta
AD = Adipose Differentiated
AM = Adipose Midway Differentiated
U = Undifferentiated Stem Cells

### Human Metabolic RTQ-PCR Panel

The plates for the Human Metabolic RTQ-PCR Panel include two control wells (genomic DNA control and chemistry control) and 211 cDNAs isolated from human tissues and cell lines with an emphasis on metabolic diseases. This panel is useful for establishing the tissue and cellular expression profiles for genes believed to play a role in the etiology and pathogenesis of obesity and/or diabetes and to confirm differential expression of such genes derived from other methods. Metabolic tissues were obtained from patients enrolled in the CuraGen Gestational Diabetes study and from autopsy tissues from Type II diabetics and age, sex and race-matched control patients. One or more of the following were used to characterize the patients: body mass index [BMI = wt (kg) / ht (m²)], serum glucose, HgbA1c. Cell lines used in this panel are widely available through the American Type Culture Collection (ATCC), a repository for cultured cell lines. RNA from human Pancreatic Islets was also obtained.

In the Gestational Diabetes study, subjects are young (18-40 years), otherwise healthy women with and without gestational diabetes undergoing routine (elective) Caesarian section. After delivery of the infant, when the surgical incisions were being repaired/closed, the obstetrician removed a small sample (less than 1cc) of the exposed metabolic tissues during the closure of each surgical level. The biopsy material was rinsed in sterile saline, blotted, and then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus), and subcutaneous adipose. Patient descriptions are as follows:
Patient 7 - Non-diabetic Caucasian and obese
Patient 8 - Non-diabetic Caucasian and obese
Patient 12 - Diabetic Caucasian with unknown BMI and on insulin
Patient 13 - Diabetic Caucasian, overweight, not on insulin
Patient 15 - Diabetic Caucasian, obese, not on insulin
Patient 17 - Diabetic Caucasian, normal weight, not on insulin
Patient 18 - Diabetic Hispanic, obese, not on insulin
Patient 19 - Non-diabetic Caucasian and normal weight
Patient 20 - Diabetic Caucasian, overweight, and on insulin
Patient 21 - Non-diabetic Caucasian and overweight
Patient 22 - Diabetic Caucasian, normal weight, on insulin
Patient 23 - Non-diabetic Caucasian and overweight
Patient 25 - Diabetic Caucasian, normal weight, not on insulin
Patient 26 - Diabetic Caucasian, obese, on insulin
Patient 27 - Diabetic Caucasian, obese, on insulin
Total RNA was isolated from metabolic tissues of 12 Type II diabetic patients and 12 matched control patients included hypothalamus, liver, pancreas, small intestine, psoas muscle, diaphragm muscle, visceral adipose, and subcutaneous adipose. The diabetics and non-diabetics were matched for age, sex, ethnicity, and BMI where possible.

The panel also contains pancreatic islets from a 22 year old male patient (with a BMI of 35) obtained from the Diabetes Research Institute at the University of Miami School of Medicine. Islet tissue was processed to total RNA at CuraGen.

Cell lines used in this panel are widely available through the American Type Culture Collection (ATCC), a repository for cultured cell lines, and were cultured at an outside facility. The RNA was extracted at CuraGen according to CuraGen protocols. All samples were then processed at CuraGen to produce single stranded cDNA.

In the labels used to identify tissues in the Human Metabolic panel, the following abbreviations are used:
P1 = placenta
Go = greater omentum
Sk = skeletal muscle
Ut = uterus
CC = Caucasian
HI = Hispanic
AA = African American
AS = Asian
Diab = Type II diabetic
Norm = Non-diabetic
Overwt = Overweight; med BMI
Obese = Hi BMI
Low BM = 20-25
Med BM = 26-30
Hi BMI = Greater than 30
M = Male
# = Patient identifier
Vis. = Visceral
SubQ = Subcutaneous

### Panel CNSD.01

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; e.g., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.

In the labels employed to identify tissues in the CNS panel, the following abbreviations are used:
PSP = Progressive supranuclear palsy
Sub Nigra = Substantia nigra
Glob Palladus= Globus palladus
Temp Pole = Temporal pole
Cing Gyr = Cingulate gyrus
BA 4 = Brodman Area 4

### Panel CNS_Neurodegeneration_V1.0

The plates for Panel CNS_Neurodegeneration_V1.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains six brains from Alzheimer's disease (AD) patients, and eight brains from "Normal controls" who showed no evidence of dementia prior to death. The eight normal control brains are divided into two categories: Controls with no dementia and no Alzheimer's like pathology (Controls) and controls with no dementia but evidence of severe Alzheimer's like pathology, (specifically senile plaque load rated as level 3 on a scale of 0-3; 0 = no evidence of plaques, 3 = severe AD senile plaque load). Within each of these brains, the following regions are represented: hippocampus, temporal cortex (Brodman Area 21), parietal cortex (Brodman area 7), and occipital cortex (Brodman area 17). These regions were chosen to encompass all levels of neurodegeneration in AD. The hippocampus is a region of early and severe neuronal loss in AD; the temporal cortex is known to show neurodegeneration in AD after the hippocampus; the parietal cortex shows moderate neuronal death in the late stages of the disease; the occipital cortex is spared in AD and therefore acts as a "control" region within AD patients. Not all brain regions are represented in all cases. ,

In the labels employed to identify tissues in the CNS_Neurodegeneration_V1.0 panel, the following abbreviations are used:
AD = Alzheimer's disease brain; patient was demented and showed AD-like pathology upon autopsy
Control = Control brains; patient not demented, showing no neuropathology
Control (Path) = Control brains; pateint not demented but showing sever AD-like pathology
SupTemporal Ctx = Superior Temporal Cortex
Inf Temporal Ctx = Inferior Temporal Cortex

### Panel CNS_Neurodegeneration_V2.0

The plates for Panel CNS_Neurodegeneration_V2.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains sixteen brains from Alzheimer's disease (AD) patients, and twenty-nine brains from "Normal controls" who showed no evidence of dementia prior to death. The twenty-nine normal control brains are divided into two categories: Fourteen controls with no dementia and no Alzheimer's like pathology (Controls) and fifteen controls with no dementia but evidence of severe Alzheimer's like pathology, (specifically senile plaque load rated as level 3 on a scale of 0-3; 0 = no evidence of plaques, 3 = severe AD senile plaque load). Tissue from the temporal cotex (Broddmann Area 21) was selected for all samples from the Harvard Brain Tissue Resource Center; from the two sample from the Human Brain and Spinal Fluid Resource Center (samples 1 and 2) tissue from the inferior and superior temporal cortex was used; each sample on the panel represents a pool of inferior and superior temporal cortex from an individual patient. The temporal cortex was chosen as it shows a loss of neurons in the intermediate stages of the disease. Selection of a region which is affected in the early stages of Alzheimer's disease (e.g., hippocampus or entorhinal cortex) could potentially result in the examination of gene expression after vulnerable neurons are lost, and missing genes involved in the actual neurodegeneration process.

In the labels employed to identify tissues in the CNS_Neurodegeneration_V2.0 panel, the following abbreviations are used:
AD = Alzheimer's disease brain; patient was demented and showed AD-like pathology upon autopsy
Control = Control brains; patient not demented, showing no neuropathology
AH3 = Control brains; pateint not demented but showing sever AD-like pathology
Inf & Sup Temp Ctx Pool = Pool of inferior and superior temporal cortex for a given individual

### A. CG164221-01: Protein kinase, Sgk

Expression of gene CG164221-O1 was assessed using the primer-probe set Ag6086, described in Table AA. Results of the RTQ-PCR runs are shown in Tables AB, AC and AD.

**Table AA. Probe Name Ag6086**

| **Primers** | **Sequences** | **Length** | **Start Position** | **SEQ ID No** |
|---|---|---|---|---|
| Forward | 5'-aattctcatcgctttcatgaag-3' | 22 | 101 | 98 |
| Probe | TET-5'-aagtcgttcagaeccatcctcctctg-3'-TAMRA | 26 | 123 | 99 |
| Reverse | 5'-ataggagttattggcaatcttctga-3'5'-ataggagttattggcaatcttctga-3' | 25 | 152 | 100 |

| Table AB. CNS_neurodegeneration_v1.0 | | | | | |
|---|---|---|---|---|---|
| **Column A - Rel. Exp.(%) Ag6086, Run 248386491** | | | | | |
| **Column B - Rel. Exp.(%) Ag6086, Run 275777034** | | | | | |
| **Tissue Name** | **A** | **B** | **Tissue Name** | **A** | **B** |
| AD 1 Hippo | 14.0 | 13.8 | Control (Path) 3 Temporal Ctx | 0.9 | 1.5 |
| AD 2 Hippo | 26.4 | 17.6 | Control (Path) 4 Temporal Ctx | 2.1 | 3.3 |
| AD 3 Hippo | 6.0 | 5.9 | AD 1 Occipital Ctx | 10.1 | 8.5 |
| AD 4 Hippo | 6.2 | 5.0 | AD 2 Occipital Ctx (Missing) | 0.0 | 0.0 |
| AD 5 Hippo | 26.4 | 25.5 | AD 3 Occipital Ctx | 0.3 | 3.2 |
| AD 6 Hippo | 67.8 | 58.2 | AD 4 Occipital Ctx | 14.1 | 11.8 |
| Control 2 Hippo | 20.2 | 19.1 | AD 5 Occipital Ctx | 20.2 | 20.7 |
| Control 4 Hippo | 12.9 | 12.2 | AD 6 Occipital Ctx | 20.2 | 18.4 |
| Control (Path) 3 Hippo | 3.1 | 4.5 | Control 1 Occipital Ctx | 1.2 | 1.3 |
| AD 1 Temporal Ctx | 22.4 | 25.9 | Control 2 Occipital Ctx | 27.9 | 31.6 |
| AD 2 Temporal Ctx | 25.9 | 22.5 | Control 3 Occipital Ctx | 5.6 | 4.3 |
| AD 3 Temporal Ctx | 3.1 | 3.3 | Control 4 Occipital Ctx | 9.3 | 6.3 |
| AD 4 Temporal Ctx | 18.3 | 17.2 | Control (Path) 1 Occipital Ctx | 23.5 | 20.3 |
| AD 5 Inf Temporal Ctx | 40.1 | 72.2 | Control (Path) 2 Occipital Ctx | 2.1 | 3.1 |
| AD 5 Sup Temporal Ctx | 47.6 | 39.8 | Control (Path) 3 Occipital Ctx | 1.9 | 2.5 |
| AD 6 Inf Temporal Ctx | **100.0** | **100.0** | Control (Path) 4 Occipital Ctx | 3.1 | 3.2 |
| AD 6 Sup Temporal Ctx | 49.3 | 52.9 | Control 1 Parietal_Ctx | 2.0 | 2.5 |
| Control 1 Temporal Ctx | 2.0 | 1.9 | Control 2 Parietal Ctx | 33.9 | 32.3 |
| Control 2 Temporal Ctx | 20.9 | 17.6 | Control 3 Parietal Ctx | 4.6 | 6.4 |
| Control 3 Temporal Ctx | 6.5 | 6.4 | Control (Path) 1 Parietal Ctx | 12.4 | 11.7 |
| Control 3 Temporal Ctx | 6.6 | 4.2 | Control (Path) 2 Parietal Ctx | 6.7 | 7.5 |
| Control (Path) 1 Temporal Ctx | 11.3 | 8.1 | Control (Path) 3 Parietal Ctx | 1.4 | 1.2 |
| Control (Path) 2 Temporal Ctx | 7.0 | 7.7 | Control (Path) 4 Parietal Ctx | 6.9 | 5.2 |

**Table AC. General_screening_panel_vl.5**

| **Column A - Rel. Exp.(%) Ag6086, Run 247775072** | | | |
|---|---|---|---|
| **Tissue Name** | **A** | **Tissue Name** | **A** |
| Adipose | 29.9 | Renal ca. TK-10 | 1.4 |
| Melanoma* Hs688(A).T | 38.2 | Bladder | 20.4 |
| Melanoma* Hs688(B).T | 17.6 | Gastric ca. (liver met.) NCI-N87 | 2.4 |
| Melanoma* M14 | 55.5 | Gastric ca. KATO III | 3.7 |
| Melanoma* LOXIMVI | 9.1 | Colon ca. SW-948 | 0.3 |
| Melanoma* SK-MEL-5 | 62.4 | Colon ca. SW480 | 0.8 |
| Squamous cell carcinoma SCC-4 | 1.9 | Colon ca.* (SW480 met) SW620 | 0.4 |
| Testis Pool | 5.9 | Colon ca. HT29 | 2.0 |
| Prostate ca.* (bone met) PC-3 | 0.9 | Colon ca. HCT-116 | 0.6 |
| Prostate Pool | 1.0 | Colon ca. CaCo-2 | 9.8 |
| Placenta | 10.6 | Colon cancer tissue | 15.9 |
| Uterus Pool | 3.0 | Colon ca. SW1116 | 0.5 |
| Ovarian ca. OVCAR-3 | 1.4 | Colon ca.Colo-205 | 0.4 |
| Ovarian ca. SK-OV-3 | 4.1 | Colon ca. SW-48 | 0.4 |
| Ovarian ca. OVCAR-4 | 1.6 | Colon Pool | 4.2 |
| Ovarian ca. OVCAR-5 | 10.7 | Small Intestine Pool | 1.2 |
| Ovarian ca. IGROV-1 | 6.2 | Stomach Pool | 3.7 |
| Ovarian ca. OVCAR-8 | 0.5 | Bone Marrow Pool | 3.5 |
| Ovary | 15.1 | Fetal Heart | 5.3 |
| Breast ca. MCF-7 | 0.9 | Heart Pool | 1.9 |
| Breast ca.MDA-MB-231 | 4.8 | Lymph Node Pool | 4.0 |
| Breast ca. BT 549 | 24.5 | Fetal Skeletal Muscle | 3.4 |
| Breast ca. T47D | 0.0 | Skeletal Muscle Pool | 6.1 |
| Breast ca. MDA-N | 11.7 | Spleen Pool | 7.0 |
| Breast Pool | 5.6 | Thymus Pool | 6.3 |
| Trachea | 8.7 | CNS cancer (glio/astro) U87-MG | 6.2 |
| Lung | 7.4 | CNS cancer (glio/astro) U-118-MG | 10.2 |
| Fetal Lung | 15.8 | CNS cancer (neuro;met)SK-N-AS | 4.9 |
| Lung ca. NCI-N417 | 0.4 | CNS cancer (astro) SF-539 | 3.4 |
| Lung ca. LX-1 | 1.8 | CNS cancer (astro) SNB-75 | 13.9 |
| Lung ca. NCI-H146 | 2.7 | CNS cancer (glio) SNB-19 | 5.1 |
| Lung ca. SHP-77 | 9.4 | CNS cancer (glio) SF-295 | 6.9 |
| Lung ca. A549 | 10.1 | Brain (Amygdala) Pool | 6.8 |
| Lung ca. NCI-H526 | 0.1 | Brain (cerebellum) | 8.0 |
| Lung ca. NCI-H23 | 100.0 | Brain (fetal) | 4.4 |
| Lung ca. NCI-H460 | 7.1 | Brain (Hippocampus) Pool | 8.4 |
| Lung ca. HOP-62 | 1.5 | Cerebral Cortex Pool | 5.5 |
| Lung ca. NCI-H522 | 0.1 | Brain (Substantia nigra) Pool | 4.8 |
| Liver | 1.0 | Brain (Thalamus) Pool | 8.2 |
| Fetal Liver | 4.8 | Brain (whole) | 7.6 |
| Liver ca. HepG2 | 1.4 | Spinal Cord Pool | 7.6 |
| Kidney Pool | 4.5 | Adrenal Gland | 59.9 |
| Fetal Kidney | 7.7 | Pituitary gland Pool | 0.5 |
| Renal ca. 786-0 | 2.5 | Salivary Gland | 2.6 |
| Renal ca. A498 | 1.7 | Thyroid (female) | 6.0 |
| Renal ca. ACHN | 6.6 | Pancreatic ca. CAPAN2 | 3.1 |
| Renal ca. UO-31 | 2.8 | Pancreas Pool | 17.1 |

**Table AD. Panel 5 Islet**

| **Column A - Rel. Exp.(%) Ag6086, Run 248045495** | | | |
|---|---|---|---|
| **Tissue Name** | **A** | **Tissue Name** | **A** |
| 97457_Patient-02go_adipose | 26.4 | 94709_Donor 2 AM - A_adipose | 4.7 |
| 97476_Patient-07sk_skeletal muscle | 40.6 | 94710_Donor 2 AM - B_adipose | 2.7 |
| 97477_Patient-07ut_uterus | 23.0 | 94711_Donor 2 AM - C_adipose | 3.4 |
| 97478_Patient-07pl_placenta | 28.1 | 94712_Donor 2 AD - A_adipose | 12.0 |
| 99167_Bayer Patient 1 | 59.5 | 94713_Donor 2 AD - B_adipose | 12.6 |
| 97482_Patient-08ut_uterus | 24.3 | 94714_Donor 2 AD - C_adipose | 14.1 |
| 97483_Pahent-08pl_placenta | 3.9 | 94742 Donor 3 U - A_Mesenchymal Stem Cells | 4.8 |
| 97486_Patient-09sk_skeletal muscle | 5.3 | 94743_Donor 3 U - B_Mesenchymal Stem Cells | 11.0 |
| 97487_Patient-09ut_uterus | 21.0 | 94730_Donor 3 AM - A_adipose | 5.6 |
| 97488_Patient-09pl_placenta | 16.4 | 94731_Donor 3 AM - B_adipose | 4.7 |
| 97492_Patient-10ut_uterus | 29.5 | 94732_Donor 3 AM - C_adipose | 4.0 |
| 97493_Patient-10pl_placenta | 40.9 | 94733_Donor 3 AD - A_adipose | 43.2 |
| 97495_Patient-11 go_adipose | 49.3 | 94734_Donor 3 AD - B_adipose | 15.5 |
| 97496 Patient-11sk skeletal muscle | 17.6 | 94735_Donor 3 AD - C_adipose | 36.9 |
| 97497_Patient-11ut_uterus | 39.0 | 77138_Liver_HepG2untreated | 9.0 |
| 97498_Patient-11pl_placenta | 19.5 | 73556 Heart Cardiac stromal cells (primary) | 20.0 |
| 97500_Patient-12go_adipose | 84.7 | 81735_Small Intestine | 31.9 |
| 97501_Patient-12sk_skeletal muscle | 67.8 | 72409_Kidney_Proximal Convoluted Tubule | 38.7 |
| 97502_Patient-12ut_uterus | 25.7 | 82685_Small intestine_Duodenum | 4.6 |
| 97503_Patient-12pl_placenta | 37.4 | 90650_Adrenal_Adrenocortical adenoma | 68.3 |
| 94721_Donor 2 U - A_Mesenchymal Stem Cells | 26.2 | 72410_Kidney_HRCE | 79.0 |
| 94722_Donor 2 U - B_Mesenchymal Stem Cells | 19.9 | 72411_Kidney_HRE | 100.0 |
| 94723_Donor 2 U - C_Mesenchymal Stem Cells | 33.0 | 73139_Uterus_Uterine smooth muscle cells | 7.8 |

**CNS_neurodegeneratton_v1.0 Summary:** Ag6086 Two experiments with same probe-primer sets are in excellent agreement. This panel confirms the expression of this gene at low levels in the brain in an independent group of individuals. This gene is found to be upregulated in the temporal cortex of Alzheimer's disease patients. This gene encodes serine/threonine-protein kinase Sgk protein. Blockade of SGK protein encoded by this gene may be of use in the treatment of this disease and decrease neuronal death.

**General_screening_panel_v1.5 Summary:** Ag6086 This gene shows ubiquitous expression with highest expression in a lung cancer NCI-H23 cell line (CT=25.5). Moderate to high levels of expression of this gene is also seen in cluster of cancer cell lines derived from pancreatic, gastric, colon, lung, liver, renal, breast, ovarian, prostate, squamous cell carcinoma, melanoma and brain cancers. Thus, expression of this gene could be used as a marker to detect the presence of these cancers. Furthermore, therapeutic modulation of the expression or function of this gene may be effective in the treatment of pancreatic, gastric, colon, lung, liver, renal, breast, ovarian, prostate, squamous cell carcinoma, melanoma and brain cancers.

Among tissues with metabolic or endocrine function, this gene is expressed at moderate to high levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at moderate levels in all regions of the central nervous system examined, including amygdala, hippocampus, substantia nigra, thalamus, cerebellum, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

Panel 5 Islet Summary: Ag6086 This gene shows wide spread expression in this panel with highest expression in kidney (CT=27.8). The moderate expression of SGK1 in adipose, kidney and skeletal muscle is consistent with literature and the expression pattern seen in panel 1.5. Notably, SGK1 is also expressed in pancreatic islets. SGK is the serine/threonine kinase implicated in development of several diabetic complications, specifically hypertension and diabetic nephropathy (Lang, F.; et al., 2000Proc. Nat. Acad. Sci. 97: 8157-8162. (PMID : 10884438); Lang F, et al. 2001, Sci STKE 108:RE17 (PMID: 11707620). It has been shown that SGK phosphorylates the cytoplasmic domains of several ion channels/transporters modulating their activity, and/or endocytosis rate. SGK gene has been mapped to the region 6q23. Interestingly, duplication of this chromosomal region causes transient neonatal diabetes mellitus characterized by hyperglycemia and predisposition toward type 2 diabetes (Temple, I. K.; et al. 1996, Hum. Molec. Genet. 5: 1117-1124. (PMID: 8842729). Together with the localization of SGK gene in loci for neonatal diabetes, the data suggest that SGK1 might modulate insulin secretion.

### B. CG50183-01: Chemokine Receptor

Expression of gene CG50183-01 was assessed using the primer-probe set Gpcr09, described in Table BA. Results of the RTQ-PCR runs are shown in Table BB.

**Table BA. Probe Name Gpcr09**

| **Primers** | **Sequences** | **Length** | **Start Position** | **SEQ ID No** |
|---|---|---|---|---|
| Forward | 5'-tgagcaaacgcatggacatc-3' | 20 | 826 | 101 |
| Probe | TET-5'-ccatccaagtcacagaaagcatcgcact- 3'TAMRA | 28 | 847 | 102 |
| Reverse | 5'-tgggttgaggcagctgtga-3' | 19 | 878 | 103 |

**Table BB. Panel 1**

| **Column A - Rel. Exp.(%) Gpcr09, Run 109665126** | | | |
|---|---|---|---|
| **Tissue Name** | **A** | **Tissue Name** | **A** |
| Endothelial cells | 0.8 | Renal ca. 786-0 | 5.8 |
| Endothelial cells (treated) | 0.8 | Renal ca. A498 | 19.2 |
| Pancreas | 17.4 | Renal ca. RXF 393 | 5.0 |
| Pancreatic ca. CAPAN 2 | 2.5 | Renal ca. ACHN | 14.8 |
| Adrenal gland | 17.2 | Renal ca. UO-31 | 11.1 |
| Thyroid | 8.5 | Renal ca. TK-10 | 13.4 |
| Salivary gland | 39.2 | Liver | 18.4 |
| Pituitary gland | 25.3 | Liver (fetal) | 7.4 |
| Brain (fetal) | 2.0 | Liver ca. (hepatoblast) HepG2 | 28.3 |
| Brain (whole) | 3.3 | Lung | 0.0 |
| Brain (amygdala) | 8.2 | Lung (fetal) | 9.1 |
| Brain (cerebellum) | 10.8 | Lung ca. (small cell) LX-1 | 7.9 |
| Brain (hippocampus) | 7.3 | Lung ca. (small cell) NCI-H69 | 13.2 |
| Brain (substantia nigra) | 27.2 | Lung ca. (s.cell var.) SHP-77 | 2.2 |
| Brain (thalamus) | 4.0 | Lung ca. (large cell)NCI-H460 | 25.5 |
| Brain (hypothalamus) | **100.0** | Lung ca. (non-sm. cell) A549 | 20.9 |
| Spinal cord | 7.8 | Lung ca. (non-s.cell) NCI-H23 | 3.6 |
| glio/astro U87-MG | 9.9 | Lung ca. (non-s.cell) HOP-62 | 12.5 |
| glio/astro U-118-MG | 6.7 | Lung ca. (non-s.cl) NCI-H522 | 19.9 |
| astrocytoma SW1783 | 4.4 | Lung ca. (squam.) SW 900 | 9.5 |
| neuro*; met SK-N-AS | 5.5 | Lung ca. (squam.) NCI-H596 | 20.2 |
| astrocytoma SF-539 | 9.0 | Mammary gland | 57.8 |
| astrocytoma SNB-75 | 4.6 | Breast ca.* (pl.ef) MCF-7 | 7.6 |
| glioma SNB-19 | 14.9 | Breast ca.* (pl.ef) MDA-MB-231 | 1.2 |
| glioma U251 | 11.7 | Breast ca.* (pl. ef) T47D | 34.4 |
| glioma SF-295 | 13.6 | Breast ca. BT-549 | 4.1 |
| Heart | 56.3 | Breast ca. MDA-N | 51.8 |
| Skeletal muscle | 6.2 | Ovary | 6.9 |
| Bone marrow | 4.8 | Ovarian ca. OVCAR-3 | 15.2 |
| Thymus | 22.1 | Ovarian ca. OVCAR-4 | 2.6 |
| Spleen | 12.6 | Ovarian ca. OVCAR-5 | 22.2 |
| Lymph node | 19.2 | Ovarian ca. OVCAR-8 | 36.1 |
| Colon (ascending) | 37.1 | Ovarian ca. IGROV-1 | 12.6 |
| Stomach | 24.5 | Ovarian ca. (ascites) SK-OV-3 | 7.5 |
| Small intestine | 55.1 | Uterus | 21.8 |
| Colon ca. SW480 | 3.8 | Placenta | 7.5 |
| Colon ca.* SW620 (SW480 met) | 5.1 | Prostate | 19.5 |
| Colon ca. HT29 | 8.4 | Prostate ca.* (bone met) PC-3 | 12.8 |
| Colon ca. HCT-116 | 8.1 | Testis | 14.8 |
| Colon ca. CaCo-2 | 11.2 | Melanoma Hs688(A).T | 7.5 |
| Colon ca. HCT-15 5 | 8.3 | Melanoma* (met) Hs688(B).T | 11.3 |
| Colon ca. HCC-2998 | 13.5 | Melanoma UACC-62 | 5.0 |
| Gastric ca. * (liver met) NCI-N87 | 36.3 | Melanoma M14 | 10.1 |
| Bladder | 30.6 | Melanoma LOX IMVI | 1.6 |
| Trachea | 19.6 | Melanoma* (met) SK-MEL-5 | 15.7 |
| Kidney | 24.1 | Melanoma SK-MEL-28 | 25.5 |
| Kidney (fetal) | 14.4 | | |

**Panel 1 Summary:** Gpcr09 Highest expression is seen in hypothalamus (CT=25). Prominent levels of expression are seen primarily in normal tissues, including mammary gland, heart, colon, stomach, and substantia nigra.

### C. CG50249-01: VOLTAGE-GATED POTASSIUM CHANNEL PROTEIN KV3.2 LIKE

Expression of gene CG50249-01 was assessed using the primer-probe set Ag2503, described in Table CA. Results of the RTQ-PCR runs are shown in Tables CB, CC, CD and CE.

**Table CA. Probe Name Ag2503**

| **Primers** | **Sequences** | **Length** | **Start Position** | **SEQ ID No** |
|---|---|---|---|---|
| Forward | 5'-gaggctctctccagtaacatca-3' | 22 | 1851 | 104 |
| Probe | TET-5'-act:ctccttgtcctctgaggcgctct-3'-TAMPA | 26 | 1880 | 105 |
| Reverse | 5'-gcagtttggttgtttggtttac-3' | 22 | 1929 | 106 |

**Table CB. CNS neurodegeneration v1.0**

| **Column A - Rel. Exp.(%) Ag2503, Run 208779478** | | | | | |
|---|---|---|---|---|---|
| **Column B - Rel. Exp.(%) Ag2503, Run 253338938** | | | | | |
| **Tissue Name** | **A** | **B** | **Tissue Name** | **A** | **B** |
| AD 1 Hippo | 4.1 | 4.4 | Control (Path) 3 Temporal Ctx | 0.6 | 1.0 |
| AD 2 Hippo | 10.5 | 9.3 | Control (Path) 4 Temporal Ctx | 26.8 | 28.1 |
| AD 3 Hippo | 1.3 | 1.2 | AD 1 Occipital Ctx | 12.1 | 12.5 |
| AD 4 Hippo | 1.6 | 1.8 | AD 2 Occipital Ctx (Missing) | 0.0 | 0.0 |
| AD 5 Hippo | 100.0 | 100.0 | AD 3 Occipital Ctx | 1.8 | 2.1 |
| AD 6 Hippo | 16.8 | 16.5 | AD 4 Occipital Ctx | 13.8 | 10.4 |
| Control 2 Hippo | 13.4 | 14.5 | AD 5 Occipital Ctx | 45.7 | 48.3 |
| Control 4 Hippo | 1.1 | 0.8 | AD 6 Occipital Ctx | 15.5 | 12.6 |
| Control (Path) 3 Hippo | 0.5 | 0.6 | Control 1 Occipital Ctx | 0.2 | 0.0 |
| AD 1 Temporal Ctx | 3.9 | 4.1 | Control 2 Occipital Ctx | 54.0 | 56.3 |
| AD 2 Temporal Ctx | 19.3 | 18.9 | Control 3 Occipital Ctx | 11.2 | 10.7 |
| AD 3 Temporal Ctx | 1.4 | 1.1 | Control 4 Occipital Ctx | 0.5 | 0.5 |
| AD 4 Temporal Ctx | 9.0 | 9.4 | Control (Path) 1 Occipital Ctx | 76.8 | 78.5 |
| AD 5 Inf Temporal Ctx | 84.1 | 83.5 | Control (Path) 2 Occipital Ctx | 9.5 | 8.4 |
| AD 5 Sup Temporal Ctx | 19.5 | 17.0 | Control (Path) 3 Occipital Ctx | 0.2 | 0.3 |
| AD 6 Inf Temporal Ctx | 18.0 | 20.2 | Control (Path) 4 Occipital Ctx | 13.0 | 14.1 |
| AD 6 Sup Temporal Ctx | 28.7 | 26.4 | Control 1 Parietal Ctx | 1.0 | 1.1 |
| Control 1 Temporal Ctx | 1.0 | 0.7 | Control 2 Parietal Ctx | 26.6 | 26.4 |
| Control 2 Temporal Ctx | 31.6 | 42.6 | Control 3 Parietal Ctx | 18.2 | 15.4 |
| Control 3 Temporal Ctx | 9.0 | 11.4 | Control (Path) 1 Parietal Ctx | 71.2 | 73.7 |
| Control 3 Temporal Ctx | 2.2 | 1.9 | Control (Path) 2 Parietal Ctx | 17.7 | 16.6 |
| Control (Path) 1 Temporal Ctx | 52.5 | 54.7 | Control (Path) 3 Parietal Ctx | 0.6 | 0.5 |
| Control (Path) 2 Temporal Ctx | 32.1 | 37.9 | Control (Path) 4 Parietal Ctx | 44.1 | 43.2 |

**Table CC. General_screening_panel_v1.4**

| **Column A - Rel. Exp.(%) Ag2503, Run 208015585** | | | | | |
|---|---|---|---|---|---|
| **Column B - Rel. Exp.(%) Ag2503, Run 212142287** | | | | | |
| **Tissue Name** | **A** | **B** | **Tissue Name** | **A** | **B** |
| Adipose | 0.0 | 0.0 | Renal ca. TK-10 | 0.0 | 0.0 |
| Melanoma* Hs688(A).T | 0.0 | 0.0 | Bladder | 0.1 | 0.1 |
| Melanoma* Hs688(B).T | 0.0 | 0.0 | Gastric ca. (liver met.) NCI-N87 | 0.1 | 0.1 |
| Melanoma* M14 | 0.0 | 0.0 | Gastric ca. KATO III | 0.0 | 0.0 |
| Melanoma* LOXIMVI | 0.0 | 0.1 | Colon ca. SW-948 | 0.0 | 0.0 |
| Melanoma* SK-MEL-5 | 0.0 | 0.1 | Colon ca. SW480 | 0.0 | 0.1 |
| Squamous cell carcinoma SCC-4 | 0.0 | 0.0 | Colon ca.* (SW480 met) SW620 | 0.0 | 0.0 |
| Testis Pool | 0.2 | 0.3 | Colon ca. HT29 | 0.1 | 0.1 |
| Prostate ca.* (bone met) PC-3 | 0.0 | 0.0 | Colon ca. HCT-116 | 0.0 | 0.0 |
| Prostate Pool | 6.4 | 7.8 | Colon ca. CaCo-2 | 0.0 | 0.0 |
| Placenta | 0.0 | 0.0 | Colon cancer tissue | 0.1 | 0.2 |
| Uterus Pool | 0.0 | 0.0 | Colon ca. SW 1116 | 0.0 | 0.0 |
| Ovarian ca. OVCAR-3 | 0.0 | 0.0 | Colon ca. Colo-205 | 0.0 | 0.0 |
| Ovarian ca. SK-OV-3 | 0.0 | 0.1 | Colon ca. SW-48 | 0.0 | 0.0 |
| Ovarian ca. OVCAR-4 | 0.0 | 0.0 | Colon Pool | 0.2 | 0.1 |
| Ovarian ca. OVCAR-5 | 8.4 | 7.2 | Small Intestine Pool | 0.2 | 0.4 |
| Ovarian ca. IGROV-1 | 0.0 | 0.0 | Stomach Pool | 0.2 | 0.0 |
| Ovarian ca. OVCAR-8 | 0.0 | 0.0 | Bone Marrow Pool | 0.0 | 0.0 |
| Ovary | 0.0 | 0.1 | Fetal Heart | 0.0 | 0.0 |
| Breast ca. MCF-7 | 0.0 | 0.2 | Heart Pool | 0.0 | 0.1 |
| Breast ca. MDA-MB-231 | 0.0 | 0.0 | Lymph Node Pool | 0.1 | 0.1 |
| Breast ca. BT 549 | 0.0 | 0.0 | Fetal Skeletal Muscle | 0.1 | 0.0 |
| Breast ca. T47D | 8.1 | 15.4 | Skeletal Muscle Pool | 0.0 | 0.1 |
| Breast ca. MDA-N | 0.0 | 0.0 | Spleen Pool | 0.0 | 0.0 |
| Breast Pool | 0.9 | 0.5 | Thymus Pool | 0.4 | 0.7 |
| Trachea | 0.2 | 0.4 | CNS cancer (glio/astro) U87-MG | 0.0 | 0.0 |
| Lung | 0.0 | 0.0 | CNS cancer (glio/astro) U-118-MG | 0.1 | 0.1 |
| Fetal Lung | 0.0 | 0.1 | CNS cancer (neuro;met) SK-N-AS | 0.0 | 0.0 |
| Lung ca. NCI-N417 | 0.0 | 0.0 | CNS cancer (astro) SF-539 | 0.0 | 0.0 |
| Lung ca. LX-1 | 0.0 | 0.0 | CNS cancer (astro) SNB-75 | 0.0 | 0.0 |
| Lung ca. NCI-H146 | 1.8 | 1.8 | CNS cancer (glio) SNB-19 | 0.0 | 0.0 |
| Lung ca. SHP-77 | 0.5 | 0.5 | CNS cancer (glio) SF-295 | 0.0 | 0.0 |
| Lung ca. A549 | 0.0 | 0.0 | Brain (Amygdala) Pool | 55.9 | 49.7 |
| Lung ca. NCI-H526 | 0.0 | 0.0 | Brain (cerebellum) | 1.1 | 1.1 |
| Lung ca. NCI-H23 | 0.0 | 0.9 | Brain (fetal) | 25.9 | 38.4 |
| Lung ca. NCI-H460 | 2.0 | 0.1 | Brain (Hippocampus) Pool | 31.0 | 35.8 |
| Lung ca. HOP-62 | 0.1 | 0.0 | Cerebral Cortex Pool | **100.0** | 80.7 |
| Lung ca. NCI-H522 | 0.0 | 0.0 | Brain (Substantia nigra) Pool | 64.2 | 64.6 |
| Liver | 0.1 | 0.0 | Brain (Thalamus) Pool | 97.3 | **100.0** |
| Fetal Liver | 0.0 | 0.3 | Brain (whole) | 66.9 | 65.5 |
| Liver ca. HepG2 | 0.0 | 0.0 | Spinal Cord Pool | 6.4 | 5.3 |
| Kidney Pool | 0.0 | 0.1 | Adrenal Gland | 0.0 | 0.0 |
| Fetal Kidney | 1.1 | 2.2 | Pituitary gland Pool | 6.6 | 5.5 |
| Renal ca. 786-0 | 0.0 | 0.0 | Salivary Gland | 0.2 | 0.1 |
| Renal ca. A498 | 0.0 | 0.0 | Thyroid (female) | 0.0 | 0.0 |
| Renal ca. ACHN | 0.4 | 0.0 | Pancreatic ca. CAPAN2 | 0.0 | 0.1 |
| Renal ca. UO-31 | 0.0 | 0.0 | Pancreas Pool | 0.2 | 0.7 |

**Table CD. Panel 1.3D**

| **Column A - Rel. Exp.(%) Ag2503, Run 160838046** | | | | | |
|---|---|---|---|---|---|
| **Column B - Rel. Exp.(%) Ag2503, Run 165519979** | | | | | |
| **Tissue Name** | **A** | **B** | **Tissue Name** | **A** | **B** |
| Liver adenocarcinoma | 0.0 | 0.0 | Kidney (fetal) | 0.0 | 0.2 |
| Pancreas | 0.0 | 0.0 | Renal ca. 786-0 | 0.0 | 0.0 |
| Pancreatic ca. CAPAN 2 | 0.0 | 0.0 | Renal ca. A498 | 0.0 | 0.0 |
| Adrenal gland | 0.4 | 0.3 | Renal ca. RXF 393 | 0.0 | 0.0 |
| Thyroid | 0.0 | 0.0. | Renal ca. ACHN | 0.0 | 0.0 |
| Salivary gland | 0.1 | 0.1 | Renal ca. UO-31 | 0.0 | 0.0 |
| Pituitary gland | 6.0 | 3.0 | Renal ca. TK-10 | 0.0 | 0.0 |
| Brain (fetal) | 9.6 | 12.1 | Liver | 0.0 | 0.0 |
| Brain (whole) | 66.9 | 80.1 | Liver (fetal) | 0.0 | 0.0 |
| Brain (amygdala) | 27.0 | 21.2 | Liver ca. (hepatoblast) HepG2 | 0.0 | 0.0 |
| Brain (cerebellum) | 0.8 | 2.0 | Lung | 0.0 | 0.0 |
| Brain (hippocampus) | **100.0** | 33.2 | Lung (fetal) | 0.0 | 0.0 |
| Brain (substantia nigra) | 5.5 | 5.9 | Lung ca. (small cell) LX-1 | 0.0 | 0.1 |
| Brain (thalamus) | 93.3 | **100.0** | Lung ca. (small cell) NCI-H69 | 0.4 | 0.0 |
| Cerebral Cortex | 84.7 | 23.7 | Lung ca. (s.cell var.) SHP-77 | 0.2 | 0.1 |
| Spinal cord | 0.8 | 0.9 | Lung ca. (large cell)NCI-H460 | 0.0 | 0.2 |
| glio/astro U87-MG | 0.0 | 0.0 | Lung ca. (non-sm. cell) A549 | 0.0 | 0.0 |
| glio/astro U-118-MG | 0.1 | 0.0 | Lung ca. (non-s.cell) NCI-H23 | 0.0 | 0.1 |
| astrocytoma SW1783 | 0.0 | 0.0 | Lung ca. (non-s.cell) HOP-62 | 0.1 | 0.0 |
| neuro*; met SK-N-AS | 0.0 | 0.0 | Lung ca. (non-s.cl) NCI-H522 | 0.0 | 0.0 |
| astrocytoma SF-539 | 0.0 | 0.0 | Lung ca. (squam.) SW 900 | 0.0 | 0.0 |
| astrocytoma SNB-75 | 0.0 | 0.0 | Lung ca. (squam.) NCI-H596 | 0.2 | 0.7 |
| glioma SNB-19 | 0.1 | 0.0 | Mammary gland | 5.1 | 1.7 |
| glioma U251 | 0.0 | 0.0 | Breast ca.* (pl.ef) MCF-7 | 0.1 | 0.0 |
| glioma SF-295 | 0.1 | 0.0 | Breast ca.* (pl.ef) MDA-MB-231 | 0.0 | 0.0 |
| Heart (fetal) | 0.0 | 0.0 | Breast ca.* (pl.ef) T47D | 0.0 | 0.0 |
| Heart | 0.0 | 0.0 | Breast ca. BT-549 | 0.0 | 0.0 |
| Skeletal muscle (fetal) | 0.3 | 1.3 | Breast ca. MDA-N | 0.0 | 0.0 |
| Skeletal muscle | 0.0 | 0.0 | Ovary | 0.0 | 0.0 |
| Bone marrow | 0.0 | 0.0 | Ovarian ca. OVCAR-3 | 0.0 | 0.0 |
| Thymus | 0.0 | 0.0 | Ovarian ca. OVCAR-4 | 0.0 | 0.0 |
| Spleen | 0.0 | 0.5 | Ovarian ca. OVCAR-5 | 1.5 | 1.0 |
| Lymph node | 0.0 | 0.0 | Ovarian ca. OVCAR-8 | 0.0 | 0.0 |
| Colorectal | 0.1 | 0.0 | Ovarian ca. IGROV-1 | 0.0 | 0.0 |
| Stomach | 0.0 | 0.0 | Ovarian ca.* (ascites) SK-OV-3 | 0.0 | 0.3 |
| Small intestine | 0.1 | 0.2 | Uterus | 0.2 | 0.0 |
| Colon ca. SW480 | 0.0 | 0.0 | Placenta | 0.0 | 0.0 |
| Colon ca.* SW620(SW480 met) | 0.0 | 0.0 | Prostate | 3.2 | 1.7 |
| Colon ca. HT29 | 0.1 | 0.0 | Prostate ca.* (bone met)PC-3 | 0.0 | 0.0 |
| Colon ca. HCT-116 | 0.0 | 0.0 | Testis | 0.0 | 0.0 |
| Colon ca. CaCo-2 | 0.0 | 0.0 | Melanoma Hs688(A).T | 0.0 | 0.0 |
| Colon ca. tissue(ODO3866) | 0.1 | 0.2 | Melanoma* (met) Hs688(B).T | 0.0 | 0.0 |
| Colon ca. HCC-2998 | 0.0 | 0.1 | Melanoma UACC-62 | 0.0 | 0.0 |
| Gastric ca.* (liver met) NCI-N87 | 0.0 | 0.1 | Melanoma M14 | 0.0 | 0.0 |
| Bladder | 0.0 | 0.0 | Melanoma LOX IMVI | 0.0 | 0.0 |
| Trachea | 0.1 | 0.0 | Melanoma* (met) SK-MEL-5 | 0.0 | 0.0 |
| Kidney | 0.0 | 0.0 | Adipose | 0.0 | 0.0 |

**Table CE. Panel CNS_1**

| **Column A - Rel. Exp.(%) Ag2503, Run 171656392** | | | |
|---|---|---|---|
| **Tissue Name** | **A** | **Tissue Name** | **A** |
| BA4 Control | 31.9 | BA17 PSP | 22.2 |
| BA4 Control2 | 65.1 | BA17 PSP2 | 10.1 |
| BA4 Alzheimer's2 | 4.0 | Sub Nigra Control | 14.8 |
| BA4 Parkinson's | 70.2 | Sub Nigra Control2 | 9.9 |
| BA4 Parkinson's2 | 100.0 | Sub Nigra Alzheimer's2 | 2.9 |
| BA4 Huntington's | 41.2 | Sub Nigra Parkinson's2 | 18.9 |
| BA4 Huntington's2 | 2.1 | Sub Nigra Huntington's | 10.5 |
| BA4 PSP | 5.3 | Sub Nigra Huntington's2 | 10.7 |
| BA4 PSP2 | 24.7 | Sub Nigra PSP2 | 0.9 |
| BA4 Depression | 10.8 | Sub Nigra Depression | 0.5 |
| BA4 Depression2 | 9.0 | Sub Nigra Depression2 | 3.1 |
| BA7 Control | 42.3 | Glob Palladus Control | 0.5 |
| BA7 Control2 | 40.9 | Glob Palladus Control2 | 1.4 |
| BA7 Alzheimer's2 | 6.4 | Glob Palladus Alzheimer's | 3.1 |
| BA7 Parkinson's | 18.8 | Glob Palladus Alzheimer's2 | 1.0 |
| BA7 Parkinson's2 | 46.3 | Glob Palladus Parkinson's | 39.8 |
| BA7 Huntington's | 57.8 | Glob Palladus Parkinson's2 | 0.8 |
| BA7 Huntington's2 | 52.9 | Glob Palladus PSP | 0.0 |
| BA7 PSP | 35.4 | Glob Palladus PSP2 | 0.8 |
| BA_7.PSP2 | 25-9 | Glob Palladus Depression | 0.2 |
| BA7 Depression | 5.2 | Temp Pole Control | 17.3 |
| BA9 Control | 23.2 | Temp Pole Control2 | 50.0 |
| BA9 Control2 | 88.3 | Temp Pole Alzheimer's | 1.7 |
| BA9 Alzheimer's | 4.9 | Temp Pole Alzheimer's2 | 3.0 |
| BA9 Alzheimer's2 | 12.8 | Temp Pole Parkinson's | 19.1 |
| BA9 Parkinson's | 25.5 | Temp Pole Parkinson's2 | 18.4 |
| BA9 Parkinson's2 | 61.6 | Temp Pole Huntington's | 34.2 |
| BA9 Huntington's | 42.3 | Temp Pole PSP | 4-1 |
| BA9 Huntington's2 | 12.4 | Temp Pole PSP2 | 2.8 |
| BA9 PSP | 9.2 | Temp Pole Depression2 | 3.8 |
| BA9 PSP2 | 4.0 | Cing Gyr Control | 73.2 |
| BA9 Depression | 3.5 | Cing Gyr Control2 | 23.8 |
| DA9 Depression2 | 8.4 | Cing Gyr Alzheimer's | 19.6 |
| BA17 Control | 58.2 | Cing Gyr Alzheimer's2 | 3.7 |
| BA17 Control2 | 62.4 | Cing Gyr Parkinson's | 21.0 |
| BA17 Atzheimer's2 | 7.3 | Cing Gyr Parkinson's2 | 26.1 |
| BA17 Parkmson's | 31.9 | Cing Gyr Huntington's | 49.7 |
| BA17 Parkinson's2 | 57.8 | Cing Gyr Huntington's2 | 11.3 |
| BA 17 Huntington's | 32.8 | Cing Gyr PSP | 5.6 |
| BA17 Huntington's2 | 12.8 | Cing Gyr PSP2 | 3.1 |
| BA17 Depression | 2.6 | Cing Gyr Depression | 2.5 |
| BA17 Depression2 | 23.3 | Cing Gyr Depression2 | 7.3 |

**CNS_neurodegeneration_v1.0 Summary:** Data obtained from RTQPCR assays performed for this panel was analyized using multivariate analysis (ANOVA). The multivariate results obtained using Ag2503 exhibits this gene has high brain-preferential expression in the hippocampus, cortex, amygdala, substantia nigra and thalamus. These regions are susceptible to the neurodegeneration associated with Alzheimer's Disease, Parkinson's disease, Huntington's disease and other pathological neurodegenerative conditions. This gene encodes a protein that is homologous to a potassium channel. Potassium channels have been implicated in neurodegenerative diseases, including Alzheimer's Disease. It has been suggested that modulating these channels to reduce outward K+ current may provide an approach to reducing neuronal degeneration in patients with Alzheimer's disease. Therefore, agents that modulate the function of this gene product could potentially reduce neuronal degeneration in patients with Alzheimer's Disease and other neurodegenerative diseases.

In addition, defective potassium channels are known to cause several CNS disorders, including epilepsy and episodic ataxia with myokymia. Therefore, modulation of the expression or function of this gene product may potentially be useful as a treatment for the symptoms produced by ataxia and epilepsy.

### References:

Jhamandas JH, et al. J Neurophysiol 2001 Sep;86(3):1312-20
Chi X, et al. Neurosci Lett 2000 Aug 18;290(1):9-12
Piccini A, et al. Neuroreport 2000 May 15;11(7):1375-9
Yu SP, et al. Neurobiol Dis 1998 Aug;5(2):81-8
Colom LV, et al. J Neurochem 1998 May;70(5):1925-34

**General_screening_panel_v1.4 Summary:** Ag2503 Two experiments with the same probe and primer set produce reults that are in excellent agreement, with highest expression in the brain. Please see CNS_neurodegeneration_v1.0 for discussion of potential role in the central nervous system.

There is also moderate to low expression in normal prostate and in cell lines derived from breast, lung, and ovarian cancer. Thus, this expression could be used as a diagnostic marker for the presence of cancers in any of those tissues. Furthemiore,inhibition of the activity of the gene product by antibodies or small molecule inhibitors could potentially be used as a treatment of these cancers.

In both experiments, there is also significantly higher levels of expression in the fetal kidney (CTs=30-31) when compared to the adult kidney (CTs=35-36). Thus, expression of this gene could be used to differentiate between adult and fetal sources of this tissue. Furthermore, the higher levels of expression in the fetal kidney suggest that this gene product may be involved in the development of this organ. Thus, therapeutic modulation of the expression or function of the protein encoded by this gene may be useful in the treatment of diseases of the kidney.

Among tissues with metabolic function, the expression of this potassium channel homolog is highest in the pituitary gland and shows very good concordance between the two independent runs. Potassium channels are involved in regulation of secretion in pituitary cells and their modulation by therapeutics such as small molecule inhibitors or antibodies could be used to modulate specific secretory activities in the pituitary.

**Panel 1.3D Summary:** Ag2503 Two experiments with the same probe and primer set produce results that are in very good agreement, with highest expression in both experiments seen in the brain. Please see CNS_neurodegeneration_v1.0.

Moderate to low expression is also observed in some cancer cell lines (lung and ovary) as well as normal prostate and breast. Thus, this expression could be used as a diagnostic marker for lung and ovarian cancers. Furthermore, inhibition of the activity of this gene product through the application of antibodies or small molecule inhibitors could effective in the treatment of lung or ovarian cancers.

As in panel 1.4, expression of this gene among metabolic tissues is highest in the pituitary. Significantly lower levels of expression are seen in the adrenal gland and in fetal skeletal muscle. Potassium channels are involved in regulation of secretion in pituitary cells and their modulation by therapeutics such as small molecule inhibitors or antibodies could be used to modulate specific secretory activities in the pituitary, as well as in other tissues.

In both experiments, there is also significantly higher levels of expression in the fetal skeletal muscle(CTs=33) when compared to expression in adult skeletal muscle(CTs=40). Thus, expression of this gene could be used to differentiate between adult and fetal sources of this tissue. Furthermore, the higher levels of expression in fetal skeletal muscle suggest that this gene product may be involved in the development of the skeletal muscle in the fetus. Thus, therapeutic modulation of the expression or function of the protein encoded by this gene may be useful in the adult to restore mass or function to weak or dystrophic muscle.

**Panel CNS_1 Summary**: Ag2503 Ubiquitous expression in this panel confirms the presence in the brain of this protein product. See NS_neurodegeneration_v1.0 for discussion of potential role in the central nervous system.

### D. CG54236-02: Cysteinyl leukotriene CYSLT2 receptor

Expression of gene CG54236-02 was assessed using the primer-probe set Ag2695, described in Table DA. Results of the RTQ-PCR runs are shown in Tables DB, DC, DD, DE and DF.

**Table DA. Probe Name Ag2695**

| **Primers** | **Sequences** | **Length Start** | **Start Position** | **SEQ ID No** |
|---|---|---|---|---|
| Forward | 5'-gggaaatgggttgtccatatat-3' | 22 | 266 | 107 |
| Probe | TET-5'-tcctgcagccttataagaagtccaca-3' | 26 | 292 | 108 |
| Reverse | 5'-atctgaaatggccagatttagc-3' | 22 | 335 | 109 |

**Table DB. AI_comprehensive panel_v1.0**

| **Column A - Rel: Exp.(%) Ag2695, Run 249247284** | | | | | |
|---|---|---|---|---|---|
| **Column B - Rel. Exp.(%) Ag2695, Run 249259794** | | | | | |
| **Tissue Name** | **A** | **B** | **Tissue Name** | **A** | **B** |
| 110967 COPD-F | 7.1 | 2.5 | 112427 Match Control Psoriasis-F | 54.0 | 53.2 |
| 110980 COPD-F | 6.2 | 0.0 | 112418 Psoriasis-M | 0.0 | 6.4 |
| 110968 COPD-M | 6.9 | 7.9 | 112723 Match Control Psoriasis-M | 0.0 | 0.0 |
| 110977 COPD-M | 12.3 | 15.2 | 112419 Psoriasis-M | 12.5 | 12.5 |
| 110989 Emphysema-F | 45.1 | 28.5 | 112424 Match Control Psoriasis- M | 19.5 | 11.0 |
| 110992 Emphysema-F | 15.4 | 7.6 | 112420 Psoriasis-M | 85.9 | 75.3 |
| 110993 Emphysema-F | 11.9 | 4.8 | 112425 Match Control Psoriasis- M | 48.0 | 58.2 |
| 110994 Emphysema-F | 2.5 | 0.0 | 104689 (MF) OA Bone-Backus | 39.0 | 59.9 |
| 110995 Emphysema-F | 12.9 | 38.4 | 104690 (MF) Adj "Normal" Bone-Backus | 21.8 | 27.7 |
| 110996 Emphysema-F | 12.2 | 0.0 | 104691 (MF) OA Synovium-Backus | 77.4 | 68.3 |
| 110997 Asthma-M | 4.2 | 3.2 | 104692 (BA) OA Cartilage-Backus | 0.0 | 0.0 |
| 111001 Asthma-F | 46.3 | 25.2 | 104694 (BA) OA Bone-Backus | 48.3 | 47.0 |
| 111002 Asthma-F | 52.5 | 27.5 | 104695 (BA) Adj "Normal" Bone-Backus | 24.8 | 21.5 |
| 111003 Atopic Asthma-F | 58.2 | 46.0 | 104696 (BA) OA Synovium-Backus | 81.8 | 49.7 |
| 111004 Atopic Asthma-F | 42.3 | 53.2 | 104700 (SS) OA Bone-Backus | 26.8 | 15.2 |
| 111005 Atopic Asthma-F | 26.4 | 26.8 | 104701 (SS) Adj "Normal" Bone-Backus | 13.5 | 11.4 |
| 111006 Atopic Asthma-F | 7.1 | 13.7 | 104702 (SS) OA Synovium-Backus | 62.0 | 49.0 |
| 111417 Allergy-M | 20.0 | 18.8 | 117093 OA Cartilage Rep7 | 89.5 | 59.0 |
| 112347 Allergy-M | 0.0 | 0.0 | 112672 OA Bone5 | 16.7 | 38.2 |
| 112349 Normal Lung-F | 0.0 | 0.0 | 112673 OA Synovium5 | 15.7 | 11.3 |
| 112357 Normal Lung-F | 34.4 | 28.9 | 112674 OA Synovial Fluid cells5 | 0.0 | 12.9 |
| 112354 Normal Lung-M | 98.6 | 55.5 | 17100 OA Cartilage Rep14 | 7.6 | 6.1 |
| 112374 Crohns-F | 5.6 | 7.2 | 112756 OA Bone9 | 22.5 | 7.9 |
| 112389 Match Control Crohns-F | 11.0 | 5.2 | 112757 OA Synovium9 | 1.7 | 0.0 |
| 112375 Crohns-F | 7.8 | 2.5 | 112758 OA Synovial Fluid Cells9 | 6.7 | 20.4 |
| 112732 Match Control Crohns-F | 43.5 | 18.6 | 117125 RA Cartilage Rep2 | 2.3 | 4.7 |
| 112725 Crohns-M | 3.2 | 0.0 | 113492 Bone2 RA | 42.3 | 26.8 |
| 112387 Match Control Crohns-M | 2.1 | 8.0 | 113493 Synovium2 RA | 16.6 | 3.7 |
| 112378 Crohns-M | 0.0 | 0.0 | 113494 Syn Fluid Cells RA | 31.2 | 23.0 |
| 112390 Match Control Crohns-M | 28.1 | 32.5 | 113499 Cartilage4 RA | 20.9 | 20.0 |
| 112726 Crohns-M | 23.2 | 25.3 | 113500 Bone4 RA | 22.8 | 39.5 |
| 112731 Match Control Crohns-M | 20.0 | 15.3 | 113501 Synovium4 RA | 26.6 | 24.8 |
| 112380 Ulcer Col-F | 11.3 | 20.7 | 113502 Syn Fluid Cells4 RA | 26.2 | 19.2 |
| 112734 Match Control Ulcer Col-F | 82.9 | 43.2 | 113495 Cartilage3 RA | 22.4 | 16.5 |
| 112384 Ulcer Col-F | 24.7 | 25.9 | 113496 Bone3RA | 13.0 | 18.4 |
| 112737 Match Control Ulcer Col-F | 8.3 | 3.7 | 113497 Synovium3 RA | 7.0 | 9.9 |
| 112386 Ulcer Co1-F | 2.2 | 3.6 | 113498 Syn Fluid Cells3 RA | 25.9 | 27.4 |
| 112738 Match Control Ulcer Col-F | 10.5 | 4.1 | 117106 Normal Cartilage Rep20 | 6.9 | 1.9 |
| 112381 Ulcer Col-M | 1.5 | 0.0 | 113663 Bone3 Normal | 0.0 | 0.0 |
| 112735 Match Control Ulcer Col-M | 11.7 | 4.5 | 113664 Synovium3 Normal | 0.0 | 0.0 |
| 112382 Ulcer Col-M | 25.5 | 16.6 | 113665 Syn Fluid Cells3 Normal | 1.2 | 0.0 |
| 112394 Match Control Ulcer Col-M | 1.3 | 2.9 | 117107 Normal Cartilage Rep22 | 12.9 | 0.0 |
| 112383 Ulcer Col-M | 12.2 | 11.7 | 113667 Bone4 Normal | 50.7 | 45.4 |
| 112736 Match Control Ulcer Col-M | 15.7 | 5.6 | 113668 Synovium4 Normal | 50.0 | 64.6 |
| 112423 Psoriasis-F | 21.2 | 18.2 | 113669 Syn Fluid Cells4 Normal | **100.0** | **100.0** |

**Table DC. CNS_neurodegeneration_v1.0**

| **Column A - Rel. Exp.(%) Ag2695, Run 209751330** | | | | | |
|---|---|---|---|---|---|
| **Column B - Rel. Exp.(%) Ag2695, Run 219966626** | | | | | |
| **Tissue Name** | **A** | **B** | **Tissue Name** | **A** | **B** |
| AD 1 Hippo | 20.4 | 10.6 | Control (Path) 3 Temporal Ctx | 49.7 | 19.6 |
| AD 2 Hippo | 51.4 | 39.0 | Control (Path) 4 Temporal Ctx | 80.1 | 71.7 |
| AD 3 Hippo | 16.0 | 9.4 | AD 1 Occipital Ctx | 19.1 | 23.5 |
| AD 4 Hippo | 17.9 | 10.7 | AD 2 Occipital Ctx (Missing) | 0.0 | 0.0 |
| AD 5 hippo | **100.0** | 88.9 | AD 3 Occipital Ctx | 17.4 | 8.7 |
| AD 6 Hippo | 33.9 | 25.2 | AD 4 Occipital Ctx | 51.8 | 33.7 |
| Control 2 Hippo | 22.8 | 21.6 | AD 5 Occipital Ctx | 14.5 | 22.5 |
| Control 4 Hippo | 48.3 | 22.7 | AD 6 Occipital Ctx | 59.0 | 44.4 |
| Control (Path) 3 Hippo | 24.8 | 9.9 | Control 1 Occipital Ctx | 43.2 | 34.9 |
| AD 1 Temporal Ctx | 26.6 | 31.4 | Control 2 Occipital Ctx | 80.1 | 57.0 |
| AD 2 Temporal Ctx | 29.5 | 40.1 | Control 3 Occipital Ctx | 46.3 | 37.6 |
| AD 3 Temporal Ctx | 7.1 | 14.2 | Control 4 Occipital Ctx | 37.1 | 11.2 |
| AD 4 Temporal Ctx | 41.5 | 41.5 | Control (Path) 1 Occipital Ctx | 87.7 | 85.3 |
| AD 5 Inf Temporal Ctx | 88.3 | 100.0 | Control (Path) 2 Occipital Ctx | 50.3 | 31.0 |
| AD 5 SupTemporal Ctx | 62.9 | 44.1 | Control (Path) 3 Occipital Ctx | 36.6 | 22.2 |
| AD 6 Inf Temporal Ctx | 35.1 | 26.4 | Control (Path) 4 Occipital Ctx | 84.7 | 71.7 |
| AD 6 Sup Temporal Ctx | 40.3 | 33.2 | Control 1 Parietal Ctx | 60.7 | 48.6 |
| Control 1 Temporal Ctx | 49.0 | 46.0 | Control 2 Parietal Ctx | 55.5 | 40.9 |
| Control 2 Temporal Ctx | 51.4 | 42.3 | Control 3 Parietal Ctx | 22.8 | 10.9 |
| Control 3 Temporal Ctx | 28.5 | 31.9 | Control (Path) 1 Parietal Ctx | 97.3 | 58.2 |
| Control 4 temporal Ctx | 22.7 | 36.3 | Control (Path) 2 Parietal Ctx | 76.3 | 46.3 |
| Control (Path) 1 Temporal Ctx | 90.8 | 70.2 | Control (Path) 3 Parietal Ctx | 25.0 | 28.5 |
| Control (Path) 2 Temporal Ctx | 64.2 | 50.3 | Control (Path) 4 Parietal Ctx | 84.7 | 72.2 |

**Table DD. Panel 1.3D**

| **Column A - Rel. Exp.(%) Ag2695, Run 153140732** | | | | | |
|---|---|---|---|---|---|
| **Column B - Rel. Exp.(%) Ag2695, Run 153830081** | | | | | |
| **Tissue Name** | **A** | **B** | **Tissue Name** | **A** | **B** |
| Liver adenocarcinoma | 0.0 | 0.0 | Kidney (fetal) | 0.0 | 3.9 |
| Pancreas | 2.1 | 4.2 | Renal ca. 786-0 | 0.0 | 0.0 |
| Pancreatic ca. CAPAN 2 | 0.0 | 0.0 | Renal ca. A498 | 0.0 | 1.9 |
| Adrenal gland | 67.4 | 100.0 | Renal ca. RXF 393 | 0.0 | 0.0 |
| Thyroid | 5.1 | 2.0 | Renal ca. ACHN | 0.0 | 0.0 |
| Salivary gland | 5.1 | 3.5 | Renal ca. UO-31 | 0.0 | 0.0 |
| Pituitary gland | 1.0 | 4.9 | Renal ca. TK-10 | 0.0 | 0.0 |
| Brain (fetal) | 3.5 | 0.0 | Liver | 1.5 | 3.9 |
| Brain (whole) | 20.3 | 16.0 | Liver (fetal) | 0.0 | 2.5 |
| Brain (amygdala) | 22.2 | 12.5 | Liver ca. (hepatoblast) HepG2 | 0.0 | 0.0 |
| Brain (cerebellum) | 2.1 | 4.5 | Lung | 18.8 | 16.2 |
| Brain (hippocampus) | 61.1 | 35.1 | Lung (fetal) | 3.7 | 3.7 |
| Brain (substantia nigra) | 9.2 | 8.8 | Lung ca. (small cell) LX-1 | 0.0 | 0.0 |
| Brain (thalamus) | 8.7 | 18.6 | Lung ca. (small cell) NCI-H69 | 0.0 | 0.0 |
| Cerebral Cortex | 45.1 | 53.2 | Lung ca. (s.cell var.) SHP-77 | 0.0 | 0.0 |
| Spinal cord | 11.3 | 8.0 | Lung ca. (large cell)NCI-H460 | 0.0 | 0.0 |
| glio/astro U87-MG | 0.0 | 0.0 | Lung ca. (non-sm. cell) A549 | 0.0 | 0.0 |
| glio/astro U-118-MG | 4.2 | 0.0 | Lung ca. (non-s.cell) NCI-H23 | 0.0 | 1.8 |
| astrocytoma SW1783 | 0.0 | 0.0 | Lung ca. (non-s.cell) HOP-62 | 0.0 | 0.0 |
| neuro*; met SK-N-AS | 0.0 | 0.0 | Lung ca. (non-s.cl) NCI-H522 | 0.0 | 0.0 |
| astrocytoma SF-539 | 9.0 | 0.0 | Lung ca. (squam.) SW 900 | 0.0 | 0.0 |
| astrocytoma SNB-75 | 0.0 | 0.0 | Lung ca. (squam.) NCI-H596 | 2.3 | 0.0 |
| glioma SNB-19 | 0.0 | 2.9 | Mammary gland | 5.2 | 9.0 |
| glioma U2S1 | 0.0 | 0.0 | Breast ca.* (pl.ef) MCF-7 | 0.0 | 0.0 |
| glioma SF-295 | 0.0 | 2.0 | Breast ca.* 231 (pl.ef) MDA-MB- 231 | 0.0 | 0.0 |
| Heart (fetal) | 17.4 | 16.5 | Breast ca.* (pl.ef) T47D | 0.0 | 0.0 |
| Heart | 41.5 | 33.0 | Breast ca. BT-549 | 0.0 | 0.0 |
| Skeletal muscle (fetal) | 8.6 | 5.2 | Breast ca. MDA-N | 0.0 | 0.0 |
| Skeletal muscle | 0.0 | 0.0 | Ovary | 16.2 | 9.1 |
| Bone marrow | 3.5 | 11.7 | Ovarian ca. OVCAR-3 | 0.0 | 0.0 |
| Thymus | 1.4 | 6.8 | Ovarian ca. OVCAR-4 | 0.0 | 0.0 |
| Spleen | **100.0** | 59.0 | Ovarian ca. OVCAR-5 | 0.0 | 0.0 |
| Lymph node | 32.3 | 26.8 | Ovarian ca. OVCAR-8 | 0.0 | 0.0 |
| Colorectal | 17.9 | 20.2 | Ovarian ca. IGROV-1 | 0.0 | 0.0 |
| Stomach | 9.5 | 0.0 | Ovarian ca.* (ascites) SK-OV-3 | 0.0 | 0.0 |
| Small intestine | 13.4 | 39.0 | Uterus | 6.5 | 4.3 |
| Colon ca. SW480 | 0.0 | 0.0 | Placenta | 49.0 | 32.5 |
| Colon ca.* SW620(SW480 met) | 0.0 | 0.0 | Prostate | 15.0 | 2.2 |
| Colon ca. HT29 | 0.0 | 1.9 | Prostate ca.* (bone met)PC-3 | 0.0 | 0.0 |
| Colon ca.HCT-116 | 0.0 | 0.0 | Testis | 2.4 | 11.0 |
| Colon ca. CaCo-2 | 0.0 | 0.0 | Melanoma Hs688(A).T | 0.0 | 0.0 |
| Colon ca. tissue(OD03866) | 11.0 | 3.4 | Melanoma* (met) Hs688(B).T | 0.0 | 0.0 |
| Colon ca. HCC-2998, | 0.6 | 0.0 | Melanoma UACC-62 | 0.0 | 0.0 |
| Gastric ca.* (liver met) NCI-N87 | 0.0 | 1.9 | Melanoma M14 | 0.0 | 0.0 |
| Bladder | 0.0 | 2.4 | Melanoma LOX IMVI | 0.0 | 0.0 |
| Trachea | 4.2 | 0.0 | Melanoma* (met) SK-MEL-5 | 4.0 | 4.3 |
| Kidney | 2.4 | 0.0 | Adipose | 15.5 | 16.7 |

**Table DE. Panel 2D**

| **Column A - Rel. Exp.(%) Ag2695, Run 153140795** | | | | | |
|---|---|---|---|---|---|
| **Column B - Rel. Exp.(%) Ag2695, Run 153789847** | | | | | |
| **Tissue Name** | **A** | **B** | **Tissue Name** | **A** | **B** |
| Normal Colon | 3.8 | 3.3 | Kidney Margin 8120608 | 1.4 | 0.5 |
| CC Well to Mod Diff (ODO3866) | 1.5 | 0.6 | Kidney Cancer 8120613 | 0.1 | 0.1 |
| CC Margin (ODO3866) | 2.7 | 0.5 | Kidney Margin 8120614 | 0.6 | 0.0 |
| CC Gr.2 rectosigmoid (ODO3868) | 0.1 | 0.2 | Kidney Cancer 9010320 | 4.0 | 2.5 |
| CC Margin (ODO3868) | 0.9 | 0.3 | Kidney Margin 9010321 | 0.4 | 1.1 |
| CC Mod Diff (OD03920) | 0.6 | 0.2 | Normal Uterus | 0.4 | 0.3 |
| CC Margin (ODO3920) | 0.5 | 0.3 | Uterus Cancer 064011 | 1.2 | 0.9 |
| CC Gr.2 ascend colon (ODO3921) | 3.2 | 2.2 | Normal Thyroid | 0.6 | 0.8 |
| CC Margin (ODO3921) | 0.8 | 0.6 | Thyroid Cancer 064010 | 7.3 | 5.7 |
| CC from Partial Hepatectomy (ODO4309) Mets | 1.6 | 2.0 | Thyroid Cancer A302152 Thyroid Cancer A302152 | 7.1 | 8.9 |
| Liver Margin (ODO4309) | 1.3 | 1.5 | Thyroid Margin A302153 | 1.3 | 1.1 |
| Colon mets to lung (OD04451-01) | 0.3 | 1.2 | Normal Breast | 1.1 | 1.8 |
| Lung Margin (OD04451-02) | 2.9 | 1.7 | Breast Cancer (OD04566) | 0.2 | 1.3 |
| Normal Prostate 6546-1 | 2.0 | 0.8 | Breast Cancer (OD04590-01) | 2.2 | 2.0 |
| Prostate Cancer (OD04410) | 1.9 | 1.7 | Breast Cancer Mets (ODO4590-03) | 6.7 | 8.5 |
| Prostate Margin (OD04410) | 3.5 | 3.9 | Breast Cancer Metastasis (OD04655-05) | 2.7 | 2.7 |
| Prostate Cancer (OD04720-01) | 1.7 | 0.5 | Breast Cancer 064006 | 0.6 | 0.6 |
| Prostate Margin (OD04720-02) | 5.0 | 4.4 | Breast Cancer 1024 | 0.7 | 0.6 |
| Normal Lung 061010 | 6.9 | 7.8 | Breast Cancer 9100266 | 0.1 | 0.3 |
| Lung Met to Muscle (ODO4286) | 2.2 | 2.0 | Breast Margin 9100265 | 0.2 | 0.5 |
| Muscle Margin (ODO4286) | 0.3 | 0.5 | Breast Cancer A209073 | 2.3 | 1.7 |
| Lung Malignant (OD03126) | 2.4 | 3.7 | Cancer Breast Margin A209073 | 0.4 | 0.3 |
| Lung Margin (OD03126) | 6.9 | 6.7 | Normal Liver | 0.6 | 0.2 |
| Lung Cancer (OD04404) | 5.5 | 2.1 | Liver Cancer 064003 | 0.8 | 0.0 |
| Lung Margin (OD04404) | 1.6 | 1.9 | Liver Cancer 1025 | 0.2 | 0.2 |
| Lung Cancer (OD04565) | 0.6 | 0.5 | Liver Cancer 1026 | 0.8 | 0:6 |
| Lung Margin (OD04565) | 1.0 | 2.0 | Liver Cancer 6004-T | 0.4 | 0.5 |
| Lung Cancer (OD04237-01) | 4.5 | 3.8 | Liver Tissue 6004-N | 0.9 | 0.3 |
| Lung Margin (OD04237-02) | 3.9 | 5.2 | Liver Cancer 6005-T | 0.2 | 0.2 |
| Ocular Mel Met to Liver (ODO4310) | 0.0 | 0.2 | Liver Tissue 6005-N | 0.3 | 0.3 |
| Liver Margin (ODO4310) | 1.7 | 0.7 | Normal Bladder | 0.9 | 1.5 |
| Melanoma Mets to Lung (OD04321) | **100.0** | **100.0** | Bladder Cancer 1023 | 0.1 | 0.4 |
| Lung Margin (OD04321) | 7.3 | 6.5 | Bladder Cancer A302173 | 0.5 | 1.2 |
| Normal Kidney | 6.4 | 5.0 | Bladder Cancer (OD04718-01) | 1.8 | 1.2 |
| Kidney Ca, Nuclear grade 2 (OD04338) | 34.2 | 33.9 | Bladder Normal Adjacent (OD04718-03) | 2.2 | 1.9 |
| Kidney Margin (OD04338) | 5.2 | 5.6 | Normal Ovary | 0.6 | 0.2 |
| Kidney Ca Nuclear grade 1/2 (OD04339) | 1.4 | 0.8 | Ovarian Cancer 064008 | 2.9 | 1.7 |
| Kidney Margin (OD04339) | 0.9 | 2.0 | Ovarian Cancer 07) (OD04768- | 14.4 | 10.8 |
| Kidney Ca, Clear cell type (OD04340) | 8.5 | 10.3 | Ovary Margin (OD04768-08) | 1.4 | 1.0 |
| Kidney Margin (OD04340) | 11.3 | 6.6 | Normal Stomach | 0.6 | 1.5 |
| Kidney Ca, Nuclear grade 3 (OD04348) | 1.4 | 1.9 | Gastric Cancer 9060358 | 0.5 | 0.7 |
| Kidney Margin (OD04348) | 5.4 | 7.3 | Stomach Margin 9060359 | 0.6 | 0.6 |
| Kidney Cancer (OD04622-01) | 49.0 | 63.7 | Gastric Cancer 9060395 | 1.1 | 1.2 |
| Kidney Margin (OD04622-03) | 0.7 | 1.2 | Stomach Margin 9060394 | 1.2 | 0.4 |
| Kidney Cancer (OD04450-01) | 1.4 | 1.4 | Gastric Cancer 9060397 | 0.9 | 0.9 |
| Kidney Margin (OD04450-03) | 5.7 | 4.6 | Stomach Margin 9060396 | 1.7 | 1.1 |
| Kidney Cancer 8120607 | 0.2 | 0.0 | Gastric Cancer 064005 | 3.0 | 2.6 |

**Table DF. Panel 4D**

| **Column A - Rel. Exp.(%) Ag2695, Run 153140809** | | | | | |
|---|---|---|---|---|---|
| **Column B - Rel. Exp.(%) Ag2695, Run 153766369** | | | | | |
| **Tissue Name** | **A** | **B** | **Tissue Name** | **A** | **B** |
| Secondary Th1 act | 1.0 | 1.6 | HUVEC IL-1beta | 0.0 | 0.0 |
| Secondary Th2 act | 21.0 | 29.5 | HUVEC IFN gamma | 0.0 | 0.0 |
| Secondary Tr1 act | 5.0 | 10.8 | HUVEC TNF alpha + IFN gamma | 0.0 | 0.0 |
| Secondary Th1 rest | 0.6 | 3.8 | HUVEC TNF alpha + IL4 | 0.0 | 0.0 |
| Secondary Th2 rest | 13.9 | 15.4 | HUVEC IL-11 | 0.0 | 0.0 |
| Secondary Tr1 rest | 6.1 | 5.0 | Lung Microvascular EC none | 0.0 | 0.0 |
| Primary Th1 act | 0.0 | 2.6 | Lung Microvascular EC TNFalpha + IL-1beta | 0.0 | 0.0 |
| Primary Th2 act | 39.0 | 54.3 | Microvascular Dermal EC none | 0.0 | 0.0 |
| Primary Tr1 act | 10.7 | 21.6 | Microsvasular Dermal EC TNFalpha + IL-1beta | 0.0 | 0.0 |
| Primary Th1 rest | 21.0 | 24.7 | Bronchial epithelium TNFalpha + IL1beta | 0.0 | 0.0 |
| Primary Th2 rest | 26.1 | 21.2 | Small airway epithelium none | 0.0 | 0.0 |
| Primary Tr1 rest | 11.7 | 19.1 | Small airway epithelium TNFalpha + IL-1beta | 0.0 | 0.0 |
| CD45RA CD4 lymphocyte act | 9.6 | 7.1 | Coronery artery SMC rest | 0.0 | 0.0 |
| CD45RO CD4 lymphocyte act | 15.6 | 17.3 | Coronery artery SMC TNFalpha + IL-1beta | 0.0 | 0.0 |
| CD8 lymphocyte act | 11.7 | 9.3 | Astrocytes rest | 0.0 | 0.5 |
| Secondary CD8 lymphocyte rest | 8.4 | 9.0 | Astrocytes TNFalpha + IL-1beta | 0.0 | 0.0 |
| Secondary CD8 lymphocyte act | 22.2 | 18.3 | KU-812 (Basophil) rest | 0.5 | 2.4 |
| CD4 lymphocyte none | 18.6 | 9.1 | KU-812 (Basophil) PMA/ionomycin | 2.9 | 1.7 |
| 2ry Th1/Th2/Tr1_anti-CD95 CH11 | 3.8 | 7.6 | CCD1106 (Keratinocytes) none | 0.0 | 0.7 |
| LAK cells rest | 43.2 | 32.5 | CCD1106 (Keratinocytes) TNFalpha + IL-1beta | 0.0 | 0.0 |
| LAK cells IL-2 | 15.9 | 18.0 | Liver cirrhosis | 4.8 | 6.3 |
| LAK cells IL-2+IL-12 | 32.3 | 30.8 | Lupus kidney | 0.6 | 0.0 |
| LAK cells IL-2+IFN gamma | 62.9 | 57.8 | NCI-H292 none | 0.0 | 0.0 |
| LAK cells IL-2+ IL-18 | 39.2 | 52.1 | NCI-H292 IL-4 | 0.0 | 0.0 |
| LAK cells PMA/ionomycin | 43.5 | 52.9 | NCI-H292 IL-9 | 0.0 | 0.0 |
| NK Cells IL-2 rest | 37.4 | 35.6 | NC1-H292 IL-13 | 0.0 | 0.0 |
| Two Way MLR 3 day | 26.1 | 23.0 | NCI-H292 IFN gamma | 0.0 | 0.0 |
| Two Way MLR 5 day | 17.0 | 10.7 | HPAEC none | 0.0 | 0.5 |
| Two Way MLR 7 day | 2.1 | 7.4 | HPAEC TNF alpha + IL-1 beta | 0.0 | 0.0 |
| PBMC rest | 19.9 | 28.3 | Lung fibroblast none | 0.0 | 0.0 |
| PBMC PWM | 28.3 | 31.4 | Lung fibroblast TNF alpha + IL-1 beta | 0.0 | 0.0 |
| PBMC PHA-L | 10.9 | 14.4 | Lung fibroblast IL-4 | 0.0 | 0.0 |
| Ramos (B cell) none | 0.0 | 0.1 | Lung fibroblast IL-9 | 0.0 | 0.0 |
| Ramos (B cell) ionomycin | 0.0 | 0.0 | Lung fibroblast IL-13 | 0.0 | 0.0 |
| B lymphocytes PWM | 9.0 | 9.5 | Lung fibroblast IFN gamma | 0.0 | 0.0 |
| B lymphocytes CD40L and IL-4 | 9.5 | 11.1 | Dermal fibroblast CCD1070 rest | 0.0 | 0.0 |
| EOL-1 dbcAMP | 1.1 | 0.0 | Dermal fibroblast CCD1070 TNF alpha | 17.1 | 20.3 |
| EOL-1 dbcAMP PMA/ionomycin | 3.7 | 0.6 | Dermal fibroblast CCD1070 IL-1 beta | 0.0 | 0.0 |
| Dendritic cells none | 3.7 | 10.3 | Dermal fibroblast IFN gamma | 0.0 | 0.0 |
| Dendritic cells LPS | 6.1 | 8.1 | Dermal fibroblast IL-4 | 1.1 | 0.0 |
| Dendritic cells anti-CD40 | 4.9 | 3.6 | IBD Colitis 2 | 1.1 | 0.0 |
| Monocytes rest | **100.0** | **100.0** | IBD Crohn's | 2.2 | 3.5 |
| Monocytes LPS | 0.0 | 0.3 | Colon | 21.5 | 15.6 |
| Macrophages rest | 0.6 | 3.7 | Lung | 17.6 | 14.4 |
| Macrophages LPS | 1.0 | 3.1 | Thymus | 11.8 | 7.1 |
| HUVEC none | 0.0 | 0.0 | Kidney | 33.7 | 20.3 |
| HUVEC starved | 0.0 | 0.0 | | | |

**Al_comprehensive panel_v1.0 Summary:** Ag2695 Two experiments with same probe-primer sets are in good agreement. Low expression of this gene is mainly seen in normal synovium and synovial fluid cells. Low expression of this gene is also seen in osteoarthritis bone, cartilage, synovium, RA bone, normal lung and a psoriasis sample.

**CNS_neurodegeneration_v1.0 Summary:** Data obtained from RTQPCR assays performed for this panel was analyized using multivariate analysis (ANOVA). The multivariate results obtained using Ag2695 in two experiments with same probe-primer sets are in good agreement. This gene is found to be down-regulated in the temporal cortex of Alzheimer's disease patients. Therefore, up-regulation of this gene or its gene product, or treatment with specific agonists for this protein encoded by this gene may be of use in reversing the dementia/memory loss associated with this disease and neuronal death.

**Panel 1.3D Summary:** Ag2695 Two experiments with same probe-primer sets are in good agreement. Highest expression of this gene is seen in adrenal gland and spleen (CTS=31.7). Significant expression of this gene is seen mainly in the normal tissues including brain, lymph node, heart, gastrointestinal tract, lung, ovary, placenta and adipose tissue. Interestingly, expression of this gene is low or undetectable in any of the cancer cell lines. Therefore, therapeutic modulation of this gene or its protein product may be useful in the treatment of cancer, metabolic and CNS disorders.

**Panel 2D Summary:** Ag2695 Two experiments with same probe-primer sets are in good agreement. Highest expression of this gene is detected in metastatic melanoma (CTs=26-27.8). High to moderate expression of this gene is also seen in normal and cancer samples from colon; lung, prostate, liver, prostate, thyroid, uterus, breast, ovary and stomach. Interestingly, expression of this gene is upregulated in ovarian, thyroid and kidney cancers compared to corresponding normal adjacent normal tissues. Therefore, therapeutic modulation of this gene or its protein product may be useful in the treatment of ovarian, thyroid and kidney cancers.

**Panel 4D Summary:** Ag2695 Two experiments with same probe-primer sets are in good agreement. Highest expression of this gene is seen in resting monocytes (CT=29.6). This gene is expressed by T lymphocytes prepared under a number of conditions at moderate levels and is expressed at significant levels in treated and untreated dendritic cells, LAK cells, PBMC, activated B lymphocytes, activated dermal fibroblasts, liver cirrhosis sample and normal tissues represented by colon, lung, thymus and kidney. Dendritic cells are powerful antigen-presenting cells (APC) whose function is pivotal in the initiation and maintenance of normal immune responses. Autoimmunity and inflammation may also be reduced by suppression of this function. Therefore, small molecule drugs or the antibodies that antagonize the function of this gene or its protein product may reduce or eliminate the symptoms in patients with several types of autoimmune and inflammatory diseases, such as lupus erythematosus, Crohn's disease, ulcerative colitis, multiple sclerosis, chronic obstructive pulmonary disease, asthma, emphysema, rheumatoid arthritis, or psoriasis.

### E. CG54566-01: Serotonin Receptor

Expression of gene CG54566-01 was assessed using the primer-probe set Ag1252, described in Table EA. Results of the RTQ-PCR runs are shown in Tables EB, EC, ED, EE, EF, EG and EH.

**Table EA. Probe Name Ag1252**

| **Primers** | **Sequences** | **Length** | **Start Position** | **SEQ ID No** |
|---|---|---|---|---|
| Forward | 5'-ccagtgccagtaccagaatatc-3' | 22 | 1210 | 110 |
| Probe | TET-5'-aaccagacactctcagctgcaggcat-3'-TAMRA | 26 | 1232 | 111 |
| Reverse | 5'-ctctctggcctctcagcaa-3' | 19 | 1275 | 112 |

**Table EB. AI_comprehensive panel_v1.0**

| **Column A - Rel. Exp.(%) Ag1252, Run 249259629** | | | |
|---|---|---|---|
| **Tissue Name** | **A** | **Tissue Name** | **A** |
| 110967 COPD-F | 0.0 | 112427 Match Control Psoriasis-F | **100.0** |
| 110980 COPD-F | 1.3 | 112418 Psoriasis-M | 16.2 |
| 110968 COPD-M | 5.5 | 112723 Match Control Psoriasis-M | 39.2 |
| 110977 COPD-M | 14.9 | 112419 Psoriasis-M | 37.9 |
| 110989 Emphysema-F | 74.7 | 112424 Match Control Psoriasis-M | 19.3 |
| 110992 Emphysema-F | 2.0 | 112420 Psoriasis-M | 65.1 |
| 110993 Emphysema-F | 24.5 | 112425 Match Control Psoriasis-M | 95.9 |
| 110994 Emphysema-F | 10.0 | 104689 (MF) OA Bone-Backus | 80.7 |
| 110995 Emphysema-F | 33.9 | 104690 (MF) Adj "Normal" Bone-Backus | 58.2 |
| 110996 Emphysema-F | 0.0 | 104691 (MF) OA Synovium-Backus | 73.2 |
| 110997 Asthma-M | 0.8 | 104692 (BA) OA Cartilage-Backus | 27.2 |
| 111001 Asthma-F | 3.3 | 104694 (BA) OA Bone-Backus | 45.1 |
| 111002 Asthma-F | 31.2 | 104695 (BA) Adj "Normal" Bone-Backus | 44.4 |
| 111003 Atopic Asthma-F | 32.8 | 104696 (BA) OA Synovium-Backus | 51.8 |
| 111004 Atopic Asthma-F | 34.4 | 104700 (SS) OA Bone-Backus | 22.4 |
| 111005 Atopic Asthma-F | 30.1 | 104701 (SS) Adj "Normal" Bone-Backus | 33.4 |
| 111006 Atopic Asthma-F | 11.9 | 104702 (SS) OA Synovium-Backus | 78.5 |
| 111417 Allergy-M | 21.0 | 117093 OA Cartilage Rep7 | 18.2 |
| 112347 Allergy-M | 0.9 | 112672 OA Bone5 | 47.0 |
| 112349 Normal Lung-F | 1.3 | 112673 OA Synovium5 | 17.8 |
| 112357 Normal Lung-F | 62.4 | 112674 OA Synovial Fluid cells5 | 15.9 |
| 112354 Normal Lung-M | 28.3 | 117100 OA Cartilage Rep14 | 6.3 |
| 112374 Crohns-F | 19.8 | 112756 OA Bone9 | 23.2 |
| 112389 Match Control Crohns-F | 9.3 | 112757 OA Synovium9 | 7.7 |
| 112375 Crohns-F | 25.0 | 112758 OA Synovial Fluid Cells9 | 15.7 |
| 112732 Match Control Crohns-F | 16.0 | 117125 RA Cartilage Rep2 | 39.5 |
| 112725 Crohns-M | 2.8 | 113492 Bone2 RA | 28.5 |
| 112387 Match Control Crohns-M | 8.3 | 113493 Synovium2 RA | 8.4 |
| 112378 Crohns-M | 2.6 | 113494 Syn Fluid Cells RA | 15.8 |
| 112390 Match Control Crohns-M | 56.6 | 113499 Cartilage4 RA | 17.7 |
| 112726 Crohns-M | 37.4 | 113500 Bone4 RA | 37.9 |
| 112731 Match Control Crohns-M | 24.3 | 113501 Synovium4 RA | 15.2 |
| 112380 Ulcer Col-F | 37.6 | 113502 Syn Fluid Cells4 RA | 15.3 |
| 112734 Match Control Ulcer Col-F | 35.4 | 113495 Cartilage3 RA | 12.8 |
| 112384 Ulcer Col-F | 42.3 | 113496 Bone3 RA | 29.5 |
| 112737 Match Control Ulcer Col-F | 11.2 | 113497 Synovium3 RA | 8.9 |
| 112386 Ulcer Col-F | 10.4 | 113498 Syn Fluid Cells3 RA | 24.5 |
| 112738 Match Control Ulcer Col-F | 2.8 | 117106 Normal Cartilage Rep20 | 2.1 |
| 112381 Ulcer Col-M | 3.6 | 113663 Bone3 Normal | 3.4 |
| 112735 Match Control Ulcer Col-M | 8.7 | 113664 Synovium3 Normal | 0.0 |
| 112382 Ulcer Col-M | 13.9 | 113665 Syn Fluid Cells3 Normal | 0.0 |
| 112394 Match Control Ulcer Col-M | 7.6 | 117107 Normal Cartilage Rep22 | 9.9 |
| 112383 Ulcer Col-M | 36.3 | 113667 Bone4 Normal | 18.4 |
| 112736 Match Control Ulcer Col-M | 11.2 | 113668 Synovium4 Normal | 15.6 |
| 112423 Psoriasis-F | 29.1 | 113669 Syn Fluid Cells4 Normal | 26.8 |

**Table EC. CNS_neurodegeneration_v1.0**

| **Column A - Rel. Exp.(%) Ag1252, Run 206228025** | | | | | |
|---|---|---|---|---|---|
| **Column B - Rel. Exp.(%) Ag1252, Run 219923396** | | | | | |
| **Tissue Name** | **A** | **B** | **Tissue Name** | **A** | **B** |
| AD 1 Hippo | 10.0 | 6.8 | Control (Path) 3 Temporal Ctx | 2.0 | 1.9 |
| AD 2 Hippo | 28.1 | 21.5 | Control (Path) 4 Temporal Ctx | 39.8 | 23.5 |
| AD 3 Hippo | 5.9 | 5.1 | AD 1 Occipital Ctx | 1.5 | 2.5 |
| AD 4 Hippo | 11.6 | 6.1 | AD 2 Occipital Ctx (Missing) | 0.0 | 0.0 |
| AD 5 Hippo | 67.4 | 80.7 | AD 3 Occipital Ctx | 1.3 | 1.7 |
| AD 6 Hippo | 55.5 | 22.8 | AD 4 Occipital Ctx | 14.6 | 11.0 |
| Control 2 Hippo | 47.3 | 38.4 | AD 5 Occipital Ctx | 59.9 | 77.9 |
| Control 4 Hippo | 0.0 | 8.7 | AD 6 Occipital Ctx | 11.2 | 20.4 |
| Control (Path) 3 Hippo | 1.0 | 2.4 | Control 1 Occipital Ctx | 2.5 | 1.8 |
| AD 1 Temporal Ctx | 6.6 | 1.4 | Control 2 Occipital Ctx | 54.3 | 45.1 |
| AD 2 Temporal Ctx | 20.0 | 13.8 | Control 3 Occipital Ctx | 18.4 | 7.3 |
| AD 3 Temporal Ctx | 2.8 | 0.8 | Control 4 Occipital Ctx | 6.3 | 2.0 |
| AD 4 Temporal Ctx | 15.7 | 10.9 | Control (Path) 1 Occipital Ctx | **100.0** | 63.3 |
| AD 5 Inf Temporal Ctx | 56.3 | **100.0** | Control (Path) 2 Occipital Ctx | 11.1 | 5.2 |
| AD 5 Sup Temporal Ctx | 30.4 | 62.4 | Control (Path) 3 Occipital Ctx | 3.8 | 4.8 |
| AD 6 Inf Temporal Ctx | 33.9 | 20.0 | Control (Path) 4 Occipital Ctx | 12.9 | 13.5 |
| AD 6 Sup Temporal Ctx | 33.4 | 24.7 | Control 1 Parietal Ctx | 4.8 | 1.3 |
| Control 1 Temporal Ctx | 5.8 | 4.8 | Control 2 Parietal Ctx | 29.3 | 94.0 |
| Control 2 Temporal Ctx | 77.9 | 46.0 | Control 3 Parietal Ctx | 18.8 | 16.0 |
| Control 3 Temporal Ctx | 17.3 | 18.3 | Control (Path) I Parietal Ctx | 100.0 | 83.5 |
| Control 3 Temporal Ctx | 8.5 | 3.9 | Control (Path) 2 Parietal Ctx | 23.5 | 16.7 |
| Control (Path) 1 Temporal Ctx | 74.7 | 47.0 | Control (Path) 3 Parietal Ctx | 3.2 | 2.0 |
| Control (Path) 2 Temporal Ctx | 70.7 | 21.0 | Control (Path) 4 Parietal Ctx | 59.5 | 30.8 |

**Table ED. General_screening_panel_v1.4**

| **Column A - Rel. Exp.(%) Ag1252, Run 212704837** | | | |
|---|---|---|---|
| **Tissue Name** | A | **Tissue Name** | **A** |
| Adipose | 11.8 | Renal ca. TK-10 | 6.0 |
| Melanoma* Hs688(A).T | 19.5 | Bladder | 4.8 |
| Melanoma* Hs688(B).T | 33.0 | Gastric ca. (liver met.) NCI-N87 | 20.0 |
| Melanoma* M14 | 19.8 | Gastric ca. KATO III | 0.2 |
| Melanoma* LOXIMVI | 35.4 | Colon ca. SW-948 | 12.7 |
| Melanoma* SK-MEL-5 | 6.3 | Colon ca. SW480 | 32.8 |
| Squamous cell carcinoma SCC-4 | 24.1 | Colon ca.* (SW480 met) SW620 | 7.9 |
| Testis Pool | 9.2 | Colon ca. HT29 | 1.3 |
| Prostate ca.* (bone met) PC-3 | 11.2 | Colon ca. HCT-116 | 49.0 |
| Prostate Pool | 7.6 | Colon ca. CaCo-2 | 9.3 |
| Placenta | 8.1 | Colon cancer tissue | 10.7 |
| Uterus Pool | 3.0 | Colon ca. SW1116 | 4.5 |
| Ovarian ca. OVCAR-3 | 12.0 | Colon ca. Colo-205 | 0.8 |
| Ovarian ca. SK-OV-3 | 15.4 | Colon ca. SW-48 | 1.3 |
| Ovarian ca. OVCAR-4 | 3.8 | Colon Pool | 10.6 |
| Ovarian ca. OVCAR-5 | 21.6 | Small Intestine Pool | 9.8 |
| Ovarian ca. IGROV-1 | 16.5 | Stomach Pool | 3.1 |
| Ovarian ca. OVCAR-8 | 9.5 | Bone Marrow Pool | 2.6 |
| Ovary | 18.3 | Fetal Heart | 2.5 |
| Breast ca. MCF-7 | 84.1 | Heart Pool | 4.2 |
| Breast ca. MDA-MB-231 | 23.5 | Lymph Node Pool | 102 |
| Breast ca. BT 549 | 34.6 | Fetal Skeletal Muscle | 1.8 |
| Breast ca. T47D | 59.5 | Skeletal Muscle Pool | 2.0 |
| Breast ca. MDA-N | 7:3 | Spleen Pool | 8.0 |
| Breast Pool | 9.5 | Thymus Pool | 18.0 |
| Trachea | 4.8 | CNS cancer (glio/astro) U87-MG | 67.4 |
| Lung | 3.3 | CNS cancer (glio/astro) U-118-MG | 38.4 |
| Fetal Lung | 10.2 | CNS cancer (neuro;met) SK-N-AS | 11.4 |
| Lung ca. NCI-N417 | 1.3 | CNS cancer (astro) SF-539 | 10.6 |
| Lung ca. LX-1 | 8.2 | CNS cancer (astro) SNB-75 | 20.3 |
| Lung ca. NCI-H146 | 38.2 | CNS cancer (glio) SNB-19 | 19.6 |
| Lung ca. SHP-77 | 2.3 | CNS cancer (glio) SF-295 | 67.4 |
| Lung ca. A549 | 15.1 | Brain (Amygdala) Pool | 10.1 |
| Lung ca. NCI-H526 | 1.2 | Brain (cerebellum) | 100.0 |
| Lung ca. NCI-H23 | 18.9 | Brain (fetal) | 17.2 |
| Lung ca. NCI-H460 | 11.9 | Brain (Hippocampus) Pool | 14.0 |
| Lung ca. HOP-62 | 29.9 | Cerebral Cortex Pool | 20.9 |
| Lung ca. NCI-H522 | 40.6 | Brain (Substantia nigra) Pool | 16.7 |
| Liver | 0.3 | Brain (Thalamus) Pool | 20.4 |
| Fetal Liver | 12.5 | Brain (whole) | 22.7 |
| Liver ca. HepG2 | 4.4 | Spinal Cord Pool | 5.4 |
| Kidney Pool | 16.7 | Adrenal Gland | 10.9 |
| Fetal Kidney | 11.6 | Pituitary gland Pool | 3.5 |
| Renal ca. 786-0 | 15.9 | Salivary Gland | 2.9 |
| Renal ca. A498 | 1.2 | Thyroid (female) | 8.4 |
| Renal ca. ACHN | 19.2 | Pancreatic ca. CAPAN2 | 11.1 |
| Renal ca. UO-31 | 32.1 | Pancreas Pool | 10.0 |

**Table EE. Panel 1.2**

| **Column A - Rel. Exp.(%) Ag1252, Run 12917_9690** | | | |
|---|---|---|---|
| **Tissue Name** | **A** | **Tissue Name** | **A** |
| Endothelial cells | 27.7 | Renal ca. 786-0 | 3.7 |
| Heart (Fetal) | 1.5 | Renal ca. A498 | 1.1 |
| Pancreas | 7.9 | Renal ca. RXF 393 | 0.9 |
| Pancreatic ca. CAPAN 2 | 2.9 | Renal ca. ACHN | 10.4 |
| Adrenal Gland | 8.3 | Renal ca. UO-31 | 11.0 |
| Thyroid | 15.7 | Renal ca. TK-10 | 3.9 |
| Salivary gland | 4.7 | Liver | 7.2 |
| Pituitary gland | 8.5 | Liver (fetal) | 3.7 |
| Brain (fetal) | 6.0 | Liver ca. (hepatoblast) HepG2 | 3.4 |
| Brain (whole) | 17.2 | Lung | 2.7 |
| Brain (amygdala) | 10.0 | Lung (fetal) | 3.1 |
| Brain (cerebellum) | 4.8 | Lung ca. (small cell) LX-1 | 4.3 |
| Brain (hippocampus) | 12.0 | Lung ca. (small cell) NCI-H69 | 1.8 |
| Brain (thalamus) | 12.2 | Lung ca. (s.cell var.) SHP-77 | 0.5 |
| Cerebral Cortex | 15.7 | Lung ca. (large cell)NCI-H460 | 27.4 |
| Spinal cord | 2.8 | Lung ca. (non-sm. cell) A549 | 6.4 |
| glio/astro U87-MG | 12.2 | Lung ca. (non-s.cell) NCI-H23 | 5.9 |
| glio/astro U-118-MG | 4.5 | Lung ca. (non-s.cell) HOP-62 | 26.4 |
| astrocytoma SW1783 | 1.9 | Lung ca. (non-s.cl) NCI-H522 | 100.0 |
| neuro*; met SK-N-AS | 4.2 | Lung ca. (squam.) SW 900 | 7.6 |
| astrocytoma SF-539 | 2.8 | Lung ca. (squam.) NCI-H596 | 2.4 |
| astrocytoma SNB-75 | 0.8 | Mammary gland | 11.2 |
| glioma SNB-19 | 11.0 | Breast ca.* (pl.ef) MCF-7 | 15.6 |
| glioma U251 | 6.9 | Breast ca.* (pl.ef) MDA-MB-231 | 2.3 |
| glioma SF-295 | 10.0 | Breast ca.* (pl. ef) T47D | 2.1 |
| Heart | 5.9 | Breast ca. BT-549 | 6.5 |
| Skeletal Muscle | 2.9 | Breast ca. MDA-N | 0.0 |
| Bone marrow | 2.4 | Ovary | 6.0 |
| Thymus | 1.5 | Ovarian ca. OVCAR-3 | 7.3 |
| Spleen | 2.9 | Ovarian ca. OVCAR-4 | 3.0 |
| Lymph node | 4.3 | Ovarian ca. OVCAR-5 | 14.1 |
| Colorectal Tissue | 1.1 | Ovarian ca. OVCAR-8 | 3.6 |
| Stomach | 3.3 | Ovarian ca. IGROV-1 | 10.4 |
| Small intestine | 3.7 | Ovarian ca. (ascites) SK-OV-3 | 6.7 |
| Colon ca. SW480 | 4.5 | Uterus | 3.5 |
| Colon ca.* SW620 (SW480 met) | 5.2 | Placenta | 13.4 |
| Colon ca. HT29 | 0.2 | Prostate | 5.9 |
| Colon ca. HCT-116 | 8.0 | Prostate ca.* (bone met) PC-3 | 12.8 |
| Colon ca. CaCo-2 | 4.1 | Testis | 7.4 |
| Colon ca. Tissue (ODO3866) | 1.2 | Melanoma Hs688(A).T | 4.3 |
| Colon ca. HCC-2998 | 8.1 | Melanoma* (met) Hs688(B).T | 3.8 |
| Gastric ca.* (liver met) NCI-N87 | 6.9 | Melanoma UACC-62 | 11.7 |
| Bladder | 12.0 | Melanoma M14 | 3.6 |
| Trachea | 2.6 | Melanoma LOX IMVI | 6.0 |
| Kidney | 8.4 | Melanoma* (met) SK-MEL-5 | 4.5 |
| Kidney (fetal) | 9.2 | | |

**Table EF. Panel 2D**

| **Column A - Rel. Exp.(%) Ag1252, Run 162309309** | | | |
|---|---|---|---|
| **Tissue Name** | **A** | **Tissue Name** | **A** |
| Normal Colon | 21.0 | Kidney Margin 8120608 | 2.2 |
| CC Well to Mod Diff (ODO3866) | 2.6 | Kidney Cancer 8120613 | 10.7 |
| CC Margin (OD03866) | 2.3 | Kidney Margin 8120614 | 2.9 |
| CC Gr.2 rectosigmoid (ODO3868) | 2.3 | Kidney Cancer 9010320 | 16.0 |
| CC Margin (ODO3868) | 0.2 | Kidney Margin 9010321 | 8.5 |
| CC Mod Diff (ODO3920) | 7.7 | Normal Uterus | 3.0 |
| CC Margin (ODO3920) | 4.2 | Uterus Cancer 064011 | 14.5 |
| CC Gr.2 ascend colon (ODO3921) | 7.3 | Normal Thyroid | 20.2 |
| CC Margin (ODO3921) | 3.6 | Thyroid Cancer 064010 | 8.9 |
| CC from Partial Hepatectomy (ODO4309) Mets | 9.1 | Thyroid Cancer A302152 | 11.0 |
| Liver Margin (ODO4309) | 9.4 | Thyroid Margin A302153 | 21.0 |
| Colon mets to lung (OD04451-01) | 7.0 | Normal Breast | 22.5 |
| Lung Margin (OD04451-02) | 7.5 | Breast Cancer (OD04566) | 40.1 |
| Normal Prostate 6546-1 | 44.1 | Breast Cancer (OD04590-01) | 20.4 |
| Prostate Cancer (OD04410) | 32.5 | Breast Cancer Mets (OD04590-03) | 26.1 |
| Prostate Margin (OD04410) | 18.7 | Breast Cancer Metastasis (OD04655-05) | **100.0** |
| Prostate Cancer (OD04720-01) | 21.8 | Breast Cancer 064006 | 10.7 |
| Prostate Margin (OD04720-02) | 31.2 | Breast Cancer 1024 | 15.7 |
| Normal Lung 061010 | 9.9 | Breast Cancer 9100266 | 14.8 |
| Lung Met to Muscle (OD04286) | 8.1 | Breast Margin 9100265 | 7.4 |
| Muscle Margin (OD04286) | 7.3 | Breast Cancer A209073 | 20.4 |
| Lung Malignant Cancer (OD03126) | 9.2 | Breast Margin A209073 | 11.4 |
| Lung Margin (OD03126) | 14.5 | Normal Liver | 11.1 |
| Lung Cancer (OD04404) | 14.5 | Liver Cancer 064003 | 2.9 |
| Lung Margin (OD04404) | 7.9 | Liver Cancer 1025 | 4.9 |
| Lung Cancer (OD04565) | 13.5 | Liver Cancer 1026 | 5.8 |
| Lung Margin (OD04565) | 7.9 | Liver Cancer 6004-T | 5.8 |
| Lung Cancer (OD04237-01) | 15.8 | Liver Tissue 6004-N | 6.1 |
| Lung Margin (OD04237-02) | 11.3 | Liver Cancer 6005-T | 8.5 |
| Ocular Mel Met to Liver (OD04310) | 6.7 | Liver Tissue 6005-N | 1.3 |
| Liver Margin (OD04310) | 6.5 | Normal Bladder | 15.3 |
| Melanoma Mets to Lung (OD04321) | 4.1 | Bladder Cancer 1023 | 2.9 |
| Lung Margin (OD04321) | 8.1 | Bladder Cancer A302173 | 8.5 |
| Normal Kidney | 27.5 | Bladder Cancer (OD04718-01) | 14.1 |
| Kidney Ca, Nuclear grade 2 (OD04338) | 17.9 | Bladder Normal Adjacent (OD04718-03) | 18.9 |
| Kidney Margin (OD04338) | 9.9 | Normal Ovary | 6.7 |
| Kidney Ca Nuclear grade 1/2 (OD04339) | 13.3 | Ovarian Cancer 064008 | 32.3 |
| Kidney Margin (OD04339) | 11.8 | Ovarian Cancer (OD04768-07) | 6.1 |
| Kidney Ca, Clear cell type (OD04340) | 23.2 | Ovary Margin (OD04768-08) | 4.6 |
| Kidney Margin (OD04340) | 14.8 | Normal Stomach | 5.6 |
| Kidney Ca, Nuclear grade 3 (OD04348) | 24.3 | Gastric Cancer 9060358 | 3.6 |
| Kidney Margin (OD04348) | 13.7 | Stomach Margin 9060359 | 5.3 |
| Kidney Cancer (OD04622-01) | 9.8 | Gastric Cancer 9060395 | 8.1 |
| Kidney Margin (OD04622-03) | 2.0 | Stomach Margin 9060394 | 4.5 |
| Kidney Cancer (OD04450-01) | 11.7 | Gastric Cancer 9060397 | 11.2 |
| Kidney Margin (OD04450-03) | 20.6 | Stomach Margin 9060396 | 2.0 |
| Kidney Cancer 8120607 | 1.1 | Gastric Cancer 064005 | 19.2 |

**Table EG. Panel 4D**

| **Column A - Rel. Exp.(%) Ag1252, Run 141902582** | | | |
|---|---|---|---|
| **Tissue Name** | **A** | **Tissue Name** | **A** |
| Secondary Th1 act | 1.0 | HUVEC IL-1beta | 23.3 |
| Secondary Th2 act | 4.6 | HUVEC IFN gamma | 55.9 |
| Secondary Tr1 act | 1.6 | HUVEC TNF alpha + IFN gamma | 8.6 |
| Secondary Th1 rest | 1.7 | HUVEC TNF alpha + IL4 | 21.5 |
| Secondary Th2 rest | 4.1 | HUVEC IL-11 | 31.9 |
| Secondary Tr1 rest | 2.3 | Lung Microvascular EC none | 58.6 |
| Primary Th1 act | 4.6 | Lung Microvascular EC TNFalpha + IL-1beta | 32.1 |
| Primary Th2 act | 4.5 | Microvascular Dermal EC none | 84.7 |
| Primary Tr1 act | 9.5 | Microsvasular Dermal EC TNFalpha + IL-1beta | 41.8 |
| Primary Th1 rest | 12.9 | Bronchial epithelium TNFalpha + IL-1beta | 27.4 |
| Primary Th2 rest | 8.5 | Small airway epithelium none | 13.3 |
| Primary Tr1 rest | 15.8 | Small airway epithelium TNFalpha + IL-1beta | 61.6 |
| CD45RA CD4 lymphocyte act | 12.6 | Coronery artery SMC rest | 50.7 |
| CD45RO CD4 lymphocyte act | 5.6 | Coronery artery SMC TNFalpha + IL-1beta | 20.3 |
| CD8 lymphocyte act | 5.4 | Astrocytes rest | 7.0 |
| Secondary CD8 lymphocyte rest | 3.2 | Astrocytes TNFalpha + IL-1beta | 13.2 |
| Secondary CD8 lymphocyte act | 1.9 | KU-812 (Basophil) rest | 9.2 |
| CD4 lymphocyte none | 4.9 | KU-812 (Basophil) PMA/ionomycin | 5.3 |
| 2ry Th1/Th2/Tr1_anti-CD95 CH11 | 11.9 | CCD1106 (Keratinocytes) none | 15.3 |
| LAK cells rest | 47.3 | CCD1106 (Keratinocytes) TNFalpha + IL-1beta | 53.2 |
| LAK cells IL-2 | 1.9 | Liver cirrhosis | 8.9 |
| LAK cells IL-2+IL-12 | 5.8 | Lupus kidney | 10.9 |
| LAK cells IL-2+IFN gamma | 6.0 | NCI-H292 none | 42.3 |
| LAK cells IL-2+ IL-18 | 5.5 | NCI-H292 IL-4 | 34.2 |
| LAK cells PMA/ionomycin | 15.2 | NCI-H292 IL-9 | 43.8 |
| NK Cells IL-2 rest | 1.1 | NCI-H292 IL-13 | 22.7 |
| Two Way MLR 3 day | 20.9 | NCI-H292 IFN gamma | 17.8 |
| Two Way MLR 5 day | 8.7 | HPAEC none | 46.7 |
| Two Way MLR 7 day | 1.1 | HPAEC TNF alpha + IL-1 beta | 59.9 |
| PBMC rest | 14.6 | Lung fibroblast none | 52.5 |
| PBMC PWM | 15.2 | Lung fibroblast TNF alpha + IL-1 beta | 17.2 |
| PBMC PHA-L | 7.0 | Lung fibroblast IL-4 | 71.7 |
| Ramos (B cell) none | 4.0 | Lung fibroblast IL-9 | 54.7 |
| Ramos (B cell) ionomycin | 7.6 | Lung fibroblast IL-13 | 81.8 |
| B lymphocytes PWM | 8.2 | Lung fibroblast IFN gamma | 73.7 |
| B lymphocytes CD40L and IL-4 | 8.5 | Dermal fibroblast CCD1070 rest | 69.3 |
| EOL-1 dbcAMP | 33.4 | Dermal fibroblast CCD1070 TNF alpha | 39.8 |
| EOL-1 dbcAMP PMA/ionomycin | 82.9 | Dermal fibroblast CCD1070 IL-1 beta | 36.6 |
| Dendritic cells none | 28.1 | Dermal fibroblast IFN gamma | 15.7 |
| Dendritic cells LPS | 12.8 | Dermal fibroblast IL-4 | 36.3 |
| Dendritic cells anti-CD40 | 35.1 | IBD Colitis 2 | 0.6 |
| Monocytes rest | 35.8 | IBD Crohn's | 2.6 |
| Monocytes LPS | 54.0 | Colon | 20.2 |
| Macrophages rest | **100.0** | Lung | 13.9 |
| Macrophages LPS | 23.3 | Thymus | 40.3 |
| HUVEC none | 37.6 | Kidney | 8.2 |
| IHUVEC starved | 70.2 | | |

**Table EH. Panel CNS_1**

| **Column A - Rel. Exp.(%) Ag1252, Run 171629874** | | | |
|---|---|---|---|
| **Tissue Name** | **A** | **Tissue Name** | **A** |
| BA4 Control | 463 | BA17 PSP | 12.2 |
| BA4 Contro12 | 77.9 | BA17 PSP2 | 1.4 |
| BA4 Alzheimer's2 | 4.7 | Sub Nigra Control | 28.9 |
| BA4 Parkinson's | 64.2 | Sub.Niga Control2 | 44.1 |
| BA4 Parkinson's2 | 80.1 | Sub Nigra Alzheimer's2 | 25.0 |
| BA4 Huntington's | 37.6 | Sub Nigra Parkinson's2 | 34.9 |
| BA4 Huntington's2 | 12.2 | Sub Nigra Huntington's | 67.4 |
| BA4 PSP | 22.5 | Sub Nigra Huntington's2 | 32.8 |
| BA4 PSP2 | 39.8 | Sub Nigra PSP2 | 9.7 |
| BA4 Depression | 28.9 | Sub Nigra Depression | 0.0 |
| BA4 Depression2 | 4.2 | Sub Nigra Depression2 | 1.1 |
| BA7 Control | 62.0 | Glob Palladus Control | 14.4 |
| BA7 Control2 | 95.3 | Glob Palladus Contiol2 | 31.2 |
| BA7 Alzheimer's2 | 5.1 | Glob Palladus Alzheimer's | 12.2 |
| BA7 Parkinson's | 23.0 | Glob Palladus Alzheimer's2 | 0.0 |
| BA7 Parkinson's2 | 55.1 | Glob Palladus Parkinson's | 71.7 |
| BA7 Huntington's | 56.3 | Glob Palladus Parkinson's2 | 19.8 |
| BA7 Huntington's2 | 44.1 | Glob Palladus PSP | 8.4 |
| BA7 PSP | 32.5 | Glob Palladus PSP2 | 12.8 |
| BA7 PSP2 | 44.4 | Glob Palladus Depression | 0.9 |
| BA7 Depression | 8.1 | Temp Pole Control | 14.7 |
| BA9 Control | 33.9 | Temp Pole Control2 | 46.3 |
| BA9 Control2 | 77.9 | Temp Pole Alzheimer's | 17.4 |
| BA9 Alzheimer's | 0.0 | Temp Pole Alzheimer's2 | 0.0 |
| BA9 Alzheimer's2 | 26.8 | Temp Pole Parkinson's | 26.4 |
| BA9 Parkinson's | 16.3 | Temp Pole Parkinson's2 | 36.3 |
| BA9 Parkinson's2 | 37.1 | Temp Pole Huntington's | 82.9 |
| BA9 Huntington's | **100.0** | Temp Pole PSP | 5.9 |
| BA9 Huntington's2 | 35.6 | Temp Pole PSP2 | 5.2 |
| BA9 PSP | 13.7 | Temp Pole Depression2 | 5.7 |
| BA9 PSP2 | 9.4 | Cing Gyr Control | 63.7 |
| BA9 Depression | 7.1 | Cing Gyr Control2 | 32.1 |
| BA9 Depression2 | 18.3 | Cing Gyr Alzheimer's | 22.1 |
| BA17 Control | 39.2 | Cing Gyr Alzheimer's2 | 1.2 |
| BA17 Control2 | 26.1 | Cing Gyr Parkinson's | 15.7 |
| BA17 Alzheimer's2 | 5.2 | Cing Gyr Parkinson's2 | 54.0 |
| BA17 Parkinson's | 65.1 | Cing Gyr Huntington's | 58.2 |
| BA17 Parkinson's2 | 51.8 | Cing GyrHuntington's2 | 25.0 |
| BA17 Huntington's | 43.2 | Cing Gyr PSP | 26.8 |
| BA17 Huntington's2 | 12.3 | Cing Gyr PSP2 | 17.0 |
| BA17 Depression | 16.7 | Cing Gyr Depression | 3.3 |
| BA17 Depression2 | 5.8 | Cing Gyr Depression2 | 1.4 |

**Al_comprehensive panel_v1.0 Summary:** Ag1252 Highest expression of this gene is seen in matched control psoriasis sample (CT=32.8)..Low expression of this gene is also seen in osteoarthitis/rheumatoid arthritis bone, cartilage, synovium and synovial fluid samples, from normal lung, emphysema, atopic asthma, asthma, Crohn's disease (normal matched control and diseased), and psoriasis (normal matched control and diseased). Therefore, therapeutic modulation of this gene product may ameliorate symptoms/conditions associated with autoimmune and inflammatory disorders including psoriasis, asthma, inflammatory bowel disease, rheumatoid arthritis and osteoarthritis.

**CNS_neurodegeneration_v1.0 Summary:** Data obtained from RTQPCR assays performed for this panel was analyized using multivariate analysis (ANOVA). The multivariate results obtained using Ag1252 in two experiments with the same probe-primer sets are in good agreement. This gene is found to be down-regulated in the temporal cortex of Alzheimer's disease patients. Therefore, up-regulation of this gene or its protein product, or treatment with specific agonists for this receptor may be of use in reversing the dementia/memory loss associated with this disease and neuronal death.

**General_screening_panel_v1.4 Summary:** Ag1252 This gene shows ubiquitous expression in this panel with highest expression in brain cerebellum (CT=26.8).This gene is expressed at high to moderate levels in all regions of the central nervous system examined, including amygdala, hippocampus, substantia nigra, thalamus, cerebellum, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

Moderate levels of expression of this gene is also seen in cluster of cancer cell lines derived from pancreatic, gastric, colon, lung, liver, renal, breast, ovarian, prostate, squamous cell carcinoma, melanoma and brain cancers. Thus, expression of this gene could be used as a marker to detect the presence of these cancers. Furthermore, therapeutic modulation of the expression or function of this gene may be effective in the treatment of pancreatic; gastric, colon, lung, liver, renal, breast, ovarian, prostate, squamous cell carcinoma, melanoma and brain cancers.

Among tissues with metabolic or endocrine function, this gene is expressed at moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

**Panel 1.2 Summary:** Ag1252 The expression of this gene shows a significant level of expression across the majority of samples in the panel and correlates with expression seen in panel 1.4. Of interest is the observation that lung cancer cell lines appear to show considerably higher expression compared to other samples, suggesting that this gene may play a role in lung cancer. Please see panel 1.4 for further discussion in the role of this gene.

**Panel 2D Summary:** Ag1213 This gene shows widespread expression in this panel. Highest expression of this gene is seen in a breast cancer (CT=29.5). There is a slight up-regulation of this gene in lung, kidney, breast and gastric cancer when compared to corresponding normal adjacent controls. Thus, therapies targeted towards this gene or its protein product may be beneficial to the treatment of lung, kidney, breast and gastric cancers.

**Panel 4D Summary:** Ag1213 Highest expression of this gene is seen in resting macrophage (CT=30.6). This gene shows wide spread expression with higher expression in resting LAK cells, eosinophils, dendritic cells, monocytes, macrophages, endothelial cells, bronchial and small airway epithelial cells, keratinocytes, mucoepidermoid NCI-H292 cells, lung and dermal fibroblasts and normal tissues represented by colon, lung, and thymus. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel CNS_1 Summary:** Ag1213 This gene encodes for a homolog of serotinin receptor 7. Serotonin receptors have been implicated in neuropsychiatric disorders including schizophrenia, bipolar disorder, depression, and Alzheimer's disease. This gene is downregulated in the cingulate gyrus, parietal cortex, and substantia nigra in depression as measured via RTQ-PCR analysis in postmortem brain tissue. Because many antidepressants are serotonin reuptake inhibitors (e.g., fluvoxamine, hypericum perforatum, clomipramine, milnacipran, etc) the downregulation of a serotonin receptor in the brains of patients suffering from chronic depression suggests that this molecule may be a primary role in the etiology of this disease and be an excellent small molecule target for the treatment of psychiatric disease.

### F. CG55912-01: CACNG4 - ION CHANNEL

Expression of gene CG55912-01 was assessed using the primer-probe set Ag2841, described in Table FA. Results of the RTQ-PCR runs are shown in Tables FB, FC, FD, FE, FF and FG.

**Table FA. Probe Name Ag2841**

| **Primers** | **Sequences** | **Length** | **Start Position** | **SEQ ID No** |
|---|---|---|---|---|
| Forward | 5'-gtctgcgtgaagatcaatcatt-3' | 22 | 283 | 113 |
| Probe | TET-5'-aggacacggactacgaccacgacag-3'-TAMRA | 25 | 311 | 114 |
| Reverse | 5'-cggaccgtacggagtagatact-3' | 22 | 341 | 115 |

**Table FB. CNS_neurodegeneration v1.0**

| **Column A - Rel. Exp.(%)Ag2841, Run 209779166** | | | |
|---|---|---|---|
| **Tissue Name** | **A** | **Tissue Name** | **A** |
| AD 1 Hippo | 15.8 | Control (Path) 3 Temporal Ctx | 0.0 |
| AD 2 Hippo | 34.6 | Control (Path) 4 Temporal Ctx | 25.9 |
| AD 3 Hippo | 15.9 | AD 1 Occipital Ctx | 6.8 |
| AD 4 Hippo | 17.0 | AD 2 Occipital Ctx (Missing) | 0.0 |
| AD 5 hippo | 85.3 | AD 3 Occipital Ctx | 24.0 |
| AD 6 Hippo | 51.1 | AD 4 Occipital Ctx | 21.8 |
| Control 2 Hippo | 21.0 | AD 5 Occipital Ctx | 0.0 |
| Control 4 Hippo | 0.0 | AD 6 Occipital Ctx | 51.8 |
| Control (Path) 3 Hippo | 0.0 | Control 1 Occipital Ctx | 6.0 |
| AD 1 Temporal Ctx | 6.3 | Control 2 Occipital Ctx | **100.0** |
| AD 2 Temporal Ctx | 16.3 | Control 3 Occipital Ctx | 39.2 |
| AD 3 Temporal Ctx | 5.0 | Control 4 Occipital Ctx | 5.1 |
| AD 4 Temporal Ctx | 7.9 | Control (Path) 1 Occipital Ctx | 46.7 |
| AD 5 Inf Temporal Ctx | 64.6 | Control (Path) 2 Occipital Ctx | 23.0 |
| AD 5 SupTemporal Ctx | 38.4 | Control (Path) 3 Occipital Ctx | 6.3 |
| AD 6 Inf Temporal Ctx | 46.3 | Control (Path) 4 Occipital Ctx | 25.3 |
| AD 6 Sup Temporal Ctx | 88.3 | Control 1 Parietal Ctx | 0.0 |
| Control 1 Temporal Ctx | 10.4 | Control 2 Parietal Ctx | 51.4 |
| Control 2 Temporal Ctx | 42.9 | Control 3 Parietal Ctx | 18.9 |
| Control 3 Temporal Ctx | 20.3 | Control (Path) Parietal Ctx | 86.5 |
| Control 4 Temporal Ctx | 8.4 | Control (Path) 2 Parietal Ctx | 31.0 |
| Control (Path) 1 Temporal Ctx | 58.2 | Control (Path) 3 Parietal Ctx | 7.5 |
| Control (Path) 2 Temporal Ctx | 49.7 | Control (Path) 4 Parietal Ctx | 63.7 |

**Table FC. Panel 1.3D**

| **Column A - Rel. Exp.(%) Ag2841, Run 161922470** | | | | | |
|---|---|---|---|---|---|
| **Column B - Rel. Exp.(%) Ag2841, Run 165721032** | | | | | |
| **Tissue Name** | **A** | **B** | **Tissue Name** | **A** | **B** |
| Liver adenocarcinoma | 3.1 | 7.2 | Kidney (fetal) | 0.0 | 0.0 |
| Pancreas | 0.0 | 0.0 | Renal ca. 786-0 | 0.0 | 0.0 |
| Pancreatic ca. CAPAN 2 | 5.7 | 5.9 | Renal ca. A498 | 0.0 | 0.0 |
| Adrenal gland | 0.0 | 0.0 | Renal ca. RXF 393 | 0.0 | 0.0 |
| Thyroid . | 0.0 | 0.0 | Renal ca. ACHN | 0.0 | 0.0 |
| Salivary gland | 0.0 | 0.0 | Renal ca. UO-31 | 0.0 | 0.0 |
| Pituitary gland | 0.0 | 0.0 | Renal ca. TK-10 | 0.0 | 0.0 |
| Brain (fetal) | 18.3 | 27.0 | Liver | 0.0 | 0.0 |
| Brain (whole) | 27.0 | 19.5 | Liver (fetal) | 0.0 | 0.0 |
| Brain (amygdala) | 77.4 | 54.3 | Liver ca. (hepatoblast) HepG2 | 0.0 | 0.0 |
| Brain (cerebellum) | 0.0 | 0.0 | Lung | 3.0 | 9.2 |
| Brain (hippocampus) | **100.0** | **100.0** | Lung (fetal) | 0.0 | 0.0 |
| Brain (substantia nigra) | 0.0 | 3.5 | Lung ca. (small cell) LX-1 | 0.0 | 0.0 |
| Brain (thalamus) | 7.9 | 0.0 | Lung ca. (small cell) NCI-H69 | 0.0 | 0.0 |
| Cerebral Cortex | 90.8 | 8.4 | Lung ca. (s.cell var.) SHP-77 | 0.0 | 3.9 |
| Spinal cord | 3.9 | 0.0 | Lung ca. (large cell)NCI-H460 | 0.0 | 0.0 |
| glio/astro U87-MG | 2.7 | 6.5 | Lung ca. (non-sm. cell) A549 | 15.0 | 0.0 |
| glio/astro U-118-MG | 0.0 | 0.0 | Lung ca. (non-s.cell) NCI-H23 | 7.2 | 12.5 |
| astrocytoma SW 1783 | 0.0 | 6.6 | Lung ca. (non-s.cell) HOP-62 | 0.0 | 0.0 |
| neuro*; met SK-N-AS | 8.4 | 0.0 | Lung ca. (non-s.cl) NCI-H522 | 12.9 | 3.2 |
| astrocytoma SF-539 | 0.0 | 0.0 | Lung ca. (squam.) SW 900 | 0.0 | 0.0 |
| astrocytoma SNB-75 | 0.0 | 0.0 | Lung ca. (squam.) NCI-H596 | 0.0 | 0.0 |
| glioma SNB-19 | 27.2 | 6.2 | Mammary gland | 0.0 | 0.0 |
| glioma U251 | 0.0 | 0.0 | Breast ca.* (pl.ef) MCF-7 | 0.0 | 0.0 |
| glioma SF-295 | 0.0 | 3.4 | Breast ca.* (pl.ef) MDA-MB-231 | 0.0 | 0.0 |
| Heart (fetal) | 0.0 | 0.0 | Breast ca.* (pl.ef) T47D | 0.0 | 0.0 |
| Heart | 0.0 | 0.0 | Breast ca. BT-549 | 0.0 | 0.0 |
| Skeletal muscle (fetal) | 0.0 | 0.0 | Breast ca. MDA-N | 0.0 | 0.0 |
| Skeletal muscle | 0.0 | 0.0 | Ovary | 0.0 | 0.0 |
| Bone marrow | 3.8 | 0.0 | Ovarian ca. OVCAR-3 | 0.0 | 0.0 |
| Thymus | 0.0 | 0.0 | Ovarian ca. OVCAR-4 | 0.0 | 22.1 |
| Spleen | 0.0 | 0.0 | Ovarian ca. OVCAR-5 | 7.0 | 0.0 |
| Lymph node | 0.0 | 0.0 | Ovarian ca. OVCAR-8 | 0.0 | 0.0 |
| Colorectal | 0.0 | 0.0 | Ovarian ca. IGROV-1 | 0.0 | 0.0 |
| Stomach | 0.0 | 0.0 | Ovarian ca.* (ascites) SK-OV-3 | 0.0 | 0.0 |
| Small intestine | 0.0 | 0.0 | Uterus | 0.0 | 2.6 |
| Colon ca. SW480 | 0.0 | 2.6 | Placenta | 0.0 | 0.0 |
| Colon ca.* SW620(SW480 met) | 0.0 | 0.0 | Prostate | 0.0 | 0.0 |
| Colon ca. THT29 | 0.0 | 0.0 | Prostate ca.* (bone met)PC-3 | 0.0 | 2.0 |
| Colon ca. HCT-116 | 0.0 | 0.0 | Testis | 21.0 | 0.0 |
| Colon ca. CaCo-2 | 0.0 | 0.0 | Melanoma Hs688(A).T | 0.0 | 0.0 |
| Colon ca. tissue(ODO3866) | 0.0 | 0.0 | Melanoma* (met) Hs688(B).T | 0.0 | 0.0 |
| Colon ca. HCC-2998 | 8.4 | 0.0 | Melanoma UACC-62 | 0.0 | 0.0 |
| Gastric ca.* (liver met) NCI-N87 | 18.0 | 4.2 | Melanoma M14 | 0.0 | 0.0 |
| Bladder | 0.0 | 0.0 | Melanoma LOX IMVI | 0.0 | 11.0 |
| Trachea | 0.0 | 0.0 | Melanoma* (met) SK-MEL-5 | 0.0 | 0.0 |
| Kidney | 0.0 | 0.0 | Adipose | 0.0 | 0.0 |

**Table FD. Panel 2D**

| **Column A - Rel. Exp.(%) Ag2841, Run 161930423** | | | |
|---|---|---|---|
| **Tissue Name** | **A** | **Tissue Name** | **A** |
| Normal Colon | 0.0 | Kidney Margin 8120608 | 0.0 |
| CC Well to Mod Diff (ODO3866) | 0.0 | Kidney Cancer 8120613 | 0.0 |
| CC Margin (OD03866) | 20.2 | Kidney Margin 8I20614 | 0.0 |
| CC Gr.2 rectosigmoid (ODO3868) | 0.0 | Kidney Cancer 9010320 | 0.0 |
| CC Margin (OD03868) | 0.0 | Kidney Margin 9010321 | 0.0 |
| CC Mod Diff (OD03920) | **100.0** | Normal Uterus | 0.0 |
| CC Margin (OD03920) | 0.0 | Uterus Cancer 06401 | 0.0 |
| CC Gr.2 ascend colon (ODO3921) | 41.8 | Normal Thyroid | 0.0 |
| CC Margin (ODO3921) | 0.0 | Thyroid Cancer 064010 | 19.1 |
| CC from Partial Hepatectomy (ODO4309) Mets | 0.0 | Thyroid Cancer A302152 | 0.0 |
| Liver Margin (ODO4309) | 0.0 | Thyroid Margin A302153 | 0.0 |
| Colon mets to lung (OD04451-01) | 0.0 | Normal Breast | 10.5 |
| Lung Margin (OD04451-02) | 0.0 | Breast Cancer (OD04566) | 0.0 |
| Normal Prostate 6546-1 | 0.0 | Breast Cancer (OD04590-01) | 0.0 |
| Prostate Cancer (OD04410) | 0.0 | Breast Cancer Mets (OD04590-03) | 0.0 |
| Prostate Margin (OD04410) | 0.0 | Breast Cancer Metastasis (OD04655-05) | 0.0 |
| Prostate Cancer (OD04720-01) | 0.0 | Breast Cancer 064006 | 0.0 |
| Prostate Margin (OD04_720-02) | 0.0 | Breast Cancer 1024 | 45.4 |
| Normal Lung 06101 | 55.9 | Breast Cancer 9100266 | 0.0 |
| Lung Met to Muscle (ODO4286) | 0.0 | Breast Margin 9100265 | 0.0 |
| Muscle Margin (ODO4286) | 0.0 | Breast Cancer A209073 | 0.0 |
| Lung Malignant Cancer (OD03126) | 0.0 | Breast Margin A209073 | 49.3 |
| Lung Margin (OD03126) | 0.0 | Normal Liver | 0.0 |
| Lung Cancer (OD04404) | 0.0 | Liver Cancer 064003 | 23.3 |
| Lung Margin (OD04404) | 0.0 | Liver Cancer 1025 | 0.0 |
| Lung Cancer (OD04565) | 0.0 | Liver Cancer 1026 | 0.0 |
| Lung Margin (OD04565) | 0.0 | Liver Cancer 6004-T | 0.0 |
| Lung Cancer (OD04237-01) | 0.0 | Liver Tissue 6004-N | 0.0 |
| Lung Margin (OD04237-02) | 0.0 | Liver Cancer 6005-T | 0.0 |
| Ocular Mel Met to Liver (ODO4310) | 0.0 | Liver Tissue 6005-N | 0.0 |
| Liver Margin (OD04310) | 0.0 | Normal Bladder | 21.6 |
| Melanoma Mets to Lung (OD04321) | 0.0 | Bladder Cancer 1023 | 0.0 |
| Lung Margin (OD04321) | 0.0 | Bladder Cancer A302173 | 14.3 |
| Normal Kidney | 0.0 | Bladder Cancer (OD04718-01) | 0.0 |
| Kidney Ca, Nuclear gade 2 (OD04338) | 0.0 | BladderNormal Adjacent (OD04718-03) | 0.0 |
| Kidney Margin (OD04338) | 0.0 | Normal Ovary | 0.0 |
| Kidney Ca Nuclear grade 1/2 (OD04339) | 0.0 | Ovarian Cancer 064008 | 16.3 |
| Kidney Margin (OD04339) | 0.0 | Ovarian Cancer (OD04768-07) | 57.8 |
| Kidney Ca, Clear cell type (OD04340) | 0.0 | Ovary Margin (OD04768-08) | 0.0 |
| Kidney Margin (OD04340) | 0.0 | Normal Stomach | 0.0 |
| KidneyCa, Nuclear grade 3 (OD04348) | 0.0 | Gastric Cancer 9060358 | 0.0 |
| Kidney Margin (OD04348) | 0.0 | Stomach Margin 9060359 | 0.0 |
| Kidney Cancer (OD04622-01) | 0.0 | Gastric Cancer 9060395 | 0.0 |
| Kidney Margin (OD04622-03) | 0.0 | Stomach Margin 9060394 | 0.0 |
| Kidney Cancer (OD04450-01) | 0.0 | Gastric Cancer 9060397 | 0.0 |
| Kidney Margin (OD04450-03) | 0.0 | Stomach Margin 9060396 | 0.0 |
| Kidney Cancer 8120607 | 0.0 | Gastric Cancer 064005 | 0.0 |

**Table FE. Panel 3D**

| **Column A - Rel. Exp.(%) Ag2841, Run 164538045** | | | |
|---|---|---|---|
| **Tissue Name** | **A** | **Tissue Name** | **A** |
| Daoy- Medulloblastoma | 11.6 | Ca Ski- Cervical epidermoid carcinoma (metastasis) | 0.0 |
| TE671- Medulloblastoma | 7.0 | ES-2- Ovarian clear cell carcinoma | 3.6 |
| D283 Med- Medulloblastoma | 0.0 | Ramos- Stimulated with PMA/ionomycin 6h | 0.0 |
| PFSK-1- Primitive Neuroectodermal | 0.0 | Ramos- Stimulated with PMA/ionomycin 14h | 0.0 |
| XF-498- CNS | 0.0 | MEG-01-Chronic myelogenous leukemia (megokaryoblast) | 3.6 |
| SNB-78- Glioma | 0.0 | Raji- Burkitt's lymphoma | 0.0 |
| SF-268- Glioblastoma | 12.8 | Daudi- Burkitt's lymphoma | 0.0 |
| T98G-Glioblastoma | 0.0 | U266- B-cell plasmacytoma | 0.0 |
| SK-N-SH- Neuroblastoma (metastasis) | 39.0 | CA46- Burkitt's lymphoma | 0.0 |
| SF-295- Glioblastoma | 0.0 | RL- non-Hodgkin's B-cell lymphoma | 0.0 |
| Cerebellum | 0.0 | JM1- pre-B-cell lymphoma | 0.0 |
| Cerebellum | 25.7 | Jurkat- T cell leukemia | 0.0 |
| NCI-H292- Mucoepidermoid lung carcinoma | 0.0 | TF-1- Erythroleukemia | 0.0 |
| DMS-114- Small cell lung cancer | 10.7 | HUT 78- T-cell lymphoma | 0.0 |
| DMS-79- Small cell lung cancer | **100.0** | U937- Histiocytic lymphoma | 0.0 |
| NCI-H146- Small cell lung cancer | 0.0 | KU-812- Myelogenous leukemia | 0.0 |
| NCI-H526- Small cell lung cancer | 16.2 | 769-P- Clear cell renal carcinoma | 0.0 |
| NCI-N417- Small cell lung cancer | 0.0 | Caki-2- Clear cell renal carcinoma | 3.8 |
| NCI-H82- Small cell lung cancer | 0.0 | SW 839- Clear cell renal carcinoma | 0.0 |
| NCI-H157-Squamous cell lung cancer (metastasis) | 7.5 | Rhabdoid kidney tumor | 36.6 |
| NCI-H1155- Large cell lung cancer | 16.8 | Hs766T-Pancreatic carcinoma (LN metastasis) | 14.3 |
| NCI-H1299- Large cell lung cancer | 39.8 | CAPAN-1- Pancreatic adenocarcinoma (liver metastasis) | 6.7 |
| NCl-H727- Lung carcinoid | 10.3 | SU86.86- Pancreatic carcinoma (liver metastasis) | 0.0 |
| NCI-UMC-11- Lung carcinoid | 0.0 | BxPC-3-Pancreatic adenocarcinoma | 0.0 |
| LX-1- Small cell lung cancer | 0.0 | HPAC- Pancreatic adenocarcinoma | 0.0 |
| Colo-205- Colon cancer | 0.0 | MIA PaCa-2- Pancreatic carcinoma | 25.7 |
| KM 12- Colon cancer | 25.7 | CFPAC-1- Pancreatic ductal adenocarcinoma | 35.4 |
| KM20L2- Colon cancer | 21.6 | PANC-1- Pancreatic epithelioid ductal carcinoma | 40.9 |
| NCI-H716- Colon cancer | 0.0 | T24- Bladder carcinma (transitional cell) | 0.0 |
| SW-48- Colon adenocarcinoma | 0.0 | 5.637- Bladder carcinoma | 0.0 |
| SW1116- Colon adenocarcinoma | 0.0 | HT-1197-Bladder carcinoma | 0.0 |
| LS 174T- Colon adenocarcinoma | 0.0 | UM-UC-3- Bladder carcinma (transitional cell) | 12.2 |
| SW-948- Colon adenocarcinoma | 0.0 | A204- Rhabdomyosarcoma | 0.0 |
| SW-480- Colon adenocarcinoma | 0.0 | HT-1080- Fibrosarcoma | 4.6 |
| NCI-SNU-5- Gastric carcinoma | 21.6 | MG-63- Osteosarcoma | 0.0 |
| KATO III- Gastric carcinoma | 4.2 | SK-LMS-1-Leiomyosarcoma (vulva) | 31.4 |
| NCI-SNU-16- Gastric carcinoma | 0.0 | SJRH30- Rhabdomyosarcoma (met to bone marrow) | 0.0 |
| NCI-SNU-1- Gastric carcinoma | 0.0 | A431- Epidermoid carcinoma | 0.0 |
| RF-1- Gastric adenocarcinoma | 0.0 | WM266-4- Melanoma | 0.0 |
| RF-48- Gastric adenocarcinoma | 0.0 | DU 145- Prostate carcinoma (brain metastasis) | 6.7 |
| MKN-45- Gastric carcinoma | 39.5 | MDA-MB-468- Breast adenocarcinoma | 0.0 |
| NCI-N87- Gastric carcinoma | 0.0 | SCC-4- Squamous cell carcinoma of tongue | 0.0 |
| OVCAR-5- Ovarian carcinoma | 0.0 | SCC-9- Squamous cell carcinoma of tongue | 0.0 |
| RL95-2- Uterine carcinoma | 11.4 | SCC-15- Squamous cell carcinoma of tongue | 0.0 |
| HelaS3- Cervical adenocarcinoma | 0.0 | CAL 27- Squamous cell carcinoma of tongue | 0.0 |

**Table FF. Panel 4D**

| **Column A - Rel. Exp.(%) Ag2841, Run 159616564** | | | |
|---|---|---|---|
| **Tissue Name** | **A** | **Tissue Name** | **A** |
| Secondary Th1 act | 0.0 | HUVEC IL-1beta | 2.7 |
| Secondary Th2 act | 0.0 | HUVEC IFN gamma | 8.8 |
| Secondary Tr1 act | 0.0 | HUVEC TNF alpha + IFN gamma | 15.9 |
| Secondary Th1 rest | 0.0 | HUVEC TNF alpha + IL4 | 2.2 |
| Secondary Th2 rest | 0.0 | HUVEC IL-11 | 13.6 |
| Secondary Tr1 rest | 0.0 | Lung Microvascular EC none | 21.5 |
| Primary Th1 act | 0.0 | Lung Microvascular EC TNFalpha + IL-1beta | 33.7 |
| Primary Th2 act | 0:0 | Microvascular Dermal EC none | 12.1 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 2.4 |
| Primary Tr1 rest | 0.0 | Small airway epithelium TNFalpha + IL-1beta | 0.0 |
| CD45RA CD4 lymphocyte act | 0.0 | Coronery artery SMC rest | 10.8 |
| CD45RO CD4 lymphocyte act | 0.0 | Coronery artery SMC TNFalpha + IL-1beta | 8.3 |
| CD8 lymphocyte act | 0.0 | Astrocytes rest | 0.0 |
| Secondary CD8 lymphocyte rest | 0.0 | Astrocytes TNFalpha + IL-1beta | 9.8 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Tb1/Th2/Tr1_anti-CD95 CH11 | 0.0 | CCD 1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 15.1 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+ IL-18 8 | 0.0 | NCI-H292 IL-4 | 0.0 |
| LAK cells PMA/iomomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 19.6 |
| Two Way MLR 7 day | 0.0 | HPAEC TNF alpha + IL-1 beta | 7.0 |
| PBMC rest | 5.9 | Lung fibroblast none | 0.0 |
| PBMC PWM | 0.0 | Lung fibroblast TNF alpha+ IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B ) ionomycin | 0.0 | cellLung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 3.6 |
| EOL-1 dbcAMP | 0.0 | Dermal fibroblast CCD1070 TNF alpha | 0.0 |
| EOL-1 dbcAMP PMA/iomomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | **100.0** |
| Macrophages rest | 0.0 | Lung | 40.1 |
| Macrophages LPS | 0.0 | Thymus | 0.0 |
| HLTVEC none | 5.8 | Kidney | 0.0 |
| HUVEC starved | 6.9 | | |

**Table FG. Panel CNS 1**

| **Column A - Rel. Exp.(%)Ag2841, Run 171669732** | | | |
|---|---|---|---|
| **Tissue Name** | **A** | **Tissue Name** | **A** |
| BA4 Control | 0.0 | BA17 PSP | 9.2 |
| BA4 Control2 | 9.2 | BA17PSP2 | 0.0 |
| BA4 Alzheimer's2 | 0.0 | Sub Nigra Control | 8.7 |
| BA4 Parkinson's | 47.6 | Sub Nigra Control2 | 35.1 |
| BA4 Parkinson's2 | 50.3 | Sub Nigra Alzheimer's2 | 0.0 |
| BA4 Huntington's | 13.2 | Sub Nigra Parkinson's2 | 0.0 |
| BA4 Huntington's2 | 11.7 | Sub Nigra Huntington's | 10.0 |
| BA4 PSP | 38.2 | Sub Nigra Huntington's2 | 25.9 |
| BA4 PSP2 | 14.4 | Sub Nigra PSP2 | 0.0 |
| BA4 Depression | 0.0 | Sub Nigra Depression | 0.0 |
| BA4 Depression2 | 0.0 | Sub Nigra Depression2 | 0.0 |
| BA7 Control | 10.1 | Glob Palladus Control | 0.0 |
| BA7 Control2 | 0.0 | Glob Palladus Control2 | 0.0 |
| BA7 Alzheimer's2 | 0.0 | Glob Palladus Alzheimer's | 0.0 |
| BA7 Parkinson's | 51.4 | Glob Palladus Alzheimer's2 | 0.0 |
| BA7 Parkinson's2 | 17.4 | Glob Palladus Parkinson's | 18.2 |
| BA7 Huntington's | 46.3 | Glob Palladus Parkinson's2 | 0.0 |
| BA7 Huntington's2 | 36.3 | Glob Palladus PSP | 0.0 |
| BA7 PSP | 46.3 | Glob Palladus PSP2 | 0.0 |
| BA7 PSP2 | 37.9 | Glob Palladus Depression | 0.0 |
| BA7 Depression | 29.9 | Temp Pole Control | 20.3 |
| BA9 Control | 0.0 | Temp Pole Control2 | 28.5 |
| BA9 Control2 | 38.2 | Temp Pole Alzheimer's | 0.0 |
| BA9 Alzheimer's | 8.2 | Temp Pole Alzheimer's2 | 0.0 |
| BA9 Alzheimer's2 | 41.2 | Temp Pole Parkinson's | 17.2 |
| BA9 Parkinson's | **100.0** | Temp Pole Parkinson's2 | 0.0 |
| BA9 Parkinson's2 | 54.3 | Temp Pole Huntington's | 51.4 |
| BA9 Huntington's | 9.3 | Temp Pole PSP | 0.0 |
| BA9 Huntington's2 | 57.8 | Temp Pole PSP2 | 0.0 |
| BA9 PSP | 0.0 | Temp Pole Depression2 | 21.5 |
| BA9 PSP2 | 0.0 | Cing Gyr Control | 51.4 |
| BA9 Depression | 0.0 | Cing Gyr Control2 | 0.0 |
| BA9 Depression2 | 14.1 | Cing Gyr Alzheimer's | 0.0 |
| BA17 Control | 51.4 | Cing Gyr Alzheimer's2 | 0.0 |
| BA17 Control2 | 25.9 | Cing Gyr Parkinson's | 38.7 |
| BA17 Alzheimer's2 | 0.0 | Cing Gyr Parkinson's2 | 13.3 |
| BA17 Parkinson's | 16.3 | Cing Gyr Huntington's | 27.2 |
| BA17 Parkinson's2 | 47.3 | Cing Gyr Huntington's2 | 30.4 |
| BA17 Huntington's | 89.5 | Cing Gyr PSP | 0.0 |
| BA17 Huntington's2 | 13.0 | Cing Gyr PSP2 | 0.0 |
| BA17 Depression | 0.0 | Cing Gyr Depression | 0.0 |
| BA17 Depression2 | 66.4 | Cing Gyr Depression2 | 0.0 |

**CNS_neurodegeneration_v1.0 Summary:** Data obtained from RTQPCR assays performed for this panel was analyized using multivariate analysis (ANOVA). The multivariate results obtained using Ag2841 indicates expression of this gene is low (CTs > 35) across all of the samples on this panel. Although levels are low for this gene, there is a significant difference in expression levels between non-demented controls and patients suffering from Alzheimer's disease, such that the levels of mRNA appear to be downregulated 2-fold in the postmortem AD brain (p = 0.0018 when analyzed by ANCOVA; estimate of RNA loaded per well used as a covariate). This gene may therefore represent a drug target for the treatment of Alzheimer's disease or other dementias.

**Panel 1.3D Summary:** Ag2841 Two experiments with same primer and probe set are in excellent agreement, with highest expression of this gene in brain hippocampus region (CT=34). Expression of this gene is exclusive to the brain region. In addition, this gene may play a role in central nervous system disorders such as Alzheimer's disease, Parkinson's disease, epilepsy, multiple sclerosis, schizophrenia and depression.

This gene codes for a homolog of neuronal voltage-gated calcium channel. In Caenorhabditis elegans voltage-gated calcium channels have been shown to direct neuronal migration. In C. elegans mutants carrying loss-of-function alleles of the calcium channel gene unc-2, the touch receptor neuron AVM and the interneuron SDQR often migrated inappropriately, leading to misplacement of their cell bodies (Tam T, Mathews E, Snutch TP, Schafer WR. (2000) Voltage-gated calcium channels direct neuronal migration in Caenorhabditis elegans. Dev Biol 226(1):104-17, PMID: 10993677). Therefore, in analogy with C. elegan unc-2, neuronal voltage-gated calcium channel encoded by this gene may also play a role in directing neuronal migration. In addition, calcium channels have been implicated in number of neurological diseases such as familial hemiplegic migraine, episodic ataxia type 2, spinocerebellar ataxia 6, and Lambert-Eaton myasthenic syndrome and other diseases (Greenberg DA. (1997) Calcium channels in neurological disease. Ann Neurol 42(3):275-82, PMID: 9307247). Therefore, therapeutic modulation of the activity of this gene may prove useful in the treatment of the different neurological diseases.

**Panel 4D Summary:** Ag2841 Low but significant expression of this gene is detected exclusively in colon (CT=34). Therefore, expression of this gene may be used to distinguish colon from the other tissues on this panel. Furthermore, expression of this gene is decreased in colon samples from patients with IBD colitis and Crohn's disease relative to normal colon. Therefore, therapeutic modulation of the activity of the calcium channel encoded by this gene may be useful in the treatment of inflammatory bowel disease.

### G. CG56001-01: 3-HYDROXYBUTYRATE DEHYDROGENASE

Expression of gene CG56001-01 was assessed using the primer-probe set Ag2868, described in Table GA. Results of the RTQ-PCR runs are shown in Tables GB, GC, GD, GE and GF.

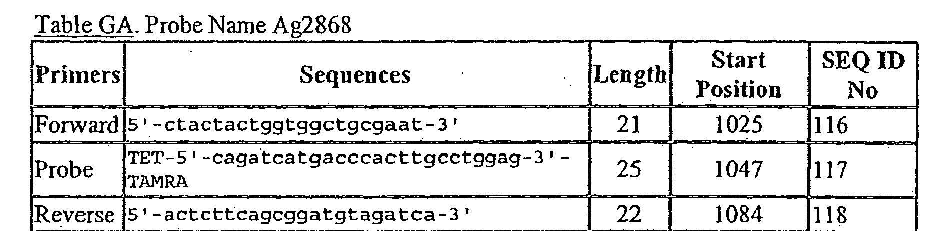

**Table GB. CNS_neurodegeneration_v1.0**

| **Column A - Rel. Exp.(%) Ag2868, Run 206485413** | | | | | |
|---|---|---|---|---|---|
| **Column B - Rel. Exp.(%) Ag2868, Run 224079571** | | | | | |
| **Tissue Name** | **A** | **B** | **Tissue Name** | **A** | **B** |
| AD 1 Hippo | 10.7 | 11.1 | Control (Path) 3 Temporal Ctx | 8.2 | 9.7 |
| AD 2 Hippo | 32.1 | 39.2 | Control (Path) 4 Temporal Ctx | 50.7 | 56.3 |
| AD 3 Hippo | 8.7 | 4.0 | AD 1 Occipital Ctx | 16.0 | 20.6 |
| AD 4 Hippo | 12.3 | 9.6 | AD 2 Occipital Ctx (Missing) | 0.0 | 0.0 |
| AD 5 Hippo | 99.3 | 74.2 | AD 3 Occipital Ctx | 6.3 | 8.4 |
| AD 6 Hippo | 34.4 | 35.8 | AD 4 Occipital Ctx | 24.8 | 22.7 |
| Control 2 Hippo | 27.9 | 29.3 | AD 5 Occipital Ctx | 48.3 | 19.3 |
| Control 4 Hippo | 15.4 | 13.1 | AD 6 Occipital Ctx | 18.2 | 43.5 |
| Control (Path) 3 Hippo | 8.7 | 12.9 | Control 1 Occipital Ctx | 6.6 | 5.5 |
| AD 1 Temporal Ctx | 12.4 | 9.7 | Control 2 Occipital Ctx | 67.4 | 60.7 |
| AD 2 Temporal Ctx | 34.9 | 40.6 | Control 3 Occipital Ctx | 26.2 | 28.9 |
| AD 3 Temporal Ctx | 6.3 | 5.0 | Control 4 Occipital Ctx | 7.6 | 5.5 |
| AD 4 Temporal Ctx | 13.4 | 28.9 | Control (Path) 1 Occipital Ctx | 92.0 | 68.8 |
| AD 5 Inf Temporal Ctx | **100.0** | **100.0** | Control (Path) 2 Occipital Ctx | 18.9 | 15.6 |
| AD 5 Sup Temporal Ctx | 57.0 | 46.3 | Control (Path) 3 Occipital Ctx | 3.8 | 2.7 |
| AD 6 Inf Temporal Ctx | 32.3 | 33.4 | Control (Path) 4 Occipital Ctx | 35.1 | 30.6 |
| AD 6 Sup Temporal Ctx | 45.4 | 39.0 | Control 1 Parietal Ctx | 15.5 | 10.1 |
| Control 1 Temporal Ctx | 10.0 | 12.6 | Control 2 Parietal Ctx | 47.0 | 36.3 |
| Control 2 Temporal Ctx | 53.6 | 43.2 | Control 3 Parietal Ctx | 17.3 | 29.5 |
| Control 3 Temporal Ctx | 28.1 | 20.7 | Control (Path) 1 Parietal Ctx | 94.6 | 77.9 |
| Control 3 Temporal Ctx | 16.8 | 15.6 | Control (Path) 2 Parietal Ctx | 35.4 | 41.2 |
| Control (Path) 1 Temporal Ctx | 73.2 | 63.3 | Control (Path) 3 Parietal Ctx | 4.3 | 6.2 |
| Control (Path) 2 Temporal Ctx | 57.8 | 43.2 | Control (Path) 4 Parietal Ctx | 68.8 | 59.5 |

**Table GC. Panel 1.3D**

| **Column A - Rel. Exp.(%) Ag2868, Run 162011291** | | | |
|---|---|---|---|
| **Tissue Name** | **A** | **Tissue Name** | **A** |
| Liver adenocarcinoma | 0.5 | Kidney (fetal) | 9.1 |
| Pancreas | 1.1 | Renal ca. 786-0 | 0.0 |
| Pancreatic ca. CAPAN 2 | 1.6 | Renal ca. A498 | 1.1 |
| Adrenal gland | 1.0 | Renal ca. RXF 393 | 2.8 |
| Thyroid | 9.9 | Renal ca. ACHN | 1.5 |
| Salivary gland | 7.0 | Renal ca. UO-31 | 2.7 |
| Pituitary gland | 3.0 | Renal ca. TK-10 | 3.4 |
| Brain (fetal) | 3.3 | Liver | 36.9 |
| Brain (whole) | 23.5 | Liver (fetal) | 24.7 |
| Brain (amygdala) | 13.7 | Liver ca. (hepatoblast) HepG2 | 5.0 |
| Brain (cerebellum) | 28.3 | Lung | 1.4 |
| Brain (hippocampus) | 31.6 | Lung (fetal) | 3.4 |
| Brain (substantia nigra) | 8.5 | Lung ca. (small cell) LX-1 | 2.8 |
| Brain (thalamus) | 16.8 | Lung ca. (small cell) NCI-H69 | 7.1 |
| Cerebral Cortex | **100.0.** | Lung ca. (s.cell var.) SHP-77 | 3.1 |
| Spinal cord | 10.2 | Lung ca. (large cell)NCI-H460 | 0.3 |
| glio/astro U87-MG | 1.3 | Lung ca. (non-sm. cell) A549 | 0.7 |
| glio/astro U-118-MG | 0.4 | Lung ca. (non-s.cell) NCI-H23 | 0.6 |
| astrocytoma SW1783 | 4.7 | Lung ca. (non-s.cell) HOP-62 | 2.8 |
| neuro*; met SK-N-AS | 1.0 | Lung ca. (non-s.cl) NCl-H522 | 0.4 |
| astrocytoma SF-539 | 6.5 | Lung ca. (squam.) SW 900 | 0.9 |
| astrocytoma SNB-75 | 1.4 | Lung ca. (squam.) NCl-H596 | 3.4 |
| glioma SNB-191 | 11.5 | Mammary gland | 9.9 |
| glioma U251 | 5.1 | Breast ca.* (pl.ef) MCF-7 | 13.3 |
| glioma SF-295 | 0.5 | Breast ca.* (pl.ef) MDA-MB-231 | 3.2 |
| Heart (fetal) | 45.1 | Breast ca.* (pl.ef) T47D | 12.2 |
| Heart | 21.9 | Breast ca. BT-549 | 1.6 |
| Skeletal muscle (fetal) | 27.9 | Breast ca. MDA-N | 3.9 |
| Skeletal muscle | 20.2 | Ovary | 10.4 |
| Bone marrow | 3.5 | Ovarian ca. OVCAR-3 | 4.5 |
| Thymus | 30.1 | Ovarian ca. OVCAR-4 | 2.6 |
| Spleen | 3.0 | Ovarian ca. OVCAR-5 | 3.7 |
| Lymph node | 2.4 | Ovarian ca. OVCAR-8 | 5.3 |
| Colorectal | 52.5 | Ovarian ca. IGROV=1 | 0.7 |
| Stomach | 6.7 | Ovarian ca.* (ascites) SK-OV-3 | 1.0 |
| Small intestine | 17.1 | Uterus | 1.2 |
| Colon ca. SW480 | 9.7 | Placenta | 0.2 |
| Colon ca.* SW620(SW480 met) | 3.8 | Prostate | 14.5 |
| Colon ca. HT29 | 13.1 | Prostate ca.* (bone met)PC-3 | 1.1 |
| Colon ca. HCT-116 | 3.5 | Testis | 2.3 |
| Colon ca. CaCo-2 | 14.1 | Melanoma Hs688(A).T | 0.1 |
| Colon ca. tissue(ODO3866) | 17.8 | Melanoma* (met) Hs688(B).T | 0.6 |
| Colon ca.HCC-2998 | 18.0 | Melanoma UACC-62 | 0.0 |
| Gastric ca.* (liver met) NCI-N87 | 7.9 | Melanoma M14 | 2.3 |
| Bladder | 5.6 | Melanoma LOX IMVI | 0.2 |
| Trachea | 28.5 | Melanoma* (met) SK-MEL-5 | 0.1 |
| Kidney | 29.9 | Adipose | 1.0 |

**Table GD. Panel 2D**

| **Column A - Rel. Exp.(%) Ag2868, Run 162011370** | | | |
|---|---|---|---|
| Tissue Name | A | Tissue Name | A |
| Normal Colon | 43.8 | Kidney Margin 8120608 | 9.7 |
| CC Well to Mod Diff (ODO3866) | 5.9 | Kidney Cancer 8120613 | 31.9 |
| CC Margin (ODO3866) | 8.9 | Kidney Margin 8120614 | 13.8 |
| CC Gr.2 rectosigmoïd (ODO3868) | 19.1 | Kidney Cancer 9010320 | 4.9 |
| CC Margin (ODO3868) | 1.4 | Kidney Margin 9010321 | 17.1 |
| CC Mod Diff (ODO3920) | 34.2 | Normal Uterus | 0.3 |
| CC Margin (ODO3920) | 15.7 | Uterus Cancer 064011 | 3.4 |
| CC Gr.2 ascend colon (ODO3921) | 38.4 | Normal Thyroid | 8.0 |
| CC Margin (ODO3921) | 16.0 | Thyroid Cancer 064010 | 7.5 |
| CC from Partial Hepatectomy (ODO4309) Mets | 28.7 | Thyroid Cancer A302152 | 6.8 |
| Liver Margin (ODO4309) | **100.0** | Thyroid Margin A302153 | 8.2 |
| Colon mets to lung (ODO4451-01) | 6.7 | Normal Breast | 4.6 |
| Lung Margin (OD04451-02) | 0.8 | Breast Cancer (OD04566) | 12.1 |
| Normal Prostate 6546-1 | 4.1 | Breast Cancer (OD04590-01) | 22.1 |
| Prostate Cancer (OD04410) | 20.3 | Breast Cancer Mets (OD04590-03) | 22.7 |
| Prostate Margin (OD04410) | 16.3 | Breast Cancer Metastasis (OD04655-05) | 23.8 |
| Prostate Cancer (OD04720-01) | 8.4 | Breast Cancer 064006 | 4.8 |
| Prostate Margin (OD04720-02) | 11.2 | Breast Cancer 1024 | 41.2 |
| Normal Lung 061010 | 6.7 | Breast Cancer 9100266 | 16.0 |
| Lung Met to Muscle (ODO4286) | 0.5 | Breast Margin 9100265 | 6.9 |
| Muscle Margin (ODO4286) | 0.3 | Breast Cancer A209073 | 5.5 |
| Lung Malignant Cancer (OD03126) | 10.4 | Breast Margin A209073 | 7.8 |
| Lung Margin (OD03126) | 4.1 | Normal Liver | 55.5 |
| Lung Cancer (OD04404) | 20.9 | Liver Cancer 064003 | 17.7 |
| Lung Margin (OD04404) | 1.4 | Liver Cancer 1025 | 70.7 |
| Lung Cancer (OD04565) | 3.7 | Liver Cancer 1026 | 20.3 |
| Lung Margin (OD04565) | 1.3 | Liver Cancer 6004-T | 90.1 |
| Lung Cancer (OD04237-01) | 14.2 | Liver Tissue 6004-N | 5.2 |
| Lung Margin (OD04237-02) | 1.0 | Liver Cancer 6005-T | 17.2 |
| Ocular Mel Met to Liver (ODO4310) | 6.8 | Liver Tissue 6005-N | 32.5 |
| Liver Margin (ODO4310) | 58.6 | Normal Bladder | 5.4 |
| Melanoma Mets to Lung (OD04321) | 5.7 | Bladder Cancer 1023 | 2.8 |
| Lung Margin (OD04321) | 2.9 | Bladder Cancer A302173 | 2.5 |
| Normal Kidney | 30.1 | Bladder Cancer (OD04718-01) | 7.2 |
| Kidney Ca, Nuclear grade 2 (OD04338) | 42.9 | Bladder Normal Adjacent (OD04718-03) | 1.4 |
| Kidney Margin (OD04338) | 17.6 | Normal Ovary | 1.5 |
| Kidney Ca Nuclear grade 1/2 (OD04339) | 7.3 | Ovarian Cancer 064008 | 9.6 |
| Kidney Margin (OD04339) | 14.7 | Ovarian Cancer (OD04768-07) | 1.4 |
| Kidney Ca, Clear cell type (OD04340) | 0.3 | Ovary Margin (OD04768-08) | 0.2 |
| Kidney Margin (OD04340) | 14.1 | Normal Stomach | 4.8 |
| Kidney Ca, Nuclear grade 3 (OD04348) | 0.2 | Gastric Cancer 9060358 | 0.6 |
| Kidney Margin (OD04348) | 7.3 | Stomach Margin 9060359 | 5.0 |
| Kidney Cancer (OD04622-01) | 9.3 | Gastric Cancer 9060395 | 7.9 |
| Kidney Margin (OD04622-03) | 4.0. | Stomach Margin 9060394 | 6.8 |
| Kidney Cancer (OD04450-01) | 10.7 | Gastric Cancer 9060397 | 16.8 |
| Kidney Margin (OD04450-03) | 11.7 | Stomach Margin 9060396 | 3.4 |
| Kidney Cancer 8120607 | 2.4 | Gastric Cancer 064005 | 8.0 |

**Table GE. Panel 4D**

| **Column A - Rel. Exp.(%) Ag2868, Run 159776784** | | | |
|---|---|---|---|
| **Tissue Name** | **A** | **Tissue Name** | **A** |
| Secondary Th1 act | 48.0 | HUVEC IL-1beta | 0.1 |
| Secondary Th2 act | 40.9 | HUVEC IFN gamma | 2.7 |
| Secondary Tr1 act | 55.1 | HUVEC TNF alpha + IFN gamma | 0.3 |
| Secondary Th1 rest | 3.1 | HUVEC TNF alpha + IL4 | 1.1 |
| Secondary Th2 rest | 7.8 | HUVEC IL-11 | 2.6 |
| Secondary Tr1 rest | 12.5 | Lung Microvascular EC none | 1.9 |
| Primary Th1 act | 64.6 | Lung Microvascular EC TNFalpha + IL-1beta | 1.8 |
| Primary Th2 act | 52.9 | Microvascular Dermal EC none | 1.0 |
| Primary Tr1 act | 88.3 | Microsvasular Dermal EC TNFalpha + IL-1beta | 1.4 |
| Primary Th1 rest | 54.0 | Bronchial epithelium TNFalpha + IL-1beta | 1.1 |
| Primary Th2 rest | 30.6 | Small airway epithelium none | 3.8 |
| Primary Tr1 rest | **100.0** | Small airway epithelium TNPalpha + IL-1beta | 14.2 |
| CD45RA CD4 lymphocyte act | 17.0 | Coronery artery SMC rest | 0.3 |
| CD45RO CD4 lymphocyte act | 33.0 | Coronery artery SMC TNFalpha + IL-1beta | 0.0 |
| CD8 lymphocyte act | 30.8 | Astrocytes rest | 3.0 |
| Secondary CD8 lymphocyte rest | 30.8 | Astrocytes TNFalpha + IL-1beta | 1.7 |
| Secondary CD8 lymphocyte act | 16.4 | KU-812 (Basophil) rest | 25.3 |
| CD4 lymphocyte none | 3.5 | KU-812 (Basophil) PMA/ionomycin | 50.0 |
| 2ry Th1/Th2/Tr1_anti-CD95 CH11 anti-CD95 | 9.9 | CCD1106 (Keratinocytes) none | 12.7 |
| LAK cells rest | 10.2 | CCD1106 (Keratinocytes) TNFalpha + IL-1beta | 0.5 |
| LAK cells IL-2 | 24.5 | Liver cirrhosis | 3.8 |
| LAK cells IL-2+IL-12 | 30.4 | Lupus kidney | 2.0 |
| LAK cells IL-2+IFN gamma | 31.4 | NCI-H292 none | 31.4 |
| LAK cells IL-2+ IL-18 | 33.0 | NCI-H292 IL-4 | 36.1 |
| LAK cells PMA/ionomycin | 1.8 | NCI-H292 IL-9 | 41.8 |
| NK Cells IL-2 rest | 14.5 | NCI-H292 IL-13 | 25.5 |
| Two Way MLR 3 day | 6.4 | NCI-H292 IFN gamma | 23.7 |
| Two Way MLR 5 day | 13.6 | HPAEC none | 1.0 |
| Two Way MLR 7 day | 11.7 | HPAEC TNF alpha + IL-1 beta | 0.2 |
| PBMC rest | 2.0 | Lung fibroblast none | 1.5 |
| PBMC PWM | 43.8 | Lung fibroblast TNF alpha + IL-1 beta | 0.3 |
| PBMC PHA-L | 22.7 | Lung fibroblast IL-4 | 1.0 |
| Ramos (B cell) none | 23.7 | Lung fibroblast IL-9 | 1.9 |
| Ramos (B cell) ionomycin | 62.9 | Lung fibroblast IL-13 | 0.7 |
| B lymphocytes PWM | 76.8 | Lung fibroblast IFN gamma | 0.5 |
| B lymphocytes CD40L and IL-4 | 26.6 | Dermal fibroblast CCD1070 rest | 1.8 |
| EOL-1 dbcAMP | 27.2 | Dermal fibroblast CCD1070 TNF alpha | 31.6 |
| EOL-1 dbcAMP PMA/ionomycin | 12.3 | Dermal fibroblast CCD1070 IL-1 beta | 2.3 |
| Dendritic cells none | 8.1 | Dermal fibroblast IFN gamma | 0.2 |
| Dendritic cells LPS | 3.1 | Dermal fibroblast IL-4 | 2.6 |
| Dendritic cells anti-CD40 | 5.9 | IBD Colitis 2 | 0.6 |
| Monocytes rest | 1.2 | IBD Crohn's | 3.0 |
| Monocytes LPS | 0.9 | Colon | 31.6 |
| Macrophages rest | 15.1 | Lung | 2.9 |
| Macrophages LPS | 1.4 | Thymus | 29.5 |
| HUVEC none | 1.7 | Kidney | 11.9 |
| HUVEC starved | 1.8 | | |

**Table GF. Panel 5 Islet**

| **Column A - Rel. Exp.(%) Ag2868, Run 233071460** | | | |
|---|---|---|---|
| **Tissue Name** | **A** | **Tissue Name** | **A** |
| 97457_Patient-02go_adipose | 1.6 | 94709_Donor 2 AM - A_adipose | 6.4 |
| 97476_Patient-07sk_skeletal muscle | 0.0 | 94710_Donor 2 AM - B_adipose | 0.0 |
| 97477_Patient-07ut_uterus | 3.5 | 94711_Donor 2 AM - C_adipose | 0.0 |
| 97478_Patient-07pl_placenta | 9.4 | 94712_Donor 2 AD-A_adipose | 4.0 |
| 99167_Bayer Patient 1 | 12.2 | 94713_Donor 2 AD - B_adipose | 2.0 |
| 97482_Patient-08ut_uterus | 0.7 | 94714_Donor 2 AD - C_adipose | 0.0 |
| 97483_Patient-08p1_placenta | 4.0 | 94742_Donor 3 U - A_Mesenchymal Stem Cells | 0.0 |
| 97486_Patient-09sk_skeletal muscle | 0.0 | 94743_Donor 3 U - B_Mesenchymal Stem Cells | 0.0 |
| 97487_Patient-09ut_uterus | 4.0 | 94730_Donor 3 AM - A_adipose | 3.9 |
| 97488_Patient-09pl_placenta | 9.8 | 94731_Donor 3 AM - B_adipose | 1.7 |
| 97492_Patient-10ut_uterus | 6.6 | 94732_Donor 3 AM - C_adipose | 4.1 |
| 97493_Patient-10pl_placenta | 6.1 | 94733_Donor 3 AD - A_adipose | 1.2 |
| 97495_Patient-11go_adipose | 7.5 | 94734_Donor 3 AD - B_adipose | 2.0 |
| 97496_Patient-11sk_skeletal muscle | 12.5 | 94735_Donor 3 AD - C_adipose | 3.7 |
| 97497_Patient-11ut_uterus | 3.9 | 77138_Liver_HepG2untreated | 85.3 |
| 97498_Patient-11pl_placenta | 5.0 | 73556 Heart Cardiac stromal cells (primary) | 0.0 |
| 97500_Patient-12go_adipose | 13.8 | 81735_Small Intestine | 58.2 |
| 97501_Patient-12sk_skeletal muscle | 20.6 | 72409_Kidney_Proximal Convoluted Tubule | 11.6 |
| 97502_Patient-12ut_uterus | 1.3 | 82685_Small intestine_Duodenum | 58.2 |
| 97503_Patient-12pl_placenta | 2.1 | 90650_Adrenal_Adrenocortical adenoma | 5.4 |
| 94721_Donor2 U - A_Mesenchymal Stem Cells | 2.9 | 72410_Kidney_HRCE | **100.0** |
| 94722_Donor 2 U - B_Mesenchymal Stem Cells | 1.5 | 72411 _Kidney_HRE | 35.1 |
| 94723_Donor 2 U - C_Mesenchymal Stem Cells | 3.3 | 73139_Uterus_Utenrine smooth muscle cells | 0.0 |

**CNS_neurodegeneration_v1.0 Summary:** Data obtained from RTQPCR assays performed for this panel was analyized using multivariate analysis (ANOVA). The multivariate results obtained using Ag2868 indicates this gene is found to be down-regulated in the temporal cortex of Alzheimer's disease patients. Therefore, up-regulation of this gene or its protein product, or treatment with specific agonists for this receptor may be of use in reversing the dementia/memory loss associated with this disease and neuronal death. This gene encodes for D-beta-hydroxybutyrate dehydrogenase homolog. D-beta-hydroxybutyrate dehydrogenase function is also controlled at the translational, post-translational and catalytic levels. (Kante A, et al., 1990, Biochim Biophys Acta 1033(3):291-7). Please see Panel 1.3D for additional discussion of role of this gene in the central nervous system.

**Panel 1.3D Summary:** Ag2868 Expression of this gene is highest in the cerebral cortex (CT=27.6). The expression of this gene in multiple brain regions is consistent with a published role for this gene in CNS energetic processes. This gene encodes a hydroxybutyrate dehydrogenase homolog. D-beta-hydroxybutyrate protects neurons in models of Alzheimer's and Parkinson's disease. Other enzymes, such as amyloid beta-peptide-binding alcohol dehydrogenase, which have been shown to possess ID-beta-hydroxybutyrate dehydrogenase activity, contribute to the protective response to metabolic stress, especially in the setting of ischemia (Kashiwaya Y, et al. Proc Nat1 Acad Sci U S A 2000 May 9;97(10):5440-4; Du Yan S, et al. J Biol Chem 2000 Sep 1;275(35):27100-9). Since this protein encoded by this gene processes D-beta-hydroxybutyrate to provide a neuronal energy source, activators of the protein encoded by this gene may be useful in treating and protecting the CNS of Alzheimer's and Parkinson's disease patients, as well as stroke.

Overall, expression of this gene appears to be largely associated with normal tissues when compared to cancer cell lines. Thus, therapeutic modulation of this gene, through the use of small molecule drugs, antibodies or protein therapeutics might be of benefit in the treatment of cancer.

This gene is also moderately expressed in a variety of metabolic tissues, including pancreas, adrenal, thyroid, pituitary, adult and fetal heart, adult and fetal skeletal muscle, adult and fetal liver and adipose. Mutations in the hydroxybutyrate dehydrogenase enzyme are associated with hypoglycemia and cardiac arrest. Activators of this enzyme could be drug targets for obesity because increased fatty acid oxidation may prevent the incorporation of fatty acids into triglylcerides, thus decreasing adipose mass.

**Panel 2D Summary:** Ag2868 The expression of this gene appears to be highest in a sample derived from normal liver tissue adjacent to a metastatic colon cancer (CT=25.9). In addition, there appears to be substantial expression associated with malignant liver tissue when compared to their associated normal adjacent tissue. Thus, therapeutic modulation of this gene, through the use of small molecule drugs, antibodies or protein therapeutics might be of benefit in the treatment of liver cancer.

**Panel 4D Summary:** Ag2868 This gene is expressed primarily in activated leukocytes, especially in T cells and B cells (CTs=27-30). It is also expressed in NCI-H292 cells and in TNF alpha treated dermal fibroblasts. The protein encoded for by this trancript has homology to hydroxybutyrate dehydrogenase, a protein that has been found in lymphocytes (Curi R, Williams JF, Newsholme EA., 1989, Pyruvate metabolism by lymphocytes: evidence for an additional ketogenic tissue. Biochem Int 19(4):755-67). Thus, the protein encoded for by this transcript may be important for cellular responses to inflammatory/activating stimuli. Therefore, therapeutics designed with the protein encoded for by this transcript could be used for the treatment of inflammatory diseases such as asthma, emphysema, COPD, arthritis, IBD and psoriasis.

**Panel 5 Islet Summary:** Ag2868 Expression of this gene is highest a in kidney cell line (CT=32.8). In addition low expression of this gene is also seen in a liver cancer cell line, and small intestine. Please see panel 1.3D for further discussion of this gene.

### H. CG56151-01: Glucose transporter type2

Expression of gene CG56151-01 was assessed using the primer-probe set Ag1681, described in Table HA. Results of the RTQ-PCR runs are shown in Tables HB, HC, HD, HE, HF and HG.

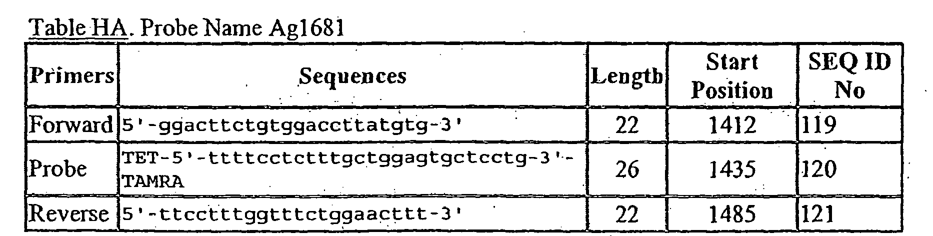

**Table HB. General screening panel v1.4**

| **Column A - Rel. Exp.(%) Ag1681, Run 208016706** | | | |
|---|---|---|---|
| **Tissue Name** | **A** | **Tissue Name** | **A** |
| Adipose | 0.0 | Renal ca. TK-10 | 0.1 |
| Melanoma* Hs688(A).T | 0.0 | Bladder | 0.4 |
| Melanoma* Hs688(B).T | 0.0 | Gastric ca. (liver met.) NCI-N87 | 0.7 |
| Melanoma* M14 | 0.2 | Gastric ca. KATO III | 0.1 |
| Melanoma* LOXIMVI | 0.2 | Colon ca. SW-948 | 0.0 |
| Melanoma* SK-MEL-5 | 0.0 | Colon ca. SW480 | 0.2 |
| Squamous cell carcinoma SCC-4 | 0.0 | Colon ca.* (SW480 met) SW620 | 0.0 |
| Testis Pool | 0.4 | Colon ca. HT29 | 0.1 |
| Prostate ca.* (bone met) PC-3 | 0.0 | Colon ca. HCT-116 | 0.1 |
| Prostate Pool | 0.0 | Colon ca. CaCo-2 | 3.7 |
| Placenta | 0.0 | Colon cancer tissue | 0.0 |
| Uterus Pool | 0.0 | Colon ca. SW1116 | 0.0 |
| Ovarian ca. OVCAR-3 | 0.4 | Colon ca. Colo-205 | 0.1 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.1 | Small Intestine Pool | 0.0 |
| Ovarian ca. IGROV-1 | 0.0 | Stomach Pool | 0.2 |
| Ovarian ca. OVCAR-8 | 0.1 | Bone Marrow Pool | 0.0 |
| Ovary | 0.2 | Fetal Heart | 0.0 |
| Breast ca. MCF-7 | 0.2 | Heart Pool | 0.1 |
| Breast ca. MDA-MB-231 | 0.0 | Lymph Node Pool | 0.1 |
| Breast ca. BT 549 | 0.0 | Fetal Skeletal Muscle | 0.1 |
| Breast ca. T47D | 0.1 | Skeletal Muscle Pool | 0.0 |
| Breast ca. MDA-N | 0.2 | Spleen Pool | 0.1 |
| Breast Pool | 0.1 | Thymus Pool | 0.0 |
| Trachea | 0.0 | CNS cancer (glio/astro) U87-MG | 0.0 |
| Lung | 0.1 | CNS cancer (glio/astro) U-118-MG | 0.0 |
| Fetal Lung | 1.2 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.1 | CNS cancer (astro) SNB-75 | 0.1 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.2 |
| Lung ca. NCI-H526 | 0.0 | Brain (cerebellum) | 0.0 |
| Lung ca. NCI-H23 | 0.2 | Brain (fetal) | 0.0 |
| Lung ca. NCI-H460 | 0.0 | Brain (Hippocampus) Pool | 0.1 |
| Lung ca. HOP-62 | 0.6 | Cerebral Cortex Pool | 0.2 |
| Lung ca. NCI-H522 | 0.0 | Brain (Substantia nigra) Pool | 0.4 |
| Liver | 23.2 | Brain (Thalamus) Pool | 0.1 |
| Fetal Liver | **100.0** | Brain (whole) | 0.4 |
| Liver ca. HepG2 | 7.4 | Spinal Cord Pool | 0.3 |
| Kidney Pool | 0.1 | Adrenal Gland | 0.1 |
| Fetal Kidney | 1.9 | Pituitary gland Pool | 0.1 |
| Renal ca. 786-0 | 0.6 | Salivary Gland | 0.0 |
| Renal ca. A498 | 0.1 | Thyroid (female) | 0.0 |
| Renal ca. ACHN | 4.8 | Pancreatic ca. CAPAN2 | 0.5 |
| Renal ca. UO-31 | 0.1 | Pancreas Pool | 0.5 |

**Table HC. Human Metabolic**

| **Column A-Rel. Exp.(%) Ag1681, Run 324668035** | | | |
|---|---|---|---|
| **Tissue Name** | **A** | **Tissue Name** | **A** |
| 137857_psoas-AA.M.Diab.-hi BMI-6 | 0.0 | 139523_pancreas-HI.M.Norm-hi BMI-31 | 0.0 |
| 135760_psoas-HI.M.Diab.-hi BMI-21 | 0.7 | 139520_pancreas-CC.M.Norm-hi BMI-29 | 0.0 |
| 134827_psoas-CC.M.Diab.-hi BMI-4 | 0.0 | 142744_pancreas-HI.M.Norm-med BMI-35 | 0.0 |
| 137860_psoas-AA.M.Diab.-med BMI-8 | 0.0 | 139545_pancreas-AA.M.Norm-med BMI-47 | 0.0 |
| 137834_psoas-CC.M.Diab.-med BMI-2 | 0.0 | 139531_pancreas-AA.M.Norm-med BMI-37 | 0.0 |
| 137828_psoas-CC.M.Diab.-med BMI-1 | 0.0 | 137871_pancreas-CC.M.Norm-med BMI-26 | 0.7 |
| 135763_psoas-HI.M.Diab.-med BMJ-23 | 0.0 | 139541_pancreas-Hi.M.Norm-low BMI-41 | 0.0 |
| 142740_psoas-AS.M.Diab.-low BMI-20 | 0.0 | 139537_pancreas-CC.M.Norm-low BM1-40 | 0.0 |
| 134834_psoas-AA.M.Diab.-low BMI-17 | 0.1 | 139533_pancreas-CC.M.Norm-low BMI-39 | 0.0 |
| 137850_psoas-AS.M.Norm-hi BMI-34 | 0.0 | 137845_pancreas-AS.M.Nonn-low BMI-28 | 0.1 |
| 135769_psoas-HI.M.Norm-hi BMI-31 | 0.0 | 143530_small intestine-AA.M.Diab.-hi BM1-6 | 0.0 |
| 135766_psoas-AA.M.Nonn-bi BMI-25 | 0.0 | 143529_small intestine-CC.M.Diab.-hi BMI-4 | 0.1 |
| 142746_psoas-AA.M.Norm-med BMI-37 | 0.0 | 143538_small intestine-HI.M.Diab.-med BMI-23 | 0.0 |
| 142745_psoas-HI.M.Norm-med BMI-35 | 0.0 | 143531_small intestine-AA.M.Diab.-med BMI-8 | 0.1 |
| 137855_psoas-AA.M.Norm-med BMI-47 | 0.0 | 143528_small intestine-CC.M.Diab.-med BMI-2 | 0.0 |
| 137844_psoas-CC.M.Norm-med BMI-26 | 0.0 | 143537_small intestine-HI.M.Diab.-low BMI-22 | 0.0 |
| 142742_psoas-CC.M.Norm-low BMI-40 | 0.0 | 143535_small intestine-AS.M.Diab.-low BMI-20 | 0.0 |
| 137873_psoas-AS.M.Norm-low BMI-28 | 0.0 | 143534_small intestine-AA.M.Diab.-low BMI-17 | 0.0 |
| 137853_psoas-HI.M.Norm-low BMI-41 | 0.0 | 143544_small intestine-AS.M.Norm-hi BMI-34 | 0.1 |
| 135775_psoas-CC.M.Norm-low BMI-39 | 0.0 | 143543_small intestine-HI.M.Norm-hi BMI-31 | 1.4 |
| 137858_diaphragm-AA.M.Diab.-hi BMI-6 | 0.0 | 143542_small intestine-CC.M.Norm-hi BMI-29 | 0.0 |
| 135772_diaphragm-AS.M.Diab-hi BMI-9 | 0.0 | 143539_small intestine-AA.M.Norm-hi BMI-25 | 0.0 |
| 135761_diaphragm-HI.M.Diab.-hi BMI-21 | 0.1 | 143548_small intestine-AA.M.Norm-med BMI-47 | 0.1 |
| 134828_diaphragm-CC.M.Diab.-hi BMI-4 | 0.0 | 143547_small intestine-AA.M.Norm-med BMI-37 | 0.1 |
| 137835_diaphragm-CC.M.Diab.-med BMI-2 | 0.0 | 143540_small intestine-CC.M.Norm-med BMI-26 | 0.0 |
| 135764_diaphragm-HI.M.Diab.-med BMI-23 | 0.0 | 143550_small intestine-CC.M.Norm-low BMI-40 | 0.0 |
| 134835_diaphragm-AA.M.Diab.-low BMI-17 | 0.0 | 143549_small intestine-CC.M.Norm-low BMI-39 | 0.0 |
| 142738_diaphragm-CC.M.Norm-hi BMI-29 | 0.0 | 143546_small intestine-HI.M.Norm-low BMI-41 | 0.1 |
| 139517_diaphragm-AS.M.Norm-hi BMI-34 | 0.0 | 143525_hypothalamus-HI.M.Diab.-hi BMI-21 | 0.0 |
| 137848_diaphragm-HI.M.Norm-hi BMI-31 | 0.0 | 143515_hypothalamus-CC.M.Diab.-hi BMI-4 | 0.0 |
| 137843_diaphragm-AA.M.Norm-hi BMI-25 | 0.0 | 143513_hypothalamus-AA.M.Diab.-hi BMI-6 | 0.2 |
| 137879_diaphragm-AA.M.Norm-med BMI-47 | 0.0 | 143507_hypothalamus-AS.M.Diab.-hî BMI-9 | 0.1 |
| 137872_diaphragm-CC.M.Norm-med BMI-26 | 0.0 | 143506_hypothalamus-CC.M.Diab.-med BMI-1 | 0.2 |
| 135773_diaphragm-HI.M.Nonm-med BMI-35 | 0.0 | 143505_hypothalamus-HI.M.Diab.-med BMI-23 | 0.1 |
| 139542_diaphragm-HI.M.Norm-low BMI-41 | 0.0 | 143509_hypothalamus-AA.M.Diab.-low BMI-17 | 0.1 |
| 137877_diaphragm-CC.M.Norm-low BMI-39 | 0.0 | 143508_hypothalamus-CC.M.Diab.-low BMI-13 | 0.1 |
| 137874_diaphragm-AS.M.Norm-low BMI-28 | 0.0 | 143503_hypothalamus-AS.M.Diab.-low BMI-20 | 0.1 |
| 141340_subQadipose-AA.M.Diab.-hi BMI-6 | 0.0 | 143522_hypothalamus-HI.M.Norm-hi BMI-31 | 0.0 |
| 137836_subQadipose-HI.M.Diab.-hi-BMI-21 | 0.0 | 143516_hypothalamus-AS.M.Norm-hi BMI-34 | 0.0 |
| 135771_subQadipose-AS.M.Diab-hi BMI-9 | 0.0 | 143511_hypothalamus-CC.M.NOrm-hi BMI-29 | 0.2 |
| 141329_subQadipose-AA.M.Diab-medbmi-8 | 0.0 | 1435_04_hypothalamus-AA.M.Norm-hi BMI-25 | 0.1 |
| 137862_subQadipose-CC.M.Diab.-med BMI-1 | 0.0 | 143517_hypothalamus-AA.M.Norm-med BMI-47 | 0.1 |
| 135762_subQadipose-HI.M.Diab.-med BMI-23 | 0.0 | 143514_hypothalamus-HI.M.Norm-med BMI-35 | 0.0 |
| 141338_subQadipose-AS.M.Diab.-low BMI-20 | 0.0 | 143521_hypothalamus-AS.M.Norm-low BMI-28 | 0.0 |
| 139547_subQadipose-HI.M.Diab.-low BMI-22 | 0.1 | 143512_hypothalamus-CC.M.Norm-low BMI-40 | 0.0 |
| 135757_subQadipose-CC.M.Diab.-low BMI-13 | 0.0 | 145454_Patient-25pl (CC.Diab.low BMI.no insulin) | 0.0 |
| 134832_subQadipose-AA.M.Diab.-low BMI-17 | 0.0 | 110916_Patient-18pl (HI.Diab.obese.no insulin) | 0.0 |
| 141332_subQadipose-HI.M.Norm-hi BMI-31 | 0.0 | 110913_Patient-18go (HI.Diab.obese.no 0.0 insulin) | 0.0 |
| 135767_subQadipose-CC.M.Norm-hi BMI-29 | 0.0 | 110911_Patient-17p1 (CC.Diab.low BMI.no insulin) | 0.0 |
| 135765_subQadipose-AS.M.Norm-hi BMI-34 | 0.0 | 110908_Patient-17go (CC.Diab.low BMI.no 0.0 insulin) | 0.0 |
| 141339_subQadipose-HI.M.Norm-med BMI-35 | 0.0 | 100752_Patient-15sk (CC.Diab.obese.no insulin) | 0.0 |
| 141334_subQadipose-CC.M.Norm-med BMI-26 | 0.0 | 0.0 97828_Patient-13pl (CC.Diab.overwt.no insulin) | 0.1 |
| 139544_subQadipose-AA.M.Norm-med BMI-47 | 0.0 | 160114_Patient 27-ut (CC.Diab.obese.insulin) | 0.0 |
| 137875_subQadipose-AA.M.Norm-med BMI-37 | 0.0 | 160113_Patient 27-pl (CC.Diab.obese.insulin) | 0.0 |
| 141331_subQadipose-AS.M.Norm-low BMI-28 | 0.0 | 160112_Patient 27-sk (CC.Diab.obese.insulin) | 0.0 |
| 137878_subQadipose-HI.M.Norm-low BMI-41 | 0.0 | 160111_Patient 27-go (CC.Diab.obese.insulin) | 0.1 |
| 137876_subQadipose-CC.M.Nonn-low BMI-39 | 0.0 | 145461_Patient-26sk (CC.Diab.obese.insulin) | 0.0 |
| 137859_vis.adipose-AA.M.Diab.-hi BMI-6 | 0.0 | 145441_Patient-22sk (CC.Diab.low BMI.insulin) | 0.1 |
| 135770_vis.adipose-AS.M.Diab-hi BMI-9 | 0.0 | 145438_Patient-22pl (CC.Diab.low BMI.insulin) | 0.0 |
| 135759_vis.adipose-HI.M.Diab.-hi BMI-21 | 0.0 | 145427_Patient-20pl (CC.Diab.overwt.insulin) | 0.1 |
| 143502_vis.adipose-CC.M.Diab.-med BMI-2 | 0.0 | 97503_Patient-12pl (CC.Diab.unknown 0.0 BMI.insulin) | 0.0 |
| 139510_vis.adipose-AA.M.Diab.-med BMI-8 | 0.0 | 145443_Patient-23pl (CC.Non-diab.overwt) | 0.0 |
| 137861_vis.adipose-CC.M.Diab.-med-1 | 0.0 | 145435_Patient-21pl (CC.Non-diab.overwt) | 0.0 |
| 137839_vis.adipose-HI.M.Diab.-med BMI-23 | 0.0 | 110921_Patient-19pl (CC.Non-diab.low BMI) | 0.0 |
| 139546_vis.adipose-HI.M.Diab.-low BMI-22 | 0.0 | 110918_Patient-19go (CC.Non-diab.low BMI) | 0.1 |
| 137831_vis.adipose-CC.M.Diab.-low BMI-13 | 0.0 | 97481_Patient-08sk (CC.Non-diab.obese) | 0.0 |
| 139522_vis.adipose-HI.M.Norm-hi BMI-31 | 0.0 | 97478_Patient-07pl (CC.Non-diab.obese) | 0.0 |
| 139516_vis.adipose-AS.M.Norm-hi BMI-34 | 0.0 | 160117 Human Islets-male, obese | 0.5 |
| 137846_vis.adipose-CCM.Norm-hi BMI-29 | 0.0 | 145474_PANC1 (pancreas carcinoma)1 | 0.7 |
| 137841_vis.adipose-AA.M.Norm-hi BMI-25 | 0.0 | 154911_Capan2 (pancreas adenocarcinoma) | 4.4 |
| 139543_vis.adipose-AA.M.Norm-med BMI-47 | 0.0 | 141190_SW579 (thyroid carcinoma) | 0.0 |
| 139532_vis.adipose-AA.M.Norm-med BMI-37 | 0.0 | 145489_SK-N-MC (neuroblastoma) 1 | 0.0 |
| 139530_vis.adipose-HI.M.Norm-med BMI-35 | 0.0 | 145495_SK-N-SH (neuroblastoma) 1 | 0.5 |
| 139539_vis.adipose-HI.M.Norm-low BMI-41 | 0.0 | 145498 US7 MG (glioblastoma) 2 | 0.0 |
| 139535_vis.adipose-CCM.Norm-low BMI-40 | 0.1 | 145484_HEp-2 (larynx carcinoma) 1 | 0.0 |
| 137852_vis.adipose-CC.M.Norm-low BMI-39 | 0.0 | 145479_A549 (lung carcinoma) | 0.0 |
| 135768_vis.adipose-AS.M.Norm-10w BMI-28 | 0.0 | 145488_A427 (lung carcinoma) 2 | 0.0 |
| 141327_liver-CC.M.Diab.-hi BMI-4 | 0.2 | 145472_FHs 738Lu (normal lung) 1 | 0.1 |
| 139514_liver-HI.M.Diab.-hi BMI-21 | 3.7 | 141187_SKW6.4 (B lymphocytes) | 0.0 |
| 139526_liver-CC.M-Diab.-med BMI-2 | 2.0 | 154644_IM-9 (immunoglobulin secreting lymphoblast) | 0.0 |
| 139511_liver-AA.M.Diab.-med BMI-8 | 1.4 | 154645_MOLT-4 (acute lympboblastic leukemia derived from peripheral blood) | 0.0 |
| 137840_liver-HI.M.Diab.-med BMI-23 | 31.0 | 154648_U-937 (histiocystic lymphoma) | 0.1 |
| 137827_liver-CC.M.Diab.-med BMI-1 | 27.2 | 154647_Daudi (Burkitt's lymphoma) | 0.1 |
| 137838_liver-HI.M.Diab.-low BMI-22 | 28.9 | 145494_SK-MEL-2 (melanoma) 2 | 0.3 |
| 135758_liver-CC.M.Diab.-low BMI-13 | 23.5 | 141176_A375 (melanoma) | 0.0 |
| 139519_liver-CC.M.Norm-hi BMI-29 | 7.9 | 154642_SW1353(humerus chondrosarcoma) | 0.1 |
| 139518_liver-AA.M.Norm-hi BMI-25 | 16.2 | 141179_HiT-1080(fibrosarcoma) | 0.0 |
| 137849_liver-AS.M.Norm-hi BMI-34 | 72.2 | 145491 MG-63 (osteosarcoma) 1 | 0.0 |
| 137847_liver-HI.M.Norm-hi BMI-31 | 26.2 | 141186 MCF7 (breast carcinoma) | 0.0 |
| 142741_liver-AA.M.Norm-med BMI-37 | **100.0** | 141193_T47D(breast carcinoma) | 0.1 |
| 141341_liver-HI.M.Norm-med BMI-35 | 8.6 | 154641 BT-20 (breast carcinoma) | 1.6 |
| 141335_liver-CC.M.Norm-med BMI-26 | 1.7 | 141175_293 (kidney transformed with adenovirus 5 DNA) | 0.0 |
| 139540_liver-HI.M.Nonn-low BMI-41 | 1.6 | 141182_HUH hepatoma 1 | 0.6 |
| 139534_liver-CC.M.Norm-low BMI-39 | 7.4 | 141184_HUH7 hepatoma 1 | 0.8 |
| 139521_liver-AS.M.Norm-low BMI-28 | 15.2 | 145478_HT1376 (bladder carcinoma) | 0.0 |
| 141328_pancreas-CC.M.Diab.-hi BMI-4 | 0.0 | 145481_SCaBER (bladder carcinoma) | 0.3 |
| 139525_pancreas-AS.M.Diab.-hi BMI-9 | 0.0 | 141192_SW620 (lymph node metastatsis, colon carcinoma) 2 | 0.1 |
| 137856_pahcreas-AA.M.Diab.-hi BMI-6 | 0.1 | 141180 HT29 (colon carcinoma) 1 | 0.2 |
| 137837_pancreas-HI.M.Diab.-hi BMI-21 | 0.6 | 141188_SW480 (colon carcinoma) 1 | 0.2 |
| 141337_pancreas-CC.M.Diab.-med BMI-2 | 0.0 | 154646 CAOV-3 (ovary adenocarcinoma) | 0.2 |
| 139527_pancreas-CC.M.Diab-med BMl-1 | 0.0 | 141194_HeLa (cervix carcinoma)-2 | 0.0 |
| 139515_pancreas-HI.M.Diab.-med BMI-23 | 0.0 | 145482_HeLa S3 (cervix carcinoma) 1 | 0.0 |
| 139512_pancreas-AA.M.Diab.-med BMI-8 | 0.0 | 145486_DU145 (prostate carcinoma) | 0.0 |
| 142739_pancreas-AS.M.Diab.-low BMI-20 | 0.0 | 154643_PC-3 (prostate adenocarcinoma) | 0.0 |
| 139513_pancreas-CC.M.Diab.-low BMI-13 | 0.0 | 154649_HCT-8 (ileocecal adenocarcinoma) 154649_HCT-8 (ileocecal adenocarcinoma) | 0.2 |
| 142743_pancreas-AA.M.Norm-hi BMI-25 | 0.0 | | |

**Table HD. Panel 1.3D**

| **Column A - Rel. Exp.(%) Ag1681, Run 146581527** | | | |
|---|---|---|---|
| **Tissue Name** | **A** | **Tissue Name** | **A** |
| Liver adenocarcinoma | 0.0 | Kidney (fetal) | 5.8 |
| Pancreas | 1.5 | Renal ca. 786-0 | 0.5 |
| Pancreatic ca. CA-PAN 2 | 0.3 | Renal ca. A498 | 0.5 |
| Adrenal gland | 0.1 | Renal ca. RXF 393 | 0.1 |
| Thyroid | 0.0 | Renal ca. ACHN | 11.7 |
| Salivary gland | 0.0 | Renal ca. UO-31 | 0.2 |
| Pituitary gland | 0.2 | Renal ca. TK-10 | 0.1 |
| Brain (fetal) | 0.0 | Liver | **100.0** |
| Brain (whole) | 0.0 | Liver (fetal) | 99.3 |
| Brain (amygdala) | 0.0 | Liver ca. (hepatoblast) HepG2 | 22.2 |
| Brain (cerebellum) | 0.0 | Lung | 0.0 |
| Brain (hippocampus) | 0.1 | Lung (fetal) | 0.0 |
| Brain (substantia nigra) | 0.0 | Lung ca. (small cell) LX-1 | 0.0 |
| Brain (thalamus) | 0.0 | Lung ca. (small cell) NCI-H69 | 0.0 |
| Cerebral Cortex | 0.1 | Lung ca. (s.cell var.) SHP-77 | 0.0 |
| Spinal cord | 0.1 | Lung ca. (large cell)NCI-H460 | 0.0 |
| glio/astro U87-MG | 0.2 | Lung ca. (non-sm.cell) A549 | 0.1 |
| glio/astro U-1 18-MG | 0.0 | Lung ca. (non-s.cell) NCI-H23 | 0.7 |
| astrocytoma SW1783 | 0.2 | Lung ca. (non-s.cell) HOP-62 | 0.5 |
| neuro*; met SK-N-AS | 0.1 | Lung ca. (non-s.cl) NCI-H522 | 0.0 |
| astrocytoma SF-539 | 0.2 | Lung ca. (squam.) SW 900 | 0.1 |
| astrocytoma SNB-75 | 0.0 | Lung ca. (squam.) NCI-H596 | 0.0 |
| glioma SNB-19 | 0.1 | Mammary gland , | 0.0 |
| glioma U251 | 0.0 | Breast ca.*(pl.ef) MCF-7 | 0.2 |
| glioma SF-295 | 0.0 | Breast ca.* (pl.ef) MDA-MB-231 | 0.2 |
| Heart (fetal) | 0.0 | Breast ca.* (pl.ef) T47D | 0.2 |
| Heart | 0.0 | Breast ca. BT-549 | 0.0 |
| Skeletal muscle (fetal) | 0.0 | Breast ca. MDA-N | 0.4 |
| Skeletal muscle | 0.0 | Ovary | 0.1 |
| Bone marrow | 0.0 | Ovarian ca. OVCAR-3 | 0.3 |
| Thymus | 0.0 | Ovarian ca. OVCAR-4 | 0.1 |
| Spleen | 0.0 | Ovarian ca. OVCAR-5 | 0.2 |
| Lymph node | 0.0 | Ovarian ca. OVCAR-8 | 0.2 |
| Colorectal | 0.3 | Ovarian ca. IGROV-1 | 0.0 |
| Stomach | 0.1 | Ovarian ca.* (ascites) SK-OV-3 | 0.0 |
| Small intestine | 7.6 | Uterus | 0.0 |
| Colon ca. SW480 | 0.3 | Placenta | 0.0 |
| Colon ca.* SW620(SW480 met) | 0.0 | Prostate | 0.0 |
| Colon ca. HT29 | 0.2 | Prostate ca.* (bone met)PC-3 | 0.0 |
| Colon ca. HCT-116 | 0.0 | Testis | 0.2 |
| Colon ca. CaCo-2 | 8.8 | Melanoma Hs688(A).T | 0.0 |
| Colon ca.tissue(OD03866) | 0.1 | Melanoma* (met) Hs688(B).T | 0.1 |
| Colon ca. HCC-2998 | 1.7 | Melanoma UACC-62 | 0.0 |
| Gastric ca.* (liver met) NCI-N87 | 0.8 | Melanoma M14 | 0.2 |
| Bladder | 0.4 | Melanoma LOX IMVI | 0.0 |
| Trachea | 0.0 | Melanoma* (met) SK-MEL-5 | 0.0 |
| Kidney | 8.9 | Adipose | 0.1 |

**Table HE. Panel 2D**

| **Column A - Rel. Exp.(%) Ag1681, Run 148168295** | | | |
|---|---|---|---|
| **Tissue Name** | **A** | **Tissue Name** | **A** |
| Normal Colon | 0.5 | Kidney Margin 8120608 | 1.7 |
| CC Well to Mod Diff (ODO3866) | 0.0 | Kidney Cancer 8120613 | 0.0 |
| CC Margin (ODO3866) | 0.0 | Kidney Margin 8120614 | 2.2 |
| CC Gr.2 rectosigmoid (ODO3868) | 0.0 | Kidney Cancer 9010320 | 0.1 |
| CC Margin (ODO3868) | 0.0 | Kidney Margin 9010321 | 4.0 |
| CC Mod Diff (ODO3920) | 0.1 | Normal Uterus | 0.0 |
| CC Margin (ODO3920) | 0.0 | Uterus Cancer 064011 | 0.0 |
| CC Gr.2 ascend colon (ODO3921) | 0.0 | Normal Thyroid | 0.1 |
| CC Margin (OD03921) | 0.0 | Thyroid Cancer 064010 | 0.2 |
| CC from Partial Hepatectomy (ODO4309) Mets | 6.6 | Thyroid Cancer A302152 | 0.1 |
| Liver Margin (ODO4309) | **100.0** | Thyroid Margin A302153 | 0.1 |
| Colon mets to lung (OD04451-01) | 0.0 | Normal Breast | 0.1 |
| Lung Margin (OD04451-02) | 0.0 | Breast Cancer (OD04566) | 0.1 |
| Normal Prostate 6546-1 | 0.0 | Breast Cancer (OD04590-01) | 0.0 |
| Prostate Cancer (OD04410) | 0.0 | Breast Cancer Mets (OD04590-03) | 0.1 |
| Prostate Margin (OD04410) | 0.0 | Breast Cancer Metastasis (OD04655-05) | 0.0 |
| Prostate Cancer (OD04720-01) | 0.0 | Breast Cancer 064006 | 0.5 |
| Prostate Margin (OD04720-02) . | 0.0 | Breast Cancer 1024 | 0.0 |
| Normal Lung 061010 | 0.1 | Breast Cancer 9100266 | 0.0 |
| Lung Met to Muscle (OD04286) | 0.0 | Breast Margin 9100265 | 0.0 |
| Muscle Margin (ODO4286) | 0.0 | Breast Cancer A209073 | 0.1 |
| Lung Malignant Cancer (OD03126) | 0.0 | Breast Margin A209073 | 0.1 |
| Lung Margin (OD03126) | 0.0 | Normal Liver | 86.5 |
| Lung Cancer (OD04404) | 0.0 | Liver Cancer 064003 | 23.5 |
| Lung Margin (OD04404) | 0.0 | Liver Cancer 1025 | 39.8 |
| Lung Cancer (OD04565) | 0.0 | Liver Cancer 1026 | 13.6 |
| Lung Margin (OD04565) | 0.0 | Liver Cancer 6004-T | 47.0 |
| Lung Cancer (OD04237-01) | 0.0 | Liver Tissue 6004-N | 8.1 |
| Lung Margin (OD04237-02) | 0.0 | Liver Cancer 6005-T | 12.6 |
| Ocular Mel Met to Liver (OD04310) | 0.0 | Liver Tissue 6005-N | 14.1 |
| Liver Margin (OD04310) | 62.0 | Normal Bladder | 0.3 |
| Melanoma Mets to Lung (OD04321) | 0.0 | Bladder Cancer 1023 | 0.0 |
| Lung Margin (OD04321) | 0.0 | Bladder Cancer A302173 | 0.0 |
| Normal Kidney | 9.2 | Bladder Cancer (OD04718-01) | 0.0 |
| Kidney Ca, Nuclear grade 2 (OD04338) (OD04718-03) | 1.0 | Bladder Normal Adjacent | 0.0 |
| Kidney Margin (OD04338) | 1.5 | Normal Ovary | 0.0 |
| Kidney Ca Nuclear grade 1/2 (OD04339) | 0.0 | Ovarian Cancer 064008 | 0.0 |
| Kidney Margin (OD04339) | 12.9 | Ovarian Cancer (OD04768-07) | 0.8 |
| Kidney Ca, Clear cell type (OD04340) | 10.4 | Ovary Margin (OD04768-08) | 0.0 |
| Kidney Margin (OD04340) | 3.7 | Normal Stomach | 0.0 |
| Kidney_Ca, Nuclear grade 3 (OD04348) | 0.3 | Gastric Cancer 9060358 | 0.0 |
| Kidney Margin (OD04348) | 2.1 | Stomach Margin 9060359 | 0.0 |
| Kidney Cancer (OD04622-01) | 1.0 | Gastric Cancer 9060395 | 0.0 |
| Kidney Margin (OD04622-03) | 0.5 | Stomach Margin 9060394 | 0.3 |
| Kidney Cancer (OD04450-01) | 0.4 | Gastric Cancer 9060397 | 0.0 |
| Kidney Margin (OD04450-03) | 2.2 | Stomach Margin 9060396 | 0.0 |
| Kidney Cancer 8120607 | 0.0 | Gastric Cancer 064005 | 0.4 |

**Table HF. Panel 5 Islet**

| **Column A - Rel. Exp.(%) Ag1681, Run 248029382** | | | |
|---|---|---|---|
| **Tissue Name** | **A** | **Tissue Name** | **A** |
| 97457_Patient-02go_adipose | 0.0 | 94709_Donor 2 AM - A_adipose | 0.0 |
| 97476_Patient-07sk_skeletal muscle | 0.0 | 94710_Donor 2 AM - B adipose | 0.0 |
| 97477_Patient-07ut_uterus | 0.0 | 94711_Donor2 AM - C_adipose | 0.0 |
| 97478_Patient-07pl_placenta | 0.7 | 94712_Donor 2 AD - A_adipose | 0.0 |
| 99167_Bayer Patient 1 | 1.3 | 94713_Donor 2 AD - B_adipose | 0.0 |
| 97482_Patient-08ut_uterus | 0.0 | 94714_Donor 2 AD - C_adipose | 0.0 |
| 97483 _Patient-08pl_placenta | 0.0 | 94742_Donor 3 U - A_Mesenchymal Stem Cells | 0.0 |
| 97486_Patient-09sk_skeletal muscle | 0.0 | 94743_Donor 3 U - B_Mesenchymal Stem Cells | 0.0 |
| 97487_Patient-09ut_uterus | 0.0 | 94730_Donor 3 AM - A_adipose | 0.0 |
| 97488_Patient-09pl_placenta | 0.0 | 94731_Donor 3 AM - B_adipose | 0.0 |
| 97492_Patient-10ut_uterus | 0.0 | 94732_Donor 3 AM - C_adipose | 0.0 |
| 97493_Patient-10pl_placenta | 0.0 | 94733_Donor 3 AD - A_adipose | 0.0 |
| 97495_Patient-1I go_adipose | 1.0 | 94734_Donor 3 AD - B_adipose | 0.0 |
| 97496_Patient-11sk_skeletal muscle | 0.0 | 94735_Donor 3 AD - C_adipose | 0.0 |
| 97497_Patient-11ut_uterus | 0.0 | 77138_Liver_HepG2untreated | 30.1 |
| 97498_Patient-1 1p1_placenta | 0.0 | 73556_Heart_Cardiac stromal cells (primary) | 0.4 |
| 97500_Patient-12go_adipose | 2.1 | 81735_Small Intestine | 17.9 |
| 97501_Patient-12sk_skeletal muscle | 0.0 | 72409_Kidney_Proximal Convoluted Tubule | 1.6 |
| 97502_Patient-12ut_uterus | 0.0 | 82685_Small intestine_Duodenum | **100.0** |
| 97503_Patient-12pl_placenta | 0.0 | 90650_Adrenal_Adrenocortical adenoma | 0.0 |
| 94721_Donor 2 U - A_Mesenchymal Stem Cells | 0.0 | 72410_Kidney_HRCE | 9.3 |
| 94722_Donor 2 U - Cells B_Mesenchymal Stem Cells | 0.0 | 72411_Kidney_HRE | 0.0 |
| 94723_Donor 2 U - C_Mesenchymal Stem Cells | 0.0 | 73139_Uterus_Uterine smooth muscle cells | 0.7 |

**Table HG. Panel 5D**

| **Column A - Rel. Exp.(%) Ag1681, Run 169271477** | | | |
|---|---|---|---|
| **Tissue Name** | **A** | **Tissue Name** | **A** |
| 97457_Patient-02go_adipose | 0.2 | 94709_Donor 2 AM - A_adipose | 0.0 |
| 97476_Patient-07sk_skeletal muscle | 0.0 94710 | Donor 2 AM - B_adipose | 0.0 |
| 97477 _Patient-07ut_uterus | 0.0 | 94711_Donor 2 AM-C_adipose | 0.0 |
| 97478_Patient-07pl_placenta | 0.0 | 94712_Donor 2 AD - A_adipose | 0.0 |
| 97481_Patient-08sk_skeietal muscle | 0.0 | 94713_Donor 2 AD - B_adipose | 0.0 |
| 97482_Patient-08ut_uterus | 0.0 | 94714_Donor 2 AD - C_adipose | 0.0 |
| 97483_Patient-08pl_placenta | 0.2 | 94742_Donor 3 U - A_Mesenchymal Stem Cells | 0.0 |
| 97486_Patient-09sk_skeletal muscle | 0.0 | 94743_Donor 3 U - B_Mesenchymal Stem Cells | 0.0 |
| 97487_Patient-09ut_uterus | 0.1 | 94730_Donor 3 AM - A_adipose | 0.0 |
| 97488_Patient-09pl_placenta | 0.0 | 94731_Donor 3 AM - B_adipose | 0.0 |
| 97492_Patient-10ut_uterus | 0.0 | 94732_Donor 3 AM - C_adipose | 0.0 |
| 97493_Patient-10pl-placenta | 0.0 | 94733_Donor 3 AD - A_adipose | 0.0 |
| 97495_Patient-11go_adipose | 0.0 | 94134_Donor 3 AD - B_adipose | 0.0 |
| 97496 Patient-11sk skeletal muscle | 0.0 | 94735 Donor 3 AD - C_adipose | 0.0 |
| 97497_Patient-11ut_uterus | 0.0 | 77138_Liver_HepG2untreated | 28.3 |
| 97498_Patient-11pl-placenta | 0.0 | 73556_Heart_Cardiac stromal cells (primary) | 0.0 |
| 97500_Patien-12go_adipose | 0.3 | 81735_Small Intestine | 12.3 |
| 97501_Patient-12sk_skeletal muscle | 0.0 | 72409_Kidney_Proximal Convoluted Tubule | 4.3 |
| 97502_Patient-12ut_uterus | 0.2 | 82685_Small intestine_Duodenum | **100.0** |
| 97503_Patient-12pl_placenta | 0.0 | 90650_Adrenal_Adrenocortical adenoma | 0.0 |
| 94721 Donor 2U - A_Mesenchymal Stem Cells | 0.0 | 72410_Kidney_HRCE | 3.6 |
| 94722_Donor 2 U - B_Mesenchymal Stem Cells | 0.0 72411 | 72411_Kidney_HRE | 0.1 |
| 94723_Donor 2 U - C_Mesenchymal Stem Cells | 0.0 | 73139_Uterus_Uterine smooth muscle cells | 0.0 |

**Ceneral_screening_panel_v1.4 Summary:** Ag1681 The CG56151-01 gene, a glucose transporter type 2 homolog, is predominantly expressed in liver. GLUT2 facilitates the transport of glucose into the liver. This gene is also expressed in brain, pancreas, and testis. This is consistent with immunocytochemistry data that shows that the Glut2 gene is expressed in insulin producing beta cells in the pancreas and aids in regulation of insulin secretion. Since the liver is responsible for gluconeogenesis, enhancing glucose uptake through GLUT2 may produce a negative feedback loop that would decrease hepatic glucose production. This could result in a lowering of blood glucose, a major therapeutic goal for the treatment of Type II (non-insulin dependent) diabetes. Thus, enhancing the function of the protein encoded by the CG56151-01 gene with an agonist antibody therapeutic could restore balance to blood glucose levels in patients with Type II diabetes.

In addition, this gene is expressed at higher levels in fetal liver and lung (CTs=29) than in the adult sources of these tissues. Thus, expression of this gene could be used to differentiate disorders or predisposition to disorders between the two sources of these tissues.

### References:

### Waeber G, et al. Mol Cell Endocrinol 1995 Oct 30;114(1-2):205-15. (PMID: 8674846)

**Human Metabolic Summary:** Ag1681 Highest expression is seen in normal liver (CT=22)samples, with lower expression seen in liver samples from diabetic patients.

**Panel 1.3D Summary:** Ag1681 Expression of the CG56151-01 gene is restricted to liver derived tissue, an important metabolic tissue, in this panel (CTs=27). This liver specific expression is consistent with expression in other panels and with published data (see reference below.) Thus, expression of this gene could be used as a marker for liver tissue. This gene encodes a glut2 homolog. Please see General_screening_panel_v1.4 for disscussion of this gene in metabolic disease.

### References:

### Rencurel F, et al. Biochem J 1996 Mar 15;314 (Pt 3):903-9. (PMID: 8615787)

**Panel 2D Summary:** Ag1681 The expression of the CG56151-01 gene appears to be highest in a sample of normal liver tissue adjacent to a colon cancer metastasis (CT=24.6). In addition, there is substantial expression in both normal and malignant liver tissue. This restricted pattern of expression in liver derived tissue is consistent with expression in the previous panels. Moreover, therapeutic modulation ofthis gene, through the use of small molecule drugs, protein therapeutics or antibodies might be benefical in the treatment of liver cancer.

**Panel 5 Islet Summary:** Ag1681 Moderate expression is seen in samples derived from small intestine and a liver cell line.

**Panel 5D Summary:** Ag1681 The expression pattern of the CG56151-01 gene, a Glut2 homolog, is limited to a liver cell line (HepG2) and small intestines. The presence of this isoform in the intestines may indicate an important role in glucose uptake from the digestive tract. Please refer to panel 1.4 for a further discussion of this gene in metabolic disease.

### I. CG56155-02: PLASMA KALLIKREIN PRECURSOR

Expression of gene CG56155-02 was assessed using the primer-probe set Ag1688, described in Table IA. Results of the RTQ-PCR runs are shown in Tables IB, IC, ID, IE, IF, IG and IH.

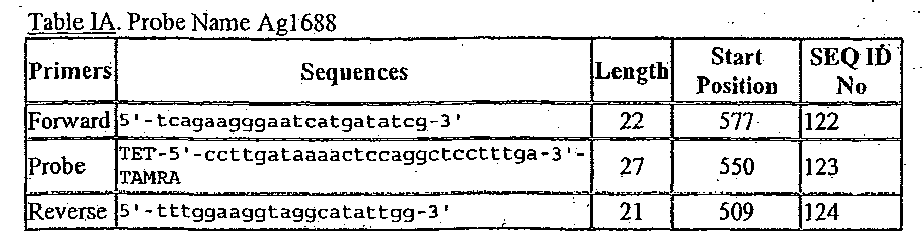

**Table IB. AI_comprehensive panel_v1.0**

| **Column A - Rel. Exp.(%) Ag1688, Run 248429492** | | | |
|---|---|---|---|
| **Tissue Name** | **A** | **Tissue Name** | **A** |
| 110967 COPD-F | 53.6 | 112427 Match Control Psoriasis-F | 77.4 |
| 110980 COPD-F | 14.2 | 112418 Psoriasis-M | 12.7 |
| 110968 COPD-M | 48.3 | 112723 Match Control Psoriasis-M | 0.0 |
| 110977 COPD-M | 53.6 | 112419 Psoriasis-M | **100.0** |
| 110989 Emphysema-F | 61.6 | 112424 Match Control Psoriasis-M | 35.6 |
| 110992 Emphysema-F | 21.6 | 112420 Psoriasis-M | 87.7 |
| 110993 Emphysema-F | 23.8 | 112425 Match Control Psoriasis-M | 29.1 |
| 110994 Emphysema-F | 20.7 | 104689 (MF) OA Bone-Backus | 50.0 |
| 110995 Emphysema-F | 55.1 | 104690 (MF) Adj "Normal" Bone-Backus | 34.9 |
| 110996 Emphysema-F | 17.8 | 104691 (MF) OA Synovium-Backus | 25.5 |
| 110997 Asthma-M | 25.2 | 104692 (BA) OA Cartilage-Backus | 37.6 |
| 111001 Asthma-F | 23.0 | 104694 (BA) OA Bone-Backus | 8.4 |
| 111002 Asthma-F | 22.1 | 104695 (BA) Adj "Normal" Bone-Backus | 34.4 |
| 111003 Atopic Asthma-F | 15.5 | 104696 (BA) OA Synovium-Backus | 6.9 |
| 111004 Atopic Asthma-F | 19.9 | 104700 (SS) OA Bone-Backus | 22.8 |
| 111005 Atopic Asthma-F | 23.8 | 104701 (SS) Adj "Normal" Bone-Backus | 42.3 |
| 111006 Atopic Asthma-F | 6.0 | 104702 (SS) OA Synovium-Backus | 29.5 |
| 111417 Allergy-M | 4.6 | 117093 OA Cartilage Rep7 | 10.6 |
| 112347 Allergy-M . | 0.0 | 112672 OA Bone5 | 94.0 |
| 112349 Normal Lung-F | 0.0 | 112673 OA Synovium5 | 43.2 |
| 112357 Normal Lung-F | 38.7 | 112674 OA Synovial Fluid cells5 | 58.6 |
| 112354 Normal Lung-M | 24.0 | 117100 OA Cartilage Rep14 | 0.0 |
| 112374 Crohns-F | 10.4 | 112756 OA Bone9 | 2.6 |
| 112389 Match Control Crohns-F | 7.4 | 112757 OA Synovium9 | 8.0 |
| 112375 Crohns-F | 4.6 | 112758 OA Synovial Fluid Cells9 | 22.1 |
| 112732 Match Control Crohns-F | 25.0 | 117125 RA Cartilage Rep2 | 22.1 |
| 112725 Crohns-M | 11.3 | 13492 Bone2 RA | 10.0 |
| 112387 Match Control Crohns-M | 1.0 | 113493 Synovium2 RA | 11.0 |
| 112378 Crohns-M | 0.0 | 113494 Syn Fluid Cells RA | 31.6 |
| 112390 Match Control Crohns-M | 44.1 | 113499 Cartilage4 RA | 47.6 |
| 112726 Crohns-M | 19.5 | 113500 Bone4 RA | 37.9 |
| 112731 Match Control Crohns-M | 58.2 | 113501 Synovium4 RA | 55.5 |
| 112380 Ulcer Col-F | 3.2 | 113502 Syn Fluid Cells4 RA | 10.0 |
| 112734 Match Control Ulcer Col-F | 56.6 | 113495 Cartilage3 RA | 20.7 |
| 112384 Ulcer Col-F | 10.1 | 113496 Bone3 RA | 16.2 |
| 112737 Match Control Ulcer Col-F | 21.6 | 113497 Synovium3 RA | 11.5 |
| 112386 Ulcer Col-F | 0.0 | 113498 Syn Fluid Cells3 RA | 25.3 |
| 112738 Match Control Ulcer Col-F | 9.3 | 117106 Normal Cartilage Rep20 | 0.0 |
| 112381 Ulcer Col-M | 0.0 | 113663 Bone3 Normal | 0.9 |
| 112735 Match Control Ulcer Col-M | 41.8 | 113664 Synovium3 Normal | 0.0 |
| 112382 Ulcer Col-M | 3.8 | 113665 Syn Fluid Cells3 Normal | 1.1 |
| 112394 M Match Control Ulcer Col- | 5.2 | 117107 Normal Cartilage Rep22 | 2.7 |
| 112383 Ulcer Col-M | 31.6 | 113667 Bone4 Normal | 8.1 |
| 112736 Match Control Ulcer Col-M | 12.9 | 113668 Synovium4 Normal | 5.8 |
| 112423 Psoriasis-F | 9.2 | 113669 Syn Fluid Cells4 Normal | 5.3 |

**Table IC. CNS neurodegeneration v1.0**

| **Column A - Rel. Exp.(%) Ag1688, Run 269217573** | | | |
|---|---|---|---|
| **Tissue Name** | **A** | **Tissue Name** | **A** |
| AD 1 Hippo | 24.5 | Control (Path) 3 Temporal Ctx | 9.7 |
| AD 2 Hippo | 34.4 | Control (Path) 4 Temporal Ctx | 41.8 |
| AD 3 Hippo | 17.9 | AD 1 Occipital Ctx | 42.3 |
| AD 4 Hippo | 18.0 | AD 2 Occipital Ctx (Missing) | 0.0 |
| AD 5 hippo | 94.6 | AD 3 Occipital Ctx | 7.1 |
| AD 6 Hippo | 34.9 | AD 4 Occipital Ctx | 26.4 |
| Control 2 Hippo | 35.4 | AD 5 Occipital Ctx | 9.9 |
| Control 4 Hippo | 50.7 | AD 6 Occipital Ctx | 27.2 |
| Control (Path) 3 Hippo | 9.3 | Control 1 Occipital Ctx | 6.3 |
| AD 1 Temporal Ctx | 31.9 | Control 2 Occipital Ctx | 49.7 |
| AD 2 Temporal Ctx | 31.4 | Control 3 Occipital Ctx | 39.2 |
| AD 3 Temporal Ctx | 20.4 | Control 4 Occipital Ctx | 26.6 |
| AD 4 Temporal Ctx | 29.5 | Control (Path) 1 Occipital Ctx | 47.3 |
| AD 5 Inf Temporal Ctx | **100.0** | Control (Path) 2 Occipital Ctx | 21.3 |
| AD 5 SupTemporal Ctx | 92.0 | Control (Path) 3 Occipital Ctx | 3.5 |
| AD 6 Inf Temporal Ctx | 43.8 | Control (Path) 4 Occipital Ctx | 17.8 |
| AD 6 Sup Temporal Ctx | 69.7 | Control I Parietal Ctx | 19.5 |
| Control 1 Temporal Ctx | 16.5 | Control 2 Parietal Ctx | 85.3 |
| Control 2 Temporal Ctx | 34.9 | Control 3 Parietal Ctx | 15.5 |
| Control 3 Temporal Ctx | 32.3 | Control (Path) 1 Parietal Ctx | 44.4 |
| Control 4 Temporal Ctx | 35.4 | Control (Path) 2 Parietal Ctx | 52.9 |
| Control (Path) 1 Temporal Ctx | 46.0 | Control (Path) 3 Parietal Ctx | 9.7 |
| Control (Path) 2 Temporal Ctx | 45.7 | Control (Path) 4 Parietal Ctx | 52.1 |

**Table ID. Panel 1.3D**

| **Column A-Rel. Exp.(%) Ag1688, Run 147249266** | | | |
|---|---|---|---|
| **Tissue Name** | **A** | **Tissue Name** | **A** |
| Liver adenocarcinoma | 0.0 | Kidney (fetal) | 9.2 |
| Pancreas | 6.7 | Renal ca. 786-0 | 0.0 |
| Pancreatic ca. CAPAN 2 | 0.2 | Renal ca. A498 | 1.7 |
| Adrenal gland | 1.8 | Renal ca. RXF 393 | 0.0 |
| Thyroid | 3.8 | Renal ca. ACHN | 0.0 |
| Salivary gland | 1.5 | Renal ca. UO-31 | 0.0 |
| Pituitary gland | 6.1 | Renal ca. TK-10 | 0.0 |
| Brain (fetal) | 0.5 | Liver | **100.0** |
| Brain (whole) | 3.6 | Liver (fetal) | 99.3 |
| Brain (amygdala) | 3.3 | Liver ca. (hepatoblast) HepG2 | 0.0 |
| Brain (cerebellum) | 0.4 | Lung | 1.3 |
| Brain (hippocampus) | 6.2 | Lung (fetal) | 1.8 |
| Brain (substantia nigra) | 1.0 | Lung ca. (small cell) LX-1 | 0.0 |
| Brain (thalamus) | 2.1 | Lung ca. (small cell) NCI-H69 | 0.0 |
| Cerebral Cortex | 6.3 | Lung ca. (s.cell var.) SHP-77 | 0.8 |
| Spinal cord | 3.1 | Lung ca. (large cell)NCI-H460 | 0.0 |
| glio/astro U87-MG | 0.0 | Lung ca. (non-sm. cell) A549 | 0.2 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 0.2 | Lung ca_ (non-s.cl).NCI-H522 | 0.0 |
| astrocytoma SF-539 | 0.0 | Lung ca. (squam.) SW 900 | 0.2 |
| astrocytoma SNB-75 | 0.1 | Lung ca. (squam.) NCI-H596 | 0.0 |
| glioma SNB-19 | 0.2 | Mammary gland | 2.9 |
| glioma U251 | 1.2 | Breast ca.* (pl.ef) MCF-7 | 0.0 |
| glioma SF-295 | 0.0 | Breast ca.* (pl.ef) MDA-MB-231 | 0.0 |
| Heart (fetal) | 0.2 | Breast ca.* (pl.ef) T47D | 0.0 |
| Heart | 1.6 | Breast ca. BT-549 | 0.0 |
| Skeletal muscle (fetal) | 0.7 | Breast ca. MDA-N | 0.0 |
| Skeletal muscle | 1.2 | Ovary | 0.0 |
| Bone marrow | 0.5 | Ovarian ca. OVCAR-3 | 0.2 |
| Thymus | 3.2 | Ovarian ca. OVCAR-4 | 0.0 |
| Spleen | 1.0 | Ovarian ca. OVCAR-5 | 0.3 |
| Lymph node | 2.9 | Ovarian ca. OVCAR-8 | 0.0 |
| Colorectal | 0.8 | Ovarian ca. IGROV-1 | 0.0 |
| Stomach | 3.3 | Ovarian ca.*(ascites) SK-OV-3 | 1.0 |
| Small intestine | 6.2 | Uterus | 1.4 |
| Colon ca. SW480 | 0.0 | Placenta | 0.4 |
| Colon ca.* SW620(SW480 met). | 0.0 | Prostate | 1.0 |
| Colon ca. HT29 | 0.0 | Prostate ca.* (bone met)PC-3 | 0.0 |
| Colon ca. HCT-116 | 0.0 | Testis | 6.1 |
| Colon ca. CaCo-2 | 0.2 | Melanoma Hs688(A).T | 0.4 |
| Colon ca. tissue(ODO3866) | 0.0 | Melanoma* (met) Hs688(B).T | 0.9 |
| Colon ca. HCC-2998 | 0.2 | Melanoma UACC-62 | 0.0 |
| Gastric ca.* (liver met) NCI-N87 | 4.4 | Melanoma M14 | 0.0 |
| Bladder | 3.1 | Melanoma LOX IMVI | 0.0 |
| Trachea | 3.0 | Melanoma* (met) SK-MEL-5 | 0.0 |
| Kidney | 6.8 | Adipose | 0.5 |

**Table IE. Panel 2D**

| **Column A - Rel. Exp.(%)Ag1688, Run 162646059** | | | |
|---|---|---|---|
| **Tissue Name** | **A** | **Tissue Name** | **A** |
| Normal Colon | 1.7 | Kidney Margin 8120608 | 0.7 |
| CC Well to Mod Diff (ODO3866) | 0.0 | Kidney Cancer 8120613 | 0.0 |
| CC Margin (ODO3866) | 0.2 | Kidney Margin 8120614 | 0.5 |
| CC Gr.2 rectosigmoid (ODO3868) | 0.2 | Kidney Cancer 9010320 | 0.2 |
| CC Margin (ODO3868) | 0.1 | Kidney Margin 9010321 | 1.0 |
| CC Mod Diff (ODO3920) | 0.1 | Normal Uterus | 0.2 |
| CC Margin (ODO3920) | 0.9 | Uterus Cancer 064011 | 0.8 |
| CC Gr.2 ascend colon (ODO3921) | 0.1 | Normal Thyroid | 0.9 |
| CC Margin (ODO3921) | 0.1 | Thyroid Cancer 064010 | 0.2 |
| CC from Partial Hepatectomy (ODO4309) Mets | 4.7 | Thyroid Cancer A302152 | 0.5 |
| Liver Margin (ODO4309) | 100.0 | Thyroid Margin A302153 | 1.0 |
| Colon mets to lung (OD04451-01) | 0.1 | Normal Breast | 0.3 |
| Lung Margin (OD04451-02) | 0.1 | Breast Cancer (OD04566) | 0.1 |
| Normal Prostate 6546-1 | 2.1 | Breast Cancer (OD04590-01) | 0.1 |
| Prostate Cancer (OD04410) | 0.6 | Breast Cancer Mets (OD04590-03) | 0.4 |
| Prostate Margin (OD04410) Prostate Margin (OD04410) | 0.5 | Breast Cancer Metastasis (OD04655-05) | 0.9 |
| Prostate Cancer (OD04720-01) | 1.1 | Breast Cancer 064006 | 0.6 |
| Prostate Margin (OD04720-02) | 1.6 | Breast Cancer 1024 | 1.2 |
| Normal Lung 061010 | 2.0 | Breast Cancer 9100266 | 0.1 |
| Lung Met to Muscle (ODO4286) | 0.0 | Breast Margin 9100265 | 0.1 |
| Muscle Margin (ODO4286) | 0.2 | Breast Cancer A209073 | 0.3 |
| Lung Malignant Cancer (OD03126) | 0.1 | Breast Margin A209073 | 0.3 |
| Lung Margin (OD03126) | 0.5 | Normal Liver | 69.7 |
| Lung Cancer (OD04404) | 0.1 | Liver Cancer 064003 | 13.7 |
| Lung Margin (OD04404) | 0.2 | Liver Cancer 1025 | 18.0 |
| Lung Cancer (OD04565) | 0.0 | Liver Cancer 1026 | 1.2 |
| Lung Margin (OD04565) | 0.1 | Liver Cancer 6004-T | 22.2 |
| Lung Cancer (OD04237-01) | 0.1 | Liver Tissue 6004-N | 1.0 |
| Lung Margin (OD04237-02) | 0.4 | Liver Cancer 6005-T | 1.9 |
| Ocular Mel Met to Liver (ODO4310) | 0.1 | Liver Tissue 6005-N | 4.2 |
| Liver Margin (ODO4310) | 77.4 | Normal Bladder | 2.7 |
| Melanoma Mets to Lung (OD04321) | 0.0 | Bladder Cancer 1023 | 0.0 |
| Lung Margin (OD04321) | 0.1 | Bladder Cancer A302173 | 0.2 |
| Normal Kidney | 12.9 | Bladder Cancer (OD04718-01) | 0.1 |
| Kidney Ca, Nuclear grade 2 (OD04338) | 3.8 | Bladder Normal Adjacent (OD04718-03) | 0.5 |
| Kidney Margin (OD04338) | 1.6 | Normal Ovary | 0.0 |
| Kidney Ca Nuclear grade 1/2 (OD04339) | 2.8 | Ovarian Cancer 064008 | 0.1 |
| Kidney Margin (OD04339) | 9.3 | Ovarian Cancer (OD04768-07) | 0.2 |
| Kidney Ca, Clear cell type (OD04340) | 1.4 | Ovary Margin (OD04768-08) | 0.1 |
| Kidney Margin (OD04340) | 4.1 | Normal Stomach | 0.3 |
| Kidney Ca, Nuclear grade 3 (OD04348) | 0.1 | Gastric Cancer 9060358 | 0.1 |
| Kidney Margin (OD04348) | 3.8 | Stomach Margin 9060359 | 0.0 |
| Kidney Cancer (OD04622-01) | 0.2 | Gastric Cancer 9060395 | 0.2 |
| Kidney Margin (OD04622-03) | 0.7 | Stomach Margin 9060394 | 0.3 |
| Kidney Cancer (OD04450-01) | 0.2 | Gastric Cancer 9060397 | 0.3 |
| Kidney Margin (OD04450-03) | 2.6 | Stomach Margin 9060396 | 0.0 |
| Kidney Cancer 8120607 | 0.0 | Gastric Cancer 064005 | 1.1 |

**Table IF. Panel 4.1D**

| **Column A - Rel. Exp.(%) Ag1688, Run 248389308** | | | |
|---|---|---|---|
| **Tissue Name** | **A** | **Tissue Name** | **A** |
| Secondary Th1 act | 1.6 | HUVEC IL-1beta | 0.0 |
| Secondary Th2 act | 1.7 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 1.3 | Bronchial epithelium TNFalpha + IL1beta | 0.0 |
| Primary Th2 rest | 1.3 | Small airway epithelium none | 0.0 |
| Primary Tr1 rest | 1.6 | Small airway epithelium TNFalpha + IL-1beta | 0.0 |
| CD45RA CD4 lymphocyte act | 3.5 | Coronery artery SMC rest | 0.0 |
| CD45RO CD4 lymphocyte act | 4.2 | Coronery artery SMC TNFalpha + IL-1beta | 0.0 |
| CD8 lymphocyte act | 3.2 | Astrocytes rest | 0.0 |
| Secondary CD8 lymphocyte rest | 1.8 | Astrocytes TNFalpha + IL-1beta | 2.4 |
| Secondary CD8 lymphocyte act | 0.0 | KU-812 (Basophil) rest | 1.8 |
| CD4 lymphocyte none | 3.8 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 6.2 | Liver cirrhosis | **100.0** |
| LAK cells IL-2+IL-12 | 0.0 | NCI-H292 none | 0.0 |
| LAK cells IL-2+IFN gamma | 1.7 | NCI-H292 IL-4 | 1.5 |
| LAK cells IL-2+ IL-18 | 3.4 | NCI-H292 IL-9 | 1.9 |
| LAK cells PMA/ionomycin | 0.0 | NCI-H292 IL-13 | 0.0 |
| NK Cells IL-2 rest | 22.1 | NCI-H292 IFN gamma | 0.0 |
| Two Way MLR 3 day | 3.3 | HPAEC none | 0.0 |
| Two Way MLR 5 day | 1.9 | HPAEC TNF alpha + IL-1 beta | 0.0 |
| Two Way MLR 7 day | 1.7 | Lung fibroblast none | 2.6 |
| PBMC rest | 1.5 | Lung fibroblast TNF alpha + IL-1 beta | 10.4 |
| PBMC PWM | 5.1 | Lung fibroblast IL-4 | 1.8 |
| PBMC PHA-L | 0.7 | Lung fibroblast IL-9 | 12.3 |
| Ramos (B cell) none | 0.0 | Lung fibroblast IL-13 | 0.0 |
| Ramos (B cell) ionomycin | 0.0 | Lung fibroblast IFN gamma | 3.1 |
| B lymphocytes PWM | 2.8 | Dermal fibroblast CCD1070 rest | 0.0 |
| B lymphocytes CD40L and IL-4 | 21.5 | Dermal fibroblast CCD1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 6.8 |
| Dendritic cells none | 2.0 | Dermal fibroblast IL-4 | 5.8 |
| Dendritic cells LPS | 0.0 | Dermal Fibroblasts rest | 0.0 |
| Dendritic cells anti-CD40 | 4.9 | Neutrophils TNFa+LPS | 0.0 |
| Monocytes rest | 0.0 | Neutrophils rest | 0.0 |
| Monocytes LPS | 0.0 | Colon | 0.0 |
| Macrophages rest | 0.0 | Lung | 1.2 |
| Macrophages LPS | 0.0 | Thymus | 0.0 |
| HUVEC none | 0.0 | Kidney | 82.9 |
| HUVEC starved | 0.0 | | |

**Table IG. Panel 5 Islet**

| **Column A - Rel. Exp.(%) Ag1688, Run 226587524** | | | |
|---|---|---|---|
| **Tissue Name** | **A** | **Tissue Name** | **A** |
| 97457_Patient-02go_adipose | 41.2 | 94709_Donor 2 AM - A_adipose | 0.0 |
| 97476 Patient-07sk skeletal muscle | 9.9 | 94710-Donor 2 AM - B_adipose | 0.0 |
| 97477_Patient-07ut_uterus | 8.1 | 94711_Donor 2 AM - C_adipose | 0.0 |
| 97478_Patient-07pl_placenta | 0.0 | 94712_Donor 2 AD - A_adipose | 11.4 |
| 99167_Bayer Patient 1 | 84.7 | 94713 Donor 2 AD - B_adipose | 0.0 |
| 97482_Patient-08ut_uterus | 2.4 | 94714_Donor 2 AD - C_adipose | 29.1 |
| 97483_Patient-08pl_placenta | 0.0 | 94742 Donor 3 U - A_Mesenchymal Stem Cells | 19.2 |
| 97486_Patient-09sk_skeletal muscle | 8.0 | 94743_Donor 3 U - B_Mesenchymal Stem Cells | 0.0 |
| 97487_Patient-09ut_uterus | 9.6 | 94730_Donor 3 AM - A_adipose | 15.0 |
| 97488_Patient-09pl_placenta | 0.0 | 94731_Donor 3 AM - B_adipose | 37.9 |
| 97492_Patient-10ut_uterus | 0.0 | 94.732_Donor 3 AM - C_adipose | 0.0 |
| 97493_Patient-10pl_placenta | 0.0 | 94733_Donor 3 AD - A_adipose | 39.2 |
| 97495_Patient-11go_adipose | 0.0 | 94734_Donor 3 AD - B_adipose | 11.4 |
| 97496 Patient-11sk_skeletal muscle | 52.9 | 94735_Donor 3 AD - C_adipose | 34.4 |
| 97497_Patient-11ut_uterus | 35.8 | 77138_Liver_HepG2untreated | 8.4 |
| 97498 _Patient-11pl_placenta | 10.5 | 73556_Heart_Cardiac stromal cells (primary) | 0.0 |
| 97500_Patient-12go_adipose | 0.0 | 81735_Small Intestine | **100.0** |
| 97501_Patient-12sk_skeletal muscle | 35.4 | 72409_Kidney_Proximal Convoluted Tubule | 9.9 |
| 97502_Patient-12ut_uterus | 20.7 | 82685_Small intestine_Duodenum | 70.2 |
| 97503_Patient-12pl_placenta | 0.0 | 90650_Adrenal_Adrenocortical adenoma | 25.5 |
| 94721_Donor 2 U - A_Mesenchymal Stem Cells | 0.0 | 72410_Kidney_HRCE | 10.4 |
| 94722 Donor 2 U - B_Mesenchymal Stem Cells | 0.0 | 72411_Kidney_HRE | 7.2 |
| 94723_Donor 2 U - C_Mesenchymal Stem Cells | 0.0 | 73139_Uterus_Uterine smooth muscle cells | 0.0 |

**Table IH. general oncology screening panel v 2.4**

| **Column A - Rel. Exp.(%) Ag1688, Run 260552690** | | | |
|---|---|---|---|
| **Tissue Name** | **A** | **Tissue Name** | **A** |
| Colon cancer 1 | 1.8 | Bladder cancer NAT 2 | 0.1 |
| Colon cancer NAT 1 | 1.0 | Bladder cancer NAT 3 | 0.0 |
| Colon cancer 2 | 0.4 | Bladder cancer NAT 4 | 1.1 |
| Colon cancer NAT 2 | 1.2 | Prostate adenocarcinoma 1 | 3.7 |
| Colon cancer 3 | 0.8 | Prostate adenocarcinoma 2 | 0.2 |
| Colon cancer NAT 3 | 2.5 | Prostate adenocarcinoma 3 | 1.2 |
| Colon malignant cancer 4 | 2.1 | Prostate adenocarcinoma 4 | 3.5 |
| Colon normal adjacent tissue 4 | 0.2 | Prostate cancer NAT 5 | 0.6 |
| Lung cancer 1 | 0.2 | Prostate adenocarcinoma 6 | 0.2 |
| Lung NAT 1 | 0.2 | Prostate adenocarcinoma 7 | 0.0 |
| Lung cancer 2 | 1.0 | Prostate adenocarcinoma 8 | 0.0 |
| Lung NAT 2 | 0.8 | Prostate adenocarcinoma 9 | 0.0 |
| Squamous cell carcinoma 3 | 0.5 | Prostate cancer NAT 10 | 0.1 |
| Lung NAT 3 | 0.0 | Kidney cancer 1 | 7.7 |
| metastatic melanoma 1 | 1.1 | KidneyNAT 1 | 5.7 |
| Melanoma 2 | 0.1 | Kidney cancer 2 | 40.1 |
| Melanoma 3 | 0.0 | Kidney NAT 2 | 23.8 |
| metastatic melanoma 4 | 2.0 | Kidney cancer 3 | 100.0 |
| metastatic melanoma 5 | 3.0 | Kidney NAT 3 | 5.6 |
| Bladder cancer 1 | 0.6 | Kidney cancer 4 | 2.0 |
| Bladder cancer NAT 1 | 0.0 | Kidney NAT 4 | 4.2 |
| Bladder cancer 2 | 0.3 | | |

**Al_comprebensive panel_v1.0 Summary:** Ag1688 Highest expression of this gene is detected in psoriasis sample (CT=31.9). Moderate to low levels of expression of this gene is also seen in samples derived from osteooarthitis/rheumatoid arthritis bone, cartilage, synovium and synovial fluid samples, from normal lung, COPD lung, emphysema, atopic asthma, asthma, Crohn's disease (normal matched control and diseased), ulcerative colitis(normal matched control and diseased), and psoriasis (normal matched control and diseased). Therefore, therapeutic modulation of this gene product may ameliorate symptoms/conditions associated with autoimmune and inflammatory disorders including psoriasis, asthma, inflammatory bowel disease, rheumatoid arthritis and osteoarthritis.

**CNS neurodegeneration v1.0 Summary: Data** obtained from RTQPCR assays performed for this panel was analyized using multivariate analysis (ANOVA). The multivariate results obtained analyzing Ag1688 confirms the expression of this gene at low levels in the brains of an independent group of individuals. However, no differential expression of this gene was detected between Alzheimer's diseased postmortem brains and those of non-demented controls in this experiment. Please see Panel 1.3D for a discussion of the potential role of this gene in treatment of central nervous system disorders.

**Panel 1.3D Summary:** Ag1688 Expression of this gene, a plasma kallikrein, is significantly higher in liver (CTs=28) than in any other sample on this panel. Thus, expression of this gene could be used as a marker of liver tissue. In addition, low levels of expression of this gene is also detected in tissues with metabolic/endocrine functions including pancreas, adrenal gland, thyroid, pituitary gland, skeletal muscle, heart, and the gastrointestinal tract. Plasma prekallikrein is a glycoprotein that participates in the surface-dependent activation of blood coagulation, fibrinolysis, kinin generation and inflammation. It is synthesized in the liver and secreted into the blood as a single polypeptide chain. It is converted to plasma kallikrein by factor XIIa. Recently, plasma kallikrein has been implicated in adipose differentiation by remodeling of the fibronectin-rich ECM of preadipocytes. Plg -/- mice show a reduction of fat deposit (Ref. 1, 2). At Curagen, it was found that plasma kallikrein significantly down-regulated in the liver of mice with 'lean' phenotype. Thus, based on Curagen GeneCalling data it is hypothesized that plasma kallikrein might cause disruption of adipose differentiation thus leading to obesity if over expressed and to a leaner phenotype if expression is below normal. Therefore, an antagonist to this gene product may be beneficial in the treatment of obesity.

Moderate to low levels of expression of this gene is also seen in some of the regions of central nervous system examined, including amygdala, hippocampus, substantia nigra, thalamus, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

### References:

Hoover-Plow J, et al. Biochem.Biophys.Res.Commun. (2001) 284, 389-394. (PMID: 11394891).
Selvarajan S, et al. Nature Cell Biol. (2001) 3, 267-275. (PMID: 11231576)

**Panel 2D Summary**: Ag1688 The expression of the CG56155-01 gene appears to be highest in a sample derived from a sample of normal liver tissue adjacent to a metastatic colon cancer CT=26.2). In addition, there is substantial expression in other samples of normal liver, and to a much lesser degree, malignant liver tissue. This liver specific expression is consistent with the expression seen in Panel 1.3D.

**Panel 4.1D Summary:** Ag1688 Highest expression of this gene is detected in liver cirrhosis (CT=31.8). In addition, moderate to low levels of expression of this gene in IL-2 treated NK cells, CD40L and IL-4 treated B lymphocytes and normal kidney. Therefore, therapeutic modulation of the protein encoded for by this gene may be useful in the treatment of inflammatory or autoimmune diseases, liver cirrhosis and fibrosis, lupus erythematosus and glomerulonephritis.

**Panel 5 Islet Summary:** Ag1688 Expression of the CG56155-01 gene is limited to pancreatic islets and small intestines. Please see Panel 1.3 for discussion of this gene in metabolic disease.

**general oncology screening panel_v_2.4 Summary:** Ag1688 Highest expression of this gene is detected in kidney cancer (CT=28.4). Higher expression of this gene is associated with cancer compared to normal kidney. Therefore, expression of this gene may be used as diagnostic marker for kidney cancer and therapeutic modulation of this gene or protein encoded by this gene may through the use of antibodies or small molecule drug may be useful in the treatment of kidney cancer.

### J. CG56262-01: Ca-binding transporter

Expression of gene CG56262-01 was assessed using the primer-probe sets Ag2896 and Ag2920, described in Tables JA and JB. Results of the RTQ-PCR runs are shown in Tables JC, JD, JE, JF and JG.

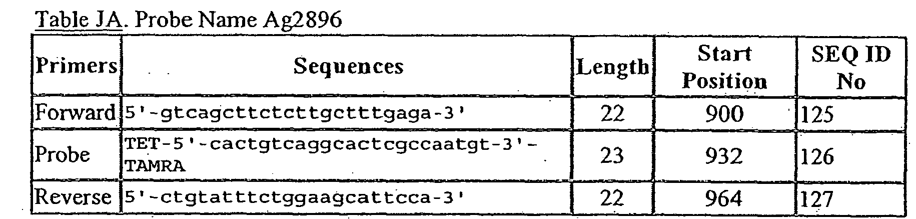

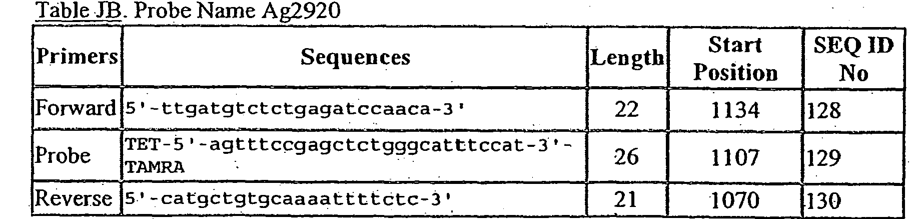

**Table JC. CNS_neurodegeneration_v1.0**

| **Column A - Rel. Exp. (%) Ag2896, Run 209734744** | | | | | |
|---|---|---|---|---|---|
| **Column B - Rel. Exp.(%) Ag2920, Run 209779301** | | | | | |
| **Tissue Name** | **A** | **B** | **Tissue Name** | **A** | **B** |
| AD 1 Hippo | 17.7 | 21.6 | Control (Path) 3 Temporal Ctx | 11.0 | 15.3 |
| AD 2 Hippo | 41.5 | 40.3 | Control (Path) 4 Temporal Ctx | 41.2 | 36.9 |
| AD 3 Hippo | 13.9 | 18.2 | AD 1 Occipital Ctx | 10.7 | 13.1 |
| AD 4 Hippo | 10.7 | 11.3 | AD 2 Occipital Ctx (Missing) | 0.0 | 0.0 |
| AD 5 Hippo | 65.1 | 54.7 | AD 3 Occipital Ctx | 8.1 | 9.2 |
| AD 6 Hippo | 62.4 | 73.2 | AD 4 Occipital Ctx | 23.5 | 25.3 |
| Control 2 Hippo | 45.4 | 51.8 | AD 5 Occipital Ctx | 45.4 | 15.7 |
| Control 4 Hippo | 15.8 | 19.8 | AD 6 Occipital Ctx | 14.7 | 44.4 |
| Control (Path) 3 Hippo | 10.6 | 12.9 | Control 1 Occipital Ctx | 6.8 | 8.4 |
| AD 1 Temporal Ctx | 17.6 | 18.9 | Control 2 Occipital Ctx | 52.1 | 57.4 |
| AD 2 Temporal Ctx | 41.5 | 41.8 | Control 3 Occipital Ctx | 14.3 | 18.8 |
| AD 3 Temporal Ctx | 10.1 | 12.9 | Control 4 Occipital Ctx | 11.0 | 12.3 |
| AD 4 Temporal Ctx | 29.9 | 27.5 | Control (Path) 1 Occipital Ctx | 80.7 | **100.0** |
| AD 5 Inf Temporal Ctx | 78.5 | 79.6 | Control (Path) 2 Occipital Ctx | 11.1 | 11.5 |
| AD 5 Sup Temporal Ctx | 47.0 | 43.5 | Control (Path) 3 Occipital Ctx | 5.3 | 7.0 |
| AD 6 Inf Temporal Ctx | 48.0 | 47.6 | Control (Path) 4 Occipital Ctx | 14.2 | 12.3 |
| AD 6 Sup Temporal Ctx | 47.3 | 55.5 | Control 1 Parietal Ctx | 13.8 | 15.4 |
| Control 1 Temporal Ctx | 14.8 | 16.6 | Control 2 Parietal Ctx | 40.6 | 40.6 |
| Control 2 Temporal Ctx | 53.6 | 66.9 | Control 3 Parietal Ctx | 2.8 | 17.8 |
| Control 3 Temporal Ctx | 24.3 | 22.8 | Control (Path) 1 Parietal Ctx | **100.0** | **100.0** |
| Control 3 Temporal Ctx | 18.0 | 15.8 | Control (Path) 2 Parietal Ctx | 25.0 | 23.2 |
| Control (Path) 1 Temporal Ctx | 86.5 | 88.3 | Control (Path) 3 Parietal Ctx | 7.1 | 9.7 |
| Control (Path) 2 Temporal Ctx | 45.1 | 50.0 | Control (Path) 4 Parietal Ctx | 46.3 | 47.6 |

**Table JD. Panel 1.3D**

| **Column A - Rel. Exp.(%) Ag2896, Run 167660338** | | | | | |
|---|---|---|---|---|---|
| **Column B - Rel. Exp.(%) Ag2920, Run 167646813** | | | | | |
| **Tissue Name** | **A** | **B** | **Tissue Name** | **A** | **B** |
| Liver adenocarcinoma | 36.6 | 40.1 | Kidney (fetal) | 23.2 | 21.6 |
| Pancreas | 4.2 | 7:4 | Renal ca. 786-0 | 15.5 | 19.6 |
| Pancreatic ca. CAPAN 2 | 10.1 | 9.3 | Renal ca. A498 | 9.5 | 9.4 |
| Adrenal gland | 3.3 | 2.8 | Renal ca. RXF 393 | 17.3 | 16.6 |
| Thyroid | 11.8 | 18.9 | Renal ca. ACHN | 10.5 | 14.5 |
| Salivary gland | 6.7 | 6.6 | Renal ca. UO-31 | 7.7 | 9.9 |
| Pituitary gland | 2.2 | 2.7 | Renal ca. TK-10 | 12.4 | 14.7 |
| Brain (fetal) | 27.0 | 27.7 | Liver | 4.3 | 3.5 |
| Brain (whole) | 812 | 74.2 | Liver (fetal) | 1.8 | 2.6 |
| Brain (amygdala) | 40.1 | 40.3 | Liver ca. (hepatoblast) HepG2 | 4.7 | 4.9 |
| Brain (cerebellum) | 30.8 | 33.0 | Lung | 5.4 | 3.2 |
| Brain (hippocampus) | 44.8 | 42.0 | Lung (fetal) | 4.8 | 4.7 |
| Brain (substantia nigra) | 23.0 | 21.5 | Lung ca. (small cell) LX-1 | 6.7 | 6.2 |
| Brain (thalamus) | 25.5 | 31.6 | Lung ca. (small cell) NCI-H69 | 0.0 | 0.1 |
| Cerebral Cortex | **100.0** | **100.0** | Lung ca.(s.cell var.)SHP-77 | 26.1 | 31.9 |
| Spinal cord | 12.6 | 12.9 | Lung ca. (large cell)NCI-H460 | 1.2 | 1.4 |
| glio/astro U87-MG | 2.2 | 2.4 | Lung ca. (non-sm. cell) A549 | 10.4 | 8.9 |
| glio/astro U-118-MG | 9.9 | 8.1 | Lung ca. (non-s.cell) NCI-H23 | 11.0 | 12.7 |
| astrocytoma SW1783 | 8.8 | 10.1 | Lung ca. (non-s.cell) HOP-62 | 4.9 | 4.7 |
| neuro*; met SK-N-AS | 4.2 | 3.3 | Lung ca. (non-s.cl) NCI-H522 | 11.4 | 11.4 |
| astrocytoma SF-539 | 5.8 | 5.4 | Lung ca. (squam.) SW 900 | 7.9 | 8.6 |
| astrocytoma SNB-75 | 10.2 | 10.5 | Lung ca. (squam.) NCI-H596 | 0.3 | 0.4 |
| glioma SNB-19 | 10.1 | 11.0 | Mammary gland | 8.3 | 8.5 |
| glioma U251 | 14.1 | 15.8 | Breast ca.* (pl.ef) MCF-7 | 7.5 | 8.1 |
| glioma SF-295 | 6.0 | 5.9 | Breast ca.* (pl.ef) MDA-MB-231 | 6.6 | 7.1 |
| Heart (fetal) | 38.7 | 40.1 | Breast ca.* (pl.ef) T47D | 16.2 | 17.0 |
| Heart | 10.7 | 9.9 | Breast ca. BT-549 | 5.8 | 5.1 |
| Skeletal muscle (fetal) | 16.0 | 11.8 | Breast ca. MDA-N | 19.5 | 22.5 |
| Skeletal muscle | 31.2 | 28.7 | Ovary | 10.4 | 10.3 |
| Bone marrow | 0.4 | 0.6 | Ovarian ca. OVCAR-3 | 11.5 | 9.2 |
| Thymus | 2.5 | 2.5 | Ovarian ca. OVCAR-4 | 35.6 | 32.5 |
| Spleen | 1.1 | 1.4 | Ovarian ca. OVCAR-5 | 31.0 | 34.2 |
| Lymph node | 2.6 | 1.7 | Ovarian ca. OVCAR-8 | 5.4 | 5.5 |
| Colorectal | 14.0 | 12.3 | Ovarian ca. IGROV-1 | 10.3 | 10.2 |
| Stomach | 4.4 | 4.0 | Ovarian ca.* (ascites) SK-OV-3 | 13.8 | 18.0 |
| Small intestine | 4.4 | 4.9 | Uterus | 6.3 | 8.4 |
| Colon ca. SW480 | 5.1 | 5.3 | Placenta | 0.0 | 0.0 |
| Colon ca.* SW620(SW480 met) | 14.9 | 18.7 | Prostate | 3.2 | 3.8 |
| Colon ca. HT29 | 6.8 | 7.2 | Prostate ca.* (bone met)PC-3 | 16.5 | 18.3 |
| Colon ca. HCT-116 | 10.7 | 10.4 | Testis | 1.4 | 1.3 |
| Colon ca. CaCo-2 | 17.0 | 21.9 | Melanoma Hs688(A).T | 2.8 | 2.6 |
| Colon ca. tissue(ODO3866) | 2.4 | 2.4 | Melanoma* (met) Hs688(B).T | 2.6 | 3.8 |
| Colon ca. HCC-2998 | 9.3 | 7.6 | Melanoma UACC-62 | 10.9 | 11.2 |
| Gastric ca.* (liver met) NCI-N87 | 5.7 | 5.5 | Melanoma M14 | 8.6 | 5.8 |
| Bladder | 5.1 | 5.1 | Melanoma LOX IMVI | 12.2 | 10.8 |
| Trachea | 1.9 | 2.8 | Melanoma* (met) SK-MEL-5 | 24.0 | 25.2 |
| Kidney | 12.4 | 17.0 | Adipose | 6.1 | 6.4 |

**Table JE. Panel 4D**

| **Column A - Rel. Exp.(%) Ag2896, Run 164401737** | | | | | |
|---|---|---|---|---|---|
| **Column B - Rel. Exp.(%) Ag2920, Run 164403312** | | | | | |
| **Tissue Name** | **A** | **B** | **Tissue Name** | **A** | **B** |
| Secondary Th1 act | 5.6 | 7.3 | HUVEC lL-1beta | 4.9 | 4.3 |
| Secondary Th2 act | 6.8 | 6.0 | HUVEC IFN gamma | 19.6 | 19.8 |
| Secondary Tr1 act | 7.4 | 7.7 | HUVEC TNF alpha + IFN gamma | 7.8 | 8.9 |
| Secondary Th1 rest | 6.4 | 6.7 | HUVEC TNF alpha + IL4 | 6.1 | 7.9 |
| Secondary Th2 rest | 7.6 | 7.1 | HUVEC IL-11 | 10.4 | 11.8 |
| Secondary Tr1 rest | 12.2 | 9.7 | Lung Microvascular EC none | 7.5 | 9.8 |
| Primary Th1 act | 12.7 | 14.5 | Lung Microvascular EC TNFalpha + IL-1beta | 5.5 | 6.1 |
| Primary Th2 act | 16.0 | 15.1 | Microvascular Dermal EC none | 13.7 | 12.6 |
| Primary Tr1 act | 26.4 | 22.1 | Microsvasular Dermal EC TNFalpha + IL-1beta | 5.7 | 6.6 |
| Primary Th1 rest | 31.6 | 33.4 | Bronchial epithelium TNFalpha + IL1 beta | 15.9 | 11.9 |
| Primary Th2 rest | 19.3 | 18.7 | Small airway epithelium none | 4.7 | 5.3 |
| Primary Tr1 rest | 14.7 | 16.5 | Small airway epithelium TNFalpha IL-1beta | 35.6 | 37.1 |
| CD45RA CD4 lymphocyte act | 6.2 | 5.1 | Coronery artery SMC rest | 7.1 | 6.7 |
| CD45RO CD4 lymphocyte act | 11.2 | 12.3 | Coronery artery SMC TNFalpha + IL-1beta | 4.6 | 5.9 |
| CD8 lymphocyte act | 11.6 | 10.8 | Astrocytes rest | 27.0 | 23.8 |
| Secondary CD8 lymphocyte rest | 8.2 | 9.9 | Astrocytes TNFalpha + IL-1beta | 30.8 | 28.1 |
| Secondary CD8 lymphocyte 3.0 3.3 act | 3.0 | 3.3 | KU-812 (Basophil) rest | 8.2 | 6.3 |
| CD4 lymphocyte none | 2.8 | 3.4 | KU-812 (Basophil) PMA/ionomycin | 22.5 | 19.9 |
| 2ry Th1/Th2/Tr1_anti-CD95 CH1 | 7.3 | 7.4 | CCD1106 (Keratinocytes) none | 11.1 | 11.7 |
| LAX cells rest | 7.1 | 6.7 | CCD1106 (Keratinocytes) TNFalpha+IL-1 beta | 6.8 | 6.3 |
| LAK cells IL-2 | 14.7 | 17.0 | Liver cirrhosis | 3.0 | 3.0 |
| LAK cells IL-2+IL-12 | 6.9 | 7.0 | Lupus kidney | 6.5 | 6.2 |
| LAK cells IL-2+IFN gamma | 12.8 | 11.0 | NCI-H292 none | 63.3 | 72.7 |
| LAK cells IL-2+ IL-18 | 6.7 | 9.0 | NCI-H292 IL-4 | 57.4 | 69.7 |
| LAK cells PMA/ionomycin | 0.9 | 0.6 | NCI-H292 IL-9 | 57.0 | 65.5 |
| NK Cells IL-2 rest | 7.2 | 6.5 | NCI-H292 IL-13 | 30.8 | 35.6 |
| Two Way MLR 3 day | 6.3 | 6.8 | NCI-H292 IFN gamma | 29.3 | 34.4 |
| Two Way MLR 5 day | 3.5 | 3.0 | HPAEC none | 10.8 | 11.7 |
| Two Way MLR 7 day | 4.2 | 4.5 | HPAEC TNF alpha + IL-1 beta | 6.9 | 6.4 |
| PBMC rest | 2.0 | 1.6 | Lung fibroblast none | 16.8 | 17.4 |
| PBMC PWM | 27.9 | 26.2 | Lung fibroblast TNF alpha + IL-1 beta | 7.4 | 8.0 |
| PBMC PHA-L | 27.5 | 26.8 | Lung fibroblast IL-4 | 30.6 | 34.6 |
| Ramos (B cell) none | 16.8 | 16.0 | Lung fibroblast IL-9 | 24.8 | 24.1 |
| Ramos (B cell) ionomycin | **100.0** | **100.0** | Lung fibroblast IL-13 | 19.6 | 21.8 |
| B lymphocytes PWM | 36.6 | 22.8 | Lung fibroblast IFN gamma | 31.4 | 37.6 |
| B lymphocytes CD40L and IL-4 | 13.5 | 14.9 | Dermal fibroblast CCD1070 rest | 10.7 | 12.0 |
| EOL-1 dbcAMP | 14.5 | 15.5 | Dermal fibroblast CCD1070 TNF alpha | 20.6 | 21.3 |
| EOL-1 dbcAMP PMA/ionomycin | 7.1 | 6.3 | Dermal fibroblast CCD1070 IL-1 beta | 6.5 | 5.9 |
| Dendritic cells none | 0.8 | 1.5 | Dermal fibroblast IFN gamma | 10.1 | 11.3 |
| Dendritic cells LPS | 0.1 | 0.2 | Dermal fibroblast IL-4 | 23.0 | 23.2 |
| Dendritic cells anti-CD40 | 0.9 | 0.7 | IBD Colitis 2 | 2.0 | 2.3 |
| Monocytes rest | 0.1 | 0.0 | IIBD Crohn's | 3.4 | 4.8 |
| Monocytes LPS | 0.2 | 0.0 | Colon | 41.5 | 50.7 |
| Macrophages rest | 4.0 | 3.4 | Lung | 15.8 | 17.2 |
| Macrophages LPS | 0.5 | 0.4 | Thymus | 57.8 | 55.5 |
| HUVEC none | 12.2 | 12.8 | Kidney | 5.0 | 8.5 |
| HUVEC starved | 21.6 | 20.4 | | | |

**Table JF. Panel 5 Islet**

| **Column A - Rel. Exp.(%) Ag2896, Run 268363565** | | | |
|---|---|---|---|
| **Tissue Name** | **A** | **Tissue Name** | **A** |
| 97457_Patient-02go_adipose | 19.6 | 94709_Donor 2 AM - A_adipose | 8.3 |
| 97476_Patient-07sk_skeletal muscle | 6.6 | 94710 Donor 2 AM - B_adipose | 8.1 |
| 97477_Patient-07ut_uterus | 31.6 | 94711_Donor 2 AM - C_adipose | 5.0 |
| 97478_Patient-07pl_pacenta | 2.6 | 94712_Donor 2 AD - A_adipose | 17.6 |
| 99167_Bayer Patient 1 | 32.5 | 94713_Donor 2 AD - B_adipose | 29.5 |
| 97482_Patient-08ut_uterus | 26.6 | 94714_Donor 2 AD - C_adipose | 25.7 |
| 97483_Patient-08pl_placenta | 1.2 | 94742 Donor 3 U - A_Mesenchymal Stem Cells | 5.7 |
| 97486_Patient-09sk_skeletal muscle | 8.3 | 94743_Donor 3 U - B_Mesenchymal Stem Cells | 5.1 |
| 97487_Patient-09ut_uterus | 47.6 | 94730_Donor 3 AM - A_adipose | 8.7 |
| 97488_Patient-09pl_placenta | 1.3 | 94731_Donor 3 AM - B_adipose | 4.4 |
| 97492_Patient-10ut_uterus | 28.3 | 94732_Donor 3 AM - C_adipose | 3.4 |
| 97493_Patient-10pl_placenta | 3.0 | 94733_Donor 3 AD - A_adipose | 9.9 |
| 97495_Patient-11go_adipose | 47.6 | 94734_Donor 3 AD - B_adipose | 4.4 |
| 97496 Patient-1 1sk skeletal muscle | 35.1 | 94735_Donor 3 AD-C_adipose | 4.6 |
| 97497_Patient-11ut_uterus | **100.0** | 77138_Liver_HepG2untreated | 54.7 |
| 97498_Patient-11pl_placenta | 0.4 | 73556_Heart_Cardiac stromal cells (primary) | 10.6 |
| 97500_Patient-12go_adipose | 17.3 | 81735_Small Intestine | 60.7 |
| 97501_Patient-12sk_skeletal muscle | 37.4 | 72409_Kidney_Proximal Convoluted Tubule | 24.1 |
| 97502_Patient-12ut_uterus | 76.3 | 82685_Small intestine_Duodenum | 18.8 |
| 97503_Patient-12pl_placenta | 0.9 | 90650_Adrenal_Adrenocortical adenoma | 3.8 |
| 94721_Donor 2 U - A_Mesenchymal Cells | 15.2 | 72410_Kidney_HRCE | 70.2 |
| 94722 Donor 2 U - B_Mesenchymal Stem Cells | 11.9 | 7241 Kidney_HRE | 59.5 |
| 94723_Donor 2 U- C_Mesenchymal Stem Cells | 9.7 | 73139_Uterus_Uterine smooth muscle cells | 8.7 |

**Table JG. Panel CNS_1**

| **Column A - Rel. Exp.(%) Ag2896, Run 171688452** | | | |
|---|---|---|---|
| **Tissue Name** | **A** | **Tissue Name** | **A** |
| BA4 Control | 22.1 | BA17 PSP | 22.4 |
| BA4 Control2 | 41.8 | BA17 PSP2 | 6.5 |
| BA4 Alzheimer's2 | 5.2 | Sub Nigra Control | 21.3 |
| BA4 Parkinson's | 39.2 | Sub Nigra Control2 | 18.3 |
| BA4 Parkinson's2 | 68.8 | Sub Nigra Alzheimer's2 | 7.2 |
| BA4 Huntington's | 28.1 | Sub Nigra Parkinson's2 | 27.7 |
| BA4 Huntington's2 | 13.9 | Sub Nigra Huntington's | 25.5 |
| BA4 PSP | 9.9 | Sub Nigra Huntington's2 | 13.6 |
| BA4 PSP2 | 25.5 | SubNigraPSP2 | 3.4 |
| BA4 Depression | 22.7 | Sub Nigra Depression | 6.3 |
| BA4 Depression2 | 6.7 | Sub Nigra Depression2 | 6.1 |
| BA7 Control | 34.9 | Glob Palladus Control | 18.9 |
| BA7 Contro12 | 27.7 | Glob Palladus Contro12 | 19.9 |
| BA7 Alzheimer's2 | 6.2 | Glob Palladus Alzheimer's | 7.2 |
| BA7Parkinson's | 18.3 | Glob Palladus Alzheimer's2 | 9.8 |
| BA7 Parkinson's2 | 38.2 | Glob Palladus Parkinson's | 100.0 |
| BA7 Huntington's | 51.1 | Glob Palladus Parkinson's2 | 20.9 |
| BA7 Huntington's2 | 38.7 | Glob Palladus PSP | 13.8 |
| BA7 PSP | 44.4 | Glob Palladus PSP2 | 12.4 |
| BA7 PSP2 | 18.9 | Glob Palladus Depression | 7.5 |
| BA7 Depression | 10.5 | Temp Pole Control | 20.0 |
| BA9 Control | 27.9 | Temp Pole Control2 | 66.9 |
| BA9 Control2 | 83.5 | Temp Pole Alzheimer's | 6.1 |
| BA9 Alzheimer's | 4.7 | Temp Pole Alzheimer's2 | 6.6 |
| BA9 Alzheimer's2 | 12.6 | Temp Pole Parkinson's | 34.6 |
| BA9 Parkinson's | 22.5 | Temp Pole Parkinson's2 | 24.0 |
| BA9 Parkinson's2 | 45.4 | Temp Pole Huntington's | 33.4 |
| BA9 Huntington's | 39.2 | Temp Pole PSP | 8.4 |
| BA9 Huntington's2 | 20.0 | Temp Pole PSP2 | 6.4 |
| BA9 PSP | 12.1 | Temp Pole Depression2 | 6.7 |
| BA9 PSP2 | 3.9 | Cing Gyr Control | 53.6 |
| BA9 Depression | 8.5 | Cing Gyr Control2 | 34.6 |
| BA9 Depression2 | 9.2 | Cing Gyr Alzheimer's | 15.9 |
| BA17 Control | 25.3 | Cing GyrAlzheimer's2 | 12.2 |
| BA17 Control2 | 34.6 | Cing Gyr Parkinson's | 24.5 |
| BA17 Aliheimer's2 | 4.3 | Cing Gyr Parkinson's2 | 30.8 |
| BA17 Parkinson's | 20.0 | Cing Gyr Huntington's | 48.0 |
| BA17 Parkinson's2 | 28.3 | Cing Gyr Huntington's2 | 16.4 |
| BA17 Huntington's | 24.1 | Cing Gyr PSP | 15.2 |
| BA17 Huntington's2 | 12.1 | Cing Gyr PSP2 | 6.0 |
| BA17 Depression | 8.7 | Cing Gyr Depression | 7.3 |
| BA17 Depression2 | 16.6 | Cing Gyr Depression2 | 11.7 |

**CNS_neurodegeneration_v1.0 Summary:** Data obtained from RTQPCR assays performed for this panel was analyized using multivariate analysis (ANOVA). The multivariate results obtained analyzing both Ag2896/Ag2920 indicates this gene is found to be down-regulated in the temporal cortex of Alzheimer's disease patients. Therefore, up-regulation of this gene or its gene product, or treatment with specific agonists for this receptor may be of use in treating dementia/memory loss associated with this disease and neuronal death.

**Panel 1.3D Summary:** Ag2896/Ag2920 Two experiments produce results that are in excellent agreement, with highest expression of this gene in the brain cerebral cortex (CTs=26). High expression of this gene is seen mainly in all the regions of the central nervous system examined, including amygdala, hippocampus, substantia nigra, thalamus, cerebellum, cerebral cortex, and spinal cord. This gene encodes a Ca binding transporter. Ca++ is critical for synaptic vesicle release (Kovacs I, Szarics E, Nyitrai G, Blandl T, Kardos J. Matching kinetics of synaptic vesicle recycling and enhanced neurotransmitter influx by Ca2+ in brain plasma membrane vesicles. Neurochem Int 1998 Nov;33(5):399-405). Thus, this gene would be an excellent small molecule target for diseases resulting from altered/inappropriate synaptic transmission such as epilepsy, schizophrenia, bipolar disorder, depression, and mania.

This gene also has moderate levels of expression adult and fetal heart, skeletal muscle and liver, and adipose. This gene product is homologous to a mitochondrial calcium-dependent transporter. Since intracellular calcium homeostasis is critically important for energy metabolism and signal transduction, modulation of this gene product may therefore be a therapeutic for metabolic and endocrine diseases.

**Panel 4D Summary:** Ag2896/Ag2920 Two experiments show moderate to low expression of this gene across a wide range of cells of this panel including epithelium, fibroblasts, and endothelial cells. Lower but still significant levels of expression are also seen in the key players of innate and adaptive immunity: monocytes/macrophages, T and B cells. However, the expression of this transcript is highest in the B lymphoma cell line, and NCI H292, a mucoepidermoid cell line (CTs=26.4-27). Thus, inhibition of the function of the protein encoded by this transcript with a small molecule drug, could lead to improvement of the symptoms of patients suffering from autoimmune and inflammatory diseases such as asthma, COPD, emphysema, psoriasis, inflammatory bowel disease, lupus erythematosus, or rheumatoid arthritis.

**Panel 5 Islet Summary**: Ag2896 This gene shows widespread expression in this panel with highest expression seen in uterus from a non-diabetic patient (CT=28.8). Significant expression of this gene is seen in adipose, skeletal muscle, uterus, kidney, small intestine and a liver cancer cell line, which is in agreement with expression seen in panel 1.3D. Please see panel 1.3D for further discussion on the role of this gene.

**Panel CNS_1 Summary:** Ag2896 This expression profile confirms the presence of this gene in the brain.

### K. CG56829-01: human TESTIS SPECIFIC SERINE

### KINASE-3

Expression of gene CG56829-01 was assessed using the primer-probe sets Ag1301b, Ag1415 and Ag3031, described in Tables KA, KB and KC. Results of the RTQ-PCR runs are shown in Tables KD, KE, KF and KG.

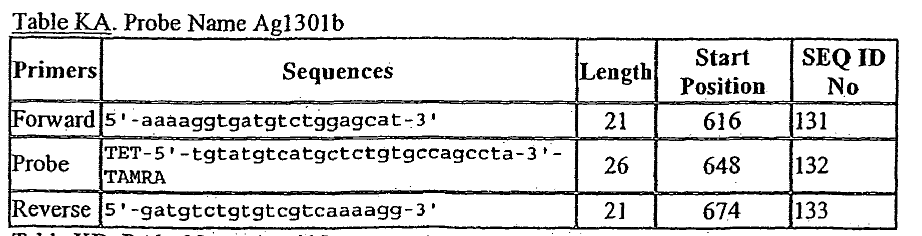

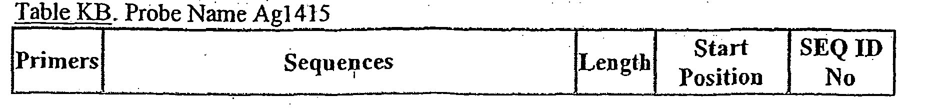

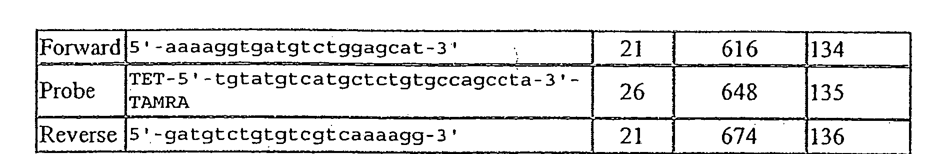

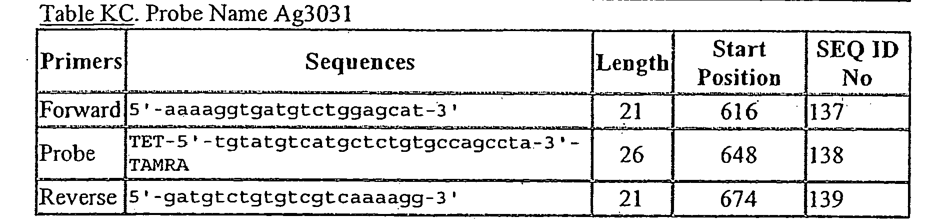

**Table KD. CNS neurodegeneration v1.0**

| **Column A - Rel. Exp.(%) Ag3031, Run 211011868** | | | | | |
|---|---|---|---|---|---|
| **Column B - Rel. Exp.(%) Ag3031, Run 225437445** | | | | | |
| **Tissue Name** | **A** | **B** | **Tissue Name** | **A** | **B** |
| AD 1 Hippo | 23.8 | 46.7 | Control (Path) 3 Temporal Ctx | 6.0 | 10.2 |
| AD 2 Hippo | 21.2 | 21.9 | Control (Path) 4 Temporal Ctx | 26.4 | 28.1 |
| AD 3 Hippo | 14.2 | 11.6 | AD 1 Occipital Ctx | 31.9 | 41.8 |
| AD 4 Hippo | 7.4 | 10.5 | AD 2 Occipital Ctx (Missing) | 0.0 | 0.0 |
| AD 5 hippo | 94.0 | 94.0 | AD 3 Occipital Ctx | 17.9 | 17.4 |
| AD 6 Hippo | 53.6 | 61.6 | AD 4 Occipital Ctx | 12.9 | 17.3 |
| Control 2 Hippo | 12.7 | 17.0 | AD 5 Occipital Ctx | 35.1 | 20.9 |
| Control 4 Hippo | 14.6 | 15.2 | AD 6 Occipital Ctx | 20.9 | 27.5 |
| Control (Path) 3 Hippo | 22.2 | 7.2 | Control 1 Occipital Ctx | 4.9 | 4.9 |
| AD 1 Temporal Ctx | 42.3 | 42.9 | Control 2 Occipital Ctx | 46.7 | 32.1 |
| AD 2 Temporal Ctx | 78.5 | 29.9 | Control 3 Occipital Ctx | 17.8 | 19.2 |
| AD 3 Temporal Ctx | 17.9 | 20.4 | Control 4 Occipital Ctx | 13.2 | 12.1 |
| AD 4 Temporal Ctx | 30.1 | 31.2 | Control (Path) 1 Occipital Ctx | 49.3 | 59.9 |
| AD 5 Inf Temporal Ctx | **100.0** | 94.6 | Control (Path) 2 Occipital Ctx | 13.6 | 8.8 |
| AD 5 SupTemporal Ctx | 59.9 | 52.5 | Control (Path) 3 Occipital Ctx | 6.1 | 4.7 |
| AD 6 Inf Temporal Ctx | 75.3 | 95.9 | Control (Path) 4 Occipital Ctx | 31.2 | 21.2 |
| AD 6 Sup Temporal Ctx | 94.6 | **100.0** | Control 1 Parietal Ctx | 10.3 | 11.1 |
| Control I Temporal Ctx | 8.5 | 7.3 | Control 2 Parietal Ctx | 56.6 | 60.7 |
| Control 2 Temporal Ctx | 16.3 | 18.8 | Control 3 Parietal Ctx | 15.4 | 15.0 |
| Control 3 Temporal Ctx | 13.0 | 15.3 | Control (Path) 1 Parietal Ctx | 34.2 | 41.8 |
| Control 4 Temporal Ctx | 12.8 | 16.6 | Control (Path) 2 Parietal Ctx | 15.4 | 20.3 |
| Control (Path) 1 Temporal Ctx | 26.8 | 31.0 | Control (Path) 3 Parietal Ctx | 8.4 | 7.0 |
| Control (Path) 2 Temporal Ctx | 25.5 | 17.8 | Control (Path) 4 Parietal Ctx | 44.1 | 31.6 |

**Table KE. Panel 1.3D**

| **Column A - Rel. Exp.(%) Ag1301b, Run 165528224** | | | | | |
|---|---|---|---|---|---|
| **Column B - Rel. Exp.(%) Ag3031, Run 167961982** | | | | | |
| **Tissue Name** | **A** | **B** | **Tissue Name** | **A** | **B** |
| Liver adenocarcinoma | 18.4 | 32.3 | Kidney (fetal) | 10.7 | 66.0 |
| Pancreas | 4.7 | 11.9 | Renal ca. 786-0 | 15.3 | 25.5 |
| Pancreatic ca. CAPAN 2 | 4.9 | 4.9 | Renal ca. A498 | 11.9 | 12.5 |
| Adrenal gland | 23.0 | 11.0 | Renal ca. RXF 393 | 14.9 | 18.0 |
| Thyroid | 7.1 | 8.5 | Renal ca. ACHN | 8.0 | 10.5 |
| Salivary gland | 10.1 | 13.7 | Renal ca. UO-31 | 6.0 | 1.1 |
| Pituitary gland | 18.2 | 9.2 | Renal ca. TK-10 | 7.4 | 18.6 |
| Brain (fetal) | 24.0 | 42.3 | Liver | 11.9 | 17.8 |
| Brain (whole) | 43.8 | 19.6 | Liver (fetal) | 20.6 | 14.7 |
| Brain (amygdala) | 20.4 | 9.6 | Liver ca. (hepatoblast) HepG2 | 22.2 | 28.7 |
| Brain (cerebellum) | 50.7 | 50.3 | Lung | 17.0 | 22.8 |
| Brain (hippocampus) | 18.7 | 8.1 | Lung (fetal) | 10.4 | 26.4 |
| Brain (substantia nigra) | 8.4 | 8.9 | Lung ca. (small cell) LX-1 | 9.1 | 17.4 |
| Brain (thalamus) | 20.0 | 10.6 | Lung ca. (small cell) NCI-H69 | 1.5 | 4.0 |
| Cerebral Cortex | 5.3 | 7.2 | Lung ca. (s.cell var.) SHP-77 | 18.4 | 57.4 |
| Spinal cord | 9.6 | 7.0 | Lung ca. (large cell)NCI-H460 | 50.3 | 4.2 |
| glio/astro U87-MG | 12.1 | 16.5 | Lung ca. (non-sm. cell) A549 | 8.0 | 28.1 |
| glio/astro U-118-MG | 15.5 | 13.4 | Lung ca. (non-s.cell) NCI-H23 | 10.2 | 18.2 |
| astrocytoma SW1783 | 6.9 | 11.1 | Lungca.(non-s.cell)HOP-62 | 20.3 | 33.0 |
| neuro*; met SK-N-AS | 11.4 | 10.9 | Lungca.(non-s.cl)NCI-H522 | 9.7 | 23.5 |
| astrocytoma SF-539 | 12.5 | 25.5 | Lung ca. (squam.) SW 900 | 4.0 | 8.5 |
| astrocytoma SNB-75 | 10.9 | 18.9 | Lung ca. (squam.) NCI-H596 | 1.6 | 4.8 |
| glioma SNB-19 | 32.1 | 25.3 | Mammary gland | 36.9 | 22.5 |
| glioma U251 | **100.0** | **100.0** | Breast ca.*(pl.ef) MCF-7 | 5.4 | 6.7 |
| glioma SF-295 | 18.4 | 46.0 | 231 | 16.7 | 7.9 |
| Heart (fetal) | 2.3 | 6.2 | Breast ca.* (pl.ef) T47D | 13.2 | 46.7 |
| Heart | 8.4 | 8.2 | Breast ca. BT-549 | 9.9 | 6.6 |
| Skeletal muscle (fetal) | 6.3 | 24.1 | Breast ca. MDA-N | 1.6 | 11.9 |
| Skeletal muscle | 20.2 | 7.9 | Ovary | 3.0 | 5.1 |
| Bone marrow | 21.8 | 25.2 | Ovarian ca. OVCAR-3 | 5.1 | 12.0 |
| Thymus | 18.2 | 38.4 | Ovarian ca. OVCAR-4 | 4.7 | 8.8 |
| Spleen | 26.6 | 14.0 | Ovarian ca. OVCAR-5 | 8.3 | 42.3 |
| Lymph node | 42.6 | 23.7 | Ovarian ca. OVCAR-8 | 2.8 | 0.9 |
| Colorectal | 16.8 | 16.8 | Ovarian ca. IGROV-1 | 2.6 | 11.7 |
| Stomach | 37.4 | 5.9 | Ovarian ca.*(ascites)SK-OV-3 | 10.0 | 29.9 |
| Small intestine | 36.6 | 14.2 | Uterus | 38.4 | 17.4 |
| Colon ca. SW480 | 3.7 | 4.5 | Placenta | 11.1 | 9.4 |
| Colon ca.* SW620(SW480 met) | 3.8 | 24.0 | Prostate | 18.6 | 8.5 |
| Colon ca. HT29 | 1.6 | 4.6 | Prostate ca.* (bone met)PC-3 | 6.9 | 14.1 |
| Colon ca. HCT-116 | 3.0 | 9.9 | Testis | 45.1 | 26.6 |
| Colon ca. CaCo-2 | 2.1 | 5.1 | Melanoma Hs688(A).T | 0.8 | 1.2 |
| Colon ca. tissue(ODO3866) | 5.4 | 3.6 | Melanoma* (met) Hs688(B).T | 3.8 | 3.4 |
| Colon ca. HCC-2998 | 6.2 | 15.6 | Melanoma UACC-62 | 4.6 | 8.0 |
| Gastric ca.* (liver met) NCI-N87 | 20.0 | 13.3 | Melanoma M14 | 28.5 | 9.9 |
| Bladder | 8.4 | 12.2 | Melanoma LOX IMVI | 0.7 | 8.1 |
| Trachea | 13.6 | 6.4 | Melanoma* (met) SK-MEL-5 | 2.2 | 2.9 |
| Kidney | 10.4 | 23.8 | Adipose | 8.1 | 12.3 |

**Table KF. Panel 2.2**

| **Column A - Rel. Exp.(%) Ag1301b, Run 173859869** | | | |
|---|---|---|---|
| **Tissue Name** | **A** | **Tissue Name** | **A** |
| Normal Colon | 39.2 | Kidney Margin (OD04348) | 79.0 |
| Colon cancer (OD06064) | 6.3 | Kidney malignant cancer (OD06204B) | 6.0 |
| Colon Margin (OD06064) | 13.0 | Kidney normal adjacent tissue (OD06204E) | 15.4 |
| Colon cancer (OD06159) | 0.0 | Kidney Cancer (OD04450-01) | 24.5 |
| Colon Margin (OD06159) | 27.0 | Kidney Margin (OD04450-03) | 23.7 |
| Colon cancer (OD06297-04) | 2.5 | Kidney Cancer 8120613 | 3.0 |
| Colon Margin (OD06297-05) | 39.8 | Kidney Margin 8120614 | 38.2 |
| CC Gr.2 ascend colon (ODO3921) | 3.8 | Kidney Cancer 9010320 | 8.0 |
| CC Margin (ODO3921) | 4.4 | Kidney Margin 9010321 | 10.8 |
| Colon cancer metastasis (OD06104) | 14.3 | Kidney Cancer 8120607 | 15.2 |
| Lung Margin (OD06104) | 17.2 | Kidney Margin 8120608 | 3.8 |
| Colon mets to lung (OD04451-01) | 0.0 | Normal Uterus | 33.7 |
| Lung Margin (OD04451-02) | 10.2 | Uterine Cancer 06401 | 33.9 |
| Normal Prostate | 30.6 | Normal Thyroid | 2.9 |
| Prostate Cancer (OD04410) | 7.6 | Thyroid Cancer 064010 | 4.4 |
| Prostate Margin (OD04410) | 25.5 | Thyroid Cancer A302152 | 29.9 |
| Normal Ovary | 25.0 | Thyroid Margin A302153 | 7.3 |
| Ovarian cancer (OD06283-03) | 8.1 | Normal Breast | 37.6 |
| Ovarian Margin (OD06283-07) | 34.4 | Breast Cancer (OD04566) | 21.6 |
| Ovarian Cancer 064008 | 46.3 | Breast Cancer 1024 | **100.0** |
| Ovarian cancer (OD06145) | 34.4 | Breast Cancer (QD04590-01) | 25.2 |
| Ovarian Margin (OD06145) | 52.9 | Breast Canter Mets (OD04590-03) | 35.6 |
| Ovarian cancer (OD06455-03) | 11.6 | Breast 05) Cancer Metastasis (OD04655- | 45.1 |
| Ovarian Margin (OD06455-07) | 18.8 | Breast Cancer 064006 | 21.2 |
| Normal Lung | 22.8 | Breast Cancer 9100266 | 26.6 |
| Invasive poor diff. lung adeno (ODO4945-01 | 18.7 | Breast Margin 9100265 | 19.3 |
| Lung Margin (ODO4945-03) | 13.9 | Breast Cancer A209073 | 6.1 |
| Lung Malignant Cancer (OD03126) | 11.7 | Breast Margin A2090734 | 35.8 |
| Lung Margin (OD03126) | 12.2 | Breast cancer (OD06083) | 49.3 |
| Lung Cancer (OD05014A) Lung Cancer (OD05014A) | 9.0 | Breast cancer node metastasis (OD06083) | 25.9 |
| Lung Margin (OD05014B) | 35.4 | Normal Liver | 36.3 |
| Lung cancer (OD06081) | 23.7 | Liver Cancer 1026 | 2.5 |
| Lung Margin (OD06081) | 27.9 | Liver Cancer 1025 | 45.7 |
| Lung Cancer (OD04237-01) | 13.3 | Liver Cancer 6004-T | 30.1 |
| Lung Margin (OD04237-02) | 28.5 | Liver Tissue 6004-N | 27.7 |
| Ocular Melanoma Metastasis | 11.5 | Liver Cancer 6005-T | 6.7 |
| Ocular Melanoma Margin (Liver) | 27.0 | Liver Tissue 6005-N | 17.4 |
| Melanoma Metastasis | 21.6 | Liver Cancer 064003 | 32.3 |
| Melanoma Margin (Lung) | 14.7 | Normal Bladder | 13.2 |
| Normal Kidney | 17.8 | Bladder Cancer 1023 | 23.0 |
| Kidney Ca, Nuclear grade 2 (OD04338) | 50.3 | Bladder Cancer A302173 | 20.0 |
| Kidney Margin (OD04338) | 24.3 | Normal Stomach | 89.5 |
| Kidney Ca Nuclear grade 1/2 (OD04339) | 62.0 | Gastric Cancer 9060397 | 5.7 |
| Kidney Margin (OD04339) | 16.3 | Stomach Margin 9060396 | 17.7 |
| Kidney Ca, Clear cell type (OD04340) | 7.3 | Gastric Cancer 9060395 | 19.6 |
| Kidney Margin (OD04340) | 10.7 | Stomach Margin 9060394 | 42.6 |
| Kidney Ca, Nuclear grade 3 (OD04348) | 4.6 | Gastric Cancer 064005 | 7.5 |

**Table KG. Panel 4D**

| **Column A - Rel. Exp.(%) Ag1301b, Run 138983163** | | | | | | | |
|---|---|---|---|---|---|---|---|
| **Column B - Rel. Exp.(%) Ag1415, Run 138642033** | | | | | | | |
| **Column C - Rel. Exp.(%) Ag3031, Run 162426783** | | | | | | | |
| **Tissue Name** | **A** | **B** | **C** | **Tissue Name** | **A B C** | | |
| Secondary Th1 act | 17.0 | 25.3 | 13.8 | HWEC IL-1beta | 11.5 | 13.0 | 11.3 |
| Secondary Th2 act | 22.7 | 18.9 | 20.0 | HUVEC IFN gamma | 28.3 | 24.7 | 20.2 |
| Secondary Tr1 act | 32.3 | 26.4 | 20.0 | HUVEC TNF alpha + IFN gamma | 3.2 | 6.6 | 12.3 |
| Secondary Th1 rest | 22.2 | 20.3 | 12.0 | HUVEC TNF alpha + IL4 | 11.7 | 9.7 | 9.7 |
| Secondary Th2 rest | 42.6 | 37.9 | 21.0 | HUVEC IL-11 | 10.1 | 8.2 | 11.6 |
| Secondary Tr1 rest | 27.9 | 27.5 | 26.8 | Lung Microvascular EC none | 33.9 | 28.7 | 25.2 |
| Primary Th1 act | 33.4 | 29.9 | 19.1 | Lung Microvascular EC TNFalpha + IL-1beta | 23.2 | 19.1 | 24.8 |
| Primary Th2 act | 28.1 | 41.2 | 13.3 | Microvascular Dermal EC none | 141.8 | 45.1 | 25.7 |
| Primary Tr1 act | 49.0 | 52.5 | 20.9 | Microsvasular Dermal EC TNFalpha + IL-1beta | 27.5 | 40.3 | 22.4 |
| Primary Th1 rest | 80.7 | 87.1 | 80.7 | Bronchial epithelium TNFalpha + IL1beta | 20.3 | 32.3 | 22.1 |
| Primary Th2 rest | 67.4 | 68.8 | 64.2 | Small airway epithelium none | 7.3 | 3.3 | 4.8 |
| Primary Tr1 rest | 46.0 | 50.3 | 54.7 | Small airway epithelium TNFalpha + IL-1beta | 34.4 | 35.1 | 26.8 |
| CD45RA CD4 lymphocyte act | 12.9 | 8.0 | 16.5 | Coronery artery SMC rest | 7.9 | 7.9 | 12.3 |
| CD45RO CD4 lymphocyte act | 33.4 | 4.4.8 | 22.4 | Coronery artery SMC TNFalpha + IL-1beta | 7.6 | 10.7 | 81 |
| CD8 lymphocyte act | 22.8 | 23.0 | 18.4 | Astrocytes rest | 7.9 | 10.3 | 12.7 |
| Secondary CD8 lymphocyte rest | 22.2 | 25.5 | 25.0 | Astrocytes TNFalpha + IL-1beta | 8.7 | 5.8 | 13.3 |
| Secondary CD8 lymphocyte act | 19.3 | 21.8 | 22.5 | KU-812 (Basophil) rest | 40.1 | 35.4 | 48.0 |
| CD4 lymphocyte none | 41.5 | 42.3 | 34.6 | KU-812 (Basophil) PMA/ionomycin | 57.8 | 61.1 | 78.5 |
| 2ry Th1/Th2/Tr1_anti-CD95 CH11 | 65.1 | 54.7 | 41.2 | CCD1106 (Keratinocytes) none | 3.8 | 8.0 | 6.6 |
| LAK cells rest | 28.7 | 37.1 | 32.3 | CCD1106 (Keratinocytes) TNFalpha+IL-1beta | 27.4 | 24.5 | 5.3 |
| LAK cells IL-2 | 38.7 | 49.0 | 36.3 | Liver cirrhosis | 17.0 | 9.4 | 5.0 |
| LAK cells IL-2+IL-12 | 26.8 | 27.4 | 26.8 | Lupus kidney | 24.1 | 23.8 | 9.5 |
| LAK cells IL-2+IFN gamma | 43.5 | 45.4 | 42.9 | NCI-H292 none | 38.7 | 49.3 | 45.4 |
| LAK cells IL-2+IL-18 | 26.2 | 25.2 | 35.6 | NCI-H292IL-4 | 58.6 | 51.4 | 46.7 |
| LAK cells PMA/ionomycin | 8.3 | 8.6 | 3.3 | NCI-H292 IL-9 | 56.3 | 46.0 | 54.3 |
| NK Cells IL-2 rest | 28.7 | 35.8 | 32.8 | NCI-H292 IL-13 | 30.4 | 31.9 | 23.3 |
| Two Way MLR 3 day | 42.3 | 49.0 | 46.3 | NCI-H292 IFN gamma | 16.7 | 28.3 | 29.1 |
| Two Way MLR 5 day | 17.9 | 16.2 | 13.5 | HP AEC none | 15.7 | 26.4 | 19.1 |
| Two Way MLR 7 day | 14.7 | 12.6 | 14.4 | HP AEC INF alpha + IL-1 beta | 23.8 | 32.5 | 27.9 |
| PBMC rest | 21.9 | 29.9 | 19.2 | Lung fibroblast none | 13.0 | 11.0 | 11.9 |
| PBMC PWM | 66.9 | 53.2 | 50.3 | Lung fibroblast TNF alpha + IL-1 beta | 8.7 | 7.2 | 13.2 |
| PBMC PHA-L | 35.6 | 46.7 | 23.3 | Lung fibroblast IL-4 | 5.6 | 12.0 | 10.5 |
| Ramos(B cell) none | 23.5 | 33.2 | 16.5 | Lung fibroblast IL-9 | 8.5 | 7.4 | 15.3 |
| Ramos (B cell) ionomycin | 53.2 | 53.2 | 49.3 | Lung fibroblast IL-13 | 20.3 | 16.3 | 8.9 |
| B lymphocytes PWM | 29.9 | 36.3 | 34.2 | Lung fibroblast IFN gamma | 11.1 | 10.4 | 13.0 |
| B lymphocytes CD40L and IL-4 | 33.2 | 36.6 | 35.8 | Dermal fibroblast CCD1070 rest | 47.0 | 11.3 | 13.8 |
| EOL-1 dbcAMP | 18.9 | 14.8 | 12.8 | Dermal fibroblast CCD1070 TNF alpha | 45.7 | 53.6 | 55.1 |
| EOL-1 dbcAMP PMA/ionomycin | 30.4 | 29.1 | 25.5 | Dermal fibroblast CCD1070 IL-1 beta | 16.8 | 17.3 | 15.4 |
| Dendritic cells none | 14.9 | 10.0 | 13.7 | Dermal fibroblast IFN gamma | 5.8 | 8.8 | 6.5 |
| Dendritic cells LPS | 15.7 | 5.8 | 8.7 | Dermal fibroblast IL-4 | 17.6 | 20.0 | 13.6 |
| Dendritic cells anti-CD40 | 12.7 | 17.7 | 15.2 | IBD Colitis 2 | 2.0 | 1.3 | 1.1 |
| Monocytes rest | 35.6 | 27.5 | 36.6 | IBD Crohn's | 3.0 | 1.4 | 1.4 |
| Monocytes LPS | 34.6 | 43.8 | 25.0 | Colon | 33.0 | 26.2 | 34.6 |
| Macrophages rest | 19.2 | 16.6 | 17.2 | Lung | 6.8 | 13.5 | 7.5 |
| Macrophages LPS | 17.9 | 16.2 | 6.7 | Thymus | **100.0** | **100.0** | 55.5 |
| HUVEC none | 9.8 | 13.2 | 17.3 | Kidney | 87.1 | 79.0 | **100.0** |
| HUVEC starved | 27.2 | 27.5 | 28.3 | | | | |

**CNS_neurodegeneration_v1.0 Summary:** Data obtained from RTQPCR assays performed for this panel was analyized using multivariate analysis (ANOVA). The multivariate results obtained analyzing Ag3031 in two experiments with the same probe and primer set produce results that are in excellent agreement. This gene, a kinase homolog, is upregulated in the temporal cortex of brains from Alzheimer's disease patients compared to th expression in temporal cortex of normal brains. Kinases have been shown to play a role in the pathogenesis of Alzheimer's disease (Morishima Y, et al. J Neurosci 2001 Oct 1;21 (19):7551-60). The dysregulation of this kinase, CG56829-01, indicates an active role for this pathway in disease pathogenesis. Thus, inhibitors of this gene or the kinase encoded by this gene may have utility in the treatment of Alzheimer's disease and other neurodegenerative diseases.

**Panel 1.3D Summary:** Ag1301b/Ag3031 Two experiments with the same probe and primer set produce results that are in excellent agreement, with highest expression of the CG56829-01 gene in a brain cancer cell line (CTs=29-30). Overall, this gene is expressed at moderate to low levels in all the samples in this panel.

This gene has low to moderate expression in several endocrine/metabolic-related tissues, including adipose, pancreas, liver, skeletal muscle and thyroid. Thus, a therapeutic modulator to this gene and/or gene-product maybe useful in the treatment of diseases which affect the endocrine system.

**Panel 2.2 Summary:** Ag1301b The CG56829-01 gene is expressed in breast cancer at a moderate level. It is also expressed at a higher level in normal gastric, prostate and colon tissues compared to the adjacent tumors. Hence, inhibition of this drug might be used for treatment of breast cancer. It could also be used as a diagnostic marker for gastric, prostate and colon cancers.

**Panel 4D Summary:** Ag1301b/Ag1415/Ag3031 Three experiments with the same probe and primer sets produce results that are in excellent agreement, with highest expression of the CG56829-01 gene in the thymus and kidney. This gene is also expressed at higher levels in resting Th1 and Th2 lymphocytes than in activated Th1 and Th2 lymphocytes. Therefore, modulation of the gene product with small molecule compounds and biomolecules may be useful as therapeutics to reduce the activation of Th1 and Th2 cells and thus reduce symptoms in patients with autoimmune and inflammatory diseases, such as Crohn's disease, ulcerative colitis, multiple sclerosis, chronic obstructive pulmonary disease, asthma, emphysema, rheumatoid arthritis, lupus erythematosus, or psoriasis.

### L. CG57183-01: fibroblast growth factor receptor

Expression of gene CG57183-01 was assessed using the primer-probe sets Ag4039, Ag4040 and Ag4045, described in Tables LA, LB and LC. Results of the RTQ-PCR runs are shown in Tables LD, LE, LF, LG, LH, LI, LJ and LK.

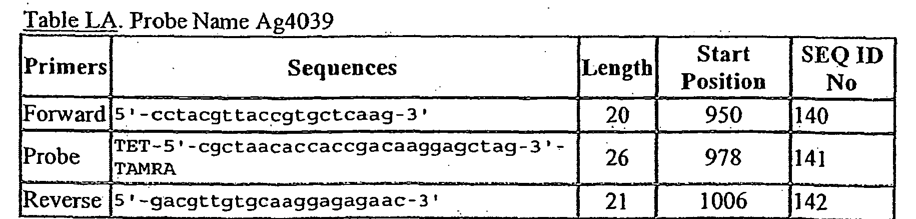

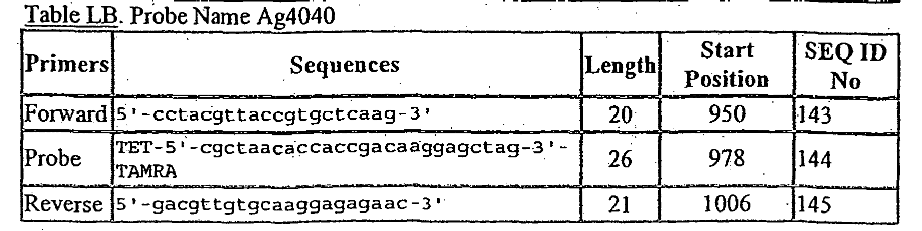

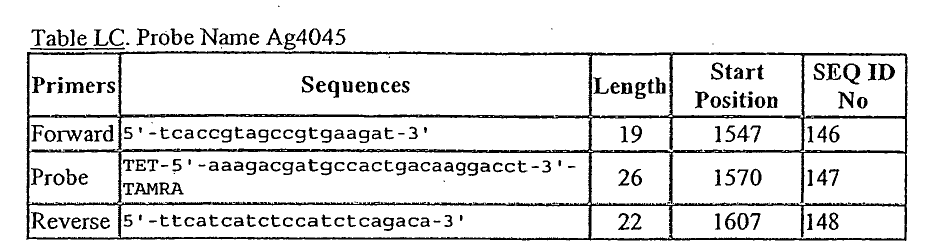

**Table LD. A1.05 chondrosarcoma**

| **Column A - Rel. Exp.(%) Ag4039, Run 316264543** | | | | | |
|---|---|---|---|---|---|
| **Column B - Rel. Exp.(%) Ag4045, Run 306518772** | | | | | |
| **Tissue Name** | **A** | **B** | **Tissue Name** | **A** | **B** |
| 138353_PMA(18hrs) | 17.9 | 5.6 | 138346_IL-1beta+Oncostatin M (6hrs) | 43.5 | 50.0 |
| 138352_IL-1beta + Oncostatin M (18hrs) | 27.4 | 2.4 | 138345_IL-1bdta+TNFa (6hrs) | 42.3 | 30.8 |
| 138351_IL-1beta+TNFa(18hrs) | 12.5 | 0.4 | 138344_IL-1beta(6hrs) | 51.1 | 22.5 |
| 138350_IL-1beta(18hrs) | 1.0 | 11.8 | 138348_Untreated-complete medium (6hrs) | 41.8 | 54.7 |
| 133354_Untreated-complete medium (18hrs) | 15.0 | 6.3 | 138349_Untreated-serum starved (6hrs) | **100.0** | **100.0** |
| 138347_PMA (6hrs) | 54.0 | 73.2 | | | |

**Table LE. AI comprehensive panel v1.0**

| **Column A - Rel. Exp.(%) Ag4039, Run 257315337** | | | | | | | |
|---|---|---|---|---|---|---|---|
| **Column B - Rel. Exp.(%) Ag4040, Run 257315338** | | | | | | | |
| **Column C - Rel. Exp.(%) Ag4045, Run 257315365** | | | | | | | |
| **Tissue Name** | **A** | **B** | **C** | **Tissue Name** | **A** | **B** | **C** |
| 110967 COPD-F | 0.0 | 0.0 | 0.0 | 112427 Match Control Psoriasis-F | 1.9 | 2.8 | 1.4 |
| 110980 COPD-F | 0.6 | 0.0 | 0.6 | 112418 Psoriasis-M | 0.2 | 0.0 | 0.1 |
| 110968 COPD-M | 0.7 | 1.5 | 0.2 | 112723 Match Control Psoriasis-M | 0.5 | 0.0 | 0.7 |
| 110977 COPD-M | 2.1 | 0.0 | 0.4 | 112419 Psoriasis-M | 0.0 | 0.5 | 0.1 |
| 110989 Emphysema-F | 1.0 | 2.4 | 1.8 | 112424 Match Control Psoriasis-M | 0.5 | 0.6 | 0.0 |
| 110992 Emphysema-F | 1.3 | 0.9 | 0.7 | 112420 Psoriasis-M | 1.4 | 2.0 | 1.9 |
| 110993 Emphysema-F | 0.4 | 0.6 | 0.3 | 112425 Match Control Psoriasis-M | 2.4 | 1.1 | 1.5 |
| 110994 Emphysema-F | 0.4 | 0.2 | 0.0 | 104689 (MF) OA Bone-Backus | 52.5 | 63.7 | 65.1 |
| 110995 Emphysema-F | 0.0 | 1.8 | 2.3 | 104690 (MF) Adj "Normal" Bone-Backus | 14.1 | 18.3 | 23.8 |
| 110996 Emphysema-F | 0.0 | 0.5 | 0.4 | 104691 (MF) OA Synovium-Backus | 0.3 | 1.1 | 0.9 |
| 110997 Asthma-M | 0.2 | 0.0 | 1.7 | 104692 (BA) OA Cartilage-Backus | **100.0** | 83.5 | 94.0 |
| 111001 Asthma-F | 1.2 | 0.8 | 0.8 | 104694 (BA) OA Bone-Backus | 83.5 | **100.0** | **100.0** |
| 111002 Asthma-F | 1.1 | 3.4 | 1.6 | 104695 (BA) Adj "Normal" Bone-Backus | 55.9 | 58.6 | 51.4 |
| 111003 Atopic Asthma-F | 2.0 | 1.0 | 2.6 | 104696 (BA) OA Synovium-Backus | 2.1 | 1.7 | 0.9 |
| 111004 Atopic Asthma-F | 2.6 | 2.3 | 10.3 | 104700 (SS) OA Bone-Backus | 22.2 | 27.7 | 34.9 |
| 111005 Atopic Asthma-F | 1.0 | 1.0 | 9.9 | 104701 (SS) Adj "Normal" Bone-Backus | 42.0 | 47.3 | 57.0 |
| 111006 Atopic Asthma-F | 1.1 | 0.0 | 0.6 | 104702 (SS) OA Synovium-Backus | 5.8 | 2.8 | 5.9 |
| 111417 Allergy-M | 0.0 | 0.5 | 3.3 | 117093 OA Cartilage Rep7 | 0.0 | 1.6 | 1.1 |
| 112347 Allergy-M | 0.0 | 0.0 | 0.0 | 112672 OA Bone5 | 1.0 | 1.3 | 2.4 |
| 112349 Normal Lung-F | 0.0 | 0.0 | 0.0 | 112673 OA Synovium5 | 0.0 | 0.0 | 0.9 |
| 112357 Normal Lung-F | 0.1 | 0.6 | 1.2 | 112674 OA Synovial Fluid cells5 | 0.0 | 0.3 | 0.3 |
| 112354 Normal Lung-M | 0.4 | 0.0 | 0.0 | 117100 OA Cartilage Rep14 | 0.5 | 0.5 | 0.4 |
| 112374Crohns-F | 0.0 | 0.5 | 0.9 | 112756 OA Bone9 | 1.1 | 1.5 | 3.1 |
| 112389 Match Control Crohns-F | 0.5 | 0.6 | 61.6 | 112757 OA Synovium9 | 0.8 | 0.8 | 0.0 |
| 112375 Crohns-F | 1.6 | 1.1 | 1.0 | 112758 OA Synovial Fluid Cells9 | 0.0 | 0.9 | 0.3 |
| 112732 Match Control Crohns-F | 1.6 | 0.7 | 49.0 | 117125 RA Cartilage Rep2 | 0.3 | 0.4 | 0.8 |
| 112725 Crohns-M | 0.0 | 0.4 | 0.0 | 113492 Bone2 RA | 1.0 | 4.8 | 11.6 |
| 112387 Match Control Crohns-M | 0.8 | 1.3 | 0.4 | 113493 Synovium2 RA | 1.0 | 0.8 | 3.8 |
| 112378 Crohns-M | 0.0 | 0.0 | 0.0 | 113494 Syn Fluid Cells RA | 2.7 | 1.5 | 7.5 |
| 112390 Match Control Crohns-M | 0.1 | 1.4 | 1.4 | 113499 Cartilage4 RA | 1.1 | 2.0 | 3.8 |
| 112726 Crohns-M | 1.5 | 1.8 | 4.2 | 113500 Bone4 RA | 0.7 | 0.0 | 5.6 |
| 112731 Match Control Crohns-M | 0.2 | 1.4 | 3.6 | 113501 Synovium4 RA | 0.0 | 1.1 | 3.9 |
| 112380 Ulcer Col-F | 2.5 | 1.2 | 5.8 | 113502 Syn Fluid Cells4 RA | 0.3 | 0.1 | 3.4 |
| 112734 Match Control Ulcer Col-F | 1.6 | 2.1 | 62.4 | 113495 Cartilage3 RA | 1.1 | 2.5 | 8.3 |
| 112384 Ulcer Col-F | 1.4 | 1.8 | 1.0 | 113496 Bone3 RA | 2.2 | 1.4 | 9.3 |
| 112737 Match Control 11273C7 Match Ulcer Col-F Control | 1.1 | 1.1 | 1.5 | 113497 Synovium3 RA | 1.5 | 0.7 | 3.8 |
| 112386 Ulcer Col-F | 0.0 | 0.2 | 1.0 | 113498 Syn Fluid Cells3 RA | 4.2 | 0.6 | 10.1 |
| 112738 Match Control Ulcer Col-F | 0.5 | 1.2 | 4.5 | 117106 Normal Cartilage Rep20 | 0.0 | 0.7 | 2.0 |
| 112381 Ulcer Col-M | 0.0 | 0.0 | 0.0 | 113663 Bone3 Normal | 0.0 | 0.0 | 0.0 |
| 112735 Match Control Ulcer Col-M | 0.0 | 0.5 | 0.1 | 113664 Synovium3 Normal | 0.0 | 0.0 | 0.0 |
| 112382 Ulcer Col-M | 0.1 | 0.0 | 19.1 | 113665 Syn Fluid Cells3 Normal | 0.0 | 0.0 | 0.0 |
| 112394 Match Control Ulcer Col-M | 0.4 | 0.0 | 0.1 | 117107 Normal Cartilage Rep22 | 0.4 | 0.0 | 0.0 |
| 112383 Ulcer Col-M | 2.3 | 2.9 | 3.6 | 113667 Bone4 Normal | 0.0 | 0.4 | 0.5 |
| 112736 Match Control Ulcer Col-M | 0.2 | 0.2 | 19.2 | 113668 Synovmm4 Normal | 0.5 | 0.2 | 0.1 |
| 112423 Psoriasis-F | 0.0 | 0.0 | 0.2 | 113669 Syn Fluid Cells4 Normal | 0.5 | 0.6 | 0.0 |

**Table LF. CNS_neurodegeneration_v1.0**

| **Column A - Rel: Exp.(%) Ag4039, Run 214151873** | | | | |
|---|---|---|---|---|
| **Column B - Rel. Exp.(%) Ag4040, Run 214151886** | | | | |
| **Column C - Rel. Exp.(%) Ag4040, Run 224341025** | | | | |
| **Column D - Rel. Exp.(%) Ag4045, Run 214291846** | | | | |
| **Tissue Name** | **A** | **B** | **C** | **D** |
| AD 1 Hippo | 25.2 | 31.6 | 23.8 | 19.8 |
| AD 2 Hippo | 61.1 | 98.6 | 64.6 | 58.6 |
| AD 3 Hippo | 9.9 | 9.7 | 11.7 | 23.8 |
| AD 4 Hippo | 34.6 | 26.8 | 33.0 | 39.5 |
| AD 5 hippo | 47.3 | 48.0 | 33.9 | 53.2 |
| AD 6 Hippo | 50.3 | 60.7 | 42.0 | 57.4 |
| Control 2 Hippo | 38.7 | 41.2 | 39.0 | 41.2 |
| Control 4 Hippo | 60.3 | 58.6 | 64.6 | 82.4 |
| Control (Path) 3 Hippo | 24.0 | 20.7 | 13.0 | 18.2 |
| AD 1 Temporal Ctx | 19.2 | 23.5 | 21.3 | 30.6 |
| AD 2 Temporal Ctx | 57.4 | 52.1 | 52.1 | 41.5 |
| AD 3 Temporal Ctx | 10.2 | 11.3 | 10.0 | 12.8 |
| AD 4 Temporal Ctx | 39.0 | 47.3 | 44.4 | 36.9 |
| AD 5 Inf Temporal Ctx | 52.1 | 52.5 | 45.7 | 45.4 |
| AD 5 SupTemporal Ctx | 42.0 | 52.9 | 33.9 | 58.2 |
| AD 6 Inf Temporal Ctx | 36.6 | 40.9 | 40.9 | 34.2 |
| AD 6 Sup Temporal Ctx | 33.2 | 48.6 | 35.6 | 31.6 |
| Control 1 Temporal Ctx | 37.9 | 31.4 | 33.0 | 32.5 |
| Control 2 Temporal Ctx | 52.1 | 52.9 | 46.3 | 39.8 |
| Control 3 Temporal Ctx | 34.2 | 33.4 | 32.8 | 33.7 |
| Control 4 Temporal Ctx | 44.4 | 50.7 | 40.9 | 46.0 |
| Control (Path) 1 Temporal Ctx | 70.7 | 77.4 | 61.6 | 66.4 |
| Control (Path) 2 Temporal Ctx | 41.2 | 72.2 | 46.0 | 57.0 |
| Control (Path) 3 Temporal Ctx | 20.4 | 27.9 | 26.8 | 26.8 |
| Control (Path) 4 Temporal Ctx | 58.2 | 76.8 | 50.3 | 66.4 |
| AD 1 Occipital Ctx | 23.0 | 24.5 | 13.0 | 20.3 |
| AD 2 Occipital Ctx (Missing) | 0.0 | 0.0 | 0.0 | 0.0 |
| AD 3 Occipital Ctx | 12.6 | 20.3 | 13.2 | 15.6 |
| AD 4 Occipital Ctx | 35.1 | 39.2 | 27.4 | 37.1 |
| AD 5 Occipital Ctx | 26.6 | 42.3 | 16.2 | 31.9 |
| AD 6 Occipital Ctx | 36.3 | 28.1 | 30.4 | 40.1 |
| Control 1 Occipital Ctx | 26.4 | 34.2 | 28.9 | 24.0 |
| Control 2 Occipital Ctx | 52.9 | 57.4 | 60.3 | 73.7 |
| Control 3 Occipital Ctx | 36.9 | 55.9 | 46.7 | 46.3 |
| Control 4 Occipital Ctx | 35.4 | 34.9 | 35.1 | 36.6 |
| Control (Path) I Occipital Ctx | **100.0** | 92.0 | **100.0** | **100.0** |
| Control (Path) 2 Occipital Ctx | 25.7 | 27.0 | 25.0 | 28.7 |
| Control (Path) 3 Occipital Ctx | 19.5 | 23.5 | 17.1 | 18.9 |
| Control (Path) 4 Occipital Ctx | 31.9 | 42.3 | 23.3 | 48.6 |
| Control 1 Parietal Ctx | 35.6 | 37.4 | 31.6 | 33.9 |
| Control 2 Parietal Ctx | 30.6 | 58.6 | 31.0 | 51.4 |
| Control 3 Parietal Ctx | 35.8 | 42.0 | 30.8 | 26.4 |
| Control (Path) 1 Parietal Ctx | 79.6 | **100.0** | 67.4 | 73.7 |
| Control (Path) 2 Parietal Ctx | 41.2 | 50.7 | 43.2 | 58.6 |
| Control (Path) 3 Parietal Ctx | 24.8 | 32.3 | 19.9 | 31.6 |
| Control (Path) 4 Parietal Ctx | 76.8 | 90.8 | 49.7 | 85.9 |

**Table LG. General screening_panel_v1.4**

| **Column A - Rel. Exp.(%) Ag4039, Run 218425990** | | | | | | | |
|---|---|---|---|---|---|---|---|
| **Column B - Rel. Exp.(%) Ag4040, Run 218426022** | | | | | | | |
| **Column C - Rel. Exp.(%) Ag4045, Run 218426255** | | | | | | | |
| **Tissue Name** | **A** | **B** | **C** | **Tissue Name** | **A** | **B** | **C** |
| Adipose | 1.3 | 0.7 | 0.9 | Renal ca. TK-10 | 31.9 | 32.8 | 27.7 |
| Melanoma* Hs688(A).T | 0.2 | 0.8 | 0.2 | Bladder | 4.0 | 3:9 | 28.1 |
| Melanoma* Hs688(B).T | 1.0 | 1.4 | 0.7 | Gastric ca. (liver met.) NCI-N87 | 0.0 | 0.0 | 2.2 |
| Melanoma* M14 | 0.2 | 0.2 | 0.0 | Gastric ca. KATO III | 0.0 | 0.0 | 0.0 |
| Melanoma* LOXIMVI | 0.5 | 1.3 | 0.4 | Colon ca. SW-948 | 0.1 | 0.0 | 4.5 |
| Melanoma* SK-MEL-5 | 1.5 | 1.5 | 1.8 | Colon ca. SW480 | 0.0 | 0.1 | 2.8 |
| Squamous cell carcinoma SCC-4 | 0.1 | 0.1 | 16.3 | Colon ca.* (SW480 met) SW620 | 0.7 | 0.3 | 2.3 |
| Testis Pool | 7.0 | 5.4 | 4.0 | Colon ca. HT29 | 0.3 | 0.1 | 17.6 |
| Prostate ca.* (bone met) PC-3 | 0.0 | 0.0 | 0.0 | Colon ca. HCT-116 | 0.4 | 0.6 | 21.6 |
| Prostate Pool | 1.5 | 1.2 | 4.9 | Colon ca. CaCo-2 | 4.2 | 4.7 | 77.4 |
| Placenta | 7.7 | 5.7 | 4.5 | Colon cancer tissue | 0.1 | 0.0 | 2.2 |
| Uterus Pool | 0.4 | 0.0 | 1.0 | Colon ca. SW1116 | 0.1 | 0.0 | 4.5 |
| Ovarian ca. OVCAR-3 | 0.0 | 0.0 | 6.0 | Colon ca. Colo-205 | 0.0 | 0.1 | 3.0 |
| Ovarian ca. SK-OV-3 | 6.8 | 6.5 | 8.7 | Colon ca. SW-48 | 0.0 | 0.0 | 1.9 |
| Ovarian ca. OVCAR-4 | 0.0 | 0.0 | 3.1 | Colon Pool | 0.9 | 0.4 | 0.7 |
| Ovarian ca. OVCAR-5 | 18.4 | 17.8 | 42.9 | Small Intestine Pool | 0.0 | 0.1 | 2.1 |
| Ovarian ca. IGROV-1 | 42.0 | 22.8 | 14.6 | Stomach Pool | 0.6 | 0.2 | 1.1 |
| Ovarian ca. OVCAR-8 | 26.2 | 26.4 | 13.9 | Bone Marrow Pool | 0.1 | 0.0 | 1.0 |
| Ovary | 0.0 | 0.2 | 0.6 | Fetal Heart | 1.0 | 0.3 | 0.7 |
| Breast ca. MCF-7 | 0.0 | 0.0 | 1.4 | Heart Pool | 0.1 | 0.4 | 0.8 |
| Breast ca. MDA-MB-231 | 4.0 | 1.7 | 1.6 | Lymph Node Pool | 0.7 | 0.1 | 1.0 |
| Breast ca. BT 549 | 2.0 | 1.8 | 1.8 | Fetal Skeletal Muscle | 0.4 | 0.1 | 0.3 |
| Breast ca. T47D | 48.3 | 47.0 | 100.0 | Skeletal Muscle Pool | 0.7 | 0.4 | 0.8 |
| Breast ca. MDA-N | 0.0 | 0.0 | 0.0 | Spleen Pool | 3.5 | 1.3 | 2.0 |
| Breast Pool Breast Pool | 0.3 | 0.3 | 1.0 | Thymus Pool 1.1 Thymus Pool | 1.0 | 0.3 | 3.3 |
| Trachea | 6.8 | 4.2 | 17.8 | CNS cancer (glio/astro) US7-MG | 0.0 | 0.0 | 0.0 |
| Lung | 0.0 | 0.1 | 0.0 | CNS cancer (glio/astro) U-118-MG | 0.0 | 0.0 | 0.0 |
| Fetal Lung | 12.6 | 10.3 | 31.4 | CNS cancer (neuro;met) SK-N-AS | 0.4 | 0.1 | 0.1 |
| Lung ca. NCI-N417 | 0.7 | 0.3 | 0.3 | CNS cancer (astro) SF-539 | 2.0 | 0.0 | 1.4 |
| Lungca.LX-1 | 0.2 | 0.3 | 12.9 | CNS cancer (astro) SNB-75 | 15.3 | 10.2 | 8.9 |
| Lung ca. NCI-H146 | 0.0 | 0.0 | 0.2 | CNS cancer (glio) SNB-19 | 30.8 | 29.5 | 17.1 |
| Lung ca. SHP-77 | 0.0 | 0.0 | 0.0 | CNS cancer (glio) SF-295 | 0.9 | 0.8 | 0.8 |
| Lung ca. A549 | 5.1 | 4.8 | 3.6 | Brain (Amygdala) Pool | 51.8 | 32.5 | 47.6 |
| Lung ca. NCI-H526 | 0.0 | 0.1 | 1.2 | Brain (cerebellum) | 68.8 | 47.6 | 60.7 |
| Lung ca. NCI-H23 | 6.7 | 6.8 | 4.2 | Brain (fetal) | 104 | 9.3 | 12.8 |
| Lung ca. NCI-H460 | 0.0 | 0.1 | 0.1 | Brain (Hippocampus) Pool | 79.0 | 39.8 | 46.7 |
| Lung ca. HOP-62 | 5.3 | 4.6 | 2.1 | Cerebral Cortex Pool | 60.7 | 55.5 | 66.0 |
| Lung ca. NCI-H522 | 6.0 | 3.0 | 2.5 | Brain (Substantia nigra) Pool | 90.8 | **100.0** | 78.5 |
| Liver | 1.5 | 1.3 | 3.4 | Brain (Thalamus) Pool | 59.5 | 55.9 | 64.6 |
| Fetal Liver | 4.0 | 4.4 | 22.8 | Brain (whole) | 43.8 | 26.8 | 24.7 |
| Liver ca. HepG2 | 74.2 | 70.2 | 61.1 | Spinal Cord Pool | **100.0** | 57.8 | 95.3 |
| Kidney Pool | 0.3 | 0.8 | 0.7 | Adrenal Gland | 1.1 | 0.5 | 0.3 |
| Fetal Kidney | 20.6 | 10.4 | 31.0 | Pituitary gland Pool | 0.7 | 0.6 | 4.9 |
| Renal ca. 786-0 | 31.9 | 20.4 | 14.9 | Salivary Gland | 0.3 | 0.0 | 0.5 |
| Renal ca. A498 | 5.6 | 1.4 | 2.2 | Thyroid (female) | 0.4 | 0.2 | 1.1 |
| Renal ca. ACHN | 5.8 | 3.0 | 2.0 | (Pancreatic ca.CAPAN2 | 0.0 | 0.0 | 0.6 |
| Renal ca. UO-31 | 0.9 | 0.8 | 0.9 | Pancreas Pool | 1.5 | 1.2 | 10.9 |

**Table LH. Oncology_cell_line_screening_panel_v3.2**

| **Column A - Rel. Exp.(%) Ag4039, Run 257342469** | | | | | | | |
|---|---|---|---|---|---|---|---|
| **Column B - Rel. Exp.(%) Ag4040, Run 257342474** | | | | | | | |
| **Column C - Rel. Exp.(%) Ag4045, Run 257342527** | | | | | | | |
| **Tissue Name** | **A** | **B** | **C** | **Tissue Name** | **A** | **B** | **C** |
| 94905_Daoy_Medullobla stoma/Cerebellum_sscD NA | 0.0 | 0.7 | 1.6 | 94954_Ca Ski_Cervical epidermoid carcinoma (metastasis)_sscDNA | 0.8 | 0.0 | 2.3 |
| 94906 TE671 Medullob lastom/Cerebellum_sscD NA | 0.7 | 2.3 | 1.0 | 94955_ES-2_Ovarian clear cell carcinoma_sscDNA | 0.0 | 0.0 | 0.0 |
| 94907_D283 Med_Medulloblastoma/C erebellum_sscDNA | 7.4 | 4.6 | 0.3 | 94957_Ramos/6h stim_ Stimulated with PMA/ionomycin 6h_sscDNA | 0.0 | 0.0 | 0.0 |
| 94908_PFSK-1_Primitive Neuroectodermal/Cerebe 11um_sscDNA | 75.3 | 82.4 | 31.9 | 94958_Ramos/14h stim_ Stimulated with PMA/ionomycin 14h_sscDNA | 0.0 | 0.0 | 0.0 |
| 94909_XF-498_CNS_sscDNA | 27.0 | 24.7 | 9.9 | 94962_MEG-01_Chronic myelogenous leukemia (megokaryoblast)_sscDNA | 5.8 | 3.8 | 3.6 |
| 94910 SNB-78_CNS/glioma_sscDNA | 0.0 | 0.0 | 0.0 | 94963_Raji_Burkitt's Iymphoma_sscDNA | 0.0 | 0.0 | 0.0 |
| 94911_SF-268_CNS/glioblastoma_s scDNA | 0.0 | 0.0 | 0.4 | 94964_Daudi_Burkitt's lymphoma_sscDNA | 0.0 | 0.0 | 0.0 |
| 94912_T98G_Glioblasto ma_sscDNA | 3.5 | 3.1 | 4.4 | 94965_U266_B-cell plasmacytoma/myeloma_ss cDNA | 0.0 | 0.4 | 0.3 |
| 96776 SK-N-SH_Neuroblastoma (metastasis)_sscDNA | 0.9 | 1.5 | 0.4 | 94968 CA46_Burkitt's lymphoma sscDNA lymphoma_sscDNA | 0.0 | 0.0 | 0.0 |
| 94913_SF-scDNA | 2.8 | 1.6 | 0.9 | B-cell lymphoma sscDNA | 0.0 | 0.0 | 0.0 |
| 132565 NT2 pool_sscDNA | 90.1 | 76.3 | 52.1 | 94972_JM1_pre-B-cell lymphoma/leukemia_sscDNA | 0.0 | 0.0 | 1.0 |
| 94914_Cerebellum_sscDNA | **100.0** | 97.9 | 25.2 | 94973_Jurkat_T cell leukemia_sscDNA | 5.5 | 6.3 | 2.0 |
| 96777_Cerebellum_sscD | **100.** | **100** | 6.5 | 94974_TF- | 2.6 | 9.7 | 4.5 |
| NA | 0 | .0 | | 1_Erythroleukemia_sscDNA | | | |
| 94916_NCI-H292_Mucoepidermold lung carcinoma_sscDNA | 0.0 | 0.0 | 6.7 | 94975_HUT 78_T-cell lymphoma_sscDNA | 6.0 | 2.9 | 4.8 |
| 94917_DMS-114_Small cell lung cancer_sscDNA | 10.5 | 9.1 | 6.4 | 94977_U937_Histiocytic lymphoma_sscDNA | 2.4 | 1.0 | 0.0 |
| 94918_DMS-79_Small cell lung cancer/neuroendocrine_s scDNA | 4.4 | 1.6 | 33.4 | 94980_KU- 812_Myelogenous leukemia sscDNA | 19.6 | 18.2 | 7.5 |
| 94919_NCI-H146_Small cell lung cancer/neuroendocnne_s scDNA | 0.0 | 0.0 | 1.1 | 94981_769-P_Clear cell renal carcinoma_sscDNA | 57.0 | 61.1 | 50.3 |
| 94920_NCI-H526_Small cell lung cancer/neuroendocrine_s scDNA | 0.9 | 2.0 | 8.6 | 94983_Caki-2_Clear cell renal carcinoma_sscDNA | 0.8 | 4.3 | 4.0 |
| 94921_NCI-N417_Small cell lung cancer/neuroendocnne_s scDNA | 0.7 | 0.4 | 0.5 | 94984_SW 839_Clear cell renal carcinoma_sscDNA | 0.0 | 0.0 | 0.0 |
| 94923_NCI-H82_Small cell lung 5.8 cancer/neuroendocrine_s scDNA | 5.8 | 1.3 | 2.6 | 94986_G401_Wilms' tumor_sscDNA | 10.2 | 6.8 | 3.5 |
| 94924_NCI-H157_Squamous cell lung cancer (metastasis)_sscDNA | 2.1 | 1.6 | 0.6 | 126768_293 cells_sscDNA | 15.8 | 23.8 | 18.6 |
| 94925_NCl-H1 55_Large cell lung cancer/neuroendocrine_sscDNA | 0.0 | 1.3 | 1.8 | 94987_Hs766T_Pancreatic carcinoma (LN metastasis)_sscDNA | 0.6 | 1.0 | 2.1 |
| 94926_NCI-H1299_Large cell lung cancer/neuroendocrine_sscDNA | 5.4 | 4.9 | 3:5 | 94988_CAPAN-1_Pancreatic adenocarcinoma (liver metastasis)_sscDNA | 1.9 | 0.0 | 0.8 |
| 94927_NCl-H727_Lung carcinoid sscDNA | 0.2 | 0.0 | 4.8 | 94989_SU86.86_Pancreatic carcinoma (liver metastasis) sscDNA | 0.0 | 0.0 | 6.1 |
| 94928 NCI-UMC-11_Lung carcinoid_sscDNA | 0.0 | 0.0 | 0.2 | 94990_BxPC-3_Pancreatic 100 adenocarcinoma_sscDNA | 0.8 | 0.4 | **100.0** |
| 94929_LX-1_Small cell lung cancer _sscDNA | 0.0 | 0.0 | 18.7 | 94991_HPAC_Pancreatic adenocarcinoma_sscDNA | 0.0 | 0.0 | 1.2 |
| 94930_Colo-205_Colon cancer_sscDNA | 0.0 | 0.0 | 14.8 | 94992_MIA PaCa- 2_Pancreatic carcinoma_sscDNA | 0.7 | 0.8 | 3.1 |
| 94931_KM12_Colon cancer sscDNA | 0.0 | 0.0 | 1.2 | 94993_CFPAC- 1_Pancreatic ductal adenocarcinoma_sscDNA | 0.7 | 1.8 | 24.0 |
| 94932 KM20L2_Colon cancer_sscDNA | 0.0 | 0.0 | 11.2 | 94994 PANC-1 Pancreatic epithelioid ductal carcinoma_sscDNA | 5.8 | 5.3 | 11.8 |
| 94933_NCI-H716_Colon cancer_sscDNA | 18.0 | 20.9 | 0.0 | 94996_T24_Bladder carcinma (transitional cell)_sscDNA | 0.0 | 0.0 | 0.0 |
| 94935_SW-48_Colon adenocarcinoma_sscDN A | 0.0 | 0.8 | 6.2 | 94997 5637 Bladder carcinoma_sscDNA | 0.0 | 0.0 | 0.8 |
| 94936_SW1116_Colon adenocarcinoma_sscDN A | 0.0 | 0.0 | 18.6 | 94998_HT-1197_Bladder carcinoma_sscDNA | 0.0 | 0.0 | 10.2 |
| 94937_Ls 74T_Colon adenocarcinoma_sscDN A | 0.7 | 0.6 | 30.4 | 94999_UM-UC-3_Bladder carcinma (transitional cell)_sscDNA | 0.6 | 5.5 | 1.7 |
| 94938_SW-948_Colon adenocarcinoma_sscDN A | 0.0 | 0.0 | 2.1 | 95000_A204_Rhabdomyos arcoma_sscDNA | 8.3 | 20.9 | 10.4 |
| 94939_SW-480_Colon adenocarcinoma_sscDN A | 0.0 | 0.0 | 8.5 | 95001_HT- 1080_Fibrosarcoma_sscDN A | 2.3 | 4.0 | 3.0 |
| 94940_NCI-SNU-5_Gastric carcinoma_sscDNA | 0.0 | 0.0 | 3.5 | 95002_MG- 63_Osteosarcoma (bone)_sscDNA | 0.0 | 0.9 | 0.7 |
| 112197 KATO III_Stomach_sscDNA | 0.0 | 0.0 | 0.0 | 95003_SK-LMS-1_Lejomyosarcoma (vulva)_sscDNA | 0.0 | 0.0 | 0.0 |
| 94943_NCI-SNU-16_Gastric carcinoma_sscDNA | 0.0 | 0.0 | 0.0 | 95004_SJRH30_Rliabdomy osarcoma (met to bone marrow)_sscDNA | 1.4 | 0.0 | 4.5 |
| 94944 NCI-SNU-1_Gastric carcinoma_sscDNA | 0.0 | 0.0 | 0.0 | 95005_A431_Epidermoid carcinoma_sscDNA | 0.0 | 0.0 | 18.6 |
| 94946_RF-1_Gastric adenocarcinoma_sscDNA | 80.1 | 0.0 | 0.0 | 4_Melanoma_sscDNA | 0.0 | 0.0 | 0.0 |
| 94947_RF-48_Gastric adenocarcinoma_sscDNA | 0.0 | 0.0 | 0.0 | 112195_DU 145_Prostate_sscDNA | 0.7 | 0.0 | 3.6 |
| 96778_MKN-45_Gastric carcinoma_sscDNA | 0.0 | 0.3 | 23.5 | 95012_MDA-MB-468_Breast adenocarcinoma_sscDNA | 0.0 | 0.0 | 11.5 |
| 94949_NCI-N87_Gastric carcinoma_sscDNA | 0.2 | 0.0 | 6.8 | 112196_SSC-4_Tongue_sscDNA | 1.5 | 0.0 | 39.5 |
| 94951_OVCAR-5_Ovarian carcinoma_sscDNA | 0.0 | 0.0 | 12.1 | 112194_SSC-9_Tongue_sscDNA | 0.7 | 0.0 | 11.5 |
| 94952_RL95-2_Uterine carcinoma_sscDNA | 0.8 | 0.0 | 19.1 | 112191_SSC-15_Tongue_sscDNA | 0.0 | 0.0 | 26.2 |
| 94953_HelaS3_Cervical adenocarcinoma_sscDN A | 3.6 | 1.0 | 7.2 | 95017_CAL 27_Squamous cell carcinoma of tongue_sscDNA | 0.0 | 0.5 | 54 0 |

**Table LI. Panel 4.1D**

| **Column A - Rel. Exp.(%) Ag4039, Run 171616916** | | | | | | | |
|---|---|---|---|---|---|---|---|
| **Column B - Rel. Exp.(%) Ag4040, Run 171616917** | | | | | | | |
| **Column C - Rel. Exp.(%) Ag4045, Run 171617030** | | | | | | | |
| **Tissue Name** | **A** | **B** | **C** | **Tissue Name** | **A** | **B** | **C** |
| Secondary Th1 act | 0.0 | 0.0 | 0.0 | HUVEC IL-1beta | 1.8 | 1.9 | 0.6 |
| Secondary Th2 act | 0.0 | 0.0 | 0.0 | HUVEC IFN gamma | 3.4 | 4.7 | 0.4 |
| Secondary Tr1 act | 6.2 | 0.0 | 0.4 | gamma TNF alpha + IFN gamma | 1.6 | 0.0 | 0.5 |
| Secondary Th1 rest | 2.4 | 0.0 | 0.0 | HUVECTNF alpha+IL4 | 0.0 | 0.5 | 1.1 |
| SecondaryTh2 rest | 0.0 | 0.0 | 0.0 | EFUVEC IL-11 | 1.6 | 3.1 | 1.2 |
| Secondary Tr1 rest | 0.0 | 2.6 | 0.0 | Lung Microvascular EC none | 25.7 | 36.9 | 7.6 |
| Primary Th1 act | 0.0 | 0.0 | 0.0 | Lung Microvascular EC TNFalpha + IL-1beta | 5.4 | 13.0 | 4.4 |
| Primary Th2 act none | 0.0 | 0.0 | 0.5 | Microvascular Dermal EC | 1.4 | 2.0 | 0.0 |
| Primary Tr1 act | 0.0 | 0.0 | 0.0 | Micrbsvasular Dermal EC TNFalpha + IL-1beta | 0.0 | 1.9 | 1.7 |
| Primary Th1 rest | 0.0 | 0.0 | 0.0 | Bronchial epithelium TNFalpha + IL1beta | 4.3 | 5.6 | 27.4 |
| Primary Th2 rest | 0.0 | 0.0 | 0.0 | Small airway epithelium none | 1.6 | 0.0 | 35.4 |
| Primary Tr1 rest | 2.4 | 3.5 | 0.0 | Small airway epithelium TNFalpha+IL-1beta | 4.3 | 0.9 | 94.0 |
| CD45RA CD4 lymphocyte act | 0.0 | 6.5 | 0.9 | Coronery artery SMC rest | 0.0 | 4.0 | 0.0 |
| CD45RO CD4 lymphocyte act | 0.0 | 0.0 | 0.0 | Coronery artery SMC TNFalpha + IL-1beta | 2.0 | 0.0 | 0.6 |
| CD8 lymphocyte act | 0.0 | 0.0 | 0.0 | Astrocytes rest | 27.9 | 493 | 3.6 |
| Secondary CD8 lymphocyte rest | 0.0 | 0.0 | 0.0 | Astrocytes TNFalpba + IL-1beta | 14.5 | 34.2 | 2.9 |
| Secondary CD8 lymphocyte act | 0.0 | 0.0 | 0.4 | KU-812 (Basophil) rest | 24.1 | 35.1 | 8.2 |
| CD4 lymphocyte none | 0.0 | 0.0 | 0.0 | KU-812 (Basophil) PMA/ionomycin | 5.1 | 9.4 | 2.7 |
| 2ry Tb1/Th2/Tr1_anti-CD95 CH11 | 2.3 | 0.0 | 0.0 | CCD1106 (Keratinocytes) none | 1.9 | 0.0 | 15.8 |
| LAK cells rest | 2.4 | 0.0 | 0.0 | CCD1106 (Keratinocytes) TNFalpha + IL-1beta | 1.8 | 4.4 | 16.5 |
| LAK cells IL-2 | 0.0 | 0.0 | 0.0 | Liver cirrhosis | 3.3 | 7.8 | 25.2 |
| LAK cells IL-2+IL-12 | 0.0 | 0.0 | 0.0 | NCI-H292 none | 0.0 | 2.7 | 5.9 |
| LAK cells IL-2+IFN gamma | 0.0 | 0.0 | 0.6 | NCI-H292 IL-4 | 0.0 | 0.0 | 7.4 |
| LAK cells IL-2+IL-18 | 0.0 | 0.0 | 0.0 | NCI-H292 IL-9 | 0.0 | 0.0 | 2.9 |
| LAK cells PMA/ionomycin | 2.9 | 0.0 | 0.4 | NCI-H292 IL-13 | 0.0 | 0.0 | 5.6 |
| NK Cells IL-2 rest | 0.0 | 0.0 | 0.0 | NCI-H292 IFN gamma | 6.1 | 0.0 | 2.3 |
| Two Way MLR 3 day | 0.0 | 0.0 | 0.0 | HPAEC none | 0.0 | 5.1 | 1.8 |
| Two Way MLR 5 day | 0.0 | 0.0 | 0.0 | HPAEC TNF alpha + IL-1 beta | 0.0 | 2.1 | 0.0 |
| Two Way MLR 7 day | 0.0 | 0.0 | 0.0 | Lung fibroblast none | 0.0 | 0.0 | 0.2 |
| PBMC rest | 4.2 | 0.0 | 0.0 | Lung fibroblast TNF alpha + IL-1 beta | 0.0 | 7.8 | 0.0 |
| PBMC PWM | 0.0 | 0.0 | 0.0 | Lung fibroblast IL-4 | 1.6 | 0.0 | 0.5 |
| PBMC PHA-L | 0.0 | 0.0 | 0.0 | Lung fibroblast IL-9 | 0.0 | 0.0 | 0.0 |
| Ramos (B cell) none | 0.0 | 0.0 | 0.0 | Lung fibroblast IL-13 | 0.0 | 0.0 | 0.0 |
| Ramos (B cell) ionomycin | 0.0 | 0.0 | 0.0 | Lung fibroblast IFN gamma | 6.0 | 2.4 | 1.3 |
| B lymphocytes PWM | 0.0 | 0.0 | 0.0 | Dermal fibroblast CCD1070 rest | 3.3 | 18.3 | 2.4 |
| B lymphocytes CD40L and IL-4 | 0.0 | 0.0 | 0.1 | Dermal fibroblast CCD1070 TNF alpha | 15.2 | 11.4 | 2.8 |
| EOL-1 dbcAMP | 14.9 | 16.3 | 9.3 | Dermal fibroblast CCD1070 IL-1 beta | 1.6 | 3.2 | 2.3 |
| EOL-1 dbcAMP PMA/ionomycin | 15.9 | 14.0 | 6.2 | Dermal fibroblast IFN gamma | 11.3 | 3.4 | 1.0 |
| Dendritic cells none | 0.0 | 0.0 | 0.0 | Dermal fibroblast IL-4 | 8.2 | 0.0 | 1.0 |
| Dendritic cells LPS | 0.0 | 0.0 | 0.0 | Dermal Fibroblasts rest | 0.0 | 3.0 | 0.3 |
| Dendritic cells anti-CD40 | 0.0 | 0.0 | 0.0 | Neutrophils TNFa+LpS | 0.0 | 0.0 | 0.4 |
| Monocytes rest | 0.0 | 0:0 | 0.0 | Neutrophils rest | 0.0 | 0.0 | 0.7 |
| Monocytes LPS | 0.0 | 0.0 | 0.0 | Colon | 2.8 | 2.9 | 11.1 |
| Macrophagesrest | 0.0 | 0.0 | 0.0 | Lung | 0.0 | 0.0 | 4.2 |
| Macrophages LPS | 0.0 | 0.0 | 0.0 | Thymus | 22.8 | 8.2 | 8.7 |
| HUVEC none | 0.0 | 0.0 | 0.0 | Kidney | **100.0** | **100.0** | **100.0** |
| HUVEC starved | 8.4 | 2.3 | 2.0 | | | | |

**Table LJ. Panel 5D**

| **Column A - Rel. Exp.(%) Ag4039, Run 257460989** | | | | | | | |
|---|---|---|---|---|---|---|---|
| **Column B - Rel. Exp.(%) Ag4040, Run 257460990** | | | | | | | |
| **Column C - Rel. Exp.(%) Ag4045, Run 257488184** | | | | | | | |
| **Tissue Name** | **A** | **B** | **C** | **Tissue Name** | **A** | **B** | **C** |
| 97457_Patient-02go_adipose | 0.0 | 2.0 | 0.0 | 94709_Donor 2 AM - A_adipose | 1.0 | 9.0 | 0.3 |
| 97476_Patient-07sk skeletal muscle | 1.4 | 0.0 | 0.7 | 94710_Donor 2 AM - B_adipose | 0.9 | 0.0 | 0.9 |
| 97477_Patient-07ut_uterus | 0.0 | 1.4 | 0.2 | 94711_Donor 2 AM - C_adipose | 0.0 | 2.4 | 0.4 |
| 97478_Patient-07pl_placenta | 8.7 | 12.7 | 4.4 | 94712_Donor 2 AD - A_adipose | 0.0 | 0.0 | 0.0 |
| 97481_Patient-08sk_skeletal muscle | 0.0 | 0.0 | 0.3 | 94713_Donor 2 AD - B_adipose | 0.0 | 0.0 | 0.0 |
| 97482_Patient-08ut_uterus | 0.0 | 0.0 | 0.0 | 94714_Donor 2 AD - C_adipose | 1.4 | 0.0 | 0.3 |
| 97483_Patient-08pl_placenta | 5.8 | 12.2 | 4.9 | 94742_Donor 3 U - A_Mesenchymal Stem Cells | 0.0 | 0.0 | 1.2 |
| 97486_Patient-09sk_skeletal muscle | 0.0 | 0.0 | 0.0 | 94743_Donor 3 U - B_Mesenchymal Stem Cells | 2.7 | 0.0 | 0.4 |
| 97487_Patient-09ut_uterus | 0.0 | 2.3 | 0.0 | 94730_Donor 3 AM - A_adipose | 1.6 | 6.2 | 1.2 |
| 97488_Patient-09pl_placenta | 13.5 | 7.7 | 6.9 | 94731_Donor 3 AM - B_adipose | 3.8 | 4.1 | 0.4 |
| 97492 Patient-10ut_uterus | 0.0 | 2.1 | 0.0 | 94732_Donor 3 AM - C_adipose | 1.3 | 2.3 | 0.8 |
| 97493_Patient-10pl_piacenta 10pl_acenta | 22.1 | 14.6 | 8.5 | 94733_Donor 3 AD - A_adipose | 6.7 | 0.0 | 1.7 |
| 97495_Patient-11go_adipose | 1.6 | 0.0 | 0.8 | 94734_Donor 3 AD - B_adipose | 3.1 | 0.0 | 0.7 |
| 97496_Patient-11sk_skeletal muscle | 0.0 | 1.3 | 0.3 | 94735_Donor 3 AD - C_adipose | 0.2 | 0.9 | 1.7 |
| 97497_Patient-11ut_uterus | 0.0 | 0.0 | 0.0 | 77138_Liver_HepG2untreated | **100.0** | **100.0** | **100.0** |
| 97498_Patient-11pl_placenta | 14.0 | 15.7 | 5.0 | 73556_Heart_Cardiac stromal cells (primary) | 0.0 | 0.0 | 0.0 |
| 97500_Patient-12go_adipose | 1.8 | 1.5 | 1.7 | 81735_Small Intestine | 0.0 | 0.0 | 8.1 |
| 97501_Patient- 12sk_skeletal muscle | 1.5 | 1.1 | 0.5 | 72409_Kidney_Proximal Convoluted Tubule | 0.0 | 0.0 | 0.0 |
| 97502_Patient- 12ut_uterus | 1.6 | 1.7 | 1.1 | 82685_Small intestine_Duodenum | 2.0 | 2.0 | 7.0 |
| 97503_Patient- 12pl _placenta | 11.3 | 11.3 | 2.7 | 90650_Adrenal_Adrenocortical adenoma | 0.0 | 0.0 | 0.3 |
| 94721_Donor 2 U - A_Mesenchymal Stem Cells | 0.0 | 0.0 | 0.9 | 72410_Kidney_HRCE | 0.0 | 1.9 | 0.4 |
| 94722_Donor 2 U - B_Mesenchymal Stem Cells | 0.0 | 0.0 | 0.0 | 72411_Kidney_HRE | 2.6 | 0.0 | 1.7 |
| 94723 Donor 2 U - C_Mesenchymal Stem Cells | 0.0 | 2.1 | 1.2 | 73139 Uterus_Uterine smooth muscle cells | 0.0 | 2.2 | 0.2 |

**Table LK. general oncology screening panel_v_2.4**

| **Column A - Rel. Exp.(%) Ag4039, Run 268362924** | | | | | | | |
|---|---|---|---|---|---|---|---|
| **Column B - Rel. Exp.(%) Ag4040, Run 268362927** | | | | | | | |
| **Column C - Rel. Exp.(%) Ag4045, Run 268362935** | | | | | | | |
| **Tissue Name** | **A** | **B** | **C** | **Tissue Name** | **A** | **B** | **C** |
| Colon cancer 1 | 2.3 | 1.0 | 6.8 | Bladder NAT 2 | 0.0 | 0.0 | 0.0 |
| Colon NAT 1 | 0.5 | 1.3 | 6.2 | Bladder NAT 3 | 0.0 | 0.0 | 1.3 |
| Colon cancer 2 | 2.6 | 1.9 | 0.3 | Bladder NAT 4 | 0.2 | 0.7 | 0.4 |
| Colon NAT 2 | 0.5 | 0.7 | 7.7 | Prostate adenocarcinoma 1 | 0.0 | 0.0 | 2.0 |
| Colon cancer 3 | 1.3 | 3.8 | 9.7 | Prostate adenocarcinoma 2 | 1.0 | 2.0 | 1.4 |
| Colon NAT 3 | 3.6 | 0.8 | 12.4 | Prostate adenocarcinoma 3 | 4.3 | 0.8 | 5.2 |
| Colon malignant cancer 4 | 0.5 | 0.0 | 8.4 | Prostate adenocarcinoma 4 | 0.7 | 0.0 | 7.6 |
| Colon NAT 4 | 1.3 | 1.0 | 1.3 | Prostate NAT 5 | 0.0 | 0.0 | 6.0 |
| Lung cancer 1 | 1.3 | 0.0 | 2.2 | Prostate adenocarcinoma 6 | 0.9 | 1.4 | 2.1 |
| Lung NAT 1 | 0.0 | 0.9 | 0.4 | Prostate adenocarcinoma 7 | 1.3 | 1.2 | 0.7 |
| Lung cancer 2 | **100.0** | **100.0** | **100.0** | Prostate adenocarcinoma 8 | 1.1 | 0.0 | 0.3 |
| Lung NAT 2 | 2.7 | 1.9 | 1.2 | Prostate adenocarcinoma 9 | 0.8 | 1.3 | 0.7 |
| Squamous cell carcinoma 3 | 0.0 | 1.3 | 33.2 | Prostate NAT 10 | 0.8 | 1.3 | 1.1 |
| Lung NAT 3 | 0.0 | 0.0 | 0.5 | Kidney cancer 1 | 13.9 | 11.6 | 8.7 |
| Metastatic melanoma 1 | 0.0 | 1.0 | 0.3 | Kidney NAT 1 | 3.5 | 6.8 | 18.4 |
| Melanoma 2 | 1.0 | 0.0 | 22.4 | Kidney cancer 2 | 66.4 | 66.9 | 32.3 |
| Melanoma 3 | 0.0 | 0.0 | 2.5 | Kidney NAT 2 | 60.3 | 28.5 | 76.3 |
| Metastatic melanoma 4 | 1.9 | 1.2 | 0.3 | Kidney cancer 3 | 25.2 | 42.3 | 49.0 |
| Metastatic melanoma 5 | 3.7 | 3.4 | 0.1 | Kidney NAT 3 | 21.5 | 13.2 | 91.4 |
| Bladder cancer 1 | 0.0 | 0.0 | 0.0 | Kidney cancer 4 | 72.2 | 43.5 | 15.7 |
| Bladder NAT 1 | 0.0 | 0.0 | 0.0 | Kidney NAT 4 | 48.6 | 53.6 | 18.7 |
| Bladder cancer 2 | 0.0 | 0.0 | 2.7 | | | | |

**AI.05 chondrosarcoma Summary:** Ag4039/Ag4045 Highest expression of this gene is detected in untreated serum starved chondrosarcoma cell line (SW1353) (CTs=29-32). Interestingly, expression of this gene appears to be somewhat down regulated upon IL-1 treatment, a potent activator of pro-inflammatory cytokines and matrix metalloproteinases which participate in the destruction of cartilage observed in Osteoarthritis (OA). Therefore, therapeutic modulation of the activity of this gene or its protein product may be important for preventing the degeneration of cartilage observed in osteoarthritis.

**Al_comprehensive panel_v1.0 Summary:** Ag4039/Ag4040/Ag4045 Three experiments with two different probe-primer sets are in good agreement with highest expression of this gene seen in osteoarthritis (OA) cartilage samples (CTs=29). Significant expression of this gene is mainly detected in samples derived from OA bone and adjacent normal bone, and OA cartilage. Therefore, therapeutic modulation of this gene or its protein product may be useful in the treatment of osteoarthritis.

**CNS_neurodegeneration_v1.0 Summary:** Data obtained from RTQPCR assays performed for this panel was analyized using multivariate analysis (ANOVA). The multivariate results obtained analyzing Ag4039/Ag4040/Ag4045 confirms the expression of this gene at low levels in the brain in an independent group of individuals. This gene is found to be down-regulated in the temporal cortex of Alzheimer's disease patients (p = 0.0007). Therefore, up-regulation of this gene or its protein product, or treatment with specific agonists for this receptor may be of use in reversing the dementia/memory loss associated with this disease and neuronal death.

**General_screening_panel_v1.4 Summary:** Ag4039/Ag4040/Ag4045 Three experiments with two different probe-primer sets are in good agreement with highest expression of this gene seen in spinal cord, brain substantia nigra and a breast cancer T47D cell line (CTs=26-27.4). High expression of this gene is mainly seen in all the regions of the central nervous system examined, including amygdala, hippocampus, substantia nigra, thalamus, cerebellum, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

**Oncology_cell_line_screening_panel_v3.2 Summary:** Ag4039/Ag4040 Two experiments with same probe-primer sets are in good agreement. Highest expression of this gene seen in cerebellum samples (CTs=31). Significant expression of this gene is seen in number of cell lines derived from brain, colon, gastric, renal and bone marrow cancers.

Ag4045 Highest expression of this gene is seen in pancreatic adenocarcinoma cell line samples (CT=30.7). Moderate to low expression of this gene is seen in several cancer cell lines derived from brain, lung, colon, gastric, ovarian, uterine, pancreatic, renal, bone marrow, bladder, epidermoid, ovarian and tongue cancers. Therefore, therapeutic modulation of this gene or its gene product may be useful in the treatment of these cancers.

**Panel 4.1D Summary:** Ag4039/Ag4040/Ag4045 Highest expression of this gene is seen in kidney (CT=31.3). Moderate to low expression of this gene is seen in eosinophils, lung microvascular endothelial cells, astrocytes, basophils, and activated dermal fibroblasts. The variant gene recognized by the probe-primer set Ag4045 also shows expression in bronchial and small airway epithelium, mucoepidermoid NCI-H292 cell line, keratinocytes, and normal tissues represented by colon, lung and thymus. Therefore, therapeutic modulation of this gene or its protein product may be useful in treatment of autoimmune and inflammatory diseases such as asthma, allergies, inflammatory bowel disease, lupus erythematosus, psoriasis, rheumatoid arthritis, and osteoarthritis.

**Panel 5D Summary:** Ag4039/Ag4040/Ag4045 Highest expression of this gene is seen in liver cancer HepG2 cell line (CTs=31.3). Low expression of this gene is also seen in placenta from diabetic and non-diabetic patient.

**general oncology screening panel_v_2.4 Summary:** Ag4039/Ag4040/Ag4045 Three experiments with two different probe-primer sets are in good agreement with highest expression of this gene seen in lung cancer sample (CTs=28-30.8). Significant expression of this gene is also seen in normal and cancer samples from kidney. The variant gene recognized by the probe-primer set Ag4045 also shows expression in normal and cancer samples derived from prostate, metastatic melanoma, lung and colon.

### M. CG57341-01: Short Chain dehydrbgenase/reductase 1

Expression of gene CG57341-01 was assessed using the primer-probe set Ag3204, described in Table MA. Results of the RTQ-PCR runs are shown in Tables MB, MC and MD.

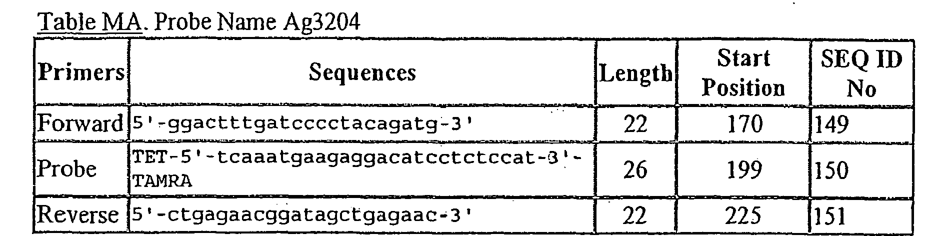

**Table MB. CNS neurodegeneration v1.0**

| **Column A - Rel. Exp.(%) Ag3204, Run 209861769** | | | | | |
|---|---|---|---|---|---|
| **Column B - Rel. Exp.(%) Ag3204, Run 249265914** | | | | | |
| **Tissue Name** | **A** | **B** | **Tissue Name** | **A** | **B** |
| AD 1 Hippo | 3.9 | 0.1 | Control (Path) 3 Temporal Ctx | 3.7 | 0.1 |
| AD 2 Hippo | 15.3 | 0.2 | Control (Path) 4 Temporal Ctx | 25.5 | 0.3 |
| AD 3 Hippo | 3.3 | 0.1 | AD 1 Occipital Ctx | 7.2 | 0.1 |
| AD 4 Hippo | 3.9 | 0.1 | AD 2 Occipital Ctx (Missing) | 0.0 | 0.0 |
| AD 5 hippo | 67.4 | 0.9 | AD 3 Occipital Ctx | 3.9 | 0.0 |
| AD 6 Hippo | 20.9 | 0.3 | AD 4 Occipital Ctx | 14.7 | 0.2 |
| Control 2 Hippo | 24.7 | 0.4 | AD 5 Occipital Ctx | 16.8 | 0.7 |
| Control 4 Hippo | 5.4 | 0.1 | AD 6 Occipital Ctx | 64.2 | 0.2 |
| Control (Path) 3 Hippo | 3.9 | 0.1 | Control 1 Occipital Ctx | 5.1 | 0.1 |
| AD 1 Temporal Ctx | 7.5 | 0.1 | Control 2 Occipital Ctx | **100.0** | 1.5 |
| AD 2 Temporal Ctx | 10.5 | 0.3 | Control 3 Occipital Ctx | 18.6 | 0.3 |
| AD 3 Temporal Ctx | 2.7 | 0.1 | Control 4 Occipital Ctx | 5.3 | **100.0** |
| AD 4 Temporal Ctx | 12.8 | 0.2 | Control (Path) 1 Occipital Ctx | 78.5 | 1.1 |
| AD 5 Inf Temporal Ctx | 57.0 | 0.0 | Control (Path) 2 Occipital Ctx | 12.1 | 0.2 |
| AD 5 SupTemporal Ctx | 22.8 | 0.4 | Control (Path) 3 Occipital Ctx | 3.3 | 0.1 |
| AD 6 Inf Temporal Ctx | 26.2 | 0.5 | Control (Path) 4 Occipital Ctx | 22.5 | 0.3 |
| AD 6 Sup Temporal Ctx | 30.6 | 0.4 | Control 1 Parietal Ctx | 7.1 | 0.1 |
| Control 1 Temporal Ctx | 7.7 | 0.1 | Control 2 Parietal Ctx | 29.1 | 0.3 |
| Control 2 Temporal Ctx | 48.0 | 0.9 | Control 3 Parietal Ctx | 23.7 | 0.3 |
| Control 3 Temporal Ctx | 17.1 | 0.2 | Control (Path) 1 Parietal Ctx | 92.7 | 1.2 |
| Control 4 Temporal Ctx | 4.6 | 0.1 | Control (Path) 2 Parietal Ctx | 24.5 | 0.5 |
| Control (Path) 1 Temporal Ctx | 55.1 | 0.8 | Control (Path) 3 Parietal Ctx | 5.3 | 0.1 |
| Control (Path) 2 Temporal Ctx | 39.5 | 0.5 | Control (Path) 4 Parietal Ctx | 44.1 | 0.5 |

**Table MC. Panel 1.3D**

| **Column A - Rel. Exp.(%) Ag3204, Run 167994669** | | | |
|---|---|---|---|
| **Tissue Name** | **A** | **Tissue Name** | **A** |
| Liver adenocarcinoma | 23.8 | Kidney (fetal) | 23.2 |
| Pancreas | 9.9 | Renal ca. 786-0 | 7.0 |
| Pancreatic ca. CAPAN 2 | 9.6 | Renal ca. A498 | 11.7 |
| Adrenal gland | 3.8 | Renal ca. RXF 393 | 8.8 |
| Thyroid | 10.0 | Renal ca. ACHN | 9.7 |
| Salivary gland | 5.9 | Renal ca. UO-31 | 2.6 |
| Pituitary gland | 10.5 | Renal ca. TK-10 | 22.8 |
| Brain (fetal) | 14.0 | Liver | 7.8 |
| Brain (whole) | 46.3 | Liver (fetal) | 8.2 |
| Brain (amygdala) | 16.8 | Liver ca. (hepatoblast) HepG2 | 36.9 |
| Brain (cerebellum) | 22.7 | Lung | 2.2 |
| Brain (hippocampus) | 18.9 | Lung (fetal) | 5.6 |
| Brain (substantia nigra) | 20.3 | Lung ca. (small cell) LX-1 | 19.1 |
| Brain (thalamus) | 26.6 | Lung ca. (small cell) NCI-H69 | 6.1 |
| Cerebral Cortex | 66.4 | Lung ca. (s.cell var.) SHP-77 | 75.3 |
| Spinal cord | 6.9 | Lung ca. (large cell)NCI-H460 | 1.5 |
| glio/astro U87-MG | 11.8 | Lung ca. (non-sm. cell) A549 | 13.4 |
| glio/astro U-118-MG | 5.0 | Lung ca. (non-s.cell) NCI-H23 | 5.0 |
| astrocytoma SW1783 | 23.7 | Lung ca. (non-s.cell) HOP-62 | 10.7 |
| neuro*; met SK-N-AS | 12.9 | Lung ca. (non-s.cl)NCI-H522 | 46.3 |
| astrocytoma SF-539 | 5.4 | Lung ca. (squam.) SW 900 | 4.8 |
| astrocytoma SNB-75 | 9.4 | Lung ca. (squam.) NCI-H596 | 23.5 |
| glioma SNB-19 | 13.9 | Mammary gland | 16.5 |
| glioma U251 | 28.5 | Breast ca.* (pl.ef) MCF-7 | 1.5 |
| glioma SF-295 | 21.9 | Breast ca.* (pl.ef) MDA-MB-231 | 13.0 |
| Heart (fetal) | 21.9 | Breast ca.* (pl.ef) T47D | 35.6 |
| Heart | 9.7 | Breast ca.BT-549 | 5.1 |
| Skeletal muscle (fetal) | 13.3 | Breast ca. MDA-N | 12.0 |
| Skeletal muscle | 18.8 | Ovary | 8.1 |
| Bone marrow | 5.2 | Ovarian ca. OVCAR-3 | 10.2 |
| Thymus | 4.7 | Ovarian ca. OVCAR-4 | 15.3 |
| Spleen | 3.1 | Ovarian ca. OVCAR-5 | 46.0 |
| Lymph node | 6.5 | Ovarian ca. OVCAR-8 | 3.5 |
| Colorectal | 69.7 | Ovarian ca. IGROV-1 | 4.4 |
| Stomach | 8.7 | Ovarian ca.* (ascites) SK-OV-3 | 45.4 |
| Small intestine | 77.4 | Uterus | 2.6 |
| Colon ca. SW480 | 26.4 | Placenta | 0.6 |
| Colon ca.*SW620(SW480 met) | 47.3 | Prostate | 5.8 |
| Colon ca. HT29 | 47.0 | Prostate ca.* (bone met)PC-3 | 4.5 |
| Colon ca. HCT-116 | 14.0 | Testis | 7.9 |
| Colon ca. CaCo-2 | 100.0 | Melanoma Hs688(A).T | 0.9 |
| Colon ca. tissue(ODO3866) | 4.9 | Melanoma* (met) Hs688(B).T | 0.8 |
| Colon ca. HCC-2998 | 28.1 | Melanoma UACC-62 | 13.0 |
| Gastric ca.* (liver met) NCI-N87 | 18.6 | Melanoma M14 | 3.2 |
| Bladder | 5.3 | Melanoma LOX IMVI | 18.2 |
| Trachea | 1.9 | Melanoma* (met) SK-MEL-5 | 15.6 |
| Kidney | 27.2 | Adipose | 6.6 |

**Table MD. Panel 4D**

| **Column A - Rel. Exp.(%) Ag3204, Run 164389446** | | | |
|---|---|---|---|
| **Tissue Name** | **A** | **Tissue Name** | **A** |
| Secondary Th1 act | 5.0 | HUVEC IL-1beta | 1.2 |
| Secondary Th2 act | 3.3 | HUVEC IFN gamma | 2.0 |
| Secondary Tr1 act | 4.0 | HUVEC TNF alpha + IFN gamma | 1.0 |
| Secondary Th1 rest | 0.7 | HUVEC TNF alpha + IL4 | 2.9 |
| Secondary Th2 rest | 0.6 | HWEC IL-11 | 2.5 |
| Secondary Tr1 rest | 0.2 | Lung Microvascular EC none | 2.9 |
| Primary Th1 act | 7.4 | Lung Microvascular EC TNFalpha + IL-1beta | 2.4 |
| Primary Th2 act | 4.6 | Microvascular Dermal EC none | 3.6 |
| Primary Tr1 act | 5.4 | Microsvasular Dermal EC TNFaipba + IL-1beta | 2.8 |
| Primary Th1 rest | 1.9 | Bronchial epithelium TNFalpha + IL-1beta | 2.5 |
| Primary Th2 rest | 1.1 | Small airway epithelium none | 2.9 |
| Primary Tr1 rest | 1.8 | Small airway epithelium TNFalpha + IL-1beta | 8.8 |
| CD45RA CD4 lymphocyte act | 1.4 | Coronery artery SMC rest | 0.6 |
| CD45RO CD4 lymphocyte act | 3.6 | Coronery artery SMC TNFalpha + IL-1beta | 0.1 |
| CD8 lymphocyte act | 3.8 | Astrocytes rest | 4.8 |
| Secondary CD8 lymphocyte rest | 1.8 | Astrocytes TNFalpha + IL-lbeta | 3.6 |
| Secondary CD8 lymphocyte act | 2.5 | KU-812 (Basophil) rest | 6.6 |
| CD4 lymphocyte none | 0.6 | KU-812 (Basophil) PMA/ionomycin | 10.2 |
| 2ry Th1/Th2/Tr1_anti-CD95 CH11 | 0.8 | CCD1106 (Keratinocytes) none | 4.0 |
| LAK cells rest | 2.2 | CCD1106 (Keratinocytes) TNFalpha + IL-1beta | 1.8 |
| LAK cells IL-2 | 2.1 | Liver cirrhosis | 1.1 |
| LAK cells IL-2+IL-12 | 2.4 | Lupus kidney | 0.4 |
| LAK cells IL-2+IFN gamma | 2.9 | NCI-H292 none | 7.0 |
| LAK cells IL-2+ IL-18 | 2.4 | NCI-H292 IL-4 | 8.8 |
| LAK cells PMA/ionomycin | 1.2 | NCI-H292 IL-9 | 7.4 |
| NK Cells IL-2 rest | 1.4 | NCI-H292 IL-13 | 3.9 |
| Two Way MLR 3 day | 1.5 | NCI-H292 IFN gamma | 4.1 |
| Two Way MLR 5 day | 1.6 | HPAEC none | 1.8 |
| Two Way MLR 7 day | 1.4 | HPAEC TNF alpha + IL-1 beta | 2.0 |
| PBMC rest | 0.0 | Lung fibroblast none | 1.4 |
| PBMC PWM | 6.7 | Lung fibroblast TNF alpha + IL-1 beta | 0.6 |
| PBMC PHA-L | 3.4 | Lung fibroblast IL-4 | 2.4 |
| Ramos (B cell) none | 3.6 | Lung fibroblast IL-9 | 2.8 |
| Ramos (B cell) ionomycin | 10.7 | Lung fibroblast IL-13 | 1.7 |
| B lymphocytes PWM | 9.4 | Lung fibroblast IFN gamma | 1.6 |
| B lymphocytes CD40L and IL-4 | 2.1 | Dermal fibroblast CCD1070 rest | 3.8 |
| EOL-1 dbcAMP | 2.2 | Dermal fibroblast CCD1070 TNF alpha | 4.1 |
| EOL-1 dbcAMP PMA/ionomycin | 0.8 | Dermal fibroblast CCD1070 IL-1 beta | 0.4 |
| Dendritic cells none | 27.0 | Dermal fibroblast IFN gamma | 1.0 |
| Dendritic cells LPS | 28.9 | Dermal fibroblast IL-4 | 2.8 |
| Dendritic cells anti-CD40 | 41.8 | IBD Colitis 2 | 0.1 |
| Monocytes rest | 0.9 | IBD Crohn's | 6.0 |
| Monocytes LPS | 0.2 | Colon | **100.0** |
| Macrophages rest | 22.5 | Lung | 2.0 |
| Macrophages LPS | 0.8 | Thymus | 11.6 |
| HUVEC none | 2.8 | Kidney | 1.6 |
| HUVEC starved | 7.4 | | |

**CNS_neurodegeneration_v1.0 Summary:** Data obtained from RTQPCR assays performed for this panel was analyized using multivariate analysis (ANOVA). The multivariate results obtained analyzing Ag3204 shows this gene is found to be significantly (p = 0.0008) downregulated in the temporal cortex of Alzheimer's disease patients when compared to controls. A close homolog of this gene has been shown to mediate neurotoxicity via amyloid beta binding. This gene may therefore be an excellent drug target for the treatment of Alzheimer's disease, specifically for blocking amyloid beta induced neuronal death. Results from a second experiment with the same probe and primer are not included. The amp plot indicates there were experimental difficulties with this run.

### References:

He XY, et al. J Biol Chem 1998 Apr 24;273(17):10741-6
**Panel 1.3D Summary:** Ag3204 The CG57341-01 gene is expressed at a low level in most of the cancer cell lines and normal tissues. There appears to be significantly higher expression in colon, lung, breast and ovarian cancer cell lines with the highest expression shown by a colon cancer cell line (CT=30.94). Thus, therapeutic inhibition of this gene product, through the use of small molecule drugs, might be of utility in the treatment of the above listed cancer types.

Among tissues with metabolic function, this gene has low levels of expression in pancreas, thyroid, pituitary, adult and fetal heart, adult and fetal liver, adult and fetal skeletal muscle, and adipose. This gene product may be a small molecule target for the treatment of metabolic and endocrine disease, including the thyroidopathies, Types I and 2 diabetes and obesity.

**Panel 4D Summary:** Ag3204 The CG57341-01 transcript is expressed at significant levels in the colon and in some types of antigen presenting cells (APC'S) including activated dendritic cells, resting macrophages, and activated B cells. This pattern of expression suggests that the protein encoded by this transcript may be involved in gut immunity, particularly in the function or maintenance of APC's. This transcript encodes a putative reductase. Therefore, regulation of reductase expression could function by modulating gut immunity and be important in the treatment of inflammatory bowel diseases.

### N. CG57460-01: N-ACETYLTRANSFERASE CAMELLO 2

Expression of gene CG57460-01 was assessed using the primer-probe sets Ag3273 and Ag3322, described in Tables NA and NB. Results of the RTQ-PCR runs are shown in Tables NC, ND and NE.

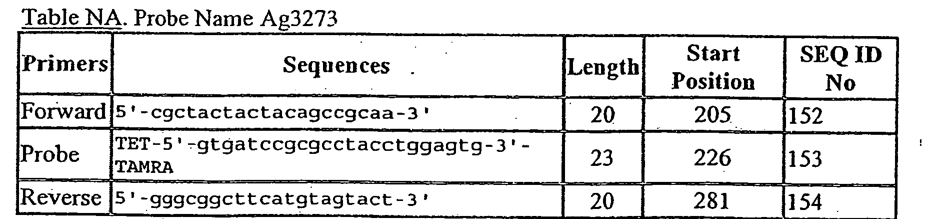

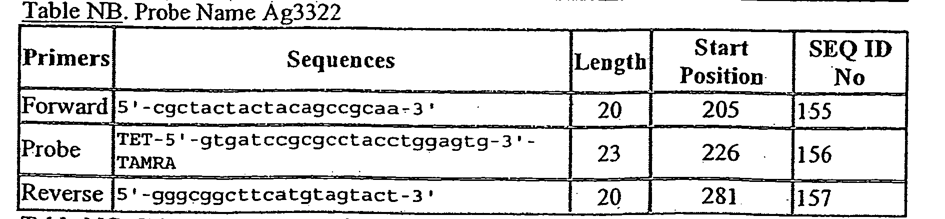

**Table NC. CNS_neurodegeneration_v1.0**

| **Column A - Rel. Exp.(%) Ag3273, Run 210038591** | | | | | |
|---|---|---|---|---|---|
| **Column B - Rel. Exp.(%) Ag3273, Run 230512515** | | | | | |
| **Tissue Name** | **A** | **B** | **Tissue Name** | **A** | **B** |
| AD 1 Hippo | 15.7 | 18.6 | Control (Path) 3 Temporal Ctx | 12.2 | 12.7 |
| AD 2 Hippo | 28.9 | 23.2 | Control (Path) 4 Temporal Ctx | 40.3 | 35.4 |
| AD 3 Hippo | 11.0 | 11.0 | AD 1 Occipital Ctx | 20.9 | 18.8 |
| AD 4 Hippo | 10.2 | 13.8 | AD 2 Occipital Ctx (Missing) | 0.0 | 0.0 |
| AD 5 hippo | 100.0 | 100.0 | AD 3 Occipital Ctx | 15.2 | 13.1 |
| AD 6 Hippo | 21.8 | 22.4 | AD 4 Occipital Ctx | 22.1 | 23.2 |
| Control 2 Hippo | 27.0 | 27.9 | AD 5 Occipital Ctx | 18.4 | 18.4 |
| Control 4 Hippo | 22.5 | 21.8 | AD 6 Occipital Ctx | 46.3 | 48.3 |
| Control (Path) 3 Hippo | 8.4 | 9.0 | Control 1 Occipital Ctx | 8.0 | 11.2 |
| AD 1 Temporal Ctx | 16.5 | 15.1 | Control 2 Occipital Ctx | 80.1 | 82.4 |
| AD 2 Temporal Ctx | 29.3 | 26.2 | Control 3 Occipital Ctx | 26.2 | 27.2 |
| AD 3 Temporal CtX | 10.9 | 12.9 | Control 4 Occipital Ctx | 14.8 | 14.1 |
| AD 4 Temporal Ctx | 22.5 | 20.3 | Control (Path) 1 Occipital Ctx | 70.2 | 68.8 |
| AD 5 Inf Temporal Ctx | 57.0 | 59.5 | Control (Path) 2 Occipital Ctx | 21.6 | 20.6 |
| AD 5 SupTemporal Ctx | 30.6 | 26.8 | Control (Path) 3 Occipital Ctx | 8.8 | 7.4 |
| AD 6 Inf Temporal Ctx | 27.2 | 30.1 | Control (Path) 4 Occipital Ctx | 36.1 | 34.2 |
| AD 6 Sup Temporal Ctx | 34.2 | 37.4 | Control 1 Parietal Ctx | 14.1 | 13.7 |
| Control 1 Temporal Ctx | 12.2 | 11.1 | Control 2 Parietal Ctx | 33.9 | 36.9 |
| Control 2 Temporal Ctx | 43.2 | 39.8 | Control 3 Parietal Ctx | 33.0 | 28.7 |
| Control 3 Temporal Ctx | 16.7 | 18.3 | Control (Path) 1 Parietal Ctx | 63.7 | 67.8 |
| Control 4 Temporal Ctx | 18.3 | 19.8 | Control (Path) 2 Parietal Ctx | 30.6 | 30.4 |
| Control (Path) 1 Temporal Ctx | 51.4 | 48.0 | Control (Path) 3 Parietal Ctx | 8.1 | 9.7 |
| Control (Path) 2 Temporal Ctx | 39.2 | 45.7 | Control (Path) 4 Parietal Ctx | 64.2 | 59.5 |

**Table ND. General screening panel v1.4**

| **Column A - Rel. Exp.(%) Ag3273, Run 215775405** | | | |
|---|---|---|---|
| **Tissue Name** | **A** | **Tissue Name** | **A** |
| Adipose | 7.1 | Renal ca. TK-10 | 4.9 |
| Melanoma* Hs688(A).T | 0.0 | Bladder | 0.9 |
| Melanoma* Hs688(B).T | 0.0 | Gastric ca. (liver met.) NCI-N87 | 2.4 |
| Melanoma* M14 | 17.6 | Gastric ca. KATO III | 0.0 |
| Melanoma* LOXIMVI | 0.9 | Colon ca. SW-948 | 0.0 |
| Melanoma* SK-MEL-5 | 4.2 | Colon ca. SW480 | 2.7 |
| Squamous cell carcinoma SCC-4 | 0.2 | Colon ca.* (SW480 met) SW620 | 1.0 |
| Testis Pool | 13.5 | Colon ca. HT29 | 0.1 |
| Prostate ca.* (bone met) PC-3 | 2.7 | Colon ca: HCT-116 | 6.0 |
| Prostate Pool | 0.2 | Colon ca. CaCo-2 | 2.0 |
| Placenta | 0.0 | Colon cancer tissue | 0.6 |
| Uterus Pool | 0.0 | Colon ca. SW1116 | 0.4 |
| Ovarian ca. OVCAR-3 | 5.4 | Colon ca. Colo-205 | 0.0 |
| Ovarian ca. SK-OV-3 | 6.8 | Colon ca. SW-48 | 0.0 |
| Ovarian ca. OVCAR-4 | 1.6 | Colon Pool | 0.4 |
| Ovarian ca. OVCAR-5 | 2.1 | Small Intestine Pool | 0.1 |
| Ovarian ca. IGROV-1 | 7.7 | Stomach Pool | 0.3 |
| Ovarian ca. OVCAR-8 | 9.6 | Bone Marrow Pool | 0.1 |
| Ovary | 0.8 | Fetal Heart | **100.0** |
| Breast ca. MCF-7 | 1.7 | Heart Pool | 0.2 |
| Breast ca. MDA-MB-231 | 0.8 | Lymph Node Pool | 0.0 |
| Breast ca. BT 549 | 7.7 | Fetal Skeletal Muscle | 0.3 |
| Breast ca. T47D | 9.3 | Skeletal Muscle Pool | 1.3 |
| Breast ca. MDA-N | 0.0 | Spleen Pool | 0.0 |
| Breast Pool | 0.4 | Thymus Pool | 0.4 |
| Trachea | 0.2 | CNS cancer (glio/astro) U87-MG | 0.0 |
| Lung | 0.0 | CNS cancer (glio/astro)U-118-MG | 0.0 |
| Fetal Lung | 0.3 | CNS cancer (neuro;met) SK-N-AS | 0.0 |
| Lung ca. NCI-N417 | 5.1 | CNS cancer (astro) SF-539 | 0.2 |
| Lung ca. LX-1 | 0.4 | CNS cancer (astro) SNB-75 | 0.6 |
| Lung ca. NCI-H146 | 12.2 | CNS cancer (glio) SNB-19 | 7.1 |
| Lung ca. SHP-77 | 2.4 | CNS cancer (glio) SF-295 | 1.8 |
| Lung ca. A549 | 3.4 | Brain (Amygdala) Pool | 31.4 |
| Lung ca. NCI-H526 | 12.0 | Brain (cerebellum) | 32.3 |
| Lung ca. NCI-H23 | 5.8 | Brain (fetal) | 9.3 |
| Lung ca. NCI-H460 | 2.3 | Brain (Hippocampus) Pool | 22.5 |
| Lung ca. HOP-62 | 1.0 | Cerebral Cortex Pool | 40.3 |
| Lung ca. NCl-H522 | 7.4 | Brain (Substantia nigra) Pool | 57.4 |
| Liver | 0.0 | Brain (Thalamus) Pool | 36.6 |
| Fetal Liver | 0.1 | Brain (whole) | 24.8 |
| Liver ca. HepG2 | 0.3 | Spinal Cord Pool | 20.2 |
| Kidney Pool | 0.2 | Adrenal Gland | 0.0 |
| Fetal Kidney | 0.7 | Pituitary gland Pool | 2.2 |
| Renal ca. 786-0 | 0.2 | Salivary Gland | 0.2 |
| Renal ca. A498 | 1.0 | Thyroid (female) | 1.1 |
| Renal ca. ACHN | 4.5 | Pancreatic ca. CAPAN2 | 0.0 |
| Renal ca. UO-31 | 3.7 | Pancreas Pool | 0.6 |

**Table NE. Panel 4D**

| **Column A - Rel. Exp.(%) Ag3273, Run 165338992** | | | |
|---|---|---|---|
| **Tissue Name** | **A** | **Tissue Name** | **A** |
| Secondary Th1 act | 1.0 | HUVEC IL-1beta | 0.0 |
| Secondary Th2 act | 2.5 | HUVEC IFN gamma | 0.0 |
| Secondary Tr1 act | 3.7 | HUVEC TNF alpha + IFN gamma | 0.0 |
| Secondary Th1 rest | 5.0 | HUVEC TNF alpha + IL4 | 0.0 |
| Secondary Th2 rest | 0.9 | HUVEC IL-11 | 0.0 |
| Secondary Tr1 rest | 6.9 | Lung Microvascular EC none | 0.0 |
| Primary Th1 act | 6.2 | Lung Microvascular EC TNFalpha + IL-1beta | 0.0 |
| Primary Th2 act | 5.9 | Microvascular Dermal EC none | 0.0 |
| Primary Tr1 act | 9.5 | Microsvasular Dermal EC TNFalpha + IL-1beta | 0.0 |
| Primary Th1 rest | 3.1 | Bronchial epithelium TNFalpha + IL1beta | 0.0 |
| Primary Th2 rest | 0.0 | Small airway epithelium none | 2.9 |
| Primary Tr1 rest | 0.0 | Small airway epithelium TNFalpha + IL-1beta | 0.0 |
| CD45RA CD4 lymphocyte act | 0.0 | Coronery artery SMC rest | 1.8 |
| CD45RO CD4 lymphocyte act | 0.0 | Coronery artery SMC TNFalpha + IL-1beta | 0.0 |
| CD8 lymphocyte act | 0.0 | Astrocytes rest | 16.5 |
| Secondary CD8 lymphocyte rest | 0.0 | Astrocytes TNFalpha + IL-1beta | 10.4 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 19.1 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 1.2 | Liver cirrhosis | 12.7 |
| LAK cells IL-2+IL-12 | 2.5 | Lupus kidney | 3.9 |
| LAK cells IL-2+IFN gamma | 4.1 | NCI-H292 none | 10.8 |
| LAK cells IL-2+IL-18 | 0.0 | NCI-H292 IL-4 | 23.2 |
| LAK cells PMA/ionomycin | 0.0 | NCI-H292 IL-9 | 22.4 |
| NK Cells IL-2 rest | 0.0 | NCI-H292 IL-13 | 10.3 |
| Two Way MLR 3 day | 1.1 | NCI-H292 IFN gamma | 18.0 |
| Two Way MLR 5 day | 0.0 | HPAEC none | 0.0 |
| Two Way MLR 7 day | 0.0 | HPAEC TNF alpha + IL-1 beta | 0.0 |
| PBMC rest | 0.0 | Lung fibroblast none | 1.9 |
| PBMC PWM | 1.2 | Lung fibroblast TNF alpha + IL-1 beta | 0.0 |
| PBMC PHA-L | 0.0 | Lung fibroblast IL-4 | 3.8 |
| Ramos (B cell) none | 0.0 | Lung fibroblast IL-9 | 0.5 |
| Ramos (B cell) ionomycin | 0.0 | Lung fibroblast IL-13 | 5.7 |
| B lymphocytes PWM | 3.5 | Lung fibroblast IFN gamma | 2.9 |
| B lymphocytes CD40L and IL-4 | 0.0 | Dermal fibroblast CCD1070 rest | 0.0 |
| EOL-1 dbcAMP | **100.0** | Dermal fibroblast CCD1070 TNF alpha | 0.0 |
| EOL-1 dbcAMP PMA/ionomycin | 25.9 | Dermal fibroblast CCD1070 IL-1 beta | 0.0 |
| Dendritic cells none | 3.8 | Dermal fibroblast IFN gamma | 2.8 |
| Dendritic cells LPS | 2.6 | Dermal fibroblast IL-4 | 4.8 |
| Dendritic cells anti-CD40 | 5.0 | IBD Colitis 2 | 0.0 |
| Monocytes rest | 0.0 | IBD Crohn's | 0.0 |
| Monocytes LPS | 0.0 | Colon | 37.4 |
| Macrophages rest | 4.2 | Lung | 8.4 |
| Macrophages LPS | 0.0 | Thymus | 40.9 |
| HUVEC none | 0.0 | Kidney | 6.3 |
| HUVEC starved | 0.0 | | |

**CNS_neurodegeneration_v1.0 Summary:** Data obtained from RTQPCR assays performed for this panel was analyized using multivariate analysis (ANOVA). The multivariate results obtained analyzing Ag3273 shows the two experiments with the same probe and primer set produced results that are in excellent agreement. This panel confirms the expression of this gene at low to moderate levels in the brains of an independent group of individuals. Expression of this gene is found to be down-regulated in the temporal cortex of Alzheimer's disease patients. Therefore, up-regulation of this gene or its protein product, or treatment with specific agonists for this protein, may be of use in reversing the dementia/memory loss associated with Alzheimer's disease and neuronal death.

**General_screening_panel_v1.4 Summary:** Ag3273 Highest expression of the CG57460-01 gene is seen in fetal heart (CT=28.6). In addition, this gene is expressed at much higher levels in fetal heart when compared to expression in the adult heart (CT=38). Thus, expression of this gene may be used to differentiate between disorders or predispositions that differ between the fetal and adult source of this tissue. In addition, the higher expression in fetal heart suggests that this protein product may be involved in the development of this organ. Therefore, therapeutic modulation of the expression or function of this gene may be useful in the treatment of heart disease.

In addition, expression of this gene appears to be upregulated in a number of cancer cell lines when compared to the normal tissues. Specifically, expression of this gene appears to be higher in ovarian, breast, lung and renal cancer cell lines when compared to their respective normal tissues. Therefore, therapeutic modulation of the activity of this gene or its protein product, using small molecule drugs, antibodies, or protein therapeutics; may be of benefit in the treatment of ovarian, breast, lung and renal cancer. The CG57460-01 gene encodes a transmembrane protein with homology to N-acetyltransferase Camello 2, a protein involved in cellular adhesion (ref. 1).

### References:

### Popsueva AE, et al. Dev Biol 2001 Jun 15;234(2):483-96 (PMID: 11397015)

**Panel 4D Summary:** Ag3273 Highest expression of the CG57460-01 is seen in eosinophils. In addition, differential expression is observed in the eosinophil cell line EOL-1 under resting conditions over that in EOL-1 cells stimulated by phorbol ester and ionomycin. Thus, this gene may be involved in eosinophil function. Therefore, therapeutic modulation of the expression or function of this gene may reduce eosinophil activation and be useful in the treatment of asthma and allergies.

In addition, significant expression in normal colon and thymus suggest a role for this gene in the normal homeostasis of these tissues. Therefore, therapeutic modulation of the expression or function of this gene may modulate immune function (T cell development) and be important for organ transplant, AIDS treatment or post chemotherapy immune reconstitiution. Furthermore, since expression of this gene is decreased in colon samples from patients with IBD colitis and Crohn's disease relative to normal colon, therapeutic modulation of the activity of the protein encoded by this gene may be useful in the treatment of inflammatory bowel disease.

### O. CG57570-01: Cation transporter

Expression of gene CG57570-01 was assessed using the primer-probe set Ag3288, described in Table OA. Results of the RTQ-PCR runs are shown in Tables OB, OC and OD.

**Table OA. Probe Name Ag3288**

| **Primers** | **Sequences** | **Length** | **Start Position** | **SEQ ID No** |
|---|---|---|---|---|
| Forward | 5'-tctacaccatcagctgtatgca-3' | 22 | 1380 | 158 |
| Probe | TET-5'-caccaccctcacactcatcttcatca-3'-TAMRA | 26 | 1411 | 159 |
| Reverse | 5'-gcagtgcagctgtcatatagaa-3' | 22 | 1439 | 160 |

**Table OB. CNS neurodegeneration, v1.0**

| **Column A - Rel. Exp.(%) Ag3288, Run 210058660** | | | | | |
|---|---|---|---|---|---|
| **Column B - Rel. Exp.(%) Ag3288, Run 229929907** | | | | | |
| **Tissue Name** | **A** | **B** | **Tissue Name** | **A** | **B** |
| AD 1 Hippo | 26.6 | 31.0 | Control (Path) 3 Temporal Ctx | 13.3 | 15.1 |
| AD 2 Hippo | 43.5 | 48.0 | Control (Path) 4 Temporal Ctx | 33.7 | 36.3 |
| AD 3 Hippo | 15.0 | 14.8 | AD 1 Occipital Ctx | 25.7 | 15.5 |
| AD 4 Hippo | 13.0 | 11.5 | AD 2 Occipital Ctx (Missing) | 0.0 | 0.0 |
| AD 5 hippo | 97.3 | 100.0 | AD 3 Occipital Ctx | 16.5 | 15.8 |
| AD 6 Hippo | **100.0** | 90.1 | AD 4 Occipital Ctx | 26.1 | 35.1 |
| Control 2 Hippo | 29.3 | 28.9 | AD 5 Occipital Ctx | 26.4 | 28.5 |
| Control 4 Hippo | 33.7 | 41.2 | AD 6 Occipital Ctx | 33.2 | 37.6 |
| Control (Path) 3 Hippo | 22.8 | 27.9 | Control 1 Occipital Ctx | 8.8 | 10.4 |
| AD 1 Temporal Ctx | 34.2 | 35.8 | Control 2 Occipital Ctx | 51.8 | 59.9 |
| AD 2 Temporal Ctx | 47.3 | 52.1 | Control 3 Occipital Ctx | 28.5 | 12.8 |
| AD 3 Temporal Ctx | 14.0 | 18.6 | Control 4 Occipital Ctx | 16.5 | 17.8 |
| AD 4 Temporal Ctx | 34.6 | 46.7 | Control (Path) 1 Occipital Ctx | 66.0 | 82.4 |
| AD 5 Inf Temporal Ctx | 80.1 | **100.0** | Control (Path) 2 Occipital Ctx | 11.3 | 14.4 |
| AD 5 SupTemporal Ctx | 64.2 | 85.9 | Control (Path) 3 Occipital Ctx | 9.5 | 8.5 |
| AD 6 Inf Temporal Ctx | 68.8 | 87.1 | Control (Path) 4 Occipital Ctx | 13.9 | 17.7 |
| AD 6 Sup Temporal Ctx | 66.4 | 87.1 | Control 1 Parietal Ctx | 11.1 | 15.8 |
| Control 1 Temporal Ctx | 11.3 | 16.2 | Control 2 Parietal Ctx | 48.0 | 66.0 |
| Control 2 Temporal Ctx | 33.0 | 36.3 | Control 3 Parietal Ctx | 17.0 | 22.2 |
| Control 3 Temporal Ctx | 19.2 | 18.9 | Control (Path) 1 Parietal Ctx | 55.5 | 66.4 |
| Control 4 Temporal Ctx | 14.7 | 17.8 | Control (Path) 2 Parietal Ctx | 30.6 | 34.2 |
| Control (Path) 1 Temporal Ctx | 32.3 | 50.7 | Control (Path) 3 Parietal Ctx | 14.3 | 11.3 |
| Control (Path) 2 Temporal Ctx | 36.9 | 13.2 | Control (Path) 4 Parietal Ctx | 37.1 | 43.2 |

**Table OC. General screening_panel_v1.4**

| **Column A - Rel. Exp.(%) Ag3288, Run 216516909** | | | |
|---|---|---|---|
| **Tissue Name** | **A** | **Tissue Name** | **A** |
| Adipose | 15.8 | Renal ca. TK-10 | 24.0 |
| Melanoma* Hs688(A).T | 38.4 | Bladder | 26.8 |
| Melanoma* Hs688(B).T | 51.4 | Gastric ca. (liver met.) NCI-N87 | 55.1 |
| Melanoma* M14 | 43.5 | Gastric ca. KATO III | 53.6 |
| Melanoma* LOXIMVI | 25.0 | Colon ca. SW-948 | 19.1 |
| Melanoma* SK-MEL-5 | 52.9 | Colon ca. SW480 | 41.2 |
| Squamous cell carcinoma SCC-4 | 16.5 | Colon ca.* (SW480 met) SW620 | 14.4 |
| Testis Pool | 44.4 | Colon ca. HT29 | 9.5 |
| Prostate ca.* (bone met) PC-3 | 57.4 | Colon ca. HCT-116 | 50.7 |
| Prostate Pool | 16.2 | Colon ca. CaCo-2 | 9.5 |
| Placenta | 4.3 | Colon cancer tissue | 7.3 |
| Uterus Pool | 4.9 | Colon.ca. SW1116 | 6.7 |
| Ovarian ca. OVCAR-3 | 37.9 | Colon ca. Colo-205 | 10.5 |
| Ovarian ca. SK-OV-3 | 39.0 | Colon ca. SW-48 | 7.3 |
| Ovarian ca. OVCAR-4 | 18.0 | Colon Pool | 15.9 |
| Ovarian ca. OVCAR-5 | 63.3 | Small Intestine Pool | 15.0 |
| Ovarian ca. IGROV-1 | 14.5 | Stomach Pool | 9.6 |
| Ovarian ca. OV CAR-8 | 12.1 | Bone Marrow Pool | 9.4 |
| Ovary | 17.2 | Fetal Heart | 72.7 |
| Breast ca. MCF-7 | 32.5 | Heart Pool | 66.9 |
| Breast ca. MDA-MB-231 | 47.3 | Lymph Node Pool | 19.3 |
| Breast ca. BT 549 | 58.2 | Fetal Skeletal Muscle | 17.6 |
| Breast ca. T47D | 73.7 | Skeletal Muscle Pool | 63.7 |
| Breast ca. MDA-N | 12.6 | Spleen Pool | 11.0 |
| Breast Pool | 14.6 | Thymus Pool | 7.5 |
| Trachea | 14.6 | CNS cancer (glio/astro) U87-MG | 30.8 |
| Lung | 7.5 | CNS cancer (glio/astro) U-118-MG | 44.1 |
| Fetal Lung | 63.7 | CNS cancer (neuro;met) SK-N-AS | 37.9 |
| Lung ca. NCI-N417 | 2.7 | CNS cancer (astro) SF-539 | 33.2 |
| Lung ca. LX-1 | 19.5 | CNS cancer (astro) SNB-75 | 34.6 |
| Lung ca. NCI-H146 | 4.9 | CNS cancer (glio) SNB-19 | 18.9 |
| Lung ca. SHP-77 | 12.0 | CNS cancer (glio) SF-295 | 64.2 |
| Lung ca. A549 | 24.8 | Brain (Amygdala) Pool | 13.4 |
| Lung ca. NCI-H526 | 8.8 | Brain (cerebellum) | 100.0 |
| Lung ca. NCI-H23 | 23.8 | Brain (fetal) | 28.5 |
| Lung ca. NCI-H460 | 29.5 | Brain (Hippocampus) Pool | 21.5 |
| Lung ca. HOP-62 | 23.0 | Cerebral Cortex Pool | 22.8 |
| Lung ca. NGI-H522 | 36.1 | Brain (Substantia nigra) Pool | 19.2 |
| Liver | 0.5 | Brain (Thalamus) Pool | 26.8 |
| Fetal Liver | 27.2 | Brain (whole) | 40.9 |
| Liver ca. HepG2 | 0.2 | Spinal Cord Pool | 19.8 |
| Kidney Pool | 23.0 | Adrenal Gland | 40.3 |
| Fetal Kidney | 21.2 | Pituitary gland Pool | 5.8 |
| Renal ca. 786-0 | 32.1 | Salivary Gland | 6.4 |
| Renal ca. A498 | 17.6 | Thyroid (female) | 17.2 |
| Renal ca. ACHN | 36.1 | Pancreatic ca. CAPAN2 | 14.8 |
| Renal ca. UO-31 | 21.0 | Pancreas Pool | 22.8 |

**Table OD. Panel 4D**

| **Column A - Rel. Exp.(%) Ag3288, Run 165007638** | | | |
|---|---|---|---|
| **Tissue Name** | **A** | **Tissue Name** | **A** |
| Secondary Th1 act | 15.0 | HUVEC -1beta 10.7 | IL |
| Secondary Th2 act | 15.8 | HUVEC IFN gamma | 9.3 |
| Secondary Tr1 act | 15.0 | HUVEC TNF alpha + IFN gamma | 20.9 |
| Secondary Th1 rest | 3.0 | HUVEC TNF alpha + IL4 | 14.9 |
| Secondary Th2 rest | 8.5 | HUVEC IL-11 | 6.6 |
| Secondary Tr1 rest | 4.4 | Lung Microvascular EC none | 9.3 |
| Primary Th1 act | 28.5 | Lung Microvascular EC TNFalpha + IL-1beta | 58.2 |
| Primary Th2 act | 19.5 | Microvascular Dermal EC none | 22.4 |
| Primary Tr1 act | 24.0 | Microsvasular Dermal EC TNFalpha + IL-1beta | 63.3 |
| Primary Th1 rest | 11.5 | Bronchial epithelium TNFalpha + IL-1beta | 20.6 |
| Primary Th2 rest | 8.5 | Small airway epithelium none | 3.8 |
| Primary Tr1 rest | 4.6 | Small airway epithelium TNFalpha + IL-1beta | 48.6 |
| CD45RA CD4 lymphocyte act | 12.2 | Coronery artery SMC rest | 11.7 |
| CD45RO CD4 lymphocyte act | 21.2 | Coronery artery SMC TNFalpha + IL-1beta | 9.1 |
| CD8 lymphocyte act | 14.2 | Astrocytes rest | 16.4 |
| Secondary CD8 lymphocyte rest | 22.1 | Astrocytes TNFalpha + IL-1beta | 12.9 |
| Secondary CD8 lymphocyte act | 10.8 | KU-812 (Basophil) rest | 16.8 |
| CD4 lymphocyte none | 5.1 | KU-812 (Basophil) PMA/ionomycin | 50.3 |
| 2ry Th1/Th2/Tr1 anti-CD95 CH11 | 4.0 | CCD1106 (Keratinocytes) none | 14.0 |
| LAK cells rest | 4.8 | CCD1106 (Keratinocytes) TNFalpha + IL-1beta | 10.8 |
| LAK cells IL-2 | 10.6 | Liver cirrhosis | 3.3 |
| LAK cells IL-2+IL-12 | 15.7 | Lupus kidney | 1.6 |
| LAK cells IL-2+IFN gamma | 19.3 | NCI-H292 none | 14.6 |
| LAK cells IL-2+ IL-18 | 14.2 | NCI-H292 IL-4 | 18.9 |
| LAK cells PMA/ionomycin | 5.7 | NCI-H292 IL-9 | 16.3 |
| NK Cells IL-2 rest | 7.9 | NCI-H292 IL-13 | 13.1 |
| Two Way MLR 3 day | 11.5 | NCI-H292 IFN gamma | 11.2 |
| Two Way MLR 5 day | 9.7 | HP AEC none | 11.5 |
| Two Way MLR 7 day | 6.7 | HPAEC TNF alpha + IL-1 beta | 31.0 |
| PBMC rest | 3.6 | Lung fibroblast none | 13.9 |
| PBMC PWM | 61.6 | Lung fibroblast TNF alpha + IL-1 beta | 13.6 |
| PBIVIC PHA-L | 31.6 | Lung fibroblast IL-4 | 28.1 |
| Ramos (B cell) none | 13.7 | Lung fibroblast IL-9 | 22.2 |
| Ramos (B cell) ionomycin | 94.6 | Lung fibroblast IL-13 | 13.5 |
| B lymphocytes PWM | 63.7 | Lung fibroblast IFN gamma | 23.7 |
| B lymphocytes CD40L and IL-4 | 100.0 | Dermal fibroblast CCD1070 rest | 30.4 |
| EOL-1 dbcAMP | 3.9 | Dermal fibroblast CCD1070 TNF alpha | 49.0 |
| EOL-1 dbcAMP PMA/ionomycin | 6.7 | Dermal fibroblast CCD1070 IL-1 beta | 15.6 |
| Dendritic cells none | 2.2 | Dermal fibroblast IFN gamma | 6.4 |
| Dendritic cells LPS | 1.9 | Dermal fibroblast IL-4 | 14.2 |
| Dendritic cells anti-CD40 | 0.8 | IBD Colitis 2 | 1.6 |
| Monocytes rest | 0.5 | IBD Crohn's | 0.9 |
| Monocytes LPS | 5.8 | Colon | 5.9 |
| Macrophages rest | 1.0 | Lung | 13.8 |
| Macrophages LPS | 7.2 | Thymus | 48.0 |
| HUVEC none | 10.6 | Kidney | 20.4 |
| HUVEC starved | 17.3 | | |

**CNS_neurodegeneration_v1.0 Summary:** Data obtained from RTOPCR assays performed for this panel was analyized using multivariate analysis (ANOVA). The multivariate results obtained analyzing Ag3288 shows results from two experiments using the same probe/primer set are in excellent agreement. This gene was found to upregulated in the temporal cortex of Alzheimer's disease (AD) patients (p = 0.0007 when analyzed by ANCOVA); the temporal cortex shows marked neuronal loss in the early to middle stages of AD. Upregulation of this gene expression, however, was not apparent in the occipital cortex, where neuronal degeneration does not occur in AD. Taken together, these data suggest that the protein encoded by this gene is involved in the pathologic process of Alzheimer's disease, making this an excellent small molecule drug target.

This gene encodes a protein with homology to cation transporters. For example, iron transporters in the brain have been shown to play an important role in age-related neurodegenerative diseases, including Parkinson's Disease, Alzheimer's disease, Huntington's disease and amyotrophic lateral sclerosis (Qian ZM, et al. Brain Res Brain Res Rev 1998 Aug;27(3):257-67).

**General_screening_panel_v1.4 Summary:** Ag3288 The CG57570-01 gene is expressed at high to moderate levels across almost all samples in this panel, with highest expression in the cerebellum (CT=26.7). This gene is also moderately expressed in all other regions of the CNS examined, including in amygdala, substantia nigra, thalamus, cerebral cortex, and spinal cord, suggesting that this gene may play a role in central nervous system disorders such as Alzheimer's disease, Parkinson's disease, epilepsy, multiple sclerosis, schizophrenia and depression.

This gene is also expressed in a number oftissues with metabolic or endocrine function, including adipose, adrenal gland, gastrointestinal tract, pancreas, skeletal muscle and thyroid. Therefore, therapeutic modulation of the activity of this gene may prove useful in the treatment of endocrine/metabolically related diseases, such as obesity and diabetes. Interestingly, this gene is differentially expressed in adult liver (CT = 34) vs fetal liver (CT = 29).

In addition, there is substantial expression of this gene associated with cancer cell lines. Therefore, therapeutic modulation of the activity of this gene or its protein product, through the use of small molecule drugs, protein therapeutics or antibodies, might be beneficial in the treatment of cancer.

**Panel 4D Summary:** Ag3288 This gene is expressed at high to moderate levels in a wide range of cell types of significance in the immune response and tissue response in health and disease, with the highest expression being detected in activated B cells (CT=25.4). Targeting this gene or its protein product with a small molecule drug or antibody therapeutic may modulate the functions of cells of the immune system and in particular of B cells as well as resident tissue cells and lead to improvement of the symptoms of patients suffering from rheumatoid diseases or B hyperglobulinemia and also other autoimmune disorders.

### P. CG57758-02: SODIUM/LITHIUM-DEPENDENT DICARBOXYLATE TRANSPORTER

Expression of gene CG57758-02 was assessed using the primer-probe sets Ag3326 and Ag3692, described in Tables PA and PB. Results of the RTQ-PCR runs are shown in Tables PC, PD, PE and PF.

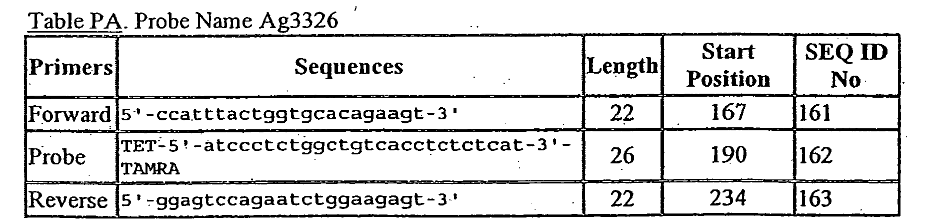

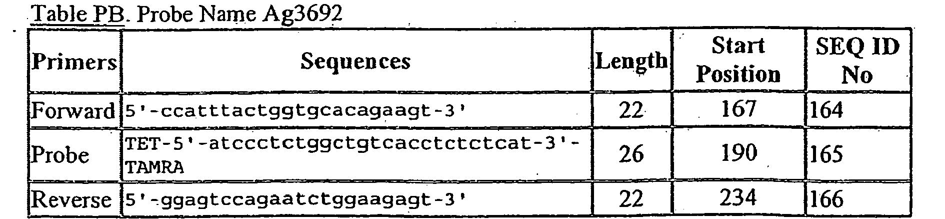

**Table PC. CNS neurodegeneration_v1.0**

| **Column A - Rel. Exp.(%) Ag3326, Run 210144197** |
|---|
| **Column B - Rel. Exp.(%) Ag3692, Run 211145262** |

| Column C - Rel. Exp.(%) Ag3692, Run 224337942 | | | | | | | |
|---|---|---|---|---|---|---|---|
| **Tissue Name** | **A** | **B** | **C** | **Tissue Name** | **A** | **B** | **C** |
| AD 1 Hippo | 2.1 | 4.3 | 1.0 | Control (Path) 3 Temporal Ctx | 8.5 | 15.3 | 12.0 |
| AD 2 Hippo | 20.9 | 28.3 | 25.0 | Control (Path) 4 Temporal Ctx | 31.2 | 36.6 | 52.1 |
| AD 3 Hippo | 0.0 | 0.9 | 0.6 | AD 1 Occipital Ctx | 2.7 | 3.0 | 0.0 |
| AD 4 Hippo | 2.1 | 7.1 | 2.6 | AD 2 Occipital Ctx (Missing) | 0.0 | 0.0 | 0.0 |
| AD 5 hippo | 72.7 | 97.9 | 85.3 | AD 3 Occipital Ctx | 1.5 | 7.2 | 1.3 |
| AD 6 Hippo | 13.7 | 18.3 | 5.5 | AD 4 Occipital Ctx | 71.7 | 35.6 | 30.6 |
| Control 2 Hippo | 14.5 | 20.2 | 15.2 | AD 5 Occipital Ctx | 25.3 | 31.9 | 12.4 |
| Control 4 Hippo | 11.7 | 7.4 | 5.1 | AD 6 Occipital Ctx | 17.2 | 19.1 | 11.2 |
| Control (Path) 3 Hippo | 6.7 | 4.4 | 4.5 | Control 1 Occipital Ctx | 7.0 | 9.0 | 8.1 |
| AD 1 Temporal Ctx | 4.0 | 1.7 | 2.8 | Control 2 Occipital Ctx | 33.2 | 44.8 | 26.1 |
| AD 2 Temporal Ctx | 80.7 | 50.7 | 37.4 | Control 3 Occipital Ctx | 30.1 | 37.6 | 21.9 |
| AD 3 Temporal Ctx | 3.6 | 0.0 | 1.1 | Control 4 Occipital Ctx | 16.3 | 12.6 | 8.2 |
| AD 4 Temporal Ctx | 19.5 | 30.6 | 15.2 | Control (Path) 1 Ctx 1 Occipital | 42.0 | 55.9 | 52.9 |
| AD 5 Inf Temporal Ctx | 100.0 | 100.0 | 99.3 | Control Ctx (Path) 2 Occipital | 6.7 | 13.0 | 7.7 |
| AD 5 SupTemporal Ctx | 32.8 | 29.1 | 33.2 | Control Ctx (Path) 3 Occipital | 8.7 | 6.6. | 5.4 |
| AD 6 Inf Temporal Ctx | 27.7 | 21.3 | 26.6 | Control Ctx (Path) 4 Occipital | 8.1 | 9.0 | 7.4 |
| AD 6 Sup Temporal Ctx | 41.8 | 53.6 | 17.0 | Control 1 Parietal Ctx | 21.2 | 23.0 | 15.3 |
| Control 1 Temporal Ctx | 12.0 | 33.9 | 18.3 | Control 2 Parietal Ctx | 48.6 | 38.2 | 22.1 |
| Control 2 Temporal Ctx | 30.1 | 49.3 | 44.4 | Control 3 Parietal Ctx | 28.3 | 34.4 | 32.8 |
| Control 3 Temporal Ctx | 38.7 | 39.5 | 33.4 | Control Ctx (Path) 1 Parietal | 78.5 | 97.3 | **100.0** |
| Control 4 Temporal Ctx | 17.6 | 25.2 | 24.1 | Control Ctx (Path) 2 Parietal | 50.7 | 50.7 | 37.9 |
| Control (Path) 1 Temporal Ctx | 69.7 | 70.7 | 49.7 | Control (Path) 3 Parietal Ctx | 10.7 | 10.1 | 9.6 |
| Control (Path) 2 Temporal Ctx | 35.4 | 50.7 | 33.4 | Control (Path) 4 Parietal Ctx | 30.6 | 24.5 | 40.9 |

**Table PD. General screening panel v1.4**

| **Column A - Rel. Exp.(%) Ag3326, Run 215678613** | | | | | |
|---|---|---|---|---|---|
| **Column B - Rel. Exp.(%) Ag3692, Run 217131191** | | | | | |
| **Tissue Name** | **A** | **B** | **Tissue Name** | **A** | **B** |
| Adipose | 0.0 | 0.0 | Renal ca. TK-10 | 11.4 | 12.0 |
| Melanoma* Hs688(A).T | 0.0 | 0.0 | Bladder | 0.0 | 0.1 |
| Melanoma* Hs688(B).T | 0.1 | 0.0 | Gastric ca. (liver met.) NCI-N87 | 0.0 | 0.0 |
| Melanoma* M14 | 0.0 | 0.0 | Gastric ca. KATO III | 0.0 | 0.0 |
| Melanoma* LOXIMVI Melanoma* LOXIMVI | 0.0 | 0.0 | Colon ca. SW-948 Colon ca. SW-948 | 0.0 | 0.0 |
| Melanoma* SK-MEL-5 | 0.0 | 0.0 | Colon ca. SW480 | 0.0 | 0.0 |
| Squamous cell carcinoma SCC-4 | 0.9 | 0.7 | Colon ca.* (SW480 met) SW620 | 0.0 | 0.0 |
| Testis Pool | 0.1 | 0.2 | Colon ca. HT29 | 0.0 | 0.0 |
| Prostate ca.* (bone met) PC-3 | 0.0 | 0.0 | Colon ca. HCT-116 | 0.0 | 0.0 |
| Prostate Pool | 0.0 | 0.0 | Colon ca. CaCo-2 | 0.0 | 0.0 |
| Placenta | 0.0 | 0.0 | Colon cancer tissue | 0.1 | 0.0 |
| Uterus Pool | 0.0 | 0.0 | Colon ca. SW 1116 | 0.0 | 0.0 |
| Ovarian ca. OVCAR-3 | 0.0 | 0.0 | Colon ca. Colo-205 | 0.0 | 0.0 |
| Ovarian ca. SK-OV-3 | 0.0 | 0.0 | Colon ca. SW-48 | 0.0 | 0.0 |
| Ovarian ca. OVCAR-4 | 0.1 | 0.0 | Colon Pool | 0.6 | 0.0 |
| Ovarian ca. OVCAR-5 | 0.0 | 0.0 | Small Intestine Pool | 0.1 | 0.0 |
| Ovarian ca. IGROV-1 | 0.0 | 0.0 | Stomach Pool | 0.0 | 0.0 |
| Ovarian ca. OVCAR-8 | 2.8 | 2.2 | Bone Marrow Pool | 0.0 | 0.1 |
| Ovary | 0.7 | 0.6 | Fetal Heart | 0.0 | 0.0 |
| Breast ca. MCF-7 | 0.0 | 0.0 | Heart Pool | 0.0 | 0.0 |
| Breast ca. MDA-MB-231 | 0.0 | 0.0 | Lymph Node Pool | 0.1 | 0.0 |
| Breast ca. BT 549 | 0.6 | 0.8 | Fetal Skeletal Muscle | 0.0 | 0.0 |
| Breast ca. T47D | 0.0 | 0.0 | Skeletal Muscle Pool | 0.0 | 0.0 |
| Breast ca. MDA-N | 0.0 | 0.0 | Spleen Pool | 0.4 | 0.2 |
| Breast Pool | 0.0 | 0.1 | Thymus Pool | 0.0 | 0.0 |
| Trachea | 0.2 | 0.1 | CNS cancer (glio/astro) U87-MG | 0.0 | 0.0 |
| Lung | 0.0 | 0.0 | CNS cancer (glio/astro) U-118- MG | 0.0 | 0.0 |
| Fetal Lung | 0.2 | 0.1 | CNS cancer (neuro;met) SK-N- AS | 0.0 | 0.0 |
| Lung ca. NCI-N417 | 0.0 | 0.0 | CNS cancer (astro) SF-539 | 0.0 | 0.0 |
| Lung ca. LX-1 | 0.0 | 0.0 | CNS cancer (astro) SNB-75 | 0.0 | 0.0 |
| Lung ca. NCI-H146 | 0.0 | 0.0 | CNS cancer (glio) SNB-19 | 0.0 | 0.0 |
| Lung ca. SHP-77 | 0.0 | 0.0 | CNS cancer (glio) SF-295 | 0.1 | 0.1 |
| Lung ca. A549 | 0.0 | 0.1 | Brain (Amygdala) Pool | 0.4 | 0.4 |
| Lung ca. NCI-H526 | 2.0 | 0.0 | Brain (cerebellum) | 1.4 | 1.0 |
| Lung ca. NCI-H23 | 0.7 | 0.6 | Brain (fetal) | 0.7 | 0.4 |
| Lung ca. NCI-H460 | 0.0 | 0.0 | Brain (Hippocampus) Pool | 0.5 | 0.7 |
| Lung ca. HOP-62 | 0.1 | 0.2 | Cerebral Cortex Pool | 1.4 | 1.5 |
| Lung ca. NCI-H522 | 0.0 | 0.0 | Brain (Substantia nigra) Pool | 1.4 | 1.4 |
| Liver | 28.7 | 24.1 | Brain (Thalamus) Pool | 1.1 | 0.9 |
| Fetal Liver | **100.0** | **100.0** | Brain (whole) | 4.1 | 3.7 |
| Liver ca. HepG2 | 29.5 | 26.2 | Spinal Cord Pool | 0.1 | 0.2 |
| Kidney Pool | 0.0 | 0.0 | Adrenal Gland | 2.6 | 1.9 |
| Fetal Kidney | 0.1 | 0.1 | Pituitary gland Pool | 0.0 | 0.2 |
| Renal ca. 786-0 | 0.0 | 0.0 | Salivary Gland | 40.9 | 35.1 |
| Renal ca. A498 | 0.0 | 0.0 | Thyroid (female) | 0.0 | 0.0 |
| Renal ca. ACHN | 0.0 | 0.0 | Pancreatic ca. CAPAN2 | 0.5 | 0.8 |
| Renal ca. UO-31 | 0.0 | 0.0 | Pancreas Pool | 0.0 | 0.0 |

**Table PE. Panel 4.1D**

| **Column A - Rel. Exp.(%) Ag3692, Run 169987356** | | | |
|---|---|---|---|
| **Tissue Name** | **A** | **Tissue Name** | **A** |
| Secondary Tn1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 11.3 |
| Primary Tr1 act | 4.2 | Microsvasular Dermal EC TNFalpha + IL-1beta | 0.0 |
| Primary Th1 rest | 0.0 | Bronchial epithelium TNFalpha+ IL1beta | 28.5 |
| Primary Th2 rest | 0.0 | Small airway epithelium none | 5.7 |
| Primary Tr1 rest | 0.0 | Small airway epithelium TNFalpha + IL-1beta | 0.0 |
| CD45RA CD4 lymphocyte act | 3.9 | Coronery artery SMC rest | 0.0 |
| CD4SRO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1 beta | 0.0 |
| Secondary CD8 lymphocyte act | 0.0 | KU-812 (Basophil) rest | 3.6 |
| CD4 lymphocyte none | 0.0 | KU-812 (Basophil) PMA/ionomycin | 4.3 |
| 2ry Th1/Th2/Tr1_anti-CD95 CH11 | 0.0 | CCD1106 (Keratinocytes) none | 10.7 |
| LAK cells rest | 0.0 | CCD1106 (Keratinocytes) TNFalpha + IL-1beta | 0.0 |
| LAK cells IL-2 | 0.0 | Liver cirrhosis | 94.0 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 3.2 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.0 |
| PBMCPWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD1070 TNF alpha | 0.0 |
| BuL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 0.0 |
| Macrophages rest | 0.0 | Lung | 0.0 |
| Macrophages LPS | 0.0 | Thymus | 2.4 |
| HUVEC none | 0.0 | Kidney | **100.0** |
| HUVEC starved | 0.0 | | |

**Table PF. Panel 5 Islet**

| **Column A - Rel. Exp.(%) Ag3326, Run 242385365** | | | |
|---|---|---|---|
| **Tissue Name** | **A** | **Tissue Name** | **A** |
| 97457_Patient-02 go_adipose | 0.0 | 94709_Donor 2 AM - A_adipose | 0.2 |
| 97476_Patient-07sk skeletal muscle | 0.0 | 94710_Donor 2 AM - B_adipose | 0.0 |
| 97477_Patient-07ut_uterus | 0.0 | 94711_Donor 2 AM - C_adipose | 0.0 |
| 97478_Patient-07pl_placenta | 0.0 | 94712_Donor 2 AD - A_adipose | 0.0 |
| 99167_Bayer Patient 1 | 0.3 | 94713_Donor 2 AD - B_adipose | 0.0 |
| 97482_Patient-08ut_uterus | 0.0 | 94714_Donor 2 AD - C_adipose | 0.0 |
| 97483_Patient-08p1_placenta | 0.0 | 94742_Donor 3 U - A_Mesenchymal Stem Cells | 0.0 |
| 97486_Patient-09sk_skeletal muscle | 0.0 | 94743_Donor 3 U - B_Mesenchymal Stem Cells | 0.0 |
| 97487_Patient-09ut_uterus | 0.0 | 94730_Donor 3 AM - A_adipose | 0.0 |
| 97488_Patient-09pl_placenta | 0.0 | 94731_Donor 3 AM - B_adipose | 0.0 |
| 97492_Patient-10ut_uterus | 0.0 | 94732_Donor 3 AM - C_adipose | 0.0 |
| 97493_Patient-10pl_placenta | 0.0 | 94733_Donor 3 AD - A_adipose | 0.0 |
| 97495_Patient-11go_adipose | 0.0 | 94734_Donor 3 AD - B_adipose | 0.0 |
| 97496_Patient-11sk_skeletal muscle | 0.0 | 94735_Donor 3 AD - C_adipose | 0.0 |
| 97497_Patient-11ut_uterus | 0.0 | 77138_Liver_HepG2untreated | **100.0** |
| 97498 _Patient-11pl_placenta | 0.0 | 73556_Heart_Cardiac stromal cells (primary) | 0.0 |
| 97500_Patient-12go_adipose | 0.1 | 81735_Small Intestine | 39.5 |
| 97501_Patient-12sk_skeletal muscle | 0.3 | 72409_Kindney_Proximal Convoluted Tubule | 0.0 |
| 97502_Patient-12ut_uterus | 0.0 | 82685_Small intestine_Duodenum | 0.0 |
| 97503_Patient-12pl_placenta | 0.0 | 90650_Adrenal_Adrenocortical adenoma | 0.0 |
| 94721_Donor 2 U - A_Mesenchymal Stem Cells | 0.0 | 72410 Kidney_HRCE | 0.0 |
| 94722 Donor 2 U - B_Mesenchymal Stem Cells | 0.0 | 72411_Kidney_HRE | 0.0 |
| 94723_Donor 2 U - C_Mesenchymal Stem Cells | 0.0 | 73139_Uterus_Uterine smooth muscle cells | 0.0 |

**CNS_neurodegen eration_v1.0 Summary:** Data obtained from RTQPCR assays performed for this panel was analyized using multivariate analysis (ANOVA). The multivariate results obtained analyzing Ag3326/Ag3692 shows three experiments tested with two primer pairs (same sequence) are in excellent agreement. This gene is found to be down-regulated in the temporal cortex of Alzheimer's disease patients. Therefore, up-regulation of this gene or its protein product, or treatment with specific agonists for this gene may be of use in reversing the dementia/memory loss associated with this disease and neuronal death.

**General_screeoing_panel_v1.4 Summary:** Ag3326/Ag3692 Two experiments with the same probe and primer set produce results that are in excellent agreement. This gene is highly expressed in fetal liver (CT=26.5-27.0) and moderately expressed in adult liver and liver cancer cell line HepG2. This result agrees with the results seen in Panel 5 (expression in HepG2). These results are in agreement with published data that show a novel sodium dicarboxylate transporter in brain, choroid plexus kidney, intestine and liver (Chen XZ, et al. J Biol Chem 1998 Aug 14;273(33):20972-81; Pajor AM, et al. Am J Physiol Cell Physiol 2001 May;280(5):C1215-23).

This gene is expressed at low levels throughout the CNS, including in amygdala, substantia nigra, thalamus, cerebellum, and cerebral cortex. Therefore, this gene may play a role in central nervous system disorders such as Parkinson's disease, epilepsy, multiple sclerosis, schizophrenia and depression.

Low but significant levels of expression are also seen in the adrenal gland. Thus, this gene product may also be involved in metabolic disorders of this gland, including adrenoleukodystrophy and congenital adrenal hyperplasia.

**Panel 4.1D Summary:** Ag3692 Significant expression of this gene is seen only in kidney and a liver cirrhosis samples in this panel (CTs=34.0). These results confirm that this gene is expressed in liver derived samples.

Panel 5 Islet Summary: Ag3326 - The highest expression of this gene is in liver cancer cell line HepG2 (CT=29.2). There is also moderate expression in the small intestine (CT=30.5).

### Q. CG59693-01: 20 alpha-hydroxysteroid dehydrogenase

Expression of gene CG59693-01 was assessed using the primer-probe set Ag3562, described in Table QA. Results of the RTQ-PCR runs are shown in Tables QB, QC, QD, QE, QF, QG, QH, QI and QJ.

**Table QA. Probe Name Ag3562**

| **Primers** | **Sequences** | **Length** | **Start Position** | **SEQ ID No** |
|---|---|---|---|---|
| Forward | 5'-ctggccaagagctacaatga-3' | 20 | 802 | 168 |
| Probe | TET-5'-catcagacagaacgtgcaggtgtttg-3'- | 26 | 828 | 169 |
| Reverse | 5'-aggccatctatggctttcat-3' | 20 | 877 | 170 |

**Table OB. Ardais Panel v.1.0**

| **Column A - Rel. Exp.(%) Ag3562, Run 263525399** | | | |
|---|---|---|---|
| **Tissue Name** | **A** | **Tissue Name** | **A** |
| 136799_Lung cancer(362) | 52.9 | 136787_lung cancer(356) | 0.1 |
| 136800_Lung NAT(363) | 0.8 | 136788_lung NAT(357) | 1.1 |
| 136813_Lung cancer(372) | **100.0** | 136806_Lung cancer(36B) | 0.1 |
| 136814_LungNAT(373) | 0.4 | 136807_LungNAT(36C) | 0.4 |
| 136815_Lung cancer(374) | 1.1 | 136789_lung cancer(358) | 0.4 |
| 136816_Lung NAT(375) | 1.6 | 136802_Lung cancer(365) | 1.6 |
| 136791_Lung cancer(35A) | 0.4 | 136803_Lung cancer(368) | 0.5 |
| 136795_Lung cancer(35E) | 1.6 | 136804_Lung cancer(369) | 1.4 |
| 136797_Lung cancer(360) | 0.4 | 136811_Lung cancer(370) | 64.2 |
| 136794_lung NAT(35D) | 1.2 | 136810_LungNAT(36F) | 3.9 |
| 136818_Lung NAT(377) | 0.5 | | |

**Table QC. CNS_neurodegencration v1.0**

| **Column A - Rel. Exp.(%) Ag3562, Run 210629741** | | | | | |
|---|---|---|---|---|---|
| **Column B - Rel. Exp.(%) Ag3562, Run 224078542** | | | | | |
| **Tissue Name** | **A** | **B** | **Tissue Name** | **A** | **B** |
| AD 1 Hippo | 47.3 | 54.7 | Control (Path) 3 Temporal Ctx | 5.0 | 8.5 |
| AD 2 Hippo | 30.1 | 31.2 | Control (Path) 4 Temporal Ctx | 18.9 | 20.6 |
| AD 3 Hippo | 9.9 | 14.6 | AD 1 Occipital Ctx | 23.0 | 41.5 |
| AD 4 Hippo | 10.4 | 11.6 | AD 2 Occipital Ctx (Missing) | 0.0 | 0.0 |
| AD 5 Hippo | 57.4 | 74.2 | AD 3 Occipital Ctx | 10.4 | 10.2 |
| AD 6 Hippo | **100.0** | **100.0** | AD 4 Occipital Ctx | 13.1 | 8.2 |
| Control 2 Hippo | 22.7 | 27.7 | AD 5 Occipital Ctx | 24.5 | 29.3 |
| Control 4 Hippo | 15.7 | 20.6 | AD 6 Occipital Ctx | 24.8 | 35.4 |
| Control (Path) 3 Hippo | 8.2 | 8.3 | Control 1 Occipital Ctx | 4.7 | 5.0 |
| AD 1 Temporal Ctx | 33.2 | 42.6 | Control 2 Occipital Ctx | 18.0 | 29.9 |
| AD 2 Temporal Ctx | 28.3 | 28.5 | Control 3 Occipital Ctx | 11.0 | 13.6 |
| AD 3 Temporal Ctx | 8.3 | 10.4 | Control 4 Occipital Ctx | 7.3 | 9.2 |
| AD 4 Temporal Ctx | 15.5 | 18.8 | Control (Path) I Occipital Ctx | 32.3 | 31.0 |
| AD 5 Inf Temporal Ctx | 55.1 | 83.5 | Control (Path) 2 Occipital Ctx | 5.2 | 8.0 |
| AD 5 Sup Temporal Ctx | 64.6 | 74.2 | Control (Path) 3 Occipital Ctx | 7.6 | 5.6 |
| AD 6 Inf Temporal Ctx | 58.2 | 68.3 | Control (Path) 4 Occipital Ctx | 10.5 | 12.5 |
| AD 6 Sup Temporal Ctx | 59.5 | 70.7 | Control 1 Parietal Ctx | 6.1 | 8.1 |
| Control 1 Temporal Ctx | 7.6 | 9.2 | Control 2 Parietal Ctx | 66.0 | 67.8 |
| Control 2 Temporal Ctx | 15.2 | 27.7 | Control 3 Parietal Ctx | 19.1 | 18.3 |
| Control 3 Temporal Ctx | 14.1 | 12.3 | Control (Path) 1 Parietal Ctx | 29.9 | 35.6 |
| Control 3 Temporal Ctx | 7.7 | 8.3 | Control (Path) 2 Parietal Ctx | 11.5 | 13.0 |
| Control (Path) 1 Temporal Ctx | 24.5 | 29.1 | Control (Path) 3 Parietal Ctx | 5.9 | 8.1 |
| Control (Path) 2 Temporal Ctx | 15.3 | 17.6 | Control (Path) 4 Parietal Ctx | 23.2 | 28.1 |

**Table QD. General_screening_panel v1.4**

| **Column A - Rel. Exp.(%) Ag3562, Run 217240778** | | | |
|---|---|---|---|
| Tissue Name | A | Tissue Name | A |
| Adipose | 2.1 | Renal ca. TK-10 | 1.5 |
| Melanoma* Hs688(A).T | 0.2 | Bladder | 0.8 |
| Melanoma* Hs688(B).T | 0.3 | Gastric ca. (liver met.) NCI-N87 | 0.1 |
| Melanoma* M14 | 0.0 | Gastric ca. KATO III | 0.4 |
| Melanoma* LOXIMVI | 0.0 | Colon ca. SW-948 | 0.6 |
| Melanoma* SK-MEL-5 | 0.0 | Colon ca. SW480 | 0.1 |
| Squamous cell carcinoma SCC-4 | 0.5 | Colon ca.* (SW480 met) SW620 | 0.5 |
| Testis Pool | 0.2 | Colon ca. HT29. | 0.5 |
| Prostate ca.* (bone met) PC-3 | 0.0 | Colon-ca. HCT-116 | 0.0 |
| Prostate Pool | 0.1 | Colon ca. CaCo-2 | 2.6 |
| Placenta | 0.0 | Colon cancer tissue | 0.4 |
| Uterus Pool | 0.1 | Colon ca. SW1116 | 0.0 |
| Ovarian ca. OVCAR-3 | 0.0 | Colon ca. Colo-205 | 3.0 |
| Ovarian ca. SK-OV-3 | 11.8 | Colon ca. SW-48 | 0.9 |
| Ovarian ca. OVCAR-4 | 0.0 | Colon Pool | 0.1 |
| Ovarian ca. OVCAR-5 | 0.3 | Small Intestine Pool | 0.1 |
| Ovarian ca. IGROV-1 | 0.5 | Stomach Pool | 0.1 |
| Ovarian ca. OVCAR-8 | 0.2 | Bone Marrow Pool | 0.1 |
| Ovary | 0.2 | Fetal Heart | 0.0 |
| Breast ca. MCF-7 | 0.5 | Heart Pool | 0.1 |
| Breast ca. MDA-MB-231 | 1.1 | Lymph Node Pool | 0.2 |
| Breast ca. BT 549 | 1.2 | Fetal Skeletal Muscle | 0.1 |
| Breast ca. T47D | 0.6 | Skeletal Muscle Pool | 0.7 |
| Breast ca. MDA-N | 0.0 | Spleen Pool | 0.0 |
| Breast Pool | 0.1 | Thymus Pool | 0.2 |
| Trachea | 1.3 | CNS cancer (glio/astro) U87-MG | 0.9 |
| Lung | 0.2 | CNS cancer (glio/asho) U-118-MG | 1.0 |
| Fetal Lung | 0.3 | CNS cancer (neuro;met) SK-N-AS | 0.3 |
| Lung ca. NCI-N417 | 0.0 | CNS cancer (astro) SF-539 | 0.1 |
| Lung ca. LX-1 | 1.6 | CNS cancer (astro) SNB-75 | 10.2 |
| Lung ca. NCI-H146 | 1.0 | CNS cancer (glio) SNB-19 | 0.4 |
| Lung ca. SHP-77 | 14.7 | CNS cancer (glio) SF-295 | 4.0 |
| Lung ca. A549 | **100.0** | Brain (Amygdala) Pool | 0.2 |
| Lung ca. NCI-H526 | 0.0 | Brain (cerebellum) | 0.1 |
| Lung ca. NCI-H23 | 0.2 | Brain (fetal) | 0.5 |
| Lung ca. NCI-H460 | 11.1 | Brain (Hippocampus) Pool | 0.1 |
| Lung ca. HOP-62 | 0.1 | Cerebral Cortex Pool | 0.1 |
| Lung ca. NCl-R522 | 0.6 | Brain (Substantia nigra) Pool | 0.2 |
| Liver | 0.7 | Brain (Thalamus) Pool | 0.2 |
| Fetal Liver | 4.1 | Brain (whole) | 0.4 |
| Liver ca. HepG2 | 2.4 | Spinal Cord Pool | 0.3 |
| Kidney Pool | 0.2 | Adrenal Gland | 0.2 |
| Fetal Kidney | 0.1 | Pituitary gland Pool | 0.0 |
| Renal ca. 786-0 | 0.3 | Salivary Gland | 0.1 |
| Renal ca. A498 | 11.2 | Thyroid (female) | 0.1 |
| Renal ca. ACHN | 0.2 | Pancreatic ca. CAPAN2 | 0.2 |
| Renal ca. UO-31 | 0.1 | Pancreas Pool | 0.2 |

**Table QE. General screening_panel_v1.6**

| **Column A - Rel. Exp.(%) Ag3562, Run 277245085** | | | |
|---|---|---|---|
| **Tissue Name** | **A** | **Tissue Name** | **A** |
| Adipose | 0.3 | Renal ca. TK-10 , | 1.5 |
| Melanoma* Hs688(A).T | 0.3 | Bladder | 0.8 |
| Melanoma* Hs688(B).T | 0.3 | Gastric ca. (liver met.) NCI-N87 | 0.1 |
| Melanoma* M14 | 0.0 | Gastric ca. KATO III | 0.3 |
| Melanoma* LOXIMVI | 0.0 | Colon ca. SW-948 | 0.5 |
| Melanoma* SK-MEL-5 | 0.0 | Colon ca. SW480 | 0.0 |
| Squamous cell carcinoma SCC-4 | 0.5 | Colon ca.* (SW480 met) SW620 | 0.6 |
| Testis Pool | 0.2 | Colon ca. HT29 | 0.6 |
| Prostate ca.* (bone met) PC-3 | 0.0 | Colon ca. HCT-116 | 0.0 |
| Prostate Pool | 0.1 | Colon ca. CaCo-2 | 2.4 |
| Placenta | 0.0 | Colon cancer tissue | 0.4 |
| Uterus Pool | 0.1 | Colon ca. SW 1116 | 0.0 |
| Ovarian ca. OVCAR-3 | 0.0 | Colon ca. Colo-205 | 4.7 |
| Ovarian ca. SK-OV-3 | 14.7 | Colon ca. SW-48 | 0.9 |
| Ovarian ca. OVCAR-4 | 0.0 | Colon Pool | 0.1 |
| Ovarian ca. OVCAR-5 | 0.3 | Small Intestine Pool | 0.1 |
| Ovarian ca. IGROV-1 | 0.4 | Stomach Pool | 0.1 |
| Ovarian ca. OVCAR-8 | 0.2 | Bone Marrow Pool | 0.0 |
| Ovary | 0.2 | Fetal Heart | 0.1 |
| Breast ca. MCF-7 | 0.4 | Heart Pool | 0.1 |
| Breast ca. MDA-MB-231 | 1.1 | Lymph Node Pool | 0.2 |
| Breast ca. BT 549 | 1.0 | Fetal Skeletal Muscle | 0.0 |
| Breast ca. T47D | 0.0 | Skeletal Muscle Pool | 0.2 |
| Breast ca. MDA-N | 0.0 | Spleen Pool | 0.0 |
| Breast Pool | 0.1 | Thymus Pool | 0.2 |
| Trachea | 1.3 | CNS cancer (glio/astro) U87=MG | 4.7 |
| Lung | 0.1 | CNS cancer (glio/astro) U-118-MG | 1.0 |
| Fetal Lung | 0.3 | CNS cancer (neuro;met) SK-N-AS | 0.3 |
| Lung ca. NCI-N417 | 0.0 | CNS cancer (astro) SF-539 | 0.1 |
| Lung ca. LX-1 | 1.6 | CNS cancer (astro) SNB-75 | 9.5 |
| Lung ca. NCI-H146 | 0.8 | CNS cancer (glio) SNB-19 | 0.4 |
| Lung ca. SHP-77 | 13.9 | CNS cancer (glio) SF-295 | 3.4 |
| Lung ca. A549 | 100.0 | Brain (Amygdala) Pool | 0.1 |
| Lung ca. NCI-H526 | 0.0 | Brain (cerebellum) | 0.2 |
| Lung ca. NCI-H23 | 0.2 | Brain (fetal) | 0.4 |
| Lung ca. NCI-H460 | 13.7 | Brain (Hippocampus) Pool | 0.1 |
| Lung ca. HOP-62 | 0.0 | Cerebral Cortex Pool | 0.1 |
| Lung ca. NCI-H522 | 0.7 | Brain (Substantia_nigra) Pool | 0.1 |
| Liver | 0.6 | Brain (Thalamus) Pool. | 0.2 |
| Fetal Liver | 4.6 | Brain (whole) | 0.4 |
| Liver ca. HepG2 | 1.9 | Spinal Cord Pool | 0.4 |
| Kidney Pool | 0.2 | Adrenal Gland | 0.2 |
| Fetal Kidney | 0.1 | Pituitary gland Pool | 0.0 |
| Renal ca. 786-0 | 0.3 | Salivary Gland | 0.1 |
| Renal ca. A498 | 11.3 | Thyroid (female) | 0.1 |
| Renal ca. ACHN | 0.2 | Pancreatic ca. CAPAN2 | 0.1 |
| Renal ca. UO-31 | 0.1 | Pancreas Pool | 0.1 |

**Table OF. HASS Panel v1.0**

| **Column A - Rel. Exp.(%) Ag3562, Run 276044499** | | | |
|---|---|---|---|
| **Tissue Name** | **A** | **Tissue Name** | **A** |
| MCF-7 C1 | 8.5 | U87-MG F1 (B) | 19.6 |
| MCF-7 C2 | 12.0 | U87-MG F2 | 5.2 |
| MCF-7 C3 | 15.7 | U87-MGF3 | 15.8 |
| MCF-7 C4 | 13.2 | U87-MG F4 | 10.2 |
| MCF-7 C5 | 20.2 | U87-MG F5 | 40.3 |
| MCF-7 C6 | 10.4 | U87-MG F6 | 92.7 |
| MCF-7 C7 | 8.8 | U87-MG F7 | 5.9 |
| MCF-7 C9 | 6.9 | U87-MG F8 . | 9.1 |
| MCF-7 C10 | 10.2 | U87-MG F9 | 1.9 |
| MCF-7 C11 | 5.8 | U87-MG F10 | 57.4 |
| MCF-7 C12 | 8.7 | U87-MG F11 | **100.0** |
| MCF-7 C13 | 7.6 | U87-MG F12 | 30.4 |
| MCF-7 C15 | 3.6 | U87-MG F13 | 10.2 |
| MCF-7 C16 | 13.2 | U87-MG F14 | 16.7 |
| MCF-7 C17 | 9.8 | U87-MG F15 | 9.5 |
| T24 D1 | 32.8 | U87-MG F16 | 66.4 |
| T24 D2 | 12.2 | U87-MG F17 | 67.4 |
| T24 D3 | 26.4 | LnCAP A1 | 0.2 |
| T24 D4 | 48.3 | LnCAP A2 | 0.2 |
| T24 D5 | 18.2 | LnCAP A3 | 0.1 |
| T24 D6 | 0.2 | LnCAP A4 | 0.1 |
| T24 D7 | 1.0 | LnCAP A5 | 0.2 |
| T24 D9 | 0.0 | LnCAP A6 | 0.2 |
| T24 D10 | 23.7 | LnCAP A7 | 0.4 |
| T24 D11 | 2.7 | LnCAP A8 | 0.6 |
| T24 D12 | 0.1 | LnCAP A9 | 0.8 |
| T24 D13 | 0.3 | LnCAP A10 | 0.2 |
| T24 D15 | 0.3 | LnCAP A11 | 0.4 |
| T24 D16 | 0.2 | LnCAP A12 | 0.1 |
| T24 D17 | 0.5 | LnCAP A13 | 0.0 |
| CAPaN B1 | 4.4 | LnCAP A14 | 0.2 |
| CAPaN B2 | 6.3 | LnCAP A1 5 | 0.3 |
| CAPaN B3 | 4.3 | LnCAP A16 | 0.2 |
| CAPaN B4 | 3.4 | LnCAP A17 | 0.5 |
| CAPaN B5 | 14.9 | Primary Astrocytes | 0.4 |
| CAPaN B6 | 2.1 | Primary Renal Proximal Tubule Epithelial cell A2 | 25.0 |
| CAPaN B7 | 1.8 | Primary melanocytes A5 | 1.7 |
| CAPaN B8 | 10.7 | 126443 - 341 medullo | 0.1 |
| CAPaN B9 | 2.6 | 126444 - 487 medullo | 0.0 |
| CAPaN B10 | 6.4 | 126445 - 425 medullo | 0.0 |
| CAPaN B11 | 16.3 | 126446 - 690 medullo | 0.2 |
| CAPaN B12 | 1.7 | 126447 - 54 adult glioma | 3.8 |
| CAPaN B13 | 1.3 | 126448 - 245 adult glioma | 0.0 |
| CAPaN B14 | 7.4 | 126449 - 317 adult glioma | 0.0 |
| CAPaN B15 5 | 1.2 | 126450 - 212 glioma | 0.1 |
| CAPaN B16 | 4.2 | 126451 - 456 glioma | 0.0 |
| CAPaN B17 | 4.2 | | |

**Table QG. Oncology_cell_ litie_screening_panel_v3.1**

| **Column A - Rel. Exp.(%) Ag3562, Run 222546381** | | | |
|---|---|---|---|
| **Tissue Name** | **A** | **Tissue Name** | **A** |
| Daoy Medulloblastoma/Cerebellum | 0.0 | Ca Ski Cervical epidermoid carcinoma (metastasis) | 0.3 |
| TE671 Medulloblastom/Cerbbellum | 0.2 | ES-2_Ovarian clear cell carcinoma | 0.0 |
| D283 Med Medulloblastoma/Cerebellum | 0.0 | Ramos/6h stim_Stimulated with PMA/ionomycin 6h | 0.0 |
| PFSK-1 Primitive Neuroectodermal/Cerebellum | 0.1 | Ramos/14h stim_Stimulated with PMA/ionomycin 14h | 0.0 |
| XF-498_CNS | 0.0 | MEG-0.1_Chromcmyelogenous leukemia (megokaryoblast) | 0.1 |
| SNB-78_CNS/glioma | 0.3 | Raji_Burkitt's lymphoma | 0.0 |
| SF-268_CNS/glioblastoma | 0.0 | Daudi_Burkitt's lymphoma | 0.0 |
| T98G_Glioblastoma | 33.2 | U266_B-cell plasmacytoma/myeloma | 0.0 |
| SK-N-SE_Neuroblastoma (metastasis) | 0.0 | CA46 Burkitt's lymphoma | 0.0 |
| SF-295_CNS/glioblastoma | 2.3 | RL_non-Hodgkin's B-cell lymphoma | 0.0 |
| Cerebellum | 0.4 | JM1_pre-B-cell lymphoma/leukemia | 0.0 |
| Cerebellum | 0.1 | Jurkat_T cell leukemia | 0.0 |
| NCI-H292 _Mucoepidermoid lung ca. | 0.3 | TF-1_Erythroleukemia | 1.6 |
| DMS-114_Small cell lung cancer | 0.0 | HUT 78_T-cell lymphoma | 0.0 |
| DMS-79_ Small cell lung cancer/neuroendocrine | 0.0 | U937_Histiocytic lymphoma | 0.0 |
| NCI-H146_Small cell lung cancer/neuroendocrine | 2.2 | KU-812_Myelogenous leukemia | 0.9 |
| NCI-H526_Small cell lung cancer/neuroendocrine | 0.0 | 769-P_Clear cell renal ca. | 1.0 |
| NCI-N417_Small cell lung cancer/neuroendocrine | 0.0 | Caki-2_Clear cell renal ca. | 63.7 |
| NCI-H82_Small cell lung cancer/neuroendocrine | 0.0 | SW 839_Clear cell renal ca. | 0.4 |
| NCI-H157_Squamous cell lung cancer (metastasis) | 0.0 | G401 Wilms' tumor | 0.0 |
| NCI-H 155_Large cell lung cancer/neuroendocrine | 0.0 | Hs766T_Pancreatic ca. (LN metastasis) | 0.2 |
| NCI-H1299_Large cell lung cancer/neuroendocrine | 0.0 | CAPAN-1_Pancreatic adenocarcinoma (liver metastasis) | 0.3 |
| NCI-H727_Lung carcinoid | 1.3 | SU86.86_Pancreatic carcinoma (liver metastasis) | 1.2 |
| NCI-UMC-11_Lung carcinoid | 100.0 | BxPC-3_Pancreatic adenocarcinoma | 3.6 |
| LX-1_Small cell lung cancer | 1.6 | HPAC_Pancreatic adenocarcinoma | 3.1 |
| Colo-205_Colon cancer | 7.5 | MIA PaCa-2_Pancreatic ca. | 0.0 |
| KM12_Colon cancer KM12_Colon cancer | 0.1 | CFPAC-1_Pancreatic ductal 0.1 adenocarcinoma | 3.9 |
| KM20L2 Colon cancer | 0.1 | PANC-1_Pancreatic epithelioid ductal ca. | 0.0 |
| NCI-H716_Colon cancer | 19.2 | T24_Bladder ca. (transitional cell) | 2.0 |
| SW-48_Colon adenocarcinoma | 2.3 | 5637_Bladder ca. | 0.1 |
| SW1116_Colon adenocarcinoma | 0.0 | HT-1197_Bladder ca. | 0.0 |
| LS 174T_Colon adenocarcinoma | 0.9 | UM-UC-3_Bladder ca. (transitional cell) | 0.1 |
| SW-948_Colon adenocarcinoma | 0.3 | A204_Rhabdomyosarcoma | 0.8 |
| SW-480_Colon adenocarcinoma | 0.5 | HT-1080_Fibrosarcoma | 0.0 |
| NCI-SNU-5_Gastric ca. | 0.0 | MG-63_Osteosarcoma(bone) | 0.0 |
| KATO Ill_Stomach | 0.1 | SK-LMS-1_Leiomyosarcoma (vulva) | 0.0 |
| NCI-SNU-16 Gastric ca. | 2.7 | SJRH30_Rhabdomyosarcoma (met to bone marrow) | 0.1 |
| NCI-SNU-1_Gastric ca. | 16.8 | A431_Epidermoid ca. | 3.8 |
| RF-1_Gastric adenocarcinoma | 0.5 | WM266-4_Melanoma | 0.1 |
| RF-48_Gastric adenocarcinoma | 0.0 | DU 145_Prostate | 0.1 |
| MKN-45_Gastric ca. | 1.6 | MDA-MB-468_Breast adenocarcinoma | 0.5 |
| NCI-N87_Gastricca. | 0.8 | SSC-4_Tongue | 0.4 |
| OVCAR-5_Ovarian ca. | 0.4 | SSC-9_Tongue | 0.2 |
| RL95-2_Uterine carcinoma | 0.5 | SSC-15_Tongue | 0.3 |
| HelaS3_Cervical adenocarcinoma | 0.9 | CAL 27_Squamous cell ca. of tongue | 0.1 |

**Table QH. Panel 2D**

| **Column A - Rel. Exp.(%) Ag3562, Run 170858350** | | | |
|---|---|---|---|
| **Tissue Name** | **A** | **Tissue Name** | **A** |
| Normal Colon | 5.0 | Kidney Margin 8120608 | 2.3 |
| CC Well to Mod Diff (ODO3866) | 1.5 | Kidney Cancer 8120613 | 4.4 |
| CC Margin (ODO3866) | 2.0 | Kidney Margin 8120614 | 4.2 |
| CC Gr.2 rectosigmoid (ODO3868) | 0.5 | Kidney Cancer 9010320 | 1.5 |
| CC Margin (ODO3868) | 0.5 | Kidney Margin 9010321 | 2.6 |
| CC Mod Diff (ODO3920) | 0.5 | Normal Uterus | 0.4 |
| CC Margin (ODO3920) | 1.2 | Uterus Cancer 064011 | 0.5 |
| CC Gr.2 ascend colon (ODO3921) | 2.1 | Normal Thyroid | 1.2 |
| CC Margin (OD03921) | 1.4 | Thyroid Cancer 064010 | 0.1 |
| CC from Partial Hepatectomy (ODO4309) Mets | 5.5 | Thyroid Cancer A302152 | 0.2 |
| Liver Margin (ODO4309) | 27.0 | Thyroid Margin A302153 | 1.6 |
| Colon mets to lung (OD04451-01) | 0.4 | Normal Breast | 5.3 |
| Lung Margin (OD04451-02) | 1.8 | Breast Cancer (OD04566) | 0.2 |
| Normal Prostate 6546-1 | 1.3 | Breast Cancer (OD04590-01) | 1.4 |
| Prostate Cancer (OD04410) | 0.4 | Breast Cancer Mets (OD04590-03) | 5.2 |
| Prostate Margin (OD04410) | 0.5 | Breast Cancer Metastasis (OD04655-05) | 1.1 |
| Prostate Cancer (OD04720-01) | 0.6 | Breast Cancer 064006 | 0.3 |
| Prostate Margin (OD04720-02) | 1.6 | Breast Cancer 1024 | 0.8 |
| Normal Lung 061010 | 4.1 | Breast Cancer 9100266 | 0.3 |
| Lung Met to Muscle (ODO4286) | 41.8 | Breast Margin 9100265 | 0.5 |
| Muscle Margin (ODO4286) | 1.7 | Breast Cancer A209073 | 0.6 |
| Lung Malignant Cancer (OD03126) | 2.0 | Breast Margin A209073 | 0.5 |
| Lung Margin (OD03126) | 2.1 | Normal Liver | 11.9 |
| Lung Cancer (OD04404) | **100.0** | Liver Cancer 064003 | 5.0 |
| Lung Margin (OD04404) | 1.7 | Liver Cancer 1025 | 14.7 |
| Lung Cancer (OD04565) | 43.2 | Liver Cancer 1026 | 3.9 |
| Lung Margin (OD04565) | 0.7 | Liver Cancer 6004-T | 14.0 |
| Lung Cancer (OD04237-01) | 0.5 | Liver Tissue 6004-N | 20.9 |
| Lung Margin (OD04237-02) | 2.2 | Liver Cancer 6005-T | 3.7 |
| Ocular Mel Met to Liver (OD04310) | 0.1 | Liver Tissue 6005-N | 6.3 |
| Liver Margin (OD04310) | 14.8 | Normal Bladder | 5.6 |
| Melanoma Mets to Lung (OD04321) | 0.1 | Bladder Cancer 1023 | 0.3 |
| Lung Margin (OD04321) | 2.3 | Bladder Cancer A302173 | 0.8 |
| Normal Kidney | 5.0 | Bladder Cancer (OD04718-01) | 0.1 |
| Kidney Ca, Nuclear grade 2 (OD04338) | 29.1 | Bladder Normal Adjacent (OD04718-03) | 0.9 |
| Kidney Margin (OD04338) | 3.1 | Normal Ovary | 0.4 |
| Kidney Ca Nuclear grade 1/2 (OD04339) | 3.7 | Ovarian Cancer 064008 | 0.5 |
| Kidney Margin (OD04339) | 5.1 | Ovarian Cancer (OD04768-07) | 0.6 |
| Kidney Ca, Clear cell type (OD04340) | 6.3 | Ovary Margin (OD04768-08) | 0.3 |
| Kidney Margin (OD04340) | 3.6 | Normal Stomach | 5.5 |
| Kidney Ca, Nuclear grade 3 (OD04348) | 0.1 | Gastric Cancer 9060358 | 1.0 |
| Kidney Margin (OD04348) | 1.8 | Stomach Margin 9060359 | 11.7 |
| Kidney Cancer (OD04622-01) | 1.9 | Gastric Cancer 9060395 | 14.4 |
| Kidney Margin (OD04622-03) | 1.5 | Stomach Margin 9060394 | 15.3 |
| Kidney Cancer (OD04450-01) | 12.0 | Gastric Cancer 9060397 | 2.1 |
| Kidney Margin (OD04450-03) | 4.5 | Stomach Margin 9060396 | 6.7 |
| Kidney Cancer 8120607 | 34.2 | Gastric Cancer 064005 | 7.6 |

**Table QI. Panel 4.1D**

| .. **Column A - Rel. Exp.(%) Ag3562, Run 169990867** | | | |
|---|---|---|---|
| **Tissue Name** | **A** | **Tissue Name** | **A** |
| Secondary Th1 act | 0.0 | HUVEC IL-1beta | 0.7 |
| Secondary Th2 act | 0.1 | HUVEC IFN gamma | 2.1 |
| Secondary Tr1 act | 0.2 | HUVEC TNF alpha + IFN gamma | 0.7 |
| Secondary Th1 rest | 0.2 | HUVEC TNF alpha + IL4 | 0.5 |
| Secondary Th2 rest | 0.1 | HUVEC IL-11 | 2.4 |
| Secondary Tr1 rest | 0.1 | Lung Microvascular EC none | 13.1 |
| Primary Th1 act | 0.0 | Lung Microvascular EC TNFalpha + IL-1beta | 10.1 |
| Primary Th2 act | 0.1 | Microvascular Dermal EC none | 11.8 |
| Primary Tr1 act | 0.0 | Microsvasular Dermal EC TNFalpha + Il-1beta | 12.8 |
| Primary Th1 rest | 0.4 | Bronchial epithelium TNFalpha + IL-1beta | 92.7 |
| Primary Th2 rest | 0.2 | Small airway epithelium none | 29.9 |
| Primary Tr1 rest | 0.1 | Small airway epithelium TNFalpha + IL-1beta | 50.3 |
| CD45RA CD4 lymphocyte act | 6.7 | Coronery artery SMC rest | 5.4 |
| CD45RO CD4 lymphocyte act | 0.4 | Coronery artery SMC TNFalpba + IL-1beta | 7.0 |
| CD8 lymphocyte act | 0.3 | Astrocytes rest | 0.6 |
| Secondary CD8 lymphocyte rest | 0.0 | Astrocytes TNFalpba + IL-1beta | 0.7 |
| Secondary CD8 lymphocyte act | 0.0 | KU-812(Basophil) rest | 16.4 |
| CD4 lymphocyte none | 0.6 | KU-812 (Basophil) PMA/ionomycin | 33.2 |
| 2ry Th1/Th2/Tr1_anti-CD95 CH11 | 0.2 | CCD1106 (Keratinocytes) none | 2.9 |
| LAK cells rest | 0.5 | CCD1106 (Keratinocytes) TNFalpha + IL-1beta | 2.3 |
| LAK cells IL-2 | 1.3 | Liver cirrhosis | 38.2 |
| LAK cells IL-2+IL-12 | 0.5 | NCI-H292 none | 11.8 |
| LAK cells IL-2+IFN gamma | 1.1 | NCI-H292 IL-4 | 7.1 |
| LAK cells IL-2+ IL-18 | 0.7 | NCI-H292 IL-9 | 22.5 |
| LAK cells PMA/ionomycin | 1.2 | NCI-H292 IL-13 | 5.2 |
| NK Cells IL-2 rest | 3.5 | NCI-H292 IFN gamma | 4.7 |
| Two Way MLR 3 day | 1.4 | HPAEC none | 5.1 |
| Two Way MLR 5 day | 0.6 | HPAEC TNF alpha + IL-1 beta | 7.6 |
| Two Way MLR 7 day | 0.3 | Lung fibroblast none | 6.8 |
| PBMC rest | 0.8 | Lung fibroblast TNF alpha + IL-1 beta | 48.6 |
| PBMC PWM | 15.0 | Lung fibroblast IL-4 | 7.5 |
| PBMC PHA-L | 0.3 | Lung fibroblast IL-9 | 7.2 |
| Ramos (B cell) none | 0.0 | Lung fibroblast IL-13 | 8.8 |
| Ramos (B cell) ionomycin | 0.0 | Lung fibroblast IFN gamma | 6.8 |
| B lymphocytes PWM | 0.2 | Dermal fibroblast CCD 1070 rest | 13.9 |
| B lymphocytes CD40L and IL-4 | 0.3 | Dermal fibroblast-CCD1070 TNF alpha | 19.9 |
| EOL-1 dbcAMP | 0.1 | Dermal fibroblast CCD 1070 IL-1 beta | 24.7 |
| EOL-1 dbcAMP PMA/ionomycin | 0.1 | Dermal fibroblast IFN gamma | 38.4 |
| Dendritic cells none | 0.8 | Dermal fibroblast IL-4 | 100.0 |
| Dendritic cells LPS | 2.9 | Dermal Fibroblasts rest | 68.3 |
| Dendritic cells anti-CD40 | 0.6 | Neutrophils TNFa+LPS | 0.2 |
| Monocytes rest | 0.1 | Neutrophils rest | 0.1 |
| Monocytes LPS | 9.7 | Colon | 14.0 |
| Macrophages rest | 2.4 | Lung | 5.8 |
| Macrophages LPS | 2.9 | Thymus . | 6.6 |
| HUVEC none | 1.0 | Kidney | 20.7 |
| HUVEC starved | 0.9 | | |

**Table QJ. Panel 5 Islet**

| **Column A - Rel. Exp.(%) Ag3562, Run 242386397** | | | |
|---|---|---|---|
| **Tissue Name** | **A** | **Tissue Name** | **A** |
| 97457_Patient-02go_adipose | 12.8 | 94709_Donor 2 AM - A_adipose | 10.2 |
| 97476 Patient-07sk skeletal muscle | 12.4 | 94710_Donor 2 AM-B_adipose | 7.1 |
| 97477_Patient-07ut_uterus | 1.6 | 94711_Donor 2 AM - C_adipose | 6.4 |
| 97478_Patient-07pl_placenta | 0.3 | 94712_Donor 2 AD - A_adipose | 17.9 |
| 99167_Payer Patient 1 | **100.0** | 94713 _Donor 2 AD-B_adipose | 15.6 |
| 97482_Patient-08ut_uterus | 0.7 | 94714_Donor 2 AD - C_adipose | 19.1 |
| 97483_Patient-08pl_placenta | 0.0 | 94742_Donor 3 U - A_Mesenchymal Stem Cells | 2.0 |
| 97486_Patient-09sk_skeletal muscle | 7.6 | 94743_Donor 3 U - B_Mesenchymal Stem Cells | 3.4 |
| 97487_Patient-09ut_uterus | 3.9 | 94730_Donor 3 AM - A_adipose | 17.6 |
| 97488_Patient-09pl_placenta | 0.4 | 94731_Donor 3 AM - B_adipose | 7.6 |
| 97492_Patient-10ut_uterus | 1.9 | 94732_Donor 3 AM - C_adipose | 9.7 |
| 97493_Patient-10pl_placenta | 0.5 | 94733_Donor 3 AD - A_adipose | 32.5 |
| 97495_Patient-11go_adipose | 7.4 | 94734_Donor 3 AD - B_adipose | 6.7 |
| 97496 Patient-11sk skeletal muscle | 8.0 | 94735 Donor 3 AD - C_adipose | 23.2 |
| 97497_Patient-11ut_uterus | 2.8 | 77138_Liver_HepG2untreated | 82.9 |
| 97498_Patient-11pl_placenta | 0.1 | 73556 Heart Cardiac stromal cells (primary) | 1.9 |
| 97500_Patient-12go_adipose | 11.3 | 81735_Small Intestine | 13.4 |
| 97501_Patient-12sk_skeletal muscle | 33.2 | 72409_Kidney_Proximal Convoluted Tubule | 3.6 |
| 97502_Patient-12ut_uterus | 3.1 | 82685_Small intestine_Duodenum | 2.5 |
| 97503_Patient-12pl_placenta | 0.4 | 90650_Adrenal_Adrenocortical adenoma | 2.4 |
| 94721_Donor 2 U - A_Mesenchymal Stem Cells | 3.8 | 72410_Kidney_HRCE | 11.6 |
| 94722_Donor 2 U - B_Mesenchymal Stem Cells | 4.3 | 72411_Kidney_HRE | 1.3 |
| 94723_Donor 2 U - C_Mesenchymal Stem Cells | 6.0 | 73139_Uterus_Uterine smooth muscle cells | 0.4 |

**Ardais Panel v.1.0 Summary:** Ag3562 Highest expression of this gene is seen in lung cancer (CT=19.1). In addition, this gene is more highly expressed in three lung cancer samples than in the corresponding normal adjacent tissue. Thus, expression of this gene could be used as a marker of this cancer. Furthemore, therapeutic modulation of the expression or function of this gene product may be useful in the treatment of lung cancer.

**CNS_nenrodegeneration_v1.0 Summary**: Data obtained from RTQPCR assays performed for this panel was analyized using multivariate analysis (ANOVA). The multivariate results obtained analyzing Ag3562 in this panel confirms the expression of the CG59693-02 gene at low levels in the brain in an independent group of individuals. This gene is found to be upregulated in the temporal cortex of Alzheimer's disease patients when analyzed by ANCOVA, (p = 0.002). Therefore, modulation of gene expression with small molecule therapeutic or biomolecules may prevent or slow the progression of Alzheimer's disease.

**General_screening_panel_v1.4 Summary:** Ag3562 Highest expression ofthe CG59693-02 gene is detected in lung cancer A549 cell line (CT=20.01). High expression of this gene is also seen in cluster of cancer cell lines derived from gastric, colon, lung, renal, breast, ovarian, prostate, squamous cell carcinoma, melanoma and brain cancers. Thus, expression of this gene could be used as a marker to detect the presence of these cancers. Furthermore, therapeutic modulation of the expression or function of this gene may be effective in the treatment of gastric, colon, lung, renal, breast, ovarian, prostate, squamous cell carcinoma, melanoma and brain cancers.

Among tissues with metabolic or endocrine function, this gene is expressed at moderate to high levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at high levels in all regions of the central nervous system examined, including amygdala, hippocampus, substantia nigra, thalamus, cerebellum, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

**General_screening_panel_v1.6 Summary:** Ag3562 Highest expression of the CG59693-02 gene is detected in lung cancer A549 cell line (CT=20.7). High expression of this gene is also seen in cluster of cancer cell lines derived from gastric, colon, lung, renal, breast, ovarian, prostate, squamous cell carcinoma, melanoma and brain cancers. Among tissues with metabolic or endocrine function, this gene is expressed at moderate to high levels in pancreas, adipose, adrenal gland, thyroid, pituitary gland, skeletal muscle, heart, liver and the gastrointestinal tract. In addition, this gene is expressed at high levels in all regions of the central nervous system examined, including amygdala, hippocampus, substantia nigra, thalamus, cerebellum, cerebral cortex, and spinal cord. This pattern is in agreement with the expression profile in General_screening_panel_vl.4. Please see panel 1.4 for a discussion of this gene

**HASS Panel v1.0 Summary:** Ag3562. The expression of CG59693-02 gene is not increased by oxygen deprivation, acidic or a serum starved environment in the breast , bladder, pancreatic and prostate cell line in this panel.

However expression is increased in a glioblastoma/astrocytoma cell line when these cells are subjected to an acidic environment (Maximum expression U87-MG F11; CT=23.96) which suggests that expression may also be upregulated in the acidic regions of brain cancers. Moderate to low expression is also shown in 2 of 5 glioma and 2 of 4 medulloblastoma tissue samples in this panel. Therapeutic modulation of this gene product using small molecule drugs may be useful in the treatment of brain cancer.

**Oncology_cell_line_screening_panel_v3.1 Summary:** Ag3562 Highest expression of the CG59693-02 gene is detected in lung carcinoid sample (CT=21.7). High to moderate levels of expression of this gene is also seen in number of cancer samples including tongue, breast, prostate, melanoma, bone marrow, bladder, pancreatic, renal, lymphoma, ovarian, cervical, uterine, gastric, lung and brain cancer. Therefore, therapeutic modulation of this gene through the use of small molecule drug may be beneficial in the treatment of these cancers.

**Panel 2D Summary**: Ag3562 Highest expression of the CG59693-02 gene is detected in lung cancer (CT=23.5). High expression of this gene is seen in number of lung cancer samples. Expression of this gene is higher in cancer sample as compared to corresponding adjacent control samples. Therefore, expression of this gene may be used as marker to detect the presence of lung cancer and therapeutic modulation of this gene through the use of small molecule drug may be useful in the treatment of lung cancer.

High to moderate levels of expression of this gene is also seen in number of cancer samples including colon, gastric, ovarian, liver, breast, thyroid, kidney, and prostate cancers. Therefore, therapeutic modulation of this gene through the use of small molecule drug may be beneficial in the treatment of these cancers.

**Panel 4.1D Summary:** Ag3562 Highest expression ofthe CG59693-02 gene is detected in IL-4 treated dermal fibroblasts (CT=25.2). This gene is expressed at moderate to low levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.5 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel 5 Islet Summary:** Ag3562 Highest expression ofthe CG59693-02 gene is detected in islet cells (Bayer patient 1) (CT=25.3). High to moderate levels of expression of this gene is also seen in adipose, skeletal muscle, placenta, uterus, liver, heart, small intestine and kidney. Therefore, therapeutic modulation of the activity of this gene may prove useful in the treatment of endocrine/metabolically related diseases, such as obesity and diabetes.

### R. CG93088-01: moncarboxylate transporter

Expression of gene CG93088-01 was assessed using the primer-probe set Ag3841, described in Table RA. Results of the RTQ-PCR runs are shown in Tables RB, RC and RD.

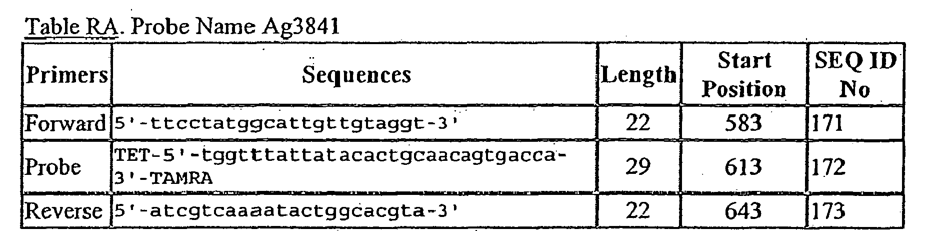

**Table RB. CNS neurodegeneration v1.0**

| **Column A - Rel. Exp.(%) Ag3841, Run 206873281** | | | | | |
|---|---|---|---|---|---|
| **Column B - Rel. Exp.(%) Ag3841, Run 224339890** | | | | | |
| Tissue Name | A | B | Tissue Name | A | B |
| AD 1 Hippo | 59.5 | 60.7 | Control (Path) 3 Temporal Ctx | 11.8 | 9.9 |
| AD 2 Hippo | 91.4 | 77.4 | Control (Path) 4 Temporal Ctx | 26.4 | 19.1 |
| AD 3 Hippo | 15.8 | 13.0 | AD 1 Occipital Ctx | 35.1 | 39.0 |
| AD 4 Hippo | 15.0 | 19.3 | AD 2 Occipital Ctx (Missing) | 0.0 | 0.0 |
| AD 5 Hippo | 43.2 | 52.5 | AD 3 Occipital Ctx | 16.8 | 16.0 |
| AD 6 Hippo | 92.7 | **100.0** | AD 4 Occipital Ctx | 31.9 | 28.1 |
| Control 2 Hippo | 25.5 | 32.3 | AD 5 Occipital Ctx | 23.8 | 13.4 |
| Control 4 Hippo | 45.7 | 54.7 | AD 6 Occipital Ctx | 15.2 | 28.1 |
| Control (Path) 3 Hippo | 16.6 | 14.8 | Control 1 Occipital Ctx | 7.0 | 6.1 |
| AD 1 Temporal Ctx | 55.1 | 54.3 | Control 2 Occipital Ctx | 35.4 | 32.3 |
| AD 2 Temporal Ctx | 61.6 | 62.0 | Control 3 Occipital Ctx | 20.6 | 18.0 |
| AD 3 Temporal Ctx | 12.9 | 16.4 | Control 4 Occipital Ctx | 16.8 | 22.7 |
| AD 4 Temporal Ctx | 42.9 | 44.1 | Control (Path) 1 Occipital Ctx | 52.5 | 50.0 |
| AD 5 Inf Temporal Ctx | 96.6 | 92.0 | Control (Path) 2 Occipital Ctx | 9.2 | 12.2 |
| AD 5 Sup Temporal Ctx | 100.0 | 83.5 | Control (Path) 3 Occipital Ctx | 5.1 | 5.3 |
| AD 6 Inf Temporal Ctx | 51.4 | 48.3 | Control (Path) 4 Occipital Ctx | 6.3 | 6.2 |
| AD 6 Sup Temporal Ctx | 56.3 | 49.0 | Control 1 Parietal Ctx | 14.4 | 15.9 |
| Control 1 Temporal Ctx | 18.4 | 15.9 | Control 2 Parietal Ctx | 63.7 | 763 |
| Control 2 Temporal Ctx | 23.0 | 27.4 | Control 3 Parietal Ctx | 14.3 | 14.9 |
| Control 3 Temporal Ctx | 12.4 | 17.9 | Control (Path) 1 Parietal Ctx | 24.0 | 28.3 |
| Control 3 Temporal Ctx | 19.9 | 25.5 | Control (Path) 2 Parietal Ctx | 25.5 | 24.8 |
| Control (Path) 1 Temporal Ctx | 22.2 | 20.7 | Control (Path) 3 Parietal Ctx | 8.8 | 7.9 |
| Control (Path) 2 Temporal Ctx | 29.1 | 26.6 | Control (Path) 4 Parietal Ctx | 27.7 | 21.2 |

**Table RC. General screening panel vl.4**

| **Column A - Rel. Exp.(%) Ag3841, Run 213604526** | | | |
|---|---|---|---|
| **Tissue Name** | **A** | **Tissue Name** | **A** |
| Adipose | 1.6 | Renal ca. TK-10 | 5.6 |
| Melanoma* Hs688(A).T | 0.0 | Bladder | 3.1 |
| Melanoma*_Hs688(B).T | 0.1 | Gastric ca. (liver met.) NCI-N87 | 2.6 |
| Melanoma* M14 | 0.0 | Gastric ca. KATO III | 0.0 |
| Melanoma* LOXIMVI | 0.0 | Colon ca. SW-948 | 0.8 |
| Melanoma* SK-MEL-5 | 0.4 | Colon ca. SW480 | 1.7 |
| Squamous cell carcinoma SCC-4 | 5.7 | Colon ca.* (SW480 met) SW620 | 2.4 |
| Testis Pool | 2.3 | Colon ca. HT29 | 0.0 |
| Prostate ca.* (bone met) PC-3 | 10.2 | Colon ca. HCT-116 | 8.7 |
| Prostate Pool | 2.3 | Colon ca. CaCo-2 | 0.6 |
| Placenta | 0.0 | Colon cancer tissue | 0.2 |
| Uterus Pool | 3.8 | Colon ca. SW1116 | 0.3 |
| Ovarian ca. OVCAR-3 | 3.0 | Colon ca. Colo-205 | 0.0 |
| Ovarian ca. SK-OV-3 | 1.9 | Colon ca. SW-48 | 0.0 |
| Ovarian ca. OVCAR-4 | 1.3 | Colon Pool | 9.9 |
| Ovarian ca. OVCAR-5 | 19.9 | Small Intestine Pool | 3.8 |
| Ovarian ca. IGROV-1 | 0.4 | Stomach Pool | 2.9 |
| Ovarian ca. OVCAR-8 | 1.6 | Bone Marrow Pool | 2.7 |
| Ovary | 30.6 | Fetal Heart | 0.8 |
| Breast ca. MCF-7 | 2.4 | Heart Pool | 1.9 |
| Breast ca. MDA-MB-231 | 0.0 | Lymph Node Pool | 8.8 |
| Breast ca. BT 549 | 3.8 | Fetal Skeletal Muscle | 1.4 |
| Breast ca.T47D | 33.9 | Skeletal Muscle Pool | 0.7 |
| Breast ca. MDA-N | 0.0 | Spleen Pool | 22.4 |
| Breast Pool | 9.7 | Thyinus Pool | 3.1 |
| Trachea | 8.2 | CNS cancer (glio/astro) U87-MG | 0.0 |
| Lung | 5.2 | CNS cancer (glio/astro) U-118-MG | 0.0 |
| Fetal Lung | 3.0 | CNS cancer (neuro;met) SK-N-AS | 3.0 |
| Lung ca. NCI-N417 | 0.7 | CNS cancer (astro) SF-539 | 2.5 |
| Lung ca. LX-1 | 0.3 | CNS cancer (astro) SNB-75 | 2.6 |
| Lung ca. NCI-H146 | 0.5 | CNS cancer (glio) SNB-19 | 0.5 |
| Lung ca. SHP-77 | 0.0 | CNS cancer (glio) SF-295 | 0.4 |
| Lung ca. A549 | 0.1 | Brain (Amygdala) Pool | 1.0 |
| Lung ca. NCI-H526 | 0.0 | Brain (cerebellum) | 5.5 |
| Lung ca. NCI-H23 | 3.8 | Brain (fetal) | 4.7 |
| Lung ca. NCI-H460 | 2.0 | Brain (Hippocampus) Pool | 2.9 |
| Lung ca. HOP-62 | 0.2 | Cerebral Cortex Pool | 2.1 |
| Lung ca. NCI-H522 | 2.4 | Brain (Substantia nigra) Pool | 1.8 |
| Liver | 0.1 | Brain (Thalamus) Pool | 2.6 |
| Fetal Liver | 8.0 | Brain (whole) | 3.1 |
| Liver ca. HepG2 | 3.1 | Spinal Cord Pool | 3.8 |
| Kidney Pool | 8.7 | Adrenal Gland | **100.0** |
| Fetal Kidney | 12.9 | Pituitary gland Pool | 2.1 |
| Renal ca. 786-0 | 0.1 | Salivary Gland | 1.1 |
| Renal ca. A498 | 0.5 | Thyroid (female) | 5.2 |
| Renal ca. ACHN | 0.7 | Pancreatic ca. CAPAN2 | 0.2 |
| Renal ca. UO-31 | 1.8 | Pancreas Pool | 5.2 |

**Table RD. Panel 4.1D**

| **Column A - Rel. Exp.(%) Ag3841, Run 170126778** | | | |
|---|---|---|---|
| Tissue Name | A | Tissue Name | A |
| Secondary Th1 act | 0.3 | HUVEC IL-1beta | 0.2 |
| Secondary Th2 act | 0.3 | HUVEC IFN gamma | 1.3 |
| Secondary Tr1 act | 0.3 | HUVEC TNF alpha + IFN gamma | 0.1 |
| Secondary Th1 rest | 0.0 | HUVEC TNF alpha + IL4 | 0.3 |
| Secondary Th2 rest | 0.1 | HUVEC IL-11 | 1.0 |
| Secondary Tr1 rest | 0.0 | Lung Microvascular EC none | 2.3 |
| Primary Th1 act | 2.4 | Lung Microvascular EC TNFalpha + IL-1beta | 1.4 |
| Primary Th2 act | 1.0 | Microvascular Dermal EC none | 0.6 |
| Primary Tr1 act | 1.0 | Microsvasular Dermal EC TNFalpha + IL-1beta | 0.1 |
| Primary Th1 rest | 0.0 | Bronchial epithelium TNFaipba + IL1beta | 4.9 |
| Primary Th2 rest | 0.0 | Small airway epithelium none | 2.9 |
| Primary Tr1 rest | 0.1 | Small airway epithelium TNFalpha + IL-1beta | 3.1 |
| CD45RA CD4 lymphocyte act | 0.2 | Coronery artery SMC rest | 3.7 |
| CD45RO CD4 lymphocyte act | 0.3 | Coronery artery SMC TNFalpha + IL-1beta | 2.3 |
| CD8 lymphocyte act | 0.2 | Astrocytes rest | 4.4 |
| Secondary CD8 lymphocyte rest | 0.1 | Astrocytes TNFalpha +IL-1beta | 2.9 |
| Secondary CD8 lymphocyte act | 0.1 | KU-812 (Basophil) rest | 18.6 |
| CD4 lymphocyte none | 0.0 | KU-812 (Basophil) PMA/ionomycin | 16.4 |
| 2ry Th1/Th2/Tr1_anti-CD95 CH11 | 0.0 | CCD1106 (Keratinocytes) none | 3.5 |
| LAK cells rest | 0.1 | CCD1106 (Keratinocytes) TNFalpha + IL-1beta | 2.5 |
| LAK cells IL-2 | 0.1 | Liver cirrhosis | 0.1 |
| LAK cells IL-2+IL-12 | 0.3 | NCI-H292 none | 0.0 |
| LAK cells IL-2+IFN gamma | 0.2 | N.CI-H292 IL-4 | 0.2 |
| LAK cells IL-2+ IL-18 | 0.5 | NCI-H292 IL-9 | 0.2 |
| LAK cells PMA/ionomycin | 0.0 | NCI-H292 IL-13 | 0.1 |
| NK Cells IL-2 rest | 0.1 | NCI-H292 IFN gamma | 0.4 |
| Two Way MLR 3 day | 0.0 | HPAEC none | 0.1 |
| Two Way MLR 5 day | 0.0 | HPAEC TNF alpha + IL-1 beta | 1.5 |
| Two Way MLR 7 day | 0.0 | Lung fibroblast none | 0.1 |
| PBMC rest | 0.0 | Lung fibroblast TNF alpha + IL-1 beta | 0.0 |
| PBMC PWM | 0.7 | Lung fibroblast IL-4 | 0.0 |
| PBMC PHA-L | 0.1 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.9 | Dermal fibroblast CCD1070 rest | 0.0 |
| B lymphocytes CD40L and IL-4 | 0.2 | Dermal fibroblast CCD1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.1 | Dermal Fibroblasts rest | 0.0 |
| Dendritic cells anti-CD40 | 0.2 | Neutrophils TNFa+LPS | 0.0 |
| Monocytes rest | 0.0 | Neutrophils rest | 0.0 |
| Monocytes LPS | 0.0 | Colon | 0.3 |
| Macrophages rest | 0.1 | Lung | 0.2 |
| Macrophages LPS | 0.0 | Thymus | 0.3 |
| HIJVEC none | 0.0 | Kidney | **100.0** |
| HUVEC starved | 0.0 | | |

**CNS_neurodegeaeration_v1.0 Summary:** Data obtained from RTQPCR assays performed for this panel was analyized using multivariate analysis (ANOVA). The multivariate results obtained analyzing Ag3841 shows two experiments tested with the same probe and primer sets are in excellent agreements. It confirms the expression of the CG93088-01 gene at low levels in the brain in an independent group of individuals. This gene is upregulated in the temporal cortex of Alzheimer's disease patients when compared with non-demented controls (p = 0.02 when analyzed by Ancova, estimate of total cDNA loaded per well used asa covariate). This gene may therefore be a small molecule target, and blockade of this transporter may slow or stop the progression of Alzheimer's disease.

**General_screening_panel_v1.4** Summary: Ag3841 Highest expression of the CG93088-01 gene is detected in adrenal gland (CT=25). In addition, this gene is also expressed at high to moderate levels in other tissues with metabolic or endocrine function, such as pancreas, adipose, thyroid, pituitary gland, skeletal muscle, heart, liver and the gastrointestinal tract. The CG93088-01 gene codes for monocarboxylate transporter, a transporter belonging to sugar transporter family. Recently, a protein belonging to this family was shown to be associated with non-insulin-dependent diabetes mellitus (NIDDM) (Ref.1). Therefore, therapeutic modulation of the activity of this gene may prove useful in the treatment of endocrine/metabolically related diseases, such as obesity and diabetes including NIDDM.

Interestingly, this gene is expressed at much higher levels in fetal (CT=28.7) when compared to adult liver (CT=35.6). This observation suggests that expression of this gene can be used to distinguish disorders or predisposition thereto between fetal and adult liver.

In addition, this gene is expressed at high to moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

### References.

### McVie-Wylie AJ, et al. (2001) Genomics 72(1):113-7 (PMID: 11247674)

**Panel 4.1D Summary:** Ag3841 Highest expression of the CG93088-01 gene is detected in kidney sample (CT=26). Therefore, antibody or small molecule therapies designed with the protein encoded for by this gene could modulate kidney function and be important in the treatment of inflammatory or autoimmune diseases that affect the kidney, including lupus and glomerulonephritis.

In addition, low to moderate expression of this gene is also seen in TNF alpha + IL-1 beta treated HPAEC, keratinocytes, basophils, astrocytes, coronery artery SMC, small airway epithelium, lung microvascular EC, microvascular dermal EC and PWM treated B lymphocytes. Interestingly, expression of this gene is stimulated in TNF alpha + IL-1 beta treated HPAEC, IFN gamma/IL- 11 treated HUVEC cells, PWM treated PBMC cells, IL-2+ IL-18 treated LAK cells, activated primary and secondary Th1, Th2, Tr1 cells as compared to their corresponding untreated or resting cells. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### Example D: Identification of Single Nucleotide Polymorphisms in NOVX nucleic acid sequences

Variant sequences are also included in this application. A variant sequence can include a single nucleotide polymorphism (SNP). A SNP can, in some instances, be referred to as a "cSNP" to denote that the nucleotide'sequence containing the SNP originates as a cDNA. A SNP can arise in several ways. For example, a SNP may be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes may result in an alteration of the amino acid encoded by the gene at the position of the SNP. Intragenic SNPs may also be silent, when a codon including a SNP encodes the same amino acid as a result of the redundancy of the genetic code. SNPs occurring outside the region of a gene, or in an intron within a gene, do not result in changes in any amino acid sequence of a protein but may result in altered regulation of the expression pattern. Examples include alteration in temporal expression, physiological response regulation, cell type expression regulation, intensity of expression, and stability of transcribed message.

SeqCalling assemblies produced by the exon linking process were selected and extended using the following criteria. Genomic clones having regions with 98% identity to all or part of the initial or extended sequence were identified by BLASTN searches using the relevant sequence to query human genomic databases. The genomic clones that resulted were selected for further analysis because this identity indicates that these clones contain the genomic locus for these SeqCalling assemblies. These sequences were analyzed for putative coding regions as well as for similarity to the known DNA and protein sequences. Programs- used for these analyses include Grail, Genscan, BLAST, HMMER, FASTA, Hybrid and other relevant programs.

Some additional genomic regions may have also been identified because selected SeqCalling assemblies map to those regions. Such SeqCalling sequences may have overlapped with regions defined by homology or exon prediction. They may also be included because the location of the fragment was in the vicinity of genomic regions identified by similarity or exon prediction that had been included in the original predicted sequence. The sequence so identified was manually assembled and then may have been extended using one or more additional sequences taken from CuraGen Corporation's human SeqCalling database. SeqCalling fragments suitable for inclusion were identified by the CuraTools™ program SeqExtend or by identifying SeqCalling fragments mapping to the appropriate regions of the genomic clones analyzed.

The regions defined by the procedures described above were then manually integrated and corrected for apparent inconsistencies that may have arisen, for example, from miscalled bases in the original fragments or from discrepancies between predicted exon junctions, EST locations and regions of sequence similarity, to derive the final sequence disclosed herein. When necessary, the process to identify and analyze SeqCalling assemblies and genomic clones was reiterated to derive the full length sequence (Alderborn et al., Determination of Single Nucleotide Polymorphisms by Real-time Pyrophosphate DNA Sequencing. Genome Research. 10 (8) 1249-1265, 2000).

Variants are reported individually but any combination of all or a select subset of variants are also included as contemplated NOVX embodiments of the invention.

**Table D1 NOV2a SNP Data CG180777-01**

| Variant | Nucleotides | | | Amino Acids | | |
|---|---|---|---|---|---|---|
| | Position | Initial | Modified | Position | Initial | Modified |
| 13382237 | 566 | A | G | 171 | His | Arg |

**Table D2 NOV4a SNP Data CG50183-01**

| Variant | **Nucleotides** | | | **Amino Acids** | | |
|---|---|---|---|---|---|---|
| | Position | Initial | Modified | Position | Initial | Modified |
| 13382220 | 148 | T | C | 49 | Val | Ala |

**Table D3 NOV10a SNP Data CG56151-01**

| Variant | **Nucleotides** | | | **Amino Acids** | | |
|---|---|---|---|---|---|---|
| | Position | Initial | Modified | Position | Initial | Modified |
| 13375160 | 77 | T | A | 13 | Thr | Thr |
| 13375159 | 86 | T | C | 16 | Thr | Thr |
| 13375158 | 242 | C | T | 68 | Pro | Pro |
| 13375157 | 367 | C | T | 110 | Thr | Ile |
| 13375156 | 421 | T | C | 128 | Met | Thr |
| 13375155 | 1301 | C | T | 421 | Phe | Phe |
| 13375153 | 1475 | C | T | 479 | Phe | Phe |
| 13375151 | 1526 | T | C | 496 | Ala | Ala |

**Table D4 NOV11a: SNP Variants for CG56155-02.**

| Variant | **Nucleotides** | | | **Amino Acids** | | |
|---|---|---|---|---|---|---|
| | Position | Initial | Modified | Position | Initial | Modified |
| 13374617 | 437 | A | G | 143 | Asn | Ser |
| 13375310 | 664 | T | G | 219 | Phe | Val |
| 13382227 | 1150 | G | T | 381 | Ala | Ser |
| 13375308 | 1210 | G | T | 401 | Glu | End |
| 13382226 | 1770 | C | T | 587 | Asn | Asn |

**Table D5 NOV18a SNP Data CG57758-02**

| **Variant** | **Nucleotides** | | | **Amino Acids** | | |
|---|---|---|---|---|---|---|
| | Position | Initial | Modified | Position | Initial | Modified |
| 13376438 | 1466 | T | C | 479 | Val | Ala |
| 13376435 | 1805 | T | C | 592 | Val | Ala |
| 13376434 | 1826 | T | C | 599 | Ile | Thr |

**Table D6 NOV19a SNP Data CG59693-01**

| **Variant** | **Nucleotides** | | | **Amino Acids** | | |
|---|---|---|---|---|---|---|
| | Position | Initial | Modified | Position | Initial | Modified |
| 13375931 | 259 | T | A | 87 | Cys | Ser |
| 13375927 | 357 | A | G | 119 | Pro | Pro |
| 13375928 | 593 | A | G | 198 | Asn | Ser |
| 13375925 | 666 | C | T | 222 | His | His |
| 13375924 | 783 | A | G | 261 | Leu | Leu |

**Table D7 NOV20a SNP Data CG93088-01**

| **Variant** | **Nucleotides** | | | **Amino Acids** | | |
|---|---|---|---|---|---|---|
| | **Position** | **Initial** | **Modified** | **Position** | **Initial** | **Modified** |
| 13377733 | 1458 | C | T | 399 | Ala | Val |

### Example E: CuraChip Gene Expression

CuraGen has developed a gene microarray (CuraChip 1.2) for target identification. It provides a high-throughput means of global mRNA expression analyses of CuraGen's collection of cDNA sequences representing the Pharmaceutically Tractable Genome (PTG). This sequence set includes genes which can be developed into protein therapeutics, or used to develop antibody or small molecule therapeutics. CuraChip 1.2 contains ~11,000 oligos representing approximately 8,500 gene loci, including (but not restricted to) kinases, ion channels, G-protein coupled receptors (GPCRs), nuclear hormone receptors, proteases, transporters, metabolic enzymes, hormones, growth factors, chemokines, cytokines, complement and coagulation factors, and cell surface receptors.

The CuraChip cDNAs were represented as 30-mer oligodeoxyribonucleotides (oligos) on a glass microchip. Hybridization methods using the longer CuraChip oligos are more specific compared to methods using 25-mer oligos. CuraChip oligos were synthesized with a linker, purified to remove truncated oligos (which can influence hybridization strength and specificity), and spotted on a glass slide. Oligo-dT primers were used to generate cRNA probes for hybridization from samples of interest. A biotin-avidin conjugation system was used to detect hybridized probes with a fluorophore-labeled secondary antibody. Gene expression was analyzed using clustering and correlation bioinformatics tools such as Spotfire® (Spotfire, Inc., 212 Elm Street, Somerville, MA 02144) and statistical tools such as multivariate analysis (MVA).

### Normalization method used in CuraChip software

The median fluorescence intensity of each spot and a background for each spot is read on a scale from 0 to 65,000. CuraGen's CuraChip software, developed in-house, has the capability to present the user with either the raw data (median intensities) or normalized data. If normalized data is chosen, the CuraChip software uses the following method to do mean normalization. The normalization is based on each slide/experiment.
- *fg_median* is the signal/foreground median for each slide/experiment;
- *bg_median* is the background median for each slide/experiment;
- *original_value* is the difference between *fg_median* and *bg_median;*
- *flag* is an indicator of a spot's success or failure, where 0 means success and 1 means failure;
- *raw_fg_mean* is the raw foreground mean for each slide/experiment;
- *raw_bg_mean* is the raw background mean for each slide/experiment;
- *trim_percentage* is the trim percentage for each slide/experiment; this could be defined by the user; currently we are using 2% as the trim percentage for each slide/experiment;
- *nSpots* is the number of spots on each slide;
- *nSlides* is the number of slides in each experiment;
- *fg_mean* is the trimmed foreground mean for each slide/experiment;
- *bg_mean* is the trimmed background mean for each slide/experiment;
- *max_fg_mean* is a constant among all slides/experiments, currently 2200.0;
- *normalized_value* is the final normalized value;
- *coeff* is the normalization co-efficient;
- MAX_VALUE is a constant representing the highest possible fluorescence reading, currently 65,000.

### Step 1. Calculate trimmed foreground and background means

For each slide/experiment, the trimmed foreground mean and the trimmed background mean of all spots are first calculated, suppose *nSpots,* on each slide. For each spot, if the data is acceptable (flag=0), we calculate the raw foreground mean and background mean are calculated by subtracting the background median from the foreground median for each spot. This is designated as a spot's "original value". (Note: If flag=1, all values are set to 0.)

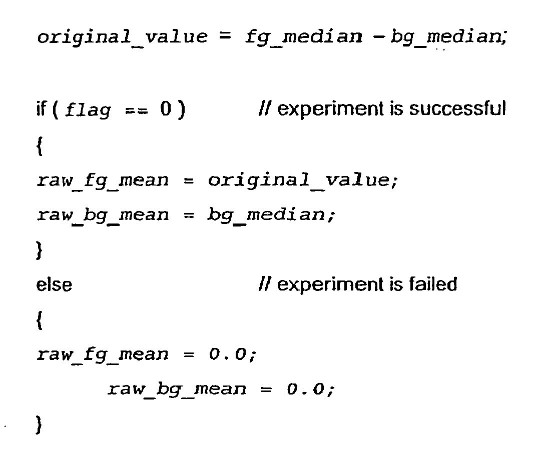

After that, the top and bottom 2% of data points are removed (trimmed) from the data set. After the above calculation, there are *nSpot* number of foreground means and background means for each slide/experiment, and both lists are sorted. For example: 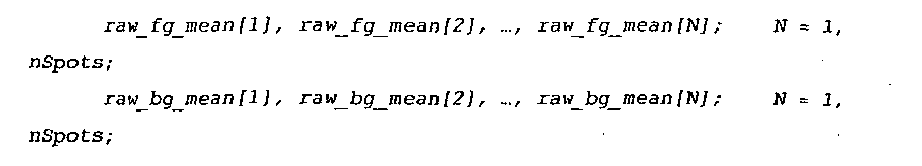 the trimmed data points are calculated for each slide/experiment. Suppose *a* is the trimmed start data point and *b* is the trimmed end data point, there are:
*a* = ***ceil***(*nSpots* * *trim_percentage* );
*b = **floor**(nSpots* *(1 - *trim*_*percentage*);

The "background mean" is calculated from the background medians for the trimmed data set. For the background mean, the average background mean is simply calculated in interval *[a,b]* then assign to *bg_mena:*

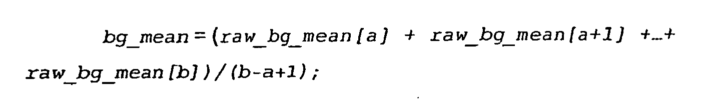

The "foreground mean" is calculated from the "original values" (i.e. background-subtracted spot signal medians); only "original values" greater than 500 are used for this calculation (excluding the trimmed top and bottom 2% ofthe data). Suppose the sum of those foreground means is *sum_raw_fg_mean* and the amount of those foreground means is k.

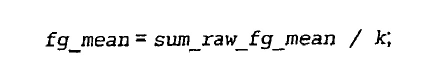

For clarity, a snippet code in Java looks like the following,

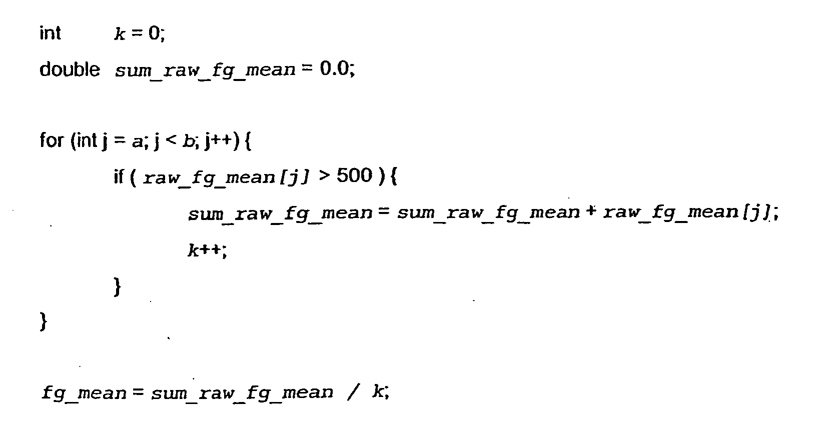

After the calculation of trimmed foreground means and background means for all slides is complete, the normalization procedure is started.

### Step 2. Normalize data

For each slide a normalization coefficient is calculated which compares the foreground mean of the slide to a fixed maximum foreground mean (2200). This coefficient is:

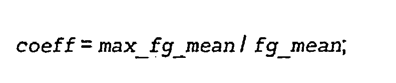

The normalized value of each spot is then calculated by multiplying the spot's "original value" by the normalization coefficient. Note that if this value is greater than the maximum reading of 65,000, then the value of 65,000 is used as the normalized value. Also note that if a spot's "original value" is less than the background value, the background value is used.

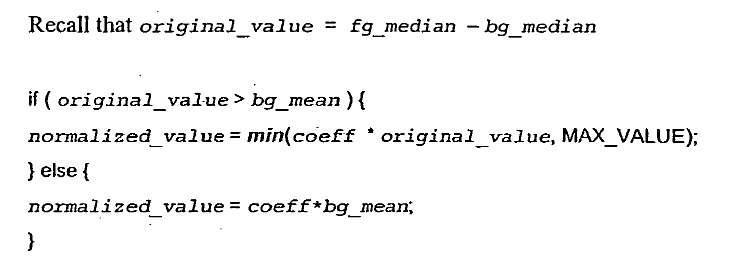

The *normalized_value* for each spot is the final (normalized) value used in the analysis

### Threshhold for CuraChip data analysis

A number of control spots are present on CuraChip 1.2 for efficiency calculations and to provide alternative normalization methods. For example, CuraChip 1.2 contains a number of empty or negative control spots, as well as positive control spots containing a dilution series of oligos that detect the highly-expressed genes Ubiquitin and glyceraldehyde-3-phosphate dehydrogenase (GAPD). An analysis of spot signal level was performed using raw data from 67 hybridizations using all oligos. The maximum signal intensity for each oligo across all 67 hybridizations was determined, and the fold-over-background for this maximum signal was calculated (i.e. if the background reading is 20 and the raw spot intensity is 100, then the fold-over-background for that spot is 5x). The negative control or empty spots do occasionally "fire" or give a signal over the background level; however, they do not fire very strongly, with 77.1% of empty spots firing <3x over background and 91.7% <5x (see burgundy bars in figure below). The positive control spots (Ubiquitin and GAPD, the light blue and dark blue bars, respectively) always fired at >100x background. The experimental oligos (CuraOligos, in yellow below) fired over the entire range of intensities, with some at low fold-over-background intensities. Since the negative control spots do fire occasionally at low levels, we have set a suggested threshhold for data analysis at >5x background.

### CG180777-01

**Results of PTG Chip 1.2**: One hundred seventy-eight samples of RNA from tissues obtained from surgically dissected tumors, non-diseased tissues from the corresponding organs and tumor xenografts grown in nude nu/nu mices were used to generate probes and run on PTG Chip 1.2. An oligo (optg2_0014957) that corresponds to CG180777-01 on the PTG Chip 1.2 was scrutinized for its expression profile. The statistical analyses identify strong expression in lung, melanomas and breast cancers.

Thus, based upon its profile, the expression of this gene could be of use as a marker for subsets of lung, melanomas and breast cancers, in addition to the subset of Kidney cancers as previously disclosed. In addition, therapeutic inhibition of the activity of the product of this gene, through the use of antibodies or small molecule drugs, may be useful in the therapy of lung, melanomas and breast cancers that express CG180777-01 and are dependent on them.

**Expression analysis of CG180777-01 using PTG Chip 1.2:** Approximately 418 samples ofRNA from tissues obtained from surgically dissected disease- and non-disease tissues, and treated and untreated cell lines, were used to generate labelled nucleic acid which was hybridized to PTG Chip 1.2. An oligo (optg2_0014957) that corresponds to CG180777-01 on the PTG Chip 1.2 was analyzed for its expression profile (Table 1).

This gene shows low expression in number of cancer cell lines, normal and cancer samples from lung, prostate, pancreas, breast, pancreas and kidney. Expression of this gene is upregulated in pancreatic and breast cancer. Therefore, expression of this gene may be used as marker to detect the presence of breast and pancreatic cancer. Furthermore, therapeutic modulation of the expression or function of this gene may be effective in the treatment of lung, pancreatic, breast, prostate, and kidney cancers.

In addition, this gene is expressed at low levels in the brains, including amygdala, anterior cingulate, thalamus, hippocampus and astrocytes. Therefore, therapeutic modulation of this gene product may be useful in the treatment of central nervous system disorders such as Parkinson's disease, epilepsy, multiple sclerosis, schizophrenia and depression.

This gene is also expressed at significant level in thyroid, and pituitary glands. Therefore, therapeutic modulation of the activity of this gene may prove useful in the treatment of endocrine/metabolically related diseases, such as obesity and diabetes.

Significant expression of this gene is also seen in fetal lung. Therefore, therapeutic modulation of this gene may be useful in the treatment of lung related diseases especially development related diseases.

Significant expression of this gene is also seen in resting and activated monocytes (THP1) cells. Upon activation with pathogens such as LPS, monocytes contribute to the innate and specific immunity by migrating to the site of tissue injury and releasing inflammatory cytokines. This release contributes to the inflammation process. Therefore, modulation of the expression of the protein encoded by this transcript may prevent the recruitment of monocytes and the initiation of the inflammatory process, and reduce the symptoms of patients suffering from autoimmune and inflammatory diseases such as asthma, allergies, inflammatory bowel disease, lupus erythematosus, or rheumatoid arthritis.

**Table E1:**

| **Sample** | **Foreground Mean** | **Background Mean** | **optg2_1201117** |
|---|---|---|---|
| Lung cancer(35C) | 2536.51 | 22.17 | 17 |
| Lung NAT(36A) | 2733.37 | 20.31 | 152 |
| Lung cancer(35E) | 2933.33 | 21.31 | 140 |
| Lung cancer(365) | 3808.15 | 19.58 | 180 |
| Lung cancer(368) | 3824.5 | 21.07 | 115.5 |
| Lung cancer(369) | 2825.08 | 18.76 | 550 |
| Lung cancer(36E) | 4152.87 | 26.78 | 60 |
| Lung NAT(36F) | 3538.73 | 23.55 | 207.5 |
| Lung cancer(370) | 4143.89 | 21.18 | 69 |
| Lung cancer(376) | 2446.38 | 20.81 | 26 |
| Lung cancer(378) | 3989.95 | 27.35 | 30 |
| Lung cancer(37A) | 4136.72 | 36.64 | 66 |
| Normal Lung 4 | 4083.27 | 28.46 | 168 |
| Normal Lung 5 | 4235.38 | 25.22 | 138 |
| CuraChip reference 1 | 3728.44 | 28.62 | 214 |
| Melanoma | 2915.57 | 20.5 | 87.5 |
| Melanoma | 2646.56 | 20.29 | 98 |
| Melanoma (19585) | 2509.13 | 23.23 | 38 |
| Normal Lung 1 | 2759.91 | 24.22 | 163.5 |
| Lung cancer(372) | 3803.04 | 27.08 | 63 |
| Lung NAT(35D) | 3771.95 | 25.68 | 283 |
| Lung NAT(361) | 2214.53 | 20.77 | 25 |
| Melanoma | 2134.94 | 21.43 | 62 |
| Normal Lung 2 | 3656.2 | 20.99 | 381 |
| Lung cancer(374) | 3295.08 | 24.19 | 746 |
| Lung cancer(36B) | 3776.14 | 21.32 | 175 |
| Lung cancer(362) | 1543.94 | 26.44 | 34 |
| Lung cancer(358) | 1929.4 | 30.01 | 83 |
| Melanoma | 2375.7 | 20.83 | 81 |
| Normal Lung 3 | 3157.31 | 22.69 | 255.5 |
| Lung NAT(375) | 4614.72 | 32.86 | 514 |
| Lung cancer(36D) | 2785.76 | 24.74 | 31 |
| Lung NAT(363) | 4348.91 | 34.21 | 247 |
| Lung cancer(35A) | 3986.34 | 29.19 | 184 |
| Melanoma | 2189.36 | 20.44 | 27 |
| Prostate cancer(B8B) | 2957.66 | 9.6 | 277 |
| Prostate cancer(B88) | 4126.76 | 33.25 | 389 |
| Prostate NAT(B93) | 3378.81 | 37.92 | 286 |
| Prostate cancer(B8C) | 3527 | 42.55 | 273.5 |
| Prostate cancer(AD5) | 4105.44 | 45.35 | 395 |
| Prostate NAT(AD6) | 4196.5 | 41.71 | 522 |
| Prostate cancer(AD7) | 2830.59 | 42.73 | 193.5 |
| Prostate NAT(AD8) | 3404.14 | 29.72 | 346 |
| Prostate cancer(ADA) | 3700.09 | 34:54 | 715 |
| Prostate NAT(AD9) | 3022.26 | 30.92 | 255.5 |
| Prostate cancer(9E7) | 3084.26 | 30.48 | 261.5 |
| Prostate cancer(A0A) | 3983.11 | 24.56 | 499 |
| Prostate cancer(9E2) | 2889.43 | 23.94 | 426 |
| Pancreatic cancer(9E4) | 4473.72 | 23.53 | 400.5 |
| Pancreatic cancer(9D8) | 3443.44 | 20.25 | 193.5 |
| Pancreatic cancer(9D4) | 3819.27 | 17.3 | 315 |
| Pancreatic cancer(9BE) | 3287.48 | 24.17 | 588 |
| Pancreatic NAT(ADB) | 2358 | 28.92 | 63.5 |
| Pancreatic NAT(ADC) | 2863.88 | 36.96 | 42 |
| Pancreatic NAT(ADD) | 3118.81 | 30.22 | 32 |
| Pancreatic NAT(AED) | 3211.96 | 26.31 | 102 |
| Colon cancer(8A3) | 1984.83 | 48.06 | 51 |
| Colon NAT(8B6) | 1682.5 | 39.46 | 61 |
| Colon NAT(9F1) | 2378.93 | 48.8 | 74 |
| Colon cancer(9F2) | 1931.28 | 46.1 | 20 |
| Colon NAT(A1D) | 2029.41 | 46.05 | 65 |
| Colon cancer(9DB) | 2278.96 | 44.08 | 16 |
| Colon NAT(A15) | 1674.01 | 45.41 | 31 |
| Colon cancer(A14) | 1360.97 | 35.04 | 11 |
| Colon NAT(ACB) | 1707.6 | 45.03 | 29 |
| Colon cancer(ACO) | 1894.33 | 46.06 | 16 |
| Colon cancer(8A4) | 1785.56 | 43.34 | 10 |
| Colon NAT(ACD) | 1797.75 | 44.1 | 48 |
| Colon cancer(AC4) | 2198.75 | 49.26 | 28 |
| Colon NAT(AC2) | 1847.84 | 43.83 | 32.5 |
| Colon cancer(AC1) | 1806.35 | 39.49 | 15 |
| Colon NAT(ACC) | 2013.34 | 39.08 | 68 |
| Colon cancer(AC3) | 1539.46 | 47.71 | 16.5 |
| Breast cancer(9B7) | 1857.03 | 46.65 | 122 |
| Breast NAT(9CF) | 1462.79 | 47.35 | 7 |
| Breast cancer(9B6) | 2133.12 | 47.71 | 54 |
| Breast cancer(9C7) | 2302.99 | 47.48 | 116 |
| Colon cancer(8A6) | 2093.72 | 45.39 | 71 |
| Breast NAT(A11) | 1508.35 | 45.43 | 29 |
| Breast cancer(A1A) | 2246.51 | 46.55 | 29 |
| Breast cancer(9F3) | 1881.09 | 46.54 | 30 |
| Breast cancer(9B8) | 2174.46 | 48.66 | 69 |
| Breast NAT(9C4) | 1670.58 | 48.93 | 43 |
| Breast cancer(9EF) | 1168.07 | 23.61 | 29.5 |
| Breast cancer(9FO) | 1506.95 | 28.54 | 55 |
| Breast cancer(9B4) | 1016.05 | 32.36 | 9 |
| Breast cancer(9EC) | 2526.83 | 47.27 | 74 |
| Colon cancer(8A7) | 1594.35 | 48.21 | 5 |
| Colon cancer(8B7) | 2091.33 | 40.64 | 31 |
| Colon cancer(8A9) | 2533.34 | 40.66 | 102 |
| Colon cancer(8AB) | 1638.43 | 30.62 | 10 |
| Colon cancer(8AC) | 1975.26 | 41.39 | 13 |
| Colon NAT(8AD) | 1851.09 | 49.21 | 86 |
| Colon cancer(8B5) | 1920.15 | 47.11 | 22.5 |
| Cervical cancer(B08) | 1393.31 | 2.02 | 0 |
| Brain cancer(9F8) | 1400.44 | 8.86 | 25.5 |
| Brain cancer(9C0) | 655.35 | 4.73 | 0 |
| Brain cancer(9F7) | 1403.07 | 0.42 | 9.5 |
| Brain cancer(A00) | 1509.09 | 3.25 | 3.5 |
| Brain NAT(A01) | - 1159.94 | 0.43 | 11 |
| Brain cancer(9DA) | 1019.67 | 0.72 | 0.5 |
| Brain cancer(9FE) | 1352.85 | 2.77 | 5 |
| Brain cancer(9C6) | 1237.61 | 3.47 | 0 |
| Brain cancer(9F6) | 917.48 | 2.17 | 0 |
| Cervical NAT(AEB) | 826.9 | 1.64 | 0 |
| Bladder NAT(23954) | 521.75 | 0.44 | 0 |
| Urinary cancer(AF6) | 1007.77 | 1.4 | 0 |
| Urinary cancer(B0C) | 1256.43 | 1.31 | 0 |
| Urinary cancer(AE4) | 1219.17 | 1.23 | 2.5 |
| Urinary NAT(B20) | 1222.48 | 1.23 | 0 |
| Urinary cancer(AE6) | 1114.91 | 1.03 | 0 |
| Urinary NAT(B04) | 655.35 | 0.07 | 0 |
| Urinary cancer(B07) | 543.73 | 1.64 | 0 |
| Urinary NAT(AF8) | 1247.4 | 0.53 | 0 |
| Cervical cancer(AFF) | 1411.18 | 4.31 | 0 |
| Ovarian cancer(9D7) | 1221.47 | 0.63 | 35 |
| Urinary cancer(AF7) | 1138.73 | 1.3 | 0 |
| Ovarian cancer(9F5) | 1298.98 | 0 | 5.5 |
| Ovarian cancer(A05) | 1134.77 | 2.16 | 17 |
| Ovarian cancer(9BC) | 505 | 0.15 | 0 |
| Ovarian cancer(9C2) | 1025.23 | 0.93 | 0 |
| Ovarian cancer(9D9) | 1203.34 | 1.53 | 0 |
| Ovarian NAT(AC7) | 685.35 | 0.54 | 0 |
| Ovarian NAT(AC9) | 716.79 | 0.85 | 0 |
| Ovarian NAT(ACA) | 628.62 | 2.38 | 0 |
| Cervical NAT(B1E) | 1293.21 | 7.01 | 0 |
| Ovarian NAT(AC5) | 542.12 | 0.99 | 0 |
| Cervical cancer(B00) | 1512.53 | 9.92 | 0 |
| Cervical NAT(AFA) | 1136.08 | 8.76 | 0 |
| Cervical cancer(B1F) | 1782.82 | 18.96 | 0 |
| Cervical NAT(B1C) | 655.35 | 2.36 | 0 |
| Brain cancer(9F9) | 1508.5 | 5.08 | 5.5 |
| Breast cancer(D34) | 2470.88 | 0 | 70 |
| Breast cancer(D35) | 2602.08 | 0 | 63 |
| Breast cancer(D36) | 2909.53 | 0 | 130.5 |
| Breast cancer(D37) | 2811.77 | 0.05 | 173 |
| Breast cancer(D38) | 2986.78 | 0.38 | 150 |
| Breast cancer(D39) | 3026.22 | 0.04 | 286 |
| Breast cancer(D3A) | 3072.62 | 0.08 | 235 |
| Breast cancer(D3B) | 2571.28 | 0.02 | 99.5 |
| Breast cancer(D3C) | 3213.98 | 0.6 | 271 |
| Breast cancer(D3D) | 3484.57 | 2.5 | 135 |
| Breast cancer(D3E) | 2958.51 | 0.17 | 458 |
| Breast cancer(D3F) | 2937.01 | 1.88 | 135 |
| Breast cancer(D40) | 2751.61 | 1.2 | 369 |
| Breast cancer(D42) | 2171.59 | 0.8 | 145 |
| Breast cancer(D43) | 2962.09 | 4.5 | 166.5 |
| Breast cancer(D44) | 2558.08 | 2.95 | 176 |
| Breast cancer(D45) | 2667.3 | 3.59 | 177.5 |
| Breast cancer(D46) | 3190.77 | 2.25 | 190.5 |
| SK-MES | 2804.32 | 0.56 | 47 |
| HLaC-79 | 3402.37 | 0 | 83 |
| H226 | 2562.59 | 0 | 0 |
| HCT-116 | 4221.68 | 0.09 | 74 |
| IGROV-1 | 3243.07 | 0 | 130.5 |
| MX-1 | 3253.75 | 0 | 211 |
| C33A | 3249.59 | 0 | 265 |
| Daudi | 2333.08 | 0.01 | 0 |
| MV522 | 2727.71 | 0.94 | 95 |
| RWP-2 | 2906.49 | 0 | 28 |
| BON | 2502.53 | 0.01 | 93 |
| MiaPaCa | 3604.78 | 0 | 155 |
| H82 | 2357.18 | 2.19 | 98 |
| H69 | 2759.55 | 0.12 | 0 |
| Caki-2 | 2687.93 | 0 | 72 |
| LNCaP | 3352.46 | 0.41 | 135.5 |
| A549 | 2593.12 | 0 | 0 |
| DU145 | 3970.51 | 0.07 | 78 |
| OVCAR-3 | 3230.65 | 0.14 | 275 |
| HT-29 | 3381.64 | 0.07 | 137 |
| DLD-2 | 3610.05 | 0.24 | 39.5 |
| MCF-7 | 3326.73 | 1.78 | 264 |
| H460 | 2464.22 | 0 | 0 |
| SW620 | 2732.11 | 0 | 0 |
| SK-OV-3 | 3519.75 | 0 | 101.5 |
| MDA-231 | 3464.04 | 0.04 | 0 |
| Caki-1 | 3801.64 | 0 | 88.5 |
| PC-3 | 2214.23 | 0 | 0 |
| LoVo | 3237.95 | 0 | 27 |
| Kidney NAT(10B1) | 3041.27 | 6.44 | 235.5 |
| Kidney cancer(10B2) | 3798.53 | 1.31 | 622.5 |
| Kidney NAT(10B3) | 3315.43 | 0 | 168 |
| Kidney cancer(10B4) | 3519.14 | 0.16 | 89.5 |
| Kidney NAT(10B5) | 3017.75 | 0 | 106.5 |
| Kidney cancer(10B6) | 3702.61 | 0 | 69.5 |
| Kidney NAT(10B7) | 3060.89 | 0 | 103.5 |
| Kidney cancer(10BA) | 3437.01 | 0 | 86.5 |
| Kidney NAT(10BB) | 3157.99 | 0 | 159.5 |
| Kidney cancer(10C0) | 3590.87 | 0.77 | 495 |
| Kidney NAT(10C1) | 3012.3 | 0 | 164.5 |
| Kidney cancer(10C4) | 3186.48 | 0.02 | 157 |
| Kidney NAT(10C5) | 3618.74 | 0 | 79 |
| Kidney cancer(10A8) | 3514.51 | 0 | 35.5 |
| KidneyNAT(10A9) | 2771.76 | 1.14 | 156.5 |
| Kidney cancer(10AA) | 3793.55 | 0.4 | 250.5 |
| Kidney NAT(10AB) | 2978.06 | 0 | 166 |
| Kidney cancer(10AC) | 3656.35 | 0.05 | 188 |
| Kidney NAT(10AD) | 3299.97 | 2.13 | 228 |
| Kidney cancer(10AE) | 3456.21 | 0.57 | 169.5 |
| Kidney NAT(10AF) | 2593.7 | 0 | 155 |
| Kidney cancer(10B0) | 3529.2 | 0.67 | 283.5 |
| Lymphoma(9BF) | 2333.81 | 0 | 0 |
| Lymphoma(9D2) | 1327.77 | 0 | 0 |
| Lymphoma(A04) | 1450.41 | 0 | 14 |
| Lymphoma(9DD) | 1095.68 | 0 | 0 |
| Lymphoma(F68) | 865.62 | 0 | 0 |
| Lymphoma(F6A) | 862.12 | 0 | 0 |
| Lymphoma(F6B) | 624.66 | 0 | 0 |
| Lymphoma(F6C) | 1621.52 | 0 | 0 |
| Lymphoma(F6D) | 864.76 | 0 | 0 |
| Lymphoma(F6E) | 1030.71 | 0 | 0 |
| Lymphoma(F6F) | 1120.14 | 0 | 0 |
| Lymphoma(F70) | 891.53 | 0 | 0 |
| Lymphoma(F71) | 915.92 | 0 | 0 |
| Lymphoma(F72) | 1199.28 | 3.13 | 0 |
| Lymphoma(F73) | 1357.46 | 0 | 0 |
| Lymphoma(F74) | 993.02 | 0 | 0 |
| Lymphoma NAT(1002) | 2069.54 | 0 | 0 |
| Lymphoma NAT(1004) | 1928.51 | 0 | 0 |
| Lymphoma NAT(1005) | 1412.32 | 0 | 0 |
| Lymphoma NAT(1007) | 1668.28 | 0 | 0 |
| Lymphoma NAT(1003) | 1919.49 | 0 | 0 |
| Lymphoma(9E3) | 1791.94 | 0 | 0 |
| Lymphoma(9D0) | 1530.59 | 0 | 0 |
| Lymphoma(9E1) | 1677.97 | 0 | 0 |
| Lymphoma(A0D) | 2656.34 | 1.4 | 1.5 |
| Lymphoma(9B5) | 2336.21 | 0 | 0 |
| Lymphoma(9D3) | 1902.13 | 0 | 0 |
| Normal Lung 4 | 4083.27 | 28.46 | 90.52 |
| Normal Lung 5 | 4235.38 | 25.22 | 71.68 |
| Normal Lung 1 | 2759.91 | 24.22 | 130.33 |
| Normal Lung 2 | 3656.2 | 20.99 | 229.25 |
| Normal Lung 3 | 3157.31 | 22.69 | 178.03 |
| SW 1353 resting 1h | 3946.45 | 0.6 | 22.86 |
| SW 1353 resting 6h | 3263.46 | 0.12 | 17.86 |
| SW 1353 resting 16h | 2311.8 | 0 | 0 |
| SW 1353 IL-1b (1 ng/) 1h | 2686.83 | 0.16 | 7.37 |
| SW 1353 IL-1b (1 ng/) 6h | 3159.94 | 2 | 6.27 |
| SW1353 IL-1b (1 ng/) 16h | 3557.88 | 0.62 | 17.93 |
| SW1353 FGF20 (1 ug/) 1h | 3512.56 | 0.69 | 26.93 |
| SW1353 FGF20 (1 ug/) 16h | 2510.14 | 0.06 | 1.75 |
| SW1353 FGF20 (5 ug/) 1h | 3448.11 | 1.01 | 22.33 |
| SW1353 FGF20 (5 ug/) 6h | 3598.07 | 0.64 | 18.65 |
| SW1353 FGF20 (5 ug/) 16h | 3687.34 | 4.42 | 21.78 |
| SW1353 FGF20 (1 ug/) IL-1b (1 ng/) 6h | 3569.19 | 0.17 | 20.34 |
| SW 1353 FGF20 (1 ug/) IL-1b (1 ng/) 16h | 3970.28 | 0.01 | 36.57 |
| SW1353 FGF20 (5 ug/) IL-1b (1 ng/) 1h | 3011.45 | 0 | 5.11 |
| SW1353 FGF20 (5 ug/) IL-1b (1 ng/) 6h | 3184.88 | 2.69 | 31.43 |
| THP-1 aCD40 (1 ug/) 1h | 3545.99 | 0.07 | 181.16 |
| THP-1 aCD40 (1 ug/) 6h | 2882.56 | 0.44 | 76.7 |
| THP-1 LPS (100 ng/) 1h | 3131.64 | 0.74 | 129.26 |
| THP-1 LPS (100 ng/) 6h | 2356.5 | 0.05 | 19.61 |
| CCD1070SK TNFa (5 ng/) 6h | 2888.01 | 0.78 | 32.76 |
| CCD1070SK TNFa (5 ng/)24h | 3029 | 0.39 | 51.57 |
| CCD1070SK IL-1b (1 ng/) 24h | 3307.91 | 5.76 | 35.58 |
| THP-1 resting | 3080.68 | 0.87 | 157.47 |
| THP-1 aCD40 (1 ug/) 24h | 2032.49 | 0.82 | 33.55 |
| THP-1 LPS (100 ng/) 24h | 1.597.29 | 3.58 | 44.76 |
| CCD1070SK IL-1b (1 ng/) 6h | 3026.56 | 3.51 | 43.61 |
| LC 18hr | 2725.87 | 0.36 | 34.7 |
| LC-IL-! 18hr | 1474.79 | 2.66 | 0 |
| Astrocyte IL 1B 1hr a | 3116.77 | 10.04 | 264.34 |
| Astrocyte IL 1B 6 hr a | 3119.7 | 16.51 | 357.18 |
| Astrocyte IL 1B 24 hr a | 3142.98 | 13.81 | 335.99 |
| SHSY 5Y Undifferentiated | 3166.23 | 14.61 | 105.61 |
| SHSY 5Y Differentiated | 2959.01 | 8.5 | 100.74 |
| LC 0hr | 1880.7 | 0 | 0 |
| Normal Fetal Kidney | 2011.8 | 0 | 7.65 |
| Normal Liver | 2053.67 | 3.81 | 0 |
| Normal Fetal Liver | 3555.17 | 0 | 3.09 |
| Normal Fetal Lung | 4164.55 | | 0 208.4 |
| Normal Salivary Gland | 3466.36 | 0 | 0 |
| Normal Fetal Skeletal Muscle | 2504.59 | 0 | 0 |
| Normal Thyroid | 3566.17 | 0 | 193.71 |
| Normal Trachea | 3596.15 | 0 | 61.18 |
| LC-IL-1 0 hr | 2560.23 | 0 | 0.86 |
| Heart pool | 3167.78 | 0 | 0 |
| Pituitary Pool | 2908.48 | 0 | 316.94 |
| Spleen Pool | 2068.62 | 0 | 5.32 |
| Stomach Pool | 2826.7 | 0 | 9.34 |
| Testis Pool | 3348.7 | 0 | 268.04 |
| Thymus Pool | 2653.82 | 0 | 45.59 |
| Small Intestine- 5 donor pool | 3795.64 | 0 | 68.39 |
| Lymph node- 5 donor pool | 4339.52 | 0 | 69.45 |
| Kidney- 5 donor pool | 3347.34 | 0 | 138.02 |
| Jurkat Resting | 3779.74 | 0.48 | 139.11 |
| Jurkat CD3 (500ng/ml) 6hr A | 2459.46 | 1.68 | 71.56 |
| Jurkat CD3 (500ng/ml) 24hr A | 1897.6 | 0.08 | 71.3 |
| Jurkat CD3 (500ng/ml)+CD28(1ug/mt) 6hr A | 1867.35 | 0.68 | 24.74 |
| Jurkat CD3 (500ng/ml)+CD28(1 ug/ml) 24hr A | 1574.07 | 0.35 | 60.1 |
| control (no treatment)_1 hr | 5400.28 | 2.01 | 65.59 |
| 10ng/ml IL-1b 1 hr | 5250.91 | 1.51 | 70.39 |
| 10ng/ml TNF-a_1 hr | 5668.06 | 2.55 | 80.73 |
| 200uM BzATP 1 hr | 5619.87 | 0.31 | 89.65 |
| control (no treatment)_5 hr | 5630.13 | 1.02 | 77.56 |
| 10ng/ml IL-1b 5 hr | 6332.12 | 10.52 | 70.36 |
| 10ng/ml TNF-a 5 hr | 6070.17 | 6.37 | 75.93 |
| 200uM BzATP 5 hr | 6425.22 | 2.39 | 99.64 |
| control (no treatment)_24 hr | 4825.87 | 3.5 | 62 |
| 10ng/ml IL-1b 24 hr | 5349.28 | 9.73 | 73.41 |
| 10ng/ml TNF-a 24 hr | 5672.31 | 4.97 | 62.25 |
| 200uM BzATP 24 hr | 4814.1 | 0.51 | 70.61 |
| Alzheimer's disease B4951 | 1854.86 | 14.76 | 33.21 |
| Alzheimer's disease B4953 | 2540.02 | 19.95 | 108.27 |
| Alzheimer's disease B5018 | 1757.68 | 22.42 | 45.06 |
| Alzheimer's disease B5019 | 1491.9 | 18.37 | 7.37 |
| Alzheimer's disease B5086 | 2247.49 | 18.04 | 66.56 |
| Alzheimer's disease B5096 | 2150.92 | 19.76 | 38.87 |
| Alzheimer's disease B5098 | 732.56 | 15.93 | 21.02 |
| Alzheimer's disease B5129 | 1841.99 | 18.04 | 9.55 |
| Alzheimer's disease B5210 | 3233.87 | 21.56 | 168.37 |
| Control B4810 | 2987.22 | 21.85 | 155.76 |
| Control B4825 | 2903.91 | 18.74 | 187.88 |
| Control B4930 | 2287.12 | 22.69 | 30.78 |
| Control B4932 | 3424.98 | 20.12 | 370.95 |
| Control B5024 | 3859.32 | 22.42 | 398.46 |
| Control B5113 | 1897 | 17.87 | 112.49 |
| Control B5140 | 1901.93 | 18.88 | 68.25 |
| Control B5190 | 1284.69 | 15.26 | 53.94 |
| Control B5220 | 2225.75 | 18.7 | 192.25 |
| Control B5245 | 2119.39 | 21.5 | 143.25 |
| AH3 B3791 | 2202.74 | 19.73 | 173.78 |
| AH3 B3855 | 1849.89 | 17.93 | 267.58 |
| AH3 B3877 | 2144.15 | 17.01 | 402.72 |
| AH3 B3893 | 2103.76 | 16.34 | 81.57 |
| AH3 B3894 | 1820.82 | 17.31 | 166.74 |
| AH3 83949 | 1607.86 | 23.9 | 58.84 |
| AH3 B4477 | 1602.58 | 20.32 | 123.55 |
| AH3 B4540 | 2260.92 | 22.79 | 252.02 |
| AH3 B4577 | 2142.42 | 22.59 | 257.75 |
| AH3 84639 | 1550.43 | 21.89 | 28.38 |
| Schizophrenia hippocampus 683 | 2468.43 | 21.23 | 58.82 |
| Depression hippocampus 487 | 1473.83 | 18 | 0 |
| Depression hippocampus 600 | 2481.12 | 10.69 | 58.97 |
| Normal hippocampus 2407a | 2624.25 | 33.19 | 18.44 |
| Normal hippocampus 1042 | 2114.72 | 21.4 | 19.25 |
| Depression hippocampus 2767 | 1448.13 | 10.6 | 21.27 |
| Depression hippocampus 567 | 1836.77 | 47.98 | 19.16 |
| Control hippocampus 3175 | 2752.14 | 14.38 | 96.72 |
| Depression hippocampus 3096 | 1735.6 | 9.4 | 30.42 |
| Depression hippocampus 1491 | 2784.26 | 17.28 | 41.09 |
| Depression hippocampus 2540 | 2241.25 | 16.73 | 28.47 |
| Schizophrenia hippocampus 2798 | 1923.26 | 16.97 | 56.05 |
| Control hippocampus 1973 | 2605.59 | 14.97 | 33.77 |
| Normal hippocampus and amygdala 2601 | 2031.45 | 13.85 | 34.11 |
| Schizophrenia hippocampus 2785 | 1621.43 | 21.03 | 0 |
| Schizophrenia hippocampus 484 | 3271.31 | 39.54 | 62.21 |
| Normal hippocampus 2556 | 2806.68 | 21.69 | 50.95 |
| Depression hippocampus 1158 | 2705.56 | 15.83 | 32.53 |
| Control hippocampus 552 | 3378.83 | 21.65 | 161.48 |
| Schizophrenia hippocampus 1737 | 2304.06 | 12.06 | 0 |
| Normal hippocampus 1239 | 3335.3 | 17.54 | 67.28 |
| Normal hippocampus 1465 | 3068.38 | 14.64 | 49.47 |
| Normal hippocampus 3080 | 1323.77 | 4.6 | 0 |
| Normal hippocampus 738 | 4229.67 | 12.96 | 146.16 |
| Schizophrenia hippocampus 2586 | 2067.82 | 12.78 | 6.92 |
| Normal hippocampus 2551 | 3306.02 | 13.41 | 156.38 |
| Depression hippocampus 588 | 2352.1 | 13.53 | 75.76 |
| Depression hippocampus 529 | 3291.7 | 13.05 | 127.65 |
| Depression hippocampus and dentate gyrus | 1686.08 | 11.37 | 27.4 |
| Schizophrenia amygdala 2586 | 2136.57 | 0.55 | 40.16 |
| Normal substantia nigra 234 | 1240.83 | 0.03 | 0 |
| Normal substantia nigra 1065 | 1515.69 | 14.19 | 0 |
| Normal substantia nigra 3236 | 1341.77 | 0 | 0 |
| Normal substantia nigra 2551 | 3718.91 | 0.43 | 64.19 |
| Normal substantia nigra 1597 | 1003.1 | 0 | 0 |
| Control thalamus 552 | 948.15 | 0.02 | 4.64 |
| Control thalamus 566 | 1698.42 | 0 | 0 |
| Control thalamus 606 | 2625.59 | 0.31 | 96.78 |
| Control thalamus 738 | 2464.52 | 0 | 42.85 |
| Control thalamus 1065 | 2991.38 | 0 | 54.42 |
| Control thalamus 1092 | 2416.75 | 0 | 36.41 |
| Control thalamus 1597 | 2389.84 | 0 | 48.79 |
| Control thalamus 2253 | 1602.4 | 0 | 0 |
| Control thalamus 2551 | 3017.98 | 0 | 120.28 |
| Depression thalamus 588 | 2245.45 | 0.29 | 31.35 |
| Depression thalamus 600 | 1442.56 | 0 | 0 |
| Depression thalamus 721 | 1921.28 | 0 | 0 |
| Depression thalamus 728 | 3113.17 | 4.33 | 48.05 |
| Depression thalamus 759 | 2433.85 | 0 | 56.95 |
| Depression thalamus 881 | 2456.7 | 0 | 15.67 |
| Schizophrenia thalamus 477 | 1952.08 | 0 | 3.38 |
| Schizophrenia thalamus 532 | 3553.95 | 0 | 68.71 |
| Schizophrenia thalamus 683 | 3798.02 | 0 | 50.11 |
| Schizophrenia thalamus 544 | 3260.82 | 0.03 | 44.87 |
| Schizophrenia thalamus 1671 | 2246.95 | 0 | 0 |
| Schizophrenia thalamus 1737 | 1958.75 | 0.01 | 0 |
| Schizophrenia thalamus 2464 | 1953.64 | 0 | 56.31 |
| Schizophrenia thalamus 2586 | 3338.49 | 0 | 28.67 |
| Depression amygdala 600 | 1936.43 | 0 | 9.09 |
| Depression amygdala 759 | 2378.2 | 0 | 69.38 |
| Depression anterior cingulate 759 | 2808.48 | 0 | 75.98 |
| Control amygdala 552 | 3675.87 | 0.36 | 151.42 |
| Control anterior cingulate 482 | 3115.46 | 0.67 | 236.56 |
| Depression anterior cingulate 721 | 1964.87 | 0 | 1.12 |
| Control amygdala 3175 | 2674.16 | 0.04 | 134.92 |
| Depression anterior cingulate 600 | 2389.83 | 0.06 | 37.74 |
| Depression anterior cingulate 588 | 2629.92 | 0 | 92.85 |
| Control anterior cingulate 3175 | 3605.03 | 0.79 | 238.61 |
| Control anterior cingulate 606 | 2414.64 | 0 | 273.33 |
| Depression anterior cingulate 567 | 2397.28 | 1.2 | 40.38 |
| Depression amygdala 588 | 3410.76 | 3.1 | 79.98 |
| Control anterior cingulate 3080 | 2445.09 | 3.01 | 54.89 |
| Control anterior cingulate 2601 | 2520 | 5.45 | 75.08 |
| Control anterior cingulate 1042 | 3118.04 | 0.76 | 87.49 |
| Control anterior cingulate 3236 | 2913.66 | 1.45 | NA |
| Control amygdala 1502 | 4253.4 | 9.62 | 147.67 |
| Control anterior cingulate 807 | 2624.08 | 8.86 | 0 |
| Control amygdala 1597 | 3710.89 | 11.88 | 141.1 |
| Parkinson's substantia nigra 2842 | 2457.51 | 0.25 | 11.64 |
| Parkinson's substantia nigra 2917 | 1548.58 | 0 | 0 |
| Schizophrenia amygdala 544 | 2009.89 | 0 | 71.15 |
| Schizophrenia amygdala 532 | 730.94 | 0 | 0 |
| Depression amygdala 2540 | 2408.1 | 0 | 28.32 |
| Parkinson's substantia nigra 2899 | 1544.99 | 0 | 0 |
| Depression anterior cingulate 881 | 3015.65 | 0 | 103.59 |

### CG181825-01

Results of PTG Chip 1.2: One hundred seventy-eight samples of RNA from tissues obtained from surgically dissected tumors, non-diseased tissues from the corresponding organs and tumor xenografts grown in nude nu/nu mices were used to generate probes and run on PTG Chip 1.2. An oligo (optg2_1206388) that corresponds to CG181825-01 on the PTG Chip 1.2 was scrutinized for its expression profile. The statistical analysis identify strong expression in lung, melanomas and breast cancers.

Thus, based upon its profile, the expression of this gene could be of use as a marker for subsets of lung, melanomas and breast cancers, in addition to the subset of Kidney cancers as previously disclosed. In addition, therapeutic inhibition of the activity of the product of this gene, through the use of antibodies or small molecule drugs, may be useful in the therapy of lung, melanomas and breast cancers that express CG181825-01 and are dependent on them.

**Expression analysis of CG181825-01 using PTG Chip 1.2:** Approximately 418 samples ofRNA from tissues obtained from surgically dissected disease- and non-disease tissues, and treated and untreated cell lines, were used to generate labelled nucleic acid which was hybridized to PTG Chip 1.2. An oligo (optg2_1206388) that corresponds to CG181825-01 on the PTG Chip 1.2 was analyzed for its expression profile (Table 1).

This gene shows low expression in number of cancer cell lines, normal and cancer samples from lung, pancreas, breast, cervix and kidney.

In addition, this gene is expressed at low levels in the brains of an independent group of individuals, especially in substantia nigra and amygdala of samples from normal patients or patients suffering with Parkinson's diseases and schizophrenia.

This gene is also expressed at significant level in thyroid, pituitary glands, fetal liver, and stomach. Therefore, therapeutic modulation of the activity of this gene may prove useful in the treatment of endocrine/metabolically related diseases, such as obesity and diabetes.

**Table E2:**

| **Sample** | **Foreground Mean** | **Background Mean** | **optg2_1206388** |
|---|---|---|---|
| Lung cancer(35C) | 2536.51 | 22.17 | 33 |
| Lung NAT(36A) | 2733.37 | 20.31 | 47.5 |
| Lung cancer(35E) | 2933.33 | 21.31 | 60 |
| Lung cancer(365) | 3808.15 | 19.58 | 159 |
| Lung cancer(368) | 3824.5 | 21.07 | 114.5 |
| Lung cancer(369) | 2825.08 | 18.76 | 40 |
| Lung cancer(36E) | 4152.87 | 26.78 | 132 |
| Lung NAT(36F) | 3538.73 | 23.55 | 87 |
| Lung cancer(370) | 4143.89 | 21.18 | 40 |
| Lung cancer(376) | 2446.38 | 20.81 | 34 |
| Lung cancer(378) | 3989.95 | 27.35 | 30 |
| Lung cancer(37A) | 4136.72 | 36.64 | 123.5 |
| Normal Lung 4 | 4083.27 | 28.46 | 85 |
| Normal Lung 5 | 4235.38 | 25.22 | 95 |
| CuraChip reference 1 | 3728.44 | 28.62 | 49 |
| Melanoma | 2915.57 | 20.5 | 25 |
| Melanoma | 2646.56 | 20.29 | 18 |
| Melanoma (19585) | 2509.13 | 23.23 | 16.5 |
| Normal Lung 1 | 2759.91 | 24.22 | 32 |
| Lungcancer(372) | 3803.04 | 27.08 | 30 |
| Lung NAT(35D) | 3771.95 | 25.68 | 147 |
| Lung NAT(361) | 2214.53 | 20.77 | 49.5 |
| Melanoma | 2134.94 | 21.43 | 17 |
| Normal Lung 2 | 3656.2 | 20.99 | 69 |
| Lung cancer(374) | 3295.08 | 24.19 | 72 |
| Lung cancer(36B) | 3776.14 | 21.32 | 84 |
| Lung cancer(362) | 1543.94 | 26.44 | 40 |
| Lung cancer(358) | 1929.4 | 30.01 | 51 |
| Melanoma | 2375.7 | 20.83 | 22 |
| Normal Lung 3 | 3157.31 | 22.69 | 34 |
| Lung NAT(375) | 4614.72 | 32.86 | 96.5 |
| Lung cancer(36D) | 2785.76 | 24.74 | 66.5 |
| Lung NAT(363) | 4348.91 | 34.21 | 76 |
| Lung cancer(35A) | 3986.34 | 29.19 | 117 |
| Melanoma | 2189.36 | 20.44 | 16 |
| Prostate cancer(B8B) | 2957.66 | 9.6 | 70.5 |
| Prostate cancer(B88) | 4126.76 | 33.25 | 18.5 |
| Prostate NAT(B93) | 3378.81 | 37.92 | 16.5 |
| Prostate cancer(B8C) | 3527 | 42.55 | 10 |
| Prostate cancer(AD5) | 4105.44 | 45.35 | 0 |
| Prostate NAT(AD6) | 4196.5 | 41.71 | 23.5 |
| Prostate cancer(AD7) | 2830.59 | 42.73 | 0 |
| Prostate NAT(AD8) | 3404.14 | 29.72 | 25 |
| Prostate cancer(ADA) | 3700.09 | 34.54 | 1 |
| Prostate NAT(AD9) | 3022.26 | 30.92 | 10 |
| Prostate cancer(9E7) | 3084.26 | 30.48 | 0.5 |
| Prostate cancer(A0A) | 3983.11 | 24.56 | 30 |
| Prostate cancer(9E2) | 2889.43 | 23.94 | 23 |
| Pancreatic cancer(9E4) | 4473.72 | 23.53 | 165 |
| Pancreatic cancer(9D8) | 3443.44 | 20.25 | 24 |
| Pancreatic cancer(9D4) | 3819.27 | 17.3 | 8.5 |
| Pancreatic cancer(9BE) | 3287.48 | 24.17 | 43 |
| Pancreatic NAT(ADB) | 2358 | 28.92 | 0 |
| Pancreatic NAT(ADC) | 2863.88 | 36.96 | 23.5 |
| Pancreatic NAT(ADD) | 3118.81 | 30.22 | 1.5 |
| Pancreatic NAT(AED) | 3211.96 | 26.31 | 42 |
| Colon cancer(8A3) | 1984.83 | 48.06 | 13 |
| Colon NAT(8B6) | 1682.5 | 39.46 | 9 |
| Colon NAT(9F1) | 2378.93 | 48.8 | 2 |
| Colon cancer(9F2) | 1931.28 | 46.1 | 10.5 |
| Colon NAT(A1D) | 2029.41 | 46.05 | 4 |
| Colon cancer(9DB) | 2278.96 | 44.08 | 3.5 |
| Colon NAT(A15) | 1674.01 | 45.41 | 0 |
| Colon cancer(A14) | 1360.97 | 35.04 | 4 |
| Colon NAT(ACB) | 1707.6 | 45.03 | 1 |
| Colon cancer(AC0) | 1894.33 | 46.06 | 0 |
| Colon cancer(8A4) | 1785.56 | 43.34 | 5 |
| Colon NAT(ACD) | 1797.75 | 44.1 | 8 |
| Colon cancer(AC4) | 2198.75 | 49.26 | 4 |
| Colon NAT(AC2) | 1847.84 | 43.83 | 1 |
| Colon cancer(AC1) | 1806.35 | 39.49 | 9 |
| Colon NAT(ACC) | 2013.34 | 39.08 | 0 |
| Colon cancer(AC3) | 1539.46 | 47.71 | 2 |
| Breast cancer(9B7) | 1857.03 | 46.65 | 15 |
| Breast NAT(9CF) | 1462.79 | 47.35 | 0 |
| Breast cancer(9B6) | 2133.12 | 47.71 | 9 |
| Breast cancer(9C7) | 2302.99 | 47.48 | 18 |
| Colon cancer(8A6) | 2093.72 | 45.39 | 6 |
| Breast NAT(A11) | 1508.35 | 45.43 | 0 |
| Breast cancer(A1A) | 2246.51 | 46.55 | 1 |
| Breast cancer(9F3) | 1881.09 | 46.54 | 6 |
| Breast cancer(9B8) | 2174.46 | 48.66 | 2 |
| Breast NAT(9C4) | 1670.58 | 48.93 | 4 |
| Breast cancer(9EF) | 1168.07 | 23.61 | 4 |
| Breast cancer(9F0) | 1506.95 | 28.54 | 0 |
| Breast cancer(9B4) | 1016.05 | 32.36 | 0 |
| Breast cancer(9EC) | 2526.83 | 47.27 | 8 |
| Colon cancer(8A7) | 1594.35 | 48.21 | 0 |
| Colon cancer(8B7) | 2091.33 | 40.64 | 4 |
| Colon cancer(8A9) | 2533.34 | 40.66 | 24 |
| Colon cancer(8AB) | 1638.43 | 30.62 | 4 |
| Colon cancer(8AC) | 1975.26 | 41.39 | 9 |
| Colon NAT(8AD) | 1851.09 | 49.21 | 1 |
| Colon cancer(8B5) | 1920.15 | 47.11 | 5.5 |
| Cervical cancer(B08) | 1393.31 | 2.02 | 186 |
| Brain cancer(9F8) | 1400.44 | 8.86 | NA |
| Brain cancer(9C0) | 655.35 | 4.73 | 0 |
| Brain cancer(9F7) | 1403.07 | 0.42 | 0 |
| Brain cancer(A00) | 1509.09 | 3.25 | 0 |
| Brain NAT(A01) | 1159.94 | 0.43 | 6 |
| Brain cancer(9DA) | 1019.67 | 0.72 | 0 |
| Brain cancer(9FE) | 1352.85 | 2.77 | 0 |
| Brain cancer(9C6) | 1237.61 | 3.47 | 0 |
| Brain cancer(9F6) | 917.48 | 2.17 | 0 |
| Cervical NAT(AEB) | 826.9 | 1.64 | NA |
| Bladder NAT(23954) | 521.75 | 0.44 | 0 |
| Urinary cancer(AF6) | 1007.77 | 1.4 | 0 |
| Urinary cancer(B0C) | 1256.43 | 1.31 | 0 |
| Urinary cancer(AE4) | 1219.17 7 | 1.23 | 0 |
| Urinary NAT(B20) | 1222.48 | 1.23 | 0 |
| Urinary cancer(AE6) | 1114.91 | 1.03 | 0 |
| Urinary NAT(B04) | 655.35 | 0.07 | 0 |
| Urinary cancer(B07) | 543.73 | 1.64 | 1 |
| Urinary NAT(AF8) | 1247.4 | 0.53 | 0 |
| Cervical cancer(AFF) | 1411.18 | 4.31 | NA |
| Ovarian cancer(9D7) | 1221.47 | 0.63 | 13 |
| Urinary cancer(AF7) | 1138.73 | 1.3 | 0 |
| Ovarian cancer(9F5) | 1298.98 | 0 | NA |
| Ovarian cancer(A05) | 1134.77 | 2.16 | 3 |
| Ovarian cancer(9BC) | 505 | 0.15 | NA |
| Ovarian cancer(9C2) | 1025.23 | 0.93 | 0 |
| Ovarian cancer(9D9) | 1203.34 | 1.53 | NA |
| Ovarian NAT(AC7) | 685.35 | 0.54 | NA |
| Ovarian NAT(AC9) | 716.79 | 0.85 | NA |
| Ovarian NAT(ACA) | 628.62 | 2.38 | NA |
| Cervical NAT(B1E) | 1293.21 | 7.01 | NA |
| Ovarian NAT(AC5) | 542.12 | 0.99 | 0 |
| Cervical cancer(B00) | 1512.53 | 9.92 | 185 |
| Cervical NAT(AFA) | 1136.08 | 8.76 | NA |
| Cervical cancer(B1F) | 1782.82 | 18.96 | NA |
| Cervical NAT(B1C) | 655.35 | 2.36 | NA |
| Brain cancer(9F9) | 1508.5 | 5.08 | NA |
| Breast cancer(D34) | 2470.88 | 0 | 48.5 |
| Breast cancer(D35) | 2602.08 | 0 | 127 |
| Breast cancer(D36) | 2909.53 | 0 | NA |
| Breast cancer(D37) | 2811.77 | 0.05 | 75 |
| Breast cancer(D38) | 2986.78 | 0.38 | 96 |
| Breast cancer(D39) | 302622 | 0.04 | 78 |
| Breast cancer(D3A) | 3072.62 | 0.08 | 115.5 |
| Breast cancer(D3B) | 2571.28 | 0.02 | 31 |
| Breast cancer(D3C) | 3213.98 | 0.6 | 99.5 |
| Breast cancer(D3D) | 3484.57 | 2.5 | 73 |
| Breast cancer(D3E) | 2958.51 | 0.17 | 106 |
| Breast cancer(D3F) | 2937.01 | 1.88 | 30 |
| Breast cancer(D40) | 2751.61 | 1.2 | 31.5 |
| Breast cancer(D42) | 2171.59 | 0.8 | 19 |
| Breast cancer(D43) | 2962.09 | 4.5 | 44 |
| Breast cancer(D44) | 2558.08 | 2.95 | 24 |
| Breast cancer(D45) | 2667.3 | 3.59 | 39 |
| Breast cancer(D46) | 3190.77 | 2.25 | 53.5 |
| SK-MES | 2804.32 | 0.56 | 130.5 |
| HLaC-79 | 3402.37 | 0 | 103.5 |
| H226 | 2562.59 | 0 | 8 |
| HCT-116 | 4221.68 | 0.09 | 10 |
| IGROV-1 | 3243.07 | 0 | 32 |
| MX-1 | 3253.75 | 0 | 0 |
| C33A | 3249.59 | 0 | 11 |
| Daudi | 2333.08 | 0!01 | 65 |
| MV522 | 2727.71 | 0.94 | 0 |
| RWP-2 | 2906.49 | 0 | 0 |
| BON | 2502.53 | 0.01 | 9 |
| MiaPaCa | 3604.78 | 0 | 39 |
| H82 | 2357.18 | 2.19 | 15 |
| H69 | 2759.55 | 0.12 | 46.5 |
| Caki-2 | 2687.93 | 0 | 1 |
| LNCaP | 3352.46 | 0.41 | 64.5 |
| A549 | 2593.12 | 0 | 9 |
| DU145 | 3970.51 | 0.07 | 44 |
| OVCAR-3 | 3230.65 | 0.14 | 51 |
| HT-29 | 3381.64 | 0.07 | 41.5 |
| DLD-2 | 3610.05 | 0.24 | 4 |
| MCF-7 | 3326.73 | 1.78 | 48 |
| H460 | 2464.22 | 0 | 102 |
| SW620 | 2732.11 | 0 | 12 |
| SK-OV-3 | 3519.75 | 0 | NA |
| MDA-231 | 3464.04 | 0.04 | 72 |
| Caki-1 | 3801.64 | 0 | 87 |
| PC-3 | 2214.23 | 0 | 77.5 |
| LoVo | 3237.95 | 0 | 49 |
| Kidney NAT(10B1) | 3041.27 | 6.44 | 187 |
| Kidney cancer(10B2) | 3798.53 | 1.31 | 34 |
| Kidney NAT(10B3) | 3315.43 | 0 | 1197 |
| Kidney cancer(10B4) | 3519.14 | 0.16 | 73 |
| Kidney NAT(10B5) | 3017.75 | 0 | 177 |
| Kidney cancer(10B6) | 3702.61 | 0 | 13403.5 |
| Kidney NAT(10B7) | 3060.89 | 0 | 330 |
| Kidney cancer(10BA) | 3437.01 | 0 | 12600.5 |
| Kidney NAT(10BB) | 3157.99 | 0 | 308 |
| Kidney cancer(10C0) | 3590.87 | 0.77 | 40 |
| Kidney NAT(10C1) | 3012.3 | 0 | 157 |
| Kidney cancer(10C4) | 3186.48 | 0.02 | 270.5 |
| Kidney NAT(10C5) | 3618.74 | 0 | 64 |
| Kidney cancer(10A8) | 3514.51 | 0 | 14 |
| Kidney NAT(10A9) | 2771.76 | 1.14 | 241.5 |
| Kidney cancer(10AA) | 3793.55 | 0.4 | 86 |
| Kidney NAT(10AB) | 2978.06 | 0 | 222.5 |
| Kidney cancer(10AC) | 3656.35 | 0.05 | 26 |
| Kidney NAT(10AD) | 3299.97 | 2.13 | 436 |
| Kidney cancer(10AE) | 3456.21 | 0.57 | 49.5 |
| Kidney NAT(10AF) | 2593.7 | 0 | 100 |
| Kidney cancer(10B0) | 3529.2 | 0.67 | 35 |
| Lymphoma(9BF) | 2333.81 | 0 | 0 |
| Lymphoma(9D2) | 1327.77 | 0 | 0 |
| Lymphoma(A04) | 1450.41 | 0 | 0 |
| Lymphoma(9DD) | 1095.68 | 0 | 0 |
| Lymphoma(F68) | 865.62 | 0 | 0 |
| Lymphoma(F6A) | 862.12 | 0 | 0 |
| Lymphoma(F6B) | 624.66 | 0 | 0 |
| Lymphoma(F6C) | 1621.52 | 0 | 0 |
| Lymphoma(F6D) | 864.76 | 0 | 0 |
| Lymphoma(F6E) | 1030.71 | 0 | 0 |
| Lymphoma(F6F) | 1120.14 | 0 | 54 |
| Lymphoma(F70) | 891.53 | 0 | 0 |
| Lymphoma(F71) | 915.92 | 0 | 0 |
| Lymphoma(F72) | 1199.28 | 3.13 | 15 |
| Lymphoma(F73) | 1357.46 | 0 | 2 |
| Lymphoma(F74) | 993.02 | 0 | 0 |
| Lymphoma NAT(1002) | 2069.54 | 0 | 0 |
| Lymphoma NAT(1004) | 1928.51 | 0 | 0 |
| Lymphoma NAT(1005) | 1412.32 | 0 | 0 |
| Lymphoma NAT(1007) | 1668.28 | 0 | 0 |
| Lymphoma NAT(1003) | 1919.49 | 0 | 0 |
| Lymphoma(9E3) | 1791.94 | 0 | 0 |
| Lymphoma(9D0) | 1530.59 | 0 | 0 |
| Lymphoma(9E1) | 1677.97 | 0 | 0 |
| Lymphoma(A0D) | 2656.34 | 1.4 | 61 |
| Lymphoma(9B5) | 2336.21 | 0 | 8 |
| Lymphoma(9D3) | 1902.13 | 0 | 0 |
| | | | |
| Normal Lung 4 | 4083.27 | 28.46 | 45.8 |
| Normal Lung 5 | 4235.38 | 25.22 | 49.35 |
| Normal Lung 1 | 2759.91 | 24.22 | 25.51 |
| Normal Lung 2 | 3656.2 | 20.99 | 41.52 |
| Normal Lung 3 | 3157.31 | 22.69 | 23.69 |
| SW1353 resting 1h | 3946.45 | 0.6 | 0 |
| SW1353 resting 6h | 3263.46 | 0.12 | 0 |
| SW 1353 resting 16h | 2311.8 | 0 | 25.69 |
| SW 1353 IL-1b (1 ng/) 1h | 2686.83 | 0.16 | 0 |
| SW 1353 IL-1b (1 ng/) 6h | 3159.94 | 2 | 8.35 |
| SW1353 IL-1b (1 ng/) 16h | 3557.88 | 0.62 | 0.93 |
| SW1353 FGF20 (1 ug/) 1h | 3512.56 | 0.69 | 0.63 |
| SW1353 FGF20 (1 ug/) 16h | 2510.14 | 0.06 | 0 |
| SW1353 FGF20 (5 ug/) 1h | 3448.11 | 1.01 | 4.15 |
| SW 1353 FGF20 (5 ug/) 6h | 3598.07 | 0.64 | 6.73 |
| SW1353 FGF20 (5 ug/) 16h | 3687.34 | 4.42 | 12.83 |
| SW1353 FGF20 (1 ug/) IL-1b(1ng/)6h | 3569.19 | 0.17 | 4.31 |
| SW1353 FGF20 (1 ug/) IL-1b (1 ng/) 16h | 3970.28 | 0.01 | 0 |
| SW1353 FGF20 (5 ug/) IL-1b (1 ng/) 1h | 3011.45 | 0 | 0 |
| SW1353 FGF20 (5 ug/) -1b (1 ng/) 6h | IL3184.88 | 2.69 | 0 |
| THP-1 aCD40 (1 ug/) 1h | 3545.99 | 0.07 | 42.81 |
| THP-1 aCD40 (1 ug/) 6h | 2882.56 | 0.44 | 28.24 |
| THP-1 LPS (100 ng/) 1h | 3131.64 | 0.74 | 77.98 |
| THP-1 LPS (100 ng/) 6h | 2356.5 | 0.05 | 75.15 |
| CCD1070SK TNFa (5 ng/) 6h | 2888.01 | 0.78 | 0 |
| CCD1070SK TNFa (5 ng/) 24h | 3029 | 0.39 | 25.42 |
| CCD1070SK IL-1b (1 ng/) 24h | 3307.91 | 5.76 | 0 |
| THP-1 resting | 3080.68 | 0.87 | 41.42 |
| THP-1 aCD40 (1 ug/) 24h | 2032.49 | 0.82 | 2.16 |
| THP-1 LPS (100 ng/) 24h | 1597.29 | 3.58 | 0 |
| CCD1070SK IL-1b (1 ng/) 6h | 3026.56 | 3.51 | 1.09 |
| LC 18hr | 2725.87 | 0.36 | 0 |
| LC-IL-! 18hr | 1474.79 | 2.66 | 0 |
| Astrocyte IL1B 1hr a | 3116.77 | 10.04 | 25.41 |
| Astrocyte IL1B 6 hr a | 3119.7 | 16.51 | 28.21 |
| Astrocyte IL 1B 24 hr a | 3142.98 | 13.81 | 26.6 |
| SHSY 5Y Undifferentiated | 3166.23 | 14.61 | 47.94 |
| SHSY 5Y Differentiated | 2959.01 | 8.5 | 33.46 |
| LC 0hr | 1880.7 | 0 | 1.17 |
| Normal Fetal Kidney | 2011.8 | 0 | 10.94 |
| Normal Liver | 2053.67 | 3.81 | 0 |
| Normal Fetal Liver | 3555.17 | 0 | 157.18 |
| Normal Fetal Lung | 4164.55 | 0 | 75.01 |
| Normal Salivary Gland | 3466.36 | 0 | 59.02 |
| Normal Fetal Skeletal Muscle | 2504.59 | 0 | 32.5 |
| Normal Thyroid | 3566.17 | 0 | 160.4 |
| Normal Trachea | 3596.15 | 0 | 100.33 |
| LC-IL-1 0 hr | 2560.23 | 0 | 2.58 |
| Heart pool | 3167.78 | 0 | 22.92 |
| Pituitary Pool | 2908.48 | 0 | 363.83 |
| Spleen Pool | 2068.62 | 0 | 36.16 |
| Stomach Pool | 2826.7 | 0 | 5169.42 |
| Testis Pool | 3348.7 | 0 | 36.46 |
| Thymus Pool | 2653.82 | 0 | 75.44 |
| Small Intestine- 5 donor pool | 3795.64 | 0 | 41.44 |
| Lymph node- 5 donor pool | 4339.52 | 0 | 108.49 |
| Kidney- 5 donor pool | 3347.34 | 0 | 12.82 |
| Jurkat Resting | 3779.74 | 0.48 | 32.89 |
| Jurkat CD3 (500ng/ml) 6hr A | 2459.46 | 1.68 | 89.9 |
| Jurkat CD3 (500ng/ml) 24hr A | 1897.6 | 0.08 | 85.79 |
| Jurkat CD3 (500ng/ml)+CD28(1ug/ml) 6hr A | 1867.35 | 0.68 | 65.39 |
| Jurkat CD3 (500ng/ml)+CD28(1ug/ml) 24hr A | 1574.07 | 0.35 | 103.43 |
| control (no treatment)_1 hr | 5400.28 | 2.01 | 46.65 |
| 10ng/ml IL-1b 1 hr | 5250.91 | 1.51 | 63.47 |
| 10ng/ml TNF-a 1 hr | 5668.06 | 2.55 | 57.06 |
| 200uM BzATP 1 hr | 5619.87 | 0.31 | 47.95 |
| control (no treatment)_5 hr | 5630.13 | 1.02 | 73.66 |
| 10ng/ml IL-1b_5 hr | 6332.12 | 10.52 | 55.94 |
| 10ng/ml TNF-a_5 hr | 6070.17 | 6.37 | 52.01 |
| 200uM BzATP 5 hr | 6425.22 | 2.39 | 155.96 |
| control (no treatment)_24 hr | 4825.87 | 3.5 | 56.3 |
| 10ng/ml IL-1b_24 hr | 5349.28 | 9.73 | 55.52 |
| 10ng/ml TNF-a 24 hr | 5672.31 | 4.97 | 85.13 |
| 200uM BzATP 24 hr | 4814.1 | 0.51 | 38.39 |
| | | | |
| Alzheimer's disease B4951 | 1854.86 | 14.76 | 13.05 |
| Alzheimer's disease B4953 | 2540.02 | 19.95 | 20.79 |
| Alzheimer's disease B5018 | 1757.68 | 22.42 | 23.78 |
| Alzheimer's disease B5019 | 1491.9 | 18.37 | 10.32 |
| Alzheimer's disease B5086 | 2247.49 | 18.04 | 11.26 |
| Alzheimer's disease B5096 | 2150.92 | 19.76 | 23.52 |
| Alzheimer's disease B5098 | 732.56 | 15.93 | 0 |
| Alzheimer's disease B5129 | 1841.99 | 18.04 | 4.78 |
| Alzheimer's disease B5210 | 3233.87 | 21.56 | 24.49 |
| Control B4810 | 2987.22 | 21.85 | 18.41 |
| Control B4825 | 2903.91 | 18.74 | 18.18 |
| Control B4930 | 2287.12 | 22.69 | 22.12 |
| Control B4932 | 3424.98 | 20.12 | 17.99 |
| Control B5024 | 3859.32 | 22.42 | 27.93 |
| Control B5113 | 1897 | 17.87 | 11.6 |
| Control B5140 | 1901.93 | 18.88 | 11.57 |
| Control B5190 | 1284.69 | 15.26 | 0 |
| Control B5220 | 2225.75 | 18.7 | 20.26 |
| Control B5245 | 2119.39 | 21.5 | 15.57 |
| AH3 B3791 | 2202.74 | 19.73 | 18.98 |
| AH3 B3855 | 1849.89 | 17.93 | 17.84 |
| AH3 B3877 | 2144.15 | 17.01 | 14.36 |
| AH3 B3893 | 2103.76 | 16.34 | 38.69 |
| AH3 B3894 | 1820.82 | 17.31 | 24.16 |
| AH3 B3949 | 1607.86 | 23.9 | 19.16 |
| AH3 B4477 | 1602.58 | 20.32 | 19.91 |
| AH3 B4540 | 2260.92 | 22.79 | 24.33 |
| AH3 B4577 | 2142.42 | 22.59 | 15.4 |
| AH3 B4639 | 1550.43 | 21.89 | 22.7 |
| Schizophrenia hippocampus 683 | 2468.43 | 21.23 | 23.17 |
| Depression hippocampus 487 | 1473.83 | 18 | 0 |
| Depression hippocampus 600 | 2481.12 | 10.69 | 19.51 |
| Normal hippocampus 2407a | 2624.25 | 33.19 | 5.45 |
| Normal hippocampus 1042 | 2114.72 | 21.4 | 26.01 |
| Depression hippocampus 2767 | 1448.13 | 10.6 | 0 |
| Depression hippocampus 567 | 1836.77 | 47.98 | 24.55 |
| Control hippocampus 3175 | 2752.14 | 14.38 | 12.79 |
| Depression hippocampus 3096 | 1735.6 | 9.4 | 29.15 |
| Depression hippocampus 1491 | 2784.26 | 17.28 | 43.46 |
| Depression hippocampus 2540 | 2241.25 | 16.73 | 19.63 |
| Schizophrenia hippocampus 2798 | 1923.26 | 16.97 | 25.74 |
| Control hippocampus 1973 | 2605.59 | 14.97 | 17.73 |
| Normal hippocampus and amygdala 2601 | 2031.45 | 13.85 | 4.33 |
| Schizophrenia hippocampus 2785 | 1621.43 | 21.03 | 0 |
| Schizophrenia hippocampus 484 | 3271.31 | 39.54 | 13.79 |
| Normal hippocampus 2556 | 2806.68 | 21.69 | 43.9 |
| Depression hippocampus 1158 | 2705.56 | 15.83 | 27.65 |
| Control hippocampus 552 | 3378.83 | 21.65 | 42 |
| Schizophrenia hippocampus 1737 | 2304.06 | 12.06 | 26.74 |
| Normal hippocampus 1239 | 3335.3 | 17.54 | 32.32 |
| Normal hippocampus 1465 | 3068.38 | 14.64 | 20.08 |
| Normal hippocampus 3080 | 1323.77 | 4.6 | 0 |
| Normal hippocampus 738 | 4229.67 | 12.96 | 31.21 |
| Schizophrenia hippocampus 2586 | 2067.82 | 12.78 | 24.47 |
| Normal hippocampus 2551 | 3306.02 | 13.41 | 40.59 |
| Depression hippocampus 588 | 2352.1 | 13.53 | 48.17 |
| Depression hippocampus 529 | 3291.7 | 13.05 | 70.51 |
| Depression hippocampus and dentate gyrus | 1686.08 | 11.37 | 13.7 |
| Schizophrenia amygdala 2586 | 2136.57 | 0.55 | 12.87 |
| Normal substantia nigra 234 | 1240.83 | 0.03 | 24.82 |
| Normal substantia nigra 1065 | 1515.69 | 14.19 | 4.35 |
| Normal substantia nigra 3236 | 1341.77 | 0 | 4.92 |
| Normal substantia nigra 2551 | 3718.91 | 0.43 | 9.47 |
| Normal substantia nigra 1597 | 1003.1 | 0 | 16.45 |
| Control thalamus 552 | 948.15 | 0.02 | 27.84 |
| Control thalamus 566 | 1698.42 | 0 | 0 |
| Control thalamus 606 | 2625.59 | 0.31 | 0 |
| Control thalamus 738 | 2464.52 | 0 | 16.96 |
| Control thalamus 1065 | 2991.38 | 0 | 9.56 |
| Control thalamus 1092 | 2416.75 | 0 | 21.85 |
| Control thalamus 1597 | 2389.84 | 0 | 11.51 |
| Control thalamus 2253 | 1602.4 | 0 | 16.48 |
| Control thalamus 2551 | 3017.98 | 0 | 7.29 |
| Depression thalamus 588 | 2245.45 | 0.29 | 0 |
| Depression thalamus 600 | 1442.56 | 0 | 4.58 |
| Depression thalamus 721 | 1921.28 | 0 | 0 |
| Depression thalamus 728 | 3113.17 | 4.33 | 21.2 |
| Depression thalamus 759 | 2433.85 | 0 | 4.52 |
| Depression thalamus 881 | 2456.7 | 0 | 4.48 |
| Schizophrenia thalamus 477 | 1952.08 | 0 | 11.27 |
| Schizophrenia thalamus 532 | 3553.95 | 0 | 22.29 |
| Schizophrenia thalamus 683 | 3798.02 | 0 | 8.69 |
| Schizophrenia thalamus 544 | 3260.82 | 0.03 | 11.47 |
| Schizophrenia thalamus 1671 | 2246.95 | 0 | 5.39 |
| Schizophrenia thalamus 1737 | 1958.75 | 0.01 | 0 |
| Schizophrenia thalamus 2464 | 1953.64 | 0 | 0 |
| Schizophrenia thalamus 2586 | 3338.49 | 0 | 19.77 |
| Depression amygdala 600 | 1936.43 | 0 | NA |
| Depression amygdala 759 | 2378.2 | 0 | 74.93 |
| Depression anterior cingulate 759 | 2808.48 | 0 | 28.2 |
| Control amygdala 552 | 3675.87 | 0.36 | 7.78 |
| Control anterior cingulate 482 | 3115.46 | 0.67 | 28.6 |
| Depression anterior cingulate 721 | 1964.87 | 0 | 0 |
| Control amygdala 3175 | 2674.16 | 0.04 | 16.87 |
| Depression anterior cingulate 600 | 2389.83 | 0.06 | 0 |
| Depression anterior cingulate 588 | 2629.92 | 0 | 10.04 |
| Control anterior cingulate 3175 | 3605.03 | 0.79 | 21.36 |
| Control anterior cingulate 606 | 2414.64 | | 0 NA |
| Depression anterior cingulate 567 | 2397.28 | 1.2 | 9.64 |
| Depression amygdala 588 | 3410.76 | 3.1 | 33.86 |
| Control anterior cingulate 3080 | 2445.09 | 3.01 | 18.9 |
| Control anterior cingulate 2601 | 2520 | 5.45 | 22.26 |
| Control anterior cingulate 1042 | 3118.04 | 0.76 | 31.75 |
| Control anterior cingulate 3236 | 2913.66 | 1.45 | 34.73 |
| Control amygdala 1502 | 4253.4 | 9.62 | 40.86 |
| Control anterior cingulate 807 | 2624.08 | 8.86 | 13.41 |
| Control amygdala 1597 | 3710.89 | 11.88 | 45.35 |
| Parkinson's substantia nigra 2842 | 2457.51 | 0.25 | 8.06 |
| Parkinson's substantia nigra 2917 | 1548.58 | 0 | 136.38 |
| Schizophrenia amygdala 544 | 2009.89 | 0 | 122.05 |
| Schizophrenia amygdala 532 | 730.94 | 0 | 252.83 |
| Depression amygdala 2540 | 2408.1 | 0 | 136.12 |
| Parkinson's substantia nigra 2899 | 1544.99 | 0 | NA |
| Depression anterior cingulate 881 | 3015.65 | 0 | 75.14 |

### OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims. The claims presented are representative of the inventions disclosed herein. Other, unclaimed inventions are also contemplated. Applicants reserve the right to pursue such inventions in later claims.

## Claims

1. An isolated polypeptide comprising an amino acid sequence selected from the group consisting of:
(1) the mature form of an amino acid sequence of SEQ ID NO: 78, 80, 82, 84, 86, 88, 90, 92, 94, 14, 16, 18, 20, 22, 24, 2, 4, 6, 8, 10, 12, 26, 28, 30, 32, 34, 36, 38, 40, 42, 44, 46, 48, 50, 52, 54, 56, 58, 60, 62, 64, 66, 68, 70, 72, 74, 76, or 96;
(2) an amino acid sequence of SEQ ID NO: 78, 80, 82, 84, 86, 88, 90, 92, 94, 14, 16, 18, 20, 22, 24, 2, 4, 6, 8, 10, 12, 26, 28, 30, 32, 34, 36, 38, 40, 42, 44, 46, 48, 50, 52, 54, 56, 58, 60, 62, 64, 66, 68, 70, 72, 74, 76, or 96;
(3) an amino acid sequence which is at least 95% identical to SEQ ID NO: 78, 80, 82, 84, 86, 88, 90, 92, 94, 14, 16, 18, 20, 22, 24, 2, 4, 6, 8, 10, 12, 26, 28, 30, 32, 34, 36, 38, 40, 42, 44, 46, 48, 50, 52, 54, 56, 58, 60, 62, 64, 66, 68, 70, 72, 74, 76, or 96; and
(4) an amino acid sequence comprising one or more conservative substitutions in SEQ ID NO: 78, 80, 82, 84, 86, 88, 90, 92, 94, 14, 16, 18, 20, 22, 24, 2, 4, 6, 8, 10, 12, 26, 28, 30, 32, 34, 36, 38, 40, 42, 44, 46, 48, 50, 52, 54, 56, 58, 60, 62, 64, 66, 68, 70, 72, 74, 76, or 96.

2. A composition comprising the polypeptide of claim 1 and a carrier.

3. The use of a therapeutic in the manufacture of a medicament for treating a syndrome associated with a human disease, the disease selected from a pathology associated with the polypeptide of claim 1, wherein the therapeutic comprises the polypeptide of claim 1.

4. A method for determining the presence or amount of the polypeptide of claim 1 in a sample, the method comprising:
(a) providing said sample;
(b) introducing said sample to an antibody that binds immunospecifically to the polypeptide; and
(c) determining the presence or amount of antibody bound to said polypeptide,
thereby determining the presence or amount of polypeptide in said sample.

5. A method for determining the presence of or predisposition to a disease associated with altered levels of expression of the polypeptide of claim 1 in a first mammalian subject, the method comprising:
a) measuring the level of expression of the polypeptide in a sample from the first mammalian subject; and
b) comparing the expression of said polypeptide in the sample of step (a) to the expression of the polypeptide present in a control sample from a second mammalian subject known not to have, or not to be predisposed to, said disease,
wherein an alteration in the level of expression of the polypeptide in the first subject as compared to the control sample indicates the presence of or predisposition to said disease.

6. Use of the polypeptide of claim 1 in the manufacture of a medicament for use in a method of treating or preventing a pathology, the method comprising administering the polypeptide of claim 1 to a subject in which such treatment or prevention is desired in an amount sufficient to treat or prevent the pathology in the subject.

7. The use of claim 6, wherein the subject is a human.

8. An isolated nucleic acid molecule comprising a nucleic acid sequence selected from the group consisting of:
(1) a nucleic acid sequence encoding the polypeptide of claim 1;
(2) a nucleic acid sequence of SEQ ID NO: 77, 79, 81, 83, 85, 87, 89, 91, 93, 13, 15, 17, 19,21,22,1,3,5,7,9,11,25,27,29,31,33,35,37,39,41,43,45,47,49,51,53,55, 57, 59, 61, 63, 65, 67, 69, 71, 73, 75, or 95;
(3) a nucleic acid sequence differs by a single nucleotide from SEQ ID NO: 77, 79, 81, 83, 85, 87, 89, 91, 93, 13, 15, 17, 19, 21, 22, 1, 3, 5, 7, 9, 11, 25, 27, 29, 31, 33, 35, 37, 39, 41, 43, 45, 47, 49, 51, 53, 55, 57, 59, 61, 63, 65, 67, 69, 71, 73, 75, or 95; and
(4) a nucleic acid sequence hybridizes under stringent conditions to the nucleotide sequence selected from the group consisting of SEQ ID NOs: 77, 79, 81, 83, 85, 87, 89, 91, 93, 13, 15, 17, 19, 21, 22, 1, 3, 5, 7, 9, 11, 25, 27, 29, 31, 33, 35, 37, 39, 41, 43, 45, 47, 49, 51, 53, 55, 57, 59, 61, 63, 65, 67, 69, 71, 73, 75, 95, or a complement of said nucleotide sequence.

9. A vector comprising the nucleic acid molecule of claim 8.

10. The vector of claim 9, further comprising a promoter operably linked to said nucleic acid molecule.

11. A cell comprising the vector of claim 9.

12. An antibody that immunospecifically binds to the polypeptide of claim 1.

13. The antibody of claim 12, wherein the antibody is a monoclonal antibody, or wherein the antibody is a humanized antibody.

14. A method for determining the presence or amount of the nucleic acid molecule of claim 8 in a sample, the method comprising:
(a) providing said sample;
(b) introducing said sample to a probe that binds to said nucleic acid molecule; and
(c) determining the presence or amount of said probe bound to said nucleic acid molecule,
thereby determining the presence or amount of the nucleic acid molecule in said sample.

15. The method of claim 14 wherein presence or amount of the nucleic acid molecule is used as a marker for cell or tissue type, or wherein the cell or tissue type is cancerous.

16. A method of producing the polypeptide of claim 1, the method comprising culturing a cell under conditions that lead to expression of the polypeptide, wherein said cell comprises a vector comprising an isolated nucleic acid molecule comprising a nucleic acid sequence selected from the group consisting of SEQ ID NOs: 77, 79, 81, 83, 85, 87, 89, 91, 93, 13, 15, 17, 19, 21, 22, 1, 3, 5, 7, 9, 11, 25, 27, 29, 31, 33, 35, 37, 39, 41, 43, 45, 47, 49, 51, 53, 55, 57, 59, 61, 63, 65, 67, 69, 71, 73, 75, 95.

17. The method of claim 21 wherein the cell is a bacterial cell, or an insect cell, or a yeast cell, or a mammalian cell.

18. An isolated polypeptide comprising an amino acid sequence at least 95% similar to SEQ ID NO: 78, wherein said amino acid sequence comprises at least one amino acid substitution, wherein said substitution is at the amino acid position selected from the group consisting of 87, 119, 198, 222, or 261 when numbered in accordance with SEQ ID NO: 78.

19. An isolated nucleic acid molecule comprising an nucleic acid sequence at least 95% similar to SEQ ID NO: 77, wherein said nucleic acid sequence comprises at least one nucleic acid substitution, wherein said substitution is at the nucleic acid position selected from the group consisting of 259, 357, 593, 666, or 783 when numbered in accordance with SEQ ID NO: 77.


### Annex to the application documents - subsequently filed sequences listing

SEQUENCE LISTING

<110> CuraGen Corporation, et al.

<120> NOVEL PROTEINS AND NUCLEIC ACIDS ENCODING SAME

<130> 21402-573C

<140> EP06077108
<141> 2003-04-01

<150> 60/370349
<151> 2002-04-05

<150> 60/384543
<151> 2002-05-30

<150> 60/370969
<151> 2002-04-08

<150> 60/403748
<151> 2002-08-15

<150> 60/372019
<151> 2002-04-12

<150> 60/374379
<151> 2002-04-22

<150> 09/779679
<151> 2001-02-08

<150> 60/181045
<151> 2000-02-08

<150> 10/055877
<151> 2002-01-22

<150> 60/262892
<151> 2001-01-19

<150> 10/038854
<151> 2001-12-31

<150> 60/259785
<151> 2001-01-04

<150> 60/294080
<151> 2001-05-29

<150> 10/044564
<151> 2002-01-11

<150> 60/261026
<151> 2001-01-11

<150> 60/313170
<151> 2001-08-17

<150> 10/092900
<151> 2002-03-07

<150> 60/299027
<151> 2001-06-18

<150> 10/115479
<151> 2002-04-02

<150> 60/283657
<151> 2001-04-13

<150> 10/160619
<151> 2002-06-03

<150> 60/295607
<151> 2001-06-04

<150> 10/287226
<151> 2002-11-04

<150> 60/333461
<151> 2001-11-27

<150> 10/307719
<151> 2002-12-02

<150> 60/339286
<151> 2001-12-11

<150> 10/093463
<151> 2002-03-08

<150> 60/274191
<151> 2001-03-08

<150> 60/287424
<151> 2001-04-30

<150> 10/161493
<151> 2002-06-03

<150> 60/337524
<151> 2001-11-16

<160> 173

<170> CuraSeqList version 0.1

<210> 1
<211> 2343
<212> DNA
<213> Homo sapiens

<220>
<221> CDS
<222> (36)..(1328)

<400> 1
gagcgctaac gtctttctgt ctccccgcgg tggtg atg acg gtg aaa act gag      53
                                       Met Thr Val Lys Thr Glu
                                        1               5

gct gct aag ggc acc ctc act tac tcc agg atg agg ggc atg gtg gca    101
Ala Ala Lys Gly Thr Leu Thr Tyr Ser Arg Met Arg Gly Met Val Ala
            10                  15                  20

att ctc atc gct ttc atg aag cag agg agg atg ggt ctg aac gac ttt    149
Ile Leu Ile Ala Phe Met Lys Gln Arg Arg Met Gly Leu Asn Asp Phe
        25                  30                  35

att cag aag att gcc aat aac tcc tat gca tgc aaa cac cct gaa gtt    197
Ile Gln Lys Ile Ala Asn Asn Ser Tyr Ala Cys Lys His Pro Glu Val
    40                  45                  50

cag tcc atc ttg aag atc tcc caa cct cag gag cct gag ctt atg aat    245
Gln Ser Ile Leu Lys Ile Ser Gln Pro Gln Glu Pro Glu Leu Met Asn
55                  60                  65                  70

gcc aac cct tct cct cca cca agt cct tct cag caa atc aac ctt ggc    293
Ala Asn Pro Ser Pro Pro Pro Ser Pro Ser Gln Gln Ile Asn Leu Gly
                75                  80                  85

ccg tcg tcc aat cct cat gct aaa cca tct gac ttt cac ttc ttg aaa    341
Pro Ser Ser Asn Pro His Ala Lys Pro Ser Asp Phe His Phe Leu Lys
            90                  95                  100

gtg atc gga aag ggc agt ttt gga aag gtt ctt cta gca aga cac aag    389
Val Ile Gly Lys Gly Ser Phe Gly Lys Val Leu Leu Ala Arg His Lys
        105                 110                 115

gca gaa gaa gtg ttc tat gca gtc aaa gtt tta cag aag aaa gca atc    437
Ala Glu Glu Val Phe Tyr Ala Val Lys Val Leu Gln Lys Lys Ala Ile
    120                 125                 130

ctg aaa aag aaa gag gag aag cat att atg tcg gag cgg aat gtt ctg    485
Leu Lys Lys Lys Glu Glu Lys His Ile Met Ser Glu Arg Asn Val Leu
135                 140                 145                 150

ttg aag aat gtg aag cac cct ttc ctg gtg ggc ctt cac ttc tct ttc    533
Leu Lys Asn Val Lys His Pro Phe Leu Val Gly Leu His Phe Ser Phe
                155                 160                 165

cag act gct gac aaa ttg tac ttt gtc cta gac tac att aat ggt gga    581
Gln Thr Ala Asp Lys Leu Tyr Phe Val Leu Asp Tyr Ile Asn Gly Gly
            170                 175                 180

gag ttg ttc tac cat ctc cag agg gaa cgc tgc ttc ctg gaa cca cgg    629
Glu Leu Phe Tyr His Leu Gln Arg Glu Arg Cys Phe Leu Glu Pro Arg
        185                 190                 195

gct cgt ttc tat gct gct gaa ata gcc agt gcc ttg ggc tac ctg cat    677
Ala Arg Phe Tyr Ala Ala Glu Ile Ala Ser Ala Leu Gly Tyr Leu His
    200                 205                 210

tca ctg aac atc gtt tat aga gac tta aaa cca gag aat att ttg cta    725
Ser Leu Asn Ile Val Tyr Arg Asp Leu Lys Pro Glu Asn Ile Leu Leu
215                 220                 225                 230

gat tca cag gga cac att gtc ctt act gac ttc gga ctc tgc aag gag    773
Asp Ser Gln Gly His Ile Val Leu Thr Asp Phe Gly Leu Cys Lys Glu
                235                 240                 245

aac att gaa cac aac agc aca aca tcc acc ttc tgt ggc acg ccg gag    821

Asn Ile Glu His Asn Ser Thr Thr Ser Thr Phe Cys Gly Thr Pro Glu
            250                 255                 260

tat ctc gca cct gag gtg ctt cat aag cag cct tat gac agg act gtg 869
Tyr Leu Ala Pro Glu Val Leu His Lys Gln Pro Tyr Asp Arg Thr Val
        265                 270                 275

gac tgg tgg tgc ctg gga gct gtc ttg tat gag atg ctg tat ggc ctg 917
Asp Trp Trp Cys Leu Gly Ala Val Leu Tyr Glu Met Leu Tyr Gly Leu
    280                 285                 290

ccg cct ttt tat agc cga aac aca gct gaa atg tac gac aac att ctg 965
Pro Pro Phe Tyr Ser Arg Asn Thr Ala Glu Met Tyr Asp Asn Ile Leu
295                 300                 305                 310

aac aag cct ctc cag ctg aaa cca aat att aca aat tcc gca aga cac 1013
Asn Lys Pro Leu Gln Leu Lys Pro Asn Ile Thr Asn Ser Ala Arg His
                315                 320                 325

ctc ctg gag ggc ctc ctg cag aag gac agg aca aag cgg ctc ggg gcc 1061
Leu Leu Glu Gly Leu Leu Gln Lys Asp Arg Thr Lys Arg Leu Gly Ala
            330                 335                 340

aag gat gac ttc atg gag att aag agt cat gtc ttc ttc tcc tta att 1109
Lys Asp Asp Phe Met Glu Ile Lys Ser His Val Phe Phe Ser Leu Ile
        345                 350                 355

aac tgg gat gat ctc att aat aag aag att act ccc cct ttt aac cca 1157
Asn Trp Asp Asp Leu Ile Asn Lys Lys Ile Thr Pro Pro Phe Asn Pro
    360                 365                 370

aat gtg agt ggg ccc aac gac cta cgg cac ttt gac ccc gag ttt acc 1205
Asn Val Ser Gly Pro Asn Asp Leu Arg His Phe Asp Pro Glu Phe Thr
375                 380                 385                 390

gaa gag cct gtc ccc aac tcc att ggc aag tcc cct gac agc gtc ctc 1253
Glu Glu Pro Val Pro Asn Ser Ile Gly Lys Ser Pro Asp Ser Val Leu
                395                 400                 405

gtc aca gcc agc gtc aag gaa gct gcc gag gct ttc cta ggc ttt tcc 1301
Val Thr Ala Ser Val Lys Glu Ala Ala Glu Ala Phe Leu Gly Phe Ser
            410                 415                 420

tat gcg cct ccc acg gac tct ttc ctc tgaaccctgt tagggcttgg 1348
Tyr Ala Pro Pro Thr Asp Ser Phe Leu
        425                 430

ttttaaagga ttttatgtgt gtttccgaat gttttagtta gccttttggt ggagccgcca 1408

gctgacagga catcttacaa gagaatttgc acatctctgg aagcttagca atcttattgc 1468

acactgttcg ctggaagctt tttgaagagc acattctcct cagtgagctc atgaggtttt 1528

catttttatt cttccttcca acgtggtgct atctctgaaa cgagcgttag agtgccgcct 1588

tagacggagg caggagtttc gttagaaagc ggacgctgtt ctaaaaaagg tctcctgcag 1648

atctgtctgg gctgtgatga cgaatattat gaaatgtgcc ttttctgaag agattgtgtt 1708

agctccaaag cttttcctat cgcagtgttt cagttcttta ttttcccttg tggatatgct 1768

gtgtgaaccg tcgtgtgagt gtggtatgcc tgatcacaga tggattttgt tataagcatc 1828

aatgtgacac ttgcaggaca ctacaacgtg ggacattgtt tgtttcttcc atatttggaa 1888

gataaattta tgtgtagact tttttgtaag atacggttaa taactaaaat ttattgaaat 1948

ggtcttgcaa tgactcgtat tcagatgctt aaagaaagca ttgctgctac aaatatttct 2008

atttttagaa agggttttta tggaccaatg ccccagttgt cagtcagagc cgttggtgtt 2068

tttcattgtt taaaatgtca cctgtaaaat gggcattatt tatgtttttt tttttgcatt 2128

cctgataatt gtatgtattg tataaagaac gtctgtacat tgggttataa cactagtata 2188

tttaaactta caggcttatt tgtaatgtaa accaccattt taatgtactg taattaacat 2248

ggttataata cgtacaatcc ttccctcatc ccatcacaca actttttttg tgtgtgataa 2308

actgattttg gtttgcaata aaaccttgaa aaata 2343

<210> 2
<211> 431
<212> PRT
<213> Homo sapiens

<400> 2
Met Thr Val Lys Thr Glu Ala Ala Lys Gly Thr Leu Thr Tyr Ser Arg
1               5                   10                  15

Met Arg Gly Met Val Ala Ile Leu Ile Ala Phe Met Lys Gln Arg Arg
            20                  25                  30

Met Gly Leu Asn Asp Phe Ile Gln Lys Ile Ala Asn Asn Ser Tyr Ala
        35                  40                  45

Cys Lys His Pro Glu Val Gln Ser Ile Leu Lys Ile Ser Gln Pro Gln
    50                  55                  60

Glu Pro Glu Leu Met Asn Ala Asn Pro Ser Pro Pro Pro Ser Pro Ser
65                  70                  75                  80

Gln Gln Ile Asn Leu Gly Pro Ser Ser Asn Pro His Ala Lys Pro Ser
                85                  90                  95

Asp Phe His Phe Leu Lys Val Ile Gly Lys Gly Ser Phe Gly Lys Val
            100                 105                 110

Leu Leu Ala Arg His Lys Ala Glu Glu Val Phe Tyr Ala Val Lys Val
        115                 120                 125

Leu Gln Lys Lys Ala Ile Leu Lys Lys Lys Glu Glu Lys His Ile Met
    130                 135                 140

Ser Glu Arg Asn Val Leu Leu Lys Asn Val Lys His Pro Phe Leu Val
145                 150                 155                 160

Gly Leu His Phe Ser Phe Gln Thr Ala Asp Lys Leu Tyr Phe Val Leu
                165                 170                 175

Asp Tyr Ile Asn Gly Gly Glu Leu Phe Tyr His Leu Gln Arg Glu Arg
            180                 185                 190

Cys Phe Leu Glu Pro Arg Ala Arg Phe Tyr Ala Ala Glu Ile Ala Ser
        195                 200                 205

Ala Leu Gly Tyr Leu His Ser Leu Asn Ile Val Tyr Arg Asp Leu Lys
    210                 215                 220

Pro Glu Asn Ile Leu Leu Asp Ser Gln Gly His Ile Val Leu Thr Asp
225                 230                 235                 240

Phe Gly Leu Cys Lys Glu Asn Ile Glu His Asn Ser Thr Thr Ser Thr
                245                 250                 255

Phe Cys Gly Thr Pro Glu Tyr Leu Ala Pro Glu Val Leu His Lys Gln
            260                 265                 270

Pro Tyr Asp Arg Thr Val Asp Trp Trp Cys Leu Gly Ala Val Leu Tyr
        275                 280                 285

Glu Met Leu Tyr Gly Leu Pro Pro Phe Tyr Ser Arg Asn Thr Ala Glu
    290                 295                 300

Met Tyr Asp Asn Ile Leu Asn Lys Pro Leu Gln Leu Lys Pro Asn Ile
305                 310                 315                 320

Thr Asn Ser Ala Arg His Leu Leu Glu Gly Leu Leu Gln Lys Asp Arg
                325                 330                 335

Thr Lys Arg Leu Gly Ala Lys Asp Asp Phe Met Glu Ile Lys Ser His
            340                 345                 350

Val Phe Phe Ser Leu Ile Asn Trp Asp Asp Leu Ile Asn Lys Lys Ile
        355                 360                 365

Thr Pro Pro Phe Asn Pro Asn Val Ser Gly Pro Asn Asp Leu Arg His
    370                 375                 380

Phe Asp Pro Glu Phe Thr Glu Glu Pro Val Pro Asn Ser Ile Gly Lys
385                 390                 395                 400

Ser Pro Asp Ser Val Leu Val Thr Ala Ser Val Lys Glu Ala Ala Glu
                405                 410                 415

Ala Phe Leu Gly Phe Ser Tyr Ala Pro Pro Thr Asp Ser Phe Leu
            420                 425                 430

<210> 3
<211> 1315
<212> DNA
<213> Homo sapiens

<220>
<221> CDS
<222> (14)..(1306)

<400> 3
caccggatcc acc atg acg gtg aaa act gag gct gct aag ggc acc ctc      49
               Met Thr Val Lys Thr Glu Ala Ala Lys Gly Thr Leu
                1               5                   10

act tac tcc agg atg agg ggc atg gtg gca att ctc atc gct ttc atg      97

Thr Tyr Ser Arg Met Arg Gly Met Val Ala Ile Leu Ile Ala Phe Met
        15                  20                  25

aag cag agg agg atg ggt ctg aac gac ttt att cag aag att gcc aat     145
Lys Gln Arg Arg Met Gly Leu Asn Asp Phe Ile Gln Lys Ile Ala Asn
    30                  35                  40

aac tcc tat gca tgc aaa cac cct gaa gtt cag tcc atc ttg aag atc     193
Asn Ser Tyr Ala Cys Lys His Pro Glu Val Gln Ser Ile Leu Lys Ile
45                  50                  55                  60

tcc caa cct cag gag cct gag ctt atg aat gcc aac cct tct cct cca     241
Ser Gln Pro Gln Glu Pro Glu Leu Met Asn Ala Asn Pro Ser Pro Pro
                65                  70                  75

cca agt cct tct cag caa atc aac ctt ggc ccg tcg tcc aat cct cat     289
Pro Ser Pro Ser Gln Gln Ile Asn Leu Gly Pro Ser Ser Asn Pro His
            80                  85                  90

gct aaa cca tct gac ttt cac ttc ttg aaa gtg atc gga aag ggc agt     337
Ala Lys Pro Ser Asp Phe His Phe Leu Lys Val Ile Gly Lys Gly Ser
        95                  100                 105

ttt gga aag gtt ctt cta gca aga cac aag gca gaa gaa gtg ttc tat     385
Phe Gly Lys Val Leu Leu Ala Arg His Lys Ala Glu Glu Val Phe Tyr
    110                 115                 120

gca gtc aaa gtt tta cag aag aaa gca atc ctg aaa aag aaa gag gag     433
Ala Val Lys Val Leu Gln Lys Lys Ala Ile Leu Lys Lys Lys Glu Glu
125                 130                 135                 140

aag cat att atg tcg gag cgg aat gtt ctg ttg aag aat gtg aag cac     481
Lys His Ile Met Ser Glu Arg Asn Val Leu Leu Lys Asn Val Lys His
                145                 150                 155

cct ttc ctg gtg ggc ctt cac ttc tct ttc cag act gct gac aaa ttg     529
Pro Phe Leu Val Gly Leu His Phe Ser Phe Gln Thr Ala Asp Lys Leu
            160                 165                 170

tac ttt gtc cta gac tac att aat ggt gga gag ttg ttc tac cat ctc     577
Tyr Phe Val Leu Asp Tyr Ile Asn Gly Gly Glu Leu Phe Tyr His Leu
        175                 180                 185

cag agg gaa cgc tgc ttc ctg gaa cca cgg gct cgt ttc tat gct gct     625
Gln Arg Glu Arg Cys Phe Leu Glu Pro Arg Ala Arg Phe Tyr Ala Ala
    190                 195                 200

gaa ata gcc agt gcc ttg ggc tac ctg cat tca ctg aac atc gtt tat     673
Glu Ile Ala Ser Ala Leu Gly Tyr Leu His Ser Leu Asn Ile Val Tyr
205                 210                 215                 220

aga gac tta aaa cca gag aat att ttg cta gat tca cag gga cac att     721
Arg Asp Leu Lys Pro Glu Asn Ile Leu Leu Asp Ser Gln Gly His Ile
                225                 230                 235

gtc ctt act gac ttc gga ctc tgc aag gag aac att gaa cac aac agc     769
Val Leu Thr Asp Phe Gly Leu Cys Lys Glu Asn Ile Glu His Asn Ser
            240                 245                 250

aca aca tcc acc ttc tgt ggc acg ccg gag tat ctc gca cct gag gtg     817
Thr Thr Ser Thr Phe Cys Gly Thr Pro Glu Tyr Leu Ala Pro Glu Val

255                 260                 265

ctt cat aag cag cct tat gac agg act gtg gac tgg tgg tgc ctg gga     865
Leu His Lys Gln Pro Tyr Asp Arg Thr Val Asp Trp Trp Cys Leu Gly
    270                 275                 280

gct gtc ttg tat gag atg ctg tat ggc ctg ccg cct ttt tat agc cga     913
Ala Val Leu Tyr Glu Met Leu Tyr Gly Leu Pro Pro Phe Tyr Ser Arg
285                 290                 295                 300

aac aca gct gaa atg tac gac aac att ctg aac aag cct ctc cag ctg     961
Asn Thr Ala Glu Met Tyr Asp Asn Ile Leu Asn Lys Pro Leu Gln Leu
                305                 310                 315

aaa cca aat att aca aat tcc gca aga cac ctc ctg gag ggc ctc ctg     1009
Lys Pro Asn Ile Thr Asn Ser Ala Arg His Leu Leu Glu Gly Leu Leu
            320                 325                 330

cag aag gac agg aca aag cgg ctc ggg gcc aag gat gac ttc atg gag     1057
Gln Lys Asp Arg Thr Lys Arg Leu Gly Ala Lys Asp Asp Phe Met Glu
        335                 340                 345

att aag agt cat gtc ttc ttc tcc tta att aac tgg gat gat ctc att     1105
Ile Lys Ser His Val Phe Phe Ser Leu Ile Asn Trp Asp Asp Leu Ile
    350                 355                 360

aat aag aag att act ccc cct ttt aac cca aat gtg agt ggg ccc aac     1153
Asn Lys Lys Ile Thr Pro Pro Phe Asn Pro Asn Val Ser Gly Pro Asn
365                 370                 375                 380

gac cta cgg cac ttt gac ccc gag ttt acc gaa gag cct gtc ccc aac     1201
Asp Leu Arg His Phe Asp Pro Glu Phe Thr Glu Glu Pro Val Pro Asn
                385                 390                 395

tcc att ggc aag tcc cct gac agc gtc ctc gtc aca gcc agc gtc aag     1249
Ser Ile Gly Lys Ser Pro Asp Ser Val Leu Val Thr Ala Ser Val Lys
            400                 405                 410

gaa gct gcc gag gct ttc cta ggc ttt tcc tat gcg cct ccc acg gac     1297
Glu Ala Ala Glu Ala Phe Leu Gly Phe Ser Tyr Ala Pro Pro Thr Asp
        415                 420                 425

tct ttc ctc ctcgagggc                                               1315
Ser Phe Leu
    430

<210> 4
<211> 431
<212> PRT
<213> Homo sapiens

<400> 4
Met Thr Val Lys Thr Glu Ala Ala Lys Gly Thr Leu Thr Tyr Ser Arg
1               5                   10                  15

Met Arg Gly Met Val Ala Ile Leu Ile Ala Phe Met Lys Gln Arg Arg
            20                  25                  30

Met Gly Leu Asn Asp Phe Ile Gln Lys Ile Ala Asn Asn Ser Tyr Ala
        35                  40                  45

Cys Lys His Pro Glu Val Gln Ser Ile Leu Lys Ile Ser Gln Pro Gln
50                  55                  60

Glu Pro Glu Leu Met Asn Ala Asn Pro Ser Pro Pro Pro Ser Pro Ser
65                  70                  75                  80

Gln Gln Ile Asn Leu Gly Pro Ser Ser Asn Pro His Ala Lys Pro Ser
                85                  90                  95

Asp Phe His Phe Leu Lys Val Ile Gly Lys Gly Ser Phe Gly Lys Val
            100                 105                 110

Leu Leu Ala Arg His Lys Ala Glu Glu Val Phe Tyr Ala Val Lys Val
        115                 120                 125

Leu Gln Lys Lys Ala Ile Leu Lys Lys Lys Glu Glu Lys His Ile Met
    130                 135                 140

Ser Glu Arg Asn Val Leu Leu Lys Asn Val Lys His Pro Phe Leu Val
145                 150                 155                 160

Gly Leu His Phe Ser Phe Gln Thr Ala Asp Lys Leu Tyr Phe Val Leu
                165                 170                 175

Asp Tyr Ile Asn Gly Gly Glu Leu Phe Tyr His Leu Gln Arg Glu Arg
            180                 185                 190

Cys Phe Leu Glu Pro Arg Ala Arg Phe Tyr Ala Ala Glu Ile Ala Ser
        195                 200                 205

Ala Leu Gly Tyr Leu His Ser Leu Asn Ile Val Tyr Arg Asp Leu Lys
    210                 215                 220

Pro Glu Asn Ile Leu Leu Asp Ser Gln Gly His Ile Val Leu Thr Asp
225                 230                 235                 240

Phe Gly Leu Cys Lys Glu Asn Ile Glu His Asn Ser Thr Thr Ser Thr
                245                 250                 255

Phe Cys Gly Thr Pro Glu Tyr Leu Ala Pro Glu Val Leu His Lys Gln
            260                 265                 270

Pro Tyr Asp Arg Thr Val Asp Trp Trp Cys Leu Gly Ala Val Leu Tyr
        275                 280                 285

Glu Met Leu Tyr Gly Leu Pro Pro Phe Tyr Ser Arg Asn Thr Ala Glu
    290                 295                 300

Met Tyr Asp Asn Ile Leu Asn Lys Pro Leu Gln Leu Lys Pro Asn Ile
305                 310                 315                 320

Thr Asn Ser Ala Arg His Leu Leu Glu Gly Leu Leu Gln Lys Asp Arg
                325                 330                 335

Thr Lys Arg Leu Gly Ala Lys Asp Asp Phe Met Glu Ile Lys Ser His
            340                 345                 350

Val Phe Phe Ser Leu Ile Asn Trp Asp Asp Leu Ile Asn Lys Lys Ile
        355                 360                 365

Thr Pro Pro Phe Asn Pro Asn Val Ser Gly Pro Asn Asp Leu Arg His

370                 375                 380

Phe Asp Pro Glu Phe Thr Glu Glu Pro Val Pro Asn Ser Ile Gly Lys
385                 390                 395                 400

Ser Pro Asp Ser Val Leu Val Thr Ala Ser Val Lys Glu Ala Ala Glu
                405                 410                 415

Ala Phe Leu Gly Phe Ser Tyr Ala Pro Pro Thr Asp Ser Phe Leu
            420                 425                 430

<210> 5
<211> 1140
<212> DNA
<213> Homo sapiens

<220>
<221> CDS
<222> (55)..(1131)

<400> 5
attggagttt ggaagttcag gagcacagga gcacaggccc acgactgcag cggg atg      57
                                                            Met
                                                            1

gac cag tac tgc atc ctg ggc cgc atc ggg gag ggc gcc cac ggc atc      105
Asp Gln Tyr Cys Ile Leu Gly Arg Ile Gly Glu Gly Ala His Gly Ile
            5                   10                  15

gtc ttc aag gcc aag cac gtg gag ccg agg gtg ggc tgg cag tgt ctg      153
Val Phe Lys Ala Lys His Val Glu Pro Arg Val Gly Trp Gln Cys Leu
        20                  25                  30

cct tct atc ctg cag act ggc gag ata gtt gcc ctc aag aag gtg gcc      201
Pro Ser Ile Leu Gln Thr Gly Glu Ile Val Ala Leu Lys Lys Val Ala
    35                  40                  45

cta agg cgg ttg gag gac ggc ttc cct aac cag gcc ctg cgg gag att      249
Leu Arg Arg Leu Glu Asp Gly Phe Pro Asn Gln Ala Leu Arg Glu Ile
50                  55                  60                  65

aag gct ctg cag gag atg gag gac aat cag tat gtg gta caa ctg aag      297
Lys Ala Leu Gln Glu Met Glu Asp Asn Gln Tyr Val Val Gln Leu Lys
                70                  75                  80

gct gtg ttc cca cac ggt gga ggc ttt gtg ctg gcc ttt gag ttc atg      345
Ala Val Phe Pro His Gly Gly Gly Phe Val Leu Ala Phe Glu Phe Met
            85                  90                  95

ctg tcg gat ctg gcc gag gtg gtg cgc cat gcc cag agg cca cta gcc      393
Leu Ser Asp Leu Ala Glu Val Val Arg His Ala Gln Arg Pro Leu Ala
        100                 105                 110

cag gca cag gtc aag agc tac ctg cag atg ctg ctc aag ggt gtc gcc      441
Gln Ala Gln Val Lys Ser Tyr Leu Gln Met Leu Leu Lys Gly Val Ala
    115                 120                 125

ttc tgc cat gcc aac aac att gta cat cgg gac ctg aaa cct gcc aac      489
Phe Cys His Ala Asn Asn Ile Val His Arg Asp Leu Lys Pro Ala Asn
130                 135                 140                 145

ctg ctc atc agc gcc tca ggc cag ctc aag ata gcg gac ttt ggc ctg     537
Leu Leu Ile Ser Ala Ser Gly Gln Leu Lys Ile Ala Asp Phe Gly Leu
                150                 155                 160

gct cga gtc ttt tcc cca gac ggc agc cac ctc tac aca cac cag gtg     585
Ala Arg Val Phe Ser Pro Asp Gly Ser His Leu Tyr Thr His Gln Val
            165                 170                 175

gcc acc agg tgg tac cga gcc ccc gag ctc ctg tat ggt gcc cgc cag     633
Ala Thr Arg Trp Tyr Arg Ala Pro Glu Leu Leu Tyr Gly Ala Arg Gln
        180                 185                 190

tat gac cag ggc gtc gat ctg tgg tct gtg ggc tgc atc atg ggg gag     681
Tyr Asp Gln Gly Val Asp Leu Trp Ser Val Gly Cys Ile Met Gly Glu
    195                 200                 205

ctg ttg aat ggg tcc ccc ctt ttc ccg ggc aag aac gat att gaa cag     729
Leu Leu Asn Gly Ser Pro Leu Phe Pro Gly Lys Asn Asp Ile Glu Gln
210                 215                 220                 225

ctt tgc tat gtg ctt cgc atc ttg ggc acc cca aac cct caa gtc tgg     777
Leu Cys Tyr Val Leu Arg Ile Leu Gly Thr Pro Asn Pro Gln Val Trp
                230                 235                 240

ccg gag ctc act gag ctg ccg gac tac aac aag atc tcc ttt aag gag     825
Pro Glu Leu Thr Glu Leu Pro Asp Tyr Asn Lys Ile Ser Phe Lys Glu
            245                 250                 255

cag gtg ccc atg ccc ctg gag gag gtg ctg cct gac gtc tct ccc cag     873
Gln Val Pro Met Pro Leu Glu Glu Val Leu Pro Asp Val Ser Pro Gln
        260                 265                 270

gca ttg gat ctg ctg ggt caa ttc ctt ctc tac cct cct cac cag cgc     921
Ala Leu Asp Leu Leu Gly Gln Phe Leu Leu Tyr Pro Pro His Gln Arg
    275                 280                 285

atc gca gct tcc aag gct ctc ctc cat cag tac ttc ttc aca gct ccc     969
Ile Ala Ala Ser Lys Ala Leu Leu His Gln Tyr Phe Phe Thr Ala Pro
290                 295                 300                 305

ctg cct gcc cat cca tct gag ctg ccg att cct cag cgt cta ggg gga     1017
Leu Pro Ala His Pro Ser Glu Leu Pro Ile Pro Gln Arg Leu Gly Gly
                310                 315                 320

cct gcc ccc aag gcc cat cca ggg ccc ccc cac atc cat gac ttc cac     1065
Pro Ala Pro Lys Ala His Pro Gly Pro Pro His Ile His Asp Phe His
            325                 330                 335

gtg gac cgg cct ctt gag gag tcg ctg ttg aac cca gag ctg att cgg     1113
Val Asp Arg Pro Leu Glu Glu Ser Leu Leu Asn Pro Glu Leu Ile Arg
        340                 345                 350

ccc ttc atc ctg gag ggg tgagaagtt                                   1140
Pro Phe Ile Leu Glu Gly
    355

<210> 6
<211> 359
<212> PRT
<213> Homo sapiens

<400> 6
Met Asp Gln Tyr Cys Ile Leu Gly Arg Ile Gly Glu Gly Ala His Gly
1               5                   10                  15

Ile Val Phe Lys Ala Lys His Val Glu Pro Arg Val Gly Trp Gln Cys
            20                  25                  30

Leu Pro Ser Ile Leu Gln Thr Gly Glu Ile Val Ala Leu Lys Lys Val
        35                  40                  45

Ala Leu Arg Arg Leu Glu Asp Gly Phe Pro Asn Gln Ala Leu Arg Glu
    50                  55                  60

Ile Lys Ala Leu Gln Glu Met Glu Asp Asn Gln Tyr Val Val Gln Leu
65                  70                  75                  80

Lys Ala Val Phe Pro His Gly Gly Gly Phe Val Leu Ala Phe Glu Phe
                85                  90                  95

Met Leu Ser Asp Leu Ala Glu Val Val Arg His Ala Gln Arg Pro Leu
            100                 105                 110

Ala Gln Ala Gln Val Lys Ser Tyr Leu Gln Met Leu Leu Lys Gly Val
        115                 120                 125

Ala Phe Cys His Ala Asn Asn Ile Val His Arg Asp Leu Lys Pro Ala
    130                 135                 140

Asn Leu Leu Ile Ser Ala Ser Gly Gln Leu Lys Ile Ala Asp Phe Gly
145                 150                 155                 160

Leu Ala Arg Val Phe Ser Pro Asp Gly Ser His Leu Tyr Thr His Gln
                165                 170                 175

Val Ala Thr Arg Trp Tyr Arg Ala Pro Glu Leu Leu Tyr Gly Ala Arg
            180                 185                 190

Gln Tyr Asp Gln Gly Val Asp Leu Trp Ser Val Gly Cys Ile Met Gly
        195                 200                 205

Glu Leu Leu Asn Gly Ser Pro Leu Phe Pro Gly Lys Asn Asp Ile Glu
    210                 215                 220

Gln Leu Cys Tyr Val Leu Arg Ile Leu Gly Thr Pro Asn Pro Gln Val
225                 230                 235                 240

Trp Pro Glu Leu Thr Glu Leu Pro Asp Tyr Asn Lys Ile Ser Phe Lys
                245                 250                 255

Glu Gln Val Pro Met Pro Leu Glu Glu Val Leu Pro Asp Val Ser Pro
            260                 265                 270

Gln Ala Leu Asp Leu Leu Gly Gln Phe Leu Leu Tyr Pro Pro His Gln
        275                 280                 285

Arg Ile Ala Ala Ser Lys Ala Leu Leu His Gln Tyr Phe Phe Thr Ala
    290                 295                 300

Pro Leu Pro Ala His Pro Ser Glu Leu Pro Ile Pro Gln Arg Leu Gly
305                 310                 315                 320

Gly Pro Ala Pro Lys Ala His Pro Gly Pro Pro His Ile His Asp Phe
                325                 330                 335

His Val Asp Arg Pro Leu Glu Glu Ser Leu Leu Asn Pro Glu Leu Ile
            340                 345                 350

Arg Pro Phe Ile Leu Glu Gly
        355

<210> 7
<211> 2078
<212> DNA
<213> Homo sapiens

<220>
<221> CDS
<222> (238)..(1593)

<400> 7
caggtgctgc gtgacagcgg agggctagga aaaggcgcag tggggcccgg agctgtcacc   60
cctgactcga cgcagcttcc gttctcctgg tgacgtggcc tacaggaacc gccccggtgg  120
tcagctgccg cgctgttgct aggcaacagc gtgcgtgctc agatcagcgt ggggtggagg  180
agaagtggag tttggaagtt caggggcaca ggggcacagg cccacgactg cagcggg     237
atg gac cag tac tgc atc ctg ggc cgc atc ggg gag ggc gcc cac ggc    285
atc gtc ttc aag gcc aag cac gtg gag act ggc gag ata att gcc ctc    333
aag aag gtg gcc cta agg cgg ttg gaa gac ggc ttc cct aac cag gcc    381
ctg cgg gag att aag gct ctg cag gag atg gag gac aat cag tat gtg    429
gta caa ctg aag gct gtg ttc cca cac ggt gga ggc ttt gtg ctg gcc    477
ttt gag ttc atg ctg tcg gat ctg gcc gag gtg gtg cgc cat gcc cag    525
agg cca cta gcc cag gca cag gtc aag agc tac ctg cag atg ctg ctc    573
aag ggt gtc gcc ttc tgc cat gcc aac aac att gta cat cgg gac ctg    621
aaa cct gcc aac ctg ctc atc agc gcc tca ggc cag ctc aag ata gcg    669
gac ttt ggc ctg gct cga gtc ttt tcc cca gac ggc agc cgc ctc tac     717
aca cac cag gtg gcc acc agg tct gtg ggc tgc atc atg ggg gag ctg     765
ttg aat ggg tcc ccc ctt ttc ccg ggc aag aac gat att gaa cag ctt     813
tgc tat gtg ctt cgc atc ttg ggc acc cca aac cct caa gtc tgg ccg     861
gag ctc act gag ctg ccg gac tac aac aag atc tcc ttt aag gag cag     909
gtg ccc atg ccc ctg gag gag gtg ctg cct gac gtc tct ccc cag gca     957
ttg gat ctg ctg ggt caa ttc ctt ctc tac cct cct cac cag cgc atc     1005
gca gct tcc aag gct ctc ctc cat cag tac ttc ttc aca gct ccc ctg     1053
cct gcc cat cca tct gag ctg ccg att cct cag cgt cta ggg gga cct     1101
gcc ccc aag gcc cat cca ggg ccc ccc cac atc cat gac ttc cac gtg     1149
gac cgg cct ctt gag gga gtc gct gtt gaa ccc aga gct gat tcg gcc     1197
ctt cat cct gga ggg gtg aga agt tgg ccc tgg tcc cgt ctg cct gct     1245
cct cag gac cac tca gtc cac ctg ttc ctc tgc cac ctg cct ggc ttc     1293
acc ctc caa ggc ctc ccc atg gcc aca gtg ggc cca cac cac acc ttg     1341
ccc ctt agc cct tgc gag ggt tgg tct cga ggc aga ggt cat gtt ccc     1389
agc caa gag tat gag aac atc cag tcg agc aga gga gat tca tgg cct    1437
gtg ctc ggt gag cct tac ctt ctg tgt gct act gac gta ccc atc agg    1485
aca gtg agc tct gct gcc agt caa ggc ctg cat atg cag aat gac gat    1533
gcc tgc ctt ggt gct gct tcc ccc gag tgc tgc ctc ctg gtc aag gag    1581
aag tgc aga gag taaggtgtcc ttatgttgga aactcaagtg gaaggaagat        1633
ttggtttggt tttattctca gagccattaa acactagttc agtatgtgag atatagattc 1693
taaaaacctc aggtggtctg cttagtctgt cttcttcctt tctcaaggga aatggctaag 1753
gtggcattgt ctcatggctc tcgtttttgg ggtcatgggg agggtagcac cagcatagcc 1813
acttttgccc tgagggactc ctgtgtgctt cacatcactg agcactcatt tagaagtgag 1873
ggagacagaa gtctaggccc agggatggct ccagttgggg atccagcagg agaccctctg 1933
cacatgaggc tggtttacca acatctactc cctcaggatg agcgtgagcc agaagcagct 1993
gtgtatttaa ggaaacaagc gttcctggaa ttaatttata aatttaataa atcccaatat 2053
aatcccaaaa aaaaaaaaaa aaaaa                                        2078

<210> 8
<211> 452
<212> PRT
<213> Homo sapiens

<400> 8

<210> 9
<211> 958
<212> DNA
<213> Homo sapiens

<220>
<221> CDS
<222> (99)..(944)

<400> 9
agccagagac aggacaccag aagagacagg agatcagaga ccagaggaac agagaagagg   60

ccccagagca aggcaaggaa cggccaaggc accaggac atg gat gca gtg gag cca 116
                                          Met Asp Ala Val Glu Pro
                                          1               5

gga gga cgt ggc tgg gcc agc atg ttg gcg tgc agg ctt tgg aaa gcc   164
Gly Gly Arg Gly Trp Ala Ser Met Leu Ala Cys Arg Leu Trp Lys Ala
            10                  15                  20

atc agc agg gcg ctg ttt gca gag ttc ctg gcc acg ggg ctg tat gtg   212
Ile Ser Arg Ala Leu Phe Ala Glu Phe Leu Ala Thr Gly Leu Tyr Val
        25                  30                  35

ttc ttt ggc gtg ggc tca gtc atg cgc tgg ccc aca gca ctt ccc tcc   260
Phe Phe Gly Val Gly Ser Val Met Arg Trp Pro Thr Ala Leu Pro Ser
    40                  45                  50

gtg cta cag att gcc atc acc ttc aac ctg gtc acc gcc atg gct gtg   308
Val Leu Gln Ile Ala Ile Thr Phe Asn Leu Val Thr Ala Met Ala Val
55                  60                  65                  70

cag gtc acc tgg aag gcc agc ggg gcc cac gcc aac ccc gcc gtg acg   356
Gln Val Thr Trp Lys Ala Ser Gly Ala His Ala Asn Pro Ala Val Thr
                75                  80                  85

ctg gcc ttc ctc gta ggc tcc cac atc tct ctg ccc cgt gct gtg gcc   404
Leu Ala Phe Leu Val Gly Ser His Ile Ser Leu Pro Arg Ala Val Ala
            90                  95                  100

tat gtg gct gcc cag ctg gtg ggg gcc acg gtg ggg gct gct ctg ctt   452
Tyr Val Ala Ala Gln Leu Val Gly Ala Thr Val Gly Ala Ala Leu Leu
        105                 110                 115

tat ggg gtc atg ccg gga gac atc cga gag acc ctt ggg atc aac gtg   500
Tyr Gly Val Met Pro Gly Asp Ile Arg Glu Thr Leu Gly Ile Asn Val
    120                 125                 130

gtc cgg aac agt gtc tca act ggc cag gcg gtg gca gtg gag ctg ctt   548
Val Arg Asn Ser Val Ser Thr Gly Gln Ala Val Ala Val Glu Leu Leu
135                 140                 145                 150

ctg acc ctg cag ctg gtg ctc tgt gtc ttc gct tcc acc gac agc cgt   596

Leu Thr Leu Gln Leu Val Leu Cys Val Phe Ala Ser Thr Asp Ser Arg
                155                 160                 165

cag aca tca ggc tcc ccg gcc acc atg att ggg atc tct gtg gca ctg     644
Gln Thr Ser Gly Ser Pro Ala Thr Met Ile Gly Ile Ser Val Ala Leu
            170                 175                 180

ggc cac ctc att ggg atc cac ttc act ggc tgc tcc atg aat cca gcc     692
Gly His Leu Ile Gly Ile His Phe Thr Gly Cys Ser Met Asn Pro Ala
        185                 190                 195

cgc tcc ttc ggc cct gcc atc atc att ggg aag ttc aca gtc cac tgg     740
Arg Ser Phe Gly Pro Ala Ile Ile Ile Gly Lys Phe Thr Val His Trp
    200                 205                 210

gtc ttc tgg gtg ggg ccc ctg atg gga gcc ctc ctg gcc tca ctg atc     788
Val Phe Trp Val Gly Pro Leu Met Gly Ala Leu Leu Ala Ser Leu Ile
215                 220                 225                 230

tac aac ttc gtc ctg ttc ccc gac acc aag aac ctg gcg cag cgg ctg     836
Tyr Asn Phe Val Leu Phe Pro Asp Thr Lys Asn Leu Ala Gln Arg Leu
                235                 240                 245

gct atc ctc aca ggc acc gta gag gtg ggg aca ggg gca ggg gca ggg     884
Ala Ile Leu Thr Gly Thr Val Glu Val Gly Thr Gly Ala Gly Ala Gly
            250                 255                 260

gcg gag ccc ctg aag aag gaa tcc cag ccg ggt tcg gga gcc gtg gag     932
Ala Glu Pro Leu Lys Lys Glu Ser Gln Pro Gly Ser Gly Ala Val Glu
        265                 270                 275

atg gag agt gtg tgaaacagcc taac                                     958
Met Glu Ser Val
    280

<210> 10
<211> 282
<212> PRT
<213> Homo sapiens

<400> 10
Met Asp Ala Val Glu Pro Gly Gly Arg Gly Trp Ala Ser Met Leu Ala
1               5                   10                  15

Cys Arg Leu Trp Lys Ala Ile Ser Arg Ala Leu Phe Ala Glu Phe Leu
            20                  25                  30

Ala Thr Gly Leu Tyr Val Phe Phe Gly Val Gly Ser Val Met Arg Trp
        35                  40                  45

Pro Thr Ala Leu Pro Ser Val Leu Gln Ile Ala Ile Thr Phe Asn Leu
    50                  55                  60

Val Thr Ala Met Ala Val Gln Val Thr Trp Lys Ala Ser Gly Ala His
65                  70                  75                  80

Ala Asn Pro Ala Val Thr Leu Ala Phe Leu Val Gly Ser His Ile Ser
                85                  90                  95

Leu Pro Arg Ala Val Ala Tyr Val Ala Ala Gln Leu Val Gly Ala Thr
            100                 105                 110

Val Gly Ala Ala Leu Leu Tyr Gly Val Met Pro Gly Asp Ile Arg Glu
        115                 120                 125

Thr Leu Gly Ile Asn Val Val Arg Asn Ser Val Ser Thr Gly Gln Ala
    130                 135                 140

Val Ala Val Glu Leu Leu Leu Thr Leu Gln Leu Val Leu Cys Val Phe
145                 150                 155                 160

Ala Ser Thr Asp Ser Arg Gln Thr Ser Gly Ser Pro Ala Thr Met Ile
                165                 170                 175

Gly Ile Ser Val Ala Leu Gly His Leu Ile Gly Ile His Phe Thr Gly
            180                 185                 190

Cys Ser Met Asn Pro Ala Arg Ser Phe Gly Pro Ala Ile Ile Ile Gly
        195                 200                 205

Lys Phe Thr Val His Trp Val Phe Trp Val Gly Pro Leu Met Gly Ala
    210                 215                 220

Leu Leu Ala Ser Leu Ile Tyr Asn Phe Val Leu Phe Pro Asp Thr Lys
225                 230                 235                 240

Asn Leu Ala Gln Arg Leu Ala Ile Leu Thr Gly Thr Val Glu Val Gly
                245                 250                 255

Thr Gly Ala Gly Ala Gly Ala Glu Pro Leu Lys Lys Glu Ser Gln Pro
            260                 265                 270

Gly Ser Gly Ala Val Glu Met Glu Ser Val
        275                 280

<210> 11
<211> 1067
<212> DNA
<213> Homo sapiens

<220>
<221> CDS
<222> (3)..(1046)

<400> 11
ct ttg gaa cag aac cag tca aca gat tat tat tat gag gaa aat gaa      47
   Leu Glu Gln Asn Gln Ser Thr Asp Tyr Tyr Tyr Glu Glu Asn Glu
   1               5                   10                  15

atg aat ggc act tat gac tac agt caa tat gaa ctg atc tgt atc aaa      95
Met Asn Gly Thr Tyr Asp Tyr Ser Gln Tyr Glu Leu Ile Cys Ile Lys
                20                  25                  30

gaa gat gtc aga gaa ttt gca aaa gtt ttc ctc cct gta ttc ctc aca     143
Glu Asp Val Arg Glu Phe Ala Lys Val Phe Leu Pro Val Phe Leu Thr
            35                  40                  45

ata gtt ttc gtc att gga ctt gca ggc aat tcc atg gta gtg gca att     191
Ile Val Phe Val Ile Gly Leu Ala Gly Asn Ser Met Val Val Ala Ile
        50                  55                  60

tat gcc tat tac aag aaa cag aga acc aaa aca gat gtg tac atc ctg     239

Tyr Ala Tyr Tyr Lys Lys Gln Arg Thr Lys Thr Asp Val Tyr Ile Leu
    65                  70                  75

aat ttg gct gta gca gat tta ctc ctt cta ttc act ctg cct ttt tgg 287
Asn Leu Ala Val Ala Asp Leu Leu Leu Leu Phe Thr Leu Pro Phe Trp
80                  85                  90                  95

gct gtt aat gca gtt cat ggg tgg gtt tta ggg aaa ata atg tgc aaa 335
Ala Val Asn Ala Val His Gly Trp Val Leu Gly Lys Ile Met Cys Lys
                100                 105                 110

ata act tca gcc ttg tac aca cta aac ttt gtc tct gga atg cag ttt 383
Ile Thr Ser Ala Leu Tyr Thr Leu Asn Phe Val Ser Gly Met Gln Phe
            115                 120                 125

ctg gct tgt atc agc ata gac aga tat gtg gca gta act aaa gtc ccc 431
Leu Ala Cys Ile Ser Ile Asp Arg Tyr Val Ala Val Thr Lys Val Pro
        130                 135                 140

agc caa tca gga gtg gga aaa cca tgc tgg atc atc tgt ttc tgt gtc 479
Ser Gln Ser Gly Val Gly Lys Pro Cys Trp Ile Ile Cys Phe Cys Val
    145                 150                 155

tgg atg gct gcc atc ttg ctg agc ata ccc cag ctg gtt ttt tat aca 527
Trp Met Ala Ala Ile Leu Leu Ser Ile Pro Gln Leu Val Phe Tyr Thr
160                 165                 170                 175

gta aat gac aat gct agg tgc att ccc att ttc ccc cgc tac cta gga 575
Val Asn Asp Asn Ala Arg Cys Ile Pro Ile Phe Pro Arg Tyr Leu Gly
                180                 185                 190

aca tca atg aaa gca ttg att caa atg cta gag atc tgc att gga ttt 623
Thr Ser Met Lys Ala Leu Ile Gln Met Leu Glu Ile Cys Ile Gly Phe
            195                 200                 205

gta gta ccc ttt ctt att atg ggg gtg tgc tac ttt atc acg gca agg 671
Val Val Pro Phe Leu Ile Met Gly Val Cys Tyr Phe Ile Thr Ala Arg
        210                 215                 220

aca ctc atg aag atg cca aac att aaa ata tct cga ccc cta aaa gtt 719
Thr Leu Met Lys Met Pro Asn Ile Lys Ile Ser Arg Pro Leu Lys Val
    225                 230                 235

ctg ctc aca gtc gtt ata gtt ttc att gtc act caa ctg cct tat aac 767
Leu Leu Thr Val Val Ile Val Phe Ile Val Thr Gln Leu Pro Tyr Asn
240                 245                 250                 255

att gtc aag ttc tgc cga gcc ata gac atc atc tac tct ctg atc acc 815
Ile Val Lys Phe Cys Arg Ala Ile Asp Ile Ile Tyr Ser Leu Ile Thr
                260                 265                 270

agc tgc aac atg agc aaa cgc atg gac atc gcc atc caa gtc aca gaa 863
Ser Cys Asn Met Ser Lys Arg Met Asp Ile Ala Ile Gln Val Thr Glu
            275                 280                 285

agc atc gca ctc ttt cac agc tgc ctc aac cca atc ctt tat gtt ttt 911
Ser Ile Ala Leu Phe His Ser Cys Leu Asn Pro Ile Leu Tyr Val Phe
        290                 295                 300

atg gga gca tct ttc aaa aac tac gtt atg aaa gtg gcc aag aaa tat 959
Met Gly Ala Ser Phe Lys Asn Tyr Val Met Lys Val Ala Lys Lys Tyr

305                 310                 315

ggg tcc tgg aga aga cag aga caa agt gtg gag gag ttt cct ttt gat    1007
Gly Ser Trp Arg Arg Gln Arg Gln Ser Val Glu Glu Phe Pro Phe Asp
320                 325                 330                 335

tct gag ggt cct aca gag cca acc agt act ttt agc att taaaggtaaa    1056
Ser Glu Gly Pro Thr Glu Pro Thr Ser Thr Phe Ser Ile
                340                 345

actgctctgc c                                                      1067

<210> 12
<211> 348
<212> PRT
<213> Homo sapiens

<400> 12
Leu Glu Gln Asn Gln Ser Thr Asp Tyr Tyr Tyr Glu Glu Asn Glu Met
1               5                   10                  15

Asn Gly Thr Tyr Asp Tyr Ser Gln Tyr Glu Leu Ile Cys Ile Lys Glu
            20                  25                  30

Asp Val Arg Glu Phe Ala Lys Val Phe Leu Pro Val Phe Leu Thr Ile
        35                  40                  45

Val Phe Val Ile Gly Leu Ala Gly Asn Ser Met Val Val Ala Ile Tyr
    50                  55                  60

Ala Tyr Tyr Lys Lys Gln Arg Thr Lys Thr Asp Val Tyr Ile Leu Asn
65                  70                  75                  80

Leu Ala Val Ala Asp Leu Leu Leu Leu Phe Thr Leu Pro Phe Trp Ala
                85                  90                  95

Val Asn Ala Val His Gly Trp Val Leu Gly Lys Ile Met Cys Lys Ile
            100                 105                 110

Thr Ser Ala Leu Tyr Thr Leu Asn Phe Val Ser Gly Met Gln Phe Leu
        115                 120                 125

Ala Cys Ile Ser Ile Asp Arg Tyr Val Ala Val Thr Lys Val Pro Ser
    130                 135                 140

Gln Ser Gly Val Gly Lys Pro Cys Trp Ile Ile Cys Phe Cys Val Trp
145                 150                 155                 160

Met Ala Ala Ile Leu Leu Ser Ile Pro Gln Leu Val Phe Tyr Thr Val
                165                 170                 175

Asn Asp Asn Ala Arg Cys Ile Pro Ile Phe Pro Arg Tyr Leu Gly Thr
            180                 185                 190

Ser Met Lys Ala Leu Ile Gln Met Leu Glu Ile Cys Ile Gly Phe Val
        195                 200                 205

Val Pro Phe Leu Ile Met Gly Val Cys Tyr Phe Ile Thr Ala Arg Thr
    210                 215                 220

Leu Met Lys Met Pro Asn Ile Lys Ile Ser Arg Pro Leu Lys Val Leu

225                 230                 235                 240

Leu Thr Val Val Ile Val Phe Ile Val Thr Gln Leu Pro Tyr Asn Ile
                245                 250                 255

Val Lys Phe Cys Arg Ala Ile Asp Ile Ile Tyr Ser Leu Ile Thr Ser
            260                 265                 270

Cys Asn Met Ser Lys Arg Met Asp Ile Ala Ile Gln Val Thr Glu Ser
        275                 280                 285

Ile Ala Leu Phe His Ser Cys Leu Asn Pro Ile Leu Tyr Val Phe Met
    290                 295                 300

Gly Ala Ser Phe Lys Asn Tyr Val Met Lys Val Ala Lys Lys Tyr Gly
305                 310                 315                 320

Ser Trp Arg Arg Gln Arg Gln Ser Val Glu Glu Phe Pro Phe Asp Ser
                325                 330                 335

Glu Gly Pro Thr Glu Pro Thr Ser Thr Phe Ser Ile
            340                 345

<210> 13
<211> 1953
<212> DNA
<213> Homo sapiens

<220>
<221> CDS
<222> (16)..(1929)

<400> 13
gtctgagtca cagag atg ggc aag atc gag aac aac gag agg gtg atc ctc      51
                 Met Gly Lys Ile Glu Asn Asn Glu Arg Val Ile Leu
                 1               5                   10

aat gtc ggg ggc acc cgg cac gaa acc tac cgc agc acc ctc aag acc      99
Asn Val Gly Gly Thr Arg His Glu Thr Tyr Arg Ser Thr Leu Lys Thr
        15                  20                  25

ctg cct gga aca cgc ctg gcc ctt ctt gcc tcc tcc gag ccc cca ggc     147
Leu Pro Gly Thr Arg Leu Ala Leu Leu Ala Ser Ser Glu Pro Pro Gly
    30                  35                  40

gac tgc ttg acc acg gcg ggc gac aag ctg cag ccg tcg ccg cct cca     195
Asp Cys Leu Thr Thr Ala Gly Asp Lys Leu Gln Pro Ser Pro Pro Pro
45                  50                  55                  60

ctg tcg ccg ccg ccg aga gcg ccc ccg ctg tcc ccc ggg cca ggc ggc     243
Leu Ser Pro Pro Pro Arg Ala Pro Pro Leu Ser Pro Gly Pro Gly Gly
                65                  70                  75

tgc ttc gag ggc ggc gcg ggc aac tgc agt tcc cgc ggc ggc agg gcc     291
Cys Phe Glu Gly Gly Ala Gly Asn Cys Ser Ser Arg Gly Gly Arg Ala
            80                  85                  90

agc gac cat ccc ggt ggc ggc cgc gag ttc ttc ttc gac cgg cac ccg     339
Ser Asp His Pro Gly Gly Gly Arg Glu Phe Phe Phe Asp Arg His Pro
        95                  100                 105

ggc gtc ttc gcc tat gtg ctc aat tac tac cgc acc ggc aag ctg cac 387
Gly Val Phe Ala Tyr Val Leu Asn Tyr Tyr Arg Thr Gly Lys Leu His
    110                 115                 120

tgc ccc gca gac gtg tgc ggg ccg ctc ttc gag gag gag ctg gcc ttc 435
Cys Pro Ala Asp Val Cys Gly Pro Leu Phe Glu Glu Glu Leu Ala Phe
125                 130                 135                 140

tgg ggc atc gac gag acc gac gtg gag ccc tgc tgc tgg atg acc tac 483
Trp Gly Ile Asp Glu Thr Asp Val Glu Pro Cys Cys Trp Met Thr Tyr
                145                 150                 155

cgg cag cac cgc gac gcc gag gag gcg ctg gac atc ttc gag acc ccc 531
Arg Gln His Arg Asp Ala Glu Glu Ala Leu Asp Ile Phe Glu Thr Pro
            160                 165                 170

gac ctc att ggc ggc gac ccc ggc gac gac gag gac ctg gcg gcc aag 579
Asp Leu Ile Gly Gly Asp Pro Gly Asp Asp Glu Asp Leu Ala Ala Lys
        175                 180                 185

agg ctg ggc atc gag gac gcg gcg ggg ctc ggg ggc ccg gac ggc aaa 627
Arg Leu Gly Ile Glu Asp Ala Ala Gly Leu Gly Gly Pro Asp Gly Lys
    190                 195                 200

tct ggc cgc tgg agg agg ctg cag ccc cgc atg tgg gcc ctc ttc gaa 675
Ser Gly Arg Trp Arg Arg Leu Gln Pro Arg Met Trp Ala Leu Phe Glu
205                 210                 215                 220

gac ccc tac tcg tcc aga gcc gcc agg ttt att gct ttt gct tct tta 723
Asp Pro Tyr Ser Ser Arg Ala Ala Arg Phe Ile Ala Phe Ala Ser Leu
                225                 230                 235

ttc ttc atc ctg gtt tca att aca act ttt tgc ctg gaa aca cat gaa 771
Phe Phe Ile Leu Val Ser Ile Thr Thr Phe Cys Leu Glu Thr His Glu
            240                 245                 250

gct ttc aat att gtt aaa aac aag aca gaa cca gtc atc aat ggc aca 819
Ala Phe Asn Ile Val Lys Asn Lys Thr Glu Pro Val Ile Asn Gly Thr
        255                 260                 265

agt gtt gtt cta cag tat gaa att gaa acg gat cct gcc ttg acg tat 867
Ser Val Val Leu Gln Tyr Glu Ile Glu Thr Asp Pro Ala Leu Thr Tyr
    270                 275                 280

gta gaa gga gtg tgt gtg gtg tgg ttt act ttt gaa ttt tta gtc cgt 915
Val Glu Gly Val Cys Val Val Trp Phe Thr Phe Glu Phe Leu Val Arg
285                 290                 295                 300

att gtt ttt tca ccc aat aaa ctt gaa ttc atc aaa aat ctc ttg aat 963
Ile Val Phe Ser Pro Asn Lys Leu Glu Phe Ile Lys Asn Leu Leu Asn
                305                 310                 315

atc att gac ttt gtg gcc atc cta cct ttc tac tta gag gtg gga ctc 1011
Ile Ile Asp Phe Val Ala Ile Leu Pro Phe Tyr Leu Glu Val Gly Leu
            320                 325                 330

agt ggg ctg tca tcc aaa gct gct aaa gat gtg ctt ggc ttc ctc agg 1059
Ser Gly Leu Ser Ser Lys Ala Ala Lys Asp Val Leu Gly Phe Leu Arg
        335                 340                 345

gtg gta agg ttt gtg agg atc ctg aga att ttc aag ctc acc cgc cat 1107

Val Val Arg Phe Val Arg Ile Leu Arg Ile Phe Lys Leu Thr Arg His
    350                 355                 360

ttt gta ggt ctg agg gtg ctt gga cat act ctt cga gct agt act aat    1155
Phe Val Gly Leu Arg Val Leu Gly His Thr Leu Arg Ala Ser Thr Asn
365                 370                 375                 380

gaa ttt ttg ctg ctg ata att ttc ctg gct cta gga gtt ttg ata ttt    1203
Glu Phe Leu Leu Leu Ile Ile Phe Leu Ala Leu Gly Val Leu Ile Phe
                385                 390                 395

gct acc atg atc tac tat gcc gag aga gtg gga gct caa cct aac gac    1251
Ala Thr Met Ile Tyr Tyr Ala Glu Arg Val Gly Ala Gln Pro Asn Asp
            400                 405                 410

cct tca gct agt gag cac aca cag ttc aaa aac att ccc att ggg ttc    1299
Pro Ser Ala Ser Glu His Thr Gln Phe Lys Asn Ile Pro Ile Gly Phe
        415                 420                 425

tgg tgg gct gta gtg acc atg act acc ctg ggt tat ggg gat atg tac    1347
Trp Trp Ala Val Val Thr Met Thr Thr Leu Gly Tyr Gly Asp Met Tyr
    430                 435                 440

ccc caa aca tgg tca ggc atg ctg gtg gga gcc ctg tgt gct ctg gct    1395
Pro Gln Thr Trp Ser Gly Met Leu Val Gly Ala Leu Cys Ala Leu Ala
445                 450                 455                 460

gga gtg ctg aca ata gcc atg cca gtg cct gtc att gtc aat aat ttt    1443
Gly Val Leu Thr Ile Ala Met Pro Val Pro Val Ile Val Asn Asn Phe
                465                 470                 475

gga atg tac tac tcc ttg gca atg gca aag cag aaa ctt cca agg aaa    1491
Gly Met Tyr Tyr Ser Leu Ala Met Ala Lys Gln Lys Leu Pro Arg Lys
            480                 485                 490

aga aag aag cac atc cct cct gct cct cag gca agc tca cct act ttt    1539
Arg Lys Lys His Ile Pro Pro Ala Pro Gln Ala Ser Ser Pro Thr Phe
        495                 500                 505

tgc aag aca gaa tta aat atg gcc tgc aat agt aca cag agt gac aca    1587
Cys Lys Thr Glu Leu Asn Met Ala Cys Asn Ser Thr Gln Ser Asp Thr
    510                 515                 520

tgt ctg ggc aaa gac aat cga ctt ctg gaa cat aac aga tca gtg tta    1635
Cys Leu Gly Lys Asp Asn Arg Leu Leu Glu His Asn Arg Ser Val Leu
525                 530                 535                 540

tca ggt gac gac agt aca gga agt gag ccg cca cta tca ccc cca gaa    1683
Ser Gly Asp Asp Ser Thr Gly Ser Glu Pro Pro Leu Ser Pro Pro Glu
                545                 550                 555

agg ctc ccc atc aga cgc tct agt acc aga gac aaa aac aga aga ggg    1731
Arg Leu Pro Ile Arg Arg Ser Ser Thr Arg Asp Lys Asn Arg Arg Gly
            560                 565                 570

gaa aca tgt ttc cta ctg acg aca ggt gat tac acg tgt gct tct gat    1779
Glu Thr Cys Phe Leu Leu Thr Thr Gly Asp Tyr Thr Cys Ala Ser Asp
        575                 580                 585

gga ggg atc agg aaa ggt tat gaa aaa tcc cga agc tta aac aac ata    1827
Gly Gly Ile Arg Lys Gly Tyr Glu Lys Ser Arg Ser Leu Asn Asn Ile

590                 595                 600

gcg ggc ttg gca ggc aat gct ctg agg ctc tct cca gta aca tca ccc     1875
Ala Gly Leu Ala Gly Asn Ala Leu Arg Leu Ser Pro Val Thr Ser Pro
605                 610                 615                 620

tac aac tct cct tgt cct ctg agg cgc tct cga tct ccc atc cca tct     1923
Tyr Asn Ser Pro Cys Pro Leu Arg Arg Ser Arg Ser Pro Ile Pro Ser
                625                 630                 635

atc ttg taaaccaaac aaccaaactg catc                                  1953
Ile Leu

<210> 14
<211> 638
<212> PRT
<213> Homo sapiens

<400> 14
Met Gly Lys Ile Glu Asn Asn Glu Arg Val Ile Leu Asn Val Gly Gly
1               5                   10                  15

Thr Arg His Glu Thr Tyr Arg Ser Thr Leu Lys Thr Leu Pro Gly Thr
            20                  25                  30

Arg Leu Ala Leu Leu Ala Ser Ser Glu Pro Pro Gly Asp Cys Leu Thr
        35                  40                  45

Thr Ala Gly Asp Lys Leu Gln Pro Ser Pro Pro Pro Leu Ser Pro Pro
    50                  55                  60

Pro Arg Ala Pro Pro Leu Ser Pro Gly Pro Gly Gly Cys Phe Glu Gly
65                  70                  75                  80

Gly Ala Gly Asn Cys Ser Ser Arg Gly Gly Arg Ala Ser Asp His Pro
                85                  90                  95

Gly Gly Gly Arg Glu Phe Phe Phe Asp Arg His Pro Gly Val Phe Ala
            100                 105                 110

Tyr Val Leu Asn Tyr Tyr Arg Thr Gly Lys Leu His Cys Pro Ala Asp
        115                 120                 125

Val Cys Gly Pro Leu Phe Glu Glu Glu Leu Ala Phe Trp Gly Ile Asp
    130                 135                 140

Glu Thr Asp Val Glu Pro Cys Cys Trp Met Thr Tyr Arg Gln His Arg
145                 150                 155                 160

Asp Ala Glu Glu Ala Leu Asp Ile Phe Glu Thr Pro Asp Leu Ile Gly
                165                 170                 175

Gly Asp Pro Gly Asp Asp Glu Asp Leu Ala Ala Lys Arg Leu Gly Ile
            180                 185                 190

Glu Asp Ala Ala Gly Leu Gly Gly Pro Asp Gly Lys Ser Gly Arg Trp
        195                 200                 205

Arg Arg Leu Gln Pro Arg Met Trp Ala Leu Phe Glu Asp Pro Tyr Ser
    210                 215                 220

Ser Arg Ala Ala Arg Phe Ile Ala Phe Ala Ser Leu Phe Phe Ile Leu
225                 230                 235                 240

Val Ser Ile Thr Thr Phe Cys Leu Glu Thr His Glu Ala Phe Asn Ile
                245                 250                 255

Val Lys Asn Lys Thr Glu Pro Val Ile Asn Gly Thr Ser Val Val Leu
            260                 265                 270

Gln Tyr Glu Ile Glu Thr Asp Pro Ala Leu Thr Tyr Val Glu Gly Val
        275                 280                 285

Cys Val Val Trp Phe Thr Phe Glu Phe Leu Val Arg Ile Val Phe Ser
    290                 295                 300

Pro Asn Lys Leu Glu Phe Ile Lys Asn Leu Leu Asn Ile Ile Asp Phe
305                 310                 315                 320

Val Ala Ile Leu Pro Phe Tyr Leu Glu Val Gly Leu Ser Gly Leu Ser
                325                 330                 335

Ser Lys Ala Ala Lys Asp Val Leu Gly Phe Leu Arg Val Val Arg Phe
            340                 345                 350

Val Arg Ile Leu Arg Ile Phe Lys Leu Thr Arg His Phe Val Gly Leu
        355                 360                 365

Arg Val Leu Gly His Thr Leu Arg Ala Ser Thr Asn Glu Phe Leu Leu
    370                 375                 380

Leu Ile Ile Phe Leu Ala Leu Gly Val Leu Ile Phe Ala Thr Met Ile
385                 390                 395                 400

Tyr Tyr Ala Glu Arg Val Gly Ala Gln Pro Asn Asp Pro Ser Ala Ser
                405                 410                 415

Glu His Thr Gln Phe Lys Asn Ile Pro Ile Gly Phe Trp Trp Ala Val
            420                 425                 430

Val Thr Met Thr Thr Leu Gly Tyr Gly Asp Met Tyr Pro Gln Thr Trp
        435                 440                 445

Ser Gly Met Leu Val Gly Ala Leu Cys Ala Leu Ala Gly Val Leu Thr
    450                 455                 460

Ile Ala Met Pro Val Pro Val Ile Val Asn Asn Phe Gly Met Tyr Tyr
465                 470                 475                 480

Ser Leu Ala Met Ala Lys Gln Lys Leu Pro Arg Lys Arg Lys Lys His
                485                 490                 495

Ile Pro Pro Ala Pro Gln Ala Ser Ser Pro Thr Phe Cys Lys Thr Glu
            500                 505                 510

Leu Asn Met Ala Cys Asn Ser Thr Gln Ser Asp Thr Cys Leu Gly Lys
        515                 520                 525

Asp Asn Arg Leu Leu Glu His Asn Arg Ser Val Leu Ser Gly Asp Asp
    530                 535                 540

Ser Thr Gly Ser Glu Pro Pro Leu Ser Pro Pro Glu Arg Leu Pro Ile
545                 550                 555                 560

Arg Arg Ser Ser Thr Arg Asp Lys Asn Arg Arg Gly Glu Thr Cys Phe
                565                 570                 575

Leu Leu Thr Thr Gly Asp Tyr Thr Cys Ala Ser Asp Gly Gly Ile Arg
            580                 585                 590

Lys Gly Tyr Glu Lys Ser Arg Ser Leu Asn Asn Ile Ala Gly Leu Ala
        595                 600                 605

Gly Asn Ala Leu Arg Leu Ser Pro Val Thr Ser Pro Tyr Asn Ser Pro
    610                 615                 620

Cys Pro Leu Arg Arg Ser Arg Ser Pro Ile Pro Ser Ile Leu
625                 630                 635

<210> 15
<211> 607
<212> DNA
<213> Homo sapiens

<220>
<221> CDS
<222> (13)..(603)

<400> 15
agatttccca cc atg ggc aag atc gag aac aac gag agg gtg atc ctc aat   51
              Met Gly Lys Ile Glu Asn Asn Glu Arg Val Ile Leu Asn
              1               5                   10

gtc ggg ggc acc cgg cac gaa acc tac cgc agc acc ctc aag acc ctg     99
Val Gly Gly Thr Arg His Glu Thr Tyr Arg Ser Thr Leu Lys Thr Leu
    15                  20                  25

cct gga aca cgc ctg gcc ctt ctt gcc tcc tcc gag ccc cca ggc gac    147
Pro Gly Thr Arg Leu Ala Leu Leu Ala Ser Ser Glu Pro Pro Gly Asp
30                  35                  40                  45

tgc ttg acc aca gcg ggc aac tgc agt tcc cgc ggc ggc agg gcc agc    195
Cys Leu Thr Thr Ala Gly Asn Cys Ser Ser Arg Gly Gly Arg Ala Ser
                50                  55                  60

gac cat ccc ggt ggc ggc cgc gag ttc ttc ttc gac cgg cat ccg ggc    243
Asp His Pro Gly Gly Gly Arg Glu Phe Phe Phe Asp Arg His Pro Gly
            65                  70                  75

gtc ttc gcc tat gtg ctc aat tac tac cgc acc ggc aag ctg cac tgt    291
Val Phe Ala Tyr Val Leu Asn Tyr Tyr Arg Thr Gly Lys Leu His Cys
        80                  85                  90

ccc gca gac gtg tgc ggg ccg ctc ttc gag gag gag ctg gcc ttc tgg    339
Pro Ala Asp Val Cys Gly Pro Leu Phe Glu Glu Glu Leu Ala Phe Trp
    95                  100                 105

ggc atc gac gag acc gac gtg gag ccc tgc tgc tgg atg acc tac cgg    387
Gly Ile Asp Glu Thr Asp Val Glu Pro Cys Cys Trp Met Thr Tyr Arg
110                 115                 120                 125

cag cac cgc gac gcc gag gag gcg ctg gac atc ttc gag acc ccc gac    435

Gln His Arg Asp Ala Glu Glu Ala Leu Asp Ile Phe Glu Thr Pro Asp
                130                 135                 140

ctc att ggc ggc gac ccc ggc gac gac gag gac ctg gcg gcc aag agg     483
Leu Ile Gly Gly Asp Pro Gly Asp Asp Glu Asp Leu Ala Ala Lys Arg
            145                 150                 155

ctg ggc atc gag gac gcg gcg ggg ctc ggg ggc ccc gac ggc aaa tct     531
Leu Gly Ile Glu Asp Ala Ala Gly Leu Gly Gly Pro Asp Gly Lys Ser
        160                 165                 170

ggc cgc tgg agg agg ctg cag ccc cgc atg tgg gcc ctc ttc gaa gac     579
Gly Arg Trp Arg Arg Leu Gln Pro Arg Met Trp Ala Leu Phe Glu Asp
    175                 180                 185

ccc tac tcg tcc aga gcc gcc agg ctcg                                607
Pro Tyr Ser Ser Arg Ala Ala Arg
190                 195

<210> 16
<211> 197
<212> PRT
<213> Homo sapiens

<400> 16
Met Gly Lys Ile Glu Asn Asn Glu Arg Val Ile Leu Asn Val Gly Gly
  1               5                  10                  15

Thr Arg His Glu Thr Tyr Arg Ser Thr Leu Lys Thr Leu Pro Gly Thr
             20                  25                  30

Arg Leu Ala Leu Leu Ala Ser Ser Glu Pro Pro Gly Asp Cys Leu Thr
         35                  40                  45

Thr Ala Gly Asn Cys Ser Ser Arg Gly Gly Arg Ala Ser Asp His Pro
     50                  55                  60

Gly Gly Gly Arg Glu Phe Phe Phe Asp Arg His Pro Gly Val Phe Ala
 65                  70                  75                  80

Tyr Val Leu Asn Tyr Tyr Arg Thr Gly Lys Leu His Cys Pro Ala Asp
                 85                  90                  95

Val Cys Gly Pro Leu Phe Glu Glu Glu Leu Ala Phe Trp Gly Ile Asp
            100                 105                 110

Glu Thr Asp Val Glu Pro Cys Cys Trp Met Thr Tyr Arg Gln His Arg
        115                 120                 125

Asp Ala Glu Glu Ala Leu Asp Ile Phe Glu Thr Pro Asp Leu Ile Gly
    130                 135                 140

Gly Asp Pro Gly Asp Asp Glu Asp Leu Ala Ala Lys Arg Leu Gly Ile
145                 150                 155                 160

Glu Asp Ala Ala Gly Leu Gly Gly Pro Asp Gly Lys Ser Gly Arg Trp
                165                 170                 175

Arg Arg Leu Gln Pro Arg Met Trp Ala Leu Phe Glu Asp Pro Tyr Ser
            180                 185                 190

Ser Arg Ala Ala Arg
        195

<210> 17
<211> 1815
<212> DNA
<213> Homo sapiens

<220>
<221> CDS
<222> (13)..(1809)

<400> 17
agatctccca cc atg ggc aag atc gag aac aac gag agg gtg atc ctc aat   51
              Met Gly Lys Ile Glu Asn Asn Glu Arg Val Ile Leu Asn
                1               5                   10

gtc ggg ggc acc cgg cac gaa acc tac cgc agc acc ctc aag acc ctg    99
Val Gly Gly Thr Arg His Glu Thr Tyr Arg Ser Thr Leu Lys Thr Leu
     15                  20                  25

cct gga aca cgc ctg gcc ctt ctt gcc tcc tcc gag ccc cca ggc aac   147
Pro Gly Thr Arg Leu Ala Leu Leu Ala Ser Ser Glu Pro Pro Gly Asn
 30                  35                  40                  45

tgc agt tcc cgc ggc ggc agg gcc agc gac cat ccc ggt ggc ggc cgc   195
Cys Ser Ser Arg Gly Gly Arg Ala Ser Asp His Pro Gly Gly Gly Arg
                 50                  55                  60

gag ttc ttc ttc gac cgg cac ccg ggc gtc ttc gcc tat gtg ctc aat   243
Glu Phe Phe Phe Asp Arg His Pro Gly Val Phe Ala Tyr Val Leu Asn
             65                  70                  75

tac tac cgc acc ggc aag ctg cac tgc ccc gca gac gtg tgc ggg ccg   291
Tyr Tyr Arg Thr Gly Lys Leu His Cys Pro Ala Asp Val Cys Gly Pro
         80                  85                  90

ctc ttc gag gag gag ctg gcc ttc tgg ggc atc gac gag acc gac gtg   339
Leu Phe Glu Glu Glu Leu Ala Phe Trp Gly Ile Asp Glu Thr Asp Val
     95                 100                 105

gag ccc tgc tgc tgg atg acc tac cgg cag cac cgc gac gcc gag gag   387
Glu Pro Cys Cys Trp Met Thr Tyr Arg Gln His Arg Asp Ala Glu Glu
110                 115                 120                 125

gcg ctg gac atc ttc gag acc ccc gac ctc att ggc ggc gac ccc ggc   435
Ala Leu Asp Ile Phe Glu Thr Pro Asp Leu Ile Gly Gly Asp Pro Gly
                130                 135                 140

gac gac gag gac ctg gcg gcc aag agg ctg ggc atc gag gac gcg gcg   483
Asp Asp Glu Asp Leu Ala Ala Lys Arg Leu Gly Ile Glu Asp Ala Ala
            145                 150                 155

ggg ctc ggg ggc ccc gac ggc aaa tct ggc cgc tgg agg agg ctg cag   531
Gly Leu Gly Gly Pro Asp Gly Lys Ser Gly Arg Trp Arg Arg Leu Gln
        160                 165                 170

ccc cgc atg tgg gcc ctc ttc gaa gac ccc tac tcg tcc aga gcc gcc   579
Pro Arg Met Trp Ala Leu Phe Glu Asp Pro Tyr Ser Ser Arg Ala Ala
    175                 180                 185

agg ttt att gct ttt gct tct tta ttc ttc atc ctg gtt tca att aca 627
Arg Phe Ile Ala Phe Ala Ser Leu Phe Phe Ile Leu Val Ser Ile Thr
190                 195                 200                 205

act ttt tgc ctg gaa aca cat gaa gct ttc aat att gtt aaa aac aag 675
Thr Phe Cys Leu Glu Thr His Glu Ala Phe Asn Ile Val Lys Asn Lys
                210                 215                 220

aca gaa cca gtc atc aat ggc aca agt gtt gtt cta cag tat gaa att 723
Thr Glu Pro Val Ile Asn Gly Thr Ser Val Val Leu Gln Tyr Glu Ile
            225                 230                 235

gaa acg gat cct gcc ttg acg tat gta gaa gga gtg tgt gtg gtg tgg 771
Glu Thr Asp Pro Ala Leu Thr Tyr Val Glu Gly Val Cys Val Val Trp
        240                 245                 250

ttt act ttt gaa ttt tta gtc cgt att gtt ttt tca ccc aat aaa ctt 819
Phe Thr Phe Glu Phe Leu Val Arg Ile Val Phe Ser Pro Asn Lys Leu
    255                 260                 265

gaa ttc atc aaa aat ctc ttg aat atc att gac ttt gtg gcc atc cta 867
Glu Phe Ile Lys Asn Leu Leu Asn Ile Ile Asp Phe Val Ala Ile Leu
270                 275                 280                 285

cct ttc tac tta gag gtg gga ctc agt ggg ctg tca tcc aaa gct gct 915
Pro Phe Tyr Leu Glu Val Gly Leu Ser Gly Leu Ser Ser Lys Ala Ala
                290                 295                 300

aaa gat gtg ctt ggc ttc ctc agg gtg gta agg ttt gtg agg atc ctg 963
Lys Asp Val Leu Gly Phe Leu Arg Val Val Arg Phe Val Arg Ile Leu
            305                 310                 315

aga att ttc aag ctc acc cgc cat ttt gta ggt ctg agg gtg ctt gga 1011
Arg Ile Phe Lys Leu Thr Arg His Phe Val Gly Leu Arg Val Leu Gly
        320                 325                 330

cat act ctt cga gct agt act aat gaa ttt ttg ctg ctg ata att ttc 1059
His Thr Leu Arg Ala Ser Thr Asn Glu Phe Leu Leu Leu Ile Ile Phe
    335                 340                 345

ctg gct cta gga gtt ttg ata ttt gct acc atg atc tac tat gcc gag 1107
Leu Ala Leu Gly Val Leu Ile Phe Ala Thr Met Ile Tyr Tyr Ala Glu
350                 355                 360                 365

aga gtg gga gct caa cct aac gac cct tca gct agt gag cac aca cag 1155
Arg Val Gly Ala Gln Pro Asn Asp Pro Ser Ala Ser Glu His Thr Gln
                370                 375                 380

ttc aaa aac att ccc att ggg ttc tgg tgg gct gta gtg acc atg act 1203
Phe Lys Asn Ile Pro Ile Gly Phe Trp Trp Ala Val Val Thr Met Thr
            385                 390                 395

acc ctg ggt tat gag gat acg tac ccc caa aca tgg tca ggc atg ctg 1251
Thr Leu Gly Tyr Glu Asp Thr Tyr Pro Gln Thr Trp Ser Gly Met Leu
        400                 405                 410

gtg gga gcc ctg tgt gct ctg gct gga gtg ctg aca ata gcc atg cca 1299
Val Gly Ala Leu Cys Ala Leu Ala Gly Val Leu Thr Ile Ala Met Pro
    415                 420                 425

gtg cct gtc att gtc aat aat ttt gga atg tac tac tcc ttg gca atg 1347

Val Pro Val Ile Val Asn Asn Phe Gly Met Tyr Tyr Ser Leu Ala Met
430                 435                 440                 445

gca aag cag aaa ctt cca agg aaa aga aag aag cac atc cct cct gct     1395
Ala Lys Gln Lys Leu Pro Arg Lys Arg Lys Lys His Ile Pro Pro Ala
                450                 455                 460

cct cag gca agc tca cct act ttt tgc aag aca gaa tta aat atg gcc     1443
Pro Gln Ala Ser Ser Pro Thr Phe Cys Lys Thr Glu Leu Asn Met Ala
            465                 470                 475

tgc aat agt aca cag agt gac aca tgt ctg ggc aaa gac aat cga ctt     1491
Cys Asn Ser Thr Gln Ser Asp Thr Cys Leu Gly Lys Asp Asn Arg Leu
        480                 485                 490

ctg gaa cat aac aga tca gtg tta tca ggt gac gac agt aca gga agt     1539
Leu Glu His Asn Arg Ser Val Leu Ser Gly Asp Asp Ser Thr Gly Ser
    495                 500                 505

gag ccg cca cta tca ccc cca gaa agg ctc ccc atc aga cgc tct agt     1587
Glu Pro Pro Leu Ser Pro Pro Glu Arg Leu Pro Ile Arg Arg Ser Ser
510                 515                 520                 525

acc aga gac aaa aac aga aga ggg gaa aca tgt ttc cta ctg acg aca     1635
Thr Arg Asp Lys Asn Arg Arg Gly Glu Thr Cys Phe Leu Leu Thr Thr
                530                 535                 540

ggt gat tac acg tgt gct tct gat gga ggg atc agg aaa gga tat gaa     1683
Gly Asp Tyr Thr Cys Ala Ser Asp Gly Gly Ile Arg Lys Gly Tyr Glu
            545                 550                 555

aaa tcc cga agc tta aac aac ata gcg ggc ttg gca ggc aat gct ctg     1731
Lys Ser Arg Ser Leu Asn Asn Ile Ala Gly Leu Ala Gly Asn Ala Leu
        560                 565                 570

agg ctc tct cca gta aca tca ccc tac aac tct cct tgt cct ctg agg     1779
Arg Leu Ser Pro Val Thr Ser Pro Tyr Asn Ser Pro Cys Pro Leu Arg
    575                 580                 585

cgc tct cga tct ccc atc cca tct atc ttg ctcgag                      1815
Arg Ser Arg Ser Pro Ile Pro Ser Ile Leu
590                 595

<210> 18
<211> 599
<212> PRT
<213> Homo sapiens

<400> 18
Met Gly Lys Ile Glu Asn Asn Glu Arg Val Ile Leu Asn Val Gly Gly
  1               5                  10                  15

Thr Arg His Glu Thr Tyr Arg Ser Thr Leu Lys Thr Leu Pro Gly Thr
             20                  25                  30

Arg Leu Ala Leu Leu Ala Ser Ser Glu Pro Pro Gly Asn Cys Ser Ser
         35                  40                  45

Arg Gly Gly Arg Ala Ser Asp His Pro Gly Gly Gly Arg Glu Phe Phe
     50                  55                  60

Phe Asp Arg His Pro Gly Val Phe Ala Tyr Val Leu Asn Tyr Tyr Arg
65                  70                  75                  80

Thr Gly Lys Leu His Cys Pro Ala Asp Val Cys Gly Pro Leu Phe Glu
                85                  90                  95

Glu Glu Leu Ala Phe Trp Gly Ile Asp Glu Thr Asp Val Glu Pro Cys
            100                 105                 110

Cys Trp Met Thr Tyr Arg Gln His Arg Asp Ala Glu Glu Ala Leu Asp
        115                 120                 125

Ile Phe Glu Thr Pro Asp Leu Ile Gly Gly Asp Pro Gly Asp Asp Glu
    130                 135                 140

Asp Leu Ala Ala Lys Arg Leu Gly Ile Glu Asp Ala Ala Gly Leu Gly
145                 150                 155                 160

Gly Pro Asp Gly Lys Ser Gly Arg Trp Arg Arg Leu Gln Pro Arg Met
                165                 170                 175

Trp Ala Leu Phe Glu Asp Pro Tyr Ser Ser Arg Ala Ala Arg Phe Ile
            180                 185                 190

Ala Phe Ala Ser Leu Phe Phe Ile Leu Val Ser Ile Thr Thr Phe Cys
        195                 200                 205

Leu Glu Thr His Glu Ala Phe Asn Ile Val Lys Asn Lys Thr Glu Pro
    210                 215                 220

Val Ile Asn Gly Thr Ser Val Val Leu Gln Tyr Glu Ile Glu Thr Asp
225                 230                 235                 240

Pro Ala Leu Thr Tyr Val Glu Gly Val Cys Val Val Trp Phe Thr Phe
                245                 250                 255

Glu Phe Leu Val Arg Ile Val Phe Ser Pro Asn Lys Leu Glu Phe Ile
            260                 265                 270

Lys Asn Leu Leu Asn Ile Ile Asp Phe Val Ala Ile Leu Pro Phe Tyr
        275                 280                 285

Leu Glu Val Gly Leu Ser Gly Leu Ser Ser Lys Ala Ala Lys Asp Val
    290                 295                 300

Leu Gly Phe Leu Arg Val Val Arg Phe Val Arg Ile Leu Arg Ile Phe
305                 310                 315                 320

Lys Leu Thr Arg His Phe Val Gly Leu Arg Val Leu Gly His Thr Leu
                325                 330                 335

Arg Ala Ser Thr Asn Glu Phe Leu Leu Leu Ile Ile Phe Leu Ala Leu
            340                 345                 350

Gly Val Leu Ile Phe Ala Thr Met Ile Tyr Tyr Ala Glu Arg Val Gly
        355                 360                 365

Ala Gln Pro Asn Asp Pro Ser Ala Ser Glu His Thr Gln Phe Lys Asn
    370                 375                 380

Ile Pro Ile Gly Phe Trp Trp Ala Val Val Thr Met Thr Thr Leu Gly

385                 390                 395                 400

Tyr Glu Asp Thr Tyr Pro Gln Thr Trp Ser Gly Met Leu Val Gly Ala
                405                 410                 415

Leu Cys Ala Leu Ala Gly Val Leu Thr Ile Ala Met Pro Val Pro Val
            420                 425                 430

Ile Val Asn Asn Phe Gly Met Tyr Tyr Ser Leu Ala Met Ala Lys Gln
        435                 440                 445

Lys Leu Pro Arg Lys Arg Lys Lys His Ile Pro Pro Ala Pro Gln Ala
    450                 455                 460

Ser Ser Pro Thr Phe Cys Lys Thr Glu Leu Asn Met Ala Cys Asn Ser
465                 470                 475                 480

Thr Gln Ser Asp Thr Cys Leu Gly Lys Asp Asn Arg Leu Leu Glu His
                485                 490                 495

Asn Arg Ser Val Leu Ser Gly Asp Asp Ser Thr Gly Ser Glu Pro Pro
            500                 505                 510

Leu Ser Pro Pro Glu Arg Leu Pro Ile Arg Arg Ser Ser Thr Arg Asp
        515                 520                 525

Lys Asn Arg Arg Gly Glu Thr Cys Phe Leu Leu Thr Thr Gly Asp Tyr
    530                 535                 540

Thr Cys Ala Ser Asp Gly Gly Ile Arg Lys Gly Tyr Glu Lys Ser Arg
545                 550                 555                 560

Ser Leu Asn Asn Ile Ala Gly Leu Ala Gly Asn Ala Leu Arg Leu Ser
                565                 570                 575

Pro Val Thr Ser Pro Tyr Asn Ser Pro Cys Pro Leu Arg Arg Ser Arg
            580                 585                 590

Ser Pro Ile Pro Ser Ile Leu
        595

<210> 19
<211> 3028
<212> DNA
<213> Homo sapiens

<220>
<221> CDS
<222> (22)..(1860)

<400> 19
agtcatgtct gagtcacaga g atg ggc aag atc gag aac aac gag agg gtg      51
                        Met Gly Lys Ile Glu Asn Asn Glu Arg Val
                        1               5                   10

atc ctc aat gtc ggg ggc acc cgg cac gaa acc tac cgc agc acc ctc      99
Ile Leu Asn Val Gly Gly Thr Arg His Glu Thr Tyr Arg Ser Thr Leu
                15                  20                  25

aag acc ctg cct gga aca cgc ctg gcc ctt ctt gcc tcc tcc gag ccc      147
Lys Thr Leu Pro Gly Thr Arg Leu Ala Leu Leu Ala Ser Ser Glu Pro

30                  35                  40

cca ggc gac tgc ttg acc acg gcg ggc gac aag ctg cag ccg tcg ccg     195
Pro Gly Asp Cys Leu Thr Thr Ala Gly Asp Lys Leu Gln Pro Ser Pro
        45                  50                  55

cct cca ctg tcg ccg ccg ccg aga gcg ccc ccg ctg tcc ccc ggg cca     243
Pro Pro Leu Ser Pro Pro Pro Arg Ala Pro Pro Leu Ser Pro Gly Pro
    60                  65                  70

ggc ggc tgc ttc gag ggc ggc gcg ggc aac tgc agt tcc cgc ggc ggc     291
Gly Gly Cys Phe Glu Gly Gly Ala Gly Asn Cys Ser Ser Arg Gly Gly
75                  80                  85                  90

agg gcc agc gac cat ccc ggt ggc ggc cgc gag ttc ttc ttc gac cgg     339
Arg Ala Ser Asp His Pro Gly Gly Gly Arg Glu Phe Phe Phe Asp Arg
                95                  100                 105

cac ccg ggc gtc ttc gcc tat gtg ctc aat tac tac cgc acc ggc aag     387
His Pro Gly Val Phe Ala Tyr Val Leu Asn Tyr Tyr Arg Thr Gly Lys
            110                 115                 120

ctg cac tgc ccc gca gac gtg tgc ggg ccg ctc ttc gag gag gag ctg     435
Leu His Cys Pro Ala Asp Val Cys Gly Pro Leu Phe Glu Glu Glu Leu
        125                 130                 135

gcc ttc tgg ggc atc gac gag acc gac gtg gag ccc tgc tgc tgg atg     483
Ala Phe Trp Gly Ile Asp Glu Thr Asp Val Glu Pro Cys Cys Trp Met
    140                 145                 150

acc tac cgg cag cac cgc gac gcc gag gag gcg ctg gac atc ttc gag     531
Thr Tyr Arg Gln His Arg Asp Ala Glu Glu Ala Leu Asp Ile Phe Glu
155                 160                 165                 170

acc ccc gac ctc att ggc ggc gac ccc ggc gac gac gag gac ctg gcg     579
Thr Pro Asp Leu Ile Gly Gly Asp Pro Gly Asp Asp Glu Asp Leu Ala
                175                 180                 185

gcc aag agg ctg ggc atc gag gac gcg gcg ggg ctc ggg ggc ccc gac     627
Ala Lys Arg Leu Gly Ile Glu Asp Ala Ala Gly Leu Gly Gly Pro Asp
            190                 195                 200

ggc aaa tct ggc cgc tgg agg agg ctg cag ccc cgc atg tgg gcc ctc     675
Gly Lys Ser Gly Arg Trp Arg Arg Leu Gln Pro Arg Met Trp Ala Leu
        205                 210                 215

ttc gaa gac ccc tac tcg tcc aga gcc gcc agg ttt att gct ttt gct     723
Phe Glu Asp Pro Tyr Ser Ser Arg Ala Ala Arg Phe Ile Ala Phe Ala
    220                 225                 230

tct tta ttc ttc atc ctg gtt tca att aca act ttt tgc ctg gaa aca     771
Ser Leu Phe Phe Ile Leu Val Ser Ile Thr Thr Phe Cys Leu Glu Thr
235                 240                 245                 250

cat gaa gct ttc aat att gtt aaa aac aag aca gaa cca gtc atc aat     819
His Glu Ala Phe Asn Ile Val Lys Asn Lys Thr Glu Pro Val Ile Asn
                255                 260                 265

ggc aca agt gtt gtt cta cag tat gaa att gaa acg gat cct gcc ttg     867
Gly Thr Ser Val Val Leu Gln Tyr Glu Ile Glu Thr Asp Pro Ala Leu
            270                 275                 280

acg tat gta gaa gga gtg tgt gtg gtg tgg ttt act ttt gaa ttt tta    915
Thr Tyr Val Glu Gly Val Cys Val Val Trp Phe Thr Phe Glu Phe Leu
        285                 290                 295

gtc cgt att gtt ttt tca ccc aat aaa ctt gaa ttc atc aaa aat ctc    963
Val Arg Ile Val Phe Ser Pro Asn Lys Leu Glu Phe Ile Lys Asn Leu
    300                 305                 310

ttg aat atc att gac ttt gtg gcc atc cta cct ttc tac tta gag gtg    1011
Leu Asn Ile Ile Asp Phe Val Ala Ile Leu Pro Phe Tyr Leu Glu Val
315                 320                 325                 330

gga ctc agt ggg ctg tca tcc aaa gct gct aaa gat gtg ctt ggc ttc    1059
Gly Leu Ser Gly Leu Ser Ser Lys Ala Ala Lys Asp Val Leu Gly Phe
                335                 340                 345

ctc agg gtg gta agg ttt gtg agg atc ctg aga att ttc aag ctc acc    1107
Leu Arg Val Val Arg Phe Val Arg Ile Leu Arg Ile Phe Lys Leu Thr
            350                 355                 360

cgc cat ttt gta ggt ctg agg gtg ctt gga cat act ctt cga gct agt    1155
Arg His Phe Val Gly Leu Arg Val Leu Gly His Thr Leu Arg Ala Ser
        365                 370                 375

act aat gaa ttt ttg ctg ctg ata att ttc ctg gct cta gga gtt ttg    1203
Thr Asn Glu Phe Leu Leu Leu Ile Ile Phe Leu Ala Leu Gly Val Leu
    380                 385                 390

ata ttt gct acc atg atc tac tat gcc gag aga gtg gga gct caa cct    1251
Ile Phe Ala Thr Met Ile Tyr Tyr Ala Glu Arg Val Gly Ala Gln Pro
395                 400                 405                 410

aac gac cct tca gct agt gag cac aca cag ttc aaa aac att ccc att    1299
Asn Asp Pro Ser Ala Ser Glu His Thr Gln Phe Lys Asn Ile Pro Ile
                415                 420                 425

ggg ttc tgg tgg gct gta gtg acc atg act acc ctg ggt tat ggg gat    1347
Gly Phe Trp Trp Ala Val Val Thr Met Thr Thr Leu Gly Tyr Gly Asp
            430                 435                 440

atg tac ccc caa aca tgg tca ggc atg ctg gtg gga gcc ctg tgt gct    1395
Met Tyr Pro Gln Thr Trp Ser Gly Met Leu Val Gly Ala Leu Cys Ala
        445                 450                 455

ctg gct gga gtg ctg aca ata gcc atg cca gtg cct gtc att gtc aat    1443
Leu Ala Gly Val Leu Thr Ile Ala Met Pro Val Pro Val Ile Val Asn
    460                 465                 470

aat ttt gga atg tac tac tcc ttg gca atg gca aag cag aaa ctt cca    1491
Asn Phe Gly Met Tyr Tyr Ser Leu Ala Met Ala Lys Gln Lys Leu Pro
475                 480                 485                 490

agg aaa aga aag aag cac atc cct cct gct cct cag gca agc tca cct    1539
Arg Lys Arg Lys Lys His Ile Pro Pro Ala Pro Gln Ala Ser Ser Pro
                495                 500                 505

act ttt tgc aag aca gaa tta aat atg gcc tgc aat agt aca cag agt    1587
Thr Phe Cys Lys Thr Glu Leu Asn Met Ala Cys Asn Ser Thr Gln Ser
            510                 515                 520

gac aca tgt ctg ggc aaa gac aat cga ctt ctg gaa cat aac aga tca 1635
Asp Thr Cys Leu Gly Lys Asp Asn Arg Leu Leu Glu His Asn Arg Ser
        525                 530                 535

gtg tta tca ggt gac gac agt aca gga agt gag ccg cca cta tca ccc 1683
Val Leu Ser Gly Asp Asp Ser Thr Gly Ser Glu Pro Pro Leu Ser Pro
    540                 545                 550

cca gaa agg ctc ccc atc aga cgc tct agt acc aga gac aaa aac aga 1731
Pro Glu Arg Leu Pro Ile Arg Arg Ser Ser Thr Arg Asp Lys Asn Arg
555                 560                 565                 570

aga ggg gaa aca tgt ttc cta ctg acg aca ggt gat tac acg tgt gct 1779
Arg Gly Glu Thr Cys Phe Leu Leu Thr Thr Gly Asp Tyr Thr Cys Ala
                575                 580                 585

tct gat gga ggg atc agg aaa gat aac tgc aaa gag gtt gtc att act 1827
Ser Asp Gly Gly Ile Arg Lys Asp Asn Cys Lys Glu Val Val Ile Thr
            590                 595                 600

ggt tac acg caa gcc gag gcc aga tct ctt act taatgacttg ggggaaggca 1880
Gly Tyr Thr Gln Ala Glu Ala Arg Ser Leu Thr
        605                 610

caaaacatga gagaaagtgt tgtacagaat ttatcatgga ttattgactg ctgagaaagg 1940

gacagtggaa tttagccata caaaggacta tactggaaac agacttctgc tgctgaatgt 2000

gccctgatgt gaccaggttg cacttggaag agatcctccg cgtcttcatg aggcacttaa 2060

agcttataaa agaactgcgg ctggaactca tctggtgctc cccatgagag tgctctgctt 2120

gtagactggc cagtgtccat gaaacaactg taaataccaa catgtgtgca tgggtcaaca 2180

gtcttggcca tttctcatca aaagaagcca aattcatgat caacatctct gaagtttcaa 2240

gtaaggccca cacttctttg aattactctt catgggccca cattaggttg tgctgtgaat 2300

tacttaaggc agtgatactg atgtagtata gttttgtctt aatttccctt atttctactt 2360

ctttggttga atctatgaac ttgattgtat aattttctta taaattactg atgtaatcag 2420

cttgtcaatt atgttgtgaa attgttagta ttcatttatc aaaaatgacc tatgtttagt 2480

cacatatttg tttagttctg ggaaattgtt atagcttaaa tggaactcac caacattatt 2540

catagtttaa gtcttttatc attattacct caattataaa tattacaaaa acataattct 2600

ggcaatgaga gtattttttt attcaatgat caaggagcaa tgtcagtata tagtagaata 2660

tcaattaaat tatatcctaa aatgtatatt ttgcataaaa gagatattct ttaatcaatt 2720

acttttttgt gagttttgtg gcgaatgaag cttgtacgtg tctttaaaac tgttgtagat 2780

gaaactgtat aagattttta catcttgctt aatcaatatt ttcagagtct attagttccc 2840

ctgggattct gaatataaca tatagcctat tataaatccc tgtatcgtgg accttttgtg 2900

aacatttcaa ggcgcatgca caaccttgat gataaccagt ggaaatgtaa ctaactgaaa 2960

tgaagaataa aaggcaaatg agctggggat aaacttgaat gttatctgat taaattactc 3020

aaattatt                                                          3028

<210> 20
<211> 613
<212> PRT
<213> Homo sapiens

<400> 20
Met Gly Lys Ile Glu Asn Asn Glu Arg Val Ile Leu Asn Val Gly Gly
1               5                   10                  15

Thr Arg His Glu Thr Tyr Arg Ser Thr Leu Lys Thr Leu Pro Gly Thr
            20                  25                  30

Arg Leu Ala Leu Leu Ala Ser Ser Glu Pro Pro Gly Asp Cys Leu Thr
        35                  40                  45

Thr Ala Gly Asp Lys Leu Gln Pro Ser Pro Pro Pro Leu Ser Pro Pro
    50                  55                  60

Pro Arg Ala Pro Pro Leu Ser Pro Gly Pro Gly Gly Cys Phe Glu Gly
65                  70                  75                  80

Gly Ala Gly Asn Cys Ser Ser Arg Gly Gly Arg Ala Ser Asp His Pro
                85                  90                  95

Gly Gly Gly Arg Glu Phe Phe Phe Asp Arg His Pro Gly Val Phe Ala
            100                 105                 110

Tyr Val Leu Asn Tyr Tyr Arg Thr Gly Lys Leu His Cys Pro Ala Asp
        115                 120                 125

Val Cys Gly Pro Leu Phe Glu Glu Glu Leu Ala Phe Trp Gly Ile Asp
    130                 135                 140

Glu Thr Asp Val Glu Pro Cys Cys Trp Met Thr Tyr Arg Gln His Arg
145                 150                 155                 160

Asp Ala Glu Glu Ala Leu Asp Ile Phe Glu Thr Pro Asp Leu Ile Gly
                165                 170                 175

Gly Asp Pro Gly Asp Asp Glu Asp Leu Ala Ala Lys Arg Leu Gly Ile
            180                 185                 190

Glu Asp Ala Ala Gly Leu Gly Gly Pro Asp Gly Lys Ser Gly Arg Trp
        195                 200                 205

Arg Arg Leu Gln Pro Arg Met Trp Ala Leu Phe Glu Asp Pro Tyr Ser
    210                 215                 220

Ser Arg Ala Ala Arg Phe Ile Ala Phe Ala Ser Leu Phe Phe Ile Leu
225                 230                 235                 240

Val Ser Ile Thr Thr Phe Cys Leu Glu Thr His Glu Ala Phe Asn Ile
                245                 250                 255

Val Lys Asn Lys Thr Glu Pro Val Ile Asn Gly Thr Ser Val Val Leu
            260                 265                 270

Gln Tyr Glu Ile Glu Thr Asp Pro Ala Leu Thr Tyr Val Glu Gly Val

        275                 280                 285

Cys Val Val Trp Phe Thr Phe Glu Phe Leu Val Arg Ile Val Phe Ser
    290                 295                 300

Pro Asn Lys Leu Glu Phe Ile Lys Asn Leu Leu Asn Ile Ile Asp Phe
305                 310                 315                 320

Val Ala Ile Leu Pro Phe Tyr Leu Glu Val Gly Leu Ser Gly Leu Ser
                325                 330                 335

Ser Lys Ala Ala Lys Asp Val Leu Gly Phe Leu Arg Val Val Arg Phe
            340                 345                 350

Val Arg Ile Leu Arg Ile Phe Lys Leu Thr Arg His Phe Val Gly Leu
        355                 360                 365

Arg Val Leu Gly His Thr Leu Arg Ala Ser Thr Asn Glu Phe Leu Leu
    370                 375                 380

Leu Ile Ile Phe Leu Ala Leu Gly Val Leu Ile Phe Ala Thr Met Ile
385                 390                 395                 400

Tyr Tyr Ala Glu Arg Val Gly Ala Gln Pro Asn Asp Pro Ser Ala Ser
                405                 410                 415

Glu His Thr Gln Phe Lys Asn Ile Pro Ile Gly Phe Trp Trp Ala Val
            420                 425                 430

Val Thr Met Thr Thr Leu Gly Tyr Gly Asp Met Tyr Pro Gln Thr Trp
        435                 440                 445

Ser Gly Met Leu Val Gly Ala Leu Cys Ala Leu Ala Gly Val Leu Thr
    450                 455                 460

Ile Ala Met Pro Val Pro Val Ile Val Asn Asn Phe Gly Met Tyr Tyr
465                 470                 475                 480

Ser Leu Ala Met Ala Lys Gln Lys Leu Pro Arg Lys Arg Lys Lys His
                485                 490                 495

Ile Pro Pro Ala Pro Gln Ala Ser Ser Pro Thr Phe Cys Lys Thr Glu
            500                 505                 510

Leu Asn Met Ala Cys Asn Ser Thr Gln Ser Asp Thr Cys Leu Gly Lys
        515                 520                 525

Asp Asn Arg Leu Leu Glu His Asn Arg Ser Val Leu Ser Gly Asp Asp
    530                 535                 540

Ser Thr Gly Ser Glu Pro Pro Leu Ser Pro Pro Glu Arg Leu Pro Ile
545                 550                 555                 560

Arg Arg Ser Ser Thr Arg Asp Lys Asn Arg Arg Gly Glu Thr Cys Phe
                565                 570                 575

Leu Leu Thr Thr Gly Asp Tyr Thr Cys Ala Ser Asp Gly Gly Ile Arg
            580                 585                 590

Lys Asp Asn Cys Lys Glu Val Val Ile Thr Gly Tyr Thr Gln Ala Glu
        595                 600                 605

Ala Arg Ser Leu Thr
    610

<210> 21
<211> 1193
<212> DNA
<213> Homo sapiens

<220>
<221> CDS
<222> (105)..(1142)

<400> 21
tgctccctgt ttcattaaaa cctagagaga tgtaatcagt aagcaagaag gaaaaaggga   60

aattcacaaa gtaacttttt gtgtctgttt ctttttaacc cagc atg gag aga aaa   116
                                                 Met Glu Arg Lys
                                                 1

ttt atg tcc ttg caa cca tcc atc tcc gta tca gaa atg gaa cca aat   164
Phe Met Ser Leu Gln Pro Ser Ile Ser Val Ser Glu Met Glu Pro Asn
5                   10                  15                  20

ggc acc ttc agc aat aac aac agc agg aac tgc aca att gaa aac ttc   212
Gly Thr Phe Ser Asn Asn Asn Ser Arg Asn Cys Thr Ile Glu Asn Phe
                25                  30                  35

aag aga gaa ttt ttc cca att gta tat ctg ata ata ttt ttc tgg gga   260
Lys Arg Glu Phe Phe Pro Ile Val Tyr Leu Ile Ile Phe Phe Trp Gly
            40                  45                  50

gtc ttg gga aat ggg ttg tcc ata tat gtt ttc ctg cag cct tat aag   308
Val Leu Gly Asn Gly Leu Ser Ile Tyr Val Phe Leu Gln Pro Tyr Lys
        55                  60                  65

aag tcc aca tct gtg aac gtt ttc atg cta aat ctg gcc att tca gat   356
Lys Ser Thr Ser Val Asn Val Phe Met Leu Asn Leu Ala Ile Ser Asp
    70                  75                  80

ctc ctg ttc ata agc acg ctt ccc ttc agg gct gac tat tat ctt aga   404
Leu Leu Phe Ile Ser Thr Leu Pro Phe Arg Ala Asp Tyr Tyr Leu Arg
85                  90                  95                  100

ggc tcc aat tgg ata ttt gga gac ctg gcc tgc agg att atg tct tat   452
Gly Ser Asn Trp Ile Phe Gly Asp Leu Ala Cys Arg Ile Met Ser Tyr
                105                 110                 115

tcc ttg tat gtc aac atg tac agc agt att tat ttc ctg acc gtg ctg   500
Ser Leu Tyr Val Asn Met Tyr Ser Ser Ile Tyr Phe Leu Thr Val Leu
            120                 125                 130

agt gtt gtg cgt ttc ctg gca atg gtt cac ccc ttt cgg ctt ctg cat   548
Ser Val Val Arg Phe Leu Ala Met Val His Pro Phe Arg Leu Leu His
        135                 140                 145

gtc acc agc atc agg agt gcc tgg atc ctc tgt ggg atc ata tgg atc   596
Val Thr Ser Ile Arg Ser Ala Trp Ile Leu Cys Gly Ile Ile Trp Ile
    150                 155                 160

ctt atc atg gct tcc tca ata atg ctc ctg gac agt ggc tct gag cag   644

Leu Ile Met Ala Ser Ser Ile Met Leu Leu Asp Ser Gly Ser Glu Gln
165                 170                 175                 180

aac ggc agt gtc aca tca tgc tta gag ctg aat ctc tat aaa att gct     692
Asn Gly Ser Val Thr Ser Cys Leu Glu Leu Asn Leu Tyr Lys Ile Ala
                185                 190                 195

aag ctg cag acc atg aac tat att gcc ttg gtg gtg ggc tgc ctg ctg     740
Lys Leu Gln Thr Met Asn Tyr Ile Ala Leu Val Val Gly Cys Leu Leu
            200                 205                 210

cca ttt ttc aca ctc agc atc tgt tat ctg ctg atc att cgg gtt ctg     788
Pro Phe Phe Thr Leu Ser Ile Cys Tyr Leu Leu Ile Ile Arg Val Leu
        215                 220                 225

tta aaa gtg gag gtc cca gaa tcg ggg ctg cgg gtt tct cac agg aag     836
Leu Lys Val Glu Val Pro Glu Ser Gly Leu Arg Val Ser His Arg Lys
    230                 235                 240

gca ctg acc acc atc atc atc acc ttg atc atc ttc ttc ttg tgt ttc     884
Ala Leu Thr Thr Ile Ile Ile Thr Leu Ile Ile Phe Phe Leu Cys Phe
245                 250                 255                 260

ctg ccc tat cac aca ctg agg acc gtc cac ttg acg aca tgg aaa gtg     932
Leu Pro Tyr His Thr Leu Arg Thr Val His Leu Thr Thr Trp Lys Val
                265                 270                 275

ggt tta tgc aaa gac aga ctg cat aaa gct ttg gtt atc aca ctg gcc     980
Gly Leu Cys Lys Asp Arg Leu His Lys Ala Leu Val Ile Thr Leu Ala
            280                 285                 290

ttg gca gca gcc aat gcc tgc ttc aat cct ctg ctc tat tac ttt gct     1028
Leu Ala Ala Ala Asn Ala Cys Phe Asn Pro Leu Leu Tyr Tyr Phe Ala
        295                 300                 305

ggg gag aat ttt aag gac aga cta aag tct gca ctc aga aaa ggc cat     1076
Gly Glu Asn Phe Lys Asp Arg Leu Lys Ser Ala Leu Arg Lys Gly His
    310                 315                 320

cca cag aag gca aag aca aag tgt gtt ttc cct gtt agt gtg tgg ttg     1124
Pro Gln Lys Ala Lys Thr Lys Cys Val Phe Pro Val Ser Val Trp Leu
325                 330                 335                 340

aga aag gaa aca aga gta taaggagctc ttagatgaga cctgttcttg           1172
Arg Lys Glu Thr Arg Val
                345

tatccttgtg tccatcttca t                                             1193

<210> 22
<211> 346
<212> PRT
<213> Homo sapiens

<400> 22
Met Glu Arg Lys Phe Met Ser Leu Gln Pro Ser Ile Ser Val Ser Glu
  1               5                  10                  15

Met Glu Pro Asn Gly Thr Phe Ser Asn Asn Asn Ser Arg Asn Cys Thr
             20                  25                  30

Ile Glu Asn Phe Lys Arg Glu Phe Phe Pro Ile Val Tyr Leu Ile Ile
        35                  40                  45

Phe Phe Trp Gly Val Leu Gly Asn Gly Leu Ser Ile Tyr Val Phe Leu
    50                  55                  60

Gln Pro Tyr Lys Lys Ser Thr Ser Val Asn Val Phe Met Leu Asn Leu
65                  70                  75                  80

Ala Ile Ser Asp Leu Leu Phe Ile Ser Thr Leu Pro Phe Arg Ala Asp
                85                  90                  95

Tyr Tyr Leu Arg Gly Ser Asn Trp Ile Phe Gly Asp Leu Ala Cys Arg
            100                 105                 110

Ile Met Ser Tyr Ser Leu Tyr Val Asn Met Tyr Ser Ser Ile Tyr Phe
        115                 120                 125

Leu Thr Val Leu Ser Val Val Arg Phe Leu Ala Met Val His Pro Phe
    130                 135                 140

Arg Leu Leu His Val Thr Ser Ile Arg Ser Ala Trp Ile Leu Cys Gly
145                 150                 155                 160

Ile Ile Trp Ile Leu Ile Met Ala Ser Ser Ile Met Leu Leu Asp Ser
                165                 170                 175

Gly Ser Glu Gln Asn Gly Ser Val Thr Ser Cys Leu Glu Leu Asn Leu
            180                 185                 190

Tyr Lys Ile Ala Lys Leu Gln Thr Met Asn Tyr Ile Ala Leu Val Val
        195                 200                 205

Gly Cys Leu Leu Pro Phe Phe Thr Leu Ser Ile Cys Tyr Leu Leu Ile
    210                 215                 220

Ile Arg Val Leu Leu Lys Val Glu Val Pro Glu Ser Gly Leu Arg Val
225                 230                 235                 240

Ser His Arg Lys Ala Leu Thr Thr Ile Ile Ile Thr Leu Ile Ile Phe
                245                 250                 255

Phe Leu Cys Phe Leu Pro Tyr His Thr Leu Arg Thr Val His Leu Thr
            260                 265                 270

Thr Trp Lys Val Gly Leu Cys Lys Asp Arg Leu His Lys Ala Leu Val
        275                 280                 285

Ile Thr Leu Ala Leu Ala Ala Ala Asn Ala Cys Phe Asn Pro Leu Leu
    290                 295                 300

Tyr Tyr Phe Ala Gly Glu Asn Phe Lys Asp Arg Leu Lys Ser Ala Leu
305                 310                 315                 320

Arg Lys Gly His Pro Gln Lys Ala Lys Thr Lys Cys Val Phe Pro Val
                325                 330                 335

Ser Val Trp Leu Arg Lys Glu Thr Arg Val
            340                 345

<210> 23

<211> 1260
<212> DNA
<213> Homo sapiens

<220>
<221> CDS
<222> (105)..(1142)

<400> 23
tgctccctgt ttcattaaaa cctagagaga tgtaatcagt aagcaagaag gaaaaaggga 60

aattcacaaa gtaacttttt gtgtctgttt ctttttaacc cagc atg gag aga aaa 116
Met Glu Arg Lys
1

ttt atg tcc ttg caa cca tcc atc tcc gta tca gaa atg gaa cca aat 164
Phe Met Ser Leu Gln Pro Ser Ile Ser Val Ser Glu Met Glu Pro Asn
5                   10                  15                  20

ggc acc ttc agc aat aac aac agc agg aac tgc aca att gaa aac ttc 212
Gly Thr Phe Ser Asn Asn Asn Ser Arg Asn Cys Thr Ile Glu Asn Phe
                25                  30                  35

aag aga gaa ttt ttc cca att gta tat ctg ata ata ttt ttc tgg gga 260
Lys Arg Glu Phe Phe Pro Ile Val Tyr Leu Ile Ile Phe Phe Trp Gly
            40                  45                  50

gtc ttg gga aat ggg ttg tcc ata tat gtt ttc ctg cag cct tat aag 308
Val Leu Gly Asn Gly Leu Ser Ile Tyr Val Phe Leu Gln Pro Tyr Lys
        55                  60                  65

aag tcc aca tct gtg aac gtt ttc atg cta aat ctg gcc att tca gat 356
Lys Ser Thr Ser Val Asn Val Phe Met Leu Asn Leu Ala Ile Ser Asp
    70                  75                  80

ctc ctg ttc ata agc acg ctt ccc ttc agg gct gac tat tat ctt aga 404
Leu Leu Phe Ile Ser Thr Leu Pro Phe Arg Ala Asp Tyr Tyr Leu Arg
85                  90                  95                  100

ggc tcc aat tgg ata ttt gga gac ctg gcc tgc agg att atg tct tat 452
Gly Ser Asn Trp Ile Phe Gly Asp Leu Ala Cys Arg Ile Met Ser Tyr
                105                 110                 115

tcc ttg tat gtc aac atg tac agc agt att tat ttc ctg acc gtg ctg 500
Ser Leu Tyr Val Asn Met Tyr Ser Ser Ile Tyr Phe Leu Thr Val Leu
            120                 125                 130

agt gtt gtg cgt ttc ctg gca atg gtt cac ccc ttt cgg ctt ctg cat 548
Ser Val Val Arg Phe Leu Ala Met Val His Pro Phe Arg Leu Leu His
        135                 140                 145

gtc acc agc atc agg agt gcc tgg atc ctc tgt ggg atc ata tgg atc 596
Val Thr Ser Ile Arg Ser Ala Trp Ile Leu Cys Gly Ile Ile Trp Ile
    150                 155                 160

ctt atc atg gct tcc tca ata atg ctc ctg gac agt ggc tct gag cag 644
Leu Ile Met Ala Ser Ser Ile Met Leu Leu Asp Ser Gly Ser Glu Gln
165                 170                 175                 180

aac ggc agt gtc aca tca tgc tta gag ctg aat ctc tat aaa att gct 692
Asn Gly Ser Val Thr Ser Cys Leu Glu Leu Asn Leu Tyr Lys Ile Ala

                185                 190                 195

aag ctg cag acc atg aac tat att gcc ttg gtg gtg ggc tgc ctg ctg 740
Lys Leu Gln Thr Met Asn Tyr Ile Ala Leu Val Val Gly Cys Leu Leu
            200                 205                 210

cca ttt ttc aca ctc agc atc tgt tat ctg ctg atc att cgg gtt ctg 788
Pro Phe Phe Thr Leu Ser Ile Cys Tyr Leu Leu Ile Ile Arg Val Leu
        215                 220                 225

tta aaa gtg gag gtc cca gaa tcg ggg ctg cgg gtt tct cac agg aag 836
Leu Lys Val Glu Val Pro Glu Ser Gly Leu Arg Val Ser His Arg Lys
    230                 235                 240

gca ctg acc acc atc atc atc acc ttg atc atc ttc ttc ttg tgt ttc 884
Ala Leu Thr Thr Ile Ile Ile Thr Leu Ile Ile Phe Phe Leu Cys Phe
245                 250                 255                 260

ctg ccc tat cac aca ctg agg acc gtc cac ttg acg aca tgg aaa gtg 932
Leu Pro Tyr His Thr Leu Arg Thr Val His Leu Thr Thr Trp Lys Val
                265                 270                 275

ggt tta tgc aaa gac aga ctg cat aaa gct ttg gtt atc aca ctg gcc 980
Gly Leu Cys Lys Asp Arg Leu His Lys Ala Leu Val Ile Thr Leu Ala
            280                 285                 290

ttg gca gca gcc aat gcc tgc ttc aat cct ctg ctc tat tac ttt gct 1028
Leu Ala Ala Ala Asn Ala Cys Phe Asn Pro Leu Leu Tyr Tyr Phe Ala
        295                 300                 305

ggg gag aat ttt aag gac aga cta aag tct gca ctc aga aaa ggc cat 1076
Gly Glu Asn Phe Lys Asp Arg Leu Lys Ser Ala Leu Arg Lys Gly His
    310                 315                 320

cca cag aag gca aag aca aag tgt gtt ttc cct gtt agt gtg tgg ttg 1124
Pro Gln Lys Ala Lys Thr Lys Cys Val Phe Pro Val Ser Val Trp Leu
325                 330                 335                 340

aga aag gaa aca aga gta taaggagctc ttagatgaga cctgttcttg 1172
Arg Lys Glu Thr Arg Val
                345

tatccttgtg tccatcttca ttcactcata gtctccaaat gactttgtat ttacatcact 1232

cccaacaaat gttgattctt aatattta 1260

<210> 24
<211> 346
<212> PRT
<213> Homo sapiens

<400> 24
Met Glu Arg Lys Phe Met Ser Leu Gln Pro Ser Ile Ser Val Ser Glu
1               5                   10                  15

Met Glu Pro Asn Gly Thr Phe Ser Asn Asn Asn Ser Arg Asn Cys Thr
            20                  25                  30

Ile Glu Asn Phe Lys Arg Glu Phe Phe Pro Ile Val Tyr Leu Ile Ile
        35                  40                  45

Phe Phe Trp Gly Val Leu Gly Asn Gly Leu Ser Ile Tyr Val Phe Leu
    50                  55                  60

Gln Pro Tyr Lys Lys Ser Thr Ser Val Asn Val Phe Met Leu Asn Leu
65                  70                  75                  80

Ala Ile Ser Asp Leu Leu Phe Ile Ser Thr Leu Pro Phe Arg Ala Asp
                85                  90                  95

Tyr Tyr Leu Arg Gly Ser Asn Trp Ile Phe Gly Asp Leu Ala Cys Arg
            100                 105                 110

Ile Met Ser Tyr Ser Leu Tyr Val Asn Met Tyr Ser Ser Ile Tyr Phe
        115                 120                 125

Leu Thr Val Leu Ser Val Val Arg Phe Leu Ala Met Val His Pro Phe
    130                 135                 140

Arg Leu Leu His Val Thr Ser Ile Arg Ser Ala Trp Ile Leu Cys Gly
145                 150                 155                 160

Ile Ile Trp Ile Leu Ile Met Ala Ser Ser Ile Met Leu Leu Asp Ser
                165                 170                 175

Gly Ser Glu Gln Asn Gly Ser Val Thr Ser Cys Leu Glu Leu Asn Leu
            180                 185                 190

Tyr Lys Ile Ala Lys Leu Gln Thr Met Asn Tyr Ile Ala Leu Val Val
        195                 200                 205

Gly Cys Leu Leu Pro Phe Phe Thr Leu Ser Ile Cys Tyr Leu Leu Ile
    210                 215                 220

Ile Arg Val Leu Leu Lys Val Glu Val Pro Glu Ser Gly Leu Arg Val
225                 230                 235                 240

Ser His Arg Lys Ala Leu Thr Thr Ile Ile Ile Thr Leu Ile Ile Phe
                245                 250                 255

Phe Leu Cys Phe Leu Pro Tyr His Thr Leu Arg Thr Val His Leu Thr
            260                 265                 270

Thr Trp Lys Val Gly Leu Cys Lys Asp Arg Leu His Lys Ala Leu Val
        275                 280                 285

Ile Thr Leu Ala Leu Ala Ala Ala Asn Ala Cys Phe Asn Pro Leu Leu
    290                 295                 300

Tyr Tyr Phe Ala Gly Glu Asn Phe Lys Asp Arg Leu Lys Ser Ala Leu
305                 310                 315                 320

Arg Lys Gly His Pro Gln Lys Ala Lys Thr Lys Cys Val Phe Pro Val
                325                 330                 335

Ser Val Trp Leu Arg Lys Glu Thr Arg Val
            340                 345

<210> 25
<211> 1358
<212> DNA
<213> Homo sapiens

<220>
<221> CDS
<222> (5)..(1348)

<400> 25
agtg atg gat gtt aac agc agc ggc cac ccg gac ctc tac ggg cgc ctc    49
     Met Asp Val Asn Ser Ser Gly His Pro Asp Leu Tyr Gly Arg Leu
     1               5                   10                  15

tgc tct ttc ctc ctg ccg gag gtg ggg ggc agg ctg ccc gac ctg agc    97
Cys Ser Phe Leu Leu Pro Glu Val Gly Gly Arg Leu Pro Asp Leu Ser
                20                  25                  30

ccc gac ggt ggc gcc gaa ccg gtc gcg gtc tcc ggg acg ccg cat ctg    145
Pro Asp Gly Gly Ala Glu Pro Val Ala Val Ser Gly Thr Pro His Leu
            35                  40                  45

ctg agc gag gtg acg gcc agc ccg gcg ccc acc tgg gac gca acc ccg    193
Leu Ser Glu Val Thr Ala Ser Pro Ala Pro Thr Trp Asp Ala Thr Pro
        50                  55                  60

ggc aat gcc tcc ggc cgc ggg gag caa atc aat caa gaa agg gcc gag    241
Gly Asn Ala Ser Gly Arg Gly Glu Gln Ile Asn Gln Glu Arg Ala Glu
    65                  70                  75

aaa gtt gtg atc ggc tct gtc ctg acg ctc atc tct ctg tct gcg atc    289
Lys Val Val Ile Gly Ser Val Leu Thr Leu Ile Ser Leu Ser Ala Ile
80                  85                  90                  95

gcg ggc aac tgc ctg gtg gta atc tct gtg tgc ttc gtc aag aag ctc    337
Ala Gly Asn Cys Leu Val Val Ile Ser Val Cys Phe Val Lys Lys Leu
                100                 105                 110

cgc cag ccc tcc aac tac ctc atc gtg tcc atg gcg ctg gcc aac ctc    385
Arg Gln Pro Ser Asn Tyr Leu Ile Val Ser Met Ala Leu Ala Asn Leu
            115                 120                 125

tcg gtg gcc atg gcg gtc atg ccc ttc atc agt gtc acc gac ctc atc    433
Ser Val Ala Met Ala Val Met Pro Phe Ile Ser Val Thr Asp Leu Ile
        130                 135                 140

ggg ggc aag tgg atc ttt gga cac ttt ttc tgt aac gtc ttc tcc gtg    481
Gly Gly Lys Trp Ile Phe Gly His Phe Phe Cys Asn Val Phe Ser Val
    145                 150                 155

aat gtc atg tgc tgc acg gcc tgg atc ttg acc ttg tac gtg atc agc    529
Asn Val Met Cys Cys Thr Ala Trp Ile Leu Thr Leu Tyr Val Ile Ser
160                 165                 170                 175

atc gac agg gac ctt ggg atc atg aag cct ctc acg tac cct atg agg    577
Ile Asp Arg Asp Leu Gly Ile Met Lys Pro Leu Thr Tyr Pro Met Arg
                180                 185                 190

cag aag ggg aaa tgc atg acg aag atg att ctt tct gtc tgc ctt ctt    625
Gln Lys Gly Lys Cys Met Thr Lys Met Ile Leu Ser Val Cys Leu Leu
            195                 200                 205

tcc gcc ttt gtc act tta cct acc att ttt ggt cgg gct cag aat gta    673
Ser Ala Phe Val Thr Leu Pro Thr Ile Phe Gly Arg Ala Gln Asn Val
        210                 215                 220

aac gat gat aag gtg tgc ttg gtc aat caa gac ttt ggc tac acg att 721
Asn Asp Asp Lys Val Cys Leu Val Asn Gln Asp Phe Gly Tyr Thr Ile
    225                 230                 235

tac tcc ccg cat ttg gca gca ttt atc ccc atg tgc gtc atg ctt ttc 769
Tyr Ser Pro His Leu Ala Ala Phe Ile Pro Met Cys Val Met Leu Phe
240                 245                 250                 255

atg tac tat cag att tac aag gcc gcc agg aaa agc gcg gcc aaa cac 817
Met Tyr Tyr Gln Ile Tyr Lys Ala Ala Arg Lys Ser Ala Ala Lys His
                260                 265                 270

agg tta cct ggc ttc cct cga gtg gag cca gac agc gta gtc acc ctg 865
Arg Leu Pro Gly Phe Pro Arg Val Glu Pro Asp Ser Val Val Thr Leu
            275                 280                 285

aat ggc aca gtg aag ttc cag gag gtg gaa gag tgt gca aac ctt tcg 913
Asn Gly Thr Val Lys Phe Gln Glu Val Glu Glu Cys Ala Asn Leu Ser
        290                 295                 300

aga ctc ctc aag cat gaa agg aaa aat atc tcc atc ttt aag cgg aaa 961
Arg Leu Leu Lys His Glu Arg Lys Asn Ile Ser Ile Phe Lys Arg Lys
    305                 310                 315

cag aaa gca gcg act acc ttg ggg atc atc gtc tgg gcc tcc acc atg 1009
Gln Lys Ala Ala Thr Thr Leu Gly Ile Ile Val Trp Ala Ser Thr Met
320                 325                 330                 335

tgc tgg ccg ccc ttt ttc ctc ctg aca gcc aga ccc ttc tgt cta tgg 1057
Cys Trp Pro Pro Phe Phe Leu Leu Thr Ala Arg Pro Phe Cys Leu Trp
                340                 345                 350

cac tgc cct tct gtc tat ggc act gcc tgc agc tgc atc cca ctg tgg 1105
His Cys Pro Ser Val Tyr Gly Thr Ala Cys Ser Cys Ile Pro Leu Trp
            355                 360                 365

gtg gag agg ata ttt cca tgg ctg ggc tat gca aac tct ctc att aac 1153
Val Glu Arg Ile Phe Pro Trp Leu Gly Tyr Ala Asn Ser Leu Ile Asn
        370                 375                 380

cct ttt att tat gcc ttc ttc aac tgg gac ctg agg acc acc tat tgc 1201
Pro Phe Ile Tyr Ala Phe Phe Asn Trp Asp Leu Arg Thr Thr Tyr Cys
    385                 390                 395

agc cgg ctc cag tgc cag tac cag aat atc aac cag aca ctc tca gct 1249
Ser Arg Leu Gln Cys Gln Tyr Gln Asn Ile Asn Gln Thr Leu Ser Ala
400                 405                 410                 415

gca ggc atg cat gaa gcc ctg aag ctt gct gag agg cca gag aga cct 1297
Ala Gly Met His Glu Ala Leu Lys Leu Ala Glu Arg Pro Glu Arg Pro
                420                 425                 430

gag ttt gtc cta caa aac tct gac tac tgt aga aaa aaa agt cat gat 1345
Glu Phe Val Leu Gln Asn Ser Asp Tyr Cys Arg Lys Lys Ser His Asp
            435                 440                 445

tca tgactgaaag 1358
Ser

<210> 26
<211> 448
<212> PRT
<213> Homo sapiens

<400> 26
Met Asp Val Asn Ser Ser Gly His Pro Asp Leu Tyr Gly Arg Leu Cys
1               5                   10                  15

Ser Phe Leu Leu Pro Glu Val Gly Gly Arg Leu Pro Asp Leu Ser Pro
            20                  25                  30

Asp Gly Gly Ala Glu Pro Val Ala Val Ser Gly Thr Pro His Leu Leu
        35                  40                  45

Ser Glu Val Thr Ala Ser Pro Ala Pro Thr Trp Asp Ala Thr Pro Gly
    50                  55                  60

Asn Ala Ser Gly Arg Gly Glu Gln Ile Asn Gln Glu Arg Ala Glu Lys
65                  70                  75                  80

Val Val Ile Gly Ser Val Leu Thr Leu Ile Ser Leu Ser Ala Ile Ala
                85                  90                  95

Gly Asn Cys Leu Val Val Ile Ser Val Cys Phe Val Lys Lys Leu Arg
            100                 105                 110

Gln Pro Ser Asn Tyr Leu Ile Val Ser Met Ala Leu Ala Asn Leu Ser
        115                 120                 125

Val Ala Met Ala Val Met Pro Phe Ile Ser Val Thr Asp Leu Ile Gly
    130                 135                 140

Gly Lys Trp Ile Phe Gly His Phe Phe Cys Asn Val Phe Ser Val Asn
145                 150                 155                 160

Val Met Cys Cys Thr Ala Trp Ile Leu Thr Leu Tyr Val Ile Ser Ile
                165                 170                 175

Asp Arg Asp Leu Gly Ile Met Lys Pro Leu Thr Tyr Pro Met Arg Gln
            180                 185                 190

Lys Gly Lys Cys Met Thr Lys Met Ile Leu Ser Val Cys Leu Leu Ser
        195                 200                 205

Ala Phe Val Thr Leu Pro Thr Ile Phe Gly Arg Ala Gln Asn Val Asn
    210                 215                 220

Asp Asp Lys Val Cys Leu Val Asn Gln Asp Phe Gly Tyr Thr Ile Tyr
225                 230                 235                 240

Ser Pro His Leu Ala Ala Phe Ile Pro Met Cys Val Met Leu Phe Met
                245                 250                 255

Tyr Tyr Gln Ile Tyr Lys Ala Ala Arg Lys Ser Ala Ala Lys His Arg
            260                 265                 270

Leu Pro Gly Phe Pro Arg Val Glu Pro Asp Ser Val Val Thr Leu Asn
        275                 280                 285

Gly Thr Val Lys Phe Gln Glu Val Glu Glu Cys Ala Asn Leu Ser Arg

290                 295                 300

Leu Leu Lys His Glu Arg Lys Asn Ile Ser Ile Phe Lys Arg Lys Gln
305                 310                 315                 320

Lys Ala Ala Thr Thr Leu Gly Ile Ile Val Trp Ala Ser Thr Met Cys
                325                 330                 335

Trp Pro Pro Phe Phe Leu Leu Thr Ala Arg Pro Phe Cys Leu Trp His
            340                 345                 350

Cys Pro Ser Val Tyr Gly Thr Ala Cys Ser Cys Ile Pro Leu Trp Val
        355                 360                 365

Glu Arg Ile Phe Pro Trp Leu Gly Tyr Ala Asn Ser Leu Ile Asn Pro
    370                 375                 380

Phe Ile Tyr Ala Phe Phe Asn Trp Asp Leu Arg Thr Thr Tyr Cys Ser
385                 390                 395                 400

Arg Leu Gln Cys Gln Tyr Gln Asn Ile Asn Gln Thr Leu Ser Ala Ala
                405                 410                 415

Gly Met His Glu Ala Leu Lys Leu Ala Glu Arg Pro Glu Arg Pro Glu
            420                 425                 430

Phe Val Leu Gln Asn Ser Asp Tyr Cys Arg Lys Lys Ser His Asp Ser
        435                 440                 445

<210> 27
<211> 1173
<212> DNA
<213> Homo sapiens

<220>
<221> CDS
<222> (55)..(1023)

<400> 27
aatatagccg gtcctgtccc cgtgttaact ggagtgctga agcgctggaa cgaa atg      57
cgg ggc ctc tgg tgc gag aag ggg gtg cag gtg ctg ctg acg acg gtg     105
ggc gcc ttc gcc gcc ttc ggc ctc atg acc atc gcc atc agc act gac     153
tac tgg ctc tac acg ggg caa caa gag cga aaa tct gtc tca aaa aat     201
aaa aga agt aag aag gac ccc ggc ggc ctc acg cac tcg ggc ctc tgg     249
agg atc tgc tgc ctg gaa ggg ttg aaa aga ggc gtc tgc gtg aag atc     297
aat cat ttc ccg gag gac acg gac tac gac cac gac agc gcg gag tat     345
cta ctc cgt acg gtc cgg gcc tcc agc atc ttc ccc atc ctt agc gcc     393
atc ctg ctg ctg ctc ggg ggt gtg tgc gtg gcg gcc tcc cgc gtc tac     441
aag tcc aag agg aac atc att ctg ggc gca ggg atc ctg ttc gtg gca     489
gca ggt ctg agc aac atc atc ggc gtg atc gtg tac atc tcc gcc aac     537
gcg ggc gag ccg ggc cga agc aga gcc aag aaa aac cac tac tcg tac     585
ggc tgg tcc ttc tac ttc ggc ggg ctg tcg ttc atc ctg gcc gag gtg     633
ata ggc gtg ctg gcc gtc aac atc tac atc gag cca gcc gag gcg cac     681
tgc cag tct cgg agc ggg acc gcg ggg ggt cgt ccg gct tcc tca cgc     729
tgc aca acg cct tcc cca agg agg cgg gcg gcg gcg tca cgg tca cgt     777
tca ccc ggc cgc ccg ccc cgc ccg cgc cac gcc acc ccg gcc aac acc     825
aac tcc acg gac atc tcc atg tac acg ctc agc cgc gac ccc tcc aag     873
ggc agc ccc cat tcc aat gcc acc acc ccc acc ccc act agc ctc aag     921
gat agg aaa aac tca ttt gtg tcc ata aaa ata aag gta aaa aag aaa     969
aaa aga aat ata tat ata tat ata tat acg ctc aac agg aaa acc acg     1017
cct gtg taggggcgcg gcgggggagc cgaggggcgt gtccggggcg cgtgcgggcg    1073
cgcgtgcatc gaggctgccg gggtcggggg cgcccccgct ttcccccgtg agcgcgctgg 1133
agactgctgg gcccgcccca cgcccaccct ccccgccccc                      1173

<210> 28
<211> 323
<212> PRT
<213> Homo sapiens

<400> 28


<210> 29
<211> 1192
<212> DNA
<213> Homo sapiens

<220>
<221> CDS
<222> (69)..(1097)

<400> 29
tgctgagggt gcatttatgt ttcagaacca ccgggaggaa ctgggccatt ctaacacccg   60

ttgctacc atg ctg gcc acc cgc ctc tcc aga ccc ctg tca cgg ctc cca   110
         Met Leu Ala Thr Arg Leu Ser Arg Pro Leu Ser Arg Leu Pro
         1               5                   10

gga aaa acc cta agt gcc tgt gat aga gaa aat gga gca agg cgc cca   158
Gly Lys Thr Leu Ser Ala Cys Asp Arg Glu Asn Gly Ala Arg Arg Pro
15                  20                  25                  30

cta ttg ctt ggt tct act tcc ttt atc ccg att ggc cgt cgg act tat   206
Leu Leu Leu Gly Ser Thr Ser Phe Ile Pro Ile Gly Arg Arg Thr Tyr
                35                  40                  45

gcc agt gcg gcg gag ccg gtg agt gga aaa gct gtc ctg gtc aca ggc   254
Ala Ser Ala Ala Glu Pro Val Ser Gly Lys Ala Val Leu Val Thr Gly
            50                  55                  60

tgt gac tct gga ttt ggg ttc tca ttg gcc aag cat ctg cat tca aaa   302
Cys Asp Ser Gly Phe Gly Phe Ser Leu Ala Lys His Leu His Ser Lys
        65                  70                  75

ggc ttc ctt gtg ttt gct ggc tgc ttg atg aag gac aaa ggc cat gat   350
Gly Phe Leu Val Phe Ala Gly Cys Leu Met Lys Asp Lys Gly His Asp
    80                  85                  90

ggg gtc aag gag ctg gac agc cta aac agt gac cga ttg aga acc gtc   398
Gly Val Lys Glu Leu Asp Ser Leu Asn Ser Asp Arg Leu Arg Thr Val
95                  100                 105                 110

cag ctc aat gtc tgc agc agc gaa gag gtg gag aaa gtg gtg gag att   446
Gln Leu Asn Val Cys Ser Ser Glu Glu Val Glu Lys Val Val Glu Ile
                115                 120                 125

gtc cgc tcg agc ctg aag gac cct gag aaa ggt atg tgg ggc ctc gtt    494
Val Arg Ser Ser Leu Lys Asp Pro Glu Lys Gly Met Trp Gly Leu Val
            130                 135                 140

aac aat gcc ggc atc tca acg ttc ggg gag gtg gag ttc acc agc ctg    542
Asn Asn Ala Gly Ile Ser Thr Phe Gly Glu Val Glu Phe Thr Ser Leu
        145                 150                 155

gag acc tac aag cag gtg gca gaa gtg aac ctt tgg ggc aca gtg cgg    590
Glu Thr Tyr Lys Gln Val Ala Glu Val Asn Leu Trp Gly Thr Val Arg
    160                 165                 170

atg acg aaa tcc ttt ctc ccc ctc atc cga agg gcc aaa ggt cgc gtc    638
Met Thr Lys Ser Phe Leu Pro Leu Ile Arg Arg Ala Lys Gly Arg Val
175                 180                 185                 190

gtc aat atc agc agc atg ctg ggc cgc atg gcc aac ccg gcc cgc tcc    686
Val Asn Ile Ser Ser Met Leu Gly Arg Met Ala Asn Pro Ala Arg Ser
                195                 200                 205

ccg tac tgc atc acc aag ttc ggg gta gag gct ttc tcg gac tgc ctg    734
Pro Tyr Cys Ile Thr Lys Phe Gly Val Glu Ala Phe Ser Asp Cys Leu
            210                 215                 220

cgc tat gag atg tac ccc ctg ggc gtg aag gtc agc gtg gtg gag ccc    782
Arg Tyr Glu Met Tyr Pro Leu Gly Val Lys Val Ser Val Val Glu Pro
        225                 230                 235

ggc aac ttc atc gct gcc acc agc ctt tac agc cct gag agc att cag    830
Gly Asn Phe Ile Ala Ala Thr Ser Leu Tyr Ser Pro Glu Ser Ile Gln
    240                 245                 250

gcc atc gcc aag aag atg tgg gag gag ctg cct gag gtc gtg cgc aag    878
Ala Ile Ala Lys Lys Met Trp Glu Glu Leu Pro Glu Val Val Arg Lys
255                 260                 265                 270

gac tac ggc aag aag tac ttt gat gaa aag atc gcc aag atg gag acc    926
Asp Tyr Gly Lys Lys Tyr Phe Asp Glu Lys Ile Ala Lys Met Glu Thr
                275                 280                 285

tac tgc agc agt ggc tcc aca gac acg tcc cct gtc atc gat gct gtc    974
Tyr Cys Ser Ser Gly Ser Thr Asp Thr Ser Pro Val Ile Asp Ala Val
            290                 295                 300

aca cac gcc ctg acc gcc acc acc ccc tac acc cgc tac cac ccc atg    1022
Thr His Ala Leu Thr Ala Thr Thr Pro Tyr Thr Arg Tyr His Pro Met
        305                 310                 315

gac tac tac tgg tgg ctg cga atg cag atc atg acc cac ttg cct gga    1070
Asp Tyr Tyr Trp Trp Leu Arg Met Gln Ile Met Thr His Leu Pro Gly
    320                 325                 330

gcc atc tcc gac atg atc tac atc cgc tgaagagtct cgctgtggcc          1117
Ala Ile Ser Asp Met Ile Tyr Ile Arg
335                 340

tctgtcaggg atccctggtg gaaggggagg ggagggagga acccatatag tcaactcttg 1177

attatccacg tgtgg                                                   1192

<210> 30

<211> 343
<212> PRT
<213> Homo sapiens

<400> 30
Met Leu Ala Thr Arg Leu Ser Arg Pro Leu Ser Arg Leu Pro Gly Lys
1               5                   10                  15

Thr Leu Ser Ala Cys Asp Arg Glu Asn Gly Ala Arg Arg Pro Leu Leu
            20                  25                  30

Leu Gly Ser Thr Ser Phe Ile Pro Ile Gly Arg Arg Thr Tyr Ala Ser
        35                  40                  45

Ala Ala Glu Pro Val Ser Gly Lys Ala Val Leu Val Thr Gly Cys Asp
    50                  55                  60

Ser Gly Phe Gly Phe Ser Leu Ala Lys His Leu His Ser Lys Gly Phe
65                  70                  75                  80

Leu Val Phe Ala Gly Cys Leu Met Lys Asp Lys Gly His Asp Gly Val
                85                  90                  95

Lys Glu Leu Asp Ser Leu Asn Ser Asp Arg Leu Arg Thr Val Gln Leu
            100                 105                 110

Asn Val Cys Ser Ser Glu Glu Val Glu Lys Val Val Glu Ile Val Arg
        115                 120                 125

Ser Ser Leu Lys Asp Pro Glu Lys Gly Met Trp Gly Leu Val Asn Asn
    130                 135                 140

Ala Gly Ile Ser Thr Phe Gly Glu Val Glu Phe Thr Ser Leu Glu Thr
145                 150                 155                 160

Tyr Lys Gln Val Ala Glu Val Asn Leu Trp Gly Thr Val Arg Met Thr
                165                 170                 175

Lys Ser Phe Leu Pro Leu Ile Arg Arg Ala Lys Gly Arg Val Val Asn
            180                 185                 190

Ile Ser Ser Met Leu Gly Arg Met Ala Asn Pro Ala Arg Ser Pro Tyr
        195                 200                 205

Cys Ile Thr Lys Phe Gly Val Glu Ala Phe Ser Asp Cys Leu Arg Tyr
    210                 215                 220

Glu Met Tyr Pro Leu Gly Val Lys Val Ser Val Val Glu Pro Gly Asn
225                 230                 235                 240

Phe Ile Ala Ala Thr Ser Leu Tyr Ser Pro Glu Ser Ile Gln Ala Ile
                245                 250                 255

Ala Lys Lys Met Trp Glu Glu Leu Pro Glu Val Val Arg Lys Asp Tyr
            260                 265                 270

Gly Lys Lys Tyr Phe Asp Glu Lys Ile Ala Lys Met Glu Thr Tyr Cys
        275                 280                 285

Ser Ser Gly Ser Thr Asp Thr Ser Pro Val Ile Asp Ala Val Thr His
    290                 295                 300

Ala Leu Thr Ala Thr Thr Pro Tyr Thr Arg Tyr His Pro Met Asp Tyr
305                 310                 315                 320

Tyr Trp Trp Leu Arg Met Gln Ile Met Thr His Leu Pro Gly Ala Ile
                325                 330                 335

Ser Asp Met Ile Tyr Ile Arg
            340

<210> 31
<211> 1166
<212> DNA
<213> Homo sapiens

<220>
<221> CDS
<222> (69)..(1097)

<400> 31
tgctgagggt gcatttatgt ttcagaacca ccgggaggaa ctgggccatt ctaacacccg  60

ttgctacc atg ctg gcc acc cgc ctc tcc aga ccc ctg tca cgg ctc cca  110
         Met Leu Ala Thr Arg Leu Ser Arg Pro Leu Ser Arg Leu Pro
         1               5                   10

gga aaa acc cta agt gcc tgt gat aga gaa aat gga gca aga cgc cca   158
Gly Lys Thr Leu Ser Ala Cys Asp Arg Glu Asn Gly Ala Arg Arg Pro
15                  20                  25                  30

cta ttg ctt ggt tct act tcc ttt atc ccg att ggc cgt cgg act tat   206
Leu Leu Leu Gly Ser Thr Ser Phe Ile Pro Ile Gly Arg Arg Thr Tyr
                35                  40                  45

gcc agt gcg gcg gag ccg gtt ggc agc aaa gct gtc ctg gtc aca ggc   254
Ala Ser Ala Ala Glu Pro Val Gly Ser Lys Ala Val Leu Val Thr Gly
            50                  55                  60

tgt gac tct gga ttt ggg ttc tca ttg gcc aag cat ctg cat tca aaa   302
Cys Asp Ser Gly Phe Gly Phe Ser Leu Ala Lys His Leu His Ser Lys
        65                  70                  75

ggc ttc ctt gtg ttt gct ggc tgc ttg atg aag gac aaa ggc cat gat   350
Gly Phe Leu Val Phe Ala Gly Cys Leu Met Lys Asp Lys Gly His Asp
    80                  85                  90

ggg gtc aag gag ctg gac agc cta aac agt gac cga ttg aga acc gtc   398
Gly Val Lys Glu Leu Asp Ser Leu Asn Ser Asp Arg Leu Arg Thr Val
95                  100                 105                 110

cag ctc aat gtc tgc agc agc gaa gag gtg gag aaa gtg gtg gag att   446
Gln Leu Asn Val Cys Ser Ser Glu Glu Val Glu Lys Val Val Glu Ile
                115                 120                 125

gtc cgc tcg agc ctg aag gac cct gag aaa ggc atg tgg ggc ctc gtt   494
Val Arg Ser Ser Leu Lys Asp Pro Glu Lys Gly Met Trp Gly Leu Val
            130                 135                 140

aac aat gcc ggc atc tca acg ttc ggg gag gtg gag ttc acc agc ctg   542
Asn Asn Ala Gly Ile Ser Thr Phe Gly Glu Val Glu Phe Thr Ser Leu
        145                 150                 155

gag acc tac aag cag gtg gca gaa gtg aac ctt tgg ggc aca gtg cgg 590
Glu Thr Tyr Lys Gln Val Ala Glu Val Asn Leu Trp Gly Thr Val Arg
    160                 165                 170

atg acg aaa tcc ttt ctc ccc ctc atc cga agg gcc aaa ggc cgc gtc 638
Met Thr Lys Ser Phe Leu Pro Leu Ile Arg Arg Ala Lys Gly Arg Val
175                 180                 185                 190

gtc aat atc agc agc atg ctg ggc cgc atg gcc aac ccg gcc cgc tcc 686
Val Asn Ile Ser Ser Met Leu Gly Arg Met Ala Asn Pro Ala Arg Ser
                195                 200                 205

ccg tac tgc atc acc aag ttc ggg gta gag gct ttc tcg gac tgc ctg 734
Pro Tyr Cys Ile Thr Lys Phe Gly Val Glu Ala Phe Ser Asp Cys Leu
            210                 215                 220

cgc tat gag atg tac ccc ctg ggc gtg aag gtc agc gtg gtg gag ccc 782
Arg Tyr Glu Met Tyr Pro Leu Gly Val Lys Val Ser Val Val Glu Pro
        225                 230                 235

ggc aac ttc atc gct gcc acc agc ctt tac agc cct gag agc att cag 830
Gly Asn Phe Ile Ala Ala Thr Ser Leu Tyr Ser Pro Glu Ser Ile Gln
    240                 245                 250

gcc atc gcc aag aag atg tgg gag gag ctg cct gag gtc gtg cgc aag 878
Ala Ile Ala Lys Lys Met Trp Glu Glu Leu Pro Glu Val Val Arg Lys
255                 260                 265                 270

gac tac ggc aag aag tac ttt gat gaa aag atc gcc aag atg gag acc 926
Asp Tyr Gly Lys Lys Tyr Phe Asp Glu Lys Ile Ala Lys Met Glu Thr
                275                 280                 285

tac tgc agc agt ggc tcc aca gac acg tcc cct gtc atc gat gct gtc 974
Tyr Cys Ser Ser Gly Ser Thr Asp Thr Ser Pro Val Ile Asp Ala Val
            290                 295                 300

aca cac gcc ctg acc gcc acc acc ccc tac acc cgc tac cac ccc atg 1022
Thr His Ala Leu Thr Ala Thr Thr Pro Tyr Thr Arg Tyr His Pro Met
        305                 310                 315

gac tac tac tgg tgg ctg cga atg cag atc atg acc cac ttg cct gga 1070
Asp Tyr Tyr Trp Trp Leu Arg Met Gln Ile Met Thr His Leu Pro Gly
    320                 325                 330

gcc atc tcc gac atg atc tac atc cgc tgaagagtct cgctgtggcc 1117
Ala Ile Ser Asp Met Ile Tyr Ile Arg
335                 340

tctgtcaggg attcctggtg gaaggggagg ggagggagga acccatata 1166

<210> 32
<211> 343
<212> PRT
<213> Homo sapiens

<400> 32
Met Leu Ala Thr Arg Leu Ser Arg Pro Leu Ser Arg Leu Pro Gly Lys
1               5                   10                  15

Thr Leu Ser Ala Cys Asp Arg Glu Asn Gly Ala Arg Arg Pro Leu Leu

            20                  25                  30

Leu Gly Ser Thr Ser Phe Ile Pro Ile Gly Arg Arg Thr Tyr Ala Ser
        35                  40                  45

Ala Ala Glu Pro Val Gly Ser Lys Ala Val Leu Val Thr Gly Cys Asp
    50                  55                  60

Ser Gly Phe Gly Phe Ser Leu Ala Lys His Leu His Ser Lys Gly Phe
65                  70                  75                  80

Leu Val Phe Ala Gly Cys Leu Met Lys Asp Lys Gly His Asp Gly Val
                85                  90                  95

Lys Glu Leu Asp Ser Leu Asn Ser Asp Arg Leu Arg Thr Val Gln Leu
            100                 105                 110

Asn Val Cys Ser Ser Glu Glu Val Glu Lys Val Val Glu Ile Val Arg
        115                 120                 125

Ser Ser Leu Lys Asp Pro Glu Lys Gly Met Trp Gly Leu Val Asn Asn
    130                 135                 140

Ala Gly Ile Ser Thr Phe Gly Glu Val Glu Phe Thr Ser Leu Glu Thr
145                 150                 155                 160

Tyr Lys Gln Val Ala Glu Val Asn Leu Trp Gly Thr Val Arg Met Thr
                165                 170                 175

Lys Ser Phe Leu Pro Leu Ile Arg Arg Ala Lys Gly Arg Val Val Asn
            180                 185                 190

Ile Ser Ser Met Leu Gly Arg Met Ala Asn Pro Ala Arg Ser Pro Tyr
        195                 200                 205

Cys Ile Thr Lys Phe Gly Val Glu Ala Phe Ser Asp Cys Leu Arg Tyr
    210                 215                 220

Glu Met Tyr Pro Leu Gly Val Lys Val Ser Val Val Glu Pro Gly Asn
225                 230                 235                 240

Phe Ile Ala Ala Thr Ser Leu Tyr Ser Pro Glu Ser Ile Gln Ala Ile
                245                 250                 255

Ala Lys Lys Met Trp Glu Glu Leu Pro Glu Val Val Arg Lys Asp Tyr
            260                 265                 270

Gly Lys Lys Tyr Phe Asp Glu Lys Ile Ala Lys Met Glu Thr Tyr Cys
        275                 280                 285

Ser Ser Gly Ser Thr Asp Thr Ser Pro Val Ile Asp Ala Val Thr His
    290                 295                 300

Ala Leu Thr Ala Thr Thr Pro Tyr Thr Arg Tyr His Pro Met Asp Tyr
305                 310                 315                 320

Tyr Trp Trp Leu Arg Met Gln Ile Met Thr His Leu Pro Gly Ala Ile
                325                 330                 335

Ser Asp Met Ile Tyr Ile Arg
            340

<210> 33
<211> 3168
<212> DNA
<213> Homo sapiens

<220>
<221> CDS
<222> (39)..(1610)

<400> 33
cacaagacct ggaattgaca ggactcccaa ctagtaca atg aca gaa gat aag gtc 56

1               5

act ggg acc ctg gtt ttc act gtc atc act gct gtg ctg ggt tcc ttc 104

            10                  15                  20

cag ttt gga tat gac att ggt gtg atc aat gca cct caa cag gta ata 152

        25                  30                  35

ata tct cac tat aga cat gtt ttg ggt gtt cca ctg gat gac cga aaa 200

    40                  45                  50

gct atc aac aac tat gtt atc aac agt aca gat gaa ctg ccc aca atc 248

55                  60                  65                  70

tca tac tca atg aac cca aaa cca acc cct tgg gct gag gaa gag act 296

                75                  80                  85

gtg gca gct gct caa cta atc acc atg ctc tgg tcc ctg tct gta tcc 344

            90                  95                  100

agc ttt gca gtt ggt gga atg act gca tca ttc ttt ggt ggg tgg ctt 392

        105                 110                 115

ggg gac aca ctt gga aga atc aaa gcc atg tta gta gca aac att ctg 440

    120                 125                 130

tca tta gtt gga gct ctc ttg atg ggg ttt tca aaa ttg gga cca tct 488

135                 140                 145                 150

cat ata ctt ata att gct gga aga agc ata tca gga cta tat tgt ggg 536

                155                 160                 165

cta att tca ggc ctg gtt cct atg tat atc ggt gaa att gct cca acc 584

            170                 175                 180

gct ctc agg gga gca ctt ggc act ttt cat cag ctg gcc atc gtc acg 632


185                 190                 195

ggc att ctt att agt cag att att ggt ctt gaa ttt atc ttg ggc aat     680

    200                 205                 210

tat gat ctg tgg cac atc ctg ctt ggc ctg tct ggt gtg cga gcc atc     728

215                 220                 225                 230

ctt cag tct ctg cta ctc ttt ttc tgt cca gaa agc ccc aga tac ctt     776

                235                 240                 245

tac atc aag tta gat gag gaa gtc aaa gca aaa caa agc ttg aaa aga     824

            250                 255                 260

ctc aga gga tat gat gat gtc acc aaa gat att aat gaa atg aga aaa     872

        265                 270                 275

gaa aga gaa gaa gca tcg agt gag cag aaa gtc tct ata att cag ctc     920

    280                 285                 290

ttc acc aat tcc agc tac cga cag cct att cta gtg gca ctg atg ctg     968

295                 300                 305                 310

cat gtg gct cag caa ttt tcc gga atc aat ggc att ttt tac tac tca     1016

                315                 320                 325

acc agc att ttt cag acg gct ggt atc agc aaa cct gtt tat gca acc     1064

            330                 335                 340

att gga gtt ggc gct gta aac atg gtt ttc act gct gtc tct gta ttc     1112

        345                 350                 355

ctt gtg gag aag gca ggg cga cgt tct ctc ttt cta att gga atg agt     1160

    360                 365                 370

ggg atg ttt gtt tgt gcc atc ttc atg tca gtg gga ctt gtg ctg ctg     1208

375                 380                 385                 390

aat aag ttc tct tgg atg agt tat gtg agc atg ata gcc atc ttc ctc     1256

                395                 400                 405

ttt gtc agc ttc ttt gaa att ggg cca ggc ccg atc ccc tgg ttc atg     1304

            410                 415                 420

gtg gct gag ttt ttc agt caa gga cca cgt cct gct gct tta gca ata     1352

        425                 430                 435

gct gca ttc agc aat tgg acc tgc aat ttc att gta gct ctg tgt ttc 1400

    440                 445                 450

cag tac att gcg gac ttc tgt gga cct tat gtg ttt ttc ctc ttt gct 1448

455                 460                 465                 470

gga gtg ctc ctg gcc ttt acc ctg ttc aca ttt ttt aaa gtt cca gaa 1496

                475                 480                 485

acc aaa gga aag tct ttt gag gaa att gct gca gaa ttc caa aag aag 1544

            490                 495                 500

agt ggc tca gcc cac agg cca aaa gct gct gta gaa atg aaa ttc cta 1592

        505                 510                 515

gga gct aca gag act gtg taaaaaaaa accctgcttt ttgacatgaa 1640

    520

cagaaacaat aagggaaccg tctgttttta aatgatgatt ccttgagcat tttatatcca 1700

catctttaag tattgtttta tttttatgtg ctctcatcag aaatgtcatc aaatattacc 1760

aaaaaagtat ttttttaagt tagagaatat atttttgatg gtaagactgt aattaagtaa 1820

accaaaaagg ctagtttatt ttgttacact aaagggcagg tggttctaat atttttagct 1880

ctgttcttta taacaaggtt cttctaaaat tgaagagatt tcaacatatc atttttttaa 1940

cacataacta gaaacctgag gatgcaacaa atatttatat atttgaatat cattaaattg 2000

gaattttctt acccatatat cttatgttaa aggagatatg gctagtggca ataagttcca 2060

tgttaaaata gacaactctt ccatttattg cactcagctt ttttcttgag tactagaatt 2120

tgtattttgc ttaaaatttt acttttgttc tgtattttca tgtggaatgg attatagagt 2180

atactaaaaa atgtctatag agaaaaactt tcatttttgg taggcttatc aaaatctttc 2240

agcactcaga aaagaaaacc attttagttc ctttatttaa tggccaaatg gtttttgcaa 2300

gatttaacac taaaaaggtt tcacctgatc atatagcgtg ggttatcagt taacattaac 2360

atctattata aaaccatgtt gattcccttc tggtacaatc ctttgagtta tagtttgctt 2420

tgctttttaa ttgaggacag cctggttttc acatacactc aaacaatcat gagtcagaca 2480

tttggtatat tacctcaaat tcctaataag tttgatcaaa tctaatgtaa gaaaatttga 2540

agtaaaggat tgatcacttt gttaaaaata ttttctgaat tattatgtct caaaataagt 2600

tgaaaaggta gggtttgagg attcctgagt gtgggcttct gaaacttcat aaatgttcag 2660

cttcagactt ttatcaaaat ccctatttaa ttttcctgga aagactgatt gttttatggt 2720

gtgttcctaa cataaaataa tcgtctcctt tgacatttcc ttctttgtct tagctgtata 2780

cagattctag ccaaactatt ctatggccat tactaacacg cattgtacac tatctatctg 2840

cctttaccta cataggcaaa ttggaaatac acagatgatt aaacagactt tagcttacag 2900

tcaattttac aattatggaa atatagttct gatgggtccc aaaagcttag cagggtgcta 2960

acgtatctct aggctgtttt ctccaccaac tggagcactg atcaatcctt cttatgtttg 3020

ctttaatgtg tattgaagaa aagcactttt taaaaagtac tctttaagag tgaaataatt 3080

aaaaaccact gaacatttgc tttgttttct aaagttgttc acatatatgt aatttagcag 3140

tccaaagaac aagaaattgt ttcttttc 3168

<210> 34
<211> 524
<212> PRT
<213> Homo sapiens

<400> 34

<210> 35

<211> 1587
<212> DNA
<213> Homo sapiens

<220>
<221> CDS
<222> (1)..(1587)

<400> 35
gga tcc acc atg aca gaa gat aag gtc act ggg acc ctg gtt ttc act     48
Gly Ser Thr Met Thr Glu Asp Lys Val Thr Gly Thr Leu Val Phe Thr
1               5                   10                  15

gtc atc act gct gtg ctg ggt tcc ttc cag ttt gga tat gac att ggt     96
Val Ile Thr Ala Val Leu Gly Ser Phe Gln Phe Gly Tyr Asp Ile Gly
            20                  25                  30

gtg atc aat gca cct caa cag gta ata ata tct cac tat aga cat gtt     144
Val Ile Asn Ala Pro Gln Gln Val Ile Ile Ser His Tyr Arg His Val
        35                  40                  45

ttg ggt gtt cca ctg gat gac cga aaa gct atc aac aac tat gtt atc     192
Leu Gly Val Pro Leu Asp Asp Arg Lys Ala Ile Asn Asn Tyr Val Ile
    50                  55                  60

aac agt aca gat gaa ctg ccc aca atc tca tac tca atg aac cca aaa     240
Asn Ser Thr Asp Glu Leu Pro Thr Ile Ser Tyr Ser Met Asn Pro Lys
65                  70                  75                  80

cca acc cct tgg gct gag gaa gag act gtg gca gct gct caa cta atc     288
Pro Thr Pro Trp Ala Glu Glu Glu Thr Val Ala Ala Ala Gln Leu Ile
                85                  90                  95

acc atg ctc tgg tcc ctg tct gta tcc agc ttt gca gtt ggt gga atg     336
Thr Met Leu Trp Ser Leu Ser Val Ser Ser Phe Ala Val Gly Gly Met
            100                 105                 110

att gca tca ttc ttt ggt ggg tgg ctt ggg gac aca ctt gga aga atc     384
Ile Ala Ser Phe Phe Gly Gly Trp Leu Gly Asp Thr Leu Gly Arg Ile
        115                 120                 125

aaa gcc atg tta gta gca aac att ctg tca tta gtt gga gct ctc ttg     432
Lys Ala Met Leu Val Ala Asn Ile Leu Ser Leu Val Gly Ala Leu Leu
    130                 135                 140

atg ggg ttt tca aaa ttg gga cca tct cat ata ctt ata att gct gga     480
Met Gly Phe Ser Lys Leu Gly Pro Ser His Ile Leu Ile Ile Ala Gly
145                 150                 155                 160

aga agc ata tca gga cta tat tgt ggg cta att tca ggc ctg gtt cct     528
Arg Ser Ile Ser Gly Leu Tyr Cys Gly Leu Ile Ser Gly Leu Val Pro
                165                 170                 175

atg tat atc ggt gaa att gct cca acc gct ctc agg gga gca ctt ggc     576
Met Tyr Ile Gly Glu Ile Ala Pro Thr Ala Leu Arg Gly Ala Leu Gly
            180                 185                 190

act ttt cat cag ctg gcc atc gtc acg ggc att ctt att agt cag att     624
Thr Phe His Gln Leu Ala Ile Val Thr Gly Ile Leu Ile Ser Gln Ile
        195                 200                 205

att ggt ctt gaa ttt atc ttg ggc aat tat gat ctg tgg cac atc ctg 672
Ile Gly Leu Glu Phe Ile Leu Gly Asn Tyr Asp Leu Trp His Ile Leu
    210                 215                 220

ctt ggc ctg tct ggt gtg cga gcc atc ctt cag tct ctg cta ctc ttt 720
Leu Gly Leu Ser Gly Val Arg Ala Ile Leu Gln Ser Leu Leu Leu Phe
225                 230                 235                 240

ttc tgt cca gaa agc ccc aga tac ctt tac atc aag tta gat gag gaa 768
Phe Cys Pro Glu Ser Pro Arg Tyr Leu Tyr Ile Lys Leu Asp Glu Glu
                245                 250                 255

gtc aaa gca aaa caa atc ttg aaa aga ctc aga gga tat gat gat gtc 816
Val Lys Ala Lys Gln Ile Leu Lys Arg Leu Arg Gly Tyr Asp Asp Val
            260                 265                 270

acc aaa gat att aat gaa atg aga aaa gaa aga gaa gaa gca tcg agt 864
Thr Lys Asp Ile Asn Glu Met Arg Lys Glu Arg Glu Glu Ala Ser Ser
        275                 280                 285

gag cag aaa gtc tct ata att cag ctc ttc acc aat tcc agc tac cga 912
Glu Gln Lys Val Ser Ile Ile Gln Leu Phe Thr Asn Ser Ser Tyr Arg
    290                 295                 300

cag cct att cta gtg gca ctg atg ctg cat gtg gct cag caa ttt tcc 960
Gln Pro Ile Leu Val Ala Leu Met Leu His Val Ala Gln Gln Phe Ser
305                 310                 315                 320

gga atc aat ggc att ttt tac tac tca acc agc att ttt cag acg gct 1008
Gly Ile Asn Gly Ile Phe Tyr Tyr Ser Thr Ser Ile Phe Gln Thr Ala
                325                 330                 335

ggt atc agc aaa cct gtt tat gca acc att gga gtt ggc gct gta aac 1056
Gly Ile Ser Lys Pro Val Tyr Ala Thr Ile Gly Val Gly Ala Val Asn
            340                 345                 350

atg gtt ttc act gct gtc tct gta ttc ctt gtg gag aag gca ggg cga 1104
Met Val Phe Thr Ala Val Ser Val Phe Leu Val Glu Lys Ala Gly Arg
        355                 360                 365

cgt tct ctc ttt cta att gga atg agt ggg atg ttt gtt tgt gcc atc 1152
Arg Ser Leu Phe Leu Ile Gly Met Ser Gly Met Phe Val Cys Ala Ile
    370                 375                 380

ttc atg tca gtg gga ctt gtg ctg ctg aat aag ttc tct tgg atg agt 1200
Phe Met Ser Val Gly Leu Val Leu Leu Asn Lys Phe Ser Trp Met Ser
385                 390                 395                 400

tat gtg agc atg ata gcc atc ttc ctc ttt gtc agc ttc ttt gaa att 1248
Tyr Val Ser Met Ile Ala Ile Phe Leu Phe Val Ser Phe Phe Glu Ile
                405                 410                 415

ggg cca ggc ccg atc ccc tgg ttc atg gtg gct gag ttt ttc agt caa 1296
Gly Pro Gly Pro Ile Pro Trp Phe Met Val Ala Glu Phe Phe Ser Gln
            420                 425                 430

gga cca cgt cct gct gct tta gca ata gct gca ttc agc aat tgg acc 1344
Gly Pro Arg Pro Ala Ala Leu Ala Ile Ala Ala Phe Ser Asn Trp Thr
        435                 440                 445

tgc aat ttc att gta gct ctg tgt ttc cag tac att gcg gac ttc tgt 1392

Cys Asn Phe Ile Val Ala Leu Cys Phe Gln Tyr Ile Ala Asp Phe Cys
    450                 455                 460

gga cct tat gtg ttt ttc ctc ttt gct gga gtg ctc ctg gcc ttt acc    1440
Gly Pro Tyr Val Phe Phe Leu Phe Ala Gly Val Leu Leu Ala Phe Thr
465                 470                 475                 480

ctg ttt aca ttt ttt aaa gtt cca gaa acc aaa gga aag tct ttt gag    1488
Leu Phe Thr Phe Phe Lys Val Pro Glu Thr Lys Gly Lys Ser Phe Glu
                485                 490                 495

gaa att gct gca gaa ttc caa aag aag agt ggc tca gcc cac agg cca    1536
Glu Ile Ala Ala Glu Phe Gln Lys Lys Ser Gly Ser Ala His Arg Pro
            500                 505                 510

aaa gct gct gta gaa atg aaa ttc cta gga gct aca gag act gtg ctc    1584
Lys Ala Ala Val Glu Met Lys Phe Leu Gly Ala Thr Glu Thr Val Leu
        515                 520                 525

gag                                                                1587
Glu

<210> 36
<211> 529
<212> PRT
<213> Homo sapiens

<400> 36
Gly Ser Thr Met Thr Glu Asp Lys Val Thr Gly Thr Leu Val Phe Thr
1               5                   10                  15

Val Ile Thr Ala Val Leu Gly Ser Phe Gln Phe Gly Tyr Asp Ile Gly
            20                  25                  30

Val Ile Asn Ala Pro Gln Gln Val Ile Ile Ser His Tyr Arg His Val
        35                  40                  45

Leu Gly Val Pro Leu Asp Asp Arg Lys Ala Ile Asn Asn Tyr Val Ile
    50                  55                  60

Asn Ser Thr Asp Glu Leu Pro Thr Ile Ser Tyr Ser Met Asn Pro Lys
65                  70                  75                  80

Pro Thr Pro Trp Ala Glu Glu Glu Thr Val Ala Ala Ala Gln Leu Ile
                85                  90                  95

Thr Met Leu Trp Ser Leu Ser Val Ser Ser Phe Ala Val Gly Gly Met
            100                 105                 110

Ile Ala Ser Phe Phe Gly Gly Trp Leu Gly Asp Thr Leu Gly Arg Ile
        115                 120                 125

Lys Ala Met Leu Val Ala Asn Ile Leu Ser Leu Val Gly Ala Leu Leu
    130                 135                 140

Met Gly Phe Ser Lys Leu Gly Pro Ser His Ile Leu Ile Ile Ala Gly
145                 150                 155                 160

Arg Ser Ile Ser Gly Leu Tyr Cys Gly Leu Ile Ser Gly Leu Val Pro
                165                 170                 175

Met Tyr Ile Gly Glu Ile Ala Pro Thr Ala Leu Arg Gly Ala Leu Gly
            180                 185                 190

Thr Phe His Gln Leu Ala Ile Val Thr Gly Ile Leu Ile Ser Gln Ile
        195                 200                 205

Ile Gly Leu Glu Phe Ile Leu Gly Asn Tyr Asp Leu Trp His Ile Leu
    210                 215                 220

Leu Gly Leu Ser Gly Val Arg Ala Ile Leu Gln Ser Leu Leu Leu Phe
225                 230                 235                 240

Phe Cys Pro Glu Ser Pro Arg Tyr Leu Tyr Ile Lys Leu Asp Glu Glu
                245                 250                 255

Val Lys Ala Lys Gln Ile Leu Lys Arg Leu Arg Gly Tyr Asp Asp Val
            260                 265                 270

Thr Lys Asp Ile Asn Glu Met Arg Lys Glu Arg Glu Glu Ala Ser Ser
        275                 280                 285

Glu Gln Lys Val Ser Ile Ile Gln Leu Phe Thr Asn Ser Ser Tyr Arg
    290                 295                 300

Gln Pro Ile Leu Val Ala Leu Met Leu His Val Ala Gln Gln Phe Ser
305                 310                 315                 320

Gly Ile Asn Gly Ile Phe Tyr Tyr Ser Thr Ser Ile Phe Gln Thr Ala
                325                 330                 335

Gly Ile Ser Lys Pro Val Tyr Ala Thr Ile Gly Val Gly Ala Val Asn
            340                 345                 350

Met Val Phe Thr Ala Val Ser Val Phe Leu Val Glu Lys Ala Gly Arg
        355                 360                 365

Arg Ser Leu Phe Leu Ile Gly Met Ser Gly Met Phe Val Cys Ala Ile
    370                 375                 380

Phe Met Ser Val Gly Leu Val Leu Leu Asn Lys Phe Ser Trp Met Ser
385                 390                 395                 400

Tyr Val Ser Met Ile Ala Ile Phe Leu Phe Val Ser Phe Phe Glu Ile
                405                 410                 415

Gly Pro Gly Pro Ile Pro Trp Phe Met Val Ala Glu Phe Phe Ser Gln
            420                 425                 430

Gly Pro Arg Pro Ala Ala Leu Ala Ile Ala Ala Phe Ser Asn Trp Thr
        435                 440                 445

Cys Asn Phe Ile Val Ala Leu Cys Phe Gln Tyr Ile Ala Asp Phe Cys
    450                 455                 460

Gly Pro Tyr Val Phe Phe Leu Phe Ala Gly Val Leu Leu Ala Phe Thr
465                 470                 475                 480

Leu Phe Thr Phe Phe Lys Val Pro Glu Thr Lys Gly Lys Ser Phe Glu
                485                 490                 495

Glu Ile Ala Ala Glu Phe Gln Lys Lys Ser Gly Ser Ala His Arg Pro
            500                 505                 510

Lys Ala Ala Val Glu Met Lys Phe Leu Gly Ala Thr Glu Thr Val Leu
        515                 520                 525

Glu

<210> 37
<211> 1587
<212> DNA
<213> Homo sapiens

<220>
<221> CDS
<222> (1)..(1587)

<400> 37
   gga tcc acc atg aca gaa gat aag gtc act ggg acc ctg gtt ttc act      48
   Gly Ser Thr Met Thr Glu Asp Lys Val Thr Gly Thr Leu Val Phe Thr
   1               5                   10                  15
   gtc atc act gct gtg ctg ggt tcc ttc cag ttt gga tat gac att ggt      96
   Val Ile Thr Ala Val Leu Gly Ser Phe Gln Phe Gly Tyr Asp Ile Gly
               20                  25                  30
   gtg atc aat gca cct caa cag gta ata ata tct cac tat aga cat gtt     144
   Val Ile Asn Ala Pro Gln Gln Val Ile Ile Ser His Tyr Arg His Val
           35                  40                  45
   ttg ggt gtt cca ctg gat gac cga aaa gct atc aac aac tat gtt atc     192
   Leu Gly Val Pro Leu Asp Asp Arg Lys Ala Ile Asn Asn Tyr Val Ile
       50                  55                  60
   aac agt aca gat gaa ctg ccc aca atc tca tac tca atg aac cca aaa     240
   Asn Ser Thr Asp Glu Leu Pro Thr Ile Ser Tyr Ser Met Asn Pro Lys
   65                  70                  75                  80
   cca acc cct tgg gct gag gaa gag act gtg gca gct gct caa cta atc     288
   Pro Thr Pro Trp Ala Glu Glu Glu Thr Val Ala Ala Ala Gln Leu Ile
                   85                  90                  95
   acc atg ctc tgg tcc ctg tct gta tcc agc ttt gca gtt ggt gga atg     336
   Thr Met Leu Trp Ser Leu Ser Val Ser Ser Phe Ala Val Gly Gly Met
               100                 105                 110
   att gca tca ttc ttt ggt ggg tgg ctt ggg gac aca ctt gga aga atc     384
   Ile Ala Ser Phe Phe Gly Gly Trp Leu Gly Asp Thr Leu Gly Arg Ile
           115                 120                 125
   aaa gcc atg tta gta gca aac att ctg tca tta gtt gga gct ctc ttg     432
   Lys Ala Met Leu Val Ala Asn Ile Leu Ser Leu Val Gly Ala Leu Leu
       130                 135                 140
   atg ggg ttt tca aaa ttg gga cca tct cat ata ctt ata att gct gga     480
   Met Gly Phe Ser Lys Leu Gly Pro Ser His Ile Leu Ile Ile Ala Gly
   145                 150                 155                 160
   aga agc ata tca gga cta tat tgt ggg cta att tca ggc ctg gtt cct     528
   Arg Ser Ile Ser Gly Leu Tyr Cys Gly Leu Ile Ser Gly Leu Val Pro
   165 170 175
   atg tat atc ggt gaa att gct cca acc gct ctc agg gga gca ctt ggc 576
   Met Tyr Ile Gly Glu Ile Ala Pro Thr Ala Leu Arg Gly Ala Leu Gly
   180 185 190
   act ttt cat cag ctg gcc atc gtc acg ggc att ctt att agt cag att 624
   Thr Phe His Gln Leu Ala Ile Val Thr Gly Ile Leu Ile Ser Gln Ile
   195 200 205
   att ggt ctt gaa ttt atc ttg ggc aat tat gat ctg tgg cac atc ctg 672
   Ile Gly Leu Glu Phe Ile Leu Gly Asn Tyr Asp Leu Trp His Ile Leu
   210 215 220
   ctt ggc ctg tct ggt gtg cga gcc atc ctt cag tct ctg cta ctc ttt 720
   Leu Gly Leu Ser Gly Val Arg Ala Ile Leu Gln Ser Leu Leu Leu Phe
   225 230 235 240
   ttc tgt cca gaa agc ccc aga tac ctt tac atc aag tta gat gag gaa 768
   Phe Cys Pro Glu Ser Pro Arg Tyr Leu Tyr Ile Lys Leu Asp Glu Glu
   245 250 255
   gtc aaa gca aaa caa agc ttg aaa aga ctc aga gga tat gat gat gtc 816
   Val Lys Ala Lys Gln Ser Leu Lys Arg Leu Arg Gly Tyr Asp Asp Val
   260 265 270
   acc aaa gat att aat gaa atg aga aaa gaa aga gaa gaa gca tcg agt 864
   Thr Lys Asp Ile Asn Glu Met Arg Lys Glu Arg Glu Glu Ala Ser Ser
   275 280 285
   gag cag aaa gtc tct ata att cag ctc ttc acc aat tcc agc tac cga 912
   Glu Gln Lys Val Ser Ile Ile Gln Leu Phe Thr Asn Ser Ser Tyr Arg
   290 295 300
   cag cct att cta gtg gca ctg atg ctg cat gtg gct cag caa ttt tcc 960
   Gln Pro Ile Leu Val Ala Leu Met Leu His Val Ala Gln Gln Phe Ser
   305 310 315 320
   gga atc aat ggc att ttt tac tac tca acc agc att ttt cag acg gct 1008
   Gly Ile Asn Gly Ile Phe Tyr Tyr Ser Thr Ser Ile Phe Gln Thr Ala
   325 330 335
   ggt atc agc aaa cct gtt tat gca acc att gga gtt ggc gct gta aac 1056
   Gly Ile Ser Lys Pro Val Tyr Ala Thr Ile Gly Val Gly Ala Val Asn
   340 345 350
   atg gtt ttc act gct gtc tct gta ttc ctt gtg gag aag gca ggg cga 1104
   Met Val Phe Thr Ala Val Ser Val Phe Leu Val Glu Lys Ala Gly Arg
   355 360 365
   cgt tct ctc ttt cta att gga atg agt ggg atg ttt gtt tgt gcc atc 1152
   Arg Ser Leu Phe Leu Ile Gly Met Ser Gly Met Phe Val Cys Ala Ile
   370 375 380
   ttc atg tca gtg gga ctt gtg ctg ctg aat aag ttc tct tgg atg agt 1200
   Phe Met Ser Val Gly Leu Val Leu Leu Asn Lys Phe Ser Trp Met Ser
   385 390 395 400
   tat gtg agc atg ata gcc atc ttc ctc ttt gtc agc ttc ttt gaa att 1248
   Tyr Val Ser Met Ile Ala Ile Phe Leu Phe Val Ser Phe Phe Glu Ile
   405 410 415
   gga cca ggc ccg atc ccc tgg ttc atg gtg gct gag ttt ttc agt caa    1296
   Gly Pro Gly Pro Ile Pro Trp Phe Met Val Ala Glu Phe Phe Ser Gln
               420                 425                 430
   gga cca cgt cct gct gct tta gca ata gct gca ttc agc aat tgg acc     1344
   Gly Pro Arg Pro Ala Ala Leu Ala Ile Ala Ala Phe Ser Asn Trp Thr
           435                 440                 445
   tgc aat ttc att gta gct ctg tgt ttc cag tac att gcg gac ttc tgt     1392
   Cys Asn Phe Ile Val Ala Leu Cys Phe Gln Tyr Ile Ala Asp Phe Cys
       450                 455                 460
   gga cct tat gtg ttt ttc ctc ttt gct gga gtg ctc ctg gcc ttt acc     1440
   Gly Pro Tyr Val Phe Phe Leu Phe Ala Gly Val Leu Leu Ala Phe Thr
   465                 470                 475                 480
   ctg ttt aca ttt ttt aaa gtt cca gaa acc aaa gga aag tct ttt gag     1488
   Leu Phe Thr Phe Phe Lys Val Pro Glu Thr Lys Gly Lys Ser Phe Glu
                   485                 490                 495
   gaa att gct gca gaa ttc caa aag aag agt ggc tca gcc cac agg cca     1536
   Glu Ile Ala Ala Glu Phe Gln Lys Lys Ser Gly Ser Ala His Arg Pro
               500                 505                 510
   aaa gct gct gta gaa atg aaa ttc cta gga gct aca gag act gtg ctc     1584
   Lys Ala Ala Val Glu Met Lys Phe Leu Gly Ala Thr Glu Thr Val Leu
           515                 520                 525
   gag                                                                 1587
   Glu


<210> 38
<211> 529
<212> PRT
<213> Homo sapiens

<400> 38
Gly Ser Thr Met Thr Glu Asp Lys Val Thr Gly Thr Leu Val Phe Thr
1               5                   10                  15

Val Ile Thr Ala Val Leu Gly Ser Phe Gln Phe Gly Tyr Asp Ile Gly
            20                  25                  30

Val Ile Asn Ala Pro Gln Gln Val Ile Ile Ser His Tyr Arg His Val
        35                  40                  45

Leu Gly Val Pro Leu Asp Asp Arg Lys Ala Ile Asn Asn Tyr Val Ile
    50                  55                  60

Asn Ser Thr Asp Glu Leu Pro Thr Ile Ser Tyr Ser Met Asn Pro Lys
65                  70                  75                  80

Pro Thr Pro Trp Ala Glu Glu Glu Thr Val Ala Ala Ala Gln Leu Ile
                85                  90                  95

Thr Met Leu Trp Ser Leu Ser Val Ser Ser Phe Ala Val Gly Gly Met
            100                 105                 110

Ile Ala Ser Phe Phe Gly Gly Trp Leu Gly Asp Thr Leu Gly Arg Ile

115                 120                 125

Lys Ala Met Leu Val Ala Asn Ile Leu Ser Leu Val Gly Ala Leu Leu
    130                 135                 140

Met Gly Phe Ser Lys Leu Gly Pro Ser His Ile Leu Ile Ile Ala Gly
145                 150                 155                 160

Arg Ser Ile Ser Gly Leu Tyr Cys Gly Leu Ile Ser Gly Leu Val Pro
                165                 170                 175

Met Tyr Ile Gly Glu Ile Ala Pro Thr Ala Leu Arg Gly Ala Leu Gly
            180                 185                 190

Thr Phe His Gln Leu Ala Ile Val Thr Gly Ile Leu Ile Ser Gln Ile
        195                 200                 205

Ile Gly Leu Glu Phe Ile Leu Gly Asn Tyr Asp Leu Trp His Ile Leu
    210                 215                 220

Leu Gly Leu Ser Gly Val Arg Ala Ile Leu Gln Ser Leu Leu Leu Phe
225                 230                 235                 240

Phe Cys Pro Glu Ser Pro Arg Tyr Leu Tyr Ile Lys Leu Asp Glu Glu
                245                 250                 255

Val Lys Ala Lys Gln Ser Leu Lys Arg Leu Arg Gly Tyr Asp Asp Val
            260                 265                 270

Thr Lys Asp Ile Asn Glu Met Arg Lys Glu Arg Glu Glu Ala Ser Ser
        275                 280                 285

Glu Gln Lys Val Ser Ile Ile Gln Leu Phe Thr Asn Ser Ser Tyr Arg
    290                 295                 300

Gln Pro Ile Leu Val Ala Leu Met Leu His Val Ala Gln Gln Phe Ser
305                 310                 315                 320

Gly Ile Asn Gly Ile Phe Tyr Tyr Ser Thr Ser Ile Phe Gln Thr Ala
                325                 330                 335

Gly Ile Ser Lys Pro Val Tyr Ala Thr Ile Gly Val Gly Ala Val Asn
            340                 345                 350

Met Val Phe Thr Ala Val Ser Val Phe Leu Val Glu Lys Ala Gly Arg
        355                 360                 365

Arg Ser Leu Phe Leu Ile Gly Met Ser Gly Met Phe Val Cys Ala Ile
    370                 375                 380

Phe Met Ser Val Gly Leu Val Leu Leu Asn Lys Phe Ser Trp Met Ser
385                 390                 395                 400

Tyr Val Ser Met Ile Ala Ile Phe Leu Phe Val Ser Phe Phe Glu Ile
                405                 410                 415

Gly Pro Gly Pro Ile Pro Trp Phe Met Val Ala Glu Phe Phe Ser Gln
            420                 425                 430

Gly Pro Arg Pro Ala Ala Leu Ala Ile Ala Ala Phe Ser Asn Trp Thr
        435                 440                 445

Cys Asn Phe Ile Val Ala Leu Cys Phe Gln Tyr Ile Ala Asp Phe Cys
    450                 455                 460

Gly Pro Tyr Val Phe Phe Leu Phe Ala Gly Val Leu Leu Ala Phe Thr
465                 470                 475                 480

Leu Phe Thr Phe Phe Lys Val Pro Glu Thr Lys Gly Lys Ser Phe Glu
                485                 490                 495

Glu Ile Ala Ala Glu Phe Gln Lys Lys Ser Gly Ser Ala His Arg Pro
            500                 505                 510

Lys Ala Ala Val Glu Met Lys Phe Leu Gly Ala Thr Glu Thr Val Leu
        515                 520                 525

Glu

<210> 39
<211> 1664
<212> DNA
<213> Homo sapiens

<220>
<221> CDS
<222> (39)..(1610)

<400> 39
cacaagacct ggaattgaca ggactcccaa ctagtaca atg aca gaa gat aag gtc   56
                                         
                                         1               5

act ggg acc ctg gtt ttc act gtc atc act gct gtg ctg ggt tcc ttc   104

            10                  15                  20

cag ttt gga tat gac att ggt gtg atc aat gca cct caa cag gta ata   152

        25                  30                  35

ata tct cac tat aga cat gtt ttg ggt gtt cca ctg gat gac cga aaa   200

    40                  45                  50

gct atc aac aac tat gtt atc aac agt aca gat gaa ctg ccc aca atc   248

55                  60                  65                  70

tca tac tca atg aac cca aaa cca acc cct tgg gct gag gaa gag act   296

                75                  80                  85

gtg gca gct gct caa cta atc acc atg ctc tgg tcc ctg tct gta tcc   344

            90                  95                  100

agc ttt gca gtt ggt gga atg act gca tca ttc ttt ggt ggg tgg ctt   392

        105                 110                 115

gggg gac aca ctt gga aga atc aaa gcc atg tta gta gca aac att ctg     440

    120                 125                 130

tca tta gtt gga gct ctc ttg atg ggg ttt tca aaa ttg gga cca tct     488

135                 140                 145                 150

cat ata ctt ata att gct gga aga agc ata tca gga cta tat tgt ggg     536

                155                 160                 165

cta att tca ggc ctg gtt cct atg tat atc ggt gaa att gct cca acc     584

            170                 175                 180

gct ctc agg gga gca ctt ggc act ttt cat cag ctg gcc atc gtc acg     632

        185                 190                 195

ggc att ctt att agt cag att att ggt ctt gaa ttt atc ttg ggc aat     680

    200                 205                 210

tat gat ctg tgg cac atc ctg ctt ggc ctg tct ggt gtg cga gcc atc     728

215                 220                 225                 230

ctt cag tct ctg cta ctc ttt ttc tgt cca gaa agc ccc aga tac ctt     776

                235                 240                 245

tac atc aag tta gat gag gaa gtc aaa gca aaa caa agc ttg aaa aga     824

            250                 255                 260

ctc aga gga tat gat gat gtc acc aaa gat att aat gaa atg aga aaa     872

        265                 270                 275

gaa aga gaa gaa gca tcg agt gag cag aaa gtc tct ata att cag ctc     920

    280                 285                 290

ttc acc aat tcc agc tac cga cag cct att cta gtg gca ctg atg ctg     968

295                 300                 305                 310

cat gtg gct cag caa ttt tcc gga atc aat ggc att ttt tac tac tca     1016

                315                 320                 325

acc agc att ttt cag acg gct ggt atc agc aaa cct gtt tat gca acc     1064

            330                 335                 340

att gga gtt ggc gct gta aac atg gtt ttc act gct gtc tct gta ttc     1112

        345                 350                 355

ctt gtg gag aag gca ggg cga cgt tct ctc ttt cta att gga atg agt     1160


    360                 365                 370

ggg atg ttt gtt tgt gcc atc ttc atg tca gtg gga ctt gtg ctg ctg    1208

375                 380                 385                 390

aat aag ttc tct tgg atg agt tat gtg agc atg ata gcc atc ttc ctc    1256

                395                 400                 405

ttt gtc agc ttc ttt gaa att ggg cca ggc ccg atc ccc tgg ttt atg    1304

            410                 415                 420

gtg gct gag ttt ttc agt caa gga cca cgt cct gct gct tta gca ata    1352

        425                 430                 435

gct gca ttc agc aat tgg acc tgc aat ttc att gta gct ctg tgt ttc    1400

    440                 445                 450

cag tac att gcg gac ttc tgt gga cct tat gtg ttt ttc ctc ttt gct    1448

455                 460                 465                 470

gga gtg ctc ctg gcc ttt acc ctg ttt aca ttt ttt aaa gtt cca gaa    1496

                475                 480                 485

acc aaa gga aag tct ttt gag gaa att gct gca gaa ttc caa aag aag    1544

            490                 495                 500

agt ggc tca gcc cac agg cca aaa gct gct gta gaa atg aaa ttc cta    1592

        505                 510                 515

gga gct aca gag act gtg taaaaaaaa accctgcttt ttggcatgaa            1640

    520

cagaaacaat aagggaaccg tctg                                         1664

<210> 40
<211> 524
<212> PRT
<213> Homo sapiens

<400> 40

<210> 41
<211> 1587
<212> DNA
<213> Homo sapiens

<220>
<221> CDS
<222> (10)..(1581)

<400> 41
ggatccacc atg aca gaa gat aag gtc act ggg acc ctg gtt ttc act gtc   51
atc act gct gtg ctg ggt tcc ttc cag ttt gga tat gac att ggt gtg     99
atc aat gca cct caa cag gta ata ata tct cac tat aga cat gtt ttg    147
ggt gtt cca ctg gat gac cga aaa gct atc aac aac tat gtt atc aac    195
agt aca gat gaa ctg ccc aca atc tca tac tca atg aac cca aaa cca    243
acc cct tgg gct gag gaa gag act gtg gca gct gct caa cta atc acc    291
atg ctc tgg tcc ctg tct gta tcc agc ttt gca gtt ggt gga atg act     339
gca tca ttc ttt ggt ggg tgg ctt ggg gac aca ctt gga aga atc aaa     387
gcc atg tta gta gca aac att ctg tca tta gtt gga gct ctc ttg atg     435
ggg ttt tca aaa ttg gga cca tct cat ata ctt ata att gct gga aga     483
agc ata tca gga cta tat tgt ggg cta att tca ggc ctg gtt cct atg     531
tat atc ggt gaa att gct cca acc gct ctc agg gga gca ctt ggc act     579
ttt cat cag ctg gcc atc gtc acg ggc att ctt att agt cag att att     627
ggt ctt gaa ttt atc ttg ggc aat tat gat ctg tgg cac atc ctg ctt     675
ggc ctg tct ggt gtg cga gcc atc ctt cag tct ctg cta ctc ttt ttc     723
tgt cca gaa agc ccc aga tac ctt tac atc aag tta gat gag gaa gtc     771
aaa gca aaa caa agc ttg aaa aga ctc aga gga tat gat gat gtc acc     819
aaa gat att aat gaa atg aga aaa gaa aga gaa gaa gca tcg agt gag     867
cag aaa gtc tct ata att cag ctc ttc acc aat tcc agc tac cga cag     915
cct att cta gtg gca ctg atg ctg cat gtg gct cag caa ttt tcc gga     963
atc aat ggc att ttt tac tac tca acc agc att ttt cag acg gct ggt     1011
atc agc aaa cct gtt tat gca acc att gga gtt ggc gct gta aac atg     1059
gtt ttc act gct gtc tct gta ttc ctt gtg gag aag gca ggg cga cgt     1107
tct ctc ttt cta att gga atg agt ggg atg ttt gtt tgt gcc atc ttc     1155
atg tca gtg gga ctt gtg ctg ctg aat aag ttc tct tgg atg agt tat     1203
gtg agc atg ata gcc atc ttc ctc ttt gtc agc ttc ttt gaa att ggg     1251
cca ggc ccg atc ccc tgg ttt atg gtg gct gag ttt ttc agt caa gga     1299
cca cgt cct gct gct tta gca ata gct gca ttc agc aat tgg acc tgc     1347
aat ttc att gta gct ctg tgt ttc cag tac att gcg gac ttc tgt gga     1395
cct tat gtg ttt ttc ctc ttt gct gga gtg ctc ctg gcc ttt acc ctg     1443
ttt aca ttt ttt aaa gtt cca gaa acc aaa gga aag tct ttt gag gaa     1491
att gct gca gaa ttc caa aag aag agt ggc tca gcc cac agg cca aaa     1539
gct gct gta gaa atg aaa ttc cta gga gct aca gag act gtg ctcgag     1587

<210> 42
<211> 524
<212> PRT
<213> Homo sapiens

<400> 42

<210> 43
<211> 1473
<212> DNA
<213> Homo sapiens

<220>
<221> CDS
<222> (7)..(1467)

<400> 43
ggatcc act gtc atc act gct gtg ctg ggt tcc ttc cag ttt gga tat      48
       Thr Val Ile Thr Ala Val Leu Gly Ser Phe Gln Phe Gly Tyr
         1               5                  10

gac att ggt gtg atc aat gca cct caa cag gta ata ata tct cac tat      96
Asp Ile Gly Val Ile Asn Ala Pro Gln Gln Val Ile Ile Ser His Tyr
 15                  20                  25                  30

aga cat gtt ttg ggt gtt cca ctg gat gac cga aaa gct atc aac aac     144
Arg His Val Leu Gly Val Pro Leu Asp Asp Arg Lys Ala Ile Asn Asn
                 35                  40                  45

tat gtt atc aac agt aca gat gaa ctg ccc aca atc tca tac tca atg     192
Tyr Val Ile Asn Ser Thr Asp Glu Leu Pro Thr Ile Ser Tyr Ser Met
             50                  55                  60

aac cca aaa cca acc cct tgg gct gag gaa gag act gtg gca gct gct     240
Asn Pro Lys Pro Thr Pro Trp Ala Glu Glu Glu Thr Val Ala Ala Ala
         65                  70                  75

caa cta atc acc atg ctc tgg tcc ctg tct gta tcc agc ttt gca gtt     288
Gln Leu Ile Thr Met Leu Trp Ser Leu Ser Val Ser Ser Phe Ala Val
    80                  85                  90

ggt gga atg act gca tca ttc ttt ggt ggg tgg ctt ggg gac aca ctt     336
Gly Gly Met Thr Ala Ser Phe Phe Gly Gly Trp Leu Gly Asp Thr Leu
 95                 100                 105                 110

gga aga atc aaa gcc atg tta gta gca aac att ctg tca tta gtt gga     384
Gly Arg Ile Lys Ala Met Leu Val Ala Asn Ile Leu Ser Leu Val Gly
                115                 120                 125

gct ctc ttg atg ggg ttt tca aaa ttg gga cca tct cat ata ctt ata     432
Ala Leu Leu Met Gly Phe Ser Lys Leu Gly Pro Ser His Ile Leu Ile
            130                 135                 140

att gct gga aga agc ata tca gga cta tat tgt ggg cta att tca ggc     480
Ile Ala Gly Arg Ser Ile Ser Gly Leu Tyr Cys Gly Leu Ile Ser Gly
        145                 150                 155

ctg gtt cct atg tat atc ggt gaa att gct cca acc gct ctc agg gga     528
Leu Val Pro Met Tyr Ile Gly Glu Ile Ala Pro Thr Ala Leu Arg Gly
    160                 165                 170

gca ctt ggc act ttt cat cag ctg gcc atc gtc acg ggc att ctt att     576
Ala Leu Gly Thr Phe His Gln Leu Ala Ile Val Thr Gly Ile Leu Ile
175                 180                 185                 190

agt cag att att ggt ctt gaa ttt atc ttg ggc aat tat gat ctg tgg     624
Ser Gln Ile Ile Gly Leu Glu Phe Ile Leu Gly Asn Tyr Asp Leu Trp
                195                 200                 205

cac atc ctg ctt ggc ctg tct ggt gtg cga gcc atc ctt cag tct ctg     672
His Ile Leu Leu Gly Leu Ser Gly Val Arg Ala Ile Leu Gln Ser Leu
            210                 215                 220

cta ctc ttt ttc tgt cca gaa agc ccc aga tac ctt tac atc aag tta     720
Leu Leu Phe Phe Cys Pro Glu Ser Pro Arg Tyr Leu Tyr Ile Lys Leu
        225                 230                 235

gat gag gaa gtc aaa gca aaa caa agc ttg aaa aga ctc aga gga tat     768
Asp Glu Glu Val Lys Ala Lys Gln Ser Leu Lys Arg Leu Arg Gly Tyr
    240                 245                 250

gat gat gtc acc aaa gat att aat gaa atg aga aaa gaa aga gaa gaa     816
Asp Asp Val Thr Lys Asp Ile Asn Glu Met Arg Lys Glu Arg Glu Glu
255                 260                 265                 270

gca tcg agt gag cag aaa gtc tct ata att cag ctc ttc acc aat tcc     864
Ala Ser Ser Glu Gln Lys Val Ser Ile Ile Gln Leu Phe Thr Asn Ser
                275                 280                 285

agc tac cga cag cct att cta gtg gca ctg atg ctg cat gtg gct cag     912
Ser Tyr Arg Gln Pro Ile Leu Val Ala Leu Met Leu His Val Ala Gln
            290                 295                 300

caa ttt tcc gga atc aat ggc att ttt tac tac tca acc agc att ttt     960
Gln Phe Ser Gly Ile Asn Gly Ile Phe Tyr Tyr Ser Thr Ser Ile Phe
        305                 310                 315

cag acg gct ggt atc agc aaa cct gtt tat gca acc att gga gtt ggc     1008

Gln Thr Ala Gly Ile Ser Lys Pro Val Tyr Ala Thr Ile Gly Val Gly
    320                 325                 330

gct gta aac atg gtt ttc act gct gtc tct gta ttc ctt gtg gag aag     1056
Ala Val Asn Met Val Phe Thr Ala Val Ser Val Phe Leu Val Glu Lys
335                 340                 345                 350

gca ggg cga cgt tct ctc ttt cta att gga atg agt ggg atg ttt gtt     1104
Ala Gly Arg Arg Ser Leu Phe Leu Ile Gly Met Ser Gly Met Phe Val
                355                 360                 365

tgt gcc atc ttc atg tca gtg gga ctt gtg ctg ctg aat aag ttc tct     1152
Cys Ala Ile Phe Met Ser Val Gly Leu Val Leu Leu Asn Lys Phe Ser
            370                 375                 380

tgg atg agt tat gtg agc atg ata gcc atc ttc ctc ttt gtc agc ttc     1200
Trp Met Ser Tyr Val Ser Met Ile Ala Ile Phe Leu Phe Val Ser Phe
        385                 390                 395

ttt gaa att ggg cca ggc ccg atc ccc tgg ttt atg gtg gct gag ttt     1248
Phe Glu Ile Gly Pro Gly Pro Ile Pro Trp Phe Met Val Ala Glu Phe
    400                 405                 410

ttc agt caa gga cca cgt cct gct gct tta gca ata gct gca ttc agc     1296
Phe Ser Gln Gly Pro Arg Pro Ala Ala Leu Ala Ile Ala Ala Phe Ser
415                 420                 425                 430

aat tgg acc tgc aat ttc att gta gct ctg tgt ttc cag tac att gcg     1344
Asn Trp Thr Cys Asn Phe Ile Val Ala Leu Cys Phe Gln Tyr Ile Ala
                435                 440                 445

gac ttc tgt gga cct tat gtg ttt ttc ctc ttt gct gga gtg ctc ctg     1392
Asp Phe Cys Gly Pro Tyr Val Phe Phe Leu Phe Ala Gly Val Leu Leu
            450                 455                 460

gcc ttt acc ctg ttt aca ttt ttt aaa gtt cca gaa acc aaa gga aag     1440
Ala Phe Thr Leu Phe Thr Phe Phe Lys Val Pro Glu Thr Lys Gly Lys
        465                 470                 475

tct ttt gag gaa att gct gca gaa ttc ctcgag                          1473
Ser Phe Glu Glu Ile Ala Ala Glu Phe
    480                 485

<210> 44
<211> 487
<212> PRT
<213> Homo sapiens

<400> 44
Thr Val Ile Thr Ala Val Leu Gly Ser Phe Gln Phe Gly Tyr Asp Ile
  1               5                  10                  15

Gly Val Ile Asn Ala Pro Gln Gln Val Ile Ile Ser His Tyr Arg His
             20                  25                  30

Val Leu Gly Val Pro Leu Asp Asp Arg Lys Ala Ile Asn Asn Tyr Val
         35                  40                  45

Ile Asn Ser Thr Asp Glu Leu Pro Thr Ile Ser Tyr Ser Met Asn Pro
     50                  55                  60

Lys Pro Thr Pro Trp Ala Glu Glu Glu Thr Val Ala Ala Ala Gln Leu
65                  70                  75                  80

Ile Thr Met Leu Trp Ser Leu Ser Val Ser Ser Phe Ala Val Gly Gly
                85                  90                  95

Met Thr Ala Ser Phe Phe Gly Gly Trp Leu Gly Asp Thr Leu Gly Arg
            100                 105                 110

Ile Lys Ala Met Leu Val Ala Asn Ile Leu Ser Leu Val Gly Ala Leu
        115                 120                 125

Leu Met Gly Phe Ser Lys Leu Gly Pro Ser His Ile Leu Ile Ile Ala
    130                 135                 140

Gly Arg Ser Ile Ser Gly Leu Tyr Cys Gly Leu Ile Ser Gly Leu Val
145                 150                 155                 160

Pro Met Tyr Ile Gly Glu Ile Ala Pro Thr Ala Leu Arg Gly Ala Leu
                165                 170                 175

Gly Thr Phe His Gln Leu Ala Ile Val Thr Gly Ile Leu Ile Ser Gln
            180                 185                 190

Ile Ile Gly Leu Glu Phe Ile Leu Gly Asn Tyr Asp Leu Trp His Ile
        195                 200                 205

Leu Leu Gly Leu Ser Gly Val Arg Ala Ile Leu Gln Ser Leu Leu Leu
    210                 215                 220

Phe Phe Cys Pro Glu Ser Pro Arg Tyr Leu Tyr Ile Lys Leu Asp Glu
225                 230                 235                 240

Glu Val Lys Ala Lys Gln Ser Leu Lys Arg Leu Arg Gly Tyr Asp Asp
                245                 250                 255

Val Thr Lys Asp Ile Asn Glu Met Arg Lys Glu Arg Glu Glu Ala Ser
            260                 265                 270

Ser Glu Gln Lys Val Ser Ile Ile Gln Leu Phe Thr Asn Ser Ser Tyr
        275                 280                 285

Arg Gln Pro Ile Leu Val Ala Leu Met Leu His Val Ala Gln Gln Phe
    290                 295                 300

Ser Gly Ile Asn Gly Ile Phe Tyr Tyr Ser Thr Ser Ile Phe Gln Thr
305                 310                 315                 320

Ala Gly Ile Ser Lys Pro Val Tyr Ala Thr Ile Gly Val Gly Ala Val
                325                 330                 335

Asn Met Val Phe Thr Ala Val Ser Val Phe Leu Val Glu Lys Ala Gly
            340                 345                 350

Arg Arg Ser Leu Phe Leu Ile Gly Met Ser Gly Met Phe Val Cys Ala
        355                 360                 365

Ile Phe Met Ser Val Gly Leu Val Leu Leu Asn Lys Phe Ser Trp Met
    370                 375                 380

Ser Tyr Val Ser Met Ile Ala Ile Phe Leu Phe Val Ser Phe Phe Glu

385                 390                 395                 400

Ile Gly Pro Gly Pro Ile Pro Trp Phe Met Val Ala Glu Phe Phe Ser
                405                 410                 415

Gln Gly Pro Arg Pro Ala Ala Leu Ala Ile Ala Ala Phe Ser Asn Trp
            420                 425                 430

Thr Cys Asn Phe Ile Val Ala Leu Cys Phe Gln Tyr Ile Ala Asp Phe
        435                 440                 445

Cys Gly Pro Tyr Val Phe Phe Leu Phe Ala Gly Val Leu Leu Ala Phe
    450                 455                 460

Thr Leu Phe Thr Phe Phe Lys Val Pro Glu Thr Lys Gly Lys Ser Phe
465                 470                 475                 480

Glu Glu Ile Ala Ala Glu Phe
                485

<210> 45
<211> 2038
<212> DNA
<213> Homo sapiens

<220>
<221> CDS
<222> (10)..(1923)

<400> 45
   gttttcaga atg att tta ttc aag caa gca act tat ttc att tcc ttg ttt   51
             Met Ile Leu Phe Lys Gln Ala Thr Tyr Phe Ile Ser Leu Phe
             1               5                   10
   gct aca gtt tcc tgt gga tgt ctg act caa ctc tat gaa aac gcc ttc     99
   Ala Thr Val Ser Cys Gly Cys Leu Thr Gln Leu Tyr Glu Asn Ala Phe
   15                  20                  25                  30
   ttc aga ggt ggg gat gta gct tcc atg tac acc cca aat gcc caa tac    147
   Phe Arg Gly Gly Asp Val Ala Ser Met Tyr Thr Pro Asn Ala Gln Tyr
                   35                  40                  45
   tgc cag atg agg tgc aca ttc cac cca agg tgt ttg cta ttc agt ttt    195
   Cys Gln Met Arg Cys Thr Phe His Pro Arg Cys Leu Leu Phe Ser Phe
               50                  55                  60
   ctt cca gca agt tca atc aat gac atg gag aaa agg ttt ggt tgc ttc    243
   Leu Pro Ala Ser Ser Ile Asn Asp Met Glu Lys Arg Phe Gly Cys Phe
           65                  70                  75
   ttg aaa gat agt gtt aca gga acc ctg cca aaa gta cat cga aca ggt    291
   Leu Lys Asp Ser Val Thr Gly Thr Leu Pro Lys Val His Arg Thr Gly
       80                  85                  90
   gca gtt tct gga cat tcc ttg aag caa tgt ggt cat caa ata agt gct    339
   Ala Val Ser Gly His Ser Leu Lys Gln Cys Gly His Gln Ile Ser Ala
   95                  100                 105                 110
   tgc cat cga gac att tat aaa gga gtt gat atg aga gga gtc aat ttt    387
   Cys His Arg Asp Ile Tyr Lys Gly Val Asp Met Arg Gly Val Asn Phe
                   115                 120                 125
   aat gtg tct aag gtt agc agt gtt gaa gaa tgc caa aaa agg tgc acc    435
   Asn Val Ser Lys Val Ser Ser Val Glu Glu Cys Gln Lys Arg Cys Thr
               130                 135                 140
   aat aac att cgc tgc cag ttt ttt tca tat gcc acg caa aca ttt cac    483
   Asn Asn Ile Arg Cys Gln Phe Phe Ser Tyr Ala Thr Gln Thr Phe His
           145                 150                 155
   aag gca gag tac cgg aac aat tgc cta tta aag tac agt ccc gga gga    531
   Lys Ala Glu Tyr Arg Asn Asn Cys Leu Leu Lys Tyr Ser Pro Gly Gly
       160                 165                 170
   aca cct acc gct ata aag gtg ctg agt aac gtg gaa tct gga ttc tca    579
   Thr Pro Thr Ala Ile Lys Val Leu Ser Asn Val Glu Ser Gly Phe Ser
   175                 180                 185                 190
   ctg aag ccc tgt gcc ctt tca gaa att ggt tgc cac atg aac atc ttc    627
   Leu Lys Pro Cys Ala Leu Ser Glu Ile Gly Cys His Met Asn Ile Phe
                   195                 200                 205
   cag cat ctt gcg ttc tca gat gtg gat gtt gcc agg ttt ctc act cca    675
   Gln His Leu Ala Phe Ser Asp Val Asp Val Ala Arg Phe Leu Thr Pro
               210                 215                 220
   gat gct ttt gtg tgt cgg acc atc tgc acc tat cac ccc aac tgc ctc    723
   Asp Ala Phe Val Cys Arg Thr Ile Cys Thr Tyr His Pro Asn Cys Leu
           225                 230                 235
   ttc ttt aca ttc tat aca aat gta tgg aaa atc gag tca caa aga aat    771
   Phe Phe Thr Phe Tyr Thr Asn Val Trp Lys Ile Glu Ser Gln Arg Asn
       240                 245                 250
   gtt tgt ctt ctt aaa aca tct gaa agt ggc aca cca agt tcc tct act    819
   Val Cys Leu Leu Lys Thr Ser Glu Ser Gly Thr Pro Ser Ser Ser Thr
   255                 260                 265                 270
   cct caa gaa aac acc ata tct gga tat agc ctt tta acc tgc aaa aga    867
   Pro Gln Glu Asn Thr Ile Ser Gly Tyr Ser Leu Leu Thr Cys Lys Arg
                   275                 280                 285
   act tta cct gaa ccc tgc cat tct aaa att tac ccg gga gtt gac ttt    915
   Thr Leu Pro Glu Pro Cys His Ser Lys Ile Tyr Pro Gly Val Asp Phe
               290                 295                 300
   gga gga gaa gaa ttg aat gtg act ttt gtt aaa gga gtg aat gtt tgc    963
   Gly Gly Glu Glu Leu Asn Val Thr Phe Val Lys Gly Val Asn Val Cys
           305                 310                 315
   caa gag act tgc aca aag atg att cgc tgt cag ttt ttc act tat tct    1011
   Gln Glu Thr Cys Thr Lys Met Ile Arg Cys Gln Phe Phe Thr Tyr Ser
       320                 325                 330
   tta ctc cca gaa gac tgt aag gaa gag aag tgt aag tgt ttc tta aga    1059
   Leu Leu Pro Glu Asp Cys Lys Glu Glu Lys Cys Lys Cys Phe Leu Arg
   335                 340                 345                 350
   tta tct atg gat ggt tct cca act agg att gcg tat ggg aca caa ggg    1107
   Leu Ser Met Asp Gly Ser Pro Thr Arg Ile Ala Tyr Gly Thr Gln Gly
                   355                 360                 365
   agc tct ggt tac tct ttg aga ttg tgt aac act ggg gac aac gct gtc     1155
   Ser Ser Gly Tyr Ser Leu Arg Leu Cys Asn Thr Gly Asp Asn Ala Val
               370                 375                 380
   tgc aca aca aaa aca agc aca cgc att gtt gga gga aca aac tct tct     1203
   Cys Thr Thr Lys Thr Ser Thr Arg Ile Val Gly Gly Thr Asn Ser Ser
           385                 390                 395
   tgg gga gag tgg ccc tgg cag gtg agc ctg cag gtg aag ctg aca gct     1251
   Trp Gly Glu Trp Pro Trp Gln Val Ser Leu Gln Val Lys Leu Thr Ala
       400                 405                 410
   cag agg cac ctg tgt gga ggg tca ctc ata gga cac cag tgg gtc ctc     1299
   Gln Arg His Leu Cys Gly Gly Ser Leu Ile Gly His Gln Trp Val Leu
   415                 420                 425                 430
   act gct gcc cac tgc ttt gat ggg ctt ccc ctg cag gat gtt tgg cgc     1347
   Thr Ala Ala His Cys Phe Asp Gly Leu Pro Leu Gln Asp Val Trp Arg
                   435                 440                 445
   atc tat agt ggc att tta aat ctg tca gac att aca aaa gat aca cct     1395
   Ile Tyr Ser Gly Ile Leu Asn Leu Ser Asp Ile Thr Lys Asp Thr Pro
               450                 455                 460
   ttc tca caa ata aaa gag att att att cac caa aac tat aaa gtc tca     1443
   Phe Ser Gln Ile Lys Glu Ile Ile Ile His Gln Asn Tyr Lys Val Ser
           465                 470                 475
   gaa ggg aat cat gat atc gcc ttg ata aaa ctc cag gct cct ttg aat     1491
   Glu Gly Asn His Asp Ile Ala Leu Ile Lys Leu Gln Ala Pro Leu Asn
       480                 485                 490
   tac act gaa ttc caa aaa cca ata tgc cta cct tcc aaa ggt gac aca     1539
   Tyr Thr Glu Phe Gln Lys Pro Ile Cys Leu Pro Ser Lys Gly Asp Thr
   495                 500                 505                 510
   agc aca att tat acc aac tgt tgg gta acc gga tgg ggc ttc tcg aag     1587
   Ser Thr Ile Tyr Thr Asn Cys Trp Val Thr Gly Trp Gly Phe Ser Lys
                   515                 520                 525
   gag aaa ggt gaa atc caa aat att cta caa aag gta aat att cct ttg     1635
   Glu Lys Gly Glu Ile Gln Asn Ile Leu Gln Lys Val Asn Ile Pro Leu
               530                 535                 540
   gta aca aat gaa gaa tgc cag aaa aga tat caa gat tat aaa ata acc     1683
   Val Thr Asn Glu Glu Cys Gln Lys Arg Tyr Gln Asp Tyr Lys Ile Thr
           545                 550                 555
   caa cgg atg gtc tgt gct ggc tat aaa gaa ggg gga aaa gat gct tgt     1731
   Gln Arg Met Val Cys Ala Gly Tyr Lys Glu Gly Gly Lys Asp Ala Cys
       560                 565                 570
   aag gga gat tca ggt ggt ccc tta gtt tgc aaa cac aac gga atg tgg     1779
   Lys Gly Asp Ser Gly Gly Pro Leu Val Cys Lys His Asn Gly Met Trp
   575                 580                 585                 590
   cgt ttg gtg ggc atc acc agc tgg ggt gaa ggc tgt gcc cgc agg gag     1827
   Arg Leu Val Gly Ile Thr Ser Trp Gly Glu Gly Cys Ala Arg Arg Glu
                   595                 600                 605
   caa cct ggt gtc tac acc aaa gtc gct gag tac atg gac tgg att tta     1875
   Gln Pro Gly Val Tyr Thr Lys Val Ala Glu Tyr Met Asp Trp Ile Leu
               610                 615                 620
   gag aaa aca cag agc agt gat gga aaa gct cag atg cag tca cca gca    1923
   Glu Lys Thr Gln Ser Ser Asp Gly Lys Ala Gln Met Gln Ser Pro Ala
           625                 630                 635
   tgagaagcag tccagagtct aggcaatttt tacaacctga gttcaagtca aattctgagc 1983
   ctggggggtc ctcatctgca aagcatgaag agtggcatct tctttgcatc ctaag      2038

<210> 46
<211> 638
<212> PRT
<213> Homo sapiens

<400> 46
Met Ile Leu Phe Lys Gln Ala Thr Tyr Phe Ile Ser Leu Phe Ala Thr
  1               5                  10                  15

Val Ser Cys Gly Cys Leu Thr Gln Leu Tyr Glu Asn Ala Phe Phe Arg
            20                  25                  30

Gly Gly Asp Val Ala Ser Met Tyr Thr Pro Asn Ala Gln Tyr Cys Gln
        35                  40                  45

Met Arg Cys Thr Phe His Pro Arg Cys Leu Leu Phe Ser Phe Leu Pro
    50                  55                  60

Ala Ser Ser Ile Asn Asp Met Glu Lys Arg Phe Gly Cys Phe Leu Lys
 65                  70                  75                  80

Asp Ser Val Thr Gly Thr Leu Pro Lys Val His Arg Thr Gly Ala Val
                 85                  90                  95

Ser Gly His Ser Leu Lys Gln Cys Gly His Gln Ile Ser Ala Cys His
            100                 105                 110

Arg Asp Ile Tyr Lys Gly Val Asp Met Arg Gly Val Asn Phe Asn Val
        115                 120                 125

Ser Lys Val Ser Ser Val Glu Glu Cys Gln Lys Arg Cys Thr Asn Asn
    130                 135                 140

Ile Arg Cys Gln Phe Phe Ser Tyr Ala Thr Gln Thr Phe His Lys Ala
145                 150                 155                 160

Glu Tyr Arg Asn Asn Cys Leu Leu Lys Tyr Ser Pro Gly Gly Thr Pro
                165                 170                 175

Thr Ala Ile Lys Val Leu Ser Asn Val Glu Ser Gly Phe Ser Leu Lys
            180                 185                 190

Pro Cys Ala Leu Ser Glu Ile Gly Cys His Met Asn Ile Phe Gln His
        195                 200                 205

Leu Ala Phe Ser Asp Val Asp Val Ala Arg Phe Leu Thr Pro Asp Ala
    210                 215                 220

Phe Val Cys Arg Thr Ile Cys Thr Tyr His Pro Asn Cys Leu Phe Phe
225                 230                 235                 240

Thr Phe Tyr Thr Asn Val Trp Lys Ile Glu Ser Gln Arg Asn Val Cys
                245                 250                 255

Leu Leu Lys Thr Ser Glu Ser Gly Thr Pro Ser Ser Ser Thr Pro Gln
            260                 265                 270

Glu Asn Thr Ile Ser Gly Tyr Ser Leu Leu Thr Cys Lys Arg Thr Leu
        275                 280                 285

Pro Glu Pro Cys His Ser Lys Ile Tyr Pro Gly Val Asp Phe Gly Gly
    290                 295                 300

Glu Glu Leu Asn Val Thr Phe Val Lys Gly Val Asn Val Cys Gln Glu
305                 310                 315                 320

Thr Cys Thr Lys Met Ile Arg Cys Gln Phe Phe Thr Tyr Ser Leu Leu
                325                 330                 335

Pro Glu Asp Cys Lys Glu Glu Lys Cys Lys Cys Phe Leu Arg Leu Ser
            340                 345                 350

Met Asp Gly Ser Pro Thr Arg Ile Ala Tyr Gly Thr Gln Gly Ser Ser
        355                 360                 365

Gly Tyr Ser Leu Arg Leu Cys Asn Thr Gly Asp Asn Ala Val Cys Thr
    370                 375                 380

Thr Lys Thr Ser Thr Arg Ile Val Gly Gly Thr Asn Ser Ser Trp Gly
385                 390                 395                 400

Glu Trp Pro Trp Gln Val Ser Leu Gln Val Lys Leu Thr Ala Gln Arg
                405                 410                 415

His Leu Cys Gly Gly Ser Leu Ile Gly His Gln Trp Val Leu Thr Ala
            420                 425                 430

Ala His Cys Phe Asp Gly Leu Pro Leu Gln Asp Val Trp Arg Ile Tyr
        435                 440                 445

Ser Gly Ile Leu Asn Leu Ser Asp Ile Thr Lys Asp Thr Pro Phe Ser
    450                 455                 460

Gln Ile Lys Glu Ile Ile Ile His Gln Asn Tyr Lys Val Ser Glu Gly
465                 470                 475                 480

Asn His Asp Ile Ala Leu Ile Lys Leu Gln Ala Pro Leu Asn Tyr Thr
                485                 490                 495

Glu Phe Gln Lys Pro Ile Cys Leu Pro Ser Lys Gly Asp Thr Ser Thr
            500                 505                 510

Ile Tyr Thr Asn Cys Trp Val Thr Gly Trp Gly Phe Ser Lys Glu Lys
        515                 520                 525

Gly Glu Ile Gln Asn Ile Leu Gln Lys Val Asn Ile Pro Leu Val Thr
    530                 535                 540

Asn Glu Glu Cys Gln Lys Arg Tyr Gln Asp Tyr Lys Ile Thr Gln Arg
545                 550                 555                 560

Met Val Cys Ala Gly Tyr Lys Glu Gly Gly Lys Asp Ala Cys Lys Gly
                565                 570                 575

Asp Ser Gly Gly Pro Leu Val Cys Lys His Asn Gly Met Trp Arg Leu
            580                 585                 590

Val Gly Ile Thr Ser Trp Gly Glu Gly Cys Ala Arg Arg Glu Gln Pro
        595                 600                 605

Gly Val Tyr Thr Lys Val Ala Glu Tyr Met Asp Trp Ile Leu Glu Lys
    610                 615                 620

Thr Gln Ser Ser Asp Gly Lys Ala Gln Met Gln Ser Pro Ala
625                 630                 635

<210> 47
<211> 1869
<212> DNA
<213> Homo sapiens

<220>
<221> CDS
<222> (1)..(1869)

<400> 47
gga tcc gga tgt ctg act caa ctc tat gaa aac gcc ttc ttc aga ggt      48
Gly Ser Gly Cys Leu Thr Gln Leu Tyr Glu Asn Ala Phe Phe Arg Gly
  1               5                  10                  15

ggg gat gta gct tcc atg tac acc cca aat gcc caa tac tgc cag atg      96
Gly Asp Val Ala Ser Met Tyr Thr Pro Asn Ala Gln Tyr Cys Gln Met
             20                  25                  30

agg tgc aca ttc cac cca agg tgt ttg cta ttc agt ttt ctt cca gca     144
Arg Cys Thr Phe His Pro Arg Cys Leu Leu Phe Ser Phe Leu Pro Ala
         35                  40                  45

agt tca atc aat gac atg gag aaa agg ttt ggt tgc ttc ttg aaa gat     192
Ser Ser Ile Asn Asp Met Glu Lys Arg Phe Gly Cys Phe Leu Lys Asp
     50                  55                  60

agt gtt aca gga acc ctg cca aaa gta cat cga aca ggt gca gtt tct     240
Ser Val Thr Gly Thr Leu Pro Lys Val His Arg Thr Gly Ala Val Ser
 65                  70                  75                  80

gga cat tcc ttg aag caa tgt ggt cat caa ata agt gct tgc cat cga     288
Gly His Ser Leu Lys Gln Cys Gly His Gln Ile Ser Ala Cys His Arg
                 85                  90                  95

gac att tat aaa gga gtt gat atg aga gga gtc aat ttt aat gtg tct     336
Asp Ile Tyr Lys Gly Val Asp Met Arg Gly Val Asn Phe Asn Val Ser
            100                 105                 110

aag gtt agc agt gtt gaa gaa tgc caa aaa agg tgc acc agt aac att     384
Lys Val Ser Ser Val Glu Glu Cys Gln Lys Arg Cys Thr Ser Asn Ile
        115                 120                 125

cgc tgc cag ttt ttt tca tat gcc acg caa aca ttt cac aag gca gag     432
Arg Cys Gln Phe Phe Ser Tyr Ala Thr Gln Thr Phe His Lys Ala Glu
    130                 135                 140

tac cgg aac aat tgc cta tta aag tac agt ccc gga gga aca cct acc     480
Tyr Arg Asn Asn Cys Leu Leu Lys Tyr Ser Pro Gly Gly Thr Pro Thr
145                 150                 155                 160

gct ata aag gtg ctg agt aac gtg gaa tct gga ttc tca ctg aag ccc     528
Ala Ile Lys Val Leu Ser Asn Val Glu Ser Gly Phe Ser Leu Lys Pro
                165                 170                 175

tgt gcc ctt tca gaa att ggt tgc cac atg aac atc ttc cag cat ctt     576
Cys Ala Leu Ser Glu Ile Gly Cys His Met Asn Ile Phe Gln His Leu
            180                 185                 190

gcg ttc tca gat gtg gat gtt gcc agg ttt ctc act cca gat gct ttt     624
Ala Phe Ser Asp Val Asp Val Ala Arg Phe Leu Thr Pro Asp Ala Phe
        195                 200                 205

gtg tgt cgg acc atc tgc acc tat cac ccc aac tgc ctc ttc ttt aca     672
Val Cys Arg Thr Ile Cys Thr Tyr His Pro Asn Cys Leu Phe Phe Thr
    210                 215                 220

ttc tat aca aat gta tgg aaa atc gag tca caa aga aat gtt tgt ctt     720
Phe Tyr Thr Asn Val Trp Lys Ile Glu Ser Gln Arg Asn Val Cys Leu
225                 230                 235                 240

ctt aaa aca tct gaa agt ggc aca cca agt tcc tct act cct caa gaa     768
Leu Lys Thr Ser Glu Ser Gly Thr Pro Ser Ser Ser Thr Pro Gln Glu
                245                 250                 255

aac acc ata tct gga tat agc ctt tta acc tgc aaa aga act tta cct     816
Asn Thr Ile Ser Gly Tyr Ser Leu Leu Thr Cys Lys Arg Thr Leu Pro
            260                 265                 270

gaa ccc tgc cat tct aaa att tac ccg gga gtt gac ttt gga gga gaa     864
Glu Pro Cys His Ser Lys Ile Tyr Pro Gly Val Asp Phe Gly Gly Glu
        275                 280                 285

gaa ttg aat gtg act ttt gtt aaa gga gtg aat gtt tgc caa gag act     912
Glu Leu Asn Val Thr Phe Val Lys Gly Val Asn Val Cys Gln Glu Thr
    290                 295                 300

tgc aca aag atg att cgc tgt cag ttt ttc act tat tct tta ctc cca     960
Cys Thr Lys Met Ile Arg Cys Gln Phe Phe Thr Tyr Ser Leu Leu Pro
305                 310                 315                 320

gaa gac tgt aag gaa gag aag tgt aag tgt ttc tta aga tta tct atg     1008
Glu Asp Cys Lys Glu Glu Lys Cys Lys Cys Phe Leu Arg Leu Ser Met
                325                 330                 335

gat ggt tct cca act agg att gcg tat ggg aca caa ggg agc tct ggt     1056
Asp Gly Ser Pro Thr Arg Ile Ala Tyr Gly Thr Gln Gly Ser Ser Gly
            340                 345                 350

tac tct ttg aga ttg tgt aac act ggg gac aac gct gtc tgc aca aca     1104
Tyr Ser Leu Arg Leu Cys Asn Thr Gly Asp Asn Ala Val Cys Thr Thr
        355                 360                 365

aaa aca agc aca cgc att gtt gga gga aca aac tct tct tgg gga gag     1152
Lys Thr Ser Thr Arg Ile Val Gly Gly Thr Asn Ser Ser Trp Gly Glu
    370                 375                 380

tgg ccc tgg cag gtg agc ctg cag gtg aag ctg aca gct cag agg cat     1200

Trp Pro Trp Gln Val Ser Leu Gln Val Lys Leu Thr Ala Gln Arg His
385                 390                 395                 400

ctg tgt gga ggg tca ctc ata gga cac cag tgg gtc ctc act gct gcc      1248
Leu Cys Gly Gly Ser Leu Ile Gly His Gln Trp Val Leu Thr Ala Ala
                405                 410                 415

cac tgc ttt gat ggg ctt ccc ctg cag gat gtt tgg cgc atc tat agt      1296
His Cys Phe Asp Gly Leu Pro Leu Gln Asp Val Trp Arg Ile Tyr Ser
            420                 425                 430

ggc att tta aat ctg tca gac att aca aaa gat aca cct ttc tca caa      1344
Gly Ile Leu Asn Leu Ser Asp Ile Thr Lys Asp Thr Pro Phe Ser Gln
        435                 440                 445

ata aaa gag att att att cac caa aac tat aaa gtc tca gaa ggg aat      1392
Ile Lys Glu Ile Ile Ile His Gln Asn Tyr Lys Val Ser Glu Gly Asn
    450                 455                 460

cat gat atc gcc ttg ata aaa ctc cag gct cct ttg aat tac act gaa      1440
His Asp Ile Ala Leu Ile Lys Leu Gln Ala Pro Leu Asn Tyr Thr Glu
465                 470                 475                 480

ttc caa aaa cca ata tgc cta cct tcc aaa ggt gac aca agc aca att      1488
Phe Gln Lys Pro Ile Cys Leu Pro Ser Lys Gly Asp Thr Ser Thr Ile
                485                 490                 495

tat acc aac tgt tgg gta acc gga tgg ggc ttc tcg aag gag aaa ggt      1536
Tyr Thr Asn Cys Trp Val Thr Gly Trp Gly Phe Ser Lys Glu Lys Gly
            500                 505                 510

gaa atc caa aat att cta caa aag gta aat att cct ttg gta aca aat      1584
Glu Ile Gln Asn Ile Leu Gln Lys Val Asn Ile Pro Leu Val Thr Asn
        515                 520                 525

gaa gaa tgc cag aaa aga tat caa gat tat aaa ata acc caa cgg atg      1632
Glu Glu Cys Gln Lys Arg Tyr Gln Asp Tyr Lys Ile Thr Gln Arg Met
    530                 535                 540

gtc tgt gct ggc tat aaa gaa ggg gga aaa gat gct tgt aag gga gat      1680
Val Cys Ala Gly Tyr Lys Glu Gly Gly Lys Asp Ala Cys Lys Gly Asp
545                 550                 555                 560

tca ggt ggt ccc tta gtt tgc aaa cac aat gga atg tgg cgt ttg gtg      1728
Ser Gly Gly Pro Leu Val Cys Lys His Asn Gly Met Trp Arg Leu Val
                565                 570                 575

ggc atc acc agc tgg ggt gaa ggc tgt gcc cgc agg gag caa cct ggt      1776
Gly Ile Thr Ser Trp Gly Glu Gly Cys Ala Arg Arg Glu Gln Pro Gly
            580                 585                 590

gtc tac acc aaa gtc gct gag tac atg gac tgg att tta gag aaa aca      1824
Val Tyr Thr Lys Val Ala Glu Tyr Met Asp Trp Ile Leu Glu Lys Thr
        595                 600                 605

cag agc agt gat gga aaa gct cag atg cag tca cca gca ctc gag          1869
Gln Ser Ser Asp Gly Lys Ala Gln Met Gln Ser Pro Ala Leu Glu
    610                 615                 620

<210> 48
<211> 623

<212> PRT
<213> Homo sapiens

<400> 48
Gly Ser Gly Cys Leu Thr Gln Leu Tyr Glu Asn Ala Phe Phe Arg Gly
1               5                   10                  15

Gly Asp Val Ala Ser Met Tyr Thr Pro Asn Ala Gln Tyr Cys Gln Met
            20                  25                  30

Arg Cys Thr Phe His Pro Arg Cys Leu Leu Phe Ser Phe Leu Pro Ala
        35                  40                  45

Ser Ser Ile Asn Asp Met Glu Lys Arg Phe Gly Cys Phe Leu Lys Asp
    50                  55                  60

Ser Val Thr Gly Thr Leu Pro Lys Val His Arg Thr Gly Ala Val Ser
65                  70                  75                  80

Gly His Ser Leu Lys Gln Cys Gly His Gln Ile Ser Ala Cys His Arg
                85                  90                  95

Asp Ile Tyr Lys Gly Val Asp Met Arg Gly Val Asn Phe Asn Val Ser
            100                 105                 110

Lys Val Ser Ser Val Glu Glu Cys Gln Lys Arg Cys Thr Ser Asn Ile
        115                 120                 125

Arg Cys Gln Phe Phe Ser Tyr Ala Thr Gln Thr Phe His Lys Ala Glu
    130                 135                 140

Tyr Arg Asn Asn Cys Leu Leu Lys Tyr Ser Pro Gly Gly Thr Pro Thr
145                 150                 155                 160

Ala Ile Lys Val Leu Ser Asn Val Glu Ser Gly Phe Ser Leu Lys Pro
                165                 170                 175

Cys Ala Leu Ser Glu Ile Gly Cys His Met Asn Ile Phe Gln His Leu
            180                 185                 190

Ala Phe Ser Asp Val Asp Val Ala Arg Phe Leu Thr Pro Asp Ala Phe
        195                 200                 205

Val Cys Arg Thr Ile Cys Thr Tyr His Pro Asn Cys Leu Phe Phe Thr
    210                 215                 220

Phe Tyr Thr Asn Val Trp Lys Ile Glu Ser Gln Arg Asn Val Cys Leu
225                 230                 235                 240

Leu Lys Thr Ser Glu Ser Gly Thr Pro Ser Ser Ser Thr Pro Gln Glu
                245                 250                 255

Asn Thr Ile Ser Gly Tyr Ser Leu Leu Thr Cys Lys Arg Thr Leu Pro
            260                 265                 270

Glu Pro Cys His Ser Lys Ile Tyr Pro Gly Val Asp Phe Gly Gly Glu
        275                 280                 285

Glu Leu Asn Val Thr Phe Val Lys Gly Val Asn Val Cys Gln Glu Thr
    290                 295                 300

Cys Thr Lys Met Ile Arg Cys Gln Phe Phe Thr Tyr Ser Leu Leu Pro
305                 310                 315                 320

Glu Asp Cys Lys Glu Glu Lys Cys Lys Cys Phe Leu Arg Leu Ser Met
                325                 330                 335

Asp Gly Ser Pro Thr Arg Ile Ala Tyr Gly Thr Gln Gly Ser Ser Gly
            340                 345                 350

Tyr Ser Leu Arg Leu Cys Asn Thr Gly Asp Asn Ala Val Cys Thr Thr
        355                 360                 365

Lys Thr Ser Thr Arg Ile Val Gly Gly Thr Asn Ser Ser Trp Gly Glu
    370                 375                 380

Trp Pro Trp Gln Val Ser Leu Gln Val Lys Leu Thr Ala Gln Arg His
385                 390                 395                 400

Leu Cys Gly Gly Ser Leu Ile Gly His Gln Trp Val Leu Thr Ala Ala
                405                 410                 415

His Cys Phe Asp Gly Leu Pro Leu Gln Asp Val Trp Arg Ile Tyr Ser
            420                 425                 430

Gly Ile Leu Asn Leu Ser Asp Ile Thr Lys Asp Thr Pro Phe Ser Gln
        435                 440                 445

Ile Lys Glu Ile Ile Ile His Gln Asn Tyr Lys Val Ser Glu Gly Asn
    450                 455                 460

His Asp Ile Ala Leu Ile Lys Leu Gln Ala Pro Leu Asn Tyr Thr Glu
465                 470                 475                 480

Phe Gln Lys Pro Ile Cys Leu Pro Ser Lys Gly Asp Thr Ser Thr Ile
                485                 490                 495

Tyr Thr Asn Cys Trp Val Thr Gly Trp Gly Phe Ser Lys Glu Lys Gly
            500                 505                 510

Glu Ile Gln Asn Ile Leu Gln Lys Val Asn Ile Pro Leu Val Thr Asn
        515                 520                 525

Glu Glu Cys Gln Lys Arg Tyr Gln Asp Tyr Lys Ile Thr Gln Arg Met
    530                 535                 540

Val Cys Ala Gly Tyr Lys Glu Gly Gly Lys Asp Ala Cys Lys Gly Asp
545                 550                 555                 560

Ser Gly Gly Pro Leu Val Cys Lys His Asn Gly Met Trp Arg Leu Val
                565                 570                 575

Gly Ile Thr Ser Trp Gly Glu Gly Cys Ala Arg Arg Glu Gln Pro Gly
            580                 585                 590

Val Tyr Thr Lys Val Ala Glu Tyr Met Asp Trp Ile Leu Glu Lys Thr
        595                 600                 605

Gln Ser Ser Asp Gly Lys Ala Gln Met Gln Ser Pro Ala Leu Glu
    610                 615                 620

<210> 49

<211> 2245
<212> DNA
<213> Homo sapiens

<220>
<221> CDS
<222> (72)..(1985)

<400> 49
agaacagctt gaagaccgtt catttttaag tgacaagaga ctcacctcca agaagcaatt 60

gtgttttcag a atg att tta ttc aag caa gca act tat ttc att tcc ttg 110
Met Ile Leu Phe Lys Gln Ala Thr Tyr Phe Ile Ser Leu
1               5                   10

ttt gct aca gtt tcc tgt gga tgt ctg act caa ctc tat gaa aac gcc 158
Phe Ala Thr Val Ser Cys Gly Cys Leu Thr Gln Leu Tyr Glu Asn Ala
    15                  20                  25

ttc ttc aga ggt ggg gat gta gct tcc atg tac acc cca aat gcc caa 206
Phe Phe Arg Gly Gly Asp Val Ala Ser Met Tyr Thr Pro Asn Ala Gln
30                  35                  40                  45

tac tgc cag atg agg tgc aca ttc cac cca agg tgt ttg cta ttc agt 254
Tyr Cys Gln Met Arg Cys Thr Phe His Pro Arg Cys Leu Leu Phe Ser
                50                  55                  60

ttt ctt cca gca agt tca atc aat gac atg gag aaa agg ttt ggt tgc 302
Phe Leu Pro Ala Ser Ser Ile Asn Asp Met Glu Lys Arg Phe Gly Cys
            65                  70                  75

ttc ttg aaa gat agt gtt aca gga acc ctg cca aaa gta cat cga aca 350
Phe Leu Lys Asp Ser Val Thr Gly Thr Leu Pro Lys Val His Arg Thr
        80                  85                  90

ggt gca gtt tct gga cat tcc ttg aag caa tgt ggt cat caa ata agt 398
Gly Ala Val Ser Gly His Ser Leu Lys Gln Cys Gly His Gln Ile Ser
    95                  100                 105

gct tgc cat cga gac att tat aaa gga gtt gat atg aga gga gtc aat 446
Ala Cys His Arg Asp Ile Tyr Lys Gly Val Asp Met Arg Gly Val Asn
110                 115                 120                 125

ttt aat gtg tct aag gtt agc agt gtt gaa gaa tgc caa aaa agg tgc 494
Phe Asn Val Ser Lys Val Ser Ser Val Glu Glu Cys Gln Lys Arg Cys
                130                 135                 140

acc aat aac att cgc tgc cag ttt ttt tca tat gcc acg caa aca ttt 542
Thr Asn Asn Ile Arg Cys Gln Phe Phe Ser Tyr Ala Thr Gln Thr Phe
            145                 150                 155

cac aag gca gag tac cgg aac aat tgc cta tta aag tac agt ccc gga 590
His Lys Ala Glu Tyr Arg Asn Asn Cys Leu Leu Lys Tyr Ser Pro Gly
        160                 165                 170

gga aca cct acc gct ata aag gtg ctg agt aac gtg gaa tct gga ttc 638
Gly Thr Pro Thr Ala Ile Lys Val Leu Ser Asn Val Glu Ser Gly Phe
    175                 180                 185

tca ctg aag ccc tgt gcc ctt tca gaa att ggt tgc cac atg aac atc 686
Ser Leu Lys Pro Cys Ala Leu Ser Glu Ile Gly Cys His Met Asn Ile

190                 195                 200                 205

ttc cag cat ctt gcg ttc tca gat gtg gat gtt gcc agg gtt ctc act     734
Phe Gln His Leu Ala Phe Ser Asp Val Asp Val Ala Arg Val Leu Thr
                210                 215                 220

cca gat gct ttt gtg tgt cgg acc atc tgc acc tat cac ccc aac tgc     782
Pro Asp Ala Phe Val Cys Arg Thr Ile Cys Thr Tyr His Pro Asn Cys
            225                 230                 235

ctc ttc ttt aca ttc tat aca aat gta tgg aaa atc gag tca caa aga     830
Leu Phe Phe Thr Phe Tyr Thr Asn Val Trp Lys Ile Glu Ser Gln Arg
        240                 245                 250

aat gtt tgt ctt ctt aaa aca tct gaa agt ggc aca cca agt tcc tct     878
Asn Val Cys Leu Leu Lys Thr Ser Glu Ser Gly Thr Pro Ser Ser Ser
    255                 260                 265

act cct caa gaa aac acc ata tct gga tat agc ctt tta acc tgc aaa     926
Thr Pro Gln Glu Asn Thr Ile Ser Gly Tyr Ser Leu Leu Thr Cys Lys
270                 275                 280                 285

aga act tta cct gaa ccc tgc cat tct aaa att tac ccg gga gtt gac     974
Arg Thr Leu Pro Glu Pro Cys His Ser Lys Ile Tyr Pro Gly Val Asp
                290                 295                 300

ttt gga gga gaa gaa ttg aat gtg act ttt gtt aaa gga gtg aat gtt     1022
Phe Gly Gly Glu Glu Leu Asn Val Thr Phe Val Lys Gly Val Asn Val
            305                 310                 315

tgc caa gag act tgc aca aag atg att cgc tgt cag ttt ttc act tat     1070
Cys Gln Glu Thr Cys Thr Lys Met Ile Arg Cys Gln Phe Phe Thr Tyr
        320                 325                 330

tct tta ctc cca gaa gac tgt aag gaa gag aag tgt aag tgt ttc tta     1118
Ser Leu Leu Pro Glu Asp Cys Lys Glu Glu Lys Cys Lys Cys Phe Leu
    335                 340                 345

aga tta tct atg gat ggt tct cca act agg att gcg tat ggg aca caa     1166
Arg Leu Ser Met Asp Gly Ser Pro Thr Arg Ile Ala Tyr Gly Thr Gln
350                 355                 360                 365

ggg agc tct ggt tac tct ttg aga ttg tgt aac act ggg gac aac tct     1214
Gly Ser Ser Gly Tyr Ser Leu Arg Leu Cys Asn Thr Gly Asp Asn Ser
                370                 375                 380

gtc tgc aca aca aaa aca agc aca cgc att gtt gga gga aca aac tct     1262
Val Cys Thr Thr Lys Thr Ser Thr Arg Ile Val Gly Gly Thr Asn Ser
            385                 390                 395

tct tgg gga gag tgg ccc tgg cag gtg agc ctg cag gtg aag ctg aca     1310
Ser Trp Gly Glu Trp Pro Trp Gln Val Ser Leu Gln Val Lys Leu Thr
        400                 405                 410

gct cag agg cac ctg tgt gga ggg tca ctc ata gga cac cag tgg gtc     1358
Ala Gln Arg His Leu Cys Gly Gly Ser Leu Ile Gly His Gln Trp Val
    415                 420                 425

ctc act gct gcc cac tgc ttt gat ggg ctt ccc ctg cag gat gtt tgg     1406
Leu Thr Ala Ala His Cys Phe Asp Gly Leu Pro Leu Gln Asp Val Trp
430                 435                 440                 445

cgc atc tat agt ggc att tta aat ctg tca gac att aca aaa gat aca 1454
Arg Ile Tyr Ser Gly Ile Leu Asn Leu Ser Asp Ile Thr Lys Asp Thr
                450                 455                 460

cct ttc tca caa ata aaa gag att att att cac caa aac tat aaa gtc 1502
Pro Phe Ser Gln Ile Lys Glu Ile Ile Ile His Gln Asn Tyr Lys Val
            465                 470                 475

tca gaa ggg aat cat gat atc gcc ttg ata aaa ctc cag gct cct ttg 1550
Ser Glu Gly Asn His Asp Ile Ala Leu Ile Lys Leu Gln Ala Pro Leu
        480                 485                 490

aat tac act gaa ttc caa aaa cca ata tgc cta cct tcc aaa ggt gac 1598
Asn Tyr Thr Glu Phe Gln Lys Pro Ile Cys Leu Pro Ser Lys Gly Asp
    495                 500                 505

aca agc aca att tat acc aac tgt tgg gta acc gga tgg ggc ttc tcg 1646
Thr Ser Thr Ile Tyr Thr Asn Cys Trp Val Thr Gly Trp Gly Phe Ser
510                 515                 520                 525

aag gag aaa ggt gaa atc caa aat att cta caa aag gta aat att cct 1694
Lys Glu Lys Gly Glu Ile Gln Asn Ile Leu Gln Lys Val Asn Ile Pro
                530                 535                 540

ttg gta aca aat gaa gaa tgc cag aaa aga tat caa gat tat aaa ata 1742
Leu Val Thr Asn Glu Glu Cys Gln Lys Arg Tyr Gln Asp Tyr Lys Ile
            545                 550                 555

acc caa cgg atg gtc tgt gct ggc tat aaa gaa ggg gga aaa gat gct 1790
Thr Gln Arg Met Val Cys Ala Gly Tyr Lys Glu Gly Gly Lys Asp Ala
        560                 565                 570

tgt aag gga gat tca ggt ggt ccc tta gtt tgc aaa cac aac gga atg 1838
Cys Lys Gly Asp Ser Gly Gly Pro Leu Val Cys Lys His Asn Gly Met
    575                 580                 585

tgg cgt ttg gtg ggc atc aca agc tgg ggt gaa ggc tgt gcc cgc agg 1886
Trp Arg Leu Val Gly Ile Thr Ser Trp Gly Glu Gly Cys Ala Arg Arg
590                 595                 600                 605

gag caa cct ggt gtc tac acc aaa gtc gct gag tac atg gac tgg att 1934
Glu Gln Pro Gly Val Tyr Thr Lys Val Ala Glu Tyr Met Asp Trp Ile
                610                 615                 620

tta gag aaa aca cag agc agt gat gga aaa gct cag atg cag tca cca 1982
Leu Glu Lys Thr Gln Ser Ser Asp Gly Lys Ala Gln Met Gln Ser Pro
            625                 630                 635

gca tgagaagcag tccagagtct aggcaatttt tacaacctga gttcaagtca 2035
Ala

aattctgagc ctggggggtc ctcatctgca aagcatggag agtggcatct tctttgcatc 2095

ctaaggacga aagacacagt gcactcagag ctgctgagga caatgtctgc tgaagcccgc 2155

tttcagcacg ccgtaaccag gggctgacaa tgcgaggtcg caactgagat ctccatgact 2215

gtgtgttgtg aaataaaatg gtgaaagatc 2245

<210> 50
<211> 638
<212> PRT
<213> Homo sapiens

<400> 50
Met Ile Leu Phe Lys Gln Ala Thr Tyr Phe Ile Ser Leu Phe Ala Thr
1               5                   10                  15

Val Ser Cys Gly Cys Leu Thr Gln Leu Tyr Glu Asn Ala Phe Phe Arg
            20                  25                  30

Gly Gly Asp Val Ala Ser Met Tyr Thr Pro Asn Ala Gln Tyr Cys Gln
        35                  40                  45

Met Arg Cys Thr Phe His Pro Arg Cys Leu Leu Phe Ser Phe Leu Pro
    50                  55                  60

Ala Ser Ser Ile Asn Asp Met Glu Lys Arg Phe Gly Cys Phe Leu Lys
65                  70                  75                  80

Asp Ser Val Thr Gly Thr Leu Pro Lys Val His Arg Thr Gly Ala Val
                85                  90                  95

Ser Gly His Ser Leu Lys Gln Cys Gly His Gln Ile Ser Ala Cys His
            100                 105                 110

Arg Asp Ile Tyr Lys Gly Val Asp Met Arg Gly Val Asn Phe Asn Val
        115                 120                 125

Ser Lys Val Ser Ser Val Glu Glu Cys Gln Lys Arg Cys Thr Asn Asn
    130                 135                 140

Ile Arg Cys Gln Phe Phe Ser Tyr Ala Thr Gln Thr Phe His Lys Ala
145                 150                 155                 160

Glu Tyr Arg Asn Asn Cys Leu Leu Lys Tyr Ser Pro Gly Gly Thr Pro
                165                 170                 175

Thr Ala Ile Lys Val Leu Ser Asn Val Glu Ser Gly Phe Ser Leu Lys
            180                 185                 190

Pro Cys Ala Leu Ser Glu Ile Gly Cys His Met Asn Ile Phe Gln His
        195                 200                 205

Leu Ala Phe Ser Asp Val Asp Val Ala Arg Val Leu Thr Pro Asp Ala
    210                 215                 220

Phe Val Cys Arg Thr Ile Cys Thr Tyr His Pro Asn Cys Leu Phe Phe
225                 230                 235                 240

Thr Phe Tyr Thr Asn Val Trp Lys Ile Glu Ser Gln Arg Asn Val Cys
                245                 250                 255

Leu Leu Lys Thr Ser Glu Ser Gly Thr Pro Ser Ser Ser Thr Pro Gln
            260                 265                 270

Glu Asn Thr Ile Ser Gly Tyr Ser Leu Leu Thr Cys Lys Arg Thr Leu
        275                 280                 285

Pro Glu Pro Cys His Ser Lys Ile Tyr Pro Gly Val Asp Phe Gly Gly

290                 295                 300

Glu Glu Leu Asn Val Thr Phe Val Lys Gly Val Asn Val Cys Gln Glu
305                 310                 315                 320

Thr Cys Thr Lys Met Ile Arg Cys Gln Phe Phe Thr Tyr Ser Leu Leu
                325                 330                 335

Pro Glu Asp Cys Lys Glu Glu Lys Cys Lys Cys Phe Leu Arg Leu Ser
            340                 345                 350

Met Asp Gly Ser Pro Thr Arg Ile Ala Tyr Gly Thr Gln Gly Ser Ser
        355                 360                 365

Gly Tyr Ser Leu Arg Leu Cys Asn Thr Gly Asp Asn Ser Val Cys Thr
    370                 375                 380

Thr Lys Thr Ser Thr Arg Ile Val Gly Gly Thr Asn Ser Ser Trp Gly
385                 390                 395                 400

Glu Trp Pro Trp Gln Val Ser Leu Gln Val Lys Leu Thr Ala Gln Arg
                405                 410                 415

His Leu Cys Gly Gly Ser Leu Ile Gly His Gln Trp Val Leu Thr Ala
            420                 425                 430

Ala His Cys Phe Asp Gly Leu Pro Leu Gln Asp Val Trp Arg Ile Tyr
        435                 440                 445

Ser Gly Ile Leu Asn Leu Ser Asp Ile Thr Lys Asp Thr Pro Phe Ser
    450                 455                 460

Gln Ile Lys Glu Ile Ile Ile His Gln Asn Tyr Lys Val Ser Glu Gly
465                 470                 475                 480

Asn His Asp Ile Ala Leu Ile Lys Leu Gln Ala Pro Leu Asn Tyr Thr
                485                 490                 495

Glu Phe Gln Lys Pro Ile Cys Leu Pro Ser Lys Gly Asp Thr Ser Thr
            500                 505                 510

Ile Tyr Thr Asn Cys Trp Val Thr Gly Trp Gly Phe Ser Lys Glu Lys
        515                 520                 525

Gly Glu Ile Gln Asn Ile Leu Gln Lys Val Asn Ile Pro Leu Val Thr
    530                 535                 540

Asn Glu Glu Cys Gln Lys Arg Tyr Gln Asp Tyr Lys Ile Thr Gln Arg
545                 550                 555                 560

Met Val Cys Ala Gly Tyr Lys Glu Gly Gly Lys Asp Ala Cys Lys Gly
                565                 570                 575

Asp Ser Gly Gly Pro Leu Val Cys Lys His Asn Gly Met Trp Arg Leu
            580                 585                 590

Val Gly Ile Thr Ser Trp Gly Glu Gly Cys Ala Arg Arg Glu Gln Pro
        595                 600                 605

Gly Val Tyr Thr Lys Val Ala Glu Tyr Met Asp Trp Ile Leu Glu Lys
    610                 615                 620

Thr Gln Ser Ser Asp Gly Lys Ala Gln Met Gln Ser Pro Ala
625                 630                 635

<210> 51
<211> 1869
<212> DNA
<213> Homo sapiens

<220>
<221> CDS
<222> (7)..(1863)

<400> 51
ggatcc gga tgt ctg act caa ctc tat gaa aac gcc ttc ttc aga ggt      48
       Gly Cys Leu Thr Gln Leu Tyr Glu Asn Ala Phe Phe Arg Gly
         1               5                  10

ggg gat gta gct tcc atg tac acc cca aat gcc caa tac tgc cag atg      96
Gly Asp Val Ala Ser Met Tyr Thr Pro Asn Ala Gln Tyr Cys Gln Met
 15                  20                  25                  30

agg tgc aca ttc cac cca agg tgt ttg cta ttc agt ttt ctt cca gca     144
Arg Cys Thr Phe His Pro Arg Cys Leu Leu Phe Ser Phe Leu Pro Ala
                 35                  40                  45

agt tca atc aat gac atg gag aaa agg ttt ggt tgc ttc ttg aaa gat     192
Ser Ser Ile Asn Asp Met Glu Lys Arg Phe Gly Cys Phe Leu Lys Asp
             50                  55                  60

agt gtt aca gga acc ctg cca aaa gta cat cga aca ggt gca gtt tct     240
Ser Val Thr Gly Thr Leu Pro Lys Val His Arg Thr Gly Ala Val Ser
         65                  70                  75

gga cat tcc ttg aag caa tgt ggt cat caa ata agt gct tgc cat cga     288
Gly His Ser Leu Lys Gln Cys Gly His Gln Ile Ser Ala Cys His Arg
     80                  85                  90

gac att tat aaa gga gtt gat atg aga gga gtc aat ttt aat gtg tct     336
Asp Ile Tyr Lys Gly Val Asp Met Arg Gly Val Asn Phe Asn Val Ser
 95                 100                 105                 110

aag gtt agc agt gtt gaa gaa tgc caa aaa agg tgc acc agt aac att     384
Lys Val Ser Ser Val Glu Glu Cys Gln Lys Arg Cys Thr Ser Asn Ile
                115                 120                 125

cgc tgc cag ttt ttt tca tat gcc acg caa aca ttt cac aag gca gag     432
Arg Cys Gln Phe Phe Ser Tyr Ala Thr Gln Thr Phe His Lys Ala Glu
            130                 135                 140

tac cgg aac aat tgc cta tta aag tac agt ccc gga gga aca cct acc     480
Tyr Arg Asn Asn Cys Leu Leu Lys Tyr Ser Pro Gly Gly Thr Pro Thr
        145                 150                 155

gct ata aag gtg ctg agt aac gtg gaa tct gga ttc tca ctg aag ccc     528
Ala Ile Lys Val Leu Ser Asn Val Glu Ser Gly Phe Ser Leu Lys Pro
    160                 165                 170

tgt gcc ctt tca gaa att ggt tgc cac atg aac atc ttc cag cat ctt     576
Cys Ala Leu Ser Glu Ile Gly Cys His Met Asn Ile Phe Gln His Leu
175                 180                 185                 190

gcg ttc tca gat gtg gat gtt gcc agg ttt ctc act cca gat gct ttt    624
Ala Phe Ser Asp Val Asp Val Ala Arg Phe Leu Thr Pro Asp Ala Phe
                195                 200                 205

gtg tgt cgg acc atc tgc acc tat cac ccc aac tgc ctc ttc ttt aca    672
Val Cys Arg Thr Ile Cys Thr Tyr His Pro Asn Cys Leu Phe Phe Thr
            210                 215                 220

ttc tat aca aat gta tgg aaa atc gag tca caa aga aat gtt tgt ctt    720
Phe Tyr Thr Asn Val Trp Lys Ile Glu Ser Gln Arg Asn Val Cys Leu
        225                 230                 235

ctt aaa aca tct gaa agt ggc aca cca agt tcc tct act cct caa gaa    768
Leu Lys Thr Ser Glu Ser Gly Thr Pro Ser Ser Ser Thr Pro Gln Glu
    240                 245                 250

aac acc ata tct gga tat agc ctt tta acc tgc aaa aga act tta cct    816
Asn Thr Ile Ser Gly Tyr Ser Leu Leu Thr Cys Lys Arg Thr Leu Pro
255                 260                 265                 270

gaa ccc tgc cat tct aaa att tac ccg gga gtt gac ttt gga gga gaa    864
Glu Pro Cys His Ser Lys Ile Tyr Pro Gly Val Asp Phe Gly Gly Glu
                275                 280                 285

gaa ttg aat gtg act ttt gtt aaa gga gtg aat gtt tgc caa gag act    912
Glu Leu Asn Val Thr Phe Val Lys Gly Val Asn Val Cys Gln Glu Thr
            290                 295                 300

tgc aca aag atg att cgc tgt cag ttt ttc act tat tct tta ctc cca    960
Cys Thr Lys Met Ile Arg Cys Gln Phe Phe Thr Tyr Ser Leu Leu Pro
        305                 310                 315

gaa gac tgt aag gaa gag aag tgt aag tgt ttc tta aga tta tct atg    1008
Glu Asp Cys Lys Glu Glu Lys Cys Lys Cys Phe Leu Arg Leu Ser Met
    320                 325                 330

gat ggt tct cca act agg att gcg tat ggg aca caa ggg agc tct ggt    1056
Asp Gly Ser Pro Thr Arg Ile Ala Tyr Gly Thr Gln Gly Ser Ser Gly
335                 340                 345                 350

tac tct ttg aga ttg tgt aac act ggg gac aac gct gtc tgc aca aca    1104
Tyr Ser Leu Arg Leu Cys Asn Thr Gly Asp Asn Ala Val Cys Thr Thr
                355                 360                 365

aaa aca agc aca cgc att gtt gga gga aca aac tct tct tgg gga gag    1152
Lys Thr Ser Thr Arg Ile Val Gly Gly Thr Asn Ser Ser Trp Gly Glu
            370                 375                 380

tgg ccc tgg cag gtg agc ctg cag gtg aag ctg aca gct cag agg cac    1200
Trp Pro Trp Gln Val Ser Leu Gln Val Lys Leu Thr Ala Gln Arg His
        385                 390                 395

ctg tgt gga ggg tca ctc ata gga cac cag tgg gtc ctc act gct gcc    1248
Leu Cys Gly Gly Ser Leu Ile Gly His Gln Trp Val Leu Thr Ala Ala
    400                 405                 410

cac tgc ttt gat ggg ctt ccc ctg cag gat gtt tgg cgc atc tat agt    1296
His Cys Phe Asp Gly Leu Pro Leu Gln Asp Val Trp Arg Ile Tyr Ser
415                 420                 425                 430

ggc att tta aat ctg tca gac att aca aaa gat aca cct ttc tca caa     1344
Gly Ile Leu Asn Leu Ser Asp Ile Thr Lys Asp Thr Pro Phe Ser Gln
                435                 440                 445

ata aaa gag att att att cac caa aac tat aaa gtc tca gaa ggg aat     1392
Ile Lys Glu Ile Ile Ile His Gln Asn Tyr Lys Val Ser Glu Gly Asn
            450                 455                 460

cat gat atc gcc ttg ata aaa ctc cag gct cct ttg aat tac act gaa     1440
His Asp Ile Ala Leu Ile Lys Leu Gln Ala Pro Leu Asn Tyr Thr Glu
        465                 470                 475

ttc caa aaa cca ata tgc cta cct tcc aaa ggt gac aca agc aca att     1488
Phe Gln Lys Pro Ile Cys Leu Pro Ser Lys Gly Asp Thr Ser Thr Ile
    480                 485                 490

tat acc aac tgt tgg gta acc gga tgg ggc ttc tcg aag gag aaa ggt     1536
Tyr Thr Asn Cys Trp Val Thr Gly Trp Gly Phe Ser Lys Glu Lys Gly
495                 500                 505                 510

gaa atc caa aat att cta caa aag gta aat att cct ttg gta aca aat     1584
Glu Ile Gln Asn Ile Leu Gln Lys Val Asn Ile Pro Leu Val Thr Asn
                515                 520                 525

gaa gaa tgc cag aaa aga tat caa gat tat aaa ata acc caa cgg atg     1632
Glu Glu Cys Gln Lys Arg Tyr Gln Asp Tyr Lys Ile Thr Gln Arg Met
            530                 535                 540

gtc tgt gct ggc tat aaa gaa ggg gga aaa gat gct tgt aag gga gat     1680
Val Cys Ala Gly Tyr Lys Glu Gly Gly Lys Asp Ala Cys Lys Gly Asp
        545                 550                 555

tca ggt ggt ccc tta gtt tgc aaa cac aat gga atg tgg cgt ttg gtg     1728
Ser Gly Gly Pro Leu Val Cys Lys His Asn Gly Met Trp Arg Leu Val
    560                 565                 570

ggc atc acc agc tgg ggt gaa ggc tgt gcc cgc agg gag caa cct ggt     1776
Gly Ile Thr Ser Trp Gly Glu Gly Cys Ala Arg Arg Glu Gln Pro Gly
575                 580                 585                 590

gtc tac acc aaa gtc gct gag tac atg gac tgg att tta gag aaa aca     1824
Val Tyr Thr Lys Val Ala Glu Tyr Met Asp Trp Ile Leu Glu Lys Thr
                595                 600                 605

cag agc agt gat gga aaa gct cag atg cag tca cca gca ctcgag          1869
Gln Ser Ser Asp Gly Lys Ala Gln Met Gln Ser Pro Ala
            610                 615

<210> 52
<211> 619
<212> PRT
<213> Homo sapiens

<400> 52
Gly Cys Leu Thr Gln Leu Tyr Glu Asn Ala Phe Phe Arg Gly Gly Asp
1               5                   10                  15

Val Ala Ser Met Tyr Thr Pro Asn Ala Gln Tyr Cys Gln Met Arg Cys
            20                  25                  30

Thr Phe His Pro Arg Cys Leu Leu Phe Ser Phe Leu Pro Ala Ser Ser

35                  40                  45

Ile Asn Asp Met Glu Lys Arg Phe Gly Cys Phe Leu Lys Asp Ser Val
    50                  55                  60

Thr Gly Thr Leu Pro Lys Val His Arg Thr Gly Ala Val Ser Gly His
65                  70                  75                  80

Ser Leu Lys Gln Cys Gly His Gln Ile Ser Ala Cys His Arg Asp Ile
                85                  90                  95

Tyr Lys Gly Val Asp Met Arg Gly Val Asn Phe Asn Val Ser Lys Val
            100                 105                 110

Ser Ser Val Glu Glu Cys Gln Lys Arg Cys Thr Ser Asn Ile Arg Cys
        115                 120                 125

Gln Phe Phe Ser Tyr Ala Thr Gln Thr Phe His Lys Ala Glu Tyr Arg
    130                 135                 140

Asn Asn Cys Leu Leu Lys Tyr Ser Pro Gly Gly Thr Pro Thr Ala Ile
145                 150                 155                 160

Lys Val Leu Ser Asn Val Glu Ser Gly Phe Ser Leu Lys Pro Cys Ala
                165                 170                 175

Leu Ser Glu Ile Gly Cys His Met Asn Ile Phe Gln His Leu Ala Phe
            180                 185                 190

Ser Asp Val Asp Val Ala Arg Phe Leu Thr Pro Asp Ala Phe Val Cys
        195                 200                 205

Arg Thr Ile Cys Thr Tyr His Pro Asn Cys Leu Phe Phe Thr Phe Tyr
    210                 215                 220

Thr Asn Val Trp Lys Ile Glu Ser Gln Arg Asn Val Cys Leu Leu Lys
225                 230                 235                 240

Thr Ser Glu Ser Gly Thr Pro Ser Ser Ser Thr Pro Gln Glu Asn Thr
                245                 250                 255

Ile Ser Gly Tyr Ser Leu Leu Thr Cys Lys Arg Thr Leu Pro Glu Pro
            260                 265                 270

Cys His Ser Lys Ile Tyr Pro Gly Val Asp Phe Gly Gly Glu Glu Leu
        275                 280                 285

Asn Val Thr Phe Val Lys Gly Val Asn Val Cys Gln Glu Thr Cys Thr
    290                 295                 300

Lys Met Ile Arg Cys Gln Phe Phe Thr Tyr Ser Leu Leu Pro Glu Asp
305                 310                 315                 320

Cys Lys Glu Glu Lys Cys Lys Cys Phe Leu Arg Leu Ser Met Asp Gly
                325                 330                 335

Ser Pro Thr Arg Ile Ala Tyr Gly Thr Gln Gly Ser Ser Gly Tyr Ser
            340                 345                 350

Leu Arg Leu Cys Asn Thr Gly Asp Asn Ala Val Cys Thr Thr Lys Thr
        355                 360                 365

Ser Thr Arg Ile Val Gly Gly Thr Asn Ser Ser Trp Gly Glu Trp Pro
    370                 375                 380

Trp Gln Val Ser Leu Gln Val Lys Leu Thr Ala Gln Arg His Leu Cys
385                 390                 395                 400

Gly Gly Ser Leu Ile Gly His Gln Trp Val Leu Thr Ala Ala His Cys
                405                 410                 415

Phe Asp Gly Leu Pro Leu Gln Asp Val Trp Arg Ile Tyr Ser Gly Ile
            420                 425                 430

Leu Asn Leu Ser Asp Ile Thr Lys Asp Thr Pro Phe Ser Gln Ile Lys
        435                 440                 445

Glu Ile Ile Ile His Gln Asn Tyr Lys Val Ser Glu Gly Asn His Asp
    450                 455                 460

Ile Ala Leu Ile Lys Leu Gln Ala Pro Leu Asn Tyr Thr Glu Phe Gln
465                 470                 475                 480

Lys Pro Ile Cys Leu Pro Ser Lys Gly Asp Thr Ser Thr Ile Tyr Thr
                485                 490                 495

Asn Cys Trp Val Thr Gly Trp Gly Phe Ser Lys Glu Lys Gly Glu Ile
            500                 505                 510

Gln Asn Ile Leu Gln Lys Val Asn Ile Pro Leu Val Thr Asn Glu Glu
        515                 520                 525

Cys Gln Lys Arg Tyr Gln Asp Tyr Lys Ile Thr Gln Arg Met Val Cys
    530                 535                 540

Ala Gly Tyr Lys Glu Gly Gly Lys Asp Ala Cys Lys Gly Asp Ser Gly
545                 550                 555                 560

Gly Pro Leu Val Cys Lys His Asn Gly Met Trp Arg Leu Val Gly Ile
                565                 570                 575

Thr Ser Trp Gly Glu Gly Cys Ala Arg Arg Glu Gln Pro Gly Val Tyr
            580                 585                 590

Thr Lys Val Ala Glu Tyr Met Asp Trp Ile Leu Glu Lys Thr Gln Ser
        595                 600                 605

Ser Asp Gly Lys Ala Gln Met Gln Ser Pro Ala
    610                 615

<210> 53
<211> 1551
<212> DNA
<213> Homo sapiens

<220>
<221> CDS
<222> (108)..(1511)

<400> 53
gcggccgcgg gagctgaccc tgcggggtcc cgggggggga gggggagccg cgaagccccc   60

actgaggccg ccgctgccgg gcctcccctc ccccccgggc gggcgcc atg cgg ggg    116
                                                    Met Arg Gly
                                                      1

agc ccg ggc gac gcg gag cgg cgg cag cgc tgg ggt cgc ctg ttc gag    164
Ser Pro Gly Asp Ala Glu Arg Arg Gln Arg Trp Gly Arg Leu Phe Glu
    5                   10                  15

gag ctg gac agt aac aag gat ggc cgc gtg gac gtg cac gag ttg cgc    212
Glu Leu Asp Ser Asn Lys Asp Gly Arg Val Asp Val His Glu Leu Arg
 20                  25                  30                  35

cag ggg ctg gcc agg ctg ggc ggg ggc aac cca gac ccc ggc gcc caa    260
Gln Gly Leu Ala Arg Leu Gly Gly Gly Asn Pro Asp Pro Gly Ala Gln
                 40                  45                  50

cag ggt atc tcc tct gag ggt gat gct gac cca gat ggc ggg ctc gac    308
Gln Gly Ile Ser Ser Glu Gly Asp Ala Asp Pro Asp Gly Gly Leu Asp
             55                  60                  65

ctg gag gaa ttt tcc cgc tat ctg cag gag cgg gaa cag cgt ctg ctg    356
Leu Glu Glu Phe Ser Arg Tyr Leu Gln Glu Arg Glu Gln Arg Leu Leu
         70                  75                  80

ctc atg ttt cac agt ctt gac cgg aac cag gat ggt cac att gat gtc    404
Leu Met Phe His Ser Leu Asp Arg Asn Gln Asp Gly His Ile Asp Val
     85                  90                  95

tct gag atc caa cag agt ttc cga gct ctg ggc att tcc atc tcg ctg    452
Ser Glu Ile Gln Gln Ser Phe Arg Ala Leu Gly Ile Ser Ile Ser Leu
100                 105                 110                 115

gag cag gct gag aaa att ttg cac agc atg gac cga gac ggc aca atg    500
Glu Gln Ala Glu Lys Ile Leu His Ser Met Asp Arg Asp Gly Thr Met
                120                 125                 130

acc att gac tgg caa gaa tgg cgc gac cac ttc ctg ttg cat tcg ctg    548
Thr Ile Asp Trp Gln Glu Trp Arg Asp His Phe Leu Leu His Ser Leu
            135                 140                 145

gaa aat gtg gag gac gtg ctg tat ttc tgg aag cat tcc acg gtc ctg    596
Glu Asn Val Glu Asp Val Leu Tyr Phe Trp Lys His Ser Thr Val Leu
        150                 155                 160

gac att ggc gag tgc ctg aca gtg ccg gac gag ttc tca aag caa gag    644
Asp Ile Gly Glu Cys Leu Thr Val Pro Asp Glu Phe Ser Lys Gln Glu
    165                 170                 175

aag ctg acg ggc atg tgg tgg aaa cag ctg gtg gcc ggc gca gtg gca    692
Lys Leu Thr Gly Met Trp Trp Lys Gln Leu Val Ala Gly Ala Val Ala
180                 185                 190                 195

ggt gcc gtg tca cgg aca ggc acg gcc cct ctg gac cgc ctc aag gtc    740
Gly Ala Val Ser Arg Thr Gly Thr Ala Pro Leu Asp Arg Leu Lys Val
                200                 205                 210

ttc att cag gtc cat gcc tca aag acc aac cgg ctg aac atc ctt ggg    788
Phe Ile Gln Val His Ala Ser Lys Thr Asn Arg Leu Asn Ile Leu Gly
            215                 220                 225

ggg ctt cga agc atg gtc ctt gag gga ggc atc cgc tgc ctg tgg cgc    836

Gly Leu Arg Ser Met Val Leu Glu Gly Gly Ile Arg Cys Leu Trp Arg
        230                 235                 240

ggc aat ggt att aat gta ctc aag att gcc ccc gag tca gct atc aag     884
Gly Asn Gly Ile Asn Val Leu Lys Ile Ala Pro Glu Ser Ala Ile Lys
    245                 250                 255

ttc atg gcc tat gaa cag gtg agg agg gcc atc ctg ggg cag cag gag     932
Phe Met Ala Tyr Glu Gln Val Arg Arg Ala Ile Leu Gly Gln Gln Glu
260                 265                 270                 275

aca ctg cat gtg cag gag cgc ttc gtg gct ggc tcc ctg gct ggt gcc     980
Thr Leu His Val Gln Glu Arg Phe Val Ala Gly Ser Leu Ala Gly Ala
                280                 285                 290

aca gcc caa acc atc att tac cct atg gag gtg ctg aag acg cgg ctg     1028
Thr Ala Gln Thr Ile Ile Tyr Pro Met Glu Val Leu Lys Thr Arg Leu
            295                 300                 305

acc ttg cgc cgg acg ggc cag tat aag ggg ctg ctg gac tgc gcc agg     1076
Thr Leu Arg Arg Thr Gly Gln Tyr Lys Gly Leu Leu Asp Cys Ala Arg
        310                 315                 320

cgt atc ctg gag agg gag ggg ccc cgt gcc ttc tac cgc ggc tac ctc     1124
Arg Ile Leu Glu Arg Glu Gly Pro Arg Ala Phe Tyr Arg Gly Tyr Leu
    325                 330                 335

ccc aac gtg ctg ggc atc atc ccc tat gcg ggc atc gac ctg gcc gtc     1172
Pro Asn Val Leu Gly Ile Ile Pro Tyr Ala Gly Ile Asp Leu Ala Val
340                 345                 350                 355

tac gag gtc ctg aag aac tgg tgg ctt cag cag tac agc cac gac tcg     1220
Tyr Glu Val Leu Lys Asn Trp Trp Leu Gln Gln Tyr Ser His Asp Ser
                360                 365                 370

gca gac cca ggc atc ctc gtg ctc ctg gcc tgc ggt acc ata tcc agc     1268
Ala Asp Pro Gly Ile Leu Val Leu Leu Ala Cys Gly Thr Ile Ser Ser
            375                 380                 385

acc tgc ggc cag ata gcc agt tac ccg ctg gcc ctg gtc cgg acc cgc     1316
Thr Cys Gly Gln Ile Ala Ser Tyr Pro Leu Ala Leu Val Arg Thr Arg
        390                 395                 400

atg cag gca caa gcc tcc atc gag ggt ggc ccc cag ctg tcc atg ctg     1364
Met Gln Ala Gln Ala Ser Ile Glu Gly Gly Pro Gln Leu Ser Met Leu
    405                 410                 415

ggt ctg cta cgt cac atc ctg tcc cag gag ggc atg cgg ggc ctc tac     1412
Gly Leu Leu Arg His Ile Leu Ser Gln Glu Gly Met Arg Gly Leu Tyr
420                 425                 430                 435

cgg ggg atc gcc ccc aac ttc atg aag gtt att cca gct gtg agc atc     1460
Arg Gly Ile Ala Pro Asn Phe Met Lys Val Ile Pro Ala Val Ser Ile
                440                 445                 450

tcc tat gtg gtc tac gag aac atg aag cag gcc ttg ggg gtc acg tcc     1508
Ser Tyr Val Val Tyr Glu Asn Met Lys Gln Ala Leu Gly Val Thr Ser
            455                 460                 465

agg tgagggaccc ggagcccgtc cccccaatcc ctcacccccc                      1551
Arg

<210> 54
<211> 468
<212> PRT
<213> Homo sapiens

<400> 54
Met Arg Gly Ser Pro Gly Asp Ala Glu Arg Arg Gln Arg Trp Gly Arg
1               5                   10                  15

Leu Phe Glu Glu Leu Asp Ser Asn Lys Asp Gly Arg Val Asp Val His
            20                  25                  30

Glu Leu Arg Gln Gly Leu Ala Arg Leu Gly Gly Gly Asn Pro Asp Pro
        35                  40                  45

Gly Ala Gln Gln Gly Ile Ser Ser Glu Gly Asp Ala Asp Pro Asp Gly
    50                  55                  60

Gly Leu Asp Leu Glu Glu Phe Ser Arg Tyr Leu Gln Glu Arg Glu Gln
65                  70                  75                  80

Arg Leu Leu Leu Met Phe His Ser Leu Asp Arg Asn Gln Asp Gly His
                85                  90                  95

Ile Asp Val Ser Glu Ile Gln Gln Ser Phe Arg Ala Leu Gly Ile Ser
            100                 105                 110

Ile Ser Leu Glu Gln Ala Glu Lys Ile Leu His Ser Met Asp Arg Asp
        115                 120                 125

Gly Thr Met Thr Ile Asp Trp Gln Glu Trp Arg Asp His Phe Leu Leu
    130                 135                 140

His Ser Leu Glu Asn Val Glu Asp Val Leu Tyr Phe Trp Lys His Ser
145                 150                 155                 160

Thr Val Leu Asp Ile Gly Glu Cys Leu Thr Val Pro Asp Glu Phe Ser
                165                 170                 175

Lys Gln Glu Lys Leu Thr Gly Met Trp Trp Lys Gln Leu Val Ala Gly
            180                 185                 190

Ala Val Ala Gly Ala Val Ser Arg Thr Gly Thr Ala Pro Leu Asp Arg
        195                 200                 205

Leu Lys Val Phe Ile Gln Val His Ala Ser Lys Thr Asn Arg Leu Asn
    210                 215                 220

Ile Leu Gly Gly Leu Arg Ser Met Val Leu Glu Gly Gly Ile Arg Cys
225                 230                 235                 240

Leu Trp Arg Gly Asn Gly Ile Asn Val Leu Lys Ile Ala Pro Glu Ser
                245                 250                 255

Ala Ile Lys Phe Met Ala Tyr Glu Gln Val Arg Arg Ala Ile Leu Gly
            260                 265                 270

Gln Gln Glu Thr Leu His Val Gln Glu Arg Phe Val Ala Gly Ser Leu
        275                 280                 285

Ala Gly Ala Thr Ala Gln Thr Ile Ile Tyr Pro Met Glu Val Leu Lys
    290                 295                 300

Thr Arg Leu Thr Leu Arg Arg Thr Gly Gln Tyr Lys Gly Leu Leu Asp
305                 310                 315                 320

Cys Ala Arg Arg Ile Leu Glu Arg Glu Gly Pro Arg Ala Phe Tyr Arg
                325                 330                 335

Gly Tyr Leu Pro Asn Val Leu Gly Ile Ile Pro Tyr Ala Gly Ile Asp
            340                 345                 350

Leu Ala Val Tyr Glu Val Leu Lys Asn Trp Trp Leu Gln Gln Tyr Ser
        355                 360                 365

His Asp Ser Ala Asp Pro Gly Ile Leu Val Leu Leu Ala Cys Gly Thr
    370                 375                 380

Ile Ser Ser Thr Cys Gly Gln Ile Ala Ser Tyr Pro Leu Ala Leu Val
385                 390                 395                 400

Arg Thr Arg Met Gln Ala Gln Ala Ser Ile Glu Gly Gly Pro Gln Leu
                405                 410                 415

Ser Met Leu Gly Leu Leu Arg His Ile Leu Ser Gln Glu Gly Met Arg
            420                 425                 430

Gly Leu Tyr Arg Gly Ile Ala Pro Asn Phe Met Lys Val Ile Pro Ala
        435                 440                 445

Val Ser Ile Ser Tyr Val Val Tyr Glu Asn Met Lys Gln Ala Leu Gly
    450                 455                 460

Val Thr Ser Arg
465

<210> 55
<211> 953
<212> DNA
<213> Homo sapiens

<220>
<221> CDS
<222> (50)..(853)

<400> 55
cagaggcagc atgagctgag agggtgatag gaaggcggcg ctagacagc atg gag gac   58
                                                      Met Glu Asp
                                                      1

ttt ctg ctc tcc aat ggg tac cag ctg ggc aag acc att ggg gaa ggg   106
Phe Leu Leu Ser Asn Gly Tyr Gln Leu Gly Lys Thr Ile Gly Glu Gly
    5                   10                  15

acc tac tca aaa gtc aaa gaa gca ttt tcc aaa aaa cac caa aga aaa   154
Thr Tyr Ser Lys Val Lys Glu Ala Phe Ser Lys Lys His Gln Arg Lys
20                  25                  30                  35

gtg gca att aaa gtt ata gac aag atg gga act tcc tca gag ttt atc   202
Val Ala Ile Lys Val Ile Asp Lys Met Gly Thr Ser Ser Glu Phe Ile

                40                  45                  50

cag aga ttc ctc cct cgg gag ctc caa atc gtc cgt acc ctg gac cac 250
Gln Arg Phe Leu Pro Arg Glu Leu Gln Ile Val Arg Thr Leu Asp His
            55                  60                  65

aag aac atc atc cag gtg tat gag atg ctg gag tct gcc gac ggg aaa 298
Lys Asn Ile Ile Gln Val Tyr Glu Met Leu Glu Ser Ala Asp Gly Lys
        70                  75                  80

atc tgc ctg gtg atg gag ctc gct gag gga ggg gat gtc ttt gac tgc 346
Ile Cys Leu Val Met Glu Leu Ala Glu Gly Gly Asp Val Phe Asp Cys
    85                  90                  95

gtg ctg aat ggg ggg cca ctg cct gaa agc cgg gcc aag gcc ctc ttc 394
Val Leu Asn Gly Gly Pro Leu Pro Glu Ser Arg Ala Lys Ala Leu Phe
100                 105                 110                 115

cgt cag atg gtt gag gcc atc cgc tac tgc cat ggc tgt ggt gtg gcc 442
Arg Gln Met Val Glu Ala Ile Arg Tyr Cys His Gly Cys Gly Val Ala
                120                 125                 130

cac cgg gac ctc aaa tgt gag aac gcc ttg ttg cag ggc ttc aac ctg 490
His Arg Asp Leu Lys Cys Glu Asn Ala Leu Leu Gln Gly Phe Asn Leu
            135                 140                 145

aag ctg act gac ttt ggc ttt gcc aag gtg ttg ccc aag tca cac cgg 538
Lys Leu Thr Asp Phe Gly Phe Ala Lys Val Leu Pro Lys Ser His Arg
        150                 155                 160

gag ctg agc cag acc ttc tgc ggc agt aca gcc tat gct gcc ccc gag 586
Glu Leu Ser Gln Thr Phe Cys Gly Ser Thr Ala Tyr Ala Ala Pro Glu
    165                 170                 175

gtg ctg cag ggc att ccc cac gat agc aaa aaa ggt gat gtc tgg agc 634
Val Leu Gln Gly Ile Pro His Asp Ser Lys Lys Gly Asp Val Trp Ser
180                 185                 190                 195

atg ggt gtg gtc ctg tat gtc atg ctc tgt gcc agc cta cct ttt gac 682
Met Gly Val Val Leu Tyr Val Met Leu Cys Ala Ser Leu Pro Phe Asp
                200                 205                 210

gac aca gac atc ccc aag atg ctg tgg cag cag cag aag ggg gtg tcc 730
Asp Thr Asp Ile Pro Lys Met Leu Trp Gln Gln Gln Lys Gly Val Ser
            215                 220                 225

ttc ccc act cat ctg agc atc tcg gcc gat tgc cag gac ctg ctc aag 778
Phe Pro Thr His Leu Ser Ile Ser Ala Asp Cys Gln Asp Leu Leu Lys
        230                 235                 240

agg ctc ctg gaa ccc gat atg atc ctc cgg cct tca att gaa gaa gtt 826
Arg Leu Leu Glu Pro Asp Met Ile Leu Arg Pro Ser Ile Glu Glu Val
    245                 250                 255

agt tgg cat cca tgg cta gca agc act tgataaaagc aatggcaagt 873
Ser Trp His Pro Trp Leu Ala Ser Thr
260                 265

gctctccaat aaagtagggg gagaaagcaa acccaaaaac ccgcttctaa aatggtgata 933

tatattttac gctttaagtt 953

<210> 56
<211> 268
<212> PRT
<213> Homo sapiens

<400> 56
Met Glu Asp Phe Leu Leu Ser Asn Gly Tyr Gln Leu Gly Lys Thr Ile
1               5                   10                  15

Gly Glu Gly Thr Tyr Ser Lys Val Lys Glu Ala Phe Ser Lys Lys His
            20                  25                  30

Gln Arg Lys Val Ala Ile Lys Val Ile Asp Lys Met Gly Thr Ser Ser
        35                  40                  45

Glu Phe Ile Gln Arg Phe Leu Pro Arg Glu Leu Gln Ile Val Arg Thr
    50                  55                  60

Leu Asp His Lys Asn Ile Ile Gln Val Tyr Glu Met Leu Glu Ser Ala
65                  70                  75                  80

Asp Gly Lys Ile Cys Leu Val Met Glu Leu Ala Glu Gly Gly Asp Val
                85                  90                  95

Phe Asp Cys Val Leu Asn Gly Gly Pro Leu Pro Glu Ser Arg Ala Lys
            100                 105                 110

Ala Leu Phe Arg Gln Met Val Glu Ala Ile Arg Tyr Cys His Gly Cys
        115                 120                 125

Gly Val Ala His Arg Asp Leu Lys Cys Glu Asn Ala Leu Leu Gln Gly
    130                 135                 140

Phe Asn Leu Lys Leu Thr Asp Phe Gly Phe Ala Lys Val Leu Pro Lys
145                 150                 155                 160

Ser His Arg Glu Leu Ser Gln Thr Phe Cys Gly Ser Thr Ala Tyr Ala
                165                 170                 175

Ala Pro Glu Val Leu Gln Gly Ile Pro His Asp Ser Lys Lys Gly Asp
            180                 185                 190

Val Trp Ser Met Gly Val Val Leu Tyr Val Met Leu Cys Ala Ser Leu
        195                 200                 205

Pro Phe Asp Asp Thr Asp Ile Pro Lys Met Leu Trp Gln Gln Gln Lys
    210                 215                 220

Gly Val Ser Phe Pro Thr His Leu Ser Ile Ser Ala Asp Cys Gln Asp
225                 230                 235                 240

Leu Leu Lys Arg Leu Leu Glu Pro Asp Met Ile Leu Arg Pro Ser Ile
                245                 250                 255

Glu Glu Val Ser Trp His Pro Trp Leu Ala Ser Thr
            260                 265

<210> 57
<211> 2490
<212> DNA

<213> Homo sapiens

<220>
<221> CDS
<222> (40)..(2457)

<400> 57
cgcgcgctgc ctgaggacgc cgcggccccc gcccccgcc atg ggc gcc cct gcc     54
                                           Met Gly Ala Pro Ala
                                           1               5

tgc gcc ctc gcg ctc tgc gtg gcc gtg gcc atc gtg gcc ggc gcc tcc     102
Cys Ala Leu Ala Leu Cys Val Ala Val Ala Ile Val Ala Gly Ala Ser
                10                  15                  20

tcg gag tcc ttg ggg acg gag cag cgc gtc gtg ggg cga gcg gca gaa     150
Ser Glu Ser Leu Gly Thr Glu Gln Arg Val Val Gly Arg Ala Ala Glu
            25                  30                  35

gtc ccg ggc cca gag ccc ggc cag cag gag cag ttg gtc ttc ggc agc     198
Val Pro Gly Pro Glu Pro Gly Gln Gln Glu Gln Leu Val Phe Gly Ser
        40                  45                  50

ggg gat gct gtg gag ctg agc tgt ccc ccg ccc ggg ggt ggt ccc atg     246
Gly Asp Ala Val Glu Leu Ser Cys Pro Pro Pro Gly Gly Gly Pro Met
    55                  60                  65

ggg ccc act gtc tgg gtc aag gat ggc aca ggg ctg gtg ccc tcg gag     294
Gly Pro Thr Val Trp Val Lys Asp Gly Thr Gly Leu Val Pro Ser Glu
70                  75                  80                  85

cgt gtc ctg gtg ggg ccc cag cgg ctg cag gtg ctg aat gcc tcc cac     342
Arg Val Leu Val Gly Pro Gln Arg Leu Gln Val Leu Asn Ala Ser His
                90                  95                  100

gag gac tcc ggg gcc tac agc tgc cgg cag cgg ctc acg cag cgc gta     390
Glu Asp Ser Gly Ala Tyr Ser Cys Arg Gln Arg Leu Thr Gln Arg Val
            105                 110                 115

ctg tgc cac ttc agt gtg cgg gtg aca gac gct cca tcc tcg gga gat     438
Leu Cys His Phe Ser Val Arg Val Thr Asp Ala Pro Ser Ser Gly Asp
        120                 125                 130

gac gaa gac ggg gag gac gag gct gag gac aca ggt gtg gac aca ggg     486
Asp Glu Asp Gly Glu Asp Glu Ala Glu Asp Thr Gly Val Asp Thr Gly
    135                 140                 145

gcc cct tac tgg aca cgg ccc gag cgg atg gac aag aag ctg ctg gcc     534
Ala Pro Tyr Trp Thr Arg Pro Glu Arg Met Asp Lys Lys Leu Leu Ala
150                 155                 160                 165

gtg ccg gcc gcc aac acc gtc cgc ttc cgc tgc cca gcc gct ggc aac     582
Val Pro Ala Ala Asn Thr Val Arg Phe Arg Cys Pro Ala Ala Gly Asn
                170                 175                 180

ccc act ccc tcc atc tcc tgg ctg aag aac ggc agg gag ttc cgc ggc     630
Pro Thr Pro Ser Ile Ser Trp Leu Lys Asn Gly Arg Glu Phe Arg Gly
            185                 190                 195

gag cac cgc att gga ggc atc aag ctg cgg cat cag cag tgg agc ctg     678
Glu His Arg Ile Gly Gly Ile Lys Leu Arg His Gln Gln Trp Ser Leu

200                 205                 210

gtc atg gaa agc gtg gtg ccc tcg gac cgc ggc aac tac acc tgc gtc     726
Val Met Glu Ser Val Val Pro Ser Asp Arg Gly Asn Tyr Thr Cys Val
    215                 220                 225

gtg gag aac aag ttt ggc agc atc cgg cag acg tac acg ctg gac gtg     774
Val Glu Asn Lys Phe Gly Ser Ile Arg Gln Thr Tyr Thr Leu Asp Val
230                 235                 240                 245

ctg gag cgc tcc ccg cac cgg ccc atc ctg cag gcg ggg ctg ccg gcc     822
Leu Glu Arg Ser Pro His Arg Pro Ile Leu Gln Ala Gly Leu Pro Ala
                250                 255                 260

aac cag acg gcg gtg ctg ggc agc gac gtg gag ttc cac tgc aag gtg     870
Asn Gln Thr Ala Val Leu Gly Ser Asp Val Glu Phe His Cys Lys Val
            265                 270                 275

tac agt gac gca cag ccc cac atc cag tgg ctc aag cac gtg gag gtg     918
Tyr Ser Asp Ala Gln Pro His Ile Gln Trp Leu Lys His Val Glu Val
        280                 285                 290

aac ggc agc aag gtg ggc ccg gac ggc aca ccc tac gtt acc gtg ctc     966
Asn Gly Ser Lys Val Gly Pro Asp Gly Thr Pro Tyr Val Thr Val Leu
    295                 300                 305

aag acg gcg ggc gct aac acc acc gac aag gag cta gag gtt ctc tcc     1014
Lys Thr Ala Gly Ala Asn Thr Thr Asp Lys Glu Leu Glu Val Leu Ser
310                 315                 320                 325

ttg cac aac gtc acc ttt gag gac gcc ggg gag tac acc tgc ctg gcg     1062
Leu His Asn Val Thr Phe Glu Asp Ala Gly Glu Tyr Thr Cys Leu Ala
                330                 335                 340

ggc aat tct att ggg ttt tct cat cac tct gcg tgg ctg gtg gtg ctg     1110
Gly Asn Ser Ile Gly Phe Ser His His Ser Ala Trp Leu Val Val Leu
            345                 350                 355

cca gcc gag gag gag ctg gtg gag gct gac gag gcg ggc agt gtg tat     1158
Pro Ala Glu Glu Glu Leu Val Glu Ala Asp Glu Ala Gly Ser Val Tyr
        360                 365                 370

gca ggc atc ctc agc tac ggg gtg ggc ttc ttc ctg ttc atc ctg gtg     1206
Ala Gly Ile Leu Ser Tyr Gly Val Gly Phe Phe Leu Phe Ile Leu Val
    375                 380                 385

gtg gcg gct gtg acg ctc tgc cgc ctg cgc agc ccc ccc aag aaa ggc     1254
Val Ala Ala Val Thr Leu Cys Arg Leu Arg Ser Pro Pro Lys Lys Gly
390                 395                 400                 405

ctg ggc tcc ccc acc gtg cac aag atc tcc cgc ttc ccg ctc aag cga     1302
Leu Gly Ser Pro Thr Val His Lys Ile Ser Arg Phe Pro Leu Lys Arg
                410                 415                 420

cag gtg tcc ctg gag tcc aac gcg tcc atg agc tcc aac aca cca ctg     1350
Gln Val Ser Leu Glu Ser Asn Ala Ser Met Ser Ser Asn Thr Pro Leu
            425                 430                 435

gtg cgc atc gca agg ctg tcc tca ggg gag ggc ccc acg ctg gcc aat     1398
Val Arg Ile Ala Arg Leu Ser Ser Gly Glu Gly Pro Thr Leu Ala Asn
        440                 445                 450

gtc tcc gag ctc gag ctg cct gcc gac ccc aaa tgg gag ctg tct cgg    1446
Val Ser Glu Leu Glu Leu Pro Ala Asp Pro Lys Trp Glu Leu Ser Arg
    455                 460                 465

gcc cgg ctg acc ctg ggc aag ccc ctt ggg gag ggc tgc ttc ggc cag    1494
Ala Arg Leu Thr Leu Gly Lys Pro Leu Gly Glu Gly Cys Phe Gly Gln
470                 475                 480                 485

gtg gtc atg gcg gag gcc atc ggc att gac aag gac cgg gcc gcc aag    1542
Val Val Met Ala Glu Ala Ile Gly Ile Asp Lys Asp Arg Ala Ala Lys
                490                 495                 500

cct gtc acc gta gcc gtg aag atg ctg aaa gac gat gcc act gac aag    1590
Pro Val Thr Val Ala Val Lys Met Leu Lys Asp Asp Ala Thr Asp Lys
            505                 510                 515

gac ctg tcg gac ctg gtg tct gag atg gag atg atg aag atg atc ggg    1638
Asp Leu Ser Asp Leu Val Ser Glu Met Glu Met Met Lys Met Ile Gly
        520                 525                 530

aaa cac aaa aac atc atc aac ctg ctg ggc gcc tgc acg cag ggc ggg    1686
Lys His Lys Asn Ile Ile Asn Leu Leu Gly Ala Cys Thr Gln Gly Gly
    535                 540                 545

ccc ctg tac gtg ctg gtg gag tac gcg gcc aag ggt aac ctg cgg gag    1734
Pro Leu Tyr Val Leu Val Glu Tyr Ala Ala Lys Gly Asn Leu Arg Glu
550                 555                 560                 565

ttt ctg cgg gcg cgg cgg ccc ccg ggc ctg gac tac tcc ttc gac acc    1782
Phe Leu Arg Ala Arg Arg Pro Pro Gly Leu Asp Tyr Ser Phe Asp Thr
                570                 575                 580

tgc aag ccg ccc gag gag cag ctc acc ttc aag gac ctg gtg tcc tgt    1830
Cys Lys Pro Pro Glu Glu Gln Leu Thr Phe Lys Asp Leu Val Ser Cys
            585                 590                 595

gcc tac cag gtg gcc cgg ggc atg gag tac ttg gcc tcc cag aag tgc    1878
Ala Tyr Gln Val Ala Arg Gly Met Glu Tyr Leu Ala Ser Gln Lys Cys
        600                 605                 610

atc cac agg gac ctg gct gcc cgc aat gtg ctg gtg acc gag gac aac    1926
Ile His Arg Asp Leu Ala Ala Arg Asn Val Leu Val Thr Glu Asp Asn
    615                 620                 625

gtg atg aag atc gca gac ttc ggg ctg gcc cgg gac gtg cac aac ctc    1974
Val Met Lys Ile Ala Asp Phe Gly Leu Ala Arg Asp Val His Asn Leu
630                 635                 640                 645

gac tac tac aag aag aca acc aac ggc cgg ctg ccc gtg aag tgg atg    2022
Asp Tyr Tyr Lys Lys Thr Thr Asn Gly Arg Leu Pro Val Lys Trp Met
                650                 655                 660

gcg cct gag gcc ttg ttt gac cga gtc tac act cac cag agt gac gtc    2070
Ala Pro Glu Ala Leu Phe Asp Arg Val Tyr Thr His Gln Ser Asp Val
            665                 670                 675

tgg tcc ttt ggg gtc ctg ctc tgg gag atc ttc acg ctg ggg ggc tcc    2118
Trp Ser Phe Gly Val Leu Leu Trp Glu Ile Phe Thr Leu Gly Gly Ser
        680                 685                 690

ccg tac ccc ggc atc cct gtg gag gag ctc ttc aag ctg ctg aag gag     2166
Pro Tyr Pro Gly Ile Pro Val Glu Glu Leu Phe Lys Leu Leu Lys Glu
    695                 700                 705

ggc cac cgc atg gac aag ccc gcc aac tgc aca cac gac ctg tac atg     2214
Gly His Arg Met Asp Lys Pro Ala Asn Cys Thr His Asp Leu Tyr Met
710                 715                 720                 725

atc atg cgg gag tgc tgg cat gcc gcg ccc tcc cag agg ccc acc ttc     2262
Ile Met Arg Glu Cys Trp His Ala Ala Pro Ser Gln Arg Pro Thr Phe
                730                 735                 740

aag cag ctg gtg gag gac ctg gac cgt gtc ctt acc gtg acg tcc acc     2310
Lys Gln Leu Val Glu Asp Leu Asp Arg Val Leu Thr Val Thr Ser Thr
            745                 750                 755

gac gag tac ctg gac ctg tcg gcg cct ttc gag cag tac tcc ccg ggt     2358
Asp Glu Tyr Leu Asp Leu Ser Ala Pro Phe Glu Gln Tyr Ser Pro Gly
        760                 765                 770

ggc cag gac acc ccc agc tcc agc tcc tca ggg gac gac tcc gtg ttt     2406
Gly Gln Asp Thr Pro Ser Ser Ser Ser Ser Gly Asp Asp Ser Val Phe
    775                 780                 785

gcc cac gac ctg ctg ccc ccg gcc cca ccc agc agt ggg ggc tcg cgg     2454
Ala His Asp Leu Leu Pro Pro Ala Pro Pro Ser Ser Gly Gly Ser Arg
790                 795                 800                 805

acg tgaagggcca ctggtcccca acaatgtgag ggg                            2490
Thr


<210> 58
<211> 806
<212> PRT
<213> Homo sapiens

<400> 58
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly

770                 775                 780

Asp Asp Ser Val Phe Ala His Asp Leu Leu Pro Pro Ala Pro Pro Ser
785                 790                 795                 800

Ser Gly Gly Ser Arg Thr
                805

<210> 59
<211> 2427
<212> DNA
<213> Homo sapiens

<220>
<221> CDS
<222> (1)..(2424)

<400> 59
atg ggc gcc cct gcc tgc gcc ctc gcg ctc tgc gtg gcc gtg gcc atc      48
gtg gcc ggc gcc tcc tcg gag tcc ttg ggg acg gag cag cgc gtc gtg      96
ggg cga gcg gca gaa gtc ccg ggc cca gag ccc ggc cag cag gag cag     144
ttg gtc ttc ggc agc ggg gat gct gtg gag ctg agc tgt ccc ccg ccc     192
ggg ggt ggt ccc atg ggg ccc act gtc tgg gtc aag gat ggc aca ggg     240
ctg gtg ccc tcg gag cgt gtc ctg gtg ggg ccc cag cgg ctg cag gtg     288
ctg aat gcc tcc cac gag gac tcc ggg gcc tac agc tgc cgg cag cgg     336
ctc acg cag cgc gta ctg tgc cac ttc agt gtg cgg gtg aca gac gct     384
cca tcc tcg gga gat gac gaa gac ggg gag gac gag gct gag gac aca     432
ggt gtg gac aca ggg gcc cct tac tgg aca cgg ccc gag cgg atg gac     480
aag aag ctg ctg gcc gtg ccg gcc gcc aac acc gtc cgc ttc cgc tgc     528
cca gcc gct ggc aac ccc act ccc tcc atc tcc tgg ctg aag aac ggc     576
agg gag ttc cgc ggc gag cac cgc att gga ggc atc aag ctg cgg cat     624
cag cag tgg agc ctg gtc atg gaa agc gtg gtg ccc tcg gac cgc ggc     672
aac tac acc tgc gtc gtg gag aac aag ttt ggc agc atc cgg cag acg     720
tac acg ctg gac gtg ctg gag cgc tcc ccg cac cgg ccc atc ctg cag     768
gcg ggg ctg ccg gcc aac cag acg gcg gtg ctg ggc agc gac gtg gag     816
ttc cac tgc aag gtg tac agt gac gca cag ccc cac atc cag tgg ctc     864
aag cac gtg gag gtg aac ggc agc aag gtg ggc ccg gac ggc aca ccc     912
tac gtt acc gtg ctc aag tcc tgg atc agt gag agt gtg gag gcc gac     960
gtg cgc ctc cgc ctg gcc aat gtg tcg gag cgg gac ggg ggc gag tac     1008
ctc tgt cga gcc acc aat ttc ata ggc gtg gcc gag aag gcc ttt tgg     1056
ctg agc gtt cac ggg ccc cga gca gcc gag gag gag ctg gtg gag gct     1104
gac gag gcg ggc agt gtg tat gca ggc atc ctc agc tac ggg gtg ggc     1152
ttc ttc ctg ttc atc ctg gtg gtg gcg gct gtg acg ctc tgc cgc ctg     1200
cgc agc ccc ccc aag aaa ggc ctg ggc tcc ccc acc gtg cac aag atc     1248
tcc cgc ttc ccg ctc aag cga cag gtg tcc ctg gag tcc aac gcg tcc    1296
atg agc tcc aac aca cca ctg gtg cgc atc gca agg ctg tcc tca ggg    1344
gag ggc ccc acg ctg gcc aat gtc tcc gag ctc gag ctg cct gcc gac    1392
ccc aaa tgg gag ctg tct cgg gcc cgg ctg acc ctg ggc aag ccc ctt    1440
ggg gag ggc tgc ttc ggc cag gtg gtc atg gcg gag gcc atc ggc att    1488
gac aag gac cgg gcc gcc aag cct gtc acc gta gcc gtg aag atg ctg    1536
aaa gac gat gcc act gac aag gac ctg tcg gac ctg gtg tct gag atg    1584
gag atg atg aag atg atc ggg aaa cac aaa aac atc atc aac ctg ctg    1632
ggc gcc tgc acg cag ggc ggg ccc ctg tac gtg ctg gtg gag tac gcg    1680
gcc aag ggt aac ctg cgg gag ttt ctg cgg gcg cgg cgg ccc ccg ggc    1728
ctg gac tac tcc ttc gac acc tgc aag ccg ccc gag gag cag ctc acc    1776
ttc aag gac ctg gtg tcc tgt gcc tac cag gtg gcc cgg ggc atg gag    1824
tac ttg gcc tcc cag aag tgc atc cac agg gac ctg gct gcc cgc aat    1872
gtg ctg gtg acc gag gac aac gtg atg aag atc gca gac ttc ggg ctg    1920
gcc cgg gac gtg cac aac ctc gac tac tac aag aag aca acc aac ggc    1968
cgg ctg ccc gtg aag tgg atg gcg cct gag gcc ttg ttt gac cga gtc    2016
tac act cac cag agt gac gtc tgg tcc ttt ggg gtc ctg ctc tgg gag     2064
atc ttc acg ctg ggg ggc tcc ccg tac ccc ggc atc cct gtg gag gag     2112
ctc ttc aag ctg ctg aag gag ggc cac cgc atg gac aag ccc gcc aac     2160
tgc aca cac gac ctg tac atg atc atg cgg gag tgc tgg cat gcc gcg     2208
ccc tcc cag agg ccc acc ttc aag cag ctg gtg gag gac ctg gac cgt     2256
gtc ctt acc gtg acg tcc acc gac gag tac ctg gac ctg tcg gcg cct     2304
ttc gag cag tac tcc ccg ggt ggc cag gac acc ccc agc tcc agc tcc     2352
tca ggg gac gac tcc gtg ttt gcc cac gac ctg ctg ccc ccg gcc cca     2400
ccc agc agt ggg ggc tcg cgg acg tga                                 2427

<210> 60
<211> 808
<212> PRT
<213> Homo sapiens

<400> 60

<210> 61
<211> 2077
<212> DNA
<213> Homo sapiens

<220>
<221> CDS
<222> (1)..(1977)

<400> 61
atg gag cgg tgg cgc gac cgg ctg gcg ctg gtg acg ggg gcc tcg ggg      48
Met Glu Arg Trp Arg Asp Arg Leu Ala Leu Val Thr Gly Ala Ser Gly
1               5                   10                  15

ggc atc ggc gcg gcc gtg gcc cgg gcc ctg gtc cag cag gga ctg aag      96
Gly Ile Gly Ala Ala Val Ala Arg Ala Leu Val Gln Gln Gly Leu Lys
            20                  25                  30

gtg gtg ggc tgc gcc cgc act gtg ggc aac atc gag gag ctg gct gct     144
Val Val Gly Cys Ala Arg Thr Val Gly Asn Ile Glu Glu Leu Ala Ala
        35                  40                  45

gaa tgt aag agt gca ggc tac ccc ggg act ttg atc ccc tac aga tgt     192
Glu Cys Lys Ser Ala Gly Tyr Pro Gly Thr Leu Ile Pro Tyr Arg Cys
    50                  55                  60

gac cta tca aat gaa gag gac atc ctc tcc atg ttc tca gct atc cgt     240
Asp Leu Ser Asn Glu Glu Asp Ile Leu Ser Met Phe Ser Ala Ile Arg
65                  70                  75                  80

tct cag cac agc ggt gta gac atc tgc atc aac aat gct ggc ttg gcc     288
Ser Gln His Ser Gly Val Asp Ile Cys Ile Asn Asn Ala Gly Leu Ala
                85                  90                  95

cgg cct gac acc ctg ctc tca ggc agc acc agt ggt tgg aag gac atg     336
Arg Pro Asp Thr Leu Leu Ser Gly Ser Thr Ser Gly Trp Lys Asp Met
            100                 105                 110

ttc aat gtg aac gtg ctg gcc ctc agc atc tgc aca cgg gaa gcc tac     384
Phe Asn Val Asn Val Leu Ala Leu Ser Ile Cys Thr Arg Glu Ala Tyr
        115                 120                 125

cag tcc atg aag gag cgg aat gtg gac gat ggg cac atc att aac atc     432
Gln Ser Met Lys Glu Arg Asn Val Asp Asp Gly His Ile Ile Asn Ile
    130                 135                 140

aat agc atg tct ggc cac cga gtg tta ccc ctg tct gtg acc cac ttc 480
Asn Ser Met Ser Gly His Arg Val Leu Pro Leu Ser Val Thr His Phe
145                 150                 155                 160

tat agt gcc acc aag tat gcc gtc act gcg ctg aca gag gga ctg agg 528
Tyr Ser Ala Thr Lys Tyr Ala Val Thr Ala Leu Thr Glu Gly Leu Arg
                165                 170                 175

caa gag ctt cgg gag gcc cag acc cac atc cga gcc acg tgg cag ctt 576
Gln Glu Leu Arg Glu Ala Gln Thr His Ile Arg Ala Thr Trp Gln Leu
. 180 185 190

cgg agg gag gag gcc gct gcc gga tat cag gca gcc atc act gtg aag 624
Arg Arg Glu Glu Ala Ala Ala Gly Tyr Gln Ala Ala Ile Thr Val Lys
195 200 205

ctg ggg ttc tgt ggc ctc cat cct ctc ccc tcg acc tcc cca aga cct 672
Leu Gly Phe Cys Gly Leu His Pro Leu Pro Ser Thr Ser Pro Arg Pro
210 215 220

ggc aaa gct cag ccc ctg aga agg ccc tct ctg ttg gcc cag tgc atc 720
Gly Lys Ala Gln Pro Leu Arg Arg Pro Ser Leu Leu Ala Gln Cys Ile
225 230 235 240

tct cca ggt gtg gtg gag aca caa ttc gcc ttc aaa ctc cac gac aag 768
Ser Pro Gly Val Val Glu ,Thr Gln Phe Ala Phe Lys Leu His Asp Lys
245 250 255

gac cct gag aag gca gct gcc acc tat gag caa atg aag tgt ctc aaa 816
Asp Pro Glu Lys Ala Ala Ala Thr Tyr Glu Gln Met Lys Cys Leu Lys
260 265 270

ccc gag gat gtg gcc gag gct gtt atc tac gtc ctc agc acc ccc gca 864
Pro Glu Asp Val Ala Glu Ala Val Ile Tyr Val Leu Ser Thr Pro Ala
275 280 285

cac atc cag att gga gac atc cag atg agg ccc acg gag cag aga gct 912
His Ile Gln Ile Gly Asp Ile Gln Met Arg Pro Thr Glu Gln Arg Ala
290 295 300

cgg cgg aga cgg ctg tcg agt acc ctt cac ctc ggt gtt ggg agc ctg 960
Arg Arg Arg Arg Leu Ser Ser Thr Leu His Leu Gly Val Gly Ser Leu
305 310 315 320

gga gcg aac tgc ggc gcg ggt tac cgc tcc cgg gga cgc agc aag ggg 1008
Gly Ala Asn Cys Gly Ala Gly Tyr Arg Ser Arg Gly Arg Ser Lys Gly
325 330 335

cat cga gtc cct ggc ggg agc tgc gcc atg gca ttg ctc tcg acc gtc 1056
His Arg Val Pro Gly Gly Ser Cys Ala Met Ala Leu Leu Ser Thr Val
340 345 350

cgg ggc gcg acc tgg ggt cgc ctc gtc acc cgt cat ttc tcc cat gca 1104
Arg Gly Ala Thr Trp Gly Arg Leu Val Thr Arg His Phe Ser His Ala
355 360 365

gcg cgg cat ggg gag cgg cct ggt ggg gag gag cta agc cgc ttg ctg 1152
Ala Arg His Gly Glu Arg Pro Gly Gly Glu Glu Leu Ser Arg Leu Leu
370 375 380

ctg gat gac ctg gtg ccg acc tct cgg ctg gag ctt ctg ttt ggc atg 1200

Leu Asp Asp Leu Val Pro Thr Ser Arg Leu Glu Leu Leu Phe Gly Met
385                 390                 395                 400

acc ccg tgt ctc ctg gct ctg cag gcc gcc cgc cgc tct gtg gcc cgg     1248
Thr Pro Cys Leu Leu Ala Leu Gln Ala Ala Arg Arg Ser Val Ala Arg
                405                 410                 415

ctc ctg ctc cag gcg ggt aaa gct ggg ctg cag ggg aag cgg gcc gag     1296
Leu Leu Leu Gln Ala Gly Lys Ala Gly Leu Gln Gly Lys Arg Ala Glu
            420                 425                 430

ctg ctc cgg atg gcc gag gcg cgg gac att cca gtt ctg cgg ccc aga     1344
Leu Leu Arg Met Ala Glu Ala Arg Asp Ile Pro Val Leu Arg Pro Arg
        435                 440                 445

cgg cag aaa ctg gac aca atg tgc cgc tac cag gtc cac cag ggt gtc     1392
Arg Gln Lys Leu Asp Thr Met Cys Arg Tyr Gln Val His Gln Gly Val
    450                 455                 460

tgc atg gag gtg agc ccg ctg cgg ccc cgg cct tgg aga gag gcc ggg     1440
Cys Met Glu Val Ser Pro Leu Arg Pro Arg Pro Trp Arg Glu Ala Gly
465                 470                 475                 480

gag gcg agc cca ggc gac gac ccc cag cag ttg tgg ctc gtc ctc gat     1488
Glu Ala Ser Pro Gly Asp Asp Pro Gln Gln Leu Trp Leu Val Leu Asp
                485                 490                 495

ggg atc cag gat ccc cgg aat ttt ggg gct gtg ctg cgt tcc gca cac     1536
Gly Ile Gln Asp Pro Arg Asn Phe Gly Ala Val Leu Arg Ser Ala His
            500                 505                 510

ttc ctc gga gtg gat aag acc aaa gcc cag cag ggc tgg ctc gtg gcc     1584
Phe Leu Gly Val Asp Lys Thr Lys Ala Gln Gln Gly Trp Leu Val Ala
        515                 520                 525

ggc acg gtg ggc tgc cca agc aca gag gat ccc cag tcc tcc gag atc     1632
Gly Thr Val Gly Cys Pro Ser Thr Glu Asp Pro Gln Ser Ser Glu Ile
    530                 535                 540

ccc atc atg agt tgc ttg gag ttc ctc tgg gaa cgg cct act ctc ctt     1680
Pro Ile Met Ser Cys Leu Glu Phe Leu Trp Glu Arg Pro Thr Leu Leu
545                 550                 555                 560

gtg ctg ggg aat gag ggc tca ggt cta tcc cag gag gtg cag gcc tcc     1728
Val Leu Gly Asn Glu Gly Ser Gly Leu Ser Gln Glu Val Gln Ala Ser
                565                 570                 575

tgc cag ctt ctc ctc acc atc ctg ccc cgg cgc cag ctg cct cct gga     1776
Cys Gln Leu Leu Leu Thr Ile Leu Pro Arg Arg Gln Leu Pro Pro Gly
            580                 585                 590

ctt gag tcc ttg aac gtc tct gtg gct gca gga att ctt ctt cac tcc     1824
Leu Glu Ser Leu Asn Val Ser Val Ala Ala Gly Ile Leu Leu His Ser
        595                 600                 605

att tgc agc cag agg aag ggt ttc ccc aca gag ggg gag aga agg cag     1872
Ile Cys Ser Gln Arg Lys Gly Phe Pro Thr Glu Gly Glu Arg Arg Gln
    610                 615                 620

ctt ctc caa gac ccc caa gaa ccc tca gcc agg tct gaa ggg ctc agc     1920
Leu Leu Gln Asp Pro Gln Glu Pro Ser Ala Arg Ser Glu Gly Leu Ser

625                 630                 635                 640

atg gct cag cac cca ggg ctg tct tca ggc cca gag aaa gag agg caa    1968
Met Ala Gln His Pro Gly Leu Ser Ser Gly Pro Glu Lys Glu Arg Gln
                645                 650                 655

aat gag ggc tgacgtggac tgtccacagt gttcatgtgc tggagtcagg            2017
Asn Glu Gly

gacggccgca cctgcctccg ccggctccag tgtgcgggga gcctctgcct gagtgtgcac 2077

<210> 62
<211> 659
<212> PRT
<213> Homo sapiens

<400> 62
Met Glu Arg Trp Arg Asp Arg Leu Ala Leu Val Thr Gly Ala Ser Gly
1               5                   10                  15

Gly Ile Gly Ala Ala Val Ala Arg Ala Leu Val Gln Gln Gly Leu Lys
            20                  25                  30

Val Val Gly Cys Ala Arg Thr Val Gly Asn Ile Glu Glu Leu Ala Ala
        35                  40                  45

Glu Cys Lys Ser Ala Gly Tyr Pro Gly Thr Leu Ile Pro Tyr Arg Cys
    50                  55                  60

Asp Leu Ser Asn Glu Glu Asp Ile Leu Ser Met Phe Ser Ala Ile Arg
65                  70                  75                  80

Ser Gln His Ser Gly Val Asp Ile Cys Ile Asn Asn Ala Gly Leu Ala
                85                  90                  95

Arg Pro Asp Thr Leu Leu Ser Gly Ser Thr Ser Gly Trp Lys Asp Met
            100                 105                 110

Phe Asn Val Asn Val Leu Ala Leu Ser Ile Cys Thr Arg Glu Ala Tyr
        115                 120                 125

Gln Ser Met Lys Glu Arg Asn Val Asp Asp Gly His Ile Ile Asn Ile
    130                 135                 140

Asn Ser Met Ser Gly His Arg Val Leu Pro Leu Ser Val Thr His Phe
145                 150                 155                 160

Tyr Ser Ala Thr Lys Tyr Ala Val Thr Ala Leu Thr Glu Gly Leu Arg
                165                 170                 175

Gln Glu Leu Arg Glu Ala Gln Thr His Ile Arg Ala Thr Trp Gln Leu
            180                 185                 190

Arg Arg Glu Glu Ala Ala Ala Gly Tyr Gln Ala Ala Ile Thr Val Lys
        195                 200                 205

Leu Gly Phe Cys Gly Leu His Pro Leu Pro Ser Thr Ser Pro Arg Pro
    210                 215                 220

Gly Lys Ala Gln Pro Leu Arg Arg Pro Ser Leu Leu Ala Gln Cys Ile

225                 230                 235                 240

Ser Pro Gly Val Val Glu Thr Gln Phe Ala Phe Lys Leu His Asp Lys
                245                 250                 255

Asp Pro Glu Lys Ala Ala Ala Thr Tyr Glu Gln Met Lys Cys Leu Lys
            260                 265                 270

Pro Glu Asp Val Ala Glu Ala Val Ile Tyr Val Leu Ser Thr Pro Ala
        275                 280                 285

His Ile Gln Ile Gly Asp Ile Gln Met Arg Pro Thr Glu Gln Arg Ala
    290                 295                 300

Arg Arg Arg Arg Leu Ser Ser Thr Leu His Leu Gly Val Gly Ser Leu
305                 310                 315                 320

Gly Ala Asn Cys Gly Ala Gly Tyr Arg Ser Arg Gly Arg Ser Lys Gly
                325                 330                 335

His Arg Val Pro Gly Gly Ser Cys Ala Met Ala Leu Leu Ser Thr Val
            340                 345                 350

Arg Gly Ala Thr Trp Gly Arg Leu Val Thr Arg His Phe Ser His Ala
        355                 360                 365

Ala Arg His Gly Glu Arg Pro Gly Gly Glu Glu Leu Ser Arg Leu Leu
    370                 375                 380

Leu Asp Asp Leu Val Pro Thr Ser Arg Leu Glu Leu Leu Phe Gly Met
385                 390                 395                 400

Thr Pro Cys Leu Leu Ala Leu Gln Ala Ala Arg Arg Ser Val Ala Arg
                405                 410                 415

Leu Leu Leu Gln Ala Gly Lys Ala Gly Leu Gln Gly Lys Arg Ala Glu
            420                 425                 430

Leu Leu Arg Met Ala Glu Ala Arg Asp Ile Pro Val Leu Arg Pro Arg
        435                 440                 445

Arg Gln Lys Leu Asp Thr Met Cys Arg Tyr Gln Val His Gln Gly Val
    450                 455                 460

Cys Met Glu Val Ser Pro Leu Arg Pro Arg Pro Trp Arg Glu Ala Gly
465                 470                 475                 480

Glu Ala Ser Pro Gly Asp Asp Pro Gln Gln Leu Trp Leu Val Leu Asp
                485                 490                 495

Gly Ile Gln Asp Pro Arg Asn Phe Gly Ala Val Leu Arg Ser Ala His
            500                 505                 510

Phe Leu Gly Val Asp Lys Thr Lys Ala Gln Gln Gly Trp Leu Val Ala
        515                 520                 525

Gly Thr Val Gly Cys Pro Ser Thr Glu Asp Pro Gln Ser Ser Glu Ile
    530                 535                 540

Pro Ile Met Ser Cys Leu Glu Phe Leu Trp Glu Arg Pro Thr Leu Leu
545                 550                 555                 560

Val Leu Gly Asn Glu Gly Ser Gly Leu Ser Gln Glu Val Gln Ala Ser
                565                 570                 575

Cys Gln Leu Leu Leu Thr Ile Leu Pro Arg Arg Gln Leu Pro Pro Gly
            580                 585                 590

Leu Glu Ser Leu Asn Val Ser Val Ala Ala Gly Ile Leu Leu His Ser
        595                 600                 605

Ile Cys Ser Gln Arg Lys Gly Phe Pro Thr Glu Gly Glu Arg Arg Gln
    610                 615                 620

Leu Leu Gln Asp Pro Gln Glu Pro Ser Ala Arg Ser Glu Gly Leu Ser
625                 630                 635                 640

Met Ala Gln His Pro Gly Leu Ser Ser Gly Pro Glu Lys Glu Arg Gln
                645                 650                 655

Asn Glu Gly

<210> 63
<211> 736
<212> DNA
<213> Homo sapiens

<220>
<221> CDS
<222> (13)..(696)

<400> 63
aaagcacccg ag atg acc ccg gct cct cca cca gga gcg cgg ccg ggc gcg   51
              Met Thr Pro Ala Pro Pro Pro Gly Ala Arg Pro Gly Ala
              1               5                   10

gcg tcc cta gcg ggc ttc gcc ggg gtg gcg tct ctg ggg cct ggg gac     99
Ala Ser Leu Ala Gly Phe Ala Gly Val Ala Ser Leu Gly Pro Gly Asp
    15                  20                  25

ccc cgc cgc gcc gct gac ccg cgc cct ctg ccc cca gcg ctg tgc ttc    147
Pro Arg Arg Ala Ala Asp Pro Arg Pro Leu Pro Pro Ala Leu Cys Phe
30                  35                  40                  45

gcc gtg agc cgc tcg ctg ctg ctg acg tgc ctg gtg ccg gcc gcg ctg    195
Ala Val Ser Arg Ser Leu Leu Leu Thr Cys Leu Val Pro Ala Ala Leu
                50                  55                  60

ctg ggc ctg cgc tac tac tac agc cgc aag gtg atc cgc gcc tac ctg    243
Leu Gly Leu Arg Tyr Tyr Tyr Ser Arg Lys Val Ile Arg Ala Tyr Leu
            65                  70                  75

gag tgc gcg ctg cac acg gac atg gcg gac atc gag cag tac tac atg    291
Glu Cys Ala Leu His Thr Asp Met Ala Asp Ile Glu Gln Tyr Tyr Met
        80                  85                  90

aag ccg ccc ggt gtg tcc ctg acc gcc cta tcc cct gca ggc tcc tgc    339
Lys Pro Pro Gly Val Ser Leu Thr Ala Leu Ser Pro Ala Gly Ser Cys
    95                  100                 105

ttc tgg gtg gcc gtg ctg gat ggc aac gtg gtg ggc att gtg gct gca    387

Phe Trp Val Ala Val Leu Asp Gly Asn Val Val Gly Ile Val Ala Ala
110                 115                 120                 125

cgg gcc cac gag gag gac aac acg gtg gag ctg ctg cgg atg tct gtg     435
Arg Ala His Glu Glu Asp Asn Thr Val Glu Leu Leu Arg Met Ser Val
                130                 135                 140

gac tca cgt ttc cga ggc aag ggc atc gcc aag gcg ctg ggc cgg aag     483
Asp Ser Arg Phe Arg Gly Lys Gly Ile Ala Lys Ala Leu Gly Arg Lys
            145                 150                 155

gtg ctg gag ttc gcc gtg gtg cac aac tac tcc gcg gtg gtg ctg ggc     531
Val Leu Glu Phe Ala Val Val His Asn Tyr Ser Ala Val Val Leu Gly
        160                 165                 170

acg acg gcc gtc aag gtg gcc gcc cac aag ctc tac gag tcg ctg ggc     579
Thr Thr Ala Val Lys Val Ala Ala His Lys Leu Tyr Glu Ser Leu Gly
    175                 180                 185

ttc aga cac atg ggc gcc agt gac cac tac gtg ctg ccg ggc atg acc     627
Phe Arg His Met Gly Ala Ser Asp His Tyr Val Leu Pro Gly Met Thr
190                 195                 200                 205

ctc tcg ctg gct gag cgc ctc ttc ttc cag gtc cgc tac cac cgc tac     675
Leu Ser Leu Ala Glu Arg Leu Phe Phe Gln Val Arg Tyr His Arg Tyr
                210                 215                 220

cgc ctg cag ctg cgc gag gag tgaccgccgc cgctcgcccg cccgcccccc     726
Arg Leu Gln Leu Arg Glu Glu
                225

cggccgccct                                                          736

<210> 64
<211> 228
<212> PRT
<213> Homo sapiens

<400> 64
Met Thr Pro Ala Pro Pro Pro Gly Ala Arg Pro Gly Ala Ala Ser Leu
  1               5                  10                  15

Ala Gly Phe Ala Gly Val Ala Ser Leu Gly Pro Gly Asp Pro Arg Arg
             20                  25                  30

Ala Ala Asp Pro Arg Pro Leu Pro Pro Ala Leu Cys Phe Ala Val Ser
         35                  40                  45

Arg Ser Leu Leu Leu Thr Cys Leu Val Pro Ala Ala Leu Leu Gly Leu
     50                  55                  60

Arg Tyr Tyr Tyr Ser Arg Lys Val Ile Arg Ala Tyr Leu Glu Cys Ala
 65                  70                  75                  80

Leu His Thr Asp Met Ala Asp Ile Glu Gln Tyr Tyr Met Lys Pro Pro
                 85                  90                  95

Gly Val Ser Leu Thr Ala Leu Ser Pro Ala Gly Ser Cys Phe Trp Val
            100                 105                 110

Ala Val Leu Asp Gly Asn Val Val Gly Ile Val Ala Ala Arg Ala His

        115                 120                 125

Glu Glu Asp Asn Thr Val Glu Leu Leu Arg Met Ser Val Asp Ser Arg
    130                 135                 140

Phe Arg Gly Lys Gly Ile Ala Lys Ala Leu Gly Arg Lys Val Leu Glu
145                 150                 155                 160

Phe Ala Val Val His Asn Tyr Ser Ala Val Val Leu Gly Thr Thr Ala
                165                 170                 175

Val Lys Val Ala Ala His Lys Leu Tyr Glu Ser Leu Gly Phe Arg His
            180                 185                 190

Met Gly Ala Ser Asp His Tyr Val Leu Pro Gly Met Thr Leu Ser Leu
        195                 200                 205

Ala Glu Arg Leu Phe Phe Gln Val Arg Tyr His Arg Tyr Arg Leu Gln
    210                 215                 220

Leu Arg Glu Glu
225

<210> 65
<211> 1748
<212> DNA
<213> Homo sapiens

<220>
<221> CDS
<222> (98)..(1648)

<400> 65
gttcacccca agactaagtt ctttcccaag ttagagaaga agagagaaag caaaaagaag 60

agaggaaagt tctcccttcc cctcctccgt gcctgtc atg tcc tct aag cca gag 115
Met Ser Ser Lys Pro Glu
1               5

ccg aag gac gtc cac caa ctg aac ggg act ggc cct tct gcc tct ccc 163
Pro Lys Asp Val His Gln Leu Asn Gly Thr Gly Pro Ser Ala Ser Pro
            10                  15                  20

tgc tct tca gat ggc cca ggg aga gag ccc ttg gct ggg acc tca gag 211
Cys Ser Ser Asp Gly Pro Gly Arg Glu Pro Leu Ala Gly Thr Ser Glu
        25                  30                  35

ttc ctg ggg cct gat ggg gct ggg gta gag gtg gtg att gag tct cgg 259
Phe Leu Gly Pro Asp Gly Ala Gly Val Glu Val Val Ile Glu Ser Arg
    40                  45                  50

gcc aac gcc aag ggg gtt cgg gag gag gac gcc ctg ctg gag aac ggg 307
Ala Asn Ala Lys Gly Val Arg Glu Glu Asp Ala Leu Leu Glu Asn Gly
55                  60                  65                  70

agc cag agc aac gaa agt gac gac gtc agc aca gac cgt ggc cct gcg 355
Ser Gln Ser Asn Glu Ser Asp Asp Val Ser Thr Asp Arg Gly Pro Ala
                75                  80                  85

cca cct tcc ccg ctc aag gag acc tcc ttt tcc atc ggg ctg caa gta 403
Pro Pro Ser Pro Leu Lys Glu Thr Ser Phe Ser Ile Gly Leu Gln Val

90                  95                  100

ctg ttt cca ttc ctc ctg gca ggc ttt ggg acc gtg gct gct ggc atg     451
Leu Phe Pro Phe Leu Leu Ala Gly Phe Gly Thr Val Ala Ala Gly Met
        105                 110                 115

gtg ttg gac atc gtg cag cac tgg gaa gtc ttc cag aag gtg aca gag     499
Val Leu Asp Ile Val Gln His Trp Glu Val Phe Gln Lys Val Thr Glu
    120                 125                 130

gtc ttc atc cta gtg cct gcg ctg ctg ggg ctc aaa ggg aac ctg gaa     547
Val Phe Ile Leu Val Pro Ala Leu Leu Gly Leu Lys Gly Asn Leu Glu
135                 140                 145                 150

atg acc ctg gca tca agg ctt tcc act gca gcg agt atc aac att gga     595
Met Thr Leu Ala Ser Arg Leu Ser Thr Ala Ala Ser Ile Asn Ile Gly
                155                 160                 165

cac atg gac aca ccc aag gag ctc tgg cgg atg atc act ggg aac atg     643
His Met Asp Thr Pro Lys Glu Leu Trp Arg Met Ile Thr Gly Asn Met
            170                 175                 180

gcc ctc atc cag gtg cag gcc acg gtg gtg ggc ttc ctg gcg tcc atc     691
Ala Leu Ile Gln Val Gln Ala Thr Val Val Gly Phe Leu Ala Ser Ile
        185                 190                 195

gca gcc gtc gtc ttt ggc tgg atc cct gat ggc cac ttc agt att ccg     739
Ala Ala Val Val Phe Gly Trp Ile Pro Asp Gly His Phe Ser Ile Pro
    200                 205                 210

cac gcc ttc ctg ctc tgt gct agc agc gtg gcc aca gcc ttc att gcc     787
His Ala Phe Leu Leu Cys Ala Ser Ser Val Ala Thr Ala Phe Ile Ala
215                 220                 225                 230

tcc ctg gta ctg ggt atg atc atg att gga gtc atc att ggc tct cgc     835
Ser Leu Val Leu Gly Met Ile Met Ile Gly Val Ile Ile Gly Ser Arg
                235                 240                 245

aag att ggg atc aac cca gac aat gtg gcc aca ccc att gct gcc agc     883
Lys Ile Gly Ile Asn Pro Asp Asn Val Ala Thr Pro Ile Ala Ala Ser
            250                 255                 260

ctg ggc gac ctc atc acc ttg gcg ctg ctc tca ggc atc agc tgg gga     931
Leu Gly Asp Leu Ile Thr Leu Ala Leu Leu Ser Gly Ile Ser Trp Gly
        265                 270                 275

ctc ctg acc tct gcc ctc tca gat cac tgg cga tac atc tac cca ctg     979
Leu Leu Thr Ser Ala Leu Ser Asp His Trp Arg Tyr Ile Tyr Pro Leu
    280                 285                 290

gtg tgt gct ttc ttt gtg gcc ctg ctg cct gtc tgg gtg gtg ctg gcc     1027
Val Cys Ala Phe Phe Val Ala Leu Leu Pro Val Trp Val Val Leu Ala
295                 300                 305                 310

cga cga agt cca gcc aca agg gag gtg ttg tac tcg ggc tgg gag cct     1075
Arg Arg Ser Pro Ala Thr Arg Glu Val Leu Tyr Ser Gly Trp Glu Pro
                315                 320                 325

gtt atc att gcc atg gcc atc agc agt gtg gga ggc ctc atc ttg gac     1123
Val Ile Ile Ala Met Ala Ile Ser Ser Val Gly Gly Leu Ile Leu Asp
            330                 335                 340

aag act gtc tca gac ccc aac ttt gct ggg atg gct gtc ttc acg cct    1171
Lys Thr Val Ser Asp Pro Asn Phe Ala Gly Met Ala Val Phe Thr Pro
        345                 350                 355

gtg att aat ggt gtt ggg ggc aat ctg gtg gca gtg cag gcc agc cgc    1219
Val Ile Asn Gly Val Gly Gly Asn Leu Val Ala Val Gln Ala Ser Arg
    360                 365                 370

atc tcc acc ttc ctg cac atg aat gga atg ccc gga gag aac tct gag    1267
Ile Ser Thr Phe Leu His Met Asn Gly Met Pro Gly Glu Asn Ser Glu
375                 380                 385                 390

caa gct cct cgc cgc tgt ccc agt cct tgt acc acc ttc ttc agc cct    1315
Gln Ala Pro Arg Arg Cys Pro Ser Pro Cys Thr Thr Phe Phe Ser Pro
                395                 400                 405

ggt gtg aat tct cgc tca gcc cgg gtc ctc ttc ctc ctc gtg gtc cca    1363
Gly Val Asn Ser Arg Ser Ala Arg Val Leu Phe Leu Leu Val Val Pro
            410                 415                 420

gga cac ctg gtg ttc ctc tac acc atc agc tgt atg cag ggc ggg cac    1411
Gly His Leu Val Phe Leu Tyr Thr Ile Ser Cys Met Gln Gly Gly His
        425                 430                 435

acc acc ctc aca ctc atc ttc atc atc ttc tat atg aca gct gca ctg    1459
Thr Thr Leu Thr Leu Ile Phe Ile Ile Phe Tyr Met Thr Ala Ala Leu
    440                 445                 450

ctc cag gtg ctg att ctc ctg tac atc gca gac tgg atg gtg cac tgg    1507
Leu Gln Val Leu Ile Leu Leu Tyr Ile Ala Asp Trp Met Val His Trp
455                 460                 465                 470

atg tgg ggc cgg ggc ctg gac ccg gac aac ttc tcc atc cca tac ttg    1555
Met Trp Gly Arg Gly Leu Asp Pro Asp Asn Phe Ser Ile Pro Tyr Leu
                475                 480                 485

act gct ctg ggg gac ctg ctt ggc act ggg ctc cta gca ctc agc ttc    1603
Thr Ala Leu Gly Asp Leu Leu Gly Thr Gly Leu Leu Ala Leu Ser Phe
            490                 495                 500

cat gtt ctc tgg ctc ata ggg gac cga gac acg gat gtc ggg gac        1648
His Val Leu Trp Leu Ile Gly Asp Arg Asp Thr Asp Val Gly Asp
        505                 510                 515

tagcttggtc actcaacatt ttccccatcc ctctgcactt tctatttgaa atttttcttt 1708

tgttcccctg tccctcctcc accccacact cccacctctt                        1748

<210> 66
<211> 517
<212> PRT
<213> Homo sapiens

<400> 66
Met Ser Ser Lys Pro Glu Pro Lys Asp Val His Gln Leu Asn Gly Thr
1               5                   10                  15

Gly Pro Ser Ala Ser Pro Cys Ser Ser Asp Gly Pro Gly Arg Glu Pro
            20                  25                  30

Leu Ala Gly Thr Ser Glu Phe Leu Gly Pro Asp Gly Ala Gly Val Glu
        35                  40                  45

Val Val Ile Glu Ser Arg Ala Asn Ala Lys Gly Val Arg Glu Glu Asp
    50                  55                  60

Ala Leu Leu Glu Asn Gly Ser Gln Ser Asn Glu Ser Asp Asp Val Ser
65                  70                  75                  80

Thr Asp Arg Gly Pro Ala Pro Pro Ser Pro Leu Lys Glu Thr Ser Phe
                85                  90                  95

Ser Ile Gly Leu Gln Val Leu Phe Pro Phe Leu Leu Ala Gly Phe Gly
            100                 105                 110

Thr Val Ala Ala Gly Met Val Leu Asp Ile Val Gln His Trp Glu Val
        115                 120                 125

Phe Gln Lys Val Thr Glu Val Phe Ile Leu Val Pro Ala Leu Leu Gly
    130                 135                 140

Leu Lys Gly Asn Leu Glu Met Thr Leu Ala Ser Arg Leu Ser Thr Ala
145                 150                 155                 160

Ala Ser Ile Asn Ile Gly His Met Asp Thr Pro Lys Glu Leu Trp Arg
                165                 170                 175

Met Ile Thr Gly Asn Met Ala Leu Ile Gln Val Gln Ala Thr Val Val
            180                 185                 190

Gly Phe Leu Ala Ser Ile Ala Ala Val Val Phe Gly Trp Ile Pro Asp
        195                 200                 205

Gly His Phe Ser Ile Pro His Ala Phe Leu Leu Cys Ala Ser Ser Val
    210                 215                 220

Ala Thr Ala Phe Ile Ala Ser Leu Val Leu Gly Met Ile Met Ile Gly
225                 230                 235                 240

Val Ile Ile Gly Ser Arg Lys Ile Gly Ile Asn Pro Asp Asn Val Ala
                245                 250                 255

Thr Pro Ile Ala Ala Ser Leu Gly Asp Leu Ile Thr Leu Ala Leu Leu
            260                 265                 270

Ser Gly Ile Ser Trp Gly Leu Leu Thr Ser Ala Leu Ser Asp His Trp
        275                 280                 285

Arg Tyr Ile Tyr Pro Leu Val Cys Ala Phe Phe Val Ala Leu Leu Pro
    290                 295                 300

Val Trp Val Val Leu Ala Arg Arg Ser Pro Ala Thr Arg Glu Val Leu
305                 310                 315                 320

Tyr Ser Gly Trp Glu Pro Val Ile Ile Ala Met Ala Ile Ser Ser Val
                325                 330                 335

Gly Gly Leu Ile Leu Asp Lys Thr Val Ser Asp Pro Asn Phe Ala Gly
            340                 345                 350

Met Ala Val Phe Thr Pro Val Ile Asn Gly Val Gly Gly Asn Leu Val

355                 360                 365

Ala Val Gln Ala Ser Arg Ile Ser Thr Phe Leu His Met Asn Gly Met
    370                 375                 380

Pro Gly Glu Asn Ser Glu Gln Ala Pro Arg Arg Cys Pro Ser Pro Cys
385                 390                 395                 400

Thr Thr Phe Phe Ser Pro Gly Val Asn Ser Arg Ser Ala Arg Val Leu
                405                 410                 415

Phe Leu Leu Val Val Pro Gly His Leu Val Phe Leu Tyr Thr Ile Ser
            420                 425                 430

Cys Met Gln Gly Gly His Thr Thr Leu Thr Leu Ile Phe Ile Ile Phe
        435                 440                 445

Tyr Met Thr Ala Ala Leu Leu Gln Val Leu Ile Leu Leu Tyr Ile Ala
    450                 455                 460

Asp Trp Met Val His Trp Met Trp Gly Arg Gly Leu Asp Pro Asp Asn
465                 470                 475                 480

Phe Ser Ile Pro Tyr Leu Thr Ala Leu Gly Asp Leu Leu Gly Thr Gly
                485                 490                 495

Leu Leu Ala Leu Ser Phe His Val Leu Trp Leu Ile Gly Asp Arg Asp
            500                 505                 510

Thr Asp Val Gly Asp
        515

<210> 67
<211> 1899
<212> DNA
<213> Homo sapiens

<220>
<221> CDS
<222> (31)..(1878)

<400> 67
cgtctcgccc gccagtctcc ctcccgcgcg atg gcc tcg gcg ctg agc tat gtc     54
tcc aag ttc aag tcc ttc gtg atc ttg ttc gtc acc ccg ctc ctg ctg     102
ctg cca ctc gtc att ctg atg ccc gcc aag gtc agt tgc tgt gcc tac     150
gtc atc atc ctc atg gcc att tac tgg tgc aca gaa gtc atc cct ctg     198
gct gtc acc tct ctc atg cct gtc ttg ctt ttc cca ctc ttc cag att     246
ctg gac tcc agg cag gtg tgt gtc cag tac atg aag gac acc aac atg 294
ctg ttc ctg ggc ggc ctc atc gtg gcc gtg gct gtg gag cgc tgg aac 342
ctg cac aag agg atc gcc ctg cgc acg ctc ctc tgg gtg ggg gcc aag 390
cct gca cgg ctg atg ctg ggc ttc atg ggc gtc aca gcc ctc ctg tcc 438
atg tgg atc agt aac acg gca acc acg gcc atg atg gtg ccc atc gtg 486
gag gcc ata ttg cag cag atg gaa gcc aca agc gca gcc acc gag gcc 534
ggc ctg gag gga caa ggt acc aca ata aac aac ctg aat gca ctg gag 582
gat gat aca gtg aaa gca gta cta gga gga aag tgt gta gct ata ata 630
agc act tac gtc aaa aaa gta gaa aaa ctt caa ata aac aat cta atg 678
aca cct ctt aaa aaa cta gaa aag caa gag caa cag gac cta ggg cct 726
ggc atc agg cct cag gac tct gcc cag tgc cag gaa gac caa gag cgg 774
aag agg ttg tgt aag gcc atg acc ctg tgc atc tgc tac gcg gcc agc 822
atc ggg ggc acc gcc acc ctg acc ggg acg gga ccc aac gtg gtg ctc 870
ctg ggc cag atg aac gag ttg ttt cct gac agc aag gac ctc gtg aac 918
ttt gct tcc tgg ttt gca ttt gcc ttt ccc aac atg ctg gtg atg ctg 966
ctg ttc gcc tgg ctg tgg ctc cag ttt gtt tac atg ttc tcc agt ttt 1014
aaa aag tcc tgg ggc tgc ggg cta gag agc aag aaa aac gag aag gct 1062
gcc ctc aag gtg ctg cag gag gag tac cgg aag ctg ggg ccc ttg tcc 1110
ttc gcg gag atc aac gtg ctg atc tgc ttc ttc ctg ctg gtc atc ctg 1158
tgg ttc tcc cga gac ccc ggc ttc atg ccc ggc tgg ctg act gtt gcc 1206
tgg gtg gag ggt gag aca aag tca gtc tcc gat gcc act gtg gcc atc 1254
ttt gtg gcc acc ctg cta ttc att gtg cct tca cag aag ccc aag ttt 1302
aac ttc cgc agc cag act gag gaa ggt aag tct cct gtt ctg atc gcc 1350
ccc cct ccc ctg ctg gat tgg aag gta acc cag gag aaa gtg ccc tgg 1398
ggc atc gtg ctg cta cta ggg ggc gga ttt gct ctg gct aaa gga tcc 1446
gag gcc tcg ggg ctg tcc gtg tgg atg ggg aag cag atg gag ccc ttg 1494
cac gca gtg ccc ccg gca gcc atc acc ttg atc ttg tcc ttg ctc gtt 1542
gcc gtg ttc act gag tgc aca agc aac gtg gcc acc acc acc ttg ttc 1590
ctg ccc atc ttt gcc tcc atg tct cgc tcc atc ggc ctc aat ccg ctg 1638
tac atc atg ctg ccc tgt acc ctg agt gcc tcc ttt gcc ttc atg ttg 1686
cct gtg gcc acc cct cca aat gcc atc gtg ttc acc tat ggg cac ctc 1734
aag gtt gct gac atg gta aaa aca gga gtc ata atg aac ata att gga 1782
gtc ttc tgt gtg ttt ttg gct gtc aac acc tgg gga cgg gcc ata ttt 1830
gac ttg gat cat ttc cct gac tgg gct aat gtg aca cat att gag act 1878
taggaagagc cacaagacca c 1899

<210> 68
<211> 616
<212> PRT
<213> Homo sapiens

<400> 68

<210> 69
<211> 1790
<212> DNA
<213> Homo sapiens

<220>
<221> CDS
<222> (16)..(1719)

<400> 69
tctccctccc gcgcg atg gcc tcg gcg ctg agc tat gtc tcc aag ttc aag      51
                 Met Ala Ser Ala Leu Ser Tyr Val Ser Lys Phe Lys
                 1               5                   10

tcc ttc gtg atc ttg ttc gtc acc ccg ctc ctg ctg ctg cca ctc gtc      99
Ser Phe Val Ile Leu Phe Val Thr Pro Leu Leu Leu Leu Pro Leu Val
        15                  20                  25

att ctg atg ccc gcc aag gtc agt tgt gcc tac gtc atc atc ctc atg     147
Ile Leu Met Pro Ala Lys Val Ser Cys Ala Tyr Val Ile Ile Leu Met
    30                  35                  40

gcc att tac tgg tgc aca gaa gtc atc cct ctg gct gtc acc tct ctc     195
Ala Ile Tyr Trp Cys Thr Glu Val Ile Pro Leu Ala Val Thr Ser Leu
45                  50                  55                  60

atg cct gtc ttg ctt ttc cca ctc ttc cag att ctg gac tcc agg cag     243
Met Pro Val Leu Leu Phe Pro Leu Phe Gln Ile Leu Asp Ser Arg Gln
                65                  70                  75

gtg tgt gtc cag tac atg aag gac acc aac atg ctg ttc ctg ggc ggc     291
Val Cys Val Gln Tyr Met Lys Asp Thr Asn Met Leu Phe Leu Gly Gly
            80                  85                  90

ctc atc gtg gcc gtg gct gtg gag cgc tgg aac ctg cac aag agg atc     339
Leu Ile Val Ala Val Ala Val Glu Arg Trp Asn Leu His Lys Arg Ile
        95                  100                 105

gcc ctg cgc acg ctc ctc tgg gtg ggg gcc aag cct gca cgg ctg atg     387
Ala Leu Arg Thr Leu Leu Trp Val Gly Ala Lys Pro Ala Arg Leu Met
    110                 115                 120

ctg ggc ttc atg ggc gtc aca gcc ctc ctg tcc atg tgg atc agt aac     435

Leu Gly Phe Met Gly Val Thr Ala Leu Leu Ser Met Trp Ile Ser Asn
125                 130                 135                 140

acg gca acc acg gcc atg atg gtg ccc atc gtg gag gcc ata ttg cag     483
Thr Ala Thr Thr Ala Met Met Val Pro Ile Val Glu Ala Ile Leu Gln
                145                 150                 155

cag atg gaa gcc aca agc gca gcc acc gag gcc ggc ctg gag ctg gtg     531
Gln Met Glu Ala Thr Ser Ala Ala Thr Glu Ala Gly Leu Glu Leu Val
            160                 165                 170

gac aag ggc aag gcc aag gag ctg cca ggg agt caa gtg att ttt gaa     579
Asp Lys Gly Lys Ala Lys Glu Leu Pro Gly Ser Gln Val Ile Phe Glu
        175                 180                 185

ggc ccc act ctg ggg cag cag gaa gac caa gag cgg aag agg ttg tgt     627
Gly Pro Thr Leu Gly Gln Gln Glu Asp Gln Glu Arg Lys Arg Leu Cys
    190                 195                 200

aag gcc atg acc ctg tgc atc tgc tac gcg gcc agc atc ggg ggc acc     675
Lys Ala Met Thr Leu Cys Ile Cys Tyr Ala Ala Ser Ile Gly Gly Thr
205                 210                 215                 220

gcc acc ctg acc ggg acg gga ccc aac gtg gtg ctc ctg ggc cag atg     723
Ala Thr Leu Thr Gly Thr Gly Pro Asn Val Val Leu Leu Gly Gln Met
                225                 230                 235

aac gag ttg ttt cct gac agc aag gac ctc gtg aac ttt gct tcc tgg     771
Asn Glu Leu Phe Pro Asp Ser Lys Asp Leu Val Asn Phe Ala Ser Trp
            240                 245                 250

ttt gca ttt gcc ttt ccc aac atg ctg gtg atg ctg ctg ttc gcc tgg     819
Phe Ala Phe Ala Phe Pro Asn Met Leu Val Met Leu Leu Phe Ala Trp
        255                 260                 265

ctg tgg ctc cag ttt gtt tac atg ttc tcc agt ttt aaa aag tcc tgg     867
Leu Trp Leu Gln Phe Val Tyr Met Phe Ser Ser Phe Lys Lys Ser Trp
    270                 275                 280

ggc tgc ggg cta gag agc aag aaa aac gag aag gct gcc ctc aag gtg     915
Gly Cys Gly Leu Glu Ser Lys Lys Asn Glu Lys Ala Ala Leu Lys Val
285                 290                 295                 300

ctg cag gag gag tac cgg aag ctg ggg ccc ttg tcc ttc gcg gag atc     963
Leu Gln Glu Glu Tyr Arg Lys Leu Gly Pro Leu Ser Phe Ala Glu Ile
                305                 310                 315

aac gtg ctg atc tgc ttc ttc ctg ctg gtc atc ctg tgg ttc tcc cga     1011
Asn Val Leu Ile Cys Phe Phe Leu Leu Val Ile Leu Trp Phe Ser Arg
            320                 325                 330

gac ccc ggc ttc atg ccc ggc tgg ctg act gtt gcc tgg gtg gag ggt     1059
Asp Pro Gly Phe Met Pro Gly Trp Leu Thr Val Ala Trp Val Glu Gly
        335                 340                 345

gag aca aag tat gtc tcc gat gcc act gtg gcc atc ttt gtg gcc acc     1107
Glu Thr Lys Tyr Val Ser Asp Ala Thr Val Ala Ile Phe Val Ala Thr
    350                 355                 360

ctg cta ttc att gtg cct tca cag aag ccc aag ttt aac ttc cgc agc     1155
Leu Leu Phe Ile Val Pro Ser Gln Lys Pro Lys Phe Asn Phe Arg Ser

365                 370                 375                 380

cag act gag gaa ggt aag tct cct gtt ctg atc gcc ccc cct ccc ctg     1203
Gln Thr Glu Glu Gly Lys Ser Pro Val Leu Ile Ala Pro Pro Pro Leu
                385                 390                 395

ctg gat tgg aag gta acc cag gag aaa gtg ccc tgg ggc atc gtg ctg     1251
Leu Asp Trp Lys Val Thr Gln Glu Lys Val Pro Trp Gly Ile Val Leu
            400                 405                 410

cta cta ggg ggc gga ttt gct ctg gct aaa gga tcc gag gcc tcg ggg     1299
Leu Leu Gly Gly Gly Phe Ala Leu Ala Lys Gly Ser Glu Ala Ser Gly
        415                 420                 425

ctg tcc gtg tgg atg ggg aag cag atg gag ccc ttg cac gca gtg ccc     1347
Leu Ser Val Trp Met Gly Lys Gln Met Glu Pro Leu His Ala Val Pro
    430                 435                 440

ccg gca gcc atc acc ttg atc ttg tcc ttg ctc gtt gcc gtg ttc act     1395
Pro Ala Ala Ile Thr Leu Ile Leu Ser Leu Leu Val Ala Val Phe Thr
445                 450                 455                 460

gag tgc aca agc aac gtg gcc acc acc acc ttg ttc ctg ccc atc ttt     1443
Glu Cys Thr Ser Asn Val Ala Thr Thr Thr Leu Phe Leu Pro Ile Phe
                465                 470                 475

gcc tcc atg tct cgc tcc atc ggc ctc aat ccg ctg tac atc atg ctg     1491
Ala Ser Met Ser Arg Ser Ile Gly Leu Asn Pro Leu Tyr Ile Met Leu
            480                 485                 490

ccc tgt acc ctg agt gcc tcc ttt gcc ttc atg ttg cct gtg gcc acc     1539
Pro Cys Thr Leu Ser Ala Ser Phe Ala Phe Met Leu Pro Val Ala Thr
        495                 500                 505

cct cca aat gcc atc gtg ttc acc tat ggg cac ctc aag gtt gct gac     1587
Pro Pro Asn Ala Ile Val Phe Thr Tyr Gly His Leu Lys Val Ala Asp
    510                 515                 520

atg gtg aaa aca gga gtc ata atg aac ata att gga gtc ttc tgt gtg     1635
Met Val Lys Thr Gly Val Ile Met Asn Ile Ile Gly Val Phe Cys Val
525                 530                 535                 540

ttt ttg gct gtc aac acc tgg gga cgg gcc ata ttt gac ttg gat cat     1683
Phe Leu Ala Val Asn Thr Trp Gly Arg Ala Ile Phe Asp Leu Asp His
                545                 550                 555

ttc cct gac tgg gct aat gtg aca cat att gag act taggaagagc          1729
Phe Pro Asp Trp Ala Asn Val Thr His Ile Glu Thr
            560                 565

cacaagacca cacacacagc ccttaccctc ctcaggacta ccgaaccttc tggcacacct 1789

t                                                                   1790

<210> 70
<211> 568
<212> PRT
<213> Homo sapiens

<400> 70
Met Ala Ser Ala Leu Ser Tyr Val Ser Lys Phe Lys Ser Phe Val Ile

1               5                   10                  15

Leu Phe Val Thr Pro Leu Leu Leu Leu Pro Leu Val Ile Leu Met Pro
            20                  25                  30

Ala Lys Val Ser Cys Ala Tyr Val Ile Ile Leu Met Ala Ile Tyr Trp
        35                  40                  45

Cys Thr Glu Val Ile Pro Leu Ala Val Thr Ser Leu Met Pro Val Leu
    50                  55                  60

Leu Phe Pro Leu Phe Gln Ile Leu Asp Ser Arg Gln Val Cys Val Gln
65                  70                  75                  80

Tyr Met Lys Asp Thr Asn Met Leu Phe Leu Gly Gly Leu Ile Val Ala
                85                  90                  95

Val Ala Val Glu Arg Trp Asn Leu His Lys Arg Ile Ala Leu Arg Thr
            100                 105                 110

Leu Leu Trp Val Gly Ala Lys Pro Ala Arg Leu Met Leu Gly Phe Met
        115                 120                 125

Gly Val Thr Ala Leu Leu Ser Met Trp Ile Ser Asn Thr Ala Thr Thr
    130                 135                 140

Ala Met Met Val Pro Ile Val Glu Ala Ile Leu Gln Gln Met Glu Ala
145                 150                 155                 160

Thr Ser Ala Ala Thr Glu Ala Gly Leu Glu Leu Val Asp Lys Gly Lys
                165                 170                 175

Ala Lys Glu Leu Pro Gly Ser Gln Val Ile Phe Glu Gly Pro Thr Leu
            180                 185                 190

Gly Gln Gln Glu Asp Gln Glu Arg Lys Arg Leu Cys Lys Ala Met Thr
        195                 200                 205

Leu Cys Ile Cys Tyr Ala Ala Ser Ile Gly Gly Thr Ala Thr Leu Thr
    210                 215                 220

Gly Thr Gly Pro Asn Val Val Leu Leu Gly Gln Met Asn Glu Leu Phe
225                 230                 235                 240

Pro Asp Ser Lys Asp Leu Val Asn Phe Ala Ser Trp Phe Ala Phe Ala
                245                 250                 255

Phe Pro Asn Met Leu Val Met Leu Leu Phe Ala Trp Leu Trp Leu Gln
            260                 265                 270

Phe Val Tyr Met Phe Ser Ser Phe Lys Lys Ser Trp Gly Cys Gly Leu
        275                 280                 285

Glu Ser Lys Lys Asn Glu Lys Ala Ala Leu Lys Val Leu Gln Glu Glu
    290                 295                 300

Tyr Arg Lys Leu Gly Pro Leu Ser Phe Ala Glu Ile Asn Val Leu Ile
305                 310                 315                 320

Cys Phe Phe Leu Leu Val Ile Leu Trp Phe Ser Arg Asp Pro Gly Phe
                325                 330                 335

Met Pro Gly Trp Leu Thr Val Ala Trp Val Glu Gly Glu Thr Lys Tyr
            340                 345                 350

Val Ser Asp Ala Thr Val Ala Ile Phe Val Ala Thr Leu Leu Phe Ile
        355                 360                 365

Val Pro Ser Gln Lys Pro Lys Phe Asn Phe Arg Ser Gln Thr Glu Glu
    370                 375                 380

Gly Lys Ser Pro Val Leu Ile Ala Pro Pro Pro Leu Leu Asp Trp Lys
385                 390                 395                 400

Val Thr Gln Glu Lys Val Pro Trp Gly Ile Val Leu Leu Leu Gly Gly
                405                 410                 415

Gly Phe Ala Leu Ala Lys Gly Ser Glu Ala Ser Gly Leu Ser Val Trp
            420                 425                 430

Met Gly Lys Gln Met Glu Pro Leu His Ala Val Pro Pro Ala Ala Ile
        435                 440                 445

Thr Leu Ile Leu Ser Leu Leu Val Ala Val Phe Thr Glu Cys Thr Ser
    450                 455                 460

Asn Val Ala Thr Thr Thr Leu Phe Leu Pro Ile Phe Ala Ser Met Ser
465                 470                 475                 480

Arg Ser Ile Gly Leu Asn Pro Leu Tyr Ile Met Leu Pro Cys Thr Leu
                485                 490                 495

Ser Ala Ser Phe Ala Phe Met Leu Pro Val Ala Thr Pro Pro Asn Ala
            500                 505                 510

Ile Val Phe Thr Tyr Gly His Leu Lys Val Ala Asp Met Val Lys Thr
        515                 520                 525

Gly Val Ile Met Asn Ile Ile Gly Val Phe Cys Val Phe Leu Ala Val
    530                 535                 540

Asn Thr Trp Gly Arg Ala Ile Phe Asp Leu Asp His Phe Pro Asp Trp
545                 550                 555                 560

Ala Asn Val Thr His Ile Glu Thr
                565

<210> 71
<211> 3147
<212> DNA
<213> Homo sapiens

<220>
<221> CDS
<222> (2)..(1705)

<400> 71
g atg gcc tcg gcg ctg agc tat gtc tcc aag ttc aag tcc ttc gtg atc   49
ttg ttc gtc acc ccg ctc ctg ctg ctg cca ctc gtc att ctg atg ccc     97
gcc aag ttt gtc agg tgt gcc tac gtc atc atc ctc atg gcc att tac     145
tgg tgc aca gaa gtc atc cct ctg gct gtc acc tct ctc atg cct gtc     193
ttg ctt ttc cca ctc ttc cag att ctg gac tcc agg cag gtg tgt gtc     241
cag tac atg aag gac acc aac atg ctg ttc ctg ggc ggc ctc atc gtg     289
gcc gtg gct gtg gag cgc tgg aac ctg cac aag agg atc gcc ctg cgc     337
acg ctc ctc tgg gtg ggg gcc aag cct gca cgg ctg atg ctg ggc ttc     385
atg ggc gtc aca gcc ctc ctg tcc atg tgg atc agt aac acg gca acc     433
acg gcc atg atg gtg ccc atc gtg gag gcc ata ttg cag cag atg gaa     481
gcc aca agc gca gcc acc gag gcc ggc ctg gag ctg gtg gac aag ggc     529
aag gcc aag gag ctg cca ggg agt caa gtg att ttt gaa ggc ccc act     577
ctg ggg cag cag gaa gac caa gag cgg aag agg ttg tgt aag gcc atg     625
acc ctg tgc atc tgc tac gcg gcc agc atc ggg ggc acc gcc acc ctg     673
acc ggg acg gga ccc aac gtg gtg ctc ctg ggc cag atg aac gag ttg     721
ttt cct gac agc aag gac ctc gtg aac ttt gct tcc tgg ttt gca ttt     769
gcc ttt ccc aac atg ctg gtg atg ctg ctg ttc gcc tgg ctg tgg ctc     817
cag ttt gtt tac atg aga ttc aat ttt aaa aag tcc tgg ggc tgc ggg 865
cta gag agc aag aaa aac gag aag gct gcc ctc aag gtg ctg cag gag 913
gag tac cgg aag ttg ggg ccc ttg tcc ttc gcg gag atc aac gtg ctg 961
atc tgc ttc ttc ctg ctg gtc atc ctg tgg ttc tcc cga gac ccc ggc 1009
ttc atg ccc ggc tgg ctg act gtt gcc tgg gtg gag ggt gag aca aag 1057
tat gtc tcc gat gcc act gtg gcc atc ttt gtg gcc acc ctg cta ttc 1105
att gtg cct tca cag aag ccc aag ttt aac ttc cgc agc cag act gag 1153
gaa gaa agg aaa act cca ttt tat ccc cct ccc ctg ctg gat tgg aag 1201
gta acc cag gag aaa gtg ccc tgg ggc atc gtg ctg cta cta ggg ggc 1249
gga ttt gct ctg gct aaa gga tcc gag gcc tcg ggg ctg tcc gtg tgg 1297
atg ggg aag cag atg gag ccc ttg cac gca gtg ccc ccg gca gcc atc 1345
acc ttg atc ttg tcc ttg ctc gtt gcc gtg ttc act gag tgc aca agc 1393
aac gtg gcc acc acc acc ttg ttc ctg ccc atc ttt gcc tcc atg tct 1441
cgc tcc atc ggc ctc aat ccg ctg tac atc atg ctg ccc tgt acc ctg 1489
agt gcc tcc ttt gcc ttc atg ttg cct gtg gcc acc cct cca aat gcc 1537
atc gtg ttc acc tat ggg cac ctc aag gtt gct gac atg gtg aaa aca    1585
gga gtc ata atg aac ata att gga gtc ttc tgt gtg ttt ttg gct gtc    1633
aac acc tgg gga cgg gcc ata ttt gac ttg gat cat ttc cct gac tgg    1681
gct aat gtg aca cat att gag act taggaagagc cacaagacca cacacacagc    1735
ccttaccctc ctcaggacta ccgaaccttc tggcacacct tgtacagagt tttggggttc 1795
acaccccaaa atgacccaac gatgtccaca caccaccaaa acccagccaa tgggccacct 1855
cttcctccaa gcccagatgc agagatggtc atgggcagct ggagggtagg ctcagaaatg 1915
aagggaaccc ctcagtgggc tgctggaccc atctttccca agccttgcca ttatctctgt 1975
gagggaggcc aggtagccga gggatcagga tgcaggctgc tgtacccgct ctgcctcaag 2035
catcccccac acagggctct ggttttcact cgcttcgtcc tagatagttt aaatgggaat 2095
cggatcccct ggttgagagc taagacaacc acctaccagt gcccatgtcc cttccagctc 2155
accttgagca gcctcagatc atctctgtca ctctggaagg gacaccccag ccagggacgg 2215
aatgcctggt cttgagcaac ctcccactgc tggagtgcga gtgggaatca gagcctcctg 2275
aagcctctgg gaactcctcc tgtggccacc accaaaggat gaggaatctg agttgccaac 2335
ttcaggacga cacctggctt gccacccaca gtgcaccaca ggccaaccta cgcccttcat 2395
cacttggttc tgttttaatc gactggcccc ctgtcccacc tctccagtga gcctccttca 2455
actccttggt cccctgttgt ctgggtcaac atttgccgag acgccttggc tggcaccctc 2515
tggggtcccc cttttctccc aggcaggtca tcttttctgg gagatgcttc ccctgccatc 2575
cccaaatagc taggatcaca ctccaagtat gggcagtgat ggcgctctgg gggccacagt 2635
gggctatcta ggccctccct cacctgaggc ccagagtgga cacagctgtt aatttccact 2695
ggctatgcca cttcagagtc tttcatgcca gcgtttgagc tcctctgggt aaaatcttcc 2755
ctttgttgac tggccttcac agccatggct ggtgacaaca gaggatcgtt gagattgagc 2815
agcgcttggt gatctctcag caaacaaccc ctgcccgtgg gccaatctac ttgaagttac 2875
tcggacaaag accccaaagt ggggcaacaa ctccagagag gctgtgggaa tcttcagaag 2935
cccccctgta agagacagac atgagagaca agcatcttct ttcccccgca agtccatttt 2995
atttccttct tgtgctgctc tggaagagag gcagtagcaa agagatgagc tcctggatgg 3055
cattttccag ggcaggagaa agtatgagag cctcaggaaa ccccatcaag gaccgagtat 3115
gtgtctggtt cctttggtgg ttggcttctg gc 3147

<210> 72
<211> 568
<212> PRT
<213> Homo sapiens

<400> 72

<210> 73
<211> 1606
<212> DNA
<213> Homo sapiens

<220>
<221> CDS
<222> (2)..(1567)

<400> 73
g atg gcc tcg gcg ctg agc tat gtc tcc aag ttc aag tcc ttc gtg atc 49
  Met Ala Ser Ala Leu Ser Tyr Val Ser Lys Phe Lys Ser Phe Val Ile
  1               5                   10                  15

ttg ttc gtc acc ccg ctc ctg ctg ctg cca ctc gtc att ctg atg ccc 97
Leu Phe Val Thr Pro Leu Leu Leu Leu Pro Leu Val Ile Leu Met Pro
            20                  25                  30

gcc aag ttt gtc agg tgt gcc tac gtc atc atc ctc atg gcc att tac 145
Ala Lys Phe Val Arg Cys Ala Tyr Val Ile Ile Leu Met Ala Ile Tyr
        35                  40                  45

tgg tgc aca gaa gtc atc cct ctg gct gtc acc tct ctc atg cct gtc 193
Trp Cys Thr Glu Val Ile Pro Leu Ala Val Thr Ser Leu Met Pro Val
    50                  55                  60

ttg ctt ttc cca ctc ttc cag att ctg gac tcc agg cag gtg tgt gtc 241
Leu Leu Phe Pro Leu Phe Gln Ile Leu Asp Ser Arg Gln Val Cys Val
65                  70                  75                  80

cag tac atg aag gac acc aac atg ctg ttc ctg ggc ggc ctc atc gtg 289
Gln Tyr Met Lys Asp Thr Asn Met Leu Phe Leu Gly Gly Leu Ile Val
                85                  90                  95

gcc gtg gct gtg gag cgc tgg aac ctg cac aag agg atc gcc ctg cgc 337
Ala Val Ala Val Glu Arg Trp Asn Leu His Lys Arg Ile Ala Leu Arg
            100                 105                 110

acg ctc ctc tgg gtg ggg gcc aag cct gca cgg ctg atg ctg ggc ttc 385
Thr Leu Leu Trp Val Gly Ala Lys Pro Ala Arg Leu Met Leu Gly Phe
        115                 120                 125

atg ggc gtc aca gcc ctc ctg tcc atg tgg atc agt aac acg gca acc 433
Met Gly Val Thr Ala Leu Leu Ser Met Trp Ile Ser Asn Thr Ala Thr
    130                 135                 140

acg gcc atg atg gtg ccc atc gtg gag gcc ata ttg cag cag atg gaa 481
Thr Ala Met Met Val Pro Ile Val Glu Ala Ile Leu Gln Gln Met Glu
145                 150                 155                 160

gcc aca agc gca gcc acc gag gcc ggc ctg gag ctg gtg gac aag ggc 529
Ala Thr Ser Ala Ala Thr Glu Ala Gly Leu Glu Leu Val Asp Lys Gly
                165                 170                 175

aag gcc aag gag ctg cca ggg agt caa gtg att ttt gaa ggc ccc act 577
Lys Ala Lys Glu Leu Pro Gly Ser Gln Val Ile Phe Glu Gly Pro Thr
            180                 185                 190

ctg ggg cag cag gaa gac caa gag cgg aag agg ttg tgt aag gcc atg 625
Leu Gly Gln Gln Glu Asp Gln Glu Arg Lys Arg Leu Cys Lys Ala Met
        195                 200                 205

acc ctg tgc atc tgc tac gcg gcc agc atc ggg ggc acc gcc acc ctg 673
Thr Leu Cys Ile Cys Tyr Ala Ala Ser Ile Gly Gly Thr Ala Thr Leu
    210                 215                 220

acc ggg acg gga ccc aac gtg gtg ctc ctg ggc cag atg aac gag ttg 721
Thr Gly Thr Gly Pro Asn Val Val Leu Leu Gly Gln Met Asn Glu Leu
225                 230                 235                 240

ttt cct gac agc aag gac ctc gtg aac ttt gct tcc tgg ttt gca ttt 769
Phe Pro Asp Ser Lys Asp Leu Val Asn Phe Ala Ser Trp Phe Ala Phe
                245                 250                 255

gcc ttt ccc aac atg ctg gtg atg ctg ctg ttc gcc tgg ctg tgg ctc 817
Ala Phe Pro Asn Met Leu Val Met Leu Leu Phe Ala Trp Leu Trp Leu
            260                 265                 270

cag ttt gtt tac atg aga ttc aat ttt aaa aag tcc tgg ggc tgc ggg 865
Gln Phe Val Tyr Met Arg Phe Asn Phe Lys Lys Ser Trp Gly Cys Gly
        275                 280                 285

cta gag agc aag aaa aac gag aag gct gcc ctc aag gtg ctg cag gag 913
Leu Glu Ser Lys Lys Asn Glu Lys Ala Ala Leu Lys Val Leu Gln Glu
    290                 295                 300

gag tac cgg aag ttg ggg ccc ttg tcc ttc gcg gag atc aac gtg ctg 961
Glu Tyr Arg Lys Leu Gly Pro Leu Ser Phe Ala Glu Ile Asn Val Leu
305                 310                 315                 320

atc tgc ttc ttc ctg ctg gtc atc ctg tgg ttc tcc cga gac ccc ggc 1009
Ile Cys Phe Phe Leu Leu Val Ile Leu Trp Phe Ser Arg Asp Pro Gly
                325                 330                 335

ttc atg ccc ggc tgg ctg act gtt gcc tgg gtg gag ggt gag aca aag 1057
Phe Met Pro Gly Trp Leu Thr Val Ala Trp Val Glu Gly Glu Thr Lys
            340                 345                 350

tat gtc tcc gat gcc act gtg gcc atc ttt gtg gcc acc ctg cta ttc 1105
Tyr Val Ser Asp Ala Thr Val Ala Ile Phe Val Ala Thr Leu Leu Phe
        355                 360                 365

att gtg cct tca cag aag ccc aag ttt aac ttc cgc agc cag act gag 1153
Ile Val Pro Ser Gln Lys Pro Lys Phe Asn Phe Arg Ser Gln Thr Glu
    370                 375                 380

gaa gaa agg aaa act cca ttt tat ccc cct ccc ctg ctg gat tgg aag 1201
Glu Glu Arg Lys Thr Pro Phe Tyr Pro Pro Pro Leu Leu Asp Trp Lys
385                 390                 395                 400

gta acc cag gag aaa gtg ccc tgg ggc atc gtg ctg cta cta ggg ggc 1249
Val Thr Gln Glu Lys Val Pro Trp Gly Ile Val Leu Leu Leu Gly Gly
                405                 410                 415

gga ttt gct ctg gct aaa gga tcc gag gcc tcg ggg ctg tcc gtg tgg 1297
Gly Phe Ala Leu Ala Lys Gly Ser Glu Ala Ser Gly Leu Ser Val Trp
            420                 425                 430

atg ggg aag cag atg gag ccc ttg cac gca gtg ccc ccg gca gcc atc 1345
Met Gly Lys Gln Met Glu Pro Leu His Ala Val Pro Pro Ala Ala Ile
        435                 440                 445

acc ttg atc ttg tcc ttg ctc gtt gcc gtg ttc act gag tgc aca agc 1393
Thr Leu Ile Leu Ser Leu Leu Val Ala Val Phe Thr Glu Cys Thr Ser
    450                 455                 460

aac gtg gcc acc acc acc ttg ttc ctg ccc atc ttt gcc tcc atg gtg     1441
Asn Val Ala Thr Thr Thr Leu Phe Leu Pro Ile Phe Ala Ser Met Val
465                 470                 475                 480

aaa aca gga gtc ata atg aac ata att gga gtc ttc tgt gtg ttt ttg     1489
Lys Thr Gly Val Ile Met Asn Ile Ile Gly Val Phe Cys Val Phe Leu
                485                 490                 495

gct gtc aac acc tgg gga cgg gcc ata ttt gac ttg gat cat ttc cct     1537
Ala Val Asn Thr Trp Gly Arg Ala Ile Phe Asp Leu Asp His Phe Pro
            500                 505                 510

gac tgg gct aat gtg aca cat att gag act taggaagagc cacaagacca     1587
Asp Trp Ala Asn Val Thr His Ile Glu Thr
        515                 520

cacacatagc ccttaccct                                                1606

<210> 74
<211> 522
<212> PRT
<213> Homo sapiens

<400> 74
Met Ala Ser Ala Leu Ser Tyr Val Ser Lys Phe Lys Ser Phe Val Ile
  1               5                  10                  15

Leu Phe Val Thr Pro Leu Leu Leu Leu Pro Leu Val Ile Leu Met Pro
             20                  25                  30

Ala Lys Phe Val Arg Cys Ala Tyr Val Ile Ile Leu Met Ala Ile Tyr
         35                  40                  45

Trp Cys Thr Glu Val Ile Pro Leu Ala Val Thr Ser Leu Met Pro Val
     50                  55                  60

Leu Leu Phe Pro Leu Phe Gln Ile Leu Asp Ser Arg Gln Val Cys Val
 65                  70                  75                  80

Gln Tyr Met Lys Asp Thr Asn Met Leu Phe Leu Gly Gly Leu Ile Val
                 85                  90                  95

Ala Val Ala Val Glu Arg Trp Asn Leu His Lys Arg Ile Ala Leu Arg
            100                 105                 110

Thr Leu Leu Trp Val Gly Ala Lys Pro Ala Arg Leu Met Leu Gly Phe
        115                 120                 125

Met Gly Val Thr Ala Leu Leu Ser Met Trp Ile Ser Asn Thr Ala Thr
    130                 135                 140

Thr Ala Met Met Val Pro Ile Val Glu Ala Ile Leu Gln Gln Met Glu
145                 150                 155                 160

Ala Thr Ser Ala Ala Thr Glu Ala Gly Leu Glu Leu Val Asp Lys Gly
                165                 170                 175

Lys Ala Lys Glu Leu Pro Gly Ser Gln Val Ile Phe Glu Gly Pro Thr
            180                 185                 190

Leu Gly Gln Gln Glu Asp Gln Glu Arg Lys Arg Leu Cys Lys Ala Met

195                 200                 205

Thr Leu Cys Ile Cys Tyr Ala Ala Ser Ile Gly Gly Thr Ala Thr Leu
    210                 215                 . 220

Thr Gly Thr Gly Pro Asn Val Val Leu Leu Gly Gln Met Asn Glu Leu
225                 230                 235                 240

Phe Pro Asp Ser Lys Asp Leu Val Asn Phe Ala Ser Trp Phe Ala Phe
                245                 250                 255

Ala Phe Pro Asn Met Leu Val Met Leu Leu Phe Ala Trp Leu Trp Leu
            260                 265                 270

Gln Phe Val Tyr Met Arg Phe Asn Phe Lys Lys Ser Trp Gly Cys Gly
        275                 280                 285

Leu Glu Ser Lys Lys Asn Glu Lys Ala Ala Leu Lys Val Leu Gln Glu
    290                 295                 300

Glu Tyr Arg Lys Leu Gly Pro Leu Ser Phe Ala Glu Ile Asn Val Leu
305                 310                 315                 320

Ile Cys Phe Phe Leu Leu Val Ile Leu Trp Phe Ser Arg Asp Pro Gly
                325                 330                 335

Phe Met Pro Gly Trp Leu Thr Val Ala Trp Val Glu Gly Glu Thr Lys
            340                 345                 350

Tyr Val Ser Asp Ala Thr Val Ala Ile Phe Val Ala Thr Leu Leu Phe
        355                 360                 365

Ile Val Pro Ser Gln Lys Pro Lys Phe Asn Phe Arg Ser Gln Thr Glu
    370                 375                 380

Glu Glu Arg Lys Thr Pro Phe Tyr Pro Pro Pro Leu Leu Asp Trp Lys
385                 390                 395                 400

Val Thr Gln Glu Lys Val Pro Trp Gly Ile Val Leu Leu Leu Gly Gly
                405                 410                 415

Gly Phe Ala Leu Ala Lys Gly Ser Glu Ala Ser Gly Leu Ser Val Trp
            420                 425                 430

Met Gly Lys Gln Met Glu Pro Leu His Ala Val Pro Pro Ala Ala Ile
        435                 440                 445

Thr Leu Ile Leu Ser Leu Leu Val Ala Val Phe Thr Glu Cys Thr Ser
    450                 455                 460

Asn Val Ala Thr Thr Thr Leu Phe Leu Pro Ile Phe Ala Ser Met Val
465                 470                 475                 480

Lys Thr Gly Val Ile Met Asn Ile Ile Gly Val Phe Cys Val Phe Leu
                485                 490                 495

Ala Val Asn Thr Trp Gly Arg Ala Ile Phe Asp Leu Asp His Phe Pro
            500                 505                 510

Asp Trp Ala Asn Val Thr His Ile Glu Thr
        515                 520

<210> 75
<211> 1781
<212> DNA
<213> Homo sapiens

<220>
<221> CDS
<222> (2)..(1549)

<400> 75
g atg gcc tcg gcg ctg agc tat gtc tcc aag ttc aag tcc ttc gtg atc 49
  Met Ala Ser Ala Leu Ser Tyr Val Ser Lys Phe Lys Ser Phe Val Ile
  1               5                   10                  15

ttg ttc gtc acc ccg ctc ctg ctg ctg cca ctc gtc att ctg atg ccc 97
Leu Phe Val Thr Pro Leu Leu Leu Leu Pro Leu Val Ile Leu Met Pro
            20                  25                  30

gcc aag ttt gtc agg tgt gcc tac gtc atc atc ctc atg gcc att tac 145
Ala Lys Phe Val Arg Cys Ala Tyr Val Ile Ile Leu Met Ala Ile Tyr
        35                  40                  45

tgg tgc aca gaa gtc atc cct ctg gct gtc acc tct ctc atg cct gtc 193
Trp Cys Thr Glu Val Ile Pro Leu Ala Val Thr Ser Leu Met Pro Val
    50                  55                  60

ttg ctt ttc cca ctc ttc cag att ctg gac tcc agg cag gtg tgt gtc 241
Leu Leu Phe Pro Leu Phe Gln Ile Leu Asp Ser Arg Gln Val Cys Val
65                  70                  75                  80

cag tac atg aag gac acc aac atg ctg ttc ctg ggc ggc ctc atc gtg 289
Gln Tyr Met Lys Asp Thr Asn Met Leu Phe Leu Gly Gly Leu Ile Val
                85                  90                  95

gcc gtg gct gtg gag cgc tgg aac ctg cac aag agg atc gcc ctg cgc 337
Ala Val Ala Val Glu Arg Trp Asn Leu His Lys Arg Ile Ala Leu Arg
            100                 105                 110

acg ctc ctc tgg gtg ggg gcc aag cct gca cgg ctg atg ctg ggc ttc 385
Thr Leu Leu Trp Val Gly Ala Lys Pro Ala Arg Leu Met Leu Gly Phe
        115                 120                 125

atg ggc gtc aca gcc ctc ctg tcc atg tgg atc agt aac acg gca acc 433
Met Gly Val Thr Ala Leu Leu Ser Met Trp Ile Ser Asn Thr Ala Thr
    130                 135                 140

acg gcc atg atg gtg ccc atc gtg gag gcc ata ttg cag cag atg gaa 481
Thr Ala Met Met Val Pro Ile Val Glu Ala Ile Leu Gln Gln Met Glu
145                 150                 155                 160

gcc aca agc gca gcc acc gag gcc ggc ctg gag ctg gtg gac aag ggc 529
Ala Thr Ser Ala Ala Thr Glu Ala Gly Leu Glu Leu Val Asp Lys Gly
                165                 170                 175

aag gcc aag gag ctg cca ggg agt caa gtg att ttt gaa ggc ccc act 577
Lys Ala Lys Glu Leu Pro Gly Ser Gln Val Ile Phe Glu Gly Pro Thr
            180                 185                 190

ctg ggg cag cag gaa gac caa gag cgg aag agg ttg tgt aag gcc atg 625
Leu Gly Gln Gln Glu Asp Gln Glu Arg Lys Arg Leu Cys Lys Ala Met

195                 200                 205

acc ctg tgc atc tgc tac gcg gcc agc atc ggg ggc acc gcc acc ctg     673
Thr Leu Cys Ile Cys Tyr Ala Ala Ser Ile Gly Gly Thr Ala Thr Leu
    210                 215                 220

acc ggg acg gga ccc aac gtg gtg ctc ctg ggc cag atg aac gag ttg     721
Thr Gly Thr Gly Pro Asn Val Val Leu Leu Gly Gln Met Asn Glu Leu
225                 230                 235                 240

ttt cct gac agc aag gac ctc gtg aac ttt gct tcc tgg ttt gca ttt     769
Phe Pro Asp Ser Lys Asp Leu Val Asn Phe Ala Ser Trp Phe Ala Phe
                245                 250                 255

gcc ttt ccc aac atg ctg gtg atg ctg ctg ttc gcc tgg ctg tgg ctc     817
Ala Phe Pro Asn Met Leu Val Met Leu Leu Phe Ala Trp Leu Trp Leu
            260                 265                 270

cag ttt gtt tac atg aga ttc aat ttt aaa aag tcc tgg ggc tgc ggg     865
Gln Phe Val Tyr Met Arg Phe Asn Phe Lys Lys Ser Trp Gly Cys Gly
        275                 280                 285

cta gag agc aag aaa aac gag aag gct gcc ctc aag gtg ctg cag gag     913
Leu Glu Ser Lys Lys Asn Glu Lys Ala Ala Leu Lys Val Leu Gln Glu
    290                 295                 300

gag tac cgg aag ttg ggg ccc ttg tcc ttc gcg gag atc aac gtg ctg     961
Glu Tyr Arg Lys Leu Gly Pro Leu Ser Phe Ala Glu Ile Asn Val Leu
305                 310                 315                 320

atc tgc ttc ttc ctg ctg gtc atc ctg tgg ttc tcc cga gac ccc ggc     1009
Ile Cys Phe Phe Leu Leu Val Ile Leu Trp Phe Ser Arg Asp Pro Gly
                325                 330                 335

ttc atg ccc ggc tgg ctg act gtt gcc tgg gtg gag ggt gag aca aag     1057
Phe Met Pro Gly Trp Leu Thr Val Ala Trp Val Glu Gly Glu Thr Lys
            340                 345                 350

tat gtc tcc gat gcc act gtg gcc atc ttt gtg gcc acc ctg cta ttc     1105
Tyr Val Ser Asp Ala Thr Val Ala Ile Phe Val Ala Thr Leu Leu Phe
        355                 360                 365

att gtg cct tca cag aag ccc aag ttt aac ttc cgc agc cag act gag     1153
Ile Val Pro Ser Gln Lys Pro Lys Phe Asn Phe Arg Ser Gln Thr Glu
    370                 375                 380

gaa gaa agg aaa act cca ttt tat ccc cct ccc ctg ctg gat tgg aag     1201
Glu Glu Arg Lys Thr Pro Phe Tyr Pro Pro Pro Leu Leu Asp Trp Lys
385                 390                 395                 400

gta acc cag gag aaa gtg ccc tgg ggc atc gtg ctg cta cta ggg ggc     1249
Val Thr Gln Glu Lys Val Pro Trp Gly Ile Val Leu Leu Leu Gly Gly
                405                 410                 415

gga ttt gct ctg gct aaa gga tcc gag gcc tcg ggg ctg tcc gtg tgg     1297
Gly Phe Ala Leu Ala Lys Gly Ser Glu Ala Ser Gly Leu Ser Val Trp
            420                 425                 430

atg ggg aag cag atg gag ccc ttg cac gca gtg ccc ccg gca gcc atc     1345
Met Gly Lys Gln Met Glu Pro Leu His Ala Val Pro Pro Ala Ala Ile
        435                 440                 445

acc ttg atc ttg tcc ttg ctc gtt gcc gtg ttc act gag tgc aca agc    1393
Thr Leu Ile Leu Ser Leu Leu Val Ala Val Phe Thr Glu Cys Thr Ser
    450                 455                 460

aac gtg gcc acc acc acc ttg ttc ctg ccc atc ttt gcc tcc atg aat    1441
Asn Val Ala Thr Thr Thr Leu Phe Leu Pro Ile Phe Ala Ser Met Asn
465                 470                 475                 480

cac gtc ccc aag agc ttc tgt gtt ctg tac ggt gat gtt gca gtg ctg    1489
His Val Pro Lys Ser Phe Cys Val Leu Tyr Gly Asp Val Ala Val Leu
                485                 490                 495

tct ttc cgc agt ctc gct cca tcg gcc tca atc cgc tgt aca tca tgc    1537
Ser Phe Arg Ser Leu Ala Pro Ser Ala Ser Ile Arg Cys Thr Ser Cys
            500                 505                 510

tgc cct gta ccc tgagtgcctc ctttgccttc atgttgcctg tggccacccc        1589
Cys Pro Val Pro
        515

tccaaatgcc atcgtgttca cctatgggca cctcaaggtt gctgacatgg tgaaaacagg 1649

agtcataatg aacataattg gagtcttctg tgtgtttttg gctgtcaaca cctggggacg 1709

ggccatattt gacttggatc atttccctga ctgggctaat gtgacacata ttgagactta 1769

ggaagagcca ca                                                      1781

<210> 76
<211> 516
<212> PRT
<213> Homo sapiens

<400> 76
Met Ala Ser Ala Leu Ser Tyr Val Ser Lys Phe Lys Ser Phe Val Ile
1               5                   10                  15

Leu Phe Val Thr Pro Leu Leu Leu Leu Pro Leu Val Ile Leu Met Pro
            20                  25                  30

Ala Lys Phe Val Arg Cys Ala Tyr Val Ile Ile Leu Met Ala Ile Tyr
        35                  40                  45

Trp Cys Thr Glu Val Ile Pro Leu Ala Val Thr Ser Leu Met Pro Val
    50                  55                  60

Leu Leu Phe Pro Leu Phe Gln Ile Leu Asp Ser Arg Gln Val Cys Val
65                  70                  75                  80

Gln Tyr Met Lys Asp Thr Asn Met Leu Phe Leu Gly Gly Leu Ile Val
                85                  90                  95

Ala Val Ala Val Glu Arg Trp Asn Leu His Lys Arg Ile Ala Leu Arg
            100                 105                 110

Thr Leu Leu Trp Val Gly Ala Lys Pro Ala Arg Leu Met Leu Gly Phe
        115                 120                 125

Met Gly Val Thr Ala Leu Leu Ser Met Trp Ile Ser Asn Thr Ala Thr
    130                 135                 140

Thr Ala Met Met Val Pro Ile Val Glu Ala Ile Leu Gln Gln Met Glu
145                 150                 155                 160

Ala Thr Ser Ala Ala Thr Glu Ala Gly Leu Glu Leu Val Asp Lys Gly
                165                 170                 175

Lys Ala Lys Glu Leu Pro Gly Ser Gln Val Ile Phe Glu Gly Pro Thr
            180                 185                 190

Leu Gly Gln Gln Glu Asp Gln Glu Arg Lys Arg Leu Cys Lys Ala Met
        195                 200                 205

Thr Leu Cys Ile Cys Tyr Ala Ala Ser Ile Gly Gly Thr Ala Thr Leu
    210                 215                 220

Thr Gly Thr Gly Pro Asn Val Val Leu Leu Gly Gln Met Asn Glu Leu
225                 230                 235                 240

Phe Pro Asp Ser Lys Asp Leu Val Asn Phe Ala Ser Trp Phe Ala Phe
                245                 250                 255

Ala Phe Pro Asn Met Leu Val Met Leu Leu Phe Ala Trp Leu Trp Leu
            260                 265                 270

Gln Phe Val Tyr Met Arg Phe Asn Phe Lys Lys Ser Trp Gly Cys Gly
        275                 280                 285

Leu Glu Ser Lys Lys Asn Glu Lys Ala Ala Leu Lys Val Leu Gln Glu
    290                 295                 300

Glu Tyr Arg Lys Leu Gly Pro Leu Ser Phe Ala Glu Ile Asn Val Leu
305                 310                 315                 320

Ile Cys Phe Phe Leu Leu Val Ile Leu Trp Phe Ser Arg Asp Pro Gly
                325                 330                 335

Phe Met Pro Gly Trp Leu Thr Val Ala Trp Val Glu Gly Glu Thr Lys
            340                 345                 350

Tyr Val Ser Asp Ala Thr Val Ala Ile Phe Val Ala Thr Leu Leu Phe
        355                 360                 365

Ile Val Pro Ser Gln Lys Pro Lys Phe Asn Phe Arg Ser Gln Thr Glu
    370                 375                 380

Glu Glu Arg Lys Thr Pro Phe Tyr Pro Pro Pro Leu Leu Asp Trp Lys
385                 390                 395                 400

Val Thr Gln Glu Lys Val Pro Trp Gly Ile Val Leu Leu Leu Gly Gly
                405                 410                 415

Gly Phe Ala Leu Ala Lys Gly Ser Glu Ala Ser Gly Leu Ser Val Trp
            420                 425                 430

Met Gly Lys Gln Met Glu Pro Leu His Ala Val Pro Pro Ala Ala Ile
        435                 440                 445

Thr Leu Ile Leu Ser Leu Leu Val Ala Val Phe Thr Glu Cys Thr Ser
    450                 455                 460

Asn Val Ala Thr Thr Thr Leu Phe Leu Pro Ile Phe Ala Ser Met Asn
465                 470                 475                 480

His Val Pro Lys Ser Phe Cys Val Leu Tyr Gly Asp Val Ala Val Leu
                485                 490                 495

Ser Phe Arg Ser Leu Ala Pro Ser Ala Ser Ile Arg Cys Thr Ser Cys
            500                 505                 510

Cys Pro Val Pro
        515

<210> 77
<211> 972
<212> DNA
<213> Homo sapiens

<220>
<221> CDS
<222> (1)..(969)

<400> 77
atg gat tcg aaa tat cag tgt gtg aag ctg aat gat ggt cac ttc atg      48
Met Asp Ser Lys Tyr Gln Cys Val Lys Leu Asn Asp Gly His Phe Met
  1               5                  10                  15

cct gtc ctg gga ttt ggc acc tat gcg cct gca gag gtt cct aaa agt      96
Pro Val Leu Gly Phe Gly Thr Tyr Ala Pro Ala Glu Val Pro Lys Ser
             20                  25                  30

aaa gct tta gag gcc acc aaa ttg gca att gaa gct ggc ttc cgc cat     144
Lys Ala Leu Glu Ala Thr Lys Leu Ala Ile Glu Ala Gly Phe Arg His
         35                  40                  45

att gat tct gct cat tta tac aat aat gag gag cag gtt gga ctg gcc     192
Ile Asp Ser Ala His Leu Tyr Asn Asn Glu Glu Gln Val Gly Leu Ala
     50                  55                  60

atc cga agc aag att gca gat ggc agt gtg aag aga gaa gac ata ttc     240
Ile Arg Ser Lys Ile Ala Asp Gly Ser Val Lys Arg Glu Asp Ile Phe
 65                  70                  75                  80

tac act tca aag ctt tgg tgc aat tcc cat cga cca gag ttg gtc cga     288
Tyr Thr Ser Lys Leu Trp Cys Asn Ser His Arg Pro Glu Leu Val Arg
                 85                  90                  95

cca gcc ttg gaa agg tca ctg aaa aat ctt caa ttg gat tat gtt gac     336
Pro Ala Leu Glu Arg Ser Leu Lys Asn Leu Gln Leu Asp Tyr Val Asp
            100                 105                 110

ctc tac ctt att cat ttt cca gtg tct gta aag cca ggt gag gaa gtg     384
Leu Tyr Leu Ile His Phe Pro Val Ser Val Lys Pro Gly Glu Glu Val
        115                 120                 125

atc cca aaa gat gaa aat gga aaa ata cta ttt gac aca gtg gat ctc     432
Ile Pro Lys Asp Glu Asn Gly Lys Ile Leu Phe Asp Thr Val Asp Leu
    130                 135                 140

tgt gcc acg tgg gag gcc gtg gag aag tgt aaa gat gca gga ttg gcc     480
Cys Ala Thr Trp Glu Ala Val Glu Lys Cys Lys Asp Ala Gly Leu Ala
145                 150                 155                 160

aag tcc atc ggg gtg tcc aac ttc aac cgc agg cag ctg gag atg atc     528
Lys Ser Ile Gly Val Ser Asn Phe Asn Arg Arg Gln Leu Glu Met Ile
                165                 170                 175

ctc aac aag cca ggg ctc aag tac aag cct gtc tgc aac cag gtg gaa     576
Leu Asn Lys Pro Gly Leu Lys Tyr Lys Pro Val Cys Asn Gln Val Glu
            180                 185                 190

tgt cat cct tac ttc aac cag aga aaa ctg ctg gat ttc tgc aag tca     624
Cys His Pro Tyr Phe Asn Gln Arg Lys Leu Leu Asp Phe Cys Lys Ser
        195                 200                 205

aaa gac att gtt ctg gtt gcc tat agt gct ctg gga tcc cac cga gaa     672
Lys Asp Ile Val Leu Val Ala Tyr Ser Ala Leu Gly Ser His Arg Glu
    210                 215                 220

gaa cca tgg gtg gac ccg aac tcc ccg gtg ctc ttg gag gac cca gtc     720
Glu Pro Trp Val Asp Pro Asn Ser Pro Val Leu Leu Glu Asp Pro Val
225                 230                 235                 240

ctt tgt gcc ttg gca aaa aag cac aag cga acc cca gcc ctg att gcc     768
Leu Cys Ala Leu Ala Lys Lys His Lys Arg Thr Pro Ala Leu Ile Ala
                245                 250                 255

ctg cgc tac cag cta cag cgt ggg gtt gtg gtc ctg gcc aag agc tac     816
Leu Arg Tyr Gln Leu Gln Arg Gly Val Val Val Leu Ala Lys Ser Tyr
            260                 265                 270

aat gag cag cgc atc aga cag aac gtg cag gtg ttt gaa ttc cag ttg     864
Asn Glu Gln Arg Ile Arg Gln Asn Val Gln Val Phe Glu Phe Gln Leu
        275                 280                 285

act tca gag gag atg aaa gcc ata gat ggc cta aac aga aat gtg cga     912
Thr Ser Glu Glu Met Lys Ala Ile Asp Gly Leu Asn Arg Asn Val Arg
    290                 295                 300

tat ttg acc ctt gat att ttt gct ggc ccc cct aat tat cca ttt tct     960
Tyr Leu Thr Leu Asp Ile Phe Ala Gly Pro Pro Asn Tyr Pro Phe Ser
305                 310                 315                 320

gat gaa tat taa                                                     972
Asp Glu Tyr


<210> 78
<211> 323
<212> PRT
<213> Homo sapiens

<400> 78
Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Thr Lys Leu Ala Ile Glu Ala Gly Phe Arg His
        35                  40                  45

Ile Asp Ser Ala His Leu Tyr Asn Asn Glu Glu Gln Val Gly Leu Ala

50                  55                  60

Ile Arg Ser Lys Ile Ala Asp Gly Ser Val Lys Arg Glu Asp Ile Phe
65                  70                  75                  80

Tyr Thr Ser Lys Leu Trp Cys Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Val Glu Lys Cys Lys Asp Ala Gly Leu Ala
145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr

<210> 79
<211> 983
<212> DNA
<213> Homo sapiens

<220>
<221> CDS
<222> (30)..(980)

<400> 79
   atggattcga tatcagtgtg tgaagctga atg atg gtc act tcg tgc ctg tcc     53
                                   Met Met Val Thr Ser Cys Leu Ser
                                   1               5
   tgg gat ttg gca cct atg cgc ctg cag agg tta ctc ccc cag gtt cct 101
   Trp Asp Leu Ala Pro Met Arg Leu Gln Arg Leu Leu Pro Gln Val Pro
   10 15 20
   aaa agt aaa gct tta gag gcc acc aaa ttg gca att gaa gct ggc ttc 149
   Lys Ser Lys Ala Leu Glu Ala Thr Lys Leu Ala Ile Glu Ala Gly Phe
   25 30 35 40
   cgc cat att gat tct gct cat tta tac aat aat gag gag cag gtt gga 197
   Arg His Ile Asp Ser Ala His Leu Tyr Asn Asn Glu Glu Gln Val Gly
   45 50 55
   ctg gcc atc cga agc aag att gca gat ggc agt gtg aag aga gaa gac 245
   Leu Ala Ile Arg Ser Lys Ile Ala Asp Gly Ser Val Lys Arg Glu Asp
   60 65 70
   ata ttc tac act tca aag ctt tgg tgc aat tcc cat cga cca gag ttg 293
   Ile Phe Tyr Thr Ser Lys Leu Trp Cys Asn Ser His Arg Pro Glu Leu
   75 80 85
   gtc cga cca gcc ttg gaa agg tca ctg aaa aat ctt caa ttg gat tat 341
   Val Arg Pro Ala Leu Glu Arg Ser Leu Lys Asn Leu Gln Leu Asp Tyr
   90 95 100
   gtt gac ctc tac ctt att cat ttt cca gtg tct gta aag cca ggt gag 389
   Val Asp Leu Tyr Leu Ile His Phe Pro Val Ser Val Lys Pro Gly Glu
   105 110 115 120
   gaa gtg atc cca aaa gat gaa agt gga aaa ata cta ttt gac aca gtg 437
   Glu Val Ile Pro Lys Asp Glu Ser Gly Lys Ile Leu Phe Asp Thr Val
   125 130 135
   gat ctc tgt gcc acg tgg gag gcc gtg gag aag tgt aaa gat gca gga 485
   Asp Leu Cys Ala Thr Trp Glu Ala Val Glu Lys Cys Lys Asp Ala Gly
   140 145 150
   ttg gcc aag tcc atc ggg gtg tcc aac ttc aac cgc agg cag ctg gag 533
   Leu Ala Lys Ser Ile Gly Val Ser Asn Phe Asn Arg Arg Gln Leu Glu
   155 160 165
   atg atc ctc aac aag cca ggg ctc aag tac aag cct gtc tgc aac cag 581
   Met Ile Leu Asn Lys Pro Gly Leu Lys Tyr Lys Pro Val Cys Asn Gln
   170 175 180
   gtg gaa tgt cat cct tac ttc aac cag aga aaa ctg ctg gat ttc tgc 629
   Val Glu Cys His Pro Tyr Phe Asn Gln Arg Lys Leu Leu Asp Phe Cys
   185 190 195 200
   aag tca aaa gac att gtt ctg gtt gcc tat agt gct ctg gga tcc cac 677
   Lys Ser Lys Asp Ile Val Leu Val Ala Tyr Ser Ala Leu Gly Ser His
   205 210 215
   cga gaa gaa cca tgg gtg gac ccg aac tcc ccg gtg ctc ttg gag gac 725
   Arg Glu Glu Pro Trp Val Asp Pro Asn Ser Pro Val Leu Leu Glu Asp
   220 225 230
   cca gtc ctt tgt gcc ttg gca aaa aag cac aag cga acc cca gcc ctg     773
   Pro Val Leu Cys Ala Leu Ala Lys Lys His Lys Arg Thr Pro Ala Leu
           235                 240                 245
   gtt gcc ctg cgc tac cag cta cag cgt ggg gtt gtg gtc ctg gcc aag     821
   Val Ala Leu Arg Tyr Gln Leu Gln Arg Gly Val Val Val Leu Ala Lys
       250                 255                 260
   agc tac aat gag cag cgc atc aga cag aac gtg cag gtg ttt gaa ttc     869
   Ser Tyr Asn Glu Gln Arg Ile Arg Gln Asn Val Gln Val Phe Glu Phe
   265                 270                 275                 280
   cag ttg act tca gag gag atg aaa gcc ata gat ggc cta aac aga aat     917
   Gln Leu Thr Ser Glu Glu Met Lys Ala Ile Asp Gly Leu Asn Arg Asn
                   285                 290                 295
   gtg cga tat ttg acc ctt gat att ttt gct ggc ccc cct aat tat cca     965
   Val Arg Tyr Leu Thr Leu Asp Ile Phe Ala Gly Pro Pro Asn Tyr Pro
               300                 305                 310
   ttt tct gat gaa tat taa                                             983
   Phe Ser Asp Glu Tyr
           315

<210> 80
<211> 317
<212> PRT
<213> Homo sapiens

<400> 80
Met Met Val Thr Ser Cys Leu Ser Trp Asp Leu Ala Pro Met Arg Leu
1               5                   10                  15

Gln Arg Leu Leu Pro Gln Val Pro Lys Ser Lys Ala Leu Glu Ala Thr
            20                  25                  30

Lys Leu Ala Ile Glu Ala Gly Phe Arg His Ile Asp Ser Ala His Leu
        35                  40                  45

Tyr Asn Asn Glu Glu Gln Val Gly Leu Ala Ile Arg Ser Lys Ile Ala
    50                  55                  60

Asp Gly Ser Val Lys Arg Glu Asp Ile Phe Tyr Thr Ser Lys Leu Trp
65                  70                  75                  80

Cys Asn Ser His Arg Pro Glu Leu Val Arg Pro Ala Leu Glu Arg Ser
                85                  90                  95

Leu Lys Asn Leu Gln Leu Asp Tyr Val Asp Leu Tyr Leu Ile His Phe
            100                 105                 110

Pro Val Ser Val Lys Pro Gly Glu Glu Val Ile Pro Lys Asp Glu Ser
        115                 120                 125

Gly Lys Ile Leu Phe Asp Thr Val Asp Leu Cys Ala Thr Trp Glu Ala
    130                 135                 140

Val Glu Lys Cys Lys Asp Ala Gly Leu Ala Lys Ser Ile Gly Val Ser
145                 150                 155                 160

Asn Phe Asn Arg Arg Gln Leu Glu Met Ile Leu Asn Lys Pro Gly Leu
                165                 170                 175

Lys Tyr Lys Pro Val Cys Asn Gln Val Glu Cys His Pro Tyr Phe Asn
            180                 185                 190

Gln Arg Lys Leu Leu Asp Phe Cys Lys Ser Lys Asp Ile Val Leu Val
        195                 200                 205

Ala Tyr Ser Ala Leu Gly Ser His Arg Glu Glu Pro Trp Val Asp Pro
    210                 215                 220

Asn Ser Pro Val Leu Leu Glu Asp Pro Val Leu Cys Ala Leu Ala Lys
225                 230                 235                 240

Lys His Lys Arg Thr Pro Ala Leu Val Ala Leu Arg Tyr Gln Leu Gln
                245                 250                 255

Arg Gly Val Val Val Leu Ala Lys Ser Tyr Asn Glu Gln Arg Ile Arg
            260                 265                 270

Gln Asn Val Gln Val Phe Glu Phe Gln Leu Thr Ser Glu Glu Met Lys
        275                 280                 285

Ala Ile Asp Gly Leu Asn Arg Asn Val Arg Tyr Leu Thr Leu Asp Ile
    290                 295                 300

Phe Ala Gly Pro Pro Asn Tyr Pro Phe Ser Asp Glu Tyr
305                 310                 315

<210> 81
<211> 972
<212> DNA
<213> Homo sapiens

<220>
<221> CDS
<222> (1)..(969)

<400> 81
atg gat tcg aaa tat cag tgt gtg aag ctg aat gat ggt cac ttc atg      48
Met Asp Ser Lys Tyr Gln Cys Val Lys Leu Asn Asp Gly His Phe Met
  1               5                  10                  15

cct gtc ctg gga ttt ggc acc tat gcg cct gca gag gtt cct aaa agt      96
Pro Val Leu Gly Phe Gly Thr Tyr Ala Pro Ala Glu Val Pro Lys Ser
             20                  25                  30

aaa gct tta gag gcc acc aaa ttg gca att gaa gct ggc ttc cgc cat     144
Lys Ala Leu Glu Ala Thr Lys Leu Ala Ile Glu Ala Gly Phe Arg His
         35                  40                  45

att gat tct gct cat tta tac aat aat gag gag cag gtt gga ctg gcc     192
Ile Asp Ser Ala His Leu Tyr Asn Asn Glu Glu Gln Val Gly Leu Ala
     50                  55                  60

atc cga agc aag att gca gat ggc agt gtg aag aga gaa gac ata ttc     240
Ile Arg Ser Lys Ile Ala Asp Gly Ser Val Lys Arg Glu Asp Ile Phe
 65                  70                  75                  80

tac act tca aag ctt tgg tgc aat tcc cat cga cca gag ttg gtc cga     288

Tyr Thr Ser Lys Leu Trp Cys Asn Ser His Arg Pro Glu Leu Val Arg
                85                  90                  95

cca gcc ttg gaa agg tca ctg aaa aat ctt caa ttg gat tat gtt gac     336
Pro Ala Leu Glu Arg Ser Leu Lys Asn Leu Gln Leu Asp Tyr Val Asp
            100                 105                 110

ctc tac ctt att cat ttt cca gtg tct gta aag cca ggt gag gaa gtg     384
Leu Tyr Leu Ile His Phe Pro Val Ser Val Lys Pro Gly Glu Glu Val
        115                 120                 125

atc cca aaa gat gaa aat gga aaa ata cta ttt gac aca gtg gat ctc     432
Ile Pro Lys Asp Glu Asn Gly Lys Ile Leu Phe Asp Thr Val Asp Leu
    130                 135                 140

tgt gcc acg tgg gag gcc gtg gag aag tgt aaa gat gca gga ttg gcc     480
Cys Ala Thr Trp Glu Ala Val Glu Lys Cys Lys Asp Ala Gly Leu Ala
145                 150                 155                 160

aag tcc atc ggg gtg tcc aac ttc aac cgc agg cag ctg gag atg atc     528
Lys Ser Ile Gly Val Ser Asn Phe Asn Arg Arg Gln Leu Glu Met Ile
                165                 170                 175

ctc aac aag cca ggg ctc aag tac aag cct gtc tgc aac cag gtg gaa     576
Leu Asn Lys Pro Gly Leu Lys Tyr Lys Pro Val Cys Asn Gln Val Glu
            180                 185                 190

tgt cat cct tac ttc aac cag aga aaa ctg ctg gat ttc tgc aag tca     624
Cys His Pro Tyr Phe Asn Gln Arg Lys Leu Leu Asp Phe Cys Lys Ser
        195                 200                 205

aaa gac att gtt ctg gtt gcc tat agt gct ctg gga tcc cac cga gaa     672
Lys Asp Ile Val Leu Val Ala Tyr Ser Ala Leu Gly Ser His Arg Glu
    210                 215                 220

gaa cca tgg gtg gac ccg aac tcc ccg gtg ctc ttg gag gac cca gtc     720
Glu Pro Trp Val Asp Pro Asn Ser Pro Val Leu Leu Glu Asp Pro Val
225                 230                 235                 240

ctt tgt gcc ttg gca aaa aag cac aag cga acc cca gcc ctg att gcc     768
Leu Cys Ala Leu Ala Lys Lys His Lys Arg Thr Pro Ala Leu Ile Ala
                245                 250                 255

ctg cgc tac cag cta cag cgt ggg gtt gtg gtc ctg gcc aag aga tac     816
Leu Arg Tyr Gln Leu Gln Arg Gly Val Val Val Leu Ala Lys Arg Tyr
            260                 265                 270

aat gag cag cgc atc aga cag aac gtg cag gtg ttt gaa ttc cag ttg     864
Asn Glu Gln Arg Ile Arg Gln Asn Val Gln Val Phe Glu Phe Gln Leu
        275                 280                 285

act tca gag gag atg aaa gcc ata gat ggc cta aac aga aat gtg cga     912
Thr Ser Glu Glu Met Lys Ala Ile Asp Gly Leu Asn Arg Asn Val Arg
    290                 295                 300

tat ttg acc ctt gat att ttt gct ggc ccc cct aat tat cca ttt tct     960
Tyr Leu Thr Leu Asp Ile Phe Ala Gly Pro Pro Asn Tyr Pro Phe Ser
305                 310                 315                 320

gat gaa tat taa                                                     972
Asp Glu Tyr

<210> 82
<211> 323
<212> PRT
<213> Homo sapiens

<400> 82
Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Thr Lys Leu Ala Ile Glu Ala Gly Phe Arg His
        35                  40                  45

Ile Asp Ser Ala His Leu Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Cys Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Val Glu Lys Cys Lys Asp Ala Gly Leu Ala
145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Arg Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr

<210> 83
<211> 994
<212> DNA
<213> Homo sapiens

<220>
<221> CDS
<222> (16)..(978)

<400> 83
gccagatctc ccacc atg gat tcg aaa tat cag tgt gtg aag ctg aat gat    51
                 Met Asp Ser Lys Tyr Gln Cys Val Lys Leu Asn Asp
                  1               5                  10

ggt cac ttc atg cct gtc ctg gga ttt ggc acc tat gcg cct gca gag    99
Gly His Phe Met Pro Val Leu Gly Phe Gly Thr Tyr Ala Pro Ala Glu
        15                  20                  25

gtt cct aaa agt aaa gct tta gag gcc acc aaa ttg gca att gaa gct    147
Val Pro Lys Ser Lys Ala Leu Glu Ala Thr Lys Leu Ala Ile Glu Ala
    30                  35                  40

ggc ttc cgc cat att gat tct gct cat tta tac aat aat gag gag cag    195
Gly Phe Arg His Ile Asp Ser Ala His Leu Tyr Asn Asn Glu Glu Gln
 45                  50                  55                  60

gtt gga ctg gcc atc cga agc aag att gca gat ggc agt gtg aag aga    243
Val Gly Leu Ala Ile Arg Ser Lys Ile Ala Asp Gly Ser Val Lys Arg
                65                  70                  75

gaa gac ata ttc tac act tca aag ctt tgg tgc aat tcc cat cga cca    291
Glu Asp Ile Phe Tyr Thr Ser Lys Leu Trp Cys Asn Ser His Arg Pro
            80                  85                  90

gag ttg gtc cga cca gcc ttg gaa agg tca ctg aaa aat ctt caa ttg    339
Glu Leu Val Arg Pro Ala Leu Glu Arg Ser Leu Lys Asn Leu Gln Leu
        95                  100                 105

gat tat gtt gac ctc tac ctt att cat ttt cca gtg tct gta aag cca    387
Asp Tyr Val Asp Leu Tyr Leu Ile His Phe Pro Val Ser Val Lys Pro
    110                 115                 120

ggt gag gaa gtg atc cca aaa gat gaa aat gga aaa ata cta ttt gac    435
Gly Glu Glu Val Ile Pro Lys Asp Glu Asn Gly Lys Ile Leu Phe Asp
125                 130                 135                 140

aca gtg gat ctc tgt gcc acg tgg gag gcc gtg gag aag tgt aaa gat    483
Thr Val Asp Leu Cys Ala Thr Trp Glu Ala Val Glu Lys Cys Lys Asp
                145                 150                 155

gca gga ttg gcc aag tcc atc ggg gtg tcc aac ttc aac cgc agg cag    531

Ala Gly Leu Ala Lys Ser Ile Gly Val Ser Asn Phe Asn Arg Arg Gln
            160                 165                 170

ctg gag atg atc ctc aac aag cca ggg ctc aag tac aag cct gtc tgc      579
Leu Glu Met Ile Leu Asn Lys Pro Gly Leu Lys Tyr Lys Pro Val Cys
        175                 180                 185

aac cag gtg gaa tgt cat cct tac ttc aac cag aga aaa ctg ctg gat      627
Asn Gln Val Glu Cys His Pro Tyr Phe Asn Gln Arg Lys Leu Leu Asp
    190                 195                 200

ttc tgc aag tca aaa gac att gtt ctg gtt gcc tat agt gct ctg gga      675
Phe Cys Lys Ser Lys Asp Ile Val Leu Val Ala Tyr Ser Ala Leu Gly
205                 210                 215                 220

tcc cac cga gaa gaa cca tgg gtg gac ccg aac tcc ccg gtg ctc ttg      723
Ser His Arg Glu Glu Pro Trp Val Asp Pro Asn Ser Pro Val Leu Leu
                225                 230                 235

gag gac cca gtc ctt tgt gcc ttg gca aaa aag cac aag cga acc cca      771
Glu Asp Pro Val Leu Cys Ala Leu Ala Lys Lys His Lys Arg Thr Pro
            240                 245                 250

gcc ctg att gcc ctg cgc tac cag cta cag cgt ggg gtt gtg gtc ctg      819
Ala Leu Ile Ala Leu Arg Tyr Gln Leu Gln Arg Gly Val Val Val Leu
        255                 260                 265

gcc aag agc tac aat gag cag cgc atc aga cag aac gtg cag gtg ttt      867
Ala Lys Ser Tyr Asn Glu Gln Arg Ile Arg Gln Asn Val Gln Val Phe
    270                 275                 280

gaa ttc cag ttg act tca gag gag atg aaa gcc ata gat ggc cta aac      915
Glu Phe Gln Leu Thr Ser Glu Glu Met Lys Ala Ile Asp Gly Leu Asn
285                 290                 295                 300

aga aat gtg cga tat ttg acc ctt gat att ttt gct ggc ccc cct aat      963
Arg Asn Val Arg Tyr Leu Thr Leu Asp Ile Phe Ala Gly Pro Pro Asn
                305                 310                 315

tat cca ttt tct gat gaatatctcg agggtg                                994
Tyr Pro Phe Ser Asp
            320

<210> 84
<211> 321
<212> PRT
<213> Homo sapiens

<400> 84
Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Thr Lys Leu Ala Ile Glu Ala Gly Phe Arg His
        35                  40                  45

Ile Asp Ser Ala His Leu Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Cys Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Val Glu Lys Cys Lys Asp Ala Gly Leu Ala
145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp


<210> 85
<211> 1219
<212> DNA
<213> Homo sapiens

<220>
<221> CDS
<222> (24)..(992)

<400> 85

tgctaaccag gccagtgaca gaa atg gat tcg aaa tac cag tgt gtg aag ctg 53
Met Asp Ser Lys Tyr Gln Cys Val Lys Leu
1               5                   10

aat gat ggt cac ttc atg cct gtc ctg gga ttt ggc acc tat gcg cct 101
Asn Asp Gly His Phe Met Pro Val Leu Gly Phe Gly Thr Tyr Ala Pro
                15                  20                  25

gca gag gtt cct aaa agt aaa gct cta gag gcc gtc aaa ttg gca ata 149
Ala Glu Val Pro Lys Ser Lys Ala Leu Glu Ala Val Lys Leu Ala Ile
            30                  35                  40

gaa gcc ggg ttc cac cat att gat tct gca cat gtt tac aat aat gag 197
Glu Ala Gly Phe His His Ile Asp Ser Ala His Val Tyr Asn Asn Glu
        45                  50                  55

gag cag gtt gga ctg gcc atc cga agc aag att gca gat ggc agt gtg 245
Glu Gln Val Gly Leu Ala Ile Arg Ser Lys Ile Ala Asp Gly Ser Val
    60                  65                  70

aag aga gaa gac ata ttc tac act tca aag ctt tgg agc aat tcc cat 293
Lys Arg Glu Asp Ile Phe Tyr Thr Ser Lys Leu Trp Ser Asn Ser His
75                  80                  85                  90

cga cca gag ttg gtc cga cca gcc ttg gaa agg tca ctg aaa aat ctt 341
Arg Pro Glu Leu Val Arg Pro Ala Leu Glu Arg Ser Leu Lys Asn Leu
                95                  100                 105

caa ttg gac tat gtt gac ctc tat ctt att cat ttt cca gtg tct gta 389
Gln Leu Asp Tyr Val Asp Leu Tyr Leu Ile His Phe Pro Val Ser Val
            110                 115                 120

aag cca ggt gag gaa gtg atc cca aaa gat gaa aat gga aaa ata cta 437
Lys Pro Gly Glu Glu Val Ile Pro Lys Asp Glu Asn Gly Lys Ile Leu
        125                 130                 135

ttt gac aca gtg gat ctc tgt gcc acg tgg gag gcc atg gag aag tgt 485
Phe Asp Thr Val Asp Leu Cys Ala Thr Trp Glu Ala Met Glu Lys Cys
    140                 145                 150

aaa gat gca gga ttg gcc aag tcc atc ggg gtg tcc aac ttc aac cac 533
Lys Asp Ala Gly Leu Ala Lys Ser Ile Gly Val Ser Asn Phe Asn His
155                 160                 165                 170

agg ctg ctg gag atg atc ctc aac aag cca ggg ctc aag tac aag cct 581
Arg Leu Leu Glu Met Ile Leu Asn Lys Pro Gly Leu Lys Tyr Lys Pro
                175                 180                 185

gtc tgc aac cag gtg gaa tgt cat cct tac ttc aac cag aga aaa ctg 629
Val Cys Asn Gln Val Glu Cys His Pro Tyr Phe Asn Gln Arg Lys Leu
            190                 195                 200

ctg gat ttc tgc aag tca aaa gac att gtt ctg gtt gcc tat agt gct 677
Leu Asp Phe Cys Lys Ser Lys Asp Ile Val Leu Val Ala Tyr Ser Ala
        205                 210                 215

ctg gga tcc cat cga gaa gaa cca tgg gtg gac ccg aac tcc ccg gtg 725
Leu Gly Ser His Arg Glu Glu Pro Trp Val Asp Pro Asn Ser Pro Val
    220                 225                 230

ctc ttg gag gac cca gtc ctt tgt gcc ttg gca aaa aag cac aag cga 773

Leu Leu Glu Asp Pro Val Leu Cys Ala Leu Ala Lys Lys His Lys Arg
235                 240                 245                 250

acc cca gcc ctg att gcc ctg cgc tac cag ctg cag cgt ggg gtt gtg     821
Thr Pro Ala Leu Ile Ala Leu Arg Tyr Gln Leu Gln Arg Gly Val Val
                255                 260                 265

gtc ctg gcc aag agc tac aat gag cag cgc atc aga cag aac gtg cag     869
Val Leu Ala Lys Ser Tyr Asn Glu Gln Arg Ile Arg Gln Asn Val Gln
            270                 275                 280

gtg ttt gaa ttc cag ttg act tca gag gag atg aaa gcc ata gat ggc     917
Val Phe Glu Phe Gln Leu Thr Ser Glu Glu Met Lys Ala Ile Asp Gly
        285                 290                 295

cta aac aga aat gtg cga tat ttg acc ctt gat att ttt gct ggc ccc     965
Leu Asn Arg Asn Val Arg Tyr Leu Thr Leu Asp Ile Phe Ala Gly Pro
    300                 305                 310

cct aat tat cca ttt tct gat gaa tat taacatggag ggcattgcat          1012
Pro Asn Tyr Pro Phe Ser Asp Glu Tyr
315                 320

gaggtctgcc agaaggccct gcgtgtggat ggtgacacag aggatggctc tatgctggtg 1072

actggacaca tcgcctctgg ttaaatctct cctgcttggc gacttcagta agctacagct 1132

aagcccatcg gccggaaaag aaagacaata attttgtttt ttcattttga aaaaattaaa 1192

tgctctctcc taaagattct tcaccta                                      1219

<210> 86
<211> 323
<212> PRT
<213> Homo sapiens

<400> 86
Met Asp Ser Lys Tyr Gln Cys Val Lys Leu Asn Asp Gly His Phe Met
  1               5                  10                  15

Pro Val Leu Gly Phe Gly Thr Tyr Ala Pro Ala Glu Val Pro Lys Ser
             20                  25                  30

Lys Ala Leu Glu Ala Val Lys Leu Ala Ile Glu Ala Gly Phe His His
         35                  40                  45

Ile Asp Ser Ala His Val Tyr Asn Asn Glu Glu Gln Val Gly Leu Ala
     50                  55                  60

Ile Arg Ser Lys Ile Ala Asp Gly Ser Val Lys Arg Glu Asp Ile Phe
 65                  70                  75                  80

Tyr Thr Ser Lys Leu Trp Ser Asn Ser His Arg Pro Glu Leu Val Arg
                 85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Met Glu Lys Cys Lys Asp Ala Gly Leu Ala
145                 150                 155                 160

Lys Ser Ile Gly Val Ser Asn Phe Asn His Arg Leu Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr


<210> 87
<211> 1001
<212> DNA
<213> Homo sapiens

<220>
<221> CDS
<222> (11)..(982)

<400> 87
catctaggcc acc atg gcc atg gat tcg aaa tat cag tgt gtg aag ctg      49
           Thr Met Ala Met Asp Ser Lys Tyr Gln Cys Val Lys Leu
            1               5                   10

aat gat ggt cac ttc atg cct gtc ctg gga ttt ggc acc tat gcg cct      97
Asn Asp Gly His Phe Met Pro Val Leu Gly Phe Gly Thr Tyr Ala Pro
    15                  20                  25

gca gag gtt cct aaa agt aaa gct tta gag gcc acc aaa ttg gca att     145
Ala Glu Val Pro Lys Ser Lys Ala Leu Glu Ala Thr Lys Leu Ala Ile
30                  35                  40                  45

gaa gct ggc ttc cgc cat att gat tct gct cat tta tac aat aat gag     193
Glu Ala Gly Phe Arg His Ile Asp Ser Ala His Leu Tyr Asn Asn Glu
                50                  55                  60

gag cag gtt gga ctg gcc atc cga agc aag att gca gat ggc agt gtg     241
Glu Gln Val Gly Leu Ala Ile Arg Ser Lys Ile Ala Asp Gly Ser Val
            65                  70                  75

aag aga gaa gac ata ttc tac act tca aag ctt tgg tgc aat tcc cat     289
Lys Arg Glu Asp Ile Phe Tyr Thr Ser Lys Leu Trp Cys Asn Ser His
        80                  85                  90

cga cca gag ttg gtc cga cca gcc ttg gaa agg tca ctg aaa aat ctt     337
Arg Pro Glu Leu Val Arg Pro Ala Leu Glu Arg Ser Leu Lys Asn Leu
    95                  100                 105

caa ttg gat tat gtt gac ctc tac ctt att cat ttt cca gtg tct gta     385
Gln Leu Asp Tyr Val Asp Leu Tyr Leu Ile His Phe Pro Val Ser Val
110                 115                 120                 125

aag cca ggt gag gaa gtg atc cca aaa gat gaa aat gga aaa ata cta     433
Lys Pro Gly Glu Glu Val Ile Pro Lys Asp Glu Asn Gly Lys Ile Leu
                130                 135                 140

ttt gac aca gtg gat ctc tgt gcc acg tgg gag gcc gtg gag aag tgt     481
Phe Asp Thr Val Asp Leu Cys Ala Thr Trp Glu Ala Val Glu Lys Cys
            145                 150                 155

aaa gat gca gga ttg gcc aag tcc atc ggg gtg tcc aac ttc aac cgc     529
Lys Asp Ala Gly Leu Ala Lys Ser Ile Gly Val Ser Asn Phe Asn Arg
        160                 165                 170

agg cag ctg gag atg atc ctc aac aag cca ggg ctc aag tac aag cct     577
Arg Gln Leu Glu Met Ile Leu Asn Lys Pro Gly Leu Lys Tyr Lys Pro
    175                 180                 185

gtc tgc aac cag gtg gaa tgt cat cct tac ttc aac cag aga aaa ctg     625
Val Cys Asn Gln Val Glu Cys His Pro Tyr Phe Asn Gln Arg Lys Leu
190                 195                 200                 205

ctg gat ttc tgc aag tca aaa gac att gtt ctg gtt gcc tat agt gct     673
Leu Asp Phe Cys Lys Ser Lys Asp Ile Val Leu Val Ala Tyr Ser Ala
                210                 215                 220

ctg gga tcc cac cga gaa gaa cca tgg gtg gac ccg aac tcc ccg gtg     721
Leu Gly Ser His Arg Glu Glu Pro Trp Val Asp Pro Asn Ser Pro Val
            225                 230                 235

ctc ttg gag gac cca gtc ctt tgt gcc ttg gca aaa aag cac aag cga     769
Leu Leu Glu Asp Pro Val Leu Cys Ala Leu Ala Lys Lys His Lys Arg
        240                 245                 250

acc cca gcc ctg att gcc ctg cgc tac cag cta cag cgt ggg gtt gtg     817
Thr Pro Ala Leu Ile Ala Leu Arg Tyr Gln Leu Gln Arg Gly Val Val
    255                 260                 265

gtc ctg gcc aag agc tac aat gag cag cgc atc aga cag aac gtg cag     865
Val Leu Ala Lys Ser Tyr Asn Glu Gln Arg Ile Arg Gln Asn Val Gln
270                 275                 280                 285

gtg ttt gaa ttc cag ttg act tca gag gag atg aaa gcc ata gat ggc     913

Val Phe Glu Phe Gln Leu Thr Ser Glu Glu Met Lys Ala Ile Asp Gly
                290                 295                 300

cta aac aga aat gtg cga tat ttg acc ctt gat att ttt gct ggc ccc     961
Leu Asn Arg Asn Val Arg Tyr Leu Thr Leu Asp Ile Phe Ala Gly Pro
            305                 310                 315

cct aat tat cca ttt tct gat gaatattaaa cgcgtgatc                    1001
Pro Asn Tyr Pro Phe Ser Asp
        320

<210> 88
<211> 324
<212> PRT
<213> Homo sapiens

<400> 88
Thr Met Ala Met Asp Ser Lys Tyr Gln Cys Val Lys Leu Asn Asp Gly
 1               5                  10                  15

His Phe Met Pro Val Leu Gly Phe Gly Thr Tyr Ala Pro Ala Glu Val
            20                  25                  30

Pro Lys Ser Lys Ala Leu Glu Ala Thr Lys Leu Ala Ile Glu Ala Gly
        35                  40                  45

Phe Arg His Ile Asp Ser Ala His Leu Tyr Asn Asn Glu Glu Gln Val
    50                  55                  60

Gly Leu Ala Ile Arg Ser Lys Ile Ala Asp Gly Ser Val Lys Arg Glu
65                  70                  75                  80

Asp Ile Phe Tyr Thr Ser Lys Leu Trp Cys Asn Ser His Arg Pro Glu
                85                  90                  95

Leu Val Arg Pro Ala Leu Glu Arg Ser Leu Lys Asn Leu Gln Leu Asp
            100                 105                 110

Tyr Val Asp Leu Tyr Leu Ile His Phe Pro Val Ser Val Lys Pro Gly
        115                 120                 125

Glu Glu Val Ile Pro Lys Asp Glu Asn Gly Lys Ile Leu Phe Asp Thr
    130                 135                 140

Val Asp Leu Cys Ala Thr Trp Glu Ala Val Glu Lys Cys Lys Asp Ala
145                 150                 155                 160

Gly Leu Ala Lys Ser Ile Gly Val Ser Asn Phe Asn Arg Arg Gln Leu
                165                 170                 175

Glu Met Ile Leu Asn Lys Pro Gly Leu Lys Tyr Lys Pro Val Cys Asn
            180                 185                 190

Gln Val Glu Cys His Pro Tyr Phe Asn Gln Arg Lys Leu Leu Asp Phe
        195                 200                 205

Cys Lys Ser Lys Asp Ile Val Leu Val Ala Tyr Ser Ala Leu Gly Ser
    210                 215                 220

His Arg Glu Glu Pro Trp Val Asp Pro Asn Ser Pro Val Leu Leu Glu
225                 230                 235                 240

Asp Pro Val Leu Cys Ala Leu Ala Lys Lys His Lys Arg Thr Pro Ala
                245                 250                 255

Leu Ile Ala Leu Arg Tyr Gln Leu Gln Arg Gly Val Val Val Leu Ala
            260                 265                 270

Lys Ser Tyr Asn Glu Gln Arg Ile Arg Gln Asn Val Gln Val Phe Glu
        275                 280                 285

Phe Gln Leu Thr Ser Glu Glu Met Lys Ala Ile Asp Gly Leu Asn Arg
    290                 295                 300

Asn Val Arg Tyr Leu Thr Leu Asp Ile Phe Ala Gly Pro Pro Asn Tyr
305                 310                 315                 320

Pro Phe Ser Asp


<210> 89
<211> 1012
<212> DNA
<213> Homo sapiens

<220>
<221> CDS
<222> (1)..(1011)

<400> 89
gcc ggt acc acc atg ggc cac cat cac cac cat cac gat tcg aaa tat      48
Ala Gly Thr Thr Met Gly His His His His His His Asp Ser Lys Tyr
  1               5                  10                  15

cag tgt gtg aag ctg aat gat ggt cac ttc atg cct gtc ctg gga ttt      96
Gln Cys Val Lys Leu Asn Asp Gly His Phe Met Pro Val Leu Gly Phe
             20                  25                  30

ggc acc tat gcg cct gca gag gtt cct aaa agt aaa gct tta gag gcc     144
Gly Thr Tyr Ala Pro Ala Glu Val Pro Lys Ser Lys Ala Leu Glu Ala
         35                  40                  45

acc aaa ttg gca att gaa gct ggc ttc cgc cat att gat tct gct cat     192
Thr Lys Leu Ala Ile Glu Ala Gly Phe Arg His Ile Asp Ser Ala His
     50                  55                  60

tta tac aat aat gag gag cag gtt gga ctg gcc atc cga agc aag att     240
Leu Tyr Asn Asn Glu Glu Gln Val Gly Leu Ala Ile Arg Ser Lys Ile
 65                  70                  75                  80

gca gat ggc agt gtg aag aga gaa gac ata ttc tac act tca aag ctt     288
Ala Asp Gly Ser Val Lys Arg Glu Asp Ile Phe Tyr Thr Ser Lys Leu
                 85                  90                  95

tgg tgc aat tcc cat cga cca gag ttg gtc cga cca gcc ttg gaa agg     336
Trp Cys Asn Ser His Arg Pro Glu Leu Val Arg Pro Ala Leu Glu Arg
            100                 105                 110

tca ctg aaa aat ctt caa ttg gat tat gtt gac ctc tac ctt att cat     384
Ser Leu Lys Asn Leu Gln Leu Asp Tyr Val Asp Leu Tyr Leu Ile His
        115                 120                 125

ttt cca gtg tct gta aag cca ggt gag gaa gtg atc cca aaa gat gaa     432
Phe Pro Val Ser Val Lys Pro Gly Glu Glu Val Ile Pro Lys Asp Glu
    130                 135                 140

aat gga aaa ata cta ttt gac aca gtg gat ctc tgt gcc acg tgg gag     480
Asn Gly Lys Ile Leu Phe Asp Thr Val Asp Leu Cys Ala Thr Trp Glu
145                 150                 155                 160

gcc gtg gag aag tgt aaa gat gca gga ttg gcc aag tcc atc ggg gtg     528
Ala Val Glu Lys Cys Lys Asp Ala Gly Leu Ala Lys Ser Ile Gly Val
                165                 170                 175

tcc aac ttc aac cgc agg cag ctg gag atg atc ctc aac aag cca ggg     576
Ser Asn Phe Asn Arg Arg Gln Leu Glu Met Ile Leu Asn Lys Pro Gly
            180                 185                 190

ctc aag tac aag cct gtc tgc aac cag gtg gaa tgt cat cct tac ttc     624
Leu Lys Tyr Lys Pro Val Cys Asn Gln Val Glu Cys His Pro Tyr Phe
        195                 200                 205

aac cag aga aaa ctg ctg gat ttc tgc aag tca aaa gac att gtt ctg     672
Asn Gln Arg Lys Leu Leu Asp Phe Cys Lys Ser Lys Asp Ile Val Leu
    210                 215                 220

gtt gcc tat agt gct ctg gga tcc cac cga gaa gaa cca tgg gtg gac     720
Val Ala Tyr Ser Ala Leu Gly Ser His Arg Glu Glu Pro Trp Val Asp
225                 230                 235                 240

ccg aac tcc ccg gtg ctc ttg gag gac cca gtc ctt tgt gcc ttg gca     768
Pro Asn Ser Pro Val Leu Leu Glu Asp Pro Val Leu Cys Ala Leu Ala
                245                 250                 255

aaa aag cac aag cga acc cca gcc ctg att gcc ctg cgc tac cag cta     816
Lys Lys His Lys Arg Thr Pro Ala Leu Ile Ala Leu Arg Tyr Gln Leu
            260                 265                 270

cag cgt ggg gtt gtg gtc ctg gcc aag agc tac aat gag cag cgc atc     864
Gln Arg Gly Val Val Val Leu Ala Lys Ser Tyr Asn Glu Gln Arg Ile
        275                 280                 285

aga cag aac gtg cag gtg ttt gaa ttc cag ttg act tca gag gag atg     912
Arg Gln Asn Val Gln Val Phe Glu Phe Gln Leu Thr Ser Glu Glu Met
    290                 295                 300

aaa gcc ata gat ggc cta aac aga aat gtg cga tat ttg acc ctt gat     960
Lys Ala Ile Asp Gly Leu Asn Arg Asn Val Arg Tyr Leu Thr Leu Asp
305                 310                 315                 320

att ttt gct ggc ccc cct aat tat cca ttt tct gat gaa tat ctc gag     1008
Ile Phe Ala Gly Pro Pro Asn Tyr Pro Phe Ser Asp Glu Tyr Leu Glu
                325                 330                 335

ggt g                                                               1012
Gly

<210> 90
<211> 337
<212> PRT
<213> Homo sapiens

<400> 90
Ala Gly Thr Thr Met Gly His His His His His His Asp Ser Lys Tyr
1               5                   10                  15

Gln Cys Val Lys Leu Asn Asp Gly His Phe Met Pro Val Leu Gly Phe
            20                  25                  30

Gly Thr Tyr Ala Pro Ala Glu Val Pro Lys Ser Lys Ala Leu Glu Ala
        35                  40                  45

Thr Lys Leu Ala Ile Glu Ala Gly Phe Arg His Ile Asp Ser Ala His
    50                  55                  60

Leu Tyr Asn Asn Glu Glu Gln Val Gly Leu Ala Ile Arg Ser Lys Ile
65                  70                  75                  80

Ala Asp Gly Ser Val Lys Arg Glu Asp Ile Phe Tyr Thr Ser Lys Leu
                85                  90                  95

Trp Cys Asn Ser His Arg Pro Glu Leu Val Arg Pro Ala Leu Glu Arg
            100                 105                 110

Ser Leu Lys Asn Leu Gln Leu Asp Tyr Val Asp Leu Tyr Leu Ile His
        115                 120                 125

Phe Pro Val Ser Val Lys Pro Gly Glu Glu Val Ile Pro Lys Asp Glu
    130                 135                 140

Asn Gly Lys Ile Leu Phe Asp Thr Val Asp Leu Cys Ala Thr Trp Glu
145                 150                 155                 160

Ala Val Glu Lys Cys Lys Asp Ala Gly Leu Ala Lys Ser Ile Gly Val
                165                 170                 175

Ser Asn Phe Asn Arg Arg Gln Leu Glu Met Ile Leu Asn Lys Pro Gly
            180                 185                 190

Leu Lys Tyr Lys Pro Val Cys Asn Gln Val Glu Cys His Pro Tyr Phe
        195                 200                 205

Asn Gln Arg Lys Leu Leu Asp Phe Cys Lys Ser Lys Asp Ile Val Leu
    210                 215                 220

Val Ala Tyr Ser Ala Leu Gly Ser His Arg Glu Glu Pro Trp Val Asp
225                 230                 235                 240

Pro Asn Ser Pro Val Leu Leu Glu Asp Pro Val Leu Cys Ala Leu Ala
                245                 250                 255

Lys Lys His Lys Arg Thr Pro Ala Leu Ile Ala Leu Arg Tyr Gln Leu
            260                 265                 270

Gln Arg Gly Val Val Val Leu Ala Lys Ser Tyr Asn Glu Gln Arg Ile
        275                 280                 285

Arg Gln Asn Val Gln Val Phe Glu Phe Gln Leu Thr Ser Glu Glu Met
    290                 295                 300

Lys Ala Ile Asp Gly Leu Asn Arg Asn Val Arg Tyr Leu Thr Leu Asp
305                 310                 315                 320

Ile Phe Ala Gly Pro Pro Asn Tyr Pro Phe Ser Asp Glu Tyr Leu Glu
                325                 330                 335

Gly

<210> 91
<211> 1225
<212> DNA
<213> Homo sapiens

<220>
<221> CDS
<222> (24)..(992)

<400> 91
tgctaaccag gccagtgaca gaa atg gat tcg aaa tac cag tgt gtg aag ctg   53
                          Met Asp Ser Lys Tyr Gln Cys Val Lys Leu
                          1               5                   10

aat gat ggt cac ttc atg cct gtc ctg gga ttt ggc acc tat gcg cct   101
Asn Asp Gly His Phe Met Pro Val Leu Gly Phe Gly Thr Tyr Ala Pro
                15                  20                  25

gca gag gtt cct aaa agt aaa gct cta gag gcc gtc aaa ttg gca ata   149
Ala Glu Val Pro Lys Ser Lys Ala Leu Glu Ala Val Lys Leu Ala Ile
            30                  35                  40

gaa gcc ggg ttc cac cat att gat tct gca cat gtt tac aat aat gag   197
Glu Ala Gly Phe His His Ile Asp Ser Ala His Val Tyr Asn Asn Glu
        45                  50                  55

gag cag gtt gga ctg gcc atc cga agc aag att gca gat ggc agt gtg   245
Glu Gln Val Gly Leu Ala Ile Arg Ser Lys Ile Ala Asp Gly Ser Val
    60                  65                  70

aag aga gaa gac ata ttc tac act tca aag ctt tgg agc aat tcc cat   293
Lys Arg Glu Asp Ile Phe Tyr Thr Ser Lys Leu Trp Ser Asn Ser His
75                  80                  85                  90

cga cca gag ttg gtc cga cca gcc ttg gaa agg tca ctg aaa aat ctt   341
Arg Pro Glu Leu Val Arg Pro Ala Leu Glu Arg Ser Leu Lys Asn Leu
                95                  100                 105

caa ttg gac tat gtt gac ctc tat ctt att cat ttt cca gtg tct gta   389
Gln Leu Asp Tyr Val Asp Leu Tyr Leu Ile His Phe Pro Val Ser Val
            110                 115                 120

aag cca ggt gag gaa gtg atc cca aaa gat gaa aat gga aaa ata cta   437
Lys Pro Gly Glu Glu Val Ile Pro Lys Asp Glu Asn Gly Lys Ile Leu
        125                 130                 135

ttt gac aca gtg gat ctc tgt gcc acg tgg gag gcc atg gag aag tgt   485
Phe Asp Thr Val Asp Leu Cys Ala Thr Trp Glu Ala Met Glu Lys Cys
    140                 145                 150

aaa gat gca gga ttg gcc aag tcc atc ggg gtg tcc aac ttc aac cac   533
Lys Asp Ala Gly Leu Ala Lys Ser Ile Gly Val Ser Asn Phe Asn His
155                 160                 165                 170

agg ctg ctg gag atg atc ctc aac aag cca ggg ctc aag tac aag cct   581

Arg Leu Leu Glu Met Ile Leu Asn Lys Pro Gly Leu Lys Tyr Lys Pro
                175                 180                 185

gtc tgc aac cag gtg gaa tgt cat cct tac ttc aac cag aga aaa ctg 629
Val Cys Asn Gln Val Glu Cys His Pro Tyr Phe Asn Gln Arg Lys Leu
            190                 195                 200

ctg gat ttc tgc aag tca aaa gac att gtt ctg gtt gcc tat agt gct 677
Leu Asp Phe Cys Lys Ser Lys Asp Ile Val Leu Val Ala Tyr Ser Ala
        205                 210                 215

ctg gga tcc cat cga gaa gaa cca tgg gtg gac ccg aac tcc ccg gtg 725
Leu Gly Ser His Arg Glu Glu Pro Trp Val Asp Pro Asn Ser Pro Val
    220                 225                 230

ctc ttg gag gac cca gtc ctt tgt gcc ttg gca aaa aag cac aag cga 773
Leu Leu Glu Asp Pro Val Leu Cys Ala Leu Ala Lys Lys His Lys Arg
235                 240                 245                 250

acc cca gcc ctg att gcc ctg cgc tac cag ctg cag cgt ggg gtt gtg 821
Thr Pro Ala Leu Ile Ala Leu Arg Tyr Gln Leu Gln Arg Gly Val Val
                255                 260                 265

gtc ctg gcc aag agc tac aat gag cag cgc atc aga cag aac gtg cag 869
Val Leu Ala Lys Ser Tyr Asn Glu Gln Arg Ile Arg Gln Asn Val Gln
            270                 275                 280

gtg ttt gaa ttc cag ttg act tca gag gag atg aaa gcc ata gat ggc 917
Val Phe Glu Phe Gln Leu Thr Ser Glu Glu Met Lys Ala Ile Asp Gly
        285                 290                 295

cta aac aga aat gtg cga tat ttg acc ctt gat att ttt gct ggc ccc 965
Leu Asn Arg Asn Val Arg Tyr Leu Thr Leu Asp Ile Phe Ala Gly Pro
    300                 305                 310

cct aat tat cca ttt tct gat gaa tat taacatggag ggcattgcat 1012
Pro Asn Tyr Pro Phe Ser Asp Glu Tyr
315                 320

gaggtctgcc agaaggccct gcgtgtggat ggtgacacag aggatggctc tatgctggtg 1072

actggacaca tcgcctctgg ttaaatctct cctgcttggc gacttcagta agctacagct 1132

aagcccatcg gccggaaaag aaagacaata attttgtttt ttcattttga aaaaattaaa 1192

tgctctctcc taaagattct tcacctaaaa aaa 1225

<210> 92
<211> 323
<212> PRT
<213> Homo sapiens

<400> 92
Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Val Lys Leu Ala Ile Glu Ala Gly Phe His His
        35                  40                  45

Ile Asp Ser Ala His Val Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Ser Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Met Glu Lys Cys Lys Asp Ala Gly Leu Ala
145                 150                 155                 160

Lys Ser Ile Gly Val Ser Asn Phe Asn His Arg Leu Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr


<210> 93
<211> 996
<212> DNA
<213> Homo sapiens

<220>

<221> CDS
<222> (16)..(984)

<400> 93
caccgcggcc gcacc atg gat tcg aaa tat cag tgt gtg aag ctg aat gat    51
                 Met Asp Ser Lys Tyr Gln Cys Val Lys Leu Asn Asp
                 1               5                   10

ggt cac ttc atg cct gtc ctg gga ttt ggc acc tat gcg cct gca gag    99
Gly His Phe Met Pro Val Leu Gly Phe Gly Thr Tyr Ala Pro Ala Glu
        15                  20                  25

gtt cct aaa agt aaa gct tta gag gcc acc aaa ttg gca att gaa gct    147
Val Pro Lys Ser Lys Ala Leu Glu Ala Thr Lys Leu Ala Ile Glu Ala
    30                  35                  40

ggc ttc cgc cat att gat tct gct cat tta tac aat aat gag gag cag    195
Gly Phe Arg His Ile Asp Ser Ala His Leu Tyr Asn Asn Glu Glu Gln
45                  50                  55                  60

gtt gga ctg gcc atc cga agc aag att gca gat ggc agt gtg aag aga    243
Val Gly Leu Ala Ile Arg Ser Lys Ile Ala Asp Gly Ser Val Lys Arg
                65                  70                  75

gaa gac ata ttc tac act tca aag ctt tgg tgc aat tcc cat cga cca    291
Glu Asp Ile Phe Tyr Thr Ser Lys Leu Trp Cys Asn Ser His Arg Pro
            80                  85                  90

gag ttg gtc cga cca gcc ttg gaa agg tca ctg aaa aat ctt caa ttg    339
Glu Leu Val Arg Pro Ala Leu Glu Arg Ser Leu Lys Asn Leu Gln Leu
        95                  100                 105

gat tat gtt gac ctc tac ctt att cat ttt cca gtg tct gta aag cca    387
Asp Tyr Val Asp Leu Tyr Leu Ile His Phe Pro Val Ser Val Lys Pro
    110                 115                 120

ggt gag gaa gtg atc cca aaa gat gaa aat gga aaa ata cta ttt gac    435
Gly Glu Glu Val Ile Pro Lys Asp Glu Asn Gly Lys Ile Leu Phe Asp
125                 130                 135                 140

aca gtg gat ctc tgt gcc acg tgg gag gcc gtg gag aag tgt aaa gat    483
Thr Val Asp Leu Cys Ala Thr Trp Glu Ala Val Glu Lys Cys Lys Asp
                145                 150                 155

gca gga ttg gcc aag tcc atc ggg gtg tcc aac ttc aac cgc agg cag    531
Ala Gly Leu Ala Lys Ser Ile Gly Val Ser Asn Phe Asn Arg Arg Gln
            160                 165                 170

ctg gag atg atc ctc aac aag cca ggg ctc aag tac aag cct gtc tgc    579
Leu Glu Met Ile Leu Asn Lys Pro Gly Leu Lys Tyr Lys Pro Val Cys
        175                 180                 185

aac cag gtg gaa tgt cat cct tac ttc aac cag aga aaa ctg ctg gat    627
Asn Gln Val Glu Cys His Pro Tyr Phe Asn Gln Arg Lys Leu Leu Asp
    190                 195                 200

ttc tgc aag tca aaa gac att gtt ctg gtt gcc tat agt gct ctg gga    675
Phe Cys Lys Ser Lys Asp Ile Val Leu Val Ala Tyr Ser Ala Leu Gly
205                 210                 215                 220

tcc cac cga gaa gaa cca tgg gtg gac ccg aac tcc ccg gtg ctc ttg    723

Ser His Arg Glu Glu Pro Trp Val Asp Pro Asn Ser Pro Val Leu Leu
                225                 230                 235

gag gac cca gtc ctt tgt gcc ttg gca aaa aag cac aag cga acc cca 771
Glu Asp Pro Val Leu Cys Ala Leu Ala Lys Lys His Lys Arg Thr Pro
            240                 245                 250

gcc ctg att gcc ctg cgc tac cag cta cag cgt ggg gtt gtg gtc ctg 819
Ala Leu Ile Ala Leu Arg Tyr Gln Leu Gln Arg Gly Val Val Val Leu
        255                 260                 265

gcc aag agc tac aat gag cag cgc atc aga cag aac gtg cag gtg ttt 867
Ala Lys Ser Tyr Asn Glu Gln Arg Ile Arg Gln Asn Val Gln Val Phe
    270                 275                 280

gaa ttc cag ttg act tca gag gag atg aaa gcc ata gat ggc cta aac 915
Glu Phe Gln Leu Thr Ser Glu Glu Met Lys Ala Ile Asp Gly Leu Asn
285                 290                 295                 300

aga aat gtg cga tat ttg acc ctt gat att ttt gct ggc ccc cct aat 963
Arg Asn Val Arg Tyr Leu Thr Leu Asp Ile Phe Ala Gly Pro Pro Asn
                305                 310                 315

tat cca ttt tct gat gaa tat taggtcgacg gc 996
Tyr Pro Phe Ser Asp Glu Tyr
            320

<210> 94
<211> 323
<212> PRT
<213> Homo sapiens

<400> 94
Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Thr Lys Leu Ala Ile Glu Ala Gly Phe Arg His
        35                  40                  45

Ile Asp Ser Ala His Leu Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Cys Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Val Glu Lys Cys Lys Asp Ala Gly Leu Ala

145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr


<210> 95
<211> 3815
<212> DNA
<213> Homo sapiens

<220>
<221> CDS
<222> (263)..(1789)

<400> 95
   cggccgcgat ccccaccaca ccaccagccc ggccgcacgg ggcactgagc cgggtgctga   60
   gcaccggagg ccccgccgag gccgggactc agatgttgaa agttaatttg tgtaaagact  120
   tatgcacgtg gtgacatgag ttctgcccag tgctctgaaa tcaaagtgaa gaaataaatc  180
   catggaagcc caggcaaatg atgggtgtag ctatgactct ctgaaggacc tgcagagaaa  240
   cgcctcctga ttttgtctta ca atg gaa ctt aaa aag tcg cct gac ggt gga   292
                            Met Glu Leu Lys Lys Ser Pro Asp Gly Gly
                            1               5                   10
   tgg ggc tgg gtg att gtg ttt gtc tcc ttc ctt act cag ttt ttg tgt    340
   Trp Gly Trp Val Ile Val Phe Val Ser Phe Leu Thr Gln Phe Leu Cys
                   15                  20                  25
   tac gga tcc cca cta gct gtt gga gtc ctg tac ata gaa tgg ctg gat 388
   Tyr Gly Ser Pro Leu Ala Val Gly Val Leu Tyr Ile Glu Trp Leu Asp
   30 35 40
   gcc ttt ggt gaa gga aaa gga aaa aca gcc tgg gtt gga tcc ctg gca 436
   Ala Phe Gly Glu Gly Lys Gly Lys Thr Ala Trp Val Gly Ser Leu Ala
   45 50 55
   agt gga gtt ggc ttg ctt gca agt cct gtc tgc agt ctc tgt gtc tca 484
   Ser Gly Val Gly Leu Leu Ala Ser Pro Val Cys Ser Leu Cys Val Ser
   60 65 70
   tct ttt gga gca aga cct gtc aca atc ttc agt ggc ttc atg gtg gct 532
   Ser Phe Gly Ala Arg Pro Val Thr Ile Phe Ser Gly Phe Met Val Ala
   75 80 85 90
   gga ggc ctg atg ttg agc agt ttt gct ccc aat atc tac ttt ctg ttt 580
   Gly Gly Leu Met Leu Ser Ser Phe Ala Pro Asn Ile Tyr Phe Leu Phe
   95 100 105
   ttt tcc tat ggc att gtt gta ggt ctt gga tgt ggt tta tta tac act 628
   Phe Ser Tyr Gly Ile Val Val Gly Leu Gly Cys Gly Leu Leu Tyr Thr
   110 115 120
   gca aca gtg acc att acg tgc cag tat ttt gac gat cgc cga ggc cta 676
   Ala Thr Val Thr Ile Thr Cys Gln Tyr Phe Asp Asp Arg Arg Gly Leu
   125 130 135
   gcg ctt ggc ctg att tca aca ggt tca agc gtt ggc ctt ttc ata tat 724
   Ala Leu Gly Leu Ile Ser Thr Gly Ser Ser Val Gly Leu Phe Ile Tyr
   140 145 150
   gct gct ctg cag agg atg ctg gtt gag ttc tat gga ctg gat gga tgc 772
   Ala Ala Leu Gln Arg Met Leu Val Glu Phe Tyr Gly Leu Asp Gly Cys
   155 160 165 170
   ttg ctg att gtg ggt gct tta gct tta aat ata tta gcc tgt ggc agt 820
   Leu Leu Ile Val Gly Ala Leu Ala Leu Asn Ile Leu Ala Cys Gly Ser
   175 180 185
   ctg atg aga ccc ctc caa tct tct gat tgt cct ttg cct aaa aaa ata 868
   Leu Met Arg Pro Leu Gln Ser Ser Asp Cys Pro Leu Pro Lys Lys Ile
   190 195 200
   gct cca gaa gat cta cca gat aaa tac tcc att tac aat gaa aaa gga 916
   Ala Pro Glu Asp Leu Pro Asp Lys Tyr Ser Ile Tyr Asn Glu Lys Gly
   205 210 215
   aag aat ctg gaa gaa aac ata aac att ctt gac aag agc tac agt agt 964
   Lys Asn Leu Glu Glu Asn Ile Asn Ile Leu Asp Lys Ser Tyr Ser Ser
   220 225 230
   gag gaa aaa tgc agg atc acg tta gcc aat ggt gac tgg aaa caa gac 1012
   Glu Glu Lys Cys Arg Ile Thr Leu Ala Asn Gly Asp Trp Lys Gln Asp
   235 240 245 250
   agc cta ctt cat aaa aac ccc aca gtg aca cac aca aaa gag cct gaa 1060
   Ser Leu Leu His Lys Asn Pro Thr Val Thr His Thr Lys Glu Pro Glu
   255 260 265
   acg tac aaa aag aaa gtt gca gaa cag aca tat ttt tgc aaa cag ctt 1108
   Thr Tyr Lys Lys Lys Val Ala Glu Gln Thr Tyr Phe Cys Lys Gln Leu
               270                 275                 280
   gcc aag agg aag tgg cag tta tat aaa aac tac tgt ggt gaa act gtg     1156
   Ala Lys Arg Lys Trp Gln Leu Tyr Lys Asn Tyr Cys Gly Glu Thr Val
           285                 290                 295
   gct ctt ttt aaa aac aaa gta ttt tca gcc ctt ttc att gct atc tta     1204
   Ala Leu Phe Lys Asn Lys Val Phe Ser Ala Leu Phe Ile Ala Ile Leu
       300                 305                 310
   ctc ttt gac atc gga ggg ttt cca cct tca tta ctt atg gaa gat gta     1252
   Leu Phe Asp Ile Gly Gly Phe Pro Pro Ser Leu Leu Met Glu Asp Val
   315                 320                 325                 330
   gca aga agt tca aac gtg aaa gaa gaa gag ttt att atg cca ctt att     1300
   Ala Arg Ser Ser Asn Val Lys Glu Glu Glu Phe Ile Met Pro Leu Ile
                   335                 340                 345
   tcc att ata ggc att atg aca gca gtt ggt aaa ctg ctt tta ggg ata     1348
   Ser Ile Ile Gly Ile Met Thr Ala Val Gly Lys Leu Leu Leu Gly Ile
               350                 355                 360
   ctg gct gac ttc aag tgg att aat acc ttg tat ctt tat gtt gct acc     1396
   Leu Ala Asp Phe Lys Trp Ile Asn Thr Leu Tyr Leu Tyr Val Ala Thr
           365                 370                 375
   tta atc atc atg ggc cta gcc ttg tgt gca att cca ttt gcc aaa agc     1444
   Leu Ile Ile Met Gly Leu Ala Leu Cys Ala Ile Pro Phe Ala Lys Ser
       380                 385                 390
   tat gtc aca ttg gcg ttg ctt tct ggg atc cta ggg ttt ctt act ggt     1492
   Tyr Val Thr Leu Ala Leu Leu Ser Gly Ile Leu Gly Phe Leu Thr Gly
   395                 400                 405                 410
   aat tgg tcc atc ttt cca tat gtg acc acg aag act gtg gga att gaa     1540
   Asn Trp Ser Ile Phe Pro Tyr Val Thr Thr Lys Thr Val Gly Ile Glu
                   415                 420                 425
   aaa tta gcc cat gcc tat ggg ata tta atg ttc ttt gct gga ctt gga     1588
   Lys Leu Ala His Ala Tyr Gly Ile Leu Met Phe Phe Ala Gly Leu Gly
               430                 435                 440
   aat agc cta gga cca cca tcg ttg ggt tgg ttt tat gac tgg acc cag     1636
   Asn Ser Leu Gly Pro Pro Ser Leu Gly Trp Phe Tyr Asp Trp Thr Gln
           445                 450                 455
   acc tat gat att gca ttt tat ttt agt ggc ttc tgc gtc ctg ctg gga     1684
   Thr Tyr Asp Ile Ala Phe Tyr Phe Ser Gly Phe Cys Val Leu Leu Gly
       460                 465                 470
   ggt ttt att ctg ctg ctg gca gcc ttg ccc tct tgg gat aca tgc aac     1732
   Gly Phe Ile Leu Leu Leu Ala Ala Leu Pro Ser Trp Asp Thr Cys Asn
   475                 480                 485                 490
   aag caa ctc ccc aag cca gct cca aca act ttc ttg tac aaa gtt gcc     1780
   Lys Gln Leu Pro Lys Pro Ala Pro Thr Thr Phe Leu Tyr Lys Val Ala
                   495                 500                 505
   tct aat gtt tagaagaata ttggaagaca ctatttttgc tattttatac             1829
   Ser Asn Val
   cacatagcaa cgatatttta acagattctc aagcaaattt tctagagtca agactatttt 1889
   ctcatagcaa aatttcacaa tgactgactc tgaatgaatt attttttttt atatatccta 1949
   ttttttatgt agtgtatgcg tagcctctat ctcgtatttt tttctatttc tcctccccac 2009
   accatcaatg ggactattct gttttgctgt tattcactag ttcttaacat tgtaaaaagt 2069
   ttgaccagcc tcagaaggct ttctctgtgt aaagaagtat aatttctctg ctgactccat 2129
   ttaatccact gcaaggcacc tagagagact gctcctattt taaaagtgat gcaagcatca 2189
   tgataagata tgtgtgaagc ccactaggaa ataaatcatt ctcttctcta tgtttgactt 2249
   gctagtaaac agaagacttc aagccagcca ggaaattaaa gtggcgacta aaacagcctt 2309
   aagaattgca gtggagcaaa ttggtcattt tttaaaaaaa tatattttaa cctacagtca 2369
   ccagttttca ttattctatt tacctcactg aagtactcgc atgttgtttg gtacccactg 2429
   agcaactgtt tcagttccta aggtatttgc tgagatgtgg gtgaactcca aatggagaag 2489
   tagtcactgt agactttctt catggttgac cactccaacc ttgctcactt ttgcttcttg 2549
   gccatccact cagctgatgt ttcctggaag tgctaatttt acctgtttcc aaattggaaa 2609
   cacatttctc aatcattccg ttctggcaaa tgggaaacat ccatttgctt tgggcacagt 2669
   ggggatgggc tgcaagttct tgcatatcct cccagtgaag catttatttg ctactatcag 2729
   attttaccac tatcaaatat aattcaaggg cagaattaaa cgtgagtgtg tgtgtgtgtg 2789
   tgtgtgtgtg tgctatgcat gctctaagtc tgcatgggat atgggaatgg aaaagggcaa 2849
   taagaaatta atacccttat gcagttgcat ttaaccttaa gaaaaatgtc cttgggataa 2909
   actccaatgt ttaatacatt gatttttttt ctaaagaaat gggttttaaa ctttggtatg 2969
   catcagaatt ccctatagat ctttttgaaa atataggtac ctgggtatca cacatagaac 3029
   ttttaattct gctggtgtag gctgttgccc aaacatctat aattttactg agctcttcaa 3089
   gtgattctga taacacagcc tggattgaga atttttataa gattggcaat ggaaaaacat 3149
   ttattctttt aaataataat ttttttaaaa cccaagaggt caggggattt tataaaccaa 3209
   tagccaagtg ttctttaaat aggaggcacc cttcccattg tgccaaaatc atcttttcat 3269
   ttattttgaa atttgtatga ttattttata cttgtatgtt gcctttcttc gaaggcgcct 3329
   gaagcacttt ataaacacaa atcctcacaa tacctctgtg aggtaggtaa atagtacttt 3389
   tctatgtagt aaacctggaa tatggagaat ttcataacag ttcattctac ttaataatgc 3449
   aataatggag ctccaagttg tcttggactt ctacaccaca ctcagacttc tggaaagttt 3509
   tctgtacctc attctttagt ccctgtcaag gttagtaaat aaaataagtg acataaaaaa 3569
   aaaaaaaaaa ctaaactact tgttgtgttg aaagttcctt tttgccagtt atgttcagga 3629
   aacccaataa cctgaaaaag tttgactttg atgtgacatc ttcatattca tcaatgctga 3689
   taattgtcca aaggcatctt cactatgtct gctaaataac atccaatgtg ggcgttatct 3749
   gttgtctagg ggatgaattt taagttacaa taaaatattt ttctttgttt tgcatcaaaa 3809
   aaaaaa 3815

<210> 96
<211> 509
<212> PRT
<213> Homo sapiens

<400> 96
Met Glu Leu Lys Lys Ser Pro Asp Gly Gly Trp Gly Trp Val Ile Val
1               5                   10                  15

Phe Val Ser Phe Leu Thr Gln Phe Leu Cys Tyr Gly Ser Pro Leu Ala
            20                  25                  30

Val Gly Val Leu Tyr Ile Glu Trp Leu Asp Ala Phe Gly Glu Gly Lys
        35                  40                  45

Gly Lys Thr Ala Trp Val Gly Ser Leu Ala Ser Gly Val Gly Leu Leu
    50                  55                  60

Ala Ser Pro Val Cys Ser Leu Cys Val Ser Ser Phe Gly Ala Arg Pro
65                  70                  75                  80

Val Thr Ile Phe Ser Gly Phe Met Val Ala Gly Gly Leu Met Leu Ser
                85                  90                  95

Ser Phe Ala Pro Asn Ile Tyr Phe Leu Phe Phe Ser Tyr Gly Ile Val
            100                 105                 110

Val Gly Leu Gly Cys Gly Leu Leu Tyr Thr Ala Thr Val Thr Ile Thr
        115                 120                 125

Cys Gln Tyr Phe Asp Asp Arg Arg Gly Leu Ala Leu Gly Leu Ile Ser
    130                 135                 140

Thr Gly Ser Ser Val Gly Leu Phe Ile Tyr Ala Ala Leu Gln Arg Met
145                 150                 155                 160

Leu Val Glu Phe Tyr Gly Leu Asp Gly Cys Leu Leu Ile Val Gly Ala
                165                 170                 175

Leu Ala Leu Asn Ile Leu Ala Cys Gly Ser Leu Met Arg Pro Leu Gln
            180                 185                 190

Ser Ser Asp Cys Pro Leu Pro Lys Lys Ile Ala Pro Glu Asp Leu Pro
        195                 200                 205

Asp Lys Tyr Ser Ile Tyr Asn Glu Lys Gly Lys Asn Leu Glu Glu Asn
    210                 215                 220

Ile Asn Ile Leu Asp Lys Ser Tyr Ser Ser Glu Glu Lys Cys Arg Ile
225                 230                 235                 240

Thr Leu Ala Asn Gly Asp Trp Lys Gln Asp Ser Leu Leu His Lys Asn

245                 250                 255

Pro Thr Val Thr His Thr Lys Glu Pro Glu Thr Tyr Lys Lys Lys Val
            260                 265                 270

Ala Glu Gln Thr Tyr Phe Cys Lys Gln Leu Ala Lys Arg Lys Trp Gln
        275                 280                 285

Leu Tyr Lys Asn Tyr Cys Gly Glu Thr Val Ala Leu Phe Lys Asn Lys
    290                 295                 300

Val Phe Ser Ala Leu Phe Ile Ala Ile Leu Leu Phe Asp Ile Gly Gly
305                 310                 315                 320

Phe Pro Pro Ser Leu Leu Met Glu Asp Val Ala Arg Ser Ser Asn Val
                325                 330                 335

Lys Glu Glu Glu Phe Ile Met Pro Leu Ile Ser Ile Ile Gly Ile Met
            340                 345                 350

Thr Ala Val Gly Lys Leu Leu Leu Gly Ile Leu Ala Asp Phe Lys Trp
        355                 360                 365

Ile Asn Thr Leu Tyr Leu Tyr Val Ala Thr Leu Ile Ile Met Gly Leu
    370                 375                 380

Ala Leu Cys Ala Ile Pro Phe Ala Lys Ser Tyr Val Thr Leu Ala Leu
385                 390                 395                 400

Leu Ser Gly Ile Leu Gly Phe Leu Thr Gly Asn Trp Ser Ile Phe Pro
                405                 410                 415

Tyr Val Thr Thr Lys Thr Val Gly Ile Glu Lys Leu Ala His Ala Tyr
            420                 425                 430

Gly Ile Leu Met Phe Phe Ala Gly Leu Gly Asn Ser Leu Gly Pro Pro
        435                 440                 445

Ser Leu Gly Trp Phe Tyr Asp Trp Thr Gln Thr Tyr Asp Ile Ala Phe
    450                 455                 460

Tyr Phe Ser Gly Phe Cys Val Leu Leu Gly Gly Phe Ile Leu Leu Leu
465                 470                 475                 480

Ala Ala Leu Pro Ser Trp Asp Thr Cys Asn Lys Gln Leu Pro Lys Pro
                485                 490                 495

Ala Pro Thr Thr Phe Leu Tyr Lys Val Ala Ser Asn Val
            500                 505

<210> 97

<400> 97
000


<210> 98
<211> 22
<212> DNA
<213> Artificial Sequence

<220>
<223> Description of Artifical Sequence: Primer/Probe

<400> 98
aattctcatc gctttcatga ag                                                22

<210> 99
<211> 26
<212> DNA
<213> Artificial Sequence

<220>
<223> Description of Artifical Sequence: Primer/Probe

<400> 99
taagtcgttc agacccatcc tcctct                                            26

<210> 100
<211> 25
<212> DNA
<213> Artificial Sequence

<220>
<223> Description of Artifical Sequence: Primer/Probe

<400> 100
ataggagtta ttggcaatct tctga                                             25

<210> 101
<211> 20
<212> DNA
<213> Artificial Sequence

<220>
<223> Description of Artifical Sequence: Primer/Probe

<400> 101
tgagcaaacg catggacatc                                                   20

<210> 102
<211> 28
<212> DNA
<213> Artificial Sequence

<220>
<223> Description of Artifical Sequence: Primer/Probe

<400> 102
tccatccaag tcacagaaag catcgcac                                          28

<210> 103
<211> 19
<212> DNA
<213> Artificial Sequence

<220>
<223> Description of Artifical Sequence: Primer/Probe

<400> 103
tgggttgagg cagctgtga                                                    19

<210> 104
<211> 22
<212> DNA
<213> Artificial Sequence

<220>
<223> Description of Artifical Sequence:  Primer/Probe

<400> 104
gaggctctct ccagtaacat ca                                                22

<210> 105
<211> 26
<212> DNA
<213> Artificial Sequence

<220>
<223> Description of Artifical Sequence:  Primer/Probe

<400> 105
tactctcctt gtcctctgag gcgctc                                            26

<210> 106
<211> 22
<212> DNA
<213> Artificial Sequence

<220>
<223> Description of Artifical Sequence:  Primer/Probe

<400> 106
gcagtttggt tgtttggttt ac                                                22

<210> 107
<211> 22
<212> DNA
<213> Artificial Sequence

<220>
<223> Description of Artifical Sequence:  Primer/Probe

<400> 107
gggaaatggg ttgtccatat at                                                22

<210> 108
<211> 26
<212> DNA
<213> Artificial Sequence

<220>
<223> Description of Artifical Sequence:  Primer/Probe

<400> 108
ttcctgcagc cttataagaa gtccac                                            26

<210> 109
<211> 22
<212> DNA
<213> Artificial Sequence

<220>

<223> Description of Artifical Sequence:  Primer/Probe

<400> 109
atctgaaatg gccagattta gc                                              22

<210> 110
<211> 22
<212> DNA
<213> Artificial Sequence

<220>
<223> Description of Artifical Sequence:  Primer/Probe

<400> 110
ccagtgccag taccagaata tc                                              22

<210> 111
<211> 26
<212> DNA
<213> Artificial Sequence

<220>
<223> Description of Artifical Sequence:  Primer/Probe

<400> 111
taaccagaca ctctcagctg caggca                                          26

<210> 112
<211> 19
<212> DNA
<213> Artificial Sequence

<220>
<223> Description of Artifical Sequence:  Primer/Probe

<400> 112
ctctctggcc tctcagcaa                                                  19

<210> 113
<211> 22
<212> DNA
<213> Artificial Sequence

<220>
<223> Description of Artifical Sequence:  Primer/Probe

<400> 113
gtctgcgtga agatcaatca tt                                              22

<210> 114
<211> 25
<212> DNA
<213> Artificial Sequence

<220>
<223> Description of Artifical Sequence:  Primer/Probe

<400> 114
taggacacgg actacgacca cgaca                                           25

<210> 115

<211> 22
<212> DNA
<213> Artificial Sequence

<220>
<223> Description of Artifical Sequence:  Primer/Probe

<400> 115
cggaccgtac ggagtagata ct                                                22

<210> 116
<211> 21
<212> DNA
<213> Artificial Sequence

<220>
<223> Description of Artifical Sequence:  Primer/Probe

<400> 116
ctactactgg tggctgcgaa t                                                 21

<210> 117
<211> 25
<212> DNA
<213> Artificial Sequence

<220>
<223> Description of Artifical Sequence:  Primer/Probe

<400> 117
tcagatcatg acccacttgc ctgga                                             25

<210> 118
<211> 22
<212> DNA
<213> Artificial Sequence

<220>
<223> Description of Artifical Sequence:  Primer/Probe

<400> 118
actcttcagc ggatgtagat ca                                                22

<210> 119
<211> 22
<212> DNA
<213> Artificial Sequence

<220>
<223> Description of Artifical Sequence:  Primer/Probe

<400> 119
ggacttctgt ggaccttatg tg                                                22

<210> 120
<211> 26
<212> DNA
<213> Artificial Sequence

<220>
<223> Description of Artifical Sequence:  Primer/Probe

<400> 120
tttttcctct ttgctggagt gctcct                                          26

<210> 121
<211> 22
<212> DNA
<213> Artificial Sequence

<220>
<223> Description of Artifical Sequence:  Primer/Probe

<400> 121
ttcctttggt ttctggaact tt                                              22

<210> 122
<211> 22
<212> DNA
<213> Artificial Sequence

<220>
<223> Description of Artifical Sequence:  Primer/Probe

<400> 122
tcagaaggga atcatgatat cg                                              22

<210> 123
<211> 27
<212> DNA
<213> Artificial Sequence

<220>
<223> Description of Artifical Sequence:  Primer/Probe

<400> 123
tccttgataa aactccaggc tcctttg                                         27

<210> 124
<211> 21
<212> DNA
<213> Artificial Sequence

<220>
<223> Description of Artifical Sequence:  Primer/Probe

<400> 124
tttggaaggt aggcatattg g                                               21

<210> 125
<211> 22
<212> DNA
<213> Artificial Sequence

<220>
<223> Description of Artifical Sequence:  Primer/Probe

<400> 125
gtcagcttct cttgctttga ga                                              22

<210> 126
<211> 23

<212> DNA
<213> Artificial Sequence

<220>
<223> Description of Artifical Sequence:  Primer/Probe

<400> 126
tcactgtcag gcactcgcca atg                                              23

<210> 127
<211> 22
<212> DNA
<213> Artificial Sequence

<220>
<223> Description of Artifical Sequence:  Primer/Probe

<400> 127
ctgtatttct ggaagcattc ca                                               22

<210> 128
<211> 22
<212> DNA
<213> Artificial Sequence

<220>
<223> Description of Artifical Sequence:  Primer/Probe

<400> 128
ttgatgtctc tgagatccaa ca                                               22

<210> 129
<211> 26
<212> DNA
<213> Artificial Sequence

<220>
<223> Description of Artifical Sequence:  Primer/Probe

<400> 129
tagtttccga gctctgggca tttcca                                           26

<210> 130
<211> 21
<212> DNA
<213> Artificial Sequence

<220>
<223> Description of Artifical Sequence:  Primer/Probe

<400> 130
catgctgtgc aaaattttct c                                                21

<210> 131
<211> 21
<212> DNA
<213> Artificial Sequence

<220>
<223> Description of Artifical Sequence:  Primer/Probe

<400> 131
aaaaggtgat gtctggagca t                                                21

<210> 132
<211> 26
<212> DNA
<213> Artificial Sequence

<220>
<223> Description of Artifical Sequence:  Primer/Probe

<400> 132
ttgtatgtca tgctctgtgc cagcct                                           26

<210> 133
<211> 21
<212> DNA
<213> Artificial Sequence

<220>
<223> Description of Artifical Sequence:  Primer/Probe

<400> 133
gatgtctgtg tcgtcaaaag g                                                21

<210> 134
<211> 21
<212> DNA
<213> Artificial Sequence

<220>
<223> Description of Artifical Sequence:  Primer/Probe

<400> 134
aaaaggtgat gtctggagca t                                                21

<210> 135
<211> 26
<212> DNA
<213> Artificial Sequence

<220>
<223> Description of Artifical Sequence:  Primer/Probe

<400> 135
ttgtatgtca tgctctgtgc cagcct                                           26

<210> 136
<211> 21
<212> DNA
<213> Artificial Sequence

<220>
<223> Description of Artifical Sequence:  Primer/Probe

<400> 136
gatgtctgtg tcgtcaaaag g                                                21

<210> 137
<211> 21
<212> DNA

<213> Artificial Sequence

<220>
<223> Description of Artifical Sequence:  Primer/Probe

<400> 137
aaaaggtgat gtctggagca t                                                21

<210> 138
<211> 26
<212> DNA
<213> Artificial Sequence

<220>
<223> Description of Artifical Sequence:  Primer/Probe

<400> 138
ttgtatgtca tgctctgtgc cagcct                                           26

<210> 139
<211> 21
<212> DNA
<213> Artificial Sequence

<220>
<223> Description of Artifical Sequence:  Primer/Probe

<400> 139
gatgtctgtg tcgtcaaaag g                                                21

<210> 140
<211> 20
<212> DNA
<213> Artificial Sequence

<220>
<223> Description of Artifical Sequence:  Primer/Probe

<400> 140
cctacgttac cgtgctcaag                                                  20

<210> 141
<211> 26
<212> DNA
<213> Artificial Sequence

<220>
<223> Description of Artifical Sequence:  Primer/Probe

<400> 141
tcgctaacac caccgacaag gagcta                                           26

<210> 142
<211> 21
<212> DNA
<213> Artificial Sequence

<220>
<223> Description of Artifical Sequence:  Primer/Probe

<400> 142

gacgttgtgc aaggagagaa c                                                21

<210> 143
<211> 20
<212> DNA
<213> Artificial Sequence

<220>
<223> Description of Artifical Sequence:  Primer/Probe

<400> 143
cctacgttac cgtgctcaag                                                  20

<210> 144
<211> 26
<212> DNA
<213> Artificial Sequence

<220>
<223> Description of Artifical Sequence:  Primer/Probe

<400> 144
tcgctaacac caccgacaag gagcta                                           26

<210> 145
<211> 21
<212> DNA
<213> Artificial Sequence

<220>
<223> Description of Artifical Sequence:  Primer/Probe

<400> 145
gacgttgtgc aaggagagaa c                                                21

<210> 146
<211> 19
<212> DNA
<213> Artificial Sequence

<220>
<223> Description of Artifical Sequence:  Primer/Probe

<400> 146
tcaccgtagc cgtgaagat                                                   19

<210> 147
<211> 26
<212> DNA
<213> Artificial Sequence

<220>
<223> Description of Artifical Sequence:  Primer/Probe

<400> 147
taaagacgat gccactgaca aggacc                                           26

<210> 148
<211> 22
<212> DNA
<213> Artificial Sequence

<220>
<223> Description of Artifical Sequence: Primer/Probe

<400> 148
ttcatcatct ccatctcaga ca                                                22

<210> 149
<211> 22
<212> DNA
<213> Artificial Sequence

<220>
<223> Description of Artifical Sequence: Primer/Probe

<400> 149
ggactttgat cccctacaga tg                                                22

<210> 150
<211> 26
<212> DNA
<213> Artificial Sequence

<220>
<223> Description of Artifical Sequence: Primer/Probe

<400> 150
ttcaaatgaa gaggacatcc tctcca                                            26

<210> 151
<211> 22
<212> DNA
<213> Artificial Sequence

<220>
<223> Description of Artifical Sequence: Primer/Probe

<400> 151
ctgagaacgg atagctgaga ac                                                22

<210> 152
<211> 20
<212> DNA
<213> Artificial Sequence

<220>
<223> Description of Artifical Sequence: Primer/Probe

<400> 152
cgctactact acagccgcaa                                                   20

<210> 153
<211> 23
<212> DNA
<213> Artificial Sequence

<220>
<223> Description of Artifical Sequence: Primer/Probe

<400> 153
tgtgatccgc gcctacctgg agt                                               23

<210> 154
<211> 20
<212> DNA
<213> Artificial Sequence

<220>
<223> Description of Artifical Sequence:  Primer/Probe

<400> 154
gggcggcttc atgtagtact                                                    20

<210> 155
<211> 20
<212> DNA
<213> Artificial Sequence

<220>
<223> Description of Artifical Sequence:  Primer/Probe

<400> 155
cgctactact acagccgcaa                                                    20

<210> 156
<211> 23
<212> DNA
<213> Artificial Sequence

<220>
<223> Description of Artifical Sequence:  Primer/Probe

<400> 156
tgtgatccgc gcctacctgg agt                                                23

<210> 157
<211> 20
<212> DNA
<213> Artificial Sequence

<220>
<223> Description of Artifical Sequence:  Primer/Probe

<400> 157
gggcggcttc atgtagtact                                                    20

<210> 158
<211> 22
<212> DNA
<213> Artificial Sequence

<220>
<223> Description of Artifical Sequence:  Primer/Probe

<400> 158
tctacaccat cagctgtatg ca                                                 22

<210> 159
<211> 26
<212> DNA
<213> Artificial Sequence

<220>
<223> Description of Artifical Sequence:  Primer/Probe

<400> 159
tcaccaccct cacactcatc ttcatc                                          26

<210> 160
<211> 22
<212> DNA
<213> Artificial Sequence

<220>
<223> Description of Artifical Sequence:  Primer/Probe

<400> 160
gcagtgcagc tgtcatatag aa                                              22

<210> 161
<211> 22
<212> DNA
<213> Artificial Sequence

<220>
<223> Description of Artifical Sequence:  Primer/Probe

<400> 161
ccatttactg gtgcacagaa gt                                              22

<210> 162
<211> 26
<212> DNA
<213> Artificial Sequence

<220>
<223> Description of Artifical Sequence:  Primer/Probe

<400> 162
tatccctctg gctgtcacct ctctca                                          26

<210> 163
<211> 22
<212> DNA
<213> Artificial Sequence

<220>
<223> Description of Artifical Sequence:  Primer/Probe

<400> 163
ggagtccaga atctggaaga gt                                              22

<210> 164
<211> 22
<212> DNA
<213> Artificial Sequence

<220>
<223> Description of Artifical Sequence:  Primer/Probe

<400> 164
ccatttactg gtgcacagaa gt                                              22

<210> 165
<211> 26
<212> DNA
<213> Artificial Sequence

<220>
<223> Description of Artifical Sequence:  Primer/Probe

<400> 165
tatccctctg gctgtcacct ctctca                                          26

<210> 166
<211> 22
<212> DNA
<213> Artificial Sequence

<220>
<223> Description of Artifical Sequence:  Primer/Probe

<400> 166
ggagtccaga atctggaaga gt                                              22

<210> 167

<400> 167
000

<210> 168
<211> 20
<212> DNA
<213> Artificial Sequence

<220>
<223> Description of Artifical Sequence:  Primer/Probe

<400> 168
ctggccaaga gctacaatga                                                 20

<210> 169
<211> 26
<212> DNA
<213> Artificial Sequence

<220>
<223> Description of Artifical Sequence:  Primer/Probe

<400> 169
tcatcagaca gaacgtgcag gtgttt                                          26

<210> 170
<211> 20
<212> DNA
<213> Artificial Sequence

<220>
<223> Description of Artifical Sequence:  Primer/Probe

<400> 170
aggccatcta tggctttcat                                                 20

<210> 171

<211> 22
<212> DNA
<213> Artificial Sequence

<220>
<223> Description of Artifical Sequence:  Primer/Probe

<400> 171
ttcctatggc attgttgtag gt                                                22

<210> 172
<211> 29
<212> DNA
<213> Artificial Sequence

<220>
<223> Description of Artifical Sequence:  Primer/Probe

<400> 172
ttggtttatt atacactgca acagtgacc                                         29

<210> 173
<211> 22
<212> DNA
<213> Artificial Sequence

<220>
<223> Description of Artifical Sequence:  Primer/Probe

<400> 173
atcgtcaaaa tactggcacg ta                                                22